# NEW EUROPEAN PATENT SPECIFICATION

(11) **EP 3 033 102 B2**
(45) Date of publication and mention of the opposition decision: **20.12.2023**
(45) Mention of the grant of the patent: 23.10.2019
(21) Application number: 14836306.2
(22) Date of filing: 13.08.2014
(51) Int. Cl.: A61K 47/69, A61K 39/00

(54) **PEPTIDE CONJUGATED PARTICLES**
PEPTIDKONJUGIERTE TEILCHEN
PARTICULES CONJUGUÉES À UN PEPTIDE

(30) Priority: 13.08.2013 US 201361865389 P; 23.08.2013 US 201361869279 P; 04.10.2013 US 201361887112 P
(43) Date of publication of application: 22.06.2016
(60) Divisional application: 19204340.4
(73) Proprietor: Northwestern University, Evanston, IL 60208 (US)
(72) Inventor: SHEA, Lonnie D, Evanston Illinois 60208 (US); MILLER, Stephen D., Evanston Illinois 60208 (US); YAP, Jonathan Woon Teck, Evanston Illinois 60208 (US); GETTS, Daniel R., Washington District of Columbia 20018 (US); MCCARTHY, Derrick, Evanston Illinois 60208 (US)
(74) Representative: Carpmaels & Ransford LLP
(86) International application number: PCT/US2014/050962
(87) International publication number: WO 2015/023796

(56) References cited:
- EP-A1- 2 255 831
- WO-A1-2010/060155
- WO-A1-2010/085509
- WO-A1-2011/103588
- WO-A2-2012/065153
- WO-A2-2012/101638
- WO-A2-2013/192532
- WO-A2-2013/192532
- WO-A2-2014/160465
- US-A1- 2005 002 999
- US-A1- 2008 124 350
- US-A1- 2008 207 515
- US-A1- 2008 268 552
- US-A1- 2009 123 509
- US-A1- 2009 304 726
- US-A1- 2009 325 931
- US-A1- 2010 151 000
- US-A1- 2012 076 831
- US-A1- 2013 202 659
- LUDVIG M SOLLID ET AL: "Nomenclature and listing of celiac disease relevant gluten T-cell epitopes restricted by HLA-DQ molecules", IMMUNOGENETICS, SPRINGER, BERLIN, DE, vol. 64, no. 6, 10 February 2012 (2012-02-10), pages 455-460, XP035054204, ISSN: 1432-1211, DOI: 10.1007/S00251-012-0599-Z
- S. SENGER ET AL: "Identification of Immunodominant Epitopes of -Gliadin in HLA-DQ8 Transgenic Mice following Oral Immunization", THE JOURNAL OF IMMUNOLOGY, vol. 175, no. 12, 8 December 2005 (2005-12-08), pages 8087-8095, XP055234593, US ISSN: 0022-1767, DOI: 10.4049/jimmunol.175.12.8087
- WAN-UK KIM ET AL: "Suppression of collagen-induced arthritis by single administration of poly(lactic-co-glycolic acid) nanoparticles entrapping type II collagen: A novel treatment strategy for induction of oral tolerance", ARTHRITIS & RHEUMATISM, vol. 46, no. 4, 1 April 2002 (2002-04-01), pages 1109-1120, XP055012144, ISSN: 0004-3591, DOI: 10.1002/art.10198
- FABIENNE DANHIER ET AL: "PLGA-based nanoparticles: An overview of biomedical applications", JOURNAL OF CONTROLLED RELEASE, vol. 161, no. 2, 4 February 2012 (2012-02-04), pages 505-522, XP055051719, ISSN: 0168-3659, DOI: 10.1016/j.jconrel.2012.01.043
- Takami Akagi ET AL: "Biodegradable Nanoparticles as Vaccine Adjuvants and Delivery Systems: Regulation of Immune Responses by Nanoparticle-Based Vaccine" In: "Bioactive Surfaces", 1 January 2011 (2011-01-01), Springer Berlin Heidelberg, Berlin, Heidelberg, XP055215260, ISSN: 0065-3195 ISBN: 978-3-64-220155-4 vol. 247, pages 31-64, DOI: 10.1007/12_2011_150, * chapter 5.2 *
- S HONARY ET AL: "Effect of Zeta Potential on the Properties of Nano-Drug Delivery Systems - A Review (Part 2)", TROPICAL JOURNAL OF PHARMACEUTICAL RESEARCH, vol. 12, no. 2, 9 May 2013 (2013-05-09), XP55344823, NG ISSN: 1596-5996, DOI: 10.4314/tjpr.v12i2.20
- GONDEAU ET AL.: 'Design of a novel class of peptide inhibitors of cyclin-dependent kinase/cyclin activation' J BIOL CHEM. vol. 80, 13 January 2005, pages 13793 - 13800, XP004404215
- KEEGAN et al.: "Biodegradable Microspheres with enhanced capacity for covalently bound surface ligands", Macromolecules, vol. 37, 2004, pages 9779-9784,
- Keegan Mark Edwin: "Biodegradable Microspheres with enhanced Capacity for Surface Ligand Conjugation", PhD Dissertation Cornell University, 1 May 2004 (2004-05-01), pages 1-118,

### SEQUENCE LISTING

<110> Cour Pharmaceuticals
<120> Peptide Conjugated Particles
<130> COUR-002/01US
<160> 5140
<170> PatentIn version 3.5
<210> 1
   <211> 21
   <212> PRT
   <213> Mus musculus
<400> 1
   Met Glu Val Gly Trp Tyr Arg Ser Pro Phe Ser Arg Val Val His Leu
   1               5                   10                  15
   Tyr Arg Asn Gly Lys
               20
<210> 2
   <211> 9
   <212> PRT
   <213> Homo sapiens
<400> 2
   Ala Ser Phe Asp Lys Ala Lys Leu Lys
   1               5
<210> 3
   <211> 14
   <212> PRT
   <213> Oryctolagus cuniculus
<400> 3
   Ala Ser Gln Lys Arg Pro Ser Gln Arg His Gly Ser Lys Tyr
   1               5                   10
<210> 4
   <211> 20
   <212> PRT
   <213> Homo sapiens
<400> 4
   Ala Ser Gln Lys Arg Pro Ser Gln Arg His Gly Ser Lys Tyr Leu Ala
   1               5                   10                  15
   Thr Ala Ser Thr
               20
<210> 5
   <211> 20
   <212> PRT
   <213> Homo sapiens
<400> 5
   Ala Thr Gly Leu Cys Phe Phe Gly Val Ala Leu Phe Cys Gly Cys Gly
   1               5                   10                  15
   His Glu Ala Leu
               20
<210> 6
   <211> 20
   <212> PRT
   <213> Homo sapiens
<400> 6
   Ala Val Pro Val Tyr Ile Tyr Phe Asn Thr Trp Thr Thr Cys Gln Ser
   1               5                   10                  15
   Ile Ala Phe Pro
               20
<210> 7
   <211> 9
   <212> PRT
   <213> Human herpesvirus
<400> 7
   Cys Leu Gly Gly Leu Leu Thr Met Val
   1 5
<210> 8
   <211> 9
   <212> PRT
   <213> Human herpesvirus
<400> 8
   Cys Arg Val Leu Cys Cys Tyr Val Leu
   1 5
<210> 9
   <211> 9
   <212> PRT
   <213> Homo sapiens
<400> 9
   Asp Ala Lys Ile Arg Ile Phe Asp Leu
   1 5
<210> 10
   <211> 18
   <212> PRT
   <213> Homo sapiens
<400> 10 Pro Leu
<210> 11
   <211> 15
   <212> PRT
   <213> Unknown
<220>
   <223> epitope involved in multiple sclerosis
<400> 11
   Glu Asn Pro Val Val Ala Phe Phe Lys Asn Ile Val Thr Pro Arg
   1 5 10 15
<210> 12
   <211> 15
   <212> PRT
   <213> Unknown
<220>
   <223> epitope involved in multiple sclerosis
<400> 12
   Glu Asn Pro Val Val Asp Phe Phe Lys Asn Ile Val Thr Pro Arg
   1               5                   10                  15
<210> 13
   <211> 15
   <212> PRT
   <213> Unknown
<220>
   <223> epitope involved in multiple sclerosis
<400> 13
   Glu Asn Pro Val Val His Phe Phe Ala Asn Ile Val Thr Pro Arg
   1               5                   10                  15
<210> 14
   <211> 17
   <212> PRT
   <213> Unknown
<220>
   <223> epitope involved in multiple sclerosis
<400> 14
   Glu Asn Pro Val Val His Phe Phe Ala Asn Ile Val Thr Pro Arg Thr
   1               5                   10                  15
   Pro
<210> 15
   <211> 15
   <212> PRT
   <213> Homo sapiens
<400> 15
   Glu Asn Pro Val Val His Phe Phe Lys Asn Ile Val Thr Pro Arg
   1               5                   10                  15
<210> 16
   <211> 17
   <212> PRT
   <213> Homo sapiens
<400> 16
   Glu Asn Pro Val Val His Phe Phe Lys Asn Ile Val Thr Pro Arg Thr
   1               5                   10                  15
   Pro
<210> 17
   <211> 20
   <212> PRT
   <213> Homo sapiens
<400> 17
   Phe Gly Asp Tyr Lys Thr Thr Ile Cys Gly Lys Gly Leu Ser Ala Thr
   1 5 10 15
   Val Thr Gly Gly
   20
<210> 18
   <211> 9
   <212> PRT
   <213> Homo sapiens
<400> 18
   Phe Leu Tyr Gly Ala Leu Leu Leu Ala
   1 5
<210> 19
   <211> 21
   <212> PRT
   <213> Homo sapiens
<400> 19
   Phe Tyr Tyr Thr Thr Gly Ala Val Arg Gln Ile Phe Gly Asp Tyr Lys
   1 5 10 15
   Thr Thr Ile Cys Gly
   20
<210> 20
   <211> 24
   <212> PRT
   <213> Human herpesvirus
<400> 20
   Gly Gly Asp Asn His Gly Arg Gly Arg Gly Arg Gly Arg Gly Arg Gly
   1 5 10 15
   Gly Gly Arg Pro Gly Ala Pro Gly
   20
<210> 21
   <211> 9
   <212> PRT
   <213> Human herpesvirus
<400> 21
   Gly Leu Cys Thr Leu Val Ala Met Leu
   1 5
<210> 22
   <211> 13
   <212> PRT
   <213> Mus musculus
<400> 22
   His Cys Leu Gly Lys Trp Leu Gly His Pro Asp Lys Phe
   1               5                   10
<210> 23
   <211> 18
   <212> PRT
   <213> Mus musculus
<400> 23
   Ile Phe Lys Leu Gly Gly Arg Asp Ser Arg Ser Gly Ser Pro Met Ala
   1               5                   10                  15
   Arg Arg
<210> 24
   <211> 15
   <212> PRT
   <213> Homo sapiens
<400> 24
   Ile Thr Arg Glu Glu Lys Pro Ala Val Thr Ala Ala Pro Lys Lys
   1               5                   10                  15
<210> 25
   <211> 13
   <212> PRT
   <213> Homo sapiens
<400> 25
   Ile Val Thr Pro Arg Thr Pro Pro Pro Ser Gln Gly Lys
   1               5                   10
<210> 26
   <211> 20
   <212> PRT
   <213> Homo sapiens
<400> 26
   Lys Gly Leu Ser Ala Thr Val Thr Gly Gly Gln Lys Gly Arg Gly Tyr
   1               5                   10                  15
   Arg Gly Gln His
               20
<210> 27
   <211> 14
   <212> PRT
   <213> Homo sapiens
<400> 27
   Lys Ile Phe Lys Leu Gly Gly Arg Asp Ser Arg Ser Gly Ser
   1 5 10
<210> 28
   <211> 20
   <212> PRT
   <213> Homo sapiens
<400> 28
   Leu Phe Cys Gly Cys Gly His Glu Ala Leu Thr Gly Thr Glu Lys Leu
   1 5 10 15
   Ile Glu Thr Tyr
   20
<210> 29
   <211> 10
   <212> PRT
   <213> Human herpesvirus
<400> 29
   Leu Leu Asp Phe Val Arg Phe Met Gly Val
   1 5 10
<210> 30
   <211> 9
   <212> PRT
   <213> Human herpesvirus
<400> 30
   Leu Leu Trp Thr Leu Val Val Leu Leu
   1 5
<210> 31
   <211> 20
   <212> PRT
   <213> Homo sapiens
<400> 31
   Leu Pro Arg His Arg Asp Thr Gly Ile Leu Asp Ser Ile Gly Arg Phe
   1 5 10 15
   Phe Gly Gly Asp
   20
<210> 32
   <211> 20
   <212> PRT
   <213> Homo sapiens
<400> 32
   Leu Val Gly Ala Pro Phe Ala Ser Leu Val Ala Thr Gly Leu Cys Phe
   1               5                   10                  15
   Phe Gly Val Ala
               20
<210> 33
   <211> 20
   <212> PRT
   <213> Homo sapiens
<400> 33
   Leu Tyr Gly Ala Leu Leu Leu Ala Glu Gly Phe Tyr Thr Thr Gly Ala
   1               5                   10                  15
   Val Arg Gln Ile
               20
<210> 34
   <211> 19
   <212> PRT
   <213> Homo sapiens
<400> 34
   Met Ala Ala Thr Tyr Asn Phe Ala Val Leu Lys Leu Met Gly Arg Phe
   1               5                   10                  15
   Thr Lys Phe
<210> 35
   <211> 13
   <212> PRT
   <213> Human herpesvirus
<400> 35
   Met Asp Arg Pro Arg Thr Pro Pro Pro Ser Tyr Ser Glu
   1               5                   10
<210> 36
   <211> 9
   <212> PRT
   <213> Human herpesvirus
<400> 36
   Asn Leu Val Pro Met Val Ala Thr Val
   1               5
<210> 37
   <211> 20
   <212> PRT
   <213> Mus musculus
<400> 37
   Asn Pro Val Val His Phe Phe Lys Asn Ile Val Thr Pro Arg Thr Pro
   1               5                   10                  15
   Pro Pro Ser Gln
               20
<210> 38
   <211> 14
   <212> PRT
   <213> Mus musculus
<400> 38
   Asn Thr Trp Thr Thr Cys Gln Ser Ile Ala Phe Pro Ser Lys
   1               5                   10
<210> 39
   <211> 20
   <212> PRT
   <213> Homo sapiens
<400> 39
   Gln Lys Gly Arg Gly Ser Arg Gly Gln His Gln Ala His Ser Leu Glu
   1               5                   10                  15
   Arg Val Cys His
               20
<210> 40
   <211> 8
   <212> PRT
   <213> Human herpesvirus
<400> 40
   Arg Ala Lys Phe Lys Gln Leu Leu
   1               5
<210> 41
   <211> 9
   <212> PRT
   <213> Human herpesvirus
<400> 41
   Arg Pro Pro Ile Phe Ile Arg Arg Leu
   1               5
<210> 42
   <211> 20
   <212> PRT
   <213> Homo sapiens
<400> 42
   Ser Lys Thr Ser Ala Ser Ile Gly Ser Leu Cys Ala Asp Ala Arg Met
   1               5                   10                  15
   Tyr Gly Val Leu
               20
<210> 43
   <211> 9
   <212> PRT
   <213> Homo sapiens
<400> 43
   Ser Leu Ser Arg Phe Ser Trp Gly Ala
   1               5
<210> 44
   <211> 9
   <212> PRT
   <213> Human herpesvirus
<400> 44
   Ser Val Arg Asp Arg Leu Ala Arg Leu
   1               5
<210> 45
   <211> 12
   <212> PRT
   <213> Unknown
<220>
   <223> epitope involved in multiple sclerosis
<400> 45
   Ser Val Ser Val Gly Met Lys Pro Ser Pro Arg Pro
   1               5                   10
<210> 46
   <211> 15
   <212> PRT
   <213> Human herpesvirus
<400> 46
   Thr Gly Gly Val Tyr His Phe Val Lys Lys His Val His Glu Ser
   1               5                   10                  15
<210> 47
   <211> 20
   <212> PRT
   <213> Homo sapiens
<400> 47
   Thr Gly Thr Glu Lys Leu Ile Glu Thr Tyr Phe Ser Lys Asn Tyr Gln
   1               5                   10                  15
   Asp Tyr Glu Tyr
               20
<210> 48
   <211> 10
   <212> PRT
   <213> Human herpesvirus
<400> 48
   Thr Pro Arg Val Thr Gly Gly Gly Ala Met
   1               5                   10
<210> 49
   <211> 20
   <212> PRT
   <213> Homo sapiens
<400> 49
   Val Asp Ala Gln Gly Thr Leu Ser Lys Ile Phe Lys Leu Gly Gly Arg
   1               5                   10                  15
   Asp Ser Arg Ser
               20
<210> 50
   <211> 13
   <212> PRT
   <213> Cavia porcellus
<400> 50
   Val His Phe Phe Lys Asn Ile Val Thr Pro Arg Thr Pro
   1               5                   10
<210> 51
   <211> 9
   <212> PRT
   <213> Homo sapiens
<400> 51
   Val Met Ala Pro Arg Thr Leu Val Leu
   1               5
<210> 52
   <211> 20
   <212> PRT
   <213> Mus musculus
<400> 52
   Val Thr Pro Arg Thr Pro Pro Pro Ser Gln Gly Lys Gly Arg Gly Leu
   1               5                   10                  15
   Ser Leu Ser Arg
               20
<210> 53
   <211> 9
   <212> PRT
   <213> Homo sapiens
<400> 53
   Val Val His Phe Phe Lys Asn Ile Val
   1               5
<210> 54
   <211> 20
   <212> PRT
   <213> Homo sapiens
<400> 54
   Trp Thr Thr Cys Gln Ser Ile Ala Phe Pro Ser Lys Thr Ser Ala Ser
   1               5                   10                  15
   Ile Gly Ser Leu
               20
<210> 55
   <211> 23
   <212> PRT
   <213> Human T-cell lymphotrophic virus
<400> 55
   Ile Gln Ala Ile Val Ser Ser Pro Cys His Asn Ser Leu Ile Leu Pro
   1               5                   10                  15
   Pro Phe Ser Leu Ser Pro Val
               20
<210> 56
   <211> 24
   <212> PRT
   <213> Human T-cell lymphotrophic virus
<400> 56
   Leu Pro Pro Thr Ala Pro Pro Leu Leu Pro His Ser Asn Leu Asp His
   1               5                   10                  15
   Ile Leu Glu Pro Ser Ile Pro Trp
               20
<210> 57
   <211> 20
   <212> PRT
   <213> Human T-cell lymphotrophic virus
<400> 57
   Pro Pro Pro Tyr Val Glu Pro Thr Ala Pro Gln Val Leu Pro Val Met
   1               5                   10                  15
   His Pro His Gly
               20
<210> 58
   <211> 23
   <212> PRT
   <213> Human T-cell lymphotrophic virus
<400> 58
   Gln Gly Gly Leu Cys Lys Ala Leu Gln Glu Gln Cys Arg Phe Pro Asn
   1               5                   10                  15
   Ile Thr Asn Ser His Val Pro
               20
<210> 59
   <211> 20
   <212> PRT
   <213> Human T-cell lymphotrophic virus
<400> 59
   Arg Ala Ser Leu Ser Thr Trp His Val Leu Tyr Ser Pro Asn Val Ser
   1               5                   10                  15
   Val Pro Ser Ser
               20
<210> 60
   <211> 10
   <212> PRT
   <213> Homo sapiens
<400> 60
   Phe Phe Lys Asn Ile Val Thr Pro Arg Thr
   1               5                   10
<210> 61
   <211> 9
   <212> PRT
   <213> Homo sapiens
<400> 61
   Phe Lys Asn Ile Val Thr Pro Arg Thr
   1               5
<210> 62
   <211> 11
   <212> PRT
   <213> Cavia porcellus
<400> 62
   His Phe Phe Lys Asn Ile Val Thr Pro Arg Thr
   1               5                   10
<210> 63
   <211> 15
   <212> PRT
   <213> Homo sapiens
<400> 63
   Lys Gly Phe Lys Gly Val Asp Ala Gln Gly Thr Leu Ser Lys Ile
   1               5                   10                  15
<210> 64
   <211> 13
   <212> PRT
   <213> Unknown
<220>
   <223> epitope involved in multiple sclerosis
<400> 64
   Val His Phe Phe Lys Asn Ile Val Thr Ala Arg Thr Pro
   1               5                   10
<210> 65
   <211> 13
   <212> PRT
   <213> Homo sapiens
<400> 65
   Val Val His Phe Phe Lys Asn Ile Val Thr Pro Arg Thr
   1               5                   10
<210> 66
   <211> 9
   <212> PRT
   <213> Human herpesvirus
<400> 66
   Ser Gln Ala Pro Leu Pro Cys Val Leu
   1 5
<210> 67
   <211> 12
   <212> PRT
   <213> Unknown
<220>
   <223> epitope involved in multiple sclerosis
<400> 67
   His Thr Gln Pro Tyr Ala Tyr Glu Ala Arg Asp His
   1 5 10
<210> 68
   <211> 15
   <212> PRT
   <213> Bos taurus
<400> 68
   Phe Lys Ala Asp Glu Lys Lys Phe Trp Gly Lys Tyr Leu Tyr Glu
   1 5 10 15
<210> 69
   <211> 16
   <212> PRT
   <213> Homo sapiens
<400> 69
   His Cys Leu Gly Lys Trp Leu Gly His Pro Asp Lys Phe Val Gly Ile
   1 5 10 15
<210> 70
   <211> 14
   <212> PRT
   <213> Mus musculus
<400> 70
   Lys Thr Thr Ile Cys Gly Lys Gly Leu Ser Ala Thr Val Thr
   1 5 10
<210> 71
   <211> 17
   <212> PRT
   <213> Homo sapiens
<400> 71
   Val Asp Asp Thr Gln Phe Val Arg Phe Asp Ser Asp Ala Ala Ser Pro
   1               5                   10                  15
   Arg
<210> 72
   <211> 22
   <212> PRT
   <213> Mus musculus
<400> 72
   Glu Lys Leu Ile Glu Thr Tyr Phe Ser Lys Asn Tyr Gln Asp Tyr Glu
   1               5                   10                  15
   Tyr Leu Ile Asn Val Ile
               20
<210> 73
   <211> 9
   <212> PRT
   <213> Mus musculus
<400> 73
   Ala Ser Gln Lys Arg Pro Ser Gln Arg
   1               5
<210> 74
   <211> 15
   <212> PRT
   <213> Homo sapiens
<400> 74
   Asp Glu Asn Pro Val Val His Phe Phe Lys Asn Ile Val Thr Pro
   1               5                   10                  15
<210> 75
   <211> 19
   <212> PRT
   <213> Homo sapiens
<400> 75
   Asp Glu Asn Pro Val Val His Phe Phe Lys Asn Ile Val Thr Pro Arg
   1               5                   10                  15
   Thr Pro Pro
<210> 76
   <211> 26
   <212> PRT
   <213> Homo sapiens
<400> 76
   Phe Lys Gly Val Asp Ala Gln Gly Thr Leu Ser Lys Ile Phe Lys Leu
   1 5 10 15
   Gly Gly Arg Asp Ser Arg Ser Gly Ser Pro
   20 25
<210> 77
   <211> 18
   <212> PRT
   <213> Homo sapiens
<400> 77
   Gly Thr Glu Lys Leu Ile Glu Thr Tyr Phe Ser Lys Asn Tyr Gln Asp
   1 5 10 15
   Tyr Glu
<210> 78
   <211> 16
   <212> PRT
   <213> Homo sapiens
<400> 78
   Gln Ser Ile Ala Phe Pro Ser Lys Thr Ser Ala Ser Ile Gly Ser Leu
   1 5 10 15
<210> 79
   <211> 25
   <212> PRT
   <213> Homo sapiens
<400> 79
   Ala Gln Gly Thr Leu Ser Lys Ile Phe Lys Leu Gly Gly Arg Asp Ser
   1 5 10 15
   Arg Ser Gly Ser Pro Met Ala Arg Arg
   20 25
<210> 80
   <211> 25
   <212> PRT
   <213> Homo sapiens
<400> 80
   Ala Ser Asp Tyr Lys Ser Ala His Lys Gly Leu Lys Gly Val Asp Ala
   1 5 10 15
   Gln Gly Thr Leu Ser Lys Ile Phe Lys
   20 25
<210> 81
   <211> 31
   <212> PRT
   <213> Homo sapiens
<400> 81
   Glu Val Gly Glu Leu Ser Arg Gly Lys Leu Tyr Ser Leu Gly Asn Gly
   1               5                   10                  15
   Arg Trp Met Leu Thr Leu Ala Lys Asn Met Glu Val Arg Ala Ile
               20                  25                  30
<210> 82
   <211> 18
   <212> PRT
   <213> Homo sapiens
<400> 82
   Gly Phe Tyr Thr Thr Gly Ala Val Arg Gln Ile Phe Gly Asp Tyr Lys
   1               5                   10                  15
   Thr Thr
<210> 83
   <211> 33
   <212> PRT
   <213> Homo sapiens
<400> 83
   Gly Asn Gly Arg Trp Met Leu Thr Leu Ala Lys Asn Met Glu Val Arg
   1               5                   10                  15
   Ala Ile Phe Thr Gly Tyr Tyr Gly Lys Gly Lys Pro Val Pro Thr Gln
               20                  25                  30
   Gly
<210> 84
   <211> 20
   <212> PRT
   <213> Homo sapiens
<400> 84
   Gly Gln Phe Arg Val Ile Gly Pro Arg His Pro Ile Arg Ala Leu Val
   1               5                   10                  15
   Gly Asp Glu Val
               20
<210> 85
   <211> 20
   <212> PRT
   <213> Homo sapiens
<400> 85
   His His Pro Ala Arg Thr Ala His Tyr Gly Ser Leu Pro Gln Lys Ser
   1               5                   10                  15
   His Gly Arg Thr
               20
<210> 86
   <211> 20
   <212> PRT
   <213> Homo sapiens
<400> 86
   Lys Asn Ile Val Thr Pro Arg Thr Pro Pro Pro Ser Gln Gly Lys Gly
   1               5                   10                  15
   Arg Gly Leu Ser
               20
<210> 87
   <211> 20
   <212> PRT
   <213> Homo sapiens
<400> 87
   Lys Tyr Leu Ala Thr Ala Ser Thr Met Asp His Ala Arg His Gly Phe
   1               5                   10                  15
   Leu Pro Arg His
               20
<210> 88
   <211> 19
   <212> PRT
   <213> Homo sapiens
<400> 88
   Leu Ser Arg Phe Ser Trp Gly Ala Glu Gly Gln Arg Pro Gly Phe Gly
   1               5                   10                  15
   Tyr Gly Gly
<210> 89
   <211> 21
   <212> PRT
   <213> Homo sapiens
<400> 89
   Met Glu Val Gly Trp Tyr Arg Pro Pro Phe Ser Arg Val Val His Leu
   1               5                   10                  15
   Tyr Arg Asn Gly Lys
               20
<210> 90
   <211> 20
   <212> PRT
   <213> Homo sapiens
<400> 90
   Asn Thr Trp Thr Thr Cys Gln Ser Ile Ala Phe Pro Ser Lys Thr Ser
   1               5                   10                  15
   Ala Ser Ile Gly
               20
<210> 91
   <211> 20
   <212> PRT
   <213> Homo sapiens
<400> 91
   Pro Ile Arg Ala Leu Val Gly Asp Glu Val Glu Leu Pro Cys Arg Ile
   1               5                   10                  15
   Ser Pro Gly Lys
               20
<210> 92
   <211> 12
   <212> PRT
   <213> Unknown
<220>
   <223> epitope involved in multiple sclerosis
<400> 92
   Gln Lys Ser Gln Arg Ser Gln Ala Glu Asn Pro Val
   1               5                   10
<210> 93
   <211> 12
   <212> PRT
   <213> Cavia porcellus
<400> 93
   Gln Lys Ser Gln Arg Ser Gln Asp Glu Asn Pro Val
   1 5 10
<210> 94
   <211> 20
   <212> PRT
   <213> Homo sapiens
<400> 94
   Gln Arg Pro Gly Phe Gly Tyr Gly Gly Arg Ala Ser Asp Tyr Lys Ser
   1 5 10 15
   Ala His Lys Gly
   20
<210> 95
   <211> 20
   <212> PRT
   <213> Homo sapiens
<400> 95
   Arg His Arg Asp Thr Gly Ile Leu Asp Ser Ile Gly Arg Phe Phe Gly
   1 5 10 15
   Gly Asp Arg Gly
   20
<210> 96
   <211> 20
   <212> PRT
   <213> Homo sapiens
<400> 96
   Ser Lys Ile Phe Lys Leu Gly Gly Arg Asp Ser Arg Ser Gly Ser Pro
   1 5 10 15
   Met Ala Arg Arg
   20
<210> 97
   <211> 21
   <212> PRT
   <213> Homo sapiens
<400> 97
   Ser Lys Thr Ser Ala Ser Ile Gly Ser Leu Cys Ala Asp Ala Arg Met
   1 5 10 15
   Tyr Gly Val Leu Pro
   20
<210> 98
   <211> 21
   <212> PRT
   <213> Homo sapiens
<400> 98
   Thr Gly Thr Glu Lys Leu Ile Glu Thr Tyr Phe Ser Lys Asn Tyr Gln
   1               5                   10                  15
   Asp Tyr Glu Tyr Leu
                   20
<210> 99
   <211> 13
   <212> PRT
   <213> Unknown
<220>
   <223> epitope involved in multiple sclerosis
<400> 99
   Val His Phe Phe Arg Asn Ile Val Thr Ala Arg Thr Pro
   1               5                   10
<210> 100
   <211> 13
   <212> PRT
   <213> Unknown
<220>
   <223> epitope involved in multiple sclerosis
<400> 100
   Val His Phe Phe Arg Asn Ile Val Thr Ala Arg Thr Pro
   1               5                   10
<210> 101
   <211> 20
   <212> PRT
   <213> Homo sapiens
<400> 101
   Val Val His Phe Phe Lys Asn Ile Val Thr Pro Arg Thr Pro Pro Pro
   1               5                   10                  15
   Ser Gln Gly Lys
               20
<210> 102
   <211> 21
   <212> PRT
   <213> Homo sapiens
<400> 102
   Ala Ser Gln Lys Arg Pro Ser Gln Arg His Gly Ser Lys Tyr Leu Ala
   1               5                   10                  15
   Thr Ala Ser Thr Met
               20
<210> 103
   <211> 44
   <212> PRT
   <213> Homo sapiens
<400> 103
   Ala Ser Gln Lys Arg Pro Ser Gln Arg His Gly Ser Lys Tyr Leu Ala
   1               5                   10                  15
   Thr Ala Ser Thr Met Asp His Ala Arg His Gly Phe Leu Pro Arg His
               20                  25                  30
   Arg Asp Thr Gly Ile Leu Asp Ser Ile Gly Arg Phe
           35                  40
<210> 104
   <211> 21
   <212> PRT
   <213> Homo sapiens
<400> 104
   Ala Ser Thr Met Asp His Ala Arg His Gly Phe Leu Pro Arg His Arg
   1               5                   10                  15
   Asp Thr Gly Ile Leu
               20
<210> 105
   <211> 23
   <212> PRT
   <213> Homo sapiens
<400> 105
   Ala Val Arg Gln Ile Phe Gly Asp Tyr Lys Thr Thr Ile Cys Gly Lys
   1               5                   10                  15
   Gly Leu Ser Ala Thr Val Thr
               20
<210> 106
   <211> 19
   <212> PRT
   <213> Homo sapiens
<400> 106
   Glu Asn Pro Val Val His Phe Phe Lys Asn Ile Val Thr Pro Arg Thr
   1               5                   10                  15
   Pro Pro Pro
<210> 107
   <211> 18
   <212> PRT
   <213> Homo sapiens
<400> 107
   Gly Phe Tyr Thr Thr Gly Ala Tyr Arg Gln Ile Phe Gly Asp Tyr Leu
   1               5                   10                  15
   Thr Thr
<210> 108
   <211> 20
   <212> PRT
   <213> Homo sapiens
<400> 108
   Gly Arg Ala Ser Asp Tyr Lys Ser Ala His Lys Gly Phe Lys Gly Val
   1               5                   10                  15
   Asp Ala Gln Gly
               20
<210> 109
   <211> 25
   <212> PRT
   <213> Homo sapiens
<400> 109
   Gly Val Leu Pro Trp Asn Ala Phe Pro Gly Lys Val Cys Gly Ser Asn
   1               5                   10                  15
   Leu Leu Ser Ile Cys Lys Thr Ala Glu
               20                  25
<210> 110
   <211> 12
   <212> PRT
   <213> Unknown
<220>
   <223> epitope involved in multiple sclerosis
<400> 110
   His Leu Ile Leu Ala Pro Ser Ile Arg His Ala Pro
   1 5 10
<210> 111
   <211> 12
   <212> PRT
   <213> Unknown
<220>
   <223> epitope involved in multiple sclerosis
<400> 111
   His Leu Val Met Ala Pro Ser Val Ala Arg Gly Leu
   1 5 10
<210> 112
   <211> 12
   <212> PRT
   <213> Unknown
<220>
   <223> epitope involved in multiple sclerosis
<400> 112
   His Val Gln Leu Ala Pro Pro Ile Ala Leu Arg His
   1 5 10
<210> 113
   <211> 12
   <212> PRT
   <213> Unknown
<220>
   <223> epitope involved in multiple sclerosis
<400> 113
   His Val Val Leu Ala Pro Asn Val Gln Ser Pro Leu
   1 5 10
<210> 114
   <211> 24
   <212> PRT
   <213> Homo sapiens
<400> 114
   His Tyr Gly Ser Leu Pro Gln Lys Ser His Gly Arg Thr Gln Asp Glu
   1 5 10 15
   Asn Pro Val Val His Phe Phe Lys
   20
<210> 115
   <211> 14
   <212> PRT
   <213> Homo sapiens
<400> 115
   Lys Lys Gln Ser Lys Pro Val Thr Thr Pro Glu Glu Ile Ala
   1 5 10
<210> 116
   <211> 33
   <212> PRT
   <213> Homo sapiens
<400> 116
   Lys Leu Ile Glu Thr Tyr Phe Ser Lys Asn Tyr Gln Asp Tyr Glu Tyr
   1 5 10 15
   Leu Ile Asn Val Ile His Ala Phe Gln Tyr Val Ile Tyr Gly Thr Ala
   20 25 30
   Ser
<210> 117
   <211> 30
   <212> PRT
   <213> Homo sapiens
<400> 117
   Leu Ala Thr Ala Ser Thr Met Asp His Ala Arg His Gly Phe Leu Pro
   1 5 10 15
   Arg His Arg Asp Thr Gly Ile Leu Asp Ser Ile Gly Arg Phe
   20 25 30
<210> 118
   <211> 23
   <212> PRT
   <213> Homo sapiens
<400> 118
   Leu Thr Phe Met Ile Ala Ala Thr Tyr Asn Phe Ala Val Leu Lys Leu
   1               5                   10                  15
   Met Gly Arg Gly Thr Lys Phe
               20
<210> 119
   <211> 20
   <212> PRT
   <213> Bos taurus
<400> 119
   Met Ile Ala Ala Thr Tyr Asn Phe Ala Val Leu Lys Leu Met Gly Arg
   1               5                   10                  15
   Gly Thr Lys Phe
               20
<210> 120
   <211> 21
   <212> PRT
   <213> Homo sapiens
<400> 120
   Arg His Gly Ser Lys Tyr Leu Ala Thr Ala Ser Thr Met Asp His Ala
   1               5                   10                  15
   Arg His Gly Phe Leu
               20
<210> 121
   <211> 22
   <212> PRT
   <213> Homo sapiens
<400> 121
   Ser Ile Ala Phe Pro Ser Lys Thr Ser Ala Ser Ile Gly Ser Leu Cys
   1               5                   10                  15
   Ala Asp Ala Arg Met Tyr
               20
<210> 122
   <211> 34
   <212> PRT
   <213> Homo sapiens
<400> 122
   Thr Gly Gly Gln Lys Gly Arg Gly Ser Arg Gly Gln His Gln Ala His
   1               5                   10                  15
   Ser Leu Glu Arg Val Cys His Cys Leu Gly Lys Trp Leu Gly His Pro
               20                  25                  30
   Asp Lys
<210> 123
   <211> 20
   <212> PRT
   <213> Bos taurus
<400> 123
   Thr Thr Ile Cys Gly Lys Gly Leu Ser Ala Thr Val Thr Gly Gly Gln
   1               5                   10                  15
   Lys Gly Arg Gly
               20
<210> 124
   <211> 14
   <212> PRT
   <213> Homo sapiens
<400> 124
   Val Val Thr Glu Ile Pro Lys Glu Glu Lys Asp Pro Gly Met
   1               5                   10
<210> 125
   <211> 12
   <212> PRT
   <213> Unknown
<220>
   <223> epitope involved in multiple sclerosis
<400> 125
   Trp His Ala Pro Gln Thr Pro Tyr Trp Ala Ser Met
   1               5                   10
<210> 126
   <211> 12
   <212> PRT
   <213> Unknown
<220>
   <223> epitope involved in multiple sclerosis
<400> 126
   Trp His Ile Pro Pro Asn Ile Gly Arg Thr Phe Ser
   1 5 10
<210> 127
   <211> 12
   <212> PRT
   <213> Unknown
<220>
   <223> epitope involved in multiple sclerosis
<400> 127
   Trp His Gln Thr Tyr Thr Ser Ser Leu Trp Glu Ser
   1 5 10
<210> 128
   <211> 12
   <212> PRT
   <213> Unknown
<220>
   <223> epitope involved in multiple sclerosis
<400> 128
   Trp His Thr Gln Pro Ala His Val Val Thr Pro Met
   1 5 10
<210> 129
   <211> 12
   <212> PRT
   <213> Unknown
<220>
   <223> epitope involved in multiple sclerosis
<400> 129
   Trp His Thr Ser Asp Pro Leu Pro Pro Ala Gln Pro
   1 5 10
<210> 130
   <211> 26
   <212> PRT
   <213> Homo sapiens
<400> 130
   Ala Leu Val Gly Asp Glu Val Glu Leu Pro Cys Arg Ile Ser Pro Gly
   1 5 10 15
   Lys Asn Ala Thr Gly Met Glu Leu Gly Trp
   20 25
<210> 131
   <211> 7
   <212> PRT
   <213> Unknown
<220>
   <223> epitope involved in multiple sclerosis
<400> 131
   Ala Arg Trp Glu Leu Asn Leu
   1 5
<210> 132
   <211> 22
   <212> PRT
   <213> Homo sapiens
<400> 132
   Ala Val Arg Gln Ile Phe Gly Asp Tyr Lys Thr Thr Ile Cys Gly Lys
   1 5 10 15
   Gly Leu Ser Ala Thr Val
   20
<210> 133
   <211> 20
   <212> PRT
   <213> Homo sapiens
<400> 133
   Asp Thr Gly Ile Leu Asp Ser Ile Gly Arg Phe Phe Gly Gly Asp Arg
   1 5 10 15
   Gly Ala Pro Lys
   20
<210> 134
   <211> 20
   <212> PRT
   <213> Homo sapiens
<400> 134
   Glu Lys Asp Arg Phe Ser Val Asn Leu Asp Val Ser His Phe Ser Pro
   1 5 10 15
   Glu Glu Leu Lys
   20
<210> 135
   <211> 7
   <212> PRT
   <213> Unknown
<220>
   <223> epitope involved in multiple sclerosis
<400> 135
   Glu Pro Leu Tyr Gly Pro Tyr
   1               5
<210> 136
   <211> 20
   <212> PRT
   <213> Homo sapiens
<400> 136
   Phe Asp Thr Gly Leu Ser Glu Met Arg Leu Glu Lys Asp Arg Phe Ser
   1               5                   10                  15
   Val Asn Leu Asp
               20
<210> 137
   <211> 26
   <212> PRT
   <213> Homo sapiens
<400> 137
   Gly Gln Phe Arg Val Ile Gly Pro Arg His Pro Ile Arg Ala Leu Val
   1               5                   10                  15
   Gly Asp Glu Val Glu Leu Pro Cys Arg Ile
               20                  25
<210> 138
   <211> 20
   <212> PRT
   <213> Homo sapiens
<400> 138
   Gly Ser Gly Lys Asp Ser His His Pro Ala Arg Thr Ala His Tyr Gly
   1               5                   10                  15
   Ser Leu Pro Gln
               20
<210> 139
   <211> 15
   <212> PRT
   <213> Homo sapiens
<400> 139
   Gly Ser Leu Pro Gln Lys Ser His Gly Arg Thr Gln Asp Glu Asn
   1 5 10 15
<210> 140
   <211> 20
   <212> PRT
   <213> Homo sapiens
<400> 140
   Gly Val Leu Thr Val Asn Gly Pro Arg Lys Gln Val Ser Gly Pro Glu
   1 5 10 15
   Arg Thr Ile Pro
   20
<210> 141
   <211> 23
   <212> PRT
   <213> Homo sapiens
<400> 141
   Gly Trp Tyr Arg Pro Pro Phe Ser Arg Val Val His Leu Tyr Arg Asn
   1 5 10 15
   Gly Lys Asp Gln Asp Gly Asp
   20
<210> 142
   <211> 20
   <212> PRT
   <213> Homo sapiens
<400> 142
   His Gly Phe Ile Ser Arg Glu Phe His Arg Lys Tyr Arg Ile Pro Ala
   1 5 10 15
   Asp Val Asp Pro
   20
<210> 143
   <211> 20
   <212> PRT
   <213> Homo sapiens
<400> 143
   His Gly Lys His Glu Glu Arg Gln Asp Glu His Gly Phe Ile Ser Arg
   1 5 10 15
   Glu Phe His Arg
   20
<210> 144
   <211> 20
   <212> PRT
   <213> Homo sapiens
<400> 144
   His Leu Leu Glu Ser Asp Leu Phe Pro Thr Ser Thr Ser Leu Ser Pro
   1               5                   10                  15
   Phe Tyr Leu Arg
               20
<210> 145
   <211> 25
   <212> PRT
   <213> Homo sapiens
<400> 145
   Ile Gly Glu Gly Lys Val Thr Leu Arg Ile Arg Asn Val Arg Phe Ser
   1               5                   10                  15
   Asp Glu Gly Gly Phe Thr Cys Phe Phe
               20                  25
<210> 146
   <211> 20
   <212> PRT
   <213> Homo sapiens
<400> 146
   Lys Tyr Arg Ile Pro Ala Asp Val Asp Pro Leu Thr Ile Thr Ser Ser
   1               5                   10                  15
   Leu Ser Ser Asp
               20
<210> 147
   <211> 7
   <212> PRT
   <213> Unknown
<220>
   <223> epitope involved in multiple sclerosis
<400> 147
   Leu Ala Thr Pro Asn Pro Ser
   1               5
<210> 148
   <211> 20
   <212> PRT
   <213> Homo sapiens
<400> 148
   Leu Thr Ile Thr Ser Ser Leu Ser Ser Asp Gly Val Leu Thr Val Asn
   1               5                   10                  15
   Gly Pro Arg Lys
               20
<210> 149
   <211> 24
   <212> PRT
   <213> Homo sapiens
<400> 149
   Leu Tyr Arg Asn Gly Lys Asp Gln Asp Gly Asp Ala Pro Glu Tyr Arg
   1               5                   10                  15
   Gly Arg Thr Glu Leu Leu Lys Asp
               20
<210> 150
   <211> 20
   <212> PRT
   <213> Homo sapiens
<400> 150
   Met Asp Ile Ala Ile His His Pro Trp Ile Arg Arg Pro Phe Phe Pro
   1               5                   10                  15
   Phe His Ser Pro
               20
<210> 151
   <211> 7
   <212> PRT
   <213> Unknown
<220>
   <223> epitope involved in multiple sclerosis
<400> 151
   Pro Glu Phe Ala Pro Trp Ala
   1               5
<210> 152
   <211> 7
   <212> PRT
   <213> Unknown
<220>
   <223> epitope involved in multiple sclerosis
<400> 152
   Pro Glu Gln Pro Pro Ile His
   1               5
<210> 153
   <211> 25
   <212> PRT
   <213> Homo sapiens
<400> 153
   Pro Glu Tyr Arg Gly Arg Thr Glu Leu Leu Lys Asp Ala Ile Gly Glu
   1               5                   10                  15
   Gly Lys Val Thr Leu Arg Ile Arg Asn
               20                  25
<210> 154
   <211> 20
   <212> PRT
   <213> Homo sapiens
<400> 154
   Pro Pro Ser Phe Leu Arg Ala Pro Ser Trp Phe Asp Thr Gly Leu Ser
   1               5                   10                  15
   Glu Met Arg Leu
               20
<210> 155
   <211> 20
   <212> PRT
   <213> Homo sapiens
<400> 155
   Pro Val Val His Phe Phe Lys Asn Ile Val Thr Pro Arg Thr Pro Pro
   1               5                   10                  15
   Pro Ser Gln Gly
               20
<210> 156
   <211> 20
   <212> PRT
   <213> Homo sapiens
<400> 156
   Gln Gly Lys Gly Arg Gly Leu Ser Leu Ser Arg Phe Ser Trp Gly Ala
   1 5 10 15
   Glu Gly Gln Arg
   20
<210> 157
   <211> 20
   <212> PRT
   <213> Homo sapiens
<400> 157
   Gln Val Ser Gly Pro Glu Arg Thr Ile Pro Ile Thr Arg Glu Glu Lys
   1 5 10 15
   Pro Ala Val Thr
   20
<210> 158
   <211> 25
   <212> PRT
   <213> Homo sapiens
<400> 158
   Arg Asp His Ser Tyr Gln Glu Glu Ala Ala Met Glu Leu Lys Val Glu
   1 5 10 15
   Asp Pro Phe Tyr Trp Val Ser Pro Gly
   20 25
<210> 159
   <211> 25
   <212> PRT
   <213> Homo sapiens
<400> 159
   Arg Phe Ser Asp Glu Gly Gly Phe Thr Cys Phe Phe Arg Asp His Ser
   1 5 10 15
   Tyr Gln Glu Glu Ala Ala Met Glu Leu
   20 25
<210> 160
   <211> 20
   <212> PRT
   <213> Homo sapiens
<400> 160
   Arg Arg Pro Phe Phe Pro Phe His Ser Pro Ser Arg Leu Phe Asp Gln
   1               5                   10                  15
   Phe Phe Gly Glu
               20
<210> 161
   <211> 24
   <212> PRT
   <213> Homo sapiens
<400> 161
<210> 162
   <211> 20
   <212> PRT
   <213> Homo sapiens
<400> 162
   Ser Arg Leu Phe Asp Gln Phe Phe Gly Glu His Leu Leu Glu Ser Asp
   1               5                   10                  15
   Leu Phe Pro Thr
               20
<210> 163
   <211> 20
   <212> PRT
   <213> Homo sapiens
<400> 163
   Ser Thr Ser Leu Ser Pro Phe Tyr Leu Arg Pro Pro Ser Phe Leu Arg
   1               5                   10                  15
   Ala Pro Ser Trp
               20
<210> 164
   <211> 7
   <212> PRT
   <213> Unknown
<220>
   <223> epitope involved in multiple sclerosis
<400> 164
   Ser Trp Tyr Ala Arg His Thr
   1 5
<210> 165
   <211> 7
   <212> PRT
   <213> Unknown
<220>
   <223> epitope involved in multiple sclerosis
<400> 165
   Ser Trp Tyr Ala Arg His Tyr
   1 5
<210> 166
   <211> 7
   <212> PRT
   <213> Unknown
<220>
   <223> epitope involved in multiple sclerosis
<400> 166
   Thr Ile Lys Asn Thr Phe Phe
   1 5
<210> 167
   <211> 14
   <212> PRT
   <213> Homo sapiens
<400> 167
   Thr Leu Ser Lys Ile Phe Lys Leu Gly Gly Arg Asp Ser Arg
   1 5 10
<210> 168
   <211> 7
   <212> PRT
   <213> Unknown
<220>
   <223> epitope involved in multiple sclerosis
<400> 168
   Thr Pro Thr Phe Met Tyr Phe
   1 5
<210> 169
   <211> 7
   <212> PRT
   <213> Unknown
<220>
   <223> epitope involved in multiple sclerosis
<400> 169
   Thr Ser Pro Ser Val Ala Tyr
   1               5
<210> 170
   <211> 7
   <212> PRT
   <213> Unknown
<220>
   <223> epitope involved in multiple sclerosis
<400> 170
   Thr Thr Pro Leu Thr Asn Leu
   1               5
<210> 171
   <211> 13
   <212> PRT
   <213> Homo sapiens
<400> 171
   Val Asp Ala Gln Gly Thr Leu Ser Lys Ile Phe Lys Leu
   1               5                   10
<210> 172
   <211> 7
   <212> PRT
   <213> Unknown
<220>
   <223> epitope involved in multiple sclerosis
<400> 172
   Val Asp His Gln His Pro Ser
   1               5
<210> 173
   <211> 20
   <212> PRT
   <213> Homo sapiens
<400> 173
   Val Lys Val Leu Gly Asp Val Ile Glu Val His Gly Lys His Glu Glu
   1               5                   10                  15
   Arg Gln Asp Glu
               20
<210> 174
   <211> 20
   <212> PRT
   <213> Homo sapiens
<400> 174
   Val Ser His Phe Ser Pro Glu Glu Leu Lys Val Lys Val Leu Gly Asp
   1               5                   10                  15
   Val Ile Glu Val
               20
<210> 175
   <211> 7
   <212> PRT
   <213> Unknown
<220>
   <223> epitope involved in multiple sclerosis
<400> 175
   Tyr Ala Gln Pro Ser Glu Arg
   1               5
<210> 176
   <211> 15
   <212> PRT
   <213> Homo sapiens
<400> 176
   Ala Ala Ala Gln Met Pro Ala Tyr Gln Glu Leu Val Glu Glu Ala
   1               5                   10                  15
<210> 177
   <211> 26
   <212> PRT
   <213> Cavia porcellus
<400> 177
   Ala Ala Arg Thr Thr His Tyr Gly Ser Leu Pro Gln Lys Ser Gln Arg
   1               5                   10                  15
   Ser Gln Asp Glu Asn Pro Val Val His Phe
               20                  25
<210> 178
   <211> 15
   <212> PRT
   <213> Homo sapiens
<400> 178
   Ala Gly Val Thr Leu Ile Ser Pro Phe Val Gly Arg Ile Leu Asp
   1 5 10 15
<210> 179
   <211> 19
   <212> PRT
   <213> Homo sapiens
<400> 179
   Ala Arg Arg Lys Val Ala Met Ala Thr Thr Tyr Tyr Phe Asp Tyr Trp
   1 5 10 15
   Gly Gln Gly
<210> 180
   <211> 20
   <212> PRT
   <213> Homo sapiens
<400> 180
   Ala Arg Thr Ala His Tyr Gly Ser Leu Pro Gln Lys Ser His Gly Arg
   1 5 10 15
   Thr Gln Asp Glu
   20
<210> 181
   <211> 15
   <212> PRT
   <213> Human herpesvirus
<400> 181
   Ala Arg Val Ala Glu His Pro Asp Ala Arg Leu Ala Trp Ala Arg
   1 5 10 15
<210> 182
   <211> 15
   <212> PRT
   <213> Homo sapiens
<400> 182
   Ala Ser Asp Leu Glu Lys Ile His Leu Asp Glu Lys Ser Phe Arg
   1 5 10 15
<210> 183
   <211> 20
   <212> PRT
   <213> Homo sapiens
<400> 183
   Ala Ser Gly Gly Thr Phe Ser Ser Phe Ala Ile Asn Trp Val Arg Gln
   1 5 10 15
   Ala Pro Gly Gln
   20
<210> 184
   <211> 19
   <212> PRT
   <213> Homo sapiens
<400> 184
   Cys Phe Tyr Gln Lys Lys Glu Glu Asp Trp Ile Cys Cys Ala Cys Gln
   1 5 10 15
   Lys Thr Arg
<210> 185
   <211> 15
   <212> PRT
   <213> Homo sapiens
<400> 185
   Asp Ala Thr Thr Asn Pro Ser Leu Ile Leu Ala Ala Ala Gln Met
   1 5 10 15
<210> 186
   <211> 15
   <212> PRT
   <213> Homo sapiens
<400> 186
   Asp Phe Leu Thr Ile Ser Pro Lys Leu Leu Gly Glu Leu Leu Gln
   1 5 10 15
<210> 187
   <211> 20
   <212> PRT
   <213> Homo sapiens
<400> 187
   Asp His Ala Arg His Gly Phe Leu Pro Arg His Arg Asp Thr Gly Ile
   1 5 10 15
   Leu Asp Ser Ile
   20
<210> 188
   <211> 15
   <212> PRT
   <213> Homo sapiens
<400> 188
   Asp Pro Gly Val Lys Ser Val Thr Lys Ile Tyr Asn Tyr Tyr Lys
   1 5 10 15
<210> 189
   <211> 15
   <212> PRT
   <213> Homo sapiens
<400> 189
   Asp Gln Met Ala Val Glu Lys Leu Ser Asp Gly Ile Arg Lys Phe
   1 5 10 15
<210> 190
   <211> 23
   <212> PRT
   <213> Homo sapiens
<400> 190
   Asp Arg Phe Ser Gly Ser Lys Ser Val Ser Thr Ala Ser Leu Thr Ile
   1 5 10 15
   Ser Gly Leu Gln Ala Glu Asp
   20
<210> 191
   <211> 19
   <212> PRT
   <213> Homo sapiens
<400> 191
   Asp Arg Lys Tyr Ser Ile Cys Lys Ser Gly Cys Phe Tyr Gln Lys Lys
   1 5 10 15
   Glu Glu Asp
<210> 192
   <211> 17
   <212> PRT
   <213> Homo sapiens
<400> 192
   Asp Ser Tyr Thr Leu Thr Glu Glu Leu Ala Tyr Ala Glu Ile Arg Val
   1 5 10 15
   Lys
<210> 193
   <211> 20
   <212> PRT
   <213> Homo sapiens
<400> 193
   Asp Tyr Lys Ser Ala His Lys Gly Phe Lys Gly Val Asp Ala Gln Gly
   1 5 10 15
   Thr Leu Ser Lys
   20
<210> 194
   <211> 15
   <212> PRT
   <213> Homo sapiens
<400> 194
   Glu Ala Gly Ile Ser Lys Asp Arg Ile Leu Ile Lys Leu Ser Ser
   1 5 10 15
<210> 195
   <211> 21
   <212> PRT
   <213> Homo sapiens
<400> 195
   Glu Asp Thr Ala Val Tyr Tyr Cys Ala Arg Asn Ser Gly Tyr Ser Ile
   1 5 10 15
   Ser Arg Asp Val Tyr
   20
<210> 196
   <211> 15
   <212> PRT
   <213> Homo sapiens
<400> 196
   Glu Lys Ser Phe Arg Trp Leu His Asn Glu Asp Gln Met Ala Val
   1 5 10 15
<210> 197
   <211> 17
   <212> PRT
   <213> Homo sapiens
<400> 197
   Glu Asn Leu His Arg Thr Phe Asp Pro His Phe Leu Arg Val Pro Cys
   1 5 10 15
   Trp
<210> 198
   <211> 20
   <212> PRT
   <213> Homo sapiens
<400> 198
   Glu Asn Pro Val Val His Phe Phe Lys Asn Ile Val Thr Pro Arg Thr
   1               5                   10                  15
   Pro Pro Pro Ser
               20
<210> 199
   <211> 22
   <212> PRT
   <213> Homo sapiens
<400> 199
   Glu Trp Met Gly Gly Ile Ile Pro Ile Phe Gly Ala Ala Asp Tyr Ala
   1               5                   10                  15
   Gln Lys Phe Gln Gly Arg
               20
<210> 200
   <211> 15
   <212> PRT
   <213> Homo sapiens
<400> 200
   Phe Asp Pro His Phe Leu Arg Val Pro Cys Trp Lys Ile Thr Leu
   1               5                   10                  15
<210> 201
   <211> 15
   <212> PRT
   <213> Homo sapiens
<400> 201
   Phe Gly Ala Glu Ile Leu Lys Lys Ile Pro Gly Arg Val Ser Thr
   1               5                   10                  15
<210> 202
   <211> 15
   <212> PRT
   <213> Homo sapiens
<400> 202
   Phe His Ala Ile Asp Glu Tyr Lys Pro Gln Asp Ala Thr Thr Asn
   1               5                   10                  15
<210> 203
   <211> 15
   <212> PRT
   <213> Measles virus
<400> 203
   Phe Ile Val Leu Ser Ile Ala Tyr Pro Thr Leu Ser Glu Ile Lys
   1               5                   10                  15
<210> 204
   <211> 20
   <212> PRT
   <213> Homo sapiens
<400> 204
   Phe Lys Gly Val Asp Ala Gln Gly Thr Leu Ser Lys Ile Phe Lys Leu
   1               5                   10                  15
   Gly Gly Arg Asp
               20
<210> 205
   <211> 15
   <212> PRT
   <213> Homo sapiens
<400> 205
   Phe Thr Thr Val Val Ala Asp Thr Gly Asp Phe His Ala Ile Asp
   1               5                   10                  15
<210> 206
   <211> 15
   <212> PRT
   <213> Homo sapiens
<400> 206
   Gly Glu Leu Leu Gln Asp Asn Ala Lys Leu Val Pro Val Leu Ser
   1               5                   10                  15
<210> 207
   <211> 21
   <212> PRT
   <213> Homo sapiens
<400> 207
   Gly Phe Thr Phe Ser Asp Tyr Tyr Met Asn Trp Ile Arg Gln Ala Pro
   1               5                   10                  15
   Gly Lys Gly Leu Glu
               20
<210> 208
   <211> 21
   <212> PRT
   <213> Homo sapiens
<400> 208
   Gly His Trp Gly Ala Trp Met Pro Ser Ser Ile Ser Ala Phe Glu Gly
   1               5                   10                  15
   Thr Cys Val Ser Ile
               20
<210> 209
   <211> 15
   <212> PRT
   <213> Homo sapiens
<400> 209
   Gly Ile His Cys Asn Met Thr Leu Leu Phe Ser Phe Ala Gln Ala
   1               5                   10                  15
<210> 210
   <211> 15
   <212> PRT
   <213> Human immunodeficiency virus
<400> 210
   Gly Ile Lys Gln Leu Gln Ala Arg Val Leu Ala Val Glu Arg Tyr
   1               5                   10                  15
<210> 211
   <211> 15
   <212> PRT
   <213> Homo sapiens
<400> 211
   Gly Ile Arg Lys Phe Ala Ala Asp Ala Val Lys Leu Glu Arg Met
   1               5                   10                  15
<210> 212
   <211> 22
   <212> PRT
   <213> Homo sapiens
<400> 212
   Gly Asn Ala Lys Asn Ser Leu Tyr Leu Gln Met His Ser Leu Arg Ala
   1               5                   10                  15
   Glu Asp Thr Ala Val Tyr
               20
<210> 213
   <211> 20
   <212> PRT
   <213> Homo sapiens
<400> 213
   Gly Arg Phe Phe Gly Gly Asp Arg Gly Ala Pro Lys Arg Gly Ser Gly
   1               5                   10                  15
   Lys Asp Ser His
               20
<210> 214
   <211> 15
   <212> PRT
   <213> Homo sapiens
<400> 214
   Gly Arg Ile Leu Asp Trp His Val Ala Asn Thr Asp Lys Lys Ser
   1               5                   10                  15
<210> 215
   <211> 15
   <212> PRT
   <213> Homo sapiens
<400> 215
   Gly Arg Val Ser Thr Glu Val Asp Ala Arg Leu Ser Phe Asp Lys
   1               5                   10                  15
<210> 216
   <211> 20
   <212> PRT
   <213> Homo sapiens
<400> 216
   Gly Val Asp Ala Gln Gly Thr Leu Ser Lys Ile Phe Lys Leu Gly Gly
   1               5                   10                  15
   Arg Asp Ser Arg
               20
<210> 217
   <211> 19
   <212> PRT
   <213> Homo sapiens
<400> 217
   His Phe Ser Ile His Cys Cys Pro Pro Phe Thr Phe Leu Asn Ser Lys
   1 5 10 15
   Lys Glu Ile
<210> 218
   <211> 20
   <212> PRT
   <213> Homo sapiens
<400> 218
   His Arg Asp Thr Gly Ile Leu Asp Ser Ile Gly Arg Phe Phe Gly Gly
   1 5 10 15
   Asp Arg Gly Ala
   20
<210> 219
   <211> 15
   <212> PRT
   <213> Homo sapiens
<400> 219
   Ile Lys Leu Ser Ser Thr Trp Glu Gly Ile Gln Ala Gly Lys Glu
   1 5 10 15
<210> 220
   <211> 20
   <212> PRT
   <213> Homo sapiens
<400> 220
   Ile Val Thr Pro Arg Thr Pro Pro Pro Ser Gln Gly Lys Gly Arg Gly
   1 5 10 15
   Leu Ser Leu Ser
   20
<210> 221
   <211> 18
   <212> PRT
   <213> Homo sapiens
<400> 221
   Lys Ile Phe Lys Leu Gly Gly Arg Asp Ser Arg Ser Gly Ser Pro Met
   1 5 10 15
   Ala Arg
<210> 222
   <211> 16
   <212> PRT
   <213> Homo sapiens
<400> 222
   Lys Leu Glu Arg Met Leu Thr Glu Arg Met Phe Asn Ala Glu Asn Gly
   1 5 10 15
<210> 223
   <211> 15
   <212> PRT
   <213> Homo sapiens
<400> 223
   Lys Leu Gly Gly Ser Gln Glu Asp Gln Ile Lys Asn Ala Ile Asp
   1 5 10 15
<210> 224
   <211> 15
   <212> PRT
   <213> Homo sapiens
<400> 224
   Lys Asn Ala Ile Asp Lys Leu Phe Val Leu Phe Gly Ala Glu Ile
   1 5 10 15
<210> 225
   <211> 15
   <212> PRT
   <213> Homo sapiens
<400> 225
   Leu Ser Phe Asp Lys Asp Ala Met Val Ala Arg Ala Arg Arg Leu
   1 5 10 15
<210> 226
   <211> 20
   <212> PRT
   <213> Homo sapiens
<400> 226
   Leu Ser Arg Phe Ser Trp Gly Ala Glu Gly Gln Arg Pro Gly Phe Gly
   1 5 10 15
   Tyr Gly Gly Arg
   20
<210> 227
   <211> 20
   <212> PRT
   <213> Homo sapiens
<400> 227
   Leu Ser Tyr Ser His Ser Asp Tyr Asp Leu Gly Lys Arg Pro Thr Lys
   1               5                   10                  15
   Asp Ser Tyr Thr
               20
<210> 228
   <211> 15
   <212> PRT
   <213> Homo sapiens
<400> 228
   Leu Val Glu Glu Ala Ile Ala Tyr Gly Arg Lys Leu Gly Gly Ser
   1               5                   10                  15
<210> 229
   <211> 26
   <212> PRT
   <213> Homo sapiens
<400> 229
   Leu Val Gly Asp Glu Val Glu Leu Pro Cys Arg Ile Ser Pro Gly Lys
   1               5                   10                  15
   Asn Ala Thr Gly Met Glu Val Gly Trp Tyr
               20                  25
<210> 230
   <211> 25
   <212> PRT
   <213> Homo sapiens
<400> 230
   Leu Tyr Arg Asn Gly Lys Asp Gln Asp Gly Asp Gln Ala Pro Glu Tyr
   1               5                   10                  15
   Arg Gly Arg Thr Glu Leu Leu Lys Asp
               20                  25
<210> 231
   <211> 20
   <212> PRT
   <213> Homo sapiens
<400> 231
   Met Ala Ser Gln Lys Arg Pro Ser Gln Arg His Gly Ser Lys Tyr Leu
   1               5                   10                  15
   Ala Thr Ala Ser
               20
<210> 232
   <211> 15
   <212> PRT
   <213> Homo sapiens
<400> 232
   Met Glu Ser Ala Leu Asp Gln Leu Lys Gln Phe Thr Thr Val Val
   1               5                   10                  15
<210> 233
   <211> 19
   <212> PRT
   <213> Homo sapiens
<400> 233
   Met Ser Gln Lys Pro Ala Lys Glu Gly Pro Arg Leu Ser Lys Asn Gln
   1               5                   10                  15
   Lys Tyr Ser
<210> 234
   <211> 15
   <212> PRT
   <213> Homo sapiens
<400> 234
   Met Ser Ser Ser Pro Val Lys Arg Gln Arg Met Glu Ser Ala Leu
   1               5                   10                  15
<210> 235
   <211> 15
   <212> PRT
   <213> Human herpesvirus
<400> 235
   Asn Pro Val Met Glu Arg Phe Ala Ala His Ala Gly Asp Leu Val
   1               5                   10                  15
<210> 236
   <211> 20
   <212> PRT
   <213> Homo sapiens
<400> 236
   Asn Ser Gly Tyr Ser Ile Ser Arg Asp Val Tyr Trp Gly Gln Gly Thr
   1               5                   10                  15
   Leu Val Thr Val
               20
<210> 237
   <211> 23
   <212> PRT
   <213> Homo sapiens
<400> 237
   Pro Cys Arg Phe Asp Phe Pro Asp Glu Leu Arg Pro Ala Val Val His
   1               5                   10                  15
   Gly Val His Tyr Phe Asn Ser
               20
<210> 238
   <211> 20
   <212> PRT
   <213> Homo sapiens
<400> 238
   Pro Gly Phe Gly Tyr Gly Gly Arg Ala Ser Asp Tyr Lys Ser Ala His
   1               5                   10                  15
   Lys Gly Phe Lys
               20
<210> 239
   <211> 23
   <212> PRT
   <213> Homo sapiens
<400> 239
   Pro Pro Phe Thr Phe Leu Asn Ser Lys Lys Glu Ile Val Asp Arg Lys
   1               5                   10                  15
   Tyr Ser Ile Cys Lys Ser Gly
               20
<210> 240
   <211> 20
   <212> PRT
   <213> Homo sapiens
<400> 240
   Pro Gln Lys Ser His Gly Arg Thr Gln Asp Glu Asn Pro Val Val His
   1               5                   10                  15
   Phe Phe Lys Asn
               20
<210> 241
   <211> 25
   <212> PRT
   <213> Homo sapiens
<400> 241
   Pro Arg Pro Glu Val Arg Pro Pro Pro Ala Lys Gln Arg Pro Pro Gln
   1               5                   10                  15
   Lys Ser Lys Gln Gln Pro Arg Ser Ser
               20                  25
<210> 242
   <211> 15
   <212> PRT
   <213> Homo sapiens
<400> 242
   Gln Ala Gly Lys Glu Leu Glu Glu Gln His Gly Ile His Cys Asn
   1               5                   10                  15
<210> 243
   <211> 11
   <212> PRT
   <213> Homo sapiens
<400> 243
   Gln Phe Gln Leu Val Glu Ser Gly Gly Gly Leu
   1               5                   10
<210> 244
   <211> 22
   <212> PRT
   <213> Homo sapiens
<400> 244
   Gln Lys Tyr Ser Glu His Phe Ser Ile His Cys Cys Pro Pro Phe Thr
   1               5                   10                  15
   Phe Leu Asn Ser Lys Lys
               20
<210> 245
   <211> 24
   <212> PRT
   <213> Homo sapiens
<400> 245
   Gln Pro Arg Ser Ser Pro Leu Arg Gly Pro Gly Ala Ser Arg Gly Gly
   1               5                   10                  15
   Ser Pro Val Lys Ala Ser Arg Phe
               20
<210> 246
   <211> 24
   <212> PRT
   <213> Homo sapiens
<400> 246
   Gln Gln His Pro Gly Lys Ala Pro Lys Val Met Ile Tyr Asp Val Thr
   1               5                   10                  15
   Lys Arg Pro Ser Gly Val Pro Asp
               20
<210> 247
   <211> 23
   <212> PRT
   <213> Homo sapiens
<400> 247
   Gln Arg Pro Pro Gln Lys Ser Lys Gln Gln Pro Arg Ser Ser Pro Leu
   1               5                   10                  15
   Arg Gly Pro Gly Ala Ser Arg
               20
<210> 248
   <211> 12
   <212> PRT
   <213> Homo sapiens
<400> 248
   Gln Ser Val Leu Thr Gln Pro Arg Ser Val Ser Gly
   1               5                   10
<210> 249
   <211> 13
   <212> PRT
   <213> Homo sapiens
<400> 249
   Gln Ser Val Leu Thr Gln Ser Pro Ser Ala Ser Ala Ser
   1               5                   10
<210> 250
   <211> 20
   <212> PRT
   <213> Homo sapiens
<400> 250
   Gln Thr Trp Gly Thr Gly Ile Glu Thr Val Phe Gly Gly Gly Thr Lys
   1 5 10 15
   Val Thr Val Leu
   20
<210> 251
   <211> 21
   <212> PRT
   <213> Homo sapiens
<400> 251
   Arg Ala Glu Asp Thr Ala Val Tyr Tyr Cys Ala Arg Arg Lys Val Ala
   1 5 10 15
   Met Ala Thr Thr Tyr
   20
<210> 252
   <211> 15
   <212> PRT
   <213> Homo sapiens
<400> 252
   Arg Ala Arg Arg Leu Ile Glu Leu Tyr Lys Glu Ala Gly Ile Ser
   1 5 10 15
<210> 253
   <211> 20
   <212> PRT
   <213> Homo sapiens
<400> 253
   Arg Phe Ser Trp Gly Ala Glu Gly Gln Arg Pro Gly Phe Gly Tyr Gly
   1 5 10 15
   Gly Arg Ala Ser
   20
<210> 254
   <211> 20
   <212> PRT
   <213> Homo sapiens
<400> 254
   Arg Gly Ser Gly Lys Asp Ser His His Pro Ala Arg Thr Ala His Tyr
   1 5 10 15
   Gly Ser Leu Pro
   20
<210> 255
   <211> 19
   <212> PRT
   <213> Homo sapiens
<400> 255
   Arg Leu Ser Lys Asn Gln Lys Tyr Ser Glu His Phe Ser Ile His Cys
   1 5 10 15
   Cys Pro Pro
<210> 256
   <211> 19
   <212> PRT
   <213> Homo sapiens
<400> 256
   Arg Pro Gly Phe Gly Tyr Gly Gly Arg Ala Ser Asp Tyr Lys Ser Ala
   1 5 10 15
   His Lys Gly
<210> 257
   <211> 15
   <212> PRT
   <213> Homo sapiens
<400> 257
   Ser Phe Ala Gln Ala Val Ala Cys Ala Glu Ala Gly Val Thr Leu
   1 5 10 15
<210> 258
   <211> 24
   <212> PRT
   <213> Homo sapiens
<400> 258
   Ser Gly Cys Phe Tyr Gln Lys Lys Glu Glu Asp Trp Ile Cys Cys Ala
   1 5 10 15
   Cys Gln Lys Thr Arg Thr Ser Arg
   20
<210> 259
   <211> 20
   <212> PRT
   <213> Homo sapiens
<400> 259
   Ser Lys Tyr Leu Ala Thr Ala Ser Thr Met Asp His Ala Arg His Gly
   1               5                   10                  15
   Phe Leu Pro Arg
               20
<210> 260
   <211> 20
   <212> PRT
   <213> Homo sapiens
<400> 260
   Ser Gln Arg His Gly Ser Lys Tyr Leu Ala Thr Ala Ser Thr Met Asp
   1               5                   10                  15
   His Ala Arg His
               20
<210> 261
   <211> 20
   <212> PRT
   <213> Homo sapiens
<400> 261
   Ser Val Lys Gly Arg Phe Thr Ile Ser Arg Gly Asn Ala Lys Asn Ser
   1               5                   10                  15
   Leu Tyr Leu Gln
               20
<210> 262
   <211> 19
   <212> PRT
   <213> Homo sapiens
<400> 262
   Ser Tyr Ala Gly Gly Ser Ala Cys Val Phe Gly Thr Gly Thr Lys Leu
   1               5                   10                  15
   Thr Val Leu
<210> 263
   <211> 15
   <212> PRT
   <213> Homo sapiens
<400> 263
   Thr Asp Lys Lys Ser Tyr Glu Pro Leu Glu Asp Pro Gly Val Lys
   1               5                   10                  15
<210> 264
   <211> 19
   <212> PRT
   <213> Homo sapiens
<400> 264
   Thr Phe Leu Asn Ser Lys Lys Glu Ile Val Asp Arg Lys Tyr Ser Ile
   1               5                   10                  15
   Cys Lys Ser
<210> 265
   <211> 15
   <212> PRT
   <213> Homo sapiens
<400> 265
   Thr Gly Glu Ile Lys Ala Leu Ala Gly Cys Asp Phe Leu Thr Ile
   1               5                   10                  15
<210> 266
   <211> 15
   <212> PRT
   <213> Homo sapiens
<400> 266
   Thr Ile Val Met Gly Ala Ser Phe Arg Asn Thr Gly Glu Ile Lys
   1               5                   10                  15
<210> 267
   <211> 20
   <212> PRT
   <213> Homo sapiens
<400> 267
   Val Ile Val Glu Met Asn Ser Ser Val Glu Ala Ile Glu Gly Ser His
   1               5                   10                  15
   Val Ser Leu Leu
               20
<210> 268
   <211> 15
   <212> PRT
   <213> Human herpesvirus
<400> 268
   Val Pro Val Leu Ala Phe Asp Ala Ala Arg Leu Arg Leu Leu Glu
   1 5 10 15
<210> 269
   <211> 15
   <212> PRT
   <213> Homo sapiens
<400> 269
   Val Pro Val Leu Ser Ala Lys Ala Ala Gln Ala Ser Asp Leu Glu
   1 5 10 15
<210> 270
   <211> 20
   <212> PRT
   <213> Unknown
<220>
   <223> epitope involved in multiple sclerosis
<400> 270
   Tyr Asp Glu Asn Pro Val Val His Phe Phe Lys Asn Ile Val Thr Pro
   1 5 10 15
   Arg Thr Pro Pro
   20
<210> 271
   <211> 15
   <212> PRT
   <213> Homo sapiens
<400> 271
   Tyr Asn Tyr Tyr Lys Lys Phe Ser Tyr Lys Thr Ile Val Met Gly
   1 5 10 15
<210> 272
   <211> 15
   <212> PRT
   <213> Homo sapiens
<400> 272
   Ala Arg Thr Ala His Tyr Gly Ser Leu Pro Gln Lys Ser His Gly
   1 5 10 15
<210> 273
   <211> 20
   <212> PRT
   <213> Cavia porcellus
<400> 273
   Ala Ser Gln Lys Arg Pro Ser Gln Arg His Gly Ser Lys Tyr Leu Ala
   1 5 10 15
   Thr Ala Ser Thr
   20
<210> 274
   <211> 38
   <212> PRT
   <213> Homo sapiens
<400> 274
   Ala Ser Gln Lys Arg Pro Ser Gln Arg His Gly Ser Lys Tyr Leu Ala
   1 5 10 15
   Thr Ala Ser Thr Met Asp His Ala Arg His Gly Phe Leu Pro Arg His
   20 25 30
   Arg Asp Thr Gly Ile Leu
   35
<210> 275
   <211> 17
   <212> PRT
   <213> Human herpesvirus
<400> 275
   Asp Ala Tyr Pro Cys Tyr Phe Phe Lys Ser Ala Cys Arg Pro Arg Ala
   1 5 10 15
   Pro
<210> 276
   <211> 17
   <212> PRT
   <213> Homo sapiens
<400> 276
   Asp Glu Asn Pro Val Val His Phe Phe Lys Asn Ile Val Thr Pro Arg
   1 5 10 15
   Thr
<210> 277
   <211> 8
   <212> PRT
   <213> Unknown
<220>
   <223> epitope involved in multiple sclerosis
<400> 277
   Glu Ala Gly Ala Gly Gly Gly Ala
   1 5
<210> 278
   <211> 12
   <212> PRT
   <213> Bos taurus
<400> 278
   Glu Asp Lys Gly Ala Cys Leu Leu Pro Lys Ile Glu
   1 5 10
<210> 279
   <211> 15
   <212> PRT
   <213> Unknown
<220>
   <223> epitope involved in multiple sclerosis
<400> 279
   Glu Asn Pro Lys Val His Phe Phe Lys Asn Ile Val Thr Pro Arg
   1 5 10 15
<210> 280
   <211> 8
   <212> PRT
   <213> Unknown
<220>
   <223> epitope involved in multiple sclerosis
<400> 280
   Glu Pro Ala Arg Ser Arg Ser Arg
   1 5
<210> 281
   <211> 15
   <212> PRT
   <213> Unknown
<220>
   <223> epitope involved in multiple sclerosis
<400> 281
   Glu Pro Met Thr Pro His Gln Trp Ile Thr Leu Tyr Arg Ser Tyr
   1 5 10 15
<210> 282
   <211> 15
   <212> PRT
   <213> Unknown
<220>
   <223> epitope involved in multiple sclerosis
<400> 282
   Phe Cys Pro Pro Ile Leu Pro Tyr Ser Ala Trp Cys Pro Val Pro
   1               5                   10                  15
<210> 283
   <211> 20
   <212> PRT
   <213> Mus musculus
<400> 283
   Gly Phe Gly Tyr Gly Gly Arg Ala Ser Asp Tyr Lys Ser Ala His Lys
   1               5                   10                  15
   Gly Phe Lys Gly
               20
<210> 284
   <211> 12
   <212> PRT
   <213> Homo sapiens
<400> 284
   Gly Lys Trp Leu Gly His Pro Asp Lys Phe Val Gly
   1               5                   10
<210> 285
   <211> 19
   <212> PRT
   <213> Homo sapiens
<400> 285
   Gly Ser Lys Tyr Leu Ala Thr Ala Ser Thr Met Asp His Ala Arg His
   1               5                   10                  15
   Gly Phe Leu
<210> 286
   <211> 20
   <212> PRT
   <213> Homo sapiens
<400> 286
   Gly Ser Lys Tyr Leu Ala Thr Ala Ser Thr Met Asp His Ala Arg His
   1 5 10 15
   Gly Phe Leu Pro
   20
<210> 287
   <211> 15
   <212> PRT
   <213> Homo sapiens
<400> 287
   His His Pro Ala Arg Thr Ala His Tyr Gly Ser Leu Pro Gln Lys
   1 5 10 15
<210> 288
   <211> 22
   <212> PRT
   <213> Homo sapiens
<400> 288
   His His Pro Ala Arg Thr Ala His Tyr Gly Ser Leu Pro Gln Lys Ser
   1 5 10 15
   His Gly Arg Thr Gln Asp
   20
<210> 289
   <211> 15
   <212> PRT
   <213> Human papillomavirus
<400> 289
   Ile Gly Gly Arg Val His Phe Phe Lys Asp Ile Ser Pro Ile Ala
   1 5 10 15
<210> 290
   <211> 11
   <212> PRT
   <213> Homo sapiens
<400> 290
   Lys Leu Ser Asp Gly Ile Arg Lys Phe Ala Ala
   1 5 10
<210> 291
   <211> 16
   <212> PRT
   <213> Homo sapiens
<400> 291
   Lys Asn Ile Val Thr Pro Arg Thr Pro Pro Pro Ser Gln Gly Lys Gly
   1 5 10 15
<210> 292
   <211> 21
   <212> PRT
   <213> Homo sapiens
<400> 292
   Lys Ser His Gly Arg Thr Gln Asp Glu Asn Pro Val Val His Phe Phe
   1 5 10 15
   Lys Asn Ile Val Thr
   20
<210> 293
   <211> 21
   <212> PRT
   <213> Homo sapiens
<400> 293
   Lys Ser His Gly Arg Thr Gln Asp Gln Asp Pro Val Val His Phe Phe
   1 5 10 15
   Lys Asn Ile Val Thr
   20
<210> 294
   <211> 9
   <212> PRT
   <213> Homo sapiens
<400> 294
   Leu Leu Phe Ser Phe Ala Gln Ala Val
   1 5
<210> 295
   <211> 20
   <212> PRT
   <213> Cavia porcellus
<400> 295
   Met Asp His Ala Arg His Gly Phe Leu Pro Arg His Arg Asp Thr Gly
   1 5 10 15
   Ile Leu Asp Ser
   20
<210> 296
   <211> 15
   <212> PRT
   <213> Unknown
<220>
   <223> epitope involved in multiple sclerosis
<400> 296
   Met Pro Val Ser Arg Leu Cys Ile Glu Leu Asp Trp Cys Pro Pro
   1               5                   10                  15
<210> 297
   <211> 20
   <212> PRT
   <213> Homo sapiens
<400> 297
   Pro Ser Gln Gly Lys Gly Arg Gly Leu Ser Leu Ser Arg Phe Ser Trp
   1               5                   10                  15
   Gly Ala Glu Gly
               20
<210> 298
   <211> 15
   <212> PRT
   <213> Unknown
<220>
   <223> epitope involved in multiple sclerosis
<400> 298
   Arg Gly Thr Gln Glu Trp Thr Glu Leu Trp Val Ser Phe Arg Ala
   1               5                   10                  15
<210> 299
   <211> 16
   <212> PRT
   <213> Homo sapiens
<400> 299
   Ser His His Pro Ala Arg Thr Ala His Tyr Gly Ser Leu Pro Gln Lys
   1               5                   10                  15
<210> 300
   <211> 15
   <212> PRT
   <213> Unknown
<220>
   <223> epitope involved in multiple sclerosis
<400> 300
   Ser Arg Gly Ser His Glu Trp Ala Val Leu Phe Arg Phe Tyr Tyr
   1 5 10 15
<210> 301
   <211> 15
   <212> PRT
   <213> Unknown
<220>
   <223> epitope involved in multiple sclerosis
<400> 301
   Ser Ser Ala Lys Ser His Cys Tyr Ala Phe Cys Ser Gly Leu Pro
   1 5 10 15
<210> 302
   <211> 12
   <212> PRT
   <213> Homo sapiens
<400> 302
   Thr Cys Phe Phe Arg Asp His Ser Tyr Gln Glu Glu
   1 5 10
<210> 303
   <211> 10
   <212> PRT
   <213> Homo sapiens
<400> 303
   Thr Gln Asp Glu Asn Pro Val Val His Phe
   1 5 10
<210> 304
   <211> 18
   <212> PRT
   <213> Homo sapiens
<400> 304
   Thr Gln Asp Gln Asp Pro Val Val His Phe Phe Lys Asn Ile Val Thr
   1 5 10 15
   Pro Arg
<210> 305
   <211> 12
   <212> PRT
   <213> Bos taurus
<400> 305
   Thr Ser Val Phe Asp Lys Leu Lys His Leu Val Asp
   1 5 10
<210> 306
   <211> 21
   <212> PRT
   <213> Mus musculus
<400> 306
   Val His Phe Phe Lys Asn Ile Val Thr Pro Arg Thr Pro Pro Pro Ser
   1               5                   10                  15
   Gln Gly Lys Gly Arg
               20
<210> 307
   <211> 10
   <212> PRT
   <213> Homo sapiens
<400> 307
   Val Val His Phe Phe Lys Asn Ile Val Thr
   1               5                   10
<210> 308
   <211> 15
   <212> PRT
   <213> Homo sapiens
<400> 308
   Tyr Gly Ser Leu Pro Gln Lys Ser His Gly Arg Thr Gln Asp Gln
   1               5                   10                  15
<210> 309
   <211> 17
   <212> PRT
   <213> Homo sapiens
<400> 309
   Tyr Lys Asp Ser His His Pro Ala Arg Thr Ala His Tyr Gly Ser Leu
   1               5                   10                  15
   Pro
<210> 310
   <211> 9
   <212> PRT
   <213> Homo sapiens
<400> 310
   Asp Tyr Lys Ser Ala His Lys Gly Phe
   1               5
<210> 311
   <211> 15
   <212> PRT
   <213> Homo sapiens
<400> 311
   Gly Lys Asp Ser His His Pro Ala Arg Thr Ala His Tyr Gly Ser
   1 5 10 15
<210> 312
   <211> 9
   <212> PRT
   <213> Homo sapiens
<400> 312
   Lys Tyr Leu Ala Thr Ala Ser Thr Met
   1 5
<210> 313
   <211> 21
   <212> PRT
   <213> Homo sapiens
<400> 313
   Asp Glu Asn Pro Val Val His Phe Phe Lys Asn Ile Val Thr Pro Arg
   1 5 10 15
   Thr Pro Pro Pro Ser
   20
<210> 314
   <211> 12
   <212> PRT
   <213> Homo sapiens
<400> 314
   Phe Phe Lys Asn Ile Val Thr Pro Arg Thr Pro Pro
   1 5 10
<210> 315
   <211> 21
   <212> PRT
   <213> Homo sapiens
<400> 315
   Thr Gly Gly Gln Lys Gly Arg Gly Ser Arg Gly Gln His Gln Ala His
   1 5 10 15
   Ser Leu Glu Arg Val
   20
<210> 316
   <211> 18
   <212> PRT
   <213> Homo sapiens
<400> 316
   Tyr Lys Thr Thr Ile Cys Gly Lys Gly Leu Ser Ala Thr Val Thr Gly
   1               5                   10                  15
   Gly Gln
<210> 317
   <211> 20
   <212> PRT
   <213> Homo sapiens
<400> 317
   Gly Leu Ser Leu Ser Arg Phe Ser Trp Gly Ala Glu Gly Gln Arg Pro
   1               5                   10                  15
   Gly Phe Gly Tyr
               20
<210> 318
   <211> 19
   <212> PRT
   <213> Homo sapiens
<400> 318
   Ala Pro Lys Arg Gly Ser Gly Lys Asp Ser His His Ala Ala Arg Thr
   1               5                   10                  15
   Ala His Tyr
<210> 319
   <211> 23
   <212> PRT
   <213> Homo sapiens
<400> 319
   Asp Ala Ile Gly Glu Gly Lys Val Thr Leu Arg Ile Arg Asn Val Arg
   1               5                   10                  15
   Phe Ser Asp Glu Gly Gly Phe
               20
<210> 320
   <211> 21
   <212> PRT
   <213> Homo sapiens
<400> 320
   Asp Ser Ile Gly Arg Phe Phe Gly Gly Asp Arg Gly Ala Pro Lys Arg
   1               5                   10                  15
   Gly Ser Gly Lys Asp
               20
<210> 321
   <211> 20
   <212> PRT
   <213> Homo sapiens
<400> 321
   Glu Leu Pro Cys Arg Ile Ser Pro Gly Lys Asn Ala Thr Gly Met Glu
   1               5                   10                  15
   Val Gly Trp Tyr
               20
<210> 322
   <211> 23
   <212> PRT
   <213> Homo sapiens
<400> 322
   Glu Tyr Arg Gly Arg Thr Glu Leu Leu Lys Asp Ala Ile Gly Glu Gly
   1               5                   10                  15
   Lys Val Thr Leu Arg Ile Arg
               20
<210> 323
   <211> 20
   <212> PRT
   <213> Homo sapiens
<400> 323
   Phe Ser Trp Gly Ala Glu Gly Gln Arg Pro Gly Phe Gly Tyr Gly Gly
   1               5                   10                  15
   Arg Ala Ser Asp
               20
<210> 324
   <211> 22
   <212> PRT
   <213> Homo sapiens
<400> 324
   Gly Gly Gln Lys Gly Arg Gly Ser Arg Gly Gln His Gln Ala His Ser
   1               5                   10                  15
   Leu Glu Arg Val Cys His
               20
<210> 325
   <211> 15
   <212> PRT
   <213> Herpes simplex virus
<400> 325
   Gly Gly Arg Arg Leu Phe Phe Val Lys Ala His Val Arg Glu Ser
   1               5                   10                  15
<210> 326
   <211> 20
   <212> PRT
   <213> Homo sapiens
<400> 326
   Gly Lys Gly Arg Gly Leu Ser Leu Ser Arg Phe Ser Trp Gly Ala Glu
   1               5                   10                  15
   Gly Gln Arg Pro
               20
<210> 327
   <211> 24
   <212> PRT
   <213> Homo sapiens
<400> 327
   Gly Leu Ser Leu Ser Arg Phe Ser Trp Gly Ala Glu Gly Gln Arg Pro
   1               5                   10                  15
   Gly Phe Gly Tyr Gly Gly Arg Ala
               20
<210> 328
   <211> 23
   <212> PRT
   <213> Homo sapiens
<400> 328
   Gly Met Glu Val Gly Trp Tyr Arg Pro Pro Phe Ser Arg Val Val His
   1               5                   10                  15
   Leu Tyr Arg Asn Gly Lys Asp
               20
<210> 329
   <211> 22
   <212> PRT
   <213> Homo sapiens
<400> 329
   Gly Gln Phe Arg Val Ile Gly Pro Arg His Pro Ile Arg Ala Leu Val
   1 5 10 15
   Gly Asp Glu Val Glu Leu
   20
<210> 330
   <211> 15
   <212> PRT
   <213> Homo sapiens
<400> 330
   Gly Thr Leu Ser Lys Ile Phe Lys Leu Gly Gly Arg Asp Ser Arg
   1 5 10 15
<210> 331
   <211> 26
   <212> PRT
   <213> Homo sapiens
<400> 331
   Gly Val Asp Ala Gln Gly Thr Leu Ser Lys Ile Phe Lys Leu Gly Gly
   1 5 10 15
   Arg Asp Ser Arg Ser Gly Ser Pro Met Ala
   20 25
<210> 332
   <211> 19
   <212> PRT
   <213> Homo sapiens
<400> 332
   His Ala Ala Arg Thr Ala His Tyr Gly Ser Leu Pro Gln Lys Ser Gln
   1 5 10 15
   Gly His Arg
<210> 333
   <211> 17
   <212> PRT
   <213> Homo sapiens
<400> 333
   His Cys Leu Gly Lys Trp Leu Gly His Pro Asp Lys Phe Val Gly Ile
   1               5                   10                  15
   Thr
<210> 334
   <211> 15
   <212> PRT
   <213> Homo sapiens
<400> 334
   Leu Gly His Pro Asp Lys Phe Val Gly Ile Thr Tyr Ala Leu Thr
   1               5                   10                  15
<210> 335
   <211> 20
   <212> PRT
   <213> Homo sapiens
<400> 335
   Leu Gly Gln Gly Pro Glu Phe Leu Thr Tyr Phe Gln Asn Glu Ala Gln
   1               5                   10                  15
   Leu Glu Lys Ser
               20
<210> 336
   <211> 20
   <212> PRT
   <213> Homo sapiens
<400> 336
   Leu Leu Ala Glu Gly Phe Tyr Thr Thr Gly Ala Val Arg Gln Ile Phe
   1               5                   10                  15
   Gly Asp Tyr Lys
               20
<210> 337
   <211> 19
   <212> PRT
   <213> Homo sapiens
<400> 337
   Asn Pro Val Val His Phe Phe Lys Asn Ile Val Thr Pro Arg Thr Pro
   1               5                   10                  15
   Pro Pro Ser
<210> 338
   <211> 20
   <212> PRT
   <213> Homo sapiens
<400> 338
   Pro Gln Asp Glu Asn Pro Val Val His Phe Phe Lys Asn Ile Val Thr
   1 5 10 15
   Pro Arg Thr Pro
   20
<210> 339
   <211> 20
   <212> PRT
   <213> Homo sapiens
<400> 339
   Pro Arg His Arg Asp Thr Gly Ile Leu Asp Ser Ile Gly Arg Phe Phe
   1 5 10 15
   Gly Gly Asp Arg
   20
<210> 340
   <211> 19
   <212> PRT
   <213> Unknown
<220>
   <223> epitope involved in multiple sclerosis
<400> 340
   Gln Ala His Ser Leu Glu Arg Val Ser His Ser Leu Gly Lys Trp Leu
   1 5 10 15
   Gly His Pro
<210> 341
   <211> 20
   <212> PRT
   <213> Homo sapiens
<400> 341
   Arg Asp Thr Gly Ile Leu Asp Ser Ile Gly Arg Phe Phe Gly Gly Asp
   1 5 10 15
   Arg Gly Ala Pro
   20
<210> 342
   <211> 20
   <212> PRT
   <213> Homo sapiens
<400> 342
   Arg Phe Phe Gly Gly Asp Arg Gly Ala Pro Lys Arg Gly Ser Gly Lys
   1 5 10 15
   Asp Ser His His
   20
<210> 343
   <211> 20
   <212> PRT
   <213> Homo sapiens
<400> 343
   Arg Thr Ala His Tyr Gly Ser Leu Pro Gln Lys Ser His Gly Arg Thr
   1 5 10 15
   Gln Asp Glu Asn
   20
<210> 344
   <211> 20
   <212> PRT
   <213> Homo sapiens
<400> 344
   Tyr Lys Ser Ala His Lys Gly Phe Lys Gly Val Asp Ala Gln Gly Thr
   1 5 10 15
   Leu Ser Lys Ile
   20
<210> 345
   <211> 25
   <212> PRT
   <213> Homo sapiens
<400> 345
   Ala Ala Arg Thr Thr His Tyr Gly Ser Leu Pro Gln Lys Ser Gln Arg
   1 5 10 15
   Ser Gln Asp Gln Asn Pro Val His Phe
   20 25
<210> 346
   <211> 25
   <212> PRT
   <213> Bos taurus
<400> 346
   Ala Glu Tyr Arg Gly Arg Val Ser Leu Val Glu Asp His Ile Ala Glu
   1               5                   10                  15
   Gly Ser Val Ala Val Arg Ile Gln Glu
               20                  25
<210> 347
   <211> 17
   <212> PRT
   <213> Unknown
<220>
   <223> epitope involved in multiple sclerosis
<400> 347
   Ala Lys Pro Val Val His Leu Phe Ala Asn Ile Val Thr Pro Arg Thr
   1               5                   10                  15
   Pro
<210> 348
   <211> 26
   <212> PRT
   <213> Bos taurus
<400> 348
   Ala Pro Phe Asp Val Ile Gly Pro Gln Glu Pro Ile Leu Ala Val Val
   1               5                   10                  15
   Gly Glu Asp Ala Glu Leu Pro Cys Arg Leu
               20                  25
<210> 349
   <211> 11
   <212> PRT
   <213> Homo sapiens
<400> 349
   Ala Ser Asp Tyr Lys Ser Ala His Lys Gly Phe
   1               5                   10
<210> 350
   <211> 19
   <212> PRT
   <213> Homo sapiens
<400> 350
   Ala Ser Gln Lys Arg Pro Ser Gln Arg His Gly Lys Tyr Leu Ala Thr
   1 5 10 15
   Ala Ser Thr
<210> 351
   <211> 20
   <212> PRT
   <213> Mus musculus
<400> 351
   Ala Ser Gln Lys Arg Pro Ser Gln Arg Ser Lys Tyr Leu Ala Thr Ala
   1 5 10 15
   Ser Thr Met Asp
   20
<210> 352
   <211> 26
   <212> PRT
   <213> Bos taurus
<400> 352
   Ala Val Val Gly Glu Asp Ala Glu Leu Pro Cys Arg Leu Ser Pro Asn
   1 5 10 15
   Val Ser Ala Lys Gly Met Glu Leu Arg Trp
   20 25
<210> 353
   <211> 11
   <212> PRT
   <213> Homo sapiens
<400> 353
   Asp Glu Asn Pro Val Val His Phe Phe Lys Asn
   1 5 10
<210> 354
   <211> 17
   <212> PRT
   <213> Homo sapiens
<400> 354
   Glu Asn Pro Val Val His Glu Phe Lys Asn Ile Val Thr Pro Arg Thr
   1 5 10 15
   Pro
<210> 355
   <211> 14
   <212> PRT
   <213> Homo sapiens
<400> 355
   Glu Asn Pro Val Val His Phe Phe Lys Asn Ile Val Thr Pro
   1 5 10
<210> 356
   <211> 15
   <212> PRT
   <213> Unknown
<220>
   <223> epitope involved in multiple sclerosis
<400> 356
   Glu Asn Pro Val Val His Phe Phe Leu Asn Ile Val Thr Pro Arg
   1 5 10 15
<210> 357
   <211> 15
   <212> PRT
   <213> Unknown
<220>
   <223> epitope involved in multiple sclerosis
<400> 357
   Glu Asn Pro Val Val His Phe Phe Arg Asn Ile Val Thr Pro Arg
   1 5 10 15
<210> 358
   <211> 19
   <212> PRT
   <213> Homo sapiens
<400> 358
   Gly Leu Ser Leu Ser Arg Phe Ser Trp Gly Ala Gln Gly Gln Lys Pro
   1 5 10 15
   Gly Phe Gly
<210> 359
   <211> 23
   <212> PRT
   <213> Homo sapiens
<400> 359
   Gly Thr Leu Ser Lys Ile Phe Lys Leu Gly Gly Arg Asp Ser Arg Ser
   1               5                   10                  15
   Gly Ser Pro Met Ala Arg Arg
               20
<210> 360
   <211> 25
   <212> PRT
   <213> Bos taurus
<400> 360
   Ile Ala Glu Gly Ser Val Ala Val Arg Ile Gln Glu Val Lys Ala Ser
   1               5                   10                  15
   Asp Asp Gly Glu Tyr Arg Cys Phe Phe
               20                  25
<210> 361
   <211> 25
   <212> PRT
   <213> Bos taurus
<400> 361
   Lys Ala Ser Asp Asp Gly Glu Tyr Arg Cys Phe Phe Arg Gln Asp Glu
   1               5                   10                  15
   Asn Tyr Glu Glu Ala Ile Val His Leu
               20                  25
<210> 362
   <211> 22
   <212> PRT
   <213> Homo sapiens
<400> 362
   Lys Ser His Gly Arg Thr Gln Asp Glu Asn Pro Val Val His Phe Phe
   1               5                   10                  15
   Lys Asn Ile Val Thr Pro
               20
<210> 363
   <211> 17
   <212> PRT
   <213> Homo sapiens
<400> 363
   Leu Ala Thr Ala Ser Thr Met Asp His Ala Arg His Gly Phe Leu Pro
   1 5 10 15
   Arg
<210> 364
   <211> 14
   <212> PRT
   <213> Homo sapiens
<400> 364
   Asn Pro Val Val His Phe Phe Lys Asn Ile Val Thr Pro Arg
   1 5 10
<210> 365
   <211> 19
   <212> PRT
   <213> Homo sapiens
<400> 365
   Gln Asp Glu Asn Pro Val Val His Phe Phe Lys Asn Ile Val Thr Pro
   1 5 10 15
   Arg Thr Pro
<210> 366
   <211> 20
   <212> PRT
   <213> Bos taurus
<400> 366
   Arg Gln Asp Glu Asn Tyr Glu Glu Ala Ile Val His Leu Lys Val Ala
   1 5 10 15
   Ala Leu Gly Ser
   20
<210> 367
   <211> 23
   <212> PRT
   <213> Bos taurus
<400> 367
   Arg Trp Phe Arg Glu Lys Val Ser Pro Ala Val Phe Val Ser Arg Glu
   1 5 10 15
   Gly Gln Glu Gln Glu Gly Glu
   20
<210> 368
   <211> 24
   <212> PRT
   <213> Bos taurus
<400> 368
   Ser Pro Asn Val Ser Ala Lys Gly Met Glu Leu Arg Trp Phe Arg Glu
   1               5                   10                  15
   Lys Val Ser Pro Ala Val Phe Val
               20
<210> 369
   <211> 18
   <212> PRT
   <213> Bos taurus
<400> 369
   Thr Leu Ser Lys Ile Phe Lys Leu Gly Gly Arg Asp Ser Arg Ser Gly
   1               5                   10                  15
   Ser Pro
<210> 370
   <211> 13
   <212> PRT
   <213> Homo sapiens
<400> 370
   Val His Phe Phe Lys Asn Ile Val Thr Pro Arg Thr Glu
   1               5                   10
<210> 371
   <211> 24
   <212> PRT
   <213> Bos taurus
<400> 371
   Val Ser Arg Glu Gly Gln Glu Gln Glu Gly Glu Met Ala Glu Tyr Arg
   1               5                   10                  15
   Gly Arg Val Ser Leu Val Glu Asp
               20
<210> 372
   <211> 23
   <212> PRT
   <213> Homo sapiens
<400> 372
   Val Thr Pro Arg Thr Pro Pro Pro Ser Gln Gly Lys Gly Arg Gly Leu
   1               5                   10                  15
   Ser Leu Ser Arg Phe Ser Trp
               20
<210> 373
   <211> 18
   <212> PRT
   <213> Homo sapiens
<400> 373
   Ala Leu Gly Gln Gly Pro Gln Phe Ile Phe Gln Tyr Tyr Glu Glu Glu
   1               5                   10                  15
   Glu Arg
<210> 374
   <211> 19
   <212> PRT
   <213> Homo sapiens
<400> 374
   Gly Leu Ser Leu Ser Arg Phe Ser Trp Gly Ala Glu Gly Gln Arg Pro
   1               5                   10                  15
   Gly Phe Gly
<210> 375
   <211> 19
   <212> PRT
   <213> Homo sapiens
<400> 375
   Gly Leu Ser Leu Ser Arg Phe Ser Trp Gly Ala Glu Gly Gln Arg Pro
   1               5                   10                  15
   Gly Phe Gly
<210> 376
   <211> 14
   <212> PRT
   <213> Homo sapiens
<400> 376
   His Lys Gly Phe Lys Gly Val Asp Ala Gln Gly Thr Leu Ser
   1               5                   10
<210> 377
   <211> 24
   <212> PRT
   <213> Homo sapiens
<400> 377
   Lys Ser His Gly Arg Thr Gln Asp Glu Asn Pro Val Val His Phe Phe
   1               5                   10                  15
   Lys Asn Ile Val Thr Pro Arg Thr
               20
<210> 378
   <211> 21
   <212> PRT
   <213> Homo sapiens
<400> 378
   Leu Gly Gln Gly Pro Glu Phe Leu Ile Tyr Phe Gln Gly Thr Gly Ala
   1               5                   10                  15
   Ala Asp Asp Ser Gly
               20
<210> 379
   <211> 20
   <212> PRT
   <213> Homo sapiens
<400> 379
   Leu Leu Lys Asp Ala Ile Gly Glu Gly Lys Val Thr Leu Arg Ile Arg
   1               5                   10                  15
   Asn Val Arg Phe
               20
<210> 380
   <211> 20
   <212> PRT
   <213> Homo sapiens
<400> 380
   Gln Ala Pro Glu Tyr Arg Gly Arg Thr Glu Leu Leu Lys Asp Ala Ile
   1               5                   10                  15
   Gly Glu Gly Lys
               20
<210> 381
   <211> 20
   <212> PRT
   <213> Homo sapiens
<400> 381
   Arg Asp His Ser Tyr Gln Glu Glu Ala Ala Met Glu Leu Lys Val Glu
   1               5                   10                  15
   Asp Pro Phe Tyr
               20
<210> 382
   <211> 20
   <212> PRT
   <213> Homo sapiens
<400> 382
   Arg Pro Pro Phe Ser Arg Val Val His Leu Tyr Arg Asn Gly Lys Asp
   1               5                   10                  15
   Gln Asp Gly Asp
               20
<210> 383
   <211> 20
   <212> PRT
   <213> Homo sapiens
<400> 383
   Ser Asp Glu Gly Gly Phe Thr Cys Phe Phe Arg Asp His Ser Tyr Gln
   1               5                   10                  15
   Glu Glu Ala Ala
   20
<210> 384
   <211> 20
   <212> PRT
   <213> Homo sapiens
<400> 384
   Ser Lys Ile Phe Lys Leu Gly Gly Arg Asp Ser Arg Ser Gly Ser Pro
   1               5                   10                  15
   Met Ala Arg Arg
               20
<210> 385
   <211> 19
   <212> PRT
   <213> Homo sapiens
<400> 385
   Ser Arg Gly Gln His Gln Ala His Ser Leu Glu Arg Val Cys His Cys
   1 5 10 15
   Leu Gly Lys
<210> 386
   <211> 20
   <212> PRT
   <213> Homo sapiens
<400> 386
   Val Thr Leu Arg Ile Arg Asn Val Arg Phe Ser Asp Glu Gly Gly Phe
   1 5 10 15
   Thr Cys Phe Phe
   20
<210> 387
   <211> 20
   <212> PRT
   <213> Homo sapiens
<400> 387
   Tyr Arg Asn Gly Lys Asp Gln Asp Gly Asp Gln Ala Pro Glu Tyr Arg
   1 5 10 15
   Gly Arg Thr Glu
   20
<210> 388
   <211> 9
   <212> PRT
   <213> Homo sapiens
<400> 388
   Leu Met Trp Ala Lys Ile Gly Pro Val
   1 5
<210> 389
   <211> 9
   <212> PRT
   <213> Homo sapiens
<400> 389
   Ser Leu Leu Leu Glu Leu Glu Glu Val
   1 5
<210> 390
   <211> 9
   <212> PRT
   <213> Homo sapiens
<400> 390
   Val Leu Phe Ser Ser Asp Phe Arg Ile
   1 5
<210> 391
   <211> 12
   <212> PRT
   <213> Homo sapiens
<400> 391
   Ala Pro Glu Tyr Arg Gly Arg Thr Glu Leu Leu Lys
   1 5 10
<210> 392
   <211> 12
   <212> PRT
   <213> Homo sapiens
<400> 392
   Ala Thr Gly Met Glu Val Gly Trp Tyr Arg Pro Pro
   1 5 10
<210> 393
   <211> 12
   <212> PRT
   <213> Homo sapiens
<400> 393
   Asp Glu Gly Gly Phe Thr Cys Phe Phe Arg Asp His
   1 5 10
<210> 394
   <211> 12
   <212> PRT
   <213> Homo sapiens
<400> 394
   Asp His Ser Tyr Gln Glu Glu Ala Ala Met Glu Leu
   1 5 10
<210> 395
   <211> 12
   <212> PRT
   <213> Homo sapiens
<400> 395
   Asp Pro Phe Tyr Trp Val Ser Pro Gly Val Leu Val
   1 5 10
<210> 396
   <211> 12
   <212> PRT
   <213> Homo sapiens
<400> 396
   Glu Glu Ala Ala Met Glu Leu Lys Val Glu Asp Pro
   1 5 10
<210> 397
   <211> 12
   <212> PRT
   <213> Homo sapiens
<400> 397
   Glu Leu Lys Val Glu Asp Pro Phe Tyr Trp Val Ser
   1 5 10
<210> 398
   <211> 12
   <212> PRT
   <213> Homo sapiens
<400> 398
   Gly Asp Glu Val Glu Leu Pro Cys Arg Ile Ser Pro
   1 5 10
<210> 399
   <211> 12
   <212> PRT
   <213> Homo sapiens
<400> 399
   Gly Glu Gly Lys Val Thr Leu Arg Ile Arg Asn Val
   1 5 10
<210> 400
   <211> 12
   <212> PRT
   <213> Homo sapiens
<400> 400
   Gly Pro Arg His Pro Ile Arg Ala Leu Val Gly Asp
   1 5 10
<210> 401
   <211> 12
   <212> PRT
   <213> Homo sapiens
<400> 401
   Gly Arg Thr Glu Leu Leu Lys Asp Ala Ile Gly Glu
   1 5 10
<210> 402
   <211> 12
   <212> PRT
   <213> Homo sapiens
<400> 402
   Ile Arg Ala Leu Val Gly Asp Glu Val Glu Leu Pro
   1 5 10
<210> 403
   <211> 19
   <212> PRT
   <213> Homo sapiens
<400> 403
   Lys Asn Ile Val Thr Pro Arg Thr Pro Pro Pro Ser Gln Gly Lys Gly
   1 5 10 15
   Arg Gly Leu
<210> 404
   <211> 12
   <212> PRT
   <213> Homo sapiens
<400> 404
   Leu Lys Asp Ala Ile Gly Glu Gly Lys Val Thr Leu
   1 5 10
<210> 405
   <211> 12
   <212> PRT
   <213> Homo sapiens
<400> 405
   Leu Pro Cys Arg Ile Ser Pro Gly Lys Asn Ala Thr
   1 5 10
<210> 406
   <211> 19
   <212> PRT
   <213> Homo sapiens
<400> 406
   Met Asp His Ala Arg His Gly Phe Leu Pro Arg His Arg Asp Thr Gly
   1               5                   10                  15
   Ile Leu Asp
<210> 407
   <211> 12
   <212> PRT
   <213> Homo sapiens
<400> 407
   Asn Val Arg Phe Ser Asp Glu Gly Gly Phe Thr Cys
   1               5                   10
<210> 408
   <211> 12
   <212> PRT
   <213> Homo sapiens
<400> 408
   Pro Pro Phe Ser Arg Val Val His Leu Tyr Arg Asn
   1               5                   10
<210> 409
   <211> 19
   <212> PRT
   <213> Homo sapiens
<400> 409
   Pro Ser Gln Gly Lys Gly Arg Gly Leu Ser Leu Ser Arg Phe Ser Trp
   1               5                   10                  15
   Gly Ala Glu
<210> 410
   <211> 12
   <212> PRT
   <213> Homo sapiens
<400> 410
   Gln Asp Gly Asp Gln Ala Pro Glu Tyr Arg Gly Arg
   1               5                   10
<210> 411
   <211> 12
   <212> PRT
   <213> Homo sapiens
<400> 411
   Gln Phe Arg Val Ile Gly Pro Arg His Pro Ile Arg
   1               5                   10
<210> 412
   <211> 19
   <212> PRT
   <213> Homo sapiens
<400> 412
   Gln Arg Pro Gly Phe Gly Tyr Gly Gly Arg Ala Ser Asp Tyr Lys Ser
   1               5                   10                  15
   Ala His Lys
<210> 413
   <211> 12
   <212> PRT
   <213> Homo sapiens
<400> 413
   Arg Asn Gly Lys Asp Gln Asp Gly Asp Gln Ala Pro
   1               5                   10
<210> 414
   <211> 19
   <212> PRT
   <213> Homo sapiens
<400> 414
   Ser Lys Ile Phe Lys Leu Gly Gly Arg Asp Ser Arg Ser Gly Ser Pro
   1               5                   10                  15
   Met Ala Arg
<210> 415
   <211> 19
   <212> PRT
   <213> Homo sapiens
<400> 415
   Ser Leu Pro Gln Lys Ser His Gly Arg Thr Gln Asp Glu Asn Pro Val
   1               5                   10                  15
   Val His Phe
<210> 416
   <211> 12
   <212> PRT
   <213> Homo sapiens
<400> 416
   Ser Pro Gly Lys Asn Ala Thr Gly Met Glu Val Gly
   1               5                   10
<210> 417
   <211> 12
   <212> PRT
   <213> Homo sapiens
<400> 417
   Ser Ser Tyr Ala Gly Gln Phe Arg Val Ile Gly Pro
   1               5                   10
<210> 418
   <211> 9
   <212> PRT
   <213> Homo sapiens
<400> 418
   Ser Trp Gly Ala Glu Gly Gln Arg Pro
   1               5
<210> 419
   <211> 10
   <212> PRT
   <213> Homo sapiens
<400> 419
   Ser Trp Gly Ala Glu Gly Gln Arg Pro Gly
   1               5                   10
<210> 420
   <211> 12
   <212> PRT
   <213> Homo sapiens
<400> 420
   Thr Leu Arg Ile Arg Asn Val Arg Phe Ser Asp Glu
   1               5                   10
<210> 421
   <211> 12
   <212> PRT
   <213> Homo sapiens
<400> 421
   Val Gly Trp Tyr Arg Pro Pro Phe Ser Arg Val Val
   1               5                   10
<210> 422
   <211> 12
   <212> PRT
   <213> Homo sapiens
<400> 422
   Val Val His Leu Tyr Arg Asn Gly Lys Asp Gln Asp
   1 5 10
<210> 423
   <211> 8
   <212> PRT
   <213> Homo sapiens
<400> 423
   Trp Gly Ala Glu Gly Gln Arg Pro
   1 5
<210> 424
   <211> 10
   <212> PRT
   <213> Homo sapiens
<400> 424
   Trp Gly Ala Glu Gly Gln Arg Pro Gly Phe
   1 5 10
<210> 425
   <211> 5
   <212> PRT
   <213> Homo sapiens
<400> 425
   Ala Ala Met Glu Leu
   1 5
<210> 426
   <211> 5
   <212> PRT
   <213> Homo sapiens
<400> 426
   Ala Asp Ala Arg Met
   1 5
<210> 427
   <211> 5
   <212> PRT
   <213> Homo sapiens
<400> 427
   Ala His Arg Glu Thr
   1 5
<210> 428
   <211> 15
   <212> PRT
   <213> Bacillus subtilis
<400> 428
   Ala Leu Ala Val Leu His Phe Tyr Pro Asp Lys Gly Ala Lys Asn
   1 5 10 15
<210> 429
   <211> 21
   <212> PRT
   <213> Unknown
<220>
   <223> epitope involved in multiple sclerosis
<400> 429
<210> 430
   <211> 21
   <212> PRT
   <213> Homo sapiens
<400> 430
   Ala Leu Gly Gln Gly Pro Gln Phe Ile Phe Gln Tyr Tyr Glu Glu Glu
   1 5 10 15
   Glu Arg Gln Arg Gly
   20
<210> 431
   <211> 21
   <212> PRT
   <213> Homo sapiens
<400> 431
   Ala Ser Thr Met Asp His Ala Arg His Gly Phe Leu Pro Arg His Arg
   1 5 10 15
   Asp Thr Gly Ile Leu
   20
<210> 432
   <211> 15
   <212> PRT
   <213> Unknown
<220>
   <223> epitope involved in multiple sclerosis
<400> 432
   Asp Phe Ala Arg Val His Phe Ile Arg Ala Leu His Gly Ser Gly
   1 5 10 15
<210> 433
   <211> 15
   <212> PRT
   <213> Haemophilus influenzae
<400> 433
   Asp Phe Ala Arg Val His Phe Ile Ser Ala Leu His Gly Ser Gly
   1 5 10 15
<210> 434
   <211> 21
   <212> PRT
   <213> Homo sapiens
<400> 434
   Asp Pro Gly His Gly Leu Arg Leu Ile His Tyr Ser Tyr Gly Val Lys
   1 5 10 15
   Asp Thr Asp Lys Gly
   20
<210> 435
   <211> 21
   <212> PRT
   <213> Homo sapiens
<400> 435
   Asp Pro Gly Leu Gly Leu Arg Leu Ile Tyr Phe Ser Tyr Asp Val Lys
   1 5 10 15
   Met Lys Glu Lys Gly
   20
<210> 436
   <211> 20
   <212> PRT
   <213> Homo sapiens
<400> 436
   Gly Asn Phe Pro Asp Arg Phe Ser Gly His Gln Phe Pro Asn Tyr Ser
   1 5 10 15
   Ser Glu Leu Asn
   20
<210> 437
   <211> 19
   <212> PRT
   <213> Homo sapiens
<400> 437
   Gly Gln Gly Pro Gln Phe Ile Phe Gln Tyr Tyr Glu Glu Glu Glu Arg
   1 5 10 15
   Gln Arg Gly
<210> 438
   <211> 15
   <212> PRT
   <213> Homo sapiens
<400> 438
   Gly Arg Ala Ser Asp Tyr Lys Ser Ala His Lys Gly Phe Lys Gly
   1 5 10 15
<210> 439
   <211> 18
   <212> PRT
   <213> Cavia porcellus
<400> 439
   Gly Thr Leu Ser Lys Ile Phe Lys Leu Gly Gly Arg Asp Ser Arg Ser
   1 5 10 15
   Gly Ser
<210> 440
   <211> 5
   <212> PRT
   <213> Homo sapiens
<400> 440
   His Arg Thr Phe Glu
   1 5
<210> 441
   <211> 5
   <212> PRT
   <213> Homo sapiens
<400> 441
   His Ser Tyr Gln Glu
   1 5
<210> 442
   <211> 6
   <212> PRT
   <213> Homo sapiens
<400> 442
   Lys Thr Gly Gln Phe Leu
   1               5
<210> 443
   <211> 21
   <212> PRT
   <213> Homo sapiens
<400> 443
   Lys Thr Arg Gly Gln Gln Val Thr Leu Arg Cys Ser Pro Lys Ser Gly
   1               5                   10                  15
   His Asp Thr Val Ser
               20
<210> 444
   <211> 20
   <212> PRT
   <213> Homo sapiens
<400> 444
   Leu Val Lys Arg Thr Gly Glu Lys Val Phe Leu Glu Cys Val Gln Asp
   1               5                   10                  15
   Met Asp His Glu
               20
<210> 445
   <211> 16
   <212> PRT
   <213> Homo sapiens
<400> 445
   Asn Ala Leu Leu Leu Gly Asp Ser Ala Leu Tyr Leu Cys Ala Ser Ser
   1               5                   10                  15
<210> 446
   <211> 24
   <212> PRT
   <213> Cavia porcellus
<400> 446
   Pro Ala Arg Thr Ala His Tyr Gly Ser Leu Pro Gln Lys Ser Gly Arg
   1               5                   10                  15
   Ser Gln Asp Glu Asn Pro Val Val
               20
<210> 447
   <211> 18
   <212> PRT
   <213> Homo sapiens
<400> 447
   Pro Lys Ser Gly His Asp Thr Val Ser Trp Tyr Gln Gln Ala Leu Gly
   1               5                   10                  15
   Gln Gly
<210> 448
   <211> 19
   <212> PRT
   <213> Homo sapiens
<400> 448
   Pro Asn Tyr Ser Ser Glu Leu Asn Val Asn Ala Leu Leu Leu Gly Asp
   1               5                   10                  15
   Ser Ala Leu
<210> 449
   <211> 40
   <212> PRT
   <213> Homo sapiens
<400> 449
   Pro Ser Gln Gly Lys Gly Arg Gly Leu Ser Leu Ser Arg Phe Ser Trp
   1               5                   10                  15
   Gly Ala Glu Gly Gln Arg Pro Gly Phe Gly Tyr Gly Gly Arg Ala Ser
               20                  25                  30
   Asp Tyr Lys Ser Ala His Lys Gly
           35                  40
<210> 450
   <211> 5
   <212> PRT
   <213> Homo sapiens
<400> 450
   Gln Ala Pro Glu Tyr
   1               5
<210> 451
   <211> 15
   <212> PRT
   <213> Mycobacterium avium
<400> 451
   Gln Arg Cys Arg Val His Phe Leu Arg Asn Val Leu Ala Gln Val
   1               5                   10                  15
<210> 452
   <211> 15
   <212> PRT
   <213> Mycobacterium tuberculosis
<400> 452
   Gln Arg Cys Arg Val His Phe Met Arg Asn Leu Tyr Thr Ala Val
   1               5                   10                  15
<210> 453
   <211> 19
   <212> PRT
   <213> Homo sapiens
<400> 453
   Gln Tyr Tyr Glu Glu Glu Glu Arg Gln Arg Gly Asn Phe Pro Asp Arg
   1               5                   10                  15
   Phe Ser Gly
<210> 454
   <211> 16
   <212> PRT
   <213> Homo sapiens
<400> 454
   Ser His Gly Arg Thr Gln Asp Glu Asn Pro Val Val His Phe Phe Lys
   1               5                   10                  15
<210> 455
   <211> 20
   <212> PRT
   <213> Homo sapiens
<400> 455
   Ser Lys Ile Phe Lys Leu Gly Gly Arg Asp Ser Arg Ser Gly Ser Pro
   1               5                   10                  15
   Met Ala Arg Arg
               20
<210> 456
   <211> 21
   <212> PRT
   <213> Homo sapiens
<400> 456
   Ser Lys Lys Phe Leu Lys Ile Met Phe Ser Tyr Asn Asn Lys Glu Leu
   1 5 10 15
   Ile Ile Asn Glu Thr
   20
<210> 457
   <211> 19
   <212> PRT
   <213> Cavia porcellus
<400> 457
   Ser Leu Ser Arg Phe Ser Trp Gly Ala Glu Gly Gln Lys Pro Gly Phe
   1 5 10 15
   Gly Tyr Gly
<210> 458
   <211> 17
   <212> PRT
   <213> Homo sapiens
<400> 458
   Ser Trp Gly Ala Glu Gly Gln Arg Pro Gly Phe Gly Tyr Gly Gly Arg
   1 5 10 15
   Ala
<210> 459
   <211> 20
   <212> PRT
   <213> Homo sapiens
<400> 459
   Ser Trp Tyr Gln Gln Ala Leu Gly Gln Gly Pro Gln Phe Ile Phe Gln
   1 5 10 15
   Tyr Tyr Glu Glu
   20
<210> 460
   <211> 22
   <212> PRT
   <213> Homo sapiens
<400> 460
   Thr Ser Gly Val Thr Gln Ser Pro His Thr Leu Ile Lys Thr Arg Gly
   1 5 10 15
   Gln Gln Val Thr Leu Arg
   20
<210> 461
   <211> 15
   <212> PRT
   <213> Staphylococcus aureus
<400> 461
   Val Leu Ala Arg Leu His Phe Tyr Arg Asn Asp Val His Lys Glu
   1 5 10 15
<210> 462
   <211> 5
   <212> PRT
   <213> Homo sapiens
<400> 462
   Val Thr Leu Arg Ile
   1 5
<210> 463
   <211> 20
   <212> PRT
   <213> Unknown
<220>
   <223> epitope involved in multiple sclerosis
<400> 463
   Tyr Asp Glu Asn Pro Val Val His Phe Phe Lys Asn Ile Val Thr Pro
   1 5 10 15
   Arg Thr Pro Pro
   20
<210> 464
   <211> 15
   <212> PRT
   <213> Influenza A virus
<400> 464
   Tyr Arg Asn Leu Val Trp Phe Ile Lys Lys Asn Thr Arg Tyr Pro
   1 5 10 15
<210> 465
   <211> 20
   <212> PRT
   <213> Homo sapiens
<400> 465
   Ala Pro Lys Arg Gly Ser Gly Lys Asp Ser His His Pro Ala Arg Thr
   1               5                   10                  15
   Ala His Tyr Gly
               20
<210> 466
   <211> 20
   <212> PRT
   <213> Homo sapiens
<400> 466
   Ala Ser Asp Tyr Lys Ser Ala His Lys Gly Phe Lys Gly Val Asp Ala
   1               5                   10                  15
   Gln Gly Thr Leu
               20
<210> 467
   <211> 23
   <212> PRT
   <213> Homo sapiens
<400> 467
   Ala Thr Ala Ser Thr Met Asp His Ala Arg His Gly Phe Leu Pro Arg
   1               5                   10                  15
   His Arg Asp Thr Gly Ile Leu
               20
<210> 468
   <211> 45
   <212> PRT
   <213> Homo sapiens
<400> 468
   Phe Gly Gly Asp Arg Gly Ala Pro Lys Arg Gly Ser Gly Lys Asp Ser
   1               5                   10                  15
   His His Pro Ala Arg Thr Ala His Tyr Gly Ser Leu Pro Gln Lys Ser
               20                  25                  30
   His Gly Arg Thr Gln Asp Glu Asn Pro Val Val His Phe
           35                  40                  45
<210> 469
   <211> 19
   <212> PRT
   <213> Homo sapiens
<400> 469
   Gly Ala Pro Lys Arg Gly Ser Gly Lys Asp Ser His His Pro Ala Arg
   1 5 10 15
   Thr Ala His
<210> 470
   <211> 18
   <212> PRT
   <213> Homo sapiens
<400> 470
   Gly Phe Gly Tyr Gly Gly Arg Ala Ser Asp Tyr Lys Ser Ala His Lys
   1 5 10 15
   Gly Phe
<210> 471
   <211> 15
   <212> PRT
   <213> Homo sapiens
<400> 471
   His Lys Gly Phe Lys Gly Val Asp Ala Gln Gly Thr Leu Ser Lys
   1 5 10 15
<210> 472
   <211> 20
   <212> PRT
   <213> Homo sapiens
<400> 472
   Ile Gly Arg Phe Phe Gly Gly Asp Arg Gly Ala Pro Lys Arg Gly Ser
   1 5 10 15
   Gly Lys Asp Ser
   20
<210> 473
   <211> 12
   <212> PRT
   <213> Cavia porcellus
<400> 473
   Ile Val Thr Pro Arg Thr Pro Pro Pro Ser Gln Gly
   1 5 10
<210> 474
   <211> 20
   <212> PRT
   <213> Homo sapiens
<400> 474
   Leu Cys Asn Met Tyr Lys Lys Asp Ser His His Pro Ala Arg Thr Ala
   1               5                   10                  15
   His Tyr Gly Ser
               20
<210> 475
   <211> 21
   <212> PRT
   <213> Homo sapiens
<400> 475
   Leu Asp Ser Ile Gly Arg Phe Phe Gly Gly Asp Arg Gly Ala Pro Lys
   1               5                   10                  15
   Arg Gly Ser Gly Lys
               20
<210> 476
   <211> 19
   <212> PRT
   <213> Homo sapiens
<400> 476
   Leu Gly Gln Gly Pro Glu Phe Leu Thr Tyr Phe Gln Asn Glu Ala Gln
   1               5                   10                  15
   Leu Lys Ser
<210> 477
   <211> 20
   <212> PRT
   <213> Homo sapiens
<400> 477
   Leu Pro Ser His Ala Arg Ser Gln Pro Gly Leu Cys Asn Met Tyr Lys
   1               5                   10                  15
   Lys Asp Ser His
               20
<210> 478
   <211> 19
   <212> PRT
   <213> Homo sapiens
<400> 478
   Pro Arg His Arg Asp Thr Gly Ile Leu Asp Ser Ile Gly Arg Phe Phe
   1 5 10 15
   Gly Gly Asp
<210> 479
   <211> 20
   <212> PRT
   <213> Homo sapiens
<400> 479
   Pro Arg Thr Pro Pro Pro Ser Gln Gly Lys Gly Ala Glu Gly Gln Arg
   1 5 10 15
   Pro Gly Phe Gly
   20
<210> 480
   <211> 20
   <212> PRT
   <213> Homo sapiens
<400> 480
   Arg Gly Ala Pro Lys Arg Gly Ser Gly Lys Val Pro Trp Leu Lys Pro
   1 5 10 15
   Gly Arg Ser Pro
   20
<210> 481
   <211> 20
   <212> PRT
   <213> Homo sapiens
<400> 481
   Ser Leu Pro Gln Lys Ser His Gly Arg Thr Gln Asp Glu Asn Pro Val
   1 5 10 15
   Val His Phe Phe
   20
<210> 482
   <211> 20
   <212> PRT
   <213> Homo sapiens
<400> 482
   Thr Gln Asp Glu Asn Pro Val Val His Phe Phe Lys Asn Ile Val Thr
   1 5 10 15
   Pro Arg Thr Pro
   20
<210> 483
   <211> 18
   <212> PRT
   <213> Homo sapiens
<400> 483
   Thr Gln Asp Gln Asp Pro Asx Asx His Phe Phe Lys Asn Ile Val Thr
   1 5 10 15
   Pro Arg
<210> 484
   <211> 20
   <212> PRT
   <213> Homo sapiens
<400> 484
   Val Pro Trp Leu Lys Pro Gly Arg Ser Pro Leu Pro Ser His Ala Arg
   1 5 10 15
   Ser Gln Pro Gly
   20
<210> 485
   <211> 24
   <212> PRT
   <213> Homo sapiens
<400> 485
   Val Thr Pro Arg Thr Pro Pro Pro Ser Gln Gly Lys Gly Arg Gly Leu
   1 5 10 15
   Ser Leu Ser Arg Phe Ser Trp Gly
   20
<210> 486
   <211> 19
   <212> PRT
   <213> Homo sapiens
<400> 486
   Ala Pro Lys Arg Gly Ser Gly Lys Asp Ser His His Pro Ala Arg Thr
   1               5                   10                  15
   Ala His Tyr
<210> 487
   <211> 27
   <212> PRT
   <213> Homo sapiens
<400> 487
   Ala Ser Gln Lys Arg Pro Ser Gln Arg His Gly Ser Lys Tyr Leu Ala
   1               5                   10                  15
   Thr Ala Ser Thr Met Asp His Ala Arg His Gly
               20                  25
<210> 488
   <211> 13
   <212> PRT
   <213> Homo sapiens
<400> 488
   Asp Pro Val Val His Phe Phe Lys Asn Ile Val Thr Pro
   1               5                   10
<210> 489
   <211> 19
   <212> PRT
   <213> Homo sapiens
<400> 489
   Glu Gly Gln Arg Pro Gly Phe Gly Tyr Gly Gly Arg Ala Ser Asp Tyr
   1               5                   10                  15
   Lys Ser Ala
<210> 490
   <211> 19
   <212> PRT
   <213> Homo sapiens
<400> 490
   Phe Phe Lys Asn Ile Val Thr Pro Arg Thr Pro Pro Pro Ser Gln Gly
   1               5                   10                  15
   Lys Gly Arg
<210> 491
   <211> 43
   <212> PRT
   <213> Homo sapiens
<400> 491
   Phe Gly Gly Asp Arg Gly Ala Pro Lys Arg Gly Ser Gly Lys Asp Ser
   1               5                   10                  15
   His His Pro Ala Arg Thr Ala His Tyr Gly Ser Leu Pro Gln Lys Ser
               20                  25                  30
   His Gly Arg Thr Gln Asp Glu Asn Pro Val Val
           35                  40
<210> 492
   <211> 12
   <212> PRT
   <213> Mus musculus
<400> 492
   Phe Lys Asn Ile Val Thr Pro Arg Thr Pro Pro Pro
   1               5                   10
<210> 493
   <211> 40
   <212> PRT
   <213> Homo sapiens
<400> 493
   Gly Leu Ser Leu Ser Arg Phe Ser Trp Gly Ala Glu Gly Gln Arg Pro
   1               5                   10                  15
   Gly Phe Gly Tyr Gly Gly Arg Ala Ser Asp Tyr Lys Ser Ala His Lys
               20                  25                  30
   Gly Phe Lys Gly Val Asp Ala Gln
           35                  40
<210> 494
   <211> 19
   <212> PRT
   <213> Homo sapiens
<400> 494
   Gly Arg Ala Ser Asp Tyr Lys Ser Ala His Lys Gly Phe Lys Gly Val
   1               5                   10                  15
   Asp Ala Gln
<210> 495
   <211> 17
   <212> PRT
   <213> Homo sapiens
<400> 495
   His His Pro Ala Arg Thr Ala His Tyr Gly Ser Leu Pro Gln Lys Ser
   1               5                   10                  15
   His
<210> 496
   <211> 19
   <212> PRT
   <213> Homo sapiens
<400> 496
   Ile Gly Arg Phe Phe Gly Gly Asp Arg Gly Ala Pro Lys Arg Gly Ser
   1               5                   10                  15
   Gly Lys Asp
<210> 497
   <211> 19
   <212> PRT
   <213> Homo sapiens
<400> 497
   Lys Gly Phe Lys Gly Val Asp Ala Gln Gly Thr Leu Ser Lys Ile Phe
   1               5                   10                  15
   Lys Leu Gly
<210> 498
   <211> 24
   <212> PRT
   <213> Homo sapiens
<400> 498
   Lys Asn Ile Val Thr Pro Arg Thr Pro Pro Pro Ser Gln Gly Lys Gly
   1               5                   10                  15
   Arg Gly Leu Ser Leu Ser Arg Phe
               20
<210> 499
   <211> 29
   <212> PRT
   <213> Homo sapiens
<400> 499
   Lys Arg Gly Ser Gly Lys Asp Ser His His Pro Ala Arg Thr Ala His
   1               5                   10                  15
   Tyr Gly Ser Leu Pro Gln Lys Ser His Gly Arg Thr Gln
               20                  25
<210> 500
   <211> 8
   <212> PRT
   <213> Homo sapiens
<400> 500
   Leu Gly Arg Ala Gly Leu Thr Tyr
   1               5
<210> 501
   <211> 19
   <212> PRT
   <213> Homo sapiens
<400> 501
   Leu Ser Leu Ser Arg Phe Ser Trp Gly Ala Glu Gly Gln Arg Pro Gly
   1               5                   10                  15
   Phe Gly Tyr
<210> 502
   <211> 18
   <212> PRT
   <213> Homo sapiens
<400> 502
   Pro Gly Phe Gly Tyr Gly Gly Arg Ala Ser Asp Tyr Lys Ser Ala His
   1               5                   10                  15
   Lys Gly
<210> 503
   <211> 19
   <212> PRT
   <213> Homo sapiens
<400> 503
   Pro Pro Pro Ser Gln Gly Lys Gly Arg Gly Leu Ser Leu Ser Arg Phe
   1               5                   10                  15
   Ser Trp Gly
<210> 504
   <211> 33
   <212> PRT
   <213> Oryctolagus cuniculus
<400> 504
   Pro Pro Pro Ser Gln Gly Lys Gly Arg Gly Thr Val Leu Ser Arg Phe
   1               5                   10                  15
   Ser Trp Gly Ala Glu Gly Gln Lys Pro Gly Phe Gly Tyr Gly Gly Arg
               20                  25                  30
   Ala
<210> 505
   <211> 17
   <212> PRT
   <213> Homo sapiens
<400> 505
   Gln Asp Glu Asn Pro Val Val His Phe Phe Lys Asn Ile Val Thr Pro
   1               5                   10                  15
   Arg
<210> 506
   <211> 23
   <212> PRT
   <213> Homo sapiens
<400> 506
   Gln Asp Glu Asn Pro Val Val His Phe Phe Lys Asn Ile Val Thr Pro
   1               5                   10                  15
   Arg Thr Pro Pro Pro Ser Gln
               20
<210> 507
   <211> 27
   <212> PRT
   <213> Homo sapiens
<400> 507
   Arg Ala Ser Asp Tyr Lys Ser Ala His Lys Gly Phe Lys Gly Val Asp
   1               5                   10                  15
   Ala Gln Gly Thr Leu Ser Lys Ile Phe Lys Leu
               20                  25
<210> 508
   <211> 26
   <212> PRT
   <213> Homo sapiens
<400> 508
   Arg Phe Phe Gly Gly Asp Arg Gly Ala Pro Lys Arg Gly Ser Gly Lys
   1               5                   10                  15
   Asp Ser His His Pro Ala Arg Thr Ala His
               20                  25
<210> 509
   <211> 26
   <212> PRT
   <213> Homo sapiens
<400> 509
   Arg Gly Leu Ser Leu Ser Arg Phe Ser Trp Gly Ala Glu Gly Gln Arg
   1               5                   10                  15
   Pro Gly Phe Gly Tyr Gly Gly Arg Ala Ser
               20                  25
<210> 510
   <211> 30
   <212> PRT
   <213> Homo sapiens
<400> 510
   Arg His Gly Phe Leu Pro Arg His Arg Asp Thr Gly Ile Leu Asp Ser
   1               5                   10                  15
   Ile Gly Arg Phe Phe Gly Gly Asp Arg Gly Ala Pro Lys Arg
               20                  25                  30
<210> 511
   <211> 19
   <212> PRT
   <213> Homo sapiens
<400> 511
   Arg His Arg Asp Thr Gly Ile Leu Asp Ser Ile Gly Arg Phe Phe Gly
   1               5                   10                  15
   Gly Asp Arg
<210> 512
   <211> 28
   <212> PRT
   <213> Homo sapiens
<400> 512
   Arg Thr Ala His Tyr Gly Ser Leu Pro Gln Lys Ser His Gly Arg Thr
   1               5                   10                  15
   Gln Asp Glu Asn Pro Val Val His Phe Phe Lys Asn
               20                  25
<210> 513
   <211> 17
   <212> PRT
   <213> Homo sapiens
<400> 513
   Ser Leu Pro Gln Lys Ser His Gly Arg Thr Gln Asp Glu Asn Pro Val
   1               5                   10                  15
   Val
<210> 514
   <211> 15
   <212> PRT
   <213> Homo sapiens
<400> 514
   Ser Ser Leu Gly Arg Ala Gly Leu Thr Tyr Glu Gln Tyr Phe Gly
   1               5                   10                  15
<210> 515
   <211> 31
   <212> PRT
   <213> Homo sapiens
<400> 515
   Thr Ala Ser Thr Met Asp His Ala Arg His Gly Phe Leu Pro Arg His
   1               5                   10                  15
   Arg Asp Thr Gly Ile Leu Asp Ser Ile Gly Arg Phe Phe Gly Gly
               20                  25                  30
<210> 516
   <211> 21
   <212> PRT
   <213> Homo sapiens
<400> 516
   Ala His Ser Leu Glu Arg Val Cys His Cys Leu Gly Lys Trp Leu Gly
   1 5 10 15
   His Pro Asp Lys Phe
   20
<210> 517
   <211> 21
   <212> PRT
   <213> Homo sapiens
<400> 517
   Ala Val Arg Gln Ile Phe Gly Asp Tyr Lys Thr Thr Ile Cys Gly Lys
   1 5 10 15
   Gly Leu Ser Ala Thr
   20
<210> 518
   <211> 14
   <212> PRT
   <213> Homo sapiens
<400> 518
   Cys Asn Met Tyr Lys Asp Ser His His Pro Ala Arg Thr Ala
   1 5 10
<210> 519
   <211> 20
   <212> PRT
   <213> Homo sapiens
<400> 519
   Cys Gln Ser Ile Ala Phe Pro Ser Lys Thr Ser Ala Ser Ile Gly Ser
   1 5 10 15
   Leu Cys Ala Asp
   20
<210> 520
   <211> 21
   <212> PRT
   <213> Homo sapiens
<400> 520
   Asp Gln Ala Pro Glu Tyr Arg Gly Arg Thr Glu Leu Leu Lys Asp Ala
   1 5 10 15
   Ile Gly Glu Gly Lys
   20
<210> 521
   <211> 20
   <212> PRT
   <213> Homo sapiens
<400> 521
   Glu Gly Lys Val Thr Leu Arg Ile Arg Asn Val Arg Phe Ser Asp Glu
   1               5                   10                  15
   Gly Gly Phe Thr
               20
<210> 522
   <211> 21
   <212> PRT
   <213> Homo sapiens
<400> 522
   Glu Leu Lys Val Glu Asp Pro Phe Tyr Trp Val Ser Pro Gly Val Leu
   1               5                   10                  15
   Val Leu Leu Ala Val
   20
<210> 523
   <211> 21
   <212> PRT
   <213> Homo sapiens
<400> 523
   Glu Val Glu Leu Pro Cys Arg Ile Ser Pro Gly Lys Asn Ala Thr Gly
   1               5                   10                  15
   Met Glu Val Gly Trp
               20
<210> 524
   <211> 21
   <212> PRT
   <213> Homo sapiens
<400> 524
   Glu Tyr Leu Ile Asn Val Ile His Ala Phe Gln Tyr Val Ile Tyr Gly
   1               5                   10                  15
   Thr Ala Ser Phe Phe
               20
<210> 525
   <211> 21
   <212> PRT
   <213> Homo sapiens
<400> 525
   Phe Phe Gly Val Ala Leu Phe Cys Gly Cys Gly His Glu Ala Leu Thr
   1               5                   10                  15
   Gly Thr Glu Lys Leu
               20
<210> 526
   <211> 19
   <212> PRT
   <213> Homo sapiens
<400> 526
   Phe Gly Gly Asp Arg Gly Ala Pro Lys Arg Gly Ser Gly Lys Val Pro
   1               5                   10                  15
   Trp Leu Lys
<210> 527
   <211> 21
   <212> PRT
   <213> Homo sapiens
<400> 527
   Phe Lys Gly Val Asp Ala Gln Gly Thr Leu Ser Lys Ile Phe Lys Leu
   1               5                   10                  15
   Gly Gly Arg Asp Ser
               20
<210> 528
   <211> 21
   <212> PRT
   <213> Homo sapiens
<400> 528
   Phe Met Ile Ala Ala Thr Tyr Asn Phe Ala Val Leu Lys Leu Met Gly
   1               5                   10                  15
   Arg Gly Thr Lys Phe
               20
<210> 529
   <211> 19
   <212> PRT
   <213> Homo sapiens
<400> 529
   Phe Ser Arg Val Val His Leu Tyr Arg Asn Gly Lys Asp Gln Asp Gly
   1 5 10 15
   Asp Gln Ala
<210> 530
   <211> 21
   <212> PRT
   <213> Homo sapiens
<400> 530
   Phe Ser Trp Gly Ala Glu Gly Gln Arg Pro Gly Phe Gly Tyr Gly Gly
   1 5 10 15
   Arg Ala Ser Asp Tyr
   20
<210> 531
   <211> 20
   <212> PRT
   <213> Homo sapiens
<400> 531
   Gly Ala Pro Lys Arg Gly Ser Gly Lys Asp Ser His His Pro Ala Arg
   1 5 10 15
   Thr Ala His Tyr
   20
<210> 532
   <211> 21
   <212> PRT
   <213> Homo sapiens
<400> 532
   Gly Cys Gly His Glu Ala Leu Thr Gly Thr Glu Lys Leu Ile Glu Thr
   1 5 10 15
   Tyr Phe Ser Lys Asn
   20
<210> 533
   <211> 21
   <212> PRT
   <213> Homo sapiens
<400> 533
   Gly Phe Leu Pro Arg His Arg Asp Thr Gly Ile Leu Asp Ser Ile Gly
   1               5                   10                  15
   Arg Phe Phe Gly Gly
               20
<210> 534
   <211> 21
   <212> PRT
   <213> Homo sapiens
<400> 534
   Gly Phe Thr Cys Phe Phe Arg Asp His Ser Tyr Gln Glu Glu Ala Ala
   1               5                   10                  15
   Met Glu Leu Lys Val
               20
<210> 535
   <211> 21
   <212> PRT
   <213> Homo sapiens
<400> 535
   Gly Phe Tyr Thr Thr Gly Ala Val Arg Gln Ile Phe Gly Asp Tyr Lys
   1               5                   10                  15
   Thr Thr Ile Cys Gly
               20
<210> 536
   <211> 21
   <212> PRT
   <213> Homo sapiens
<400> 536
   Gly Ile Leu Asp Ser Ile Gly Arg Phe Phe Gly Gly Asp Arg Gly Ala
   1               5                   10                  15
   Pro Lys Arg Gly Ser
               20
<210> 537
   <211> 21
   <212> PRT
   <213> Homo sapiens
<400> 537
   Gly Gln Phe Arg Val Ile Gly Pro Arg His Pro Ile Arg Ala Leu Val
   1 5 10 15
   Gly Asp Glu Val Glu
   20
<210> 538
   <211> 21
   <212> PRT
   <213> Homo sapiens
<400> 538
   Gly Arg Gly Ser Arg Gly Gln His Gln Ala His Ser Leu Glu Arg Val
   1 5 10 15
   Cys His Cys Leu Gly
   20
<210> 539
   <211> 21
   <212> PRT
   <213> Homo sapiens
<400> 539
   Gly Arg Ser Pro Leu Pro Ser His Ala Arg Ser Gln Pro Gly Leu Cys
   1 5 10 15
   Asn Met Tyr Lys Asp
   20
<210> 540
   <211> 20
   <212> PRT
   <213> Homo sapiens
<400> 540
   Gly Val Leu Pro Trp Asn Ala Phe Pro Gly Lys Val Cys Gly Ser Asn
   1 5 10 15
   Leu Leu Ser Ile
   20
<210> 541
   <211> 21
   <212> PRT
   <213> Homo sapiens
<400> 541
   His Cys Leu Gly Lys Trp Leu Gly His Pro Asp Lys Phe Val Gly Ile
   1               5                   10                  15
   Thr Tyr Ala Leu Thr
               20
<210> 542
   <211> 20
   <212> PRT
   <213> Homo sapiens
<400> 542
   His Gly Ser Lys Tyr Leu Ala Thr Ala Ser Thr Met Asp His Ala Arg
   1               5                   10                  15
   His Gly Phe Leu
               20
<210> 543
   <211> 12
   <212> PRT
   <213> Homo sapiens
<400> 543
   His His Pro Ala Arg Thr Ala His Tyr Gly Ser Leu
   1               5                   10
<210> 544
   <211> 21
   <212> PRT
   <213> Homo sapiens
<400> 544
   His Pro Ile Arg Ala Leu Val Gly Asp Glu Val Glu Leu Pro Cys Arg
   1               5                   10                  15
   Ile Ser Pro Gly Lys
               20
<210> 545
   <211> 21
   <212> PRT
   <213> Homo sapiens
<400> 545
   Ile Thr Val Gly Leu Val Phe Leu Cys Leu Gln Tyr Arg Leu Arg Gly
   1               5                   10                  15
   Lys Leu Arg Ala Glu
               20
<210> 546
   <211> 21
   <212> PRT
   <213> Homo sapiens
<400> 546
   Lys Gly Arg Gly Leu Ser Leu Ser Arg Phe Ser Trp Gly Ala Glu Gly
   1               5                   10                  15
   Gln Arg Pro Gly Phe
               20
<210> 547
   <211> 21
   <212> PRT
   <213> Homo sapiens
<400> 547
   Lys Thr Thr Ile Cys Gly Lys Gly Leu Ser Ala Thr Val Thr Gly Gly
   1               5                   10                  15
   Gln Lys Gly Arg Gly
               20
<210> 548
   <211> 22
   <212> PRT
   <213> Homo sapiens
<400> 548
   Leu Asp Ser Ile Gly Arg Phe Phe Gly Gly Asp Arg Gly Ala Pro Lys
   1               5                   10                  15
   Arg Gly Ser Gly Lys Asp
               20
<210> 549
   <211> 21
   <212> PRT
   <213> Homo sapiens
<400> 549
   Leu Gln Tyr Arg Leu Arg Gly Lys Leu Arg Ala Glu Ile Glu Asn Leu
   1               5                   10                  15
   His Arg Thr Phe Asp
               20
<210> 550
   <211> 21
   <212> PRT
   <213> Homo sapiens
<400> 550
   Leu Ser Lys Ile Phe Lys Leu Gly Gly Arg Asp Ser Arg Ser Gly Ser
   1               5                   10                  15
   Pro Met Ala Arg Arg
               20
<210> 551
   <211> 21
   <212> PRT
   <213> Homo sapiens
<400> 551
   Leu Tyr Gly Ala Leu Leu Leu Ala Glu Gly Phe Tyr Thr Thr Gly Ala
   1               5                   10                  15
   Val Arg Gln Ile Phe
               20
<210> 552
   <211> 21
   <212> PRT
   <213> Homo sapiens
<400> 552
   Met Tyr Lys Asp Ser His His Pro Ala Arg Thr Ala His Tyr Gly Ser
   1               5                   10                  15
   Leu Pro Gln Lys Ser
               20
<210> 553
   <211> 21
   <212> PRT
   <213> Homo sapiens
<400> 553
   Pro Gly Phe Gly Tyr Gly Gly Arg Ala Ser Asp Tyr Lys Ser Ala His
   1               5                   10                  15
   Lys Gly Phe Lys Gly
               20
<210> 554
   <211> 21
   <212> PRT
   <213> Homo sapiens
<400> 554
   Pro Gly Lys Asn Ala Thr Gly Met Glu Val Gly Trp Tyr Arg Pro Pro
   1               5                   10                  15
   Phe Ser Arg Val Val
               20
<210> 555
   <211> 16
   <212> PRT
   <213> Homo sapiens
<400> 555
   Pro Lys Arg Gly Ser Gly Lys Asp Ser His His Pro Ala Arg Thr Ala
   1               5                   10                  15
<210> 556
   <211> 16
   <212> PRT
   <213> Homo sapiens
<400> 556
   Pro Lys Arg Gly Ser Gly Lys Val Pro Trp Leu Lys Pro Gly Arg Ser
   1               5                   10                  15
<210> 557
   <211> 21
   <212> PRT
   <213> Homo sapiens
<400> 557
   Pro Gln Lys Ser His Gly Arg Thr Gln Asp Glu Asn Pro Val Val His
   1               5                   10                  15
   Phe Phe Lys Asn Ile
               20
<210> 558
   <211> 21
   <212> PRT
   <213> Homo sapiens
<400> 558
   Pro Arg Thr Pro Pro Pro Ser Gln Gly Lys Gly Arg Gly Leu Ser Leu
   1               5                   10                  15
   Ser Arg Phe Ser Trp
               20
<210> 559
   <211> 21
   <212> PRT
   <213> Homo sapiens
<400> 559
   Arg Ala Glu Ile Glu Asn Leu His Arg Thr Phe Asp Pro His Phe Leu
   1               5                   10                  15
   Arg Val Pro Cys Trp
               20
<210> 560
   <211> 21
   <212> PRT
   <213> Homo sapiens
<400> 560
   Arg Asn Val Arg Phe Ser Asp Glu Gly Gly Phe Thr Cys Phe Phe Arg
   1               5                   10                  15
   Asp His Ser Tyr Gln
               20
<210> 561
   <211> 21
   <212> PRT
   <213> Homo sapiens
<400> 561
   Ser Asp Tyr Lys Ser Ala His Lys Gly Phe Lys Gly Val Asp Ala Gln
   1               5                   10                  15
   Gly Thr Leu Ser Lys
               20
<210> 562
   <211> 21
   <212> PRT
   <213> Homo sapiens
<400> 562
   Ser Gly Lys Val Pro Trp Leu Lys Pro Gly Arg Ser Pro Leu Pro Ser
   1               5                   10                  15
   His Ala Arg Ser Gln
               20
<210> 563
   <211> 11
   <212> PRT
   <213> Homo sapiens
<400> 563
   Ser Ile Gly Arg Phe Phe Gly Gly Asp Arg Gly
   1 5 10
<210> 564
   <211> 21
   <212> PRT
   <213> Homo sapiens
<400> 564
   Ser Leu Cys Ala Asp Ala Arg Met Tyr Gly Val Leu Pro Trp Asn Ala
   1 5 10 15
   Phe Pro Gly Lys Val
   20
<210> 565
   <211> 20
   <212> PRT
   <213> Homo sapiens
<400> 565
   Ser Tyr Gln Glu Glu Ala Ala Met Glu Leu Lys Val Glu Asp Pro Phe
   1 5 10 15
   Tyr Trp Val Ser
   20
<210> 566
   <211> 21
   <212> PRT
   <213> Homo sapiens
<400> 566
   Thr Glu Leu Leu Lys Asp Ala Ile Gly Glu Gly Lys Val Thr Leu Arg
   1 5 10 15
   Ile Arg Asn Val Arg
   20
<210> 567
   <211> 20
   <212> PRT
   <213> Homo sapiens
<400> 567
   Thr Phe Asp Pro His Phe Leu Arg Val Pro Cys Trp Lys Ile Thr Leu
   1 5 10 15
   Phe Val Ile Val
   20
<210> 568
   <211> 21
   <212> PRT
   <213> Homo sapiens
<400> 568
   Thr Pro Arg Thr Pro Pro Pro Ser Gln Gly Lys Gly Ala Glu Gly Gln
   1 5 10 15
   Arg Pro Gly Phe Gly
   20
<210> 569
   <211> 21
   <212> PRT
   <213> Homo sapiens
<400> 569
   Val Ala Leu Ile Ile Cys Tyr Asn Trp Leu His Arg Arg Leu Ala Gly
   1 5 10 15
   Gln Phe Leu Glu Glu
   20
<210> 570
   <211> 21
   <212> PRT
   <213> Homo sapiens
<400> 570
   Val Phe Ala Cys Ser Ala Val Pro Val Tyr Ile Tyr Phe Asn Thr Trp
   1 5 10 15
   Thr Thr Cys Gln Ser
   20
<210> 571
   <211> 21
   <212> PRT
   <213> Homo sapiens
<400> 571
   Val Ile Val Pro Val Leu Gly Pro Leu Val Ala Leu Ile Ile Cys Tyr
   1 5 10 15
   Asn Trp Leu His Arg
   20
<210> 572
   <211> 21
   <212> PRT
   <213> Homo sapiens
<400> 572
   Tyr Phe Ser Lys Asn Tyr Gln Asp Tyr Glu Tyr Leu Ile Asn Val Ile
   1 5 10 15
   His Ala Phe Gln Tyr
   20
<210> 573
   <211> 21
   <212> PRT
   <213> Homo sapiens
<400> 573
   Tyr Ile Tyr Phe Asn Thr Trp Thr Thr Cys Gln Ser Ile Ala Phe Pro
   1 5 10 15
   Ser Lys Thr Ser Ala
   20
<210> 574
   <211> 20
   <212> PRT
   <213> Homo sapiens
<400> 574
   Tyr Asn Trp Leu His Arg Arg Leu Ala Gly Gln Phe Leu Glu Glu Leu
   1 5 10 15
   Arg Asn Pro Phe
   20
<210> 575
   <211> 21
   <212> PRT
   <213> Homo sapiens
<400> 575
   Tyr Arg Asn Gly Lys Asp Gln Asp Gly Asp Gln Ala Pro Glu Tyr Arg
   1 5 10 15
   Gly Arg Thr Glu Leu
   20
<210> 576
   <211> 32
   <212> PRT
   <213> Homo sapiens
<400> 576
   Ala Ala Phe Val Gly Ala Ala Ala Thr Leu Val Ser Leu Leu Thr Phe
   1 5 10 15
   Met Ile Ala Ala Thr Tyr Asn Phe Ala Val Leu Lys Leu Met Gly Arg
   20 25 30
<210> 577
   <211> 14
   <212> PRT
   <213> Mus musculus
<400> 577
   Ala Cys Ser Ala Val Thr Val Tyr Ile Tyr Phe Asn Thr Trp
   1 5 10
<210> 578
   <211> 19
   <212> PRT
   <213> Homo sapiens
<400> 578
   Ala Ser Asp Tyr Lys Ser Ala His Lys Gly Phe Lys Gly Val Asp Ala
   1 5 10 15
   Gln Gly Thr
<210> 579
   <211> 20
   <212> PRT
   <213> Homo sapiens
<400> 579
   Ala Val Arg Gln Ile Phe Gly Asp Tyr Lys Thr Thr Ile Cys Gly Lys
   1 5 10 15
   Gly Leu Ser Ala
   20
<210> 580
   <211> 16
   <212> PRT
   <213> Mus musculus
<400> 580
   Asp Tyr Lys Thr Thr Ile Cys Gly Lys Gly Leu Ser Ala Thr Val Thr
   1               5                   10                  15
<210> 581
   <211> 31
   <212> PRT
   <213> Homo sapiens
<400> 581
   Phe Gly Val Ala Leu Phe Cys Gly Cys Glu Val Glu Ala Leu Thr Gly
   1               5                   10                  15
   Thr Glu Lys Leu Ile Glu Thr Tyr Phe Ser Lys Asn Tyr Gln Asp
               20                  25                  30
<210> 582
   <211> 19
   <212> PRT
   <213> Homo sapiens
<400> 582
   Phe Lys Gly Val Asp Ala Gln Gly Thr Leu Ser Lys Ile Phe Lys Leu
   1               5                   10                  15
   Gly Gly Arg
<210> 583
   <211> 14
   <212> PRT
   <213> Mus musculus
<400> 583
   Phe Ser Lys Asn Tyr Gln Asp Tyr Glu Tyr Leu Ile Asn Val
   1               5                   10
<210> 584
   <211> 19
   <212> PRT
   <213> Homo sapiens
<400> 584
   Gly Leu Arg Leu Ile His Tyr Ser Val Gly Ala Gly Ile Thr Asp Gln
   1 5 10 15
   Gly Glu Val
<210> 585
   <211> 40
   <212> PRT
   <213> Homo sapiens
<400> 585
   Gly Ser Lys Tyr Leu Ala Thr Ala Ser Thr Met Asp His Ala Arg His
   1 5 10 15
   Gly Phe Leu Pro Arg His Arg Asp Thr Gly Ile Leu Asp Ser Ile Gly
   20 25 30
   Arg Phe Phe Gly Gly Asp Arg Gly
   35 40
<210> 586
   <211> 32
   <212> PRT
   <213> Homo sapiens
<400> 586
   Gly Thr Ala Ser Phe Phe Phe Leu Tyr Gly Ala Leu Leu Leu Ala Tyr
   1 5 10 15
   Gly Phe Tyr Thr Thr Gly Ala Val Arg Gln Ile Phe Gly Asp Tyr Lys
   20 25 30
<210> 587
   <211> 19
   <212> PRT
   <213> Homo sapiens
<400> 587
   His His Pro Ala Arg Thr Ala His Tyr Gly Ser Leu Pro Gln Lys Ser
   1 5 10 15
   His Gly Arg
<210> 588
   <211> 29
   <212> PRT
   <213> Homo sapiens
<400> 588
   Ile Gly Arg Phe Phe Gly Gly Asp Arg Gly Ala Pro Lys Arg Gly Ser
   1               5                   10                  15
   Gly Lys Asp Ser His His Pro Ala Arg Thr Ala His Tyr
               20                  25
<210> 589
   <211> 32
   <212> PRT
   <213> Homo sapiens
<400> 589
   Lys Gly Arg Gly Ser Arg Gly Gln His Gln Ala His Ser Leu Glu Arg
   1               5                   10                  15
   Val Cys His Cys Leu Gly Cys Trp Leu Gly His Pro Asp Lys Phe Val
               20                  25                  30
<210> 590
   <211> 32
   <212> PRT
   <213> Homo sapiens
<400> 590
   Lys Val Cys Gly Ser Asn Leu Leu Ser Ile Cys Lys Thr Ala Glu Phe
   1               5                   10                  15
   Gln Met Thr Phe His Leu Phe Ile Ala Ala Phe Val Gly Ala Ala Ala
               20                  25                  30
<210> 591
   <211> 15
   <212> PRT
   <213> Mus musculus
<400> 591
   Leu Phe Ile Ala Ala Phe Val Gly Ala Ala Ala Thr Leu Val Ser
   1               5                   10                  15
<210> 592
   <211> 32
   <212> PRT
   <213> Homo sapiens
<400> 592
   Leu Gly His Pro Asp Lys Phe Val Gly Ile Thr Tyr Ala Leu Thr Val
   1               5                   10                  15
   Val Trp Leu Leu Val Phe Ala Cys Ser Ala Val Pro Val Tyr Ile Tyr
               20                  25                  30
<210> 593
   <211> 31
   <212> PRT
   <213> Homo sapiens
<400> 593
   Leu Leu Glu Cys Cys Ala Arg Cys Leu Val Gly Ala Pro Phe Ala Ser
   1               5                   10                  15
   Leu Val Ala Thr Gly Leu Cys Phe Phe Gly Val Ala Leu Phe Cys
               20                  25                  30
<210> 594
   <211> 35
   <212> PRT
   <213> Mus musculus
<400> 594
   Pro Trp Asn Ala Phe Pro Gly Lys Val Cys Gly Ser Asn Leu Leu Ser
   1               5                   10                  15
   Ile Cys Lys Thr Ala Glu Phe Gln Met Thr Phe His Leu Phe Ile Ala
               20                  25                  30
   Ala Phe Val
           35
<210> 595
   <211> 20
   <212> PRT
   <213> Homo sapiens
<400> 595
   Gln Asp Glu Asn Pro Val Val His Phe Phe Lys Asn Ile Val Thr Pro
   1               5                   10                  15
   Arg Thr Pro Pro
                                   20
<210> 596
   <211> 31
   <212> PRT
   <213> Homo sapiens
<400> 596
   Arg Gln Ile Phe Gly Asp Tyr Lys Thr Thr Cys Gly Lys Gly Leu Ser
   1               5                   10                  15
   Ala Thr Val Thr Gly Gly Gln Lys Gly Arg Gly Ser Arg Gly Gln
               20                  25                  30
<210> 597
   <211> 32
   <212> PRT
   <213> Homo sapiens
<400> 597
   Ser Ala Ser Ile Gly Thr Leu Cys Ala Asp Ala Arg Met Tyr Gly Val
   1               5                   10                  15
   Leu Pro Trp Asn Ala Phe Phe Gly Lys Val Cys Gly Ser Asn Leu Leu
               20                  25                  30
<210> 598
   <211> 32
   <212> PRT
   <213> Homo sapiens
<400> 598
   Ser Ala Val Pro Val Tyr Ile Tyr Phe Asn Thr Trp Thr Thr Cys Gln
   1               5                   10                  15
   Ser Ile Ala Ala Pro Cys Lys Thr Ser Ala Ser Ile Gly Thr Leu Cys
               20                  25                  30
<210> 599
   <211> 15
   <212> PRT
   <213> Mus musculus
<400> 599
   Thr Val Thr Gly Gly Gln Lys Gly Arg Gly Ser Arg Gly Gln His
   1               5                   10                  15
<210> 600
   <211> 32
   <212> PRT
   <213> Homo sapiens
<400> 600
   Tyr Phe Ser Lys Asn Tyr Gln Asp Tyr Glu Tyr Leu Ile Asn Val Ile
   1               5                   10                  15
   His Ala Phe Gln Tyr Val Ile Tyr Gly Thr Ala Ser Phe Phe Phe Leu
               20                  25                  30
<210> 601
   <211> 12
   <212> PRT
   <213> Homo sapiens
<400> 601
   Pro Val Val His Phe Phe Lys Asn Ile Val Thr Pro
   1 5 10
<210> 602
   <211> 9
   <212> PRT
   <213> Homo sapiens
<400> 602
   Lys Val Glu Asp Pro Phe Tyr Trp Val
   1 5
<210> 603
   <211> 11
   <212> PRT
   <213> Homo sapiens
<400> 603
   Pro Thr Arg Ala Pro Ser Gly Pro Arg Pro Pro
   1 5 10
<210> 604
   <211> 11
   <212> PRT
   <213> Homo sapiens
<400> 604
   Arg Arg Arg Glu Gly Pro Asp Arg Ser Pro Arg
   1 5 10
<210> 605
   <211> 15
   <212> PRT
   <213> Human adenovirus
<400> 605
   Asp Phe Glu Val Val Thr Phe Leu Lys Asp Val Leu Pro Glu Phe
   1 5 10 15
<210> 606
   <211> 15
   <212> PRT
   <213> Pseudomonas aeruginosa
<400> 606
   Asp Arg Leu Leu Met Leu Phe Ala Lys Asp Val Val Ser Arg Asn
   1 5 10 15
<210> 607
   <211> 15
   <212> PRT
   <213> Unknown
<220>
   <223> epitope involved in multiple sclerosis
<400> 607
   Glu Asn Pro Val Val His Tyr Phe Arg Asn Ile Val Thr Pro Arg
   1 5 10 15
<210> 608
   <211> 15
   <212> PRT
   <213> Human herpesvirus
<400> 608
   Phe Arg Gln Leu Val His Phe Val Arg Asp Phe Ala Gln Leu Leu
   1 5 10 15
<210> 609
   <211> 13
   <212> PRT
   <213> Herpes simplex virus
<400> 609
   Gly Gly Arg Arg Leu Phe Phe Val Lys Ala His Val Arg
   1 5 10
<210> 610
   <211> 15
   <212> PRT
   <213> Mammalian orthoreovirus
<400> 610
   Met Ala Arg Ala Ala Phe Leu Phe Lys Thr Val Gly Phe Gly Gly
   1 5 10 15
<210> 611
   <211> 9
   <212> PRT
   <213> Homo sapiens
<400> 611
   Lys Leu Ile Glu Thr Tyr Phe Ser Lys
   1 5
<210> 612
   <211> 9
   <212> PRT
   <213> Saccharomyces cerevisiae
<400> 612
   Lys Leu Val Glu Thr Tyr Ile Ser Lys
   1 5
<210> 613
   <211> 21
   <212> PRT
   <213> Homo sapiens
<400> 613
   Ala Ala Gly Arg Gly Leu Gln Leu Leu Phe Tyr Ser Ile Gly Ile Asp
   1 5 10 15
   Gln Ile Ser Ser Glu
   20
<210> 614
   <211> 21
   <212> PRT
   <213> Homo sapiens
<400> 614
   Ala Leu Gly Gln Gly Pro Glu Phe Leu Thr Tyr Phe Asn Tyr Glu Ala
   1 5 10 15
   Gln Pro Asp Lys Ser
   20
<210> 615
   <211> 21
   <212> PRT
   <213> Homo sapiens
<400> 615
   Ala Leu Gly Gln Gly Pro Glu Phe Leu Thr Tyr Phe Asn Tyr Glu Ala
   1 5 10 15
   Gln Gln Asp Lys Ser
   20
<210> 616
   <211> 21
   <212> PRT
   <213> Homo sapiens
<400> 616
   Ala Leu Gly Gln Gly Pro Glu Phe Leu Thr Tyr Phe Gln Asn Glu Ala
   1 5 10 15
   Gln Leu Asp Lys Ser
   20
<210> 617
   <211> 21
   <212> PRT
   <213> Homo sapiens
<400> 617
   Asp Leu Gly His Gly Leu Arg Leu Ile His Tyr Ser Tyr Gly Val Gln
   1 5 10 15
   Asp Thr Asn Lys Gly
   20
<210> 618
   <211> 21
   <212> PRT
   <213> Homo sapiens
<400> 618
   Asp Leu Gly Leu Gly Leu Arg Leu Ile His Tyr Ser Asn Thr Ala Gly
   1 5 10 15
   Thr Thr Gly Lys Gly
   20
<210> 619
   <211> 21
   <212> PRT
   <213> Homo sapiens
<400> 619
   Asp Pro Gly Glu Gly Pro Val Leu Leu Ile Ala Leu Tyr Lys Ala Gly
   1 5 10 15
   Glu Leu Thr Ser Asn
   20
<210> 620
   <211> 21
   <212> PRT
   <213> Homo sapiens
<400> 620
   Asp Pro Gly Lys Gly Pro Glu Phe Leu Phe Thr Leu Tyr Ser Ala Gly
   1 5 10 15
   Glu Glu Lys Glu Lys
   20
<210> 621
   <211> 21
   <212> PRT
   <213> Homo sapiens
<400> 621
   Asp Pro Gly Lys Ser Leu Glu Ser Leu Phe Val Leu Leu Ser Asn Gly
   1 5 10 15
   Ala Val Lys Gln Glu
   20
<210> 622
   <211> 21
   <212> PRT
   <213> Homo sapiens
<400> 622
   Asp Pro Gly Met Glu Leu His Leu Ile His Tyr Ser Tyr Gly Val Asn
   1 5 10 15
   Ser Thr Glu Lys Gly
   20
<210> 623
   <211> 21
   <212> PRT
   <213> Homo sapiens
<400> 623
   Asp Pro Gly Met Gly Leu Lys Leu Ile Tyr Tyr Ser Val Gly Ala Gly
   1 5 10 15
   Ile Thr Asp Lys Gly
   20
<210> 624
   <211> 21
   <212> PRT
   <213> Homo sapiens
<400> 624
   Asp Pro Gly Met Gly Leu Arg Leu Ile His Tyr Ser Val Gly Ala Gly
   1               5                   10                  15
   Ile Thr Asp Gln Gly
               20
<210> 625
   <211> 21
   <212> PRT
   <213> Homo sapiens
<400> 625
   Asp Pro Gly Met Gly Leu Arg Leu Ile His Tyr Ser Val Gly Glu Gly
   1               5                   10                  15
   Thr Thr Ala Lys Gly
               20
<210> 626
   <211> 21
   <212> PRT
   <213> Homo sapiens
<400> 626
   Asp Pro Gly Met Gly Leu Arg Arg Ile His Tyr Ser Val Ala Ala Gly
   1               5                   10                  15
   Ile Thr Asp Lys Gly
               20
<210> 627
   <211> 21
   <212> PRT
   <213> Homo sapiens
<400> 627
   Asp Pro Gly Arg Gly Pro Val Phe Leu Leu Leu Ile Arg Glu Asn Glu
   1               5                   10                  15
   Lys Glu Lys Arg Lys
               20
<210> 628
   <211> 21
   <212> PRT
   <213> Homo sapiens
<400> 628
   Asp Ser Lys Lys Phe Leu Lys Ile Met Phe Ser Tyr Asn Asn Lys Glu
   1 5 10 15
   Leu Ile Ile Asn Glu
   20
<210> 629
   <211> 21
   <212> PRT
   <213> Homo sapiens
<400> 629
   Asp Thr Gly Arg Gly Pro Val Ser Leu Thr Ile Met Thr Phe Ser Glu
   1 5 10 15
   Asn Thr Lys Ser Asn
   20
<210> 630
   <211> 20
   <212> PRT
   <213> Homo sapiens
<400> 630
   Glu Lys Lys Ala Pro Thr Phe Leu Phe Met Leu Thr Ser Ser Gly Ile
   1 5 10 15
   Glu Lys Lys Ser
   20
<210> 631
   <211> 21
   <212> PRT
   <213> Homo sapiens
<400> 631
   Glu Leu Gly Lys Gly Pro Gln Leu Ile Ile Asp Ile Arg Ser Asn Val
   1 5 10 15
   Gly Glu Lys Lys Asp
   20
<210> 632
   <211> 21
   <212> PRT
   <213> Homo sapiens
<400> 632
   Glu Leu Gly Lys Arg Pro Gln Leu Ile Ile Asp Ile Arg Ser Asn Val
   1 5 10 15
   Gly Glu Lys Lys Asp
   20
<210> 633
   <211> 21
   <212> PRT
   <213> Homo sapiens
<400> 633
   Glu Pro Gly Ala Gly Leu Gln Leu Leu Thr Tyr Ile Phe Ser Asn Met
   1 5 10 15
   Asp Met Lys Gln Asp
   20
<210> 634
   <211> 21
   <212> PRT
   <213> Homo sapiens
<400> 634
   Glu Pro Gly Glu Gly Pro Val Leu Leu Val Thr Val Val Thr Gly Gly
   1 5 10 15
   Glu Val Lys Lys Leu
   20
<210> 635
   <211> 21
   <212> PRT
   <213> Homo sapiens
<400> 635
   Glu Ser Gly Lys Gly Pro Gln Phe Ile Ile Asp Ile Arg Ser Asn Met
   1 5 10 15
   Asp Lys Arg Gln Gly
   20
<210> 636
   <211> 21
   <212> PRT
   <213> Homo sapiens
<400> 636
   Glu Thr Ala Lys Thr Pro Glu Ala Leu Phe Val Met Thr Leu Asn Gly
   1 5 10 15
   Asp Glu Lys Lys Lys
   20
<210> 637
   <211> 18
   <212> PRT
   <213> Homo sapiens
<400> 637
   Phe Pro Gly Cys Ala Pro Arg Leu Leu Val Lys Gly Ser Lys Pro Ser
   1               5                   10                  15
   Gln Gln
<210> 638
   <211> 21
   <212> PRT
   <213> Homo sapiens
<400> 638
   Phe Pro Gly Lys Gly Pro Ala Leu Leu Ile Ala Ile Arg Pro Asp Val
   1               5                   10                  15
   Ser Glu Lys Lys Glu
               20
<210> 639
   <211> 22
   <212> PRT
   <213> Homo sapiens
<400> 639
   Phe Pro Lys Lys Ser Leu Met Leu Met Ala Thr Ser Asn Glu Gly Ser
   1               5                   10                  15
   Lys Ala Thr Tyr Glu Gln
               20
<210> 640
   <211> 22
   <212> PRT
   <213> Homo sapiens
<400> 640
   Phe Pro Lys Lys Ser Leu Met Gln Ile Ala Thr Ser Asn Glu Gly Ser
   1               5                   10                  15
   Lys Ala Thr Tyr Glu Gln
               20
<210> 641
   <211> 22
   <212> PRT
   <213> Homo sapiens
<400> 641
   Phe Pro Lys Gln Ser Leu Met Leu Met Ala Thr Ser Asn Glu Gly Ser
   1               5                   10                  15
   Lys Ala Thr Tyr Glu Gln
               20
<210> 642
   <211> 19
   <212> PRT
   <213> Homo sapiens
<400> 642
   Phe Pro Ser Gln Gly Pro Arg Phe Ile Ile Gln Gly Tyr Lys Thr Lys
   1               5                   10                  15
   Val Thr Asn
<210> 643
   <211> 21
   <212> PRT
   <213> Homo sapiens
<400> 643
   Gly Pro Gly Gln Asp Pro Gln Phe Leu Ile Ser Phe Tyr Glu Lys Met
   1               5                   10                  15
   Gln Ser Asp Lys Gly
               20
<210> 644
   <211> 20
   <212> PRT
   <213> Homo sapiens
<400> 644
   His Ala Gly Glu Ala Pro Thr Phe Leu Ser Tyr Asn Val Leu Asp Gly
   1               5                   10                  15
   Leu Glu Glu Lys
               20
<210> 645
   <211> 20
   <212> PRT
   <213> Homo sapiens
<400> 645
   His Asp Gly Gly Ala Pro Thr Phe Leu Ser Tyr Asn Gly Leu Asp Gly
   1 5 10 15
   Leu Glu Glu Thr
   20
<210> 646
   <211> 19
   <212> PRT
   <213> Homo sapiens
<400> 646
   His Pro Ala Gly Ala Ile Val Ser Leu Phe Met Leu Ser Ser Gln Lys
   1 5 10 15
   Lys Lys His
<210> 647
   <211> 22
   <212> PRT
   <213> Homo sapiens
<400> 647
   His Pro Asn Lys Gly Leu Gln Leu Leu Leu Lys Tyr Thr Ser Ala Ala
   1 5 10 15
   Thr Leu Val Lys Gly Ile
   20
<210> 648
   <211> 19
   <212> PRT
   <213> Homo sapiens
<400> 648
   Ile His Ser Gln Gly Pro Gln Tyr Ile Ile His Gly Leu Lys Asn Asn
   1 5 10 15
   Glu Thr Asn
<210> 649
   <211> 21
   <212> PRT
   <213> Homo sapiens
<400> 649
   Ile Leu Gly Gln Lys Val Glu Phe Leu Val Ser Phe Tyr Asn Asn Glu
   1 5 10 15
   Ile Ser Glu Lys Ser
   20
<210> 650
   <211> 21
   <212> PRT
   <213> Homo sapiens
<400> 650
   Lys Ala Lys Lys Pro Pro Glu Leu Met Phe Val Tyr Ser Tyr Glu Lys
   1 5 10 15
   Leu Ser Ile Asn Glu
   20
<210> 651
   <211> 21
   <212> PRT
   <213> Homo sapiens
<400> 651
   Lys His Gly Glu Ala Pro Val Phe Leu Met Ile Leu Leu Lys Gly Gly
   1 5 10 15
   Glu Gln Lys Gly His
   20
<210> 652
   <211> 21
   <212> PRT
   <213> Homo sapiens
<400> 652
   Lys His Gly Glu Ala Pro Val Phe Leu Met Ile Leu Leu Lys Gly Gly
   1 5 10 15
   Glu Gln Met Arg Arg
   20
<210> 653
   <211> 21
   <212> PRT
   <213> Homo sapiens
<400> 653
   Lys Leu Glu Glu Glu Leu Lys Phe Leu Val Tyr Phe Gln Asn Glu Glu
   1 5 10 15
   Leu Ile Gln Lys Ala
   20
<210> 654
   <211> 21
   <212> PRT
   <213> Homo sapiens
<400> 654
   Lys Ser Ser Gln Ala Pro Lys Leu Leu Phe His Tyr Tyr Asp Lys Asp
   1 5 10 15
   Phe Asn Asn Glu Ala
   20
<210> 655
   <211> 21
   <212> PRT
   <213> Homo sapiens
<400> 655
   Lys Ser Ser Gln Ala Pro Lys Leu Leu Phe His Tyr Tyr Asn Lys Asp
   1 5 10 15
   Phe Asn Asn Glu Ala
   20
<210> 656
   <211> 21
   <212> PRT
   <213> Homo sapiens
<400> 656
   Lys Tyr Gly Glu Gly Leu Ile Phe Leu Met Met Leu Gln Lys Gly Gly
   1 5 10 15
   Glu Glu Lys Ser His
   20
<210> 657
   <211> 19
   <212> PRT
   <213> Homo sapiens
<400> 657
   Leu Pro Ser Gln Gly Pro Glu Tyr Val Ile His Gly Leu Thr Ser Asn
   1 5 10 15
   Val Asn Asn
<210> 658
   <211> 19
   <212> PRT
   <213> Homo sapiens
<400> 658
   Asn Pro Trp Gly Gln Leu Ile Asn Leu Phe Tyr Ile Pro Ser Gly Thr
   1               5                   10                  15
   Lys Gln Asn
<210> 659
   <211> 21
   <212> PRT
   <213> Homo sapiens
<400> 659
   Asn Gln Asn Lys Glu Phe Met Leu Leu Ile Ser Phe Gln Asn Glu Gln
   1               5                   10                  15
   Val Leu Gln Glu Thr
               20
<210> 660
   <211> 21
   <212> PRT
   <213> Homo sapiens
<400> 660
   Asn Ser Gly Arg Gly Leu Val His Leu Ile Leu Ile Arg Ser Asn Glu
   1               5                   10                  15
   Arg Glu Lys His Ser
               20
<210> 661
   <211> 22
   <212> PRT
   <213> Homo sapiens
<400> 661
   Pro Pro Ser Gly Glu Leu Val Phe Leu Ile Arg Arg Asn Ser Phe Asp
   1               5                   10                  15
   Glu Gln Asn Glu Ile Ser
               20
<210> 662
   <211> 22
   <212> PRT
   <213> Homo sapiens
<400> 662
   Pro Pro Ser Arg Gln Met Ile Leu Val Ile Arg Gln Glu Ala Tyr Lys
   1               5                   10                  15
   Gln Gln Asn Ala Thr Glu
               20
<210> 663
   <211> 22
   <212> PRT
   <213> Homo sapiens
<400> 663
   Pro Ser Ser Gly Glu Met Ile Phe Leu Ile Tyr Gln Gly Ser Tyr Asp
   1               5                   10                  15
   Glu Gln Asn Ala Thr Glu
               20
<210> 664
   <211> 21
   <212> PRT
   <213> Homo sapiens
<400> 664
   Arg Pro Gly Gly His Pro Val Phe Leu Ile Gln Leu Val Lys Ser Gly
   1               5                   10                  15
   Glu Val Lys Lys Gln
               20
<210> 665
   <211> 21
   <212> PRT
   <213> Homo sapiens
<400> 665
   Ser Ala Lys Lys Pro Leu Glu Leu Met Phe Val Tyr Asn Phe Lys Glu
   1               5                   10                  15
   Gln Thr Glu Asn Asn
               20
<210> 666
   <211> 21
   <212> PRT
   <213> Homo sapiens
<400> 666
   Ser Ala Lys Lys Pro Leu Glu Leu Met Phe Val Tyr Ser Leu Glu Glu
   1               5                   10                  15
   Arg Val Glu Asn Asn
               20
<210> 667
   <211> 21
   <212> PRT
   <213> Homo sapiens
<400> 667
   Ser Leu Asp Gln Gly Leu Gln Phe Leu Ile His Tyr Tyr Asn Gly Glu
   1               5                   10                  15
   Glu Arg Ala Lys Gly
               20
<210> 668
   <211> 21
   <212> PRT
   <213> Homo sapiens
<400> 668
   Ser Leu Asp Gln Gly Leu Gln Phe Leu Ile Gln Tyr Tyr Asn Gly Glu
   1               5                   10                  15
   Glu Arg Ala Lys Gly
               20
<210> 669
   <211> 21
   <212> PRT
   <213> Homo sapiens
<400> 669
   Thr Leu Glu Glu Glu Leu Lys Phe Phe Ile Tyr Phe Gln Asn Glu Glu
   1               5                   10                  15
   Ile Ile Gln Lys Ala
               20
<210> 670
   <211> 21
   <212> PRT
   <213> Homo sapiens
<400> 670
   Thr Leu Gly Gln Gly Leu Gln Phe Leu Phe Glu Tyr Phe Ser Glu Thr
   1               5                   10                  15
   Gln Arg Asn Lys Gly
               20
<210> 671
   <211> 21
   <212> PRT
   <213> Homo sapiens
<400> 671
   Thr Leu Gly Gln Gly Pro Glu Phe Leu Thr Tyr Phe Gln Asn Glu Ala
   1               5                   10                  15
   Gln Leu Glu Lys Ser
               20
<210> 672
   <211> 16
   <212> PRT
   <213> Homo sapiens
<400> 672
   Val Phe Leu Cys Leu Gln Tyr Arg Leu Arg Gly Lys Leu Arg Ala Glu
   1               5                   10                  15
<210> 673
   <211> 21
   <212> PRT
   <213> Homo sapiens
<400> 673
   Val Leu Gly Gln Gly Pro Gln Phe Ile Phe Gln Tyr Tyr Glu Lys Glu
   1               5                   10                  15
   Glu Arg Gly Arg Gly
               20
<210> 674
   <211> 21
   <212> PRT
   <213> Homo sapiens
<400> 674
   Val Leu Lys Asn Glu Phe Lys Phe Leu Val Ser Phe Gln Asn Glu Asn
   1               5                   10                  15
   Val Phe Asp Glu Thr
               20
<210> 675
   <211> 21
   <212> PRT
   <213> Homo sapiens
<400> 675
   Val Met Gly Lys Glu Ile Lys Phe Leu Leu His Phe Val Lys Glu Ser
   1               5                   10                  15
   Lys Gln Asp Glu Ser
               20
<210> 676
   <211> 21
   <212> PRT
   <213> Homo sapiens
<400> 676
   Tyr Pro Ala Glu Gly Pro Thr Phe Leu Ile Ser Ile Ser Ser Ile Lys
   1               5                   10                  15
   Asp Lys Asn Glu Asp
               20
<210> 677
   <211> 21
   <212> PRT
   <213> Homo sapiens
<400> 677
   Tyr Pro Gly Glu Gly Leu Gln Leu Leu Leu Lys Ala Thr Lys Ala Asp
   1               5                   10                  15
   Asp Lys Gly Ser Asn
               20
<210> 678
   <211> 22
   <212> PRT
   <213> Homo sapiens
<400> 678
   Tyr Pro Asn Gln Gly Leu Gln Leu Leu Leu Lys Tyr Leu Ser Gly Ser
   1               5                   10                  15
   Thr Leu Val Glu Ser Ile
               20
<210> 679
   <211> 22
   <212> PRT
   <213> Homo sapiens
<400> 679
   Tyr Pro Asn Gln Gly Leu Gln Leu Leu Leu Lys Tyr Leu Ser Gly Ser
   1               5                   10                  15
   Thr Leu Val Lys Gly Ile
               20
<210> 680
   <211> 22
   <212> PRT
   <213> Homo sapiens
<400> 680
   Tyr Pro Asn Gln Gly Leu Gln Leu Leu Leu Lys Tyr Thr Ser Ala Ala
   1               5                   10                  15
   Thr Leu Val Lys Gly Ile
               20
<210> 681
   <211> 22
   <212> PRT
   <213> Homo sapiens
<400> 681
   Tyr Pro Asn Gln Gly Leu Gln Leu Leu Leu Lys Tyr Thr Ser Gly Ala
   1               5                   10                  15
   Thr Leu Val Lys Gly Ile
               20
<210> 682
   <211> 22
   <212> PRT
   <213> Homo sapiens
<400> 682
   Tyr Pro Asn Gln Gly Leu Gln Leu Leu Leu Lys Tyr Thr Thr Gly Ala
   1               5                   10                  15
   Thr Leu Val Lys Gly Ile
               20
<210> 683
   <211> 22
   <212> PRT
   <213> Homo sapiens
<400> 683
   Tyr Pro Asn Arg Gly Leu Gln Phe Leu Leu Lys Tyr Ile Thr Gly Asp
   1               5                   10                  15
   Asn Leu Val Lys Gly Ser
               20
<210> 684
   <211> 17
   <212> PRT
   <213> Homo sapiens
<400> 684
   Tyr Pro Ser Lys Pro Leu Gln Leu Leu Gln Arg Glu Thr Met Glu Asn
   1               5                   10                  15
   Ser
<210> 685
   <211> 20
   <212> PRT
   <213> Homo sapiens
<400> 685
   Tyr Ser Gly Lys Ser Pro Glu Leu Ile Met Phe Ile Tyr Ser Asn Gly
   1               5                   10                  15
   Asp Lys Glu Asp
               20
<210> 686
   <211> 18
   <212> PRT
   <213> Homo sapiens
<400> 686
   Tyr Ser Arg Gln Arg Leu Gln Leu Leu Leu Arg His Ile Ser Arg Glu
   1               5                   10                  15
   Ser Ile
<210> 687
   <211> 8
   <212> PRT
   <213> Homo sapiens
<400> 687
   Ala Asp Ala Arg Met Tyr Gly Val
   1 5
<210> 688
   <211> 20
   <212> PRT
   <213> Human herpesvirus
<400> 688
   Asp Pro Gly Glu Gly Pro Ser Thr Gly Pro Arg Gly Gln Gly Asp Gly
   1 5 10 15
   Gly Arg Arg Lys
   20
<210> 689
   <211> 20
   <212> PRT
   <213> Human herpesvirus
<400> 689
   Glu Ala Asp Tyr Phe Glu Tyr His Gln Glu Gly Gly Pro Asp Gly Glu
   1 5 10 15
   Pro Asp Val Pro
   20
<210> 690
   <211> 8
   <212> PRT
   <213> Homo sapiens
<400> 690
   Glu Ala Leu Thr Gly Thr Glu Lys
   1 5
<210> 691
   <211> 20
   <212> PRT
   <213> Human herpesvirus
<400> 691
   Gly Cys Lys Gly Thr His Gly Gly Thr Gly Ala Gly Ala Gly Ala Gly
   1 5 10 15
   Gly Ala Gly Ala
   20
<210> 692
   <211> 31
   <212> PRT
   <213> Human herpesvirus
<400> 692
   Gly Glu Ala Asp Tyr Phe Glu Tyr His Gln Glu Gly Gly Pro Asp Gly
   1               5                   10                  15
   Glu Pro Asp Val Pro Pro Gly Ala Ile Glu Gln Gly Pro Ala Asp
               20                  25                  30
<210> 693
   <211> 20
   <212> PRT
   <213> Human herpesvirus
<400> 693
   Gly Gly Pro Asp Gly Glu Pro Asp Val Pro Pro Gly Ala Ile Glu Gln
   1               5                   10                  15
   Gly Pro Ala Asp
               20
<210> 694
   <211> 29
   <212> PRT
   <213> Human herpesvirus
<400> 694
   Gly Gly Pro Asp Gly Glu Pro Asp Val Pro Pro Gly Ala Ile Glu Gln
   1               5                   10                  15
   Gly Pro Ala Asp Asp Pro Gly Glu Gly Pro Ser Thr Gly
               20                  25
<210> 695
   <211> 20
   <212> PRT
   <213> Human herpesvirus
<400> 695
   Gly Gly Arg Pro Gly Ala Pro Gly Gly Ser Gly Ser Gly Pro Arg His
   1               5                   10                  15
   Arg Asp Gly Val
               20
<210> 696
   <211> 20
   <212> PRT
   <213> Human herpesvirus
<400> 696
   Gly Asn Gly Leu Gly Glu Lys Gly Asp Thr Ser Gly Pro Glu Gly Ser
   1               5                   10                  15
   Gly Gly Ser Gly
               20
<210> 697
   <211> 20
   <212> PRT
   <213> Human herpesvirus
<400> 697
   Gly Arg Arg Pro Phe Phe His Pro Val Gly Glu Ala Asp Tyr Phe Glu
   1               5                   10                  15
   Tyr His Gln Glu
               20
<210> 698
   <211> 20
   <212> PRT
   <213> Human herpesvirus
<400> 698
   Gly Ser Gly Pro Arg His Arg Asp Gly Val Arg Arg Pro Gln Lys Arg
   1               5                   10                  15
   Pro Ser Cys Ile
               20
<210> 699
   <211> 30
   <212> PRT
   <213> Human herpesvirus
<400> 699
   Gly Ser Gly Pro Arg His Arg Asp Gly Val Arg Arg Pro Gln Lys Arg
   1               5                   10                  15
   Pro Ser Cys Ile Gly Cys Lys Gly Thr His Gly Gly Thr Gly
               20                  25                  30
<210> 700
   <211> 9
   <212> PRT
   <213> Homo sapiens
<400> 700
   His Ala Arg His Gly Phe Leu Pro Arg
   1 5
<210> 701
   <211> 9
   <212> PRT
   <213> Homo sapiens
<400> 701
   Ile Ile Cys Tyr Asn Trp Leu His Arg
   1 5
<210> 702
   <211> 9
   <212> PRT
   <213> Homo sapiens
<400> 702
   Lys Leu Arg Ala Glu Ile Glu Asn Leu
   1 5
<210> 703
   <211> 9
   <212> PRT
   <213> Homo sapiens
<400> 703
   Lys Gln Arg Ile Lys Ser Gln Glu Arg
   1 5
<210> 704
   <211> 10
   <212> PRT
   <213> Homo sapiens
<400> 704
   Lys Arg Pro Ser Gln Arg His Gly Ser Lys
   1 5 10
<210> 705
   <211> 10
   <212> PRT
   <213> Homo sapiens
<400> 705
   Leu Leu Ala Val Leu Pro Val Leu Leu Leu
   1 5 10
<210> 706
   <211> 9
   <212> PRT
   <213> Homo sapiens
<400> 706
   Leu Leu Glu Cys Cys Ala Arg Cys Leu
   1 5
<210> 707
   <211> 9
   <212> PRT
   <213> Homo sapiens
<400> 707
   Leu Gln Tyr Arg Leu Arg Gly Lys Leu
   1 5
<210> 708
   <211> 8
   <212> PRT
   <213> Homo sapiens
<400> 708
   Leu Arg Ile Arg Asn Val Arg Phe
   1 5
<210> 709
   <211> 9
   <212> PRT
   <213> Homo sapiens
<400> 709
   Leu Arg Lys Phe Ser Ser Leu Cys Tyr
   1 5
<210> 710
   <211> 20
   <212> PRT
   <213> Human herpesvirus
<400> 710
   Met Ser Asp Glu Gly Pro Gly Thr Gly Pro Gly Asn Gly Leu Gly Glu
   1 5 10 15
   Lys Gly Asp Thr
   20
<210> 711
   <211> 20
   <212> PRT
   <213> Human herpesvirus
<400> 711
   Pro Gly Ala Ile Glu Gln Gly Pro Ala Asp Asp Pro Gly Glu Gly Pro
   1 5 10 15
   Ser Thr Gly Pro
   20
<210> 712
   <211> 7
   <212> PRT
   <213> Human herpesvirus
<400> 712
   Pro Pro Pro Gly Arg Arg Pro
   1 5
<210> 713
   <211> 20
   <212> PRT
   <213> Human herpesvirus
<400> 713
   Pro Gln Arg Arg Gly Gly Asp Asn His Gly Arg Gly Arg Gly Arg Gly
   1 5 10 15
   Arg Gly Arg Gly
   20
<210> 714
   <211> 20
   <212> PRT
   <213> Human herpesvirus
<400> 714
   Arg Gly Arg Gly Arg Gly Arg Gly Arg Gly Gly Gly Arg Pro Gly Ala
   1 5 10 15
   Pro Gly Gly Ser
   20
<210> 715
   <211> 20
   <212> PRT
   <213> Human herpesvirus
<400> 715
   Arg Arg Pro Gln Lys Arg Pro Ser Cys Ile Gly Cys Lys Gly Thr His
   1 5 10 15
   Gly Gly Thr Gly
   20
<210> 716
   <211> 9
   <212> PRT
   <213> Homo sapiens
<400> 716
   Arg Thr Glu Leu Leu Lys Asp Ala Ile
   1 5
<210> 717
   <211> 10
   <212> PRT
   <213> Homo sapiens
<400> 717
   Arg Val Val His Leu Tyr Arg Asn Gly Lys
   1 5 10
<210> 718
   <211> 20
   <212> PRT
   <213> Human herpesvirus
<400> 718
   Ser Gly Pro Glu Gly Ser Gly Gly Ser Gly Pro Gln Arg Arg Gly Gly
   1 5 10 15
   Asp Asn His Gly
   20
<210> 719
   <211> 20
   <212> PRT
   <213> Human herpesvirus
<400> 719
   Ser Gly Ser Pro Pro Arg Arg Pro Pro Pro Gly Arg Arg Pro Phe Phe
   1 5 10 15
   His Pro Val Gly
   20
<210> 720
   <211> 9
   <212> PRT
   <213> Homo sapiens
<400> 720
   Ser Leu Asp Val Met Ala Ser Gln Lys
   1 5
<210> 721
   <211> 36
   <212> PRT
   <213> Human herpesvirus
<400> 721
   Ser Ser Gln Ser Ser Ser Ser Gly Ser Pro Pro Arg Arg Pro Pro Pro
   1               5                   10                  15
   Gly Arg Arg Pro Phe Phe His Pro Val Gly Glu Ala Asp Tyr Phe Glu
               20                  25                  30
   Tyr His Gln Glu
           35
<210> 722
   <211> 9
   <212> PRT
   <213> Homo sapiens
<400> 722
   Thr Ala His Tyr Gly Ser Leu Pro Gln
   1               5
<210> 723
   <211> 20
   <212> PRT
   <213> Homo sapiens
<400> 723
   Leu Ser Leu Ser Arg Phe Ser Trp Gly Ala Glu Gly Gln Arg Pro Gly
   1               5                   10                  15
   Phe Gly Tyr Gly
               20
<210> 724
   <211> 19
   <212> PRT
   <213> Homo sapiens
<400> 724
   Ala Ala His Leu Pro Ala Glu Phe Thr Pro Ala Val His Ala Ser Leu
   1               5                   10                  15
   Asp Lys Phe
<210> 725
   <211> 15
   <212> PRT
   <213> Homo sapiens
<400> 725
   Ala Ala Val Gly Thr Ser Ala Ala Pro Val Pro Ser Asp Asn His
   1 5 10 15
<210> 726
   <211> 13
   <212> PRT
   <213> Homo sapiens
<400> 726
   Ala Glu Pro Pro Pro Pro Pro Pro Glu Pro Ala Arg Ile
   1 5 10
<210> 727
   <211> 12
   <212> PRT
   <213> Homo sapiens
<400> 727
   Ala Gly Glu Tyr Asp His Leu Pro Glu Gln Ala Phe
   1 5 10
<210> 728
   <211> 11
   <212> PRT
   <213> Homo sapiens
<400> 728
   Ala Leu Met Glu Gly Gly Glu Asn Gly Pro Trp
   1 5 10
<210> 729
   <211> 11
   <212> PRT
   <213> Homo sapiens
<400> 729
   Ala Leu Met Leu Ala Ile Ser His Gly Arg Gln
   1 5 10
<210> 730
   <211> 9
   <212> PRT
   <213> Homo sapiens
<400> 730
   Ala Leu Tyr Ser Gly Val His Lys Lys
   1 5
<210> 731
   <211> 15
   <212> PRT
   <213> Homo sapiens
<400> 731
   Ala Gln Val Ile Ile Leu Asn His Pro Gly Gln Ile Ser Ala Gly
   1 5 10 15
<210> 732
   <211> 12
   <212> PRT
   <213> Homo sapiens
<400> 732
   Ala Arg Lys Leu Ile Gly Asp Pro Asn Leu Glu Phe
   1 5 10
<210> 733
   <211> 10
   <212> PRT
   <213> Homo sapiens
<400> 733
   Ala Ser Ser Ser Val Leu Ser Ser Pro Ala
   1 5 10
<210> 734
   <211> 13
   <212> PRT
   <213> Homo sapiens
<400> 734
   Ala Ser Val Asp Leu Lys Asn Thr Gly Arg Glu Glu Phe
   1 5 10
<210> 735
   <211> 14
   <212> PRT
   <213> Homo sapiens
<400> 735
   Ala Ser Val Asp Leu Lys Asn Thr Gly Arg Glu Glu Phe Leu
   1 5 10
<210> 736
   <211> 16
   <212> PRT
   <213> Homo sapiens
<400> 736
   Ala Ser Val Asp Leu Lys Asn Thr Gly Arg Glu Glu Phe Leu Thr Ala
   1 5 10 15
<210> 737
   <211> 10
   <212> PRT
   <213> Homo sapiens
<400> 737
   Ala Thr Ala Leu Pro Thr Gly Thr Ser Lys
   1 5 10
<210> 738
   <211> 9
   <212> PRT
   <213> Homo sapiens
<400> 738
   Ala Thr Ala Asn Gln Ile Ile Leu Lys
   1 5
<210> 739
   <211> 9
   <212> PRT
   <213> Homo sapiens
<400> 739
   Ala Thr Ile Ile Asp Ile Leu Thr Lys
   1 5
<210> 740
   <211> 9
   <212> PRT
   <213> Homo sapiens
<400> 740
   Ala Thr Val Ala Gly Val His Val Lys
   1 5
<210> 741
   <211> 12
   <212> PRT
   <213> Homo sapiens
<400> 741
   Ala Thr Val Arg Gln Lys Leu Gln Asp Glu Thr Asn
   1 5 10
<210> 742
   <211> 9
   <212> PRT
   <213> Homo sapiens
<400> 742
   Ala Thr Val Thr Pro Ser Pro Val Lys
   1 5
<210> 743
   <211> 9
   <212> PRT
   <213> Homo sapiens
<400> 743
   Ala Val Ala Leu Pro Leu Gln Thr Lys
   1 5
<210> 744
   <211> 12
   <212> PRT
   <213> Homo sapiens
<400> 744
   Ala Val Ile Leu Pro Pro Leu Ser Pro Tyr Phe Lys
   1 5 10
<210> 745
   <211> 9
   <212> PRT
   <213> Homo sapiens
<400> 745
   Ala Val Gln Arg Thr Leu Leu Glu Lys
   1 5
<210> 746
   <211> 9
   <212> PRT
   <213> Homo sapiens
<400> 746
   Ala Val Thr Asp Val Ile Ile Ala Lys
   1 5
<210> 747
   <211> 17
   <212> PRT
   <213> Homo sapiens
<400> 747
   Ala Val Val Asp Lys Ala Ala Glu Ala Gly Gly Ala Glu Glu Gln Tyr
   1 5 10 15
   Gly
<210> 748
   <211> 9
   <212> PRT
   <213> Homo sapiens
<400> 748
   Ala Val Tyr Ser His Val Ile Gln Lys
   1 5
<210> 749
   <211> 12
   <212> PRT
   <213> Homo sapiens
<400> 749
   Ala Trp Lys Arg Ala Glu Ala Ile Glu Ser Ser Leu
   1 5 10
<210> 750
   <211> 10
   <212> PRT
   <213> Homo sapiens
<400> 750
   Cys Pro Leu Ser Leu Ala Ala Gln Leu Asp
   1 5 10
<210> 751
   <211> 10
   <212> PRT
   <213> Homo sapiens
<400> 751
   Asp Glu Ser Gly Pro Ser Ile Val His Arg
   1 5 10
<210> 752
   <211> 13
   <212> PRT
   <213> Homo sapiens
<400> 752
   Asp Glu Trp Val Lys Pro Lys Asp Met Leu Gly Pro Lys
   1 5 10
<210> 753
   <211> 18
   <212> PRT
   <213> Homo sapiens
<400> 753
   Asp Phe Ala Ile Val Lys Phe Thr Lys Val Leu Leu Gln Tyr Thr Gly
   1 5 10 15
   His Ile
<210> 754
   <211> 14
   <212> PRT
   <213> Homo sapiens
<400> 754
   Asp Gly Val Ile Lys Val Phe Asn Asp Met Lys Val Arg Lys
   1 5 10
<210> 755
   <211> 13
   <212> PRT
   <213> Homo sapiens
<400> 755
   Asp Leu Pro Ser Glu Ala Ser Ala Ser Thr Ala Gly Pro
   1 5 10
<210> 756
   <211> 10
   <212> PRT
   <213> Homo sapiens
<400> 756
   Asp Leu Pro Trp Phe Arg Thr Leu Ser Trp
   1 5 10
<210> 757
   <211> 13
   <212> PRT
   <213> Homo sapiens
<400> 757
   Asp Leu Ser Gln Val Ala Ala Ile Ser Asn Asp Leu Lys
   1 5 10
<210> 758
   <211> 14
   <212> PRT
   <213> Homo sapiens
<400> 758
   Asp Pro Pro Leu Ile Ala Leu Asp Lys Asp Ala Pro Leu Arg
   1 5 10
<210> 759
   <211> 9
   <212> PRT
   <213> Homo sapiens
<400> 759
   Asp Thr Asp His Tyr Phe Leu Arg Tyr
   1 5
<210> 760
   <211> 11
   <212> PRT
   <213> Homo sapiens
<400> 760
   Asp Thr Gly Ile Leu Asp Ser Ile Gly Arg Phe
   1 5 10
<210> 761
   <211> 14
   <212> PRT
   <213> Homo sapiens
<400> 761
   Asp Val Ala Ser Val Gln Gln Gln Arg Gln Glu Gln Ser Tyr
   1 5 10
<210> 762
   <211> 15
   <212> PRT
   <213> Homo sapiens
<400> 762
   Asp Val Gly Val Tyr Arg Ala Val Thr Pro Gln Gly Arg Pro Asp
   1 5 10 15
<210> 763
   <211> 10
   <212> PRT
   <213> Homo sapiens
<400> 763
   Asp Val Ile Gly Asp Leu His Gln Cys Gln
   1 5 10
<210> 764
   <211> 9
   <212> PRT
   <213> Homo sapiens
<400> 764
   Asp Val Ile Arg Leu Ile Met Gln Tyr
   1 5
<210> 765
   <211> 12
   <212> PRT
   <213> Homo sapiens
<400> 765
   Glu Ile Ile Glu Glu Thr Lys Val Glu Asp Glu Lys
   1 5 10
<210> 766
   <211> 15
   <212> PRT
   <213> Homo sapiens
<400> 766
   Glu Ile Ile Val Thr His Phe Pro Phe Asp Glu Gln Asn Cys Ser
   1 5 10 15
<210> 767
   <211> 10
   <212> PRT
   <213> Homo sapiens
<400> 767
   Glu Leu Ile Asn Asn Glu Leu Ser His Phe
   1 5 10
<210> 768
   <211> 17
   <212> PRT
   <213> Homo sapiens
<400> 768
   Glu Pro Gly Glu Pro Glu Phe Lys Tyr Ile Gly Asn Met His Gly Asn
   1 5 10 15
   Glu
<210> 769
   <211> 15
   <212> PRT
   <213> Homo sapiens
<400> 769
   Glu Pro Asn Ala Ile Val Ser Asp Gly Lys Tyr His Val Val Arg
   1 5 10 15
<210> 770
   <211> 10
   <212> PRT
   <213> Homo sapiens
<400> 770
   Glu Arg Ile Ala Gly Leu Glu Gln Glu Asp
   1 5 10
<210> 771
   <211> 11
   <212> PRT
   <213> Homo sapiens
<400> 771
   Glu Arg Thr Ala Lys Glu Leu Asp Thr Asn Thr
   1 5 10
<210> 772
   <211> 15
   <212> PRT
   <213> Homo sapiens
<400> 772
   Glu Ser Asn Ala Ile Ile Asn Asp Gly Lys Tyr His Val Val Arg
   1 5 10 15
<210> 773
   <211> 11
   <212> PRT
   <213> Homo sapiens
<400> 773
   Glu Thr Asp Leu Leu Asp Ile Arg Ser Glu Tyr
   1 5 10
<210> 774
   <211> 10
   <212> PRT
   <213> Homo sapiens
<400> 774
   Glu Val Gly Gly Glu Ala Leu Gly Arg Leu
   1 5 10
<210> 775
   <211> 9
   <212> PRT
   <213> Homo sapiens
<400> 775
   Phe Gly Glu Ser Leu Pro Ala Arg Phe
   1 5
<210> 776
   <211> 10
   <212> PRT
   <213> Homo sapiens
<400> 776
   Phe Gly Tyr Ser Asn Arg Val Val Asp Leu
   1 5 10
<210> 777
   <211> 13
   <212> PRT
   <213> Homo sapiens
<400> 777
   Phe Ile Thr Ala Asp His Leu Ile Glu Lys Leu Arg Thr
   1 5 10
<210> 778
   <211> 12
   <212> PRT
   <213> Homo sapiens
<400> 778
   Phe Leu Gly Val Ala Tyr Gly Ala Thr Arg Tyr Asn
   1 5 10
<210> 779
   <211> 14
   <212> PRT
   <213> Homo sapiens
<400> 779
   Phe Leu Ser Phe Pro Thr Thr Lys Thr Tyr Phe Pro His Phe
   1 5 10
<210> 780
   <211> 12
   <212> PRT
   <213> Homo sapiens
<400> 780
   Phe Arg Asp Gly Asp Ile Leu Gly Lys Tyr Val Asp
   1 5 10
<210> 781
   <211> 11
   <212> PRT
   <213> Homo sapiens
<400> 781
   Phe Arg Asp Gln Glu Gly Gln Asp Val Leu Leu
   1 5 10
<210> 782
   <211> 10
   <212> PRT
   <213> Homo sapiens
<400> 782
   Phe Arg Glu Leu Asn Glu Ala Leu Glu Leu
   1 5 10
<210> 783
   <211> 12
   <212> PRT
   <213> Homo sapiens
<400> 783
   Phe Arg Ile Asp Ser Val Ser Glu Gly Asn Ala Gly
   1 5 10
<210> 784
   <211> 13
   <212> PRT
   <213> Homo sapiens
<400> 784
   Phe Ser Glu Thr Gly Ala Gly Lys His Val Pro Arg Ala
   1 5 10
<210> 785
   <211> 15
   <212> PRT
   <213> Homo sapiens
<400> 785
   Phe Ser Glu Thr Gly Ala Gly Lys His Val Pro Arg Ala Val Phe
   1 5 10 15
<210> 786
   <211> 10
   <212> PRT
   <213> Homo sapiens
<400> 786
   Phe Ser Gln Phe Gly Pro Ile Glu Arg Ala
   1 5 10
<210> 787
   <211> 10
   <212> PRT
   <213> Homo sapiens
<400> 787
   Phe Ser Val Ala Thr Val Gln Thr Thr Trp
   1 5 10
<210> 788
   <211> 12
   <212> PRT
   <213> Homo sapiens
<400> 788
   Phe Thr Pro Ala Val His Ala Ser Leu Asp Lys Phe
   1 5 10
<210> 789
   <211> 8
   <212> PRT
   <213> Homo sapiens
<400> 789
   Phe Thr Arg Ser Leu Phe Val Asn
   1 5
<210> 790
   <211> 9
   <212> PRT
   <213> Homo sapiens
<400> 790
   Phe Val Asn Asp Ile Phe Glu Arg Ile
   1 5
<210> 791
   <211> 8
   <212> PRT
   <213> Homo sapiens
<400> 791
   Phe Val Gln Met Met Thr Ala Lys
   1 5
<210> 792
   <211> 14
   <212> PRT
   <213> Homo sapiens
<400> 792
   Gly His Leu Tyr Arg Glu Asp Gln Thr Ser Pro Ala Pro Gly
   1 5 10
<210> 793
   <211> 16
   <212> PRT
   <213> Homo sapiens
<400> 793
   Gly Lys Val Asn Val Asp Glu Val Gly Gly Glu Ala Leu Gly Arg Leu
   1               5                   10                  15
<210> 794
   <211> 17
   <212> PRT
   <213> Homo sapiens
<400> 794
   Gly Lys Val Asn Val Asp Glu Val Gly Gly Glu Ala Leu Gly Arg Leu
   1               5                   10                  15
   Leu
<210> 795
   <211> 14
   <212> PRT
   <213> Homo sapiens
<400> 795
   Gly Asn Arg Ile Ala Gln Trp Gln Ser Phe Gln Leu Glu Gly
   1               5                   10
<210> 796
   <211> 10
   <212> PRT
   <213> Homo sapiens
<400> 796
   Gly Arg Thr Leu Gln Asn Thr Met Val Asn
   1               5                   10
<210> 797
   <211> 12
   <212> PRT
   <213> Homo sapiens
<400> 797
   Gly Arg Thr Thr Gly Ile Val Met Asp Ser Gly Asp
   1               5                   10
<210> 798
   <211> 9
   <212> PRT
   <213> Homo sapiens
<400> 798
   Gly Thr Ala Asp Val His Phe Glu Arg
   1               5
<210> 799
   <211> 9
   <212> PRT
   <213> Homo sapiens
<400> 799
   Gly Thr Phe Gly Gly Leu Gly Ser Lys
   1 5
<210> 800
   <211> 9
   <212> PRT
   <213> Homo sapiens
<400> 800
   Gly Thr Ile Asn Ile Val His Pro Lys
   1 5
<210> 801
   <211> 10
   <212> PRT
   <213> Homo sapiens
<400> 801
   Gly Val Ile Asp Val Ile Thr Lys Thr Trp
   1 5 10
<210> 802
   <211> 17
   <212> PRT
   <213> Homo sapiens
<400> 802
   Gly Trp Ser Ser His Pro Pro Pro Ser Pro Cys Arg Glu Gln Glu Ile
   1 5 10 15
   Arg
<210> 803
   <211> 15
   <212> PRT
   <213> Homo sapiens
<400> 803
   His Gly Ser Lys Tyr Leu Ala Thr Ala Ser Thr Met Asp His Ala
   1 5 10 15
<210> 804
   <211> 9
   <212> PRT
   <213> Homo sapiens
<400> 804
   His Ile Ile Leu Val Leu Ser Gly Lys
   1 5
<210> 805
   <211> 9
   <212> PRT
   <213> Homo sapiens
<400> 805
   His Ser Glu Arg His Val Leu Leu Tyr
   1 5
<210> 806
   <211> 9
   <212> PRT
   <213> Homo sapiens
<400> 806
   Ile Phe Ala Asn Ala His Thr Tyr His
   1 5
<210> 807
   <211> 8
   <212> PRT
   <213> Homo sapiens
<400> 807
   Ile Phe Glu Lys Ser Phe Val Asn
   1 5
<210> 808
   <211> 11
   <212> PRT
   <213> Homo sapiens
<400> 808
   Ile Ile Glu Glu Thr Lys Val Glu Asp Glu Lys
   1 5 10
<210> 809
   <211> 9
   <212> PRT
   <213> Homo sapiens
<400> 809
   Ile Ile Tyr Lys Gly Gly Thr Ser Arg
   1 5
<210> 810
   <211> 9
   <212> PRT
   <213> Homo sapiens
<400> 810
   Ile Pro Met Gly Lys Ser Met Leu Val
   1 5
<210> 811
   <211> 10
   <212> PRT
   <213> Homo sapiens
<400> 811
   Ile Pro Asn Lys Asn Arg Ala Glu Gln Leu
   1 5 10
<210> 812
   <211> 13
   <212> PRT
   <213> Homo sapiens
<400> 812
   Ile Arg Glu Ala Asp Ile Asp Gly Asp Gly Gln Val Asn
   1 5 10
<210> 813
   <211> 12
   <212> PRT
   <213> Homo sapiens
<400> 813
   Ile Arg Glu Thr Ser Leu Asp Thr Lys Ser Val Ser
   1 5 10
<210> 814
   <211> 12
   <212> PRT
   <213> Homo sapiens
<400> 814
   Ile Ser Lys Leu Ser Ala Lys Phe Gly Gly Gln Ala
   1 5 10
<210> 815
   <211> 16
   <212> PRT
   <213> Homo sapiens
<400> 815
   Ile Ser Lys Gln Glu Tyr Asp Glu Ser Gly Pro Ser Ile Val His Arg
   1 5 10 15
<210> 816
   <211> 17
   <212> PRT
   <213> Homo sapiens
<400> 816
   Ile Ser Lys Gln Glu Tyr Asp Glu Ser Gly Pro Ser Ile Val His Arg
   1 5 10 15
   Lys
<210> 817
   <211> 17
   <212> PRT
   <213> Homo sapiens
<400> 817
   Ile Ser Trp Tyr Asp Asn Glu Phe Gly Tyr Ser Asn Arg Val Val Asp
   1 5 10 15
   Leu
<210> 818
   <211> 13
   <212> PRT
   <213> Homo sapiens
<400> 818
   Ile Thr Ala Asn Ala Thr Glu Pro Gln Lys Ala Glu Asn
   1 5 10
<210> 819
   <211> 12
   <212> PRT
   <213> Homo sapiens
<400> 819
   Ile Thr Gln Asn Gly Asp Tyr Gln Leu Arg Thr Gln
   1 5 10
<210> 820
   <211> 14
   <212> PRT
   <213> Homo sapiens
<400> 820
   Ile Thr Ser Ile Val Lys Asp Ser Ser Ala Ala Arg Asn Gly
   1 5 10
<210> 821
   <211> 9
   <212> PRT
   <213> Homo sapiens
<400> 821
   Ile Val Ala Gly Thr Leu Thr Asn Lys
   1 5
<210> 822
   <211> 16
   <212> PRT
   <213> Homo sapiens
<400> 822
   Ile Val Thr Pro Arg Thr Pro Pro Pro Ser Gln Gly Lys Gly Arg Gly
   1 5 10 15
<210> 823
   <211> 18
   <212> PRT
   <213> Homo sapiens
<400> 823
   Ile Val Thr Pro Arg Thr Pro Pro Pro Ser Gln Gly Lys Gly Arg Gly
   1 5 10 15
   Leu Ser
<210> 824
   <211> 11
   <212> PRT
   <213> Homo sapiens
<400> 824
   Ile Val Val Tyr Pro Val His Gly Phe Met Tyr
   1 5 10
<210> 825
   <211> 9
   <212> PRT
   <213> Homo sapiens
<400> 825
   Lys Ile Ala Asp Arg Phe Leu Leu Tyr
   1 5
<210> 826
   <211> 15
   <212> PRT
   <213> Homo sapiens
<400> 826
   Lys Ile Trp Arg Pro Asp Leu Val Leu Tyr Asn Asn Ala Asp Gly
   1 5 10 15
<210> 827
   <211> 10
   <212> PRT
   <213> Homo sapiens
<400> 827
   Lys Pro Arg Asp Val Ser Ser Val Glu Leu
   1 5 10
<210> 828
   <211> 11
   <212> PRT
   <213> Homo sapiens
<400> 828
   Lys Pro Thr Ile Ser Val Lys Glu Ala Phe Tyr
   1 5 10
<210> 829
   <211> 17
   <212> PRT
   <213> Homo sapiens
<400> 829
   Lys Ser Ala Ile Glu Gly Ile Lys Tyr Ile Ala Glu Thr Met Lys Ser
   1 5 10 15
   Asp
<210> 830
   <211> 9
   <212> PRT
   <213> Homo sapiens
<400> 830
   Lys Thr Tyr Gly Glu Ile Phe Glu Lys
   1 5
<210> 831
   <211> 14
   <212> PRT
   <213> Homo sapiens
<400> 831
   Lys Val Lys Lys Ile Tyr Leu Asp Glu Lys Arg Leu Leu Ala
   1 5 10
<210> 832
   <211> 15
   <212> PRT
   <213> Homo sapiens
<400> 832
   Lys Val Lys Lys Ile Tyr Leu Asp Glu Lys Arg Leu Leu Ala Gly
   1 5 10 15
<210> 833
   <211> 16
   <212> PRT
   <213> Homo sapiens
<400> 833
   Lys Val Lys Lys Ile Tyr Leu Asp Glu Lys Arg Leu Leu Ala Gly Asp
   1 5 10 15
<210> 834
   <211> 13
   <212> PRT
   <213> Homo sapiens
<400> 834
   Lys Val Val Pro Glu Met Thr Glu Ile Leu Lys Lys Lys
   1 5 10
<210> 835
   <211> 11
   <212> PRT
   <213> Homo sapiens
<400> 835
   Lys Val Tyr Asn Glu Ala Gly Val Thr Phe Thr
   1 5 10
<210> 836
   <211> 12
   <212> PRT
   <213> Homo sapiens
<400> 836
   Lys Tyr Leu Pro Gly Pro Leu Gln Asp Met Phe Lys
   1 5 10
<210> 837
   <211> 11
   <212> PRT
   <213> Homo sapiens
<400> 837
   Leu Ala Ser Gly Val His Gly Leu Pro Leu Leu
   1 5 10
<210> 838
   <211> 12
   <212> PRT
   <213> Homo sapiens
<400> 838
   Leu Ala Ser Val Ser Thr Val Leu Thr Ser Lys Tyr
   1 5 10
<210> 839
   <211> 13
   <212> PRT
   <213> Homo sapiens
<400> 839
   Leu Asp Phe Leu Lys Ala Val Asp Thr Asn Arg Ala Ser
   1 5 10
<210> 840
   <211> 11
   <212> PRT
   <213> Homo sapiens
<400> 840
   Leu Asp Thr His Pro Leu Pro Leu Val Glu Val
   1 5 10
<210> 841
   <211> 15
   <212> PRT
   <213> Homo sapiens
<400> 841
   Leu Glu Arg Ile Gln Ile Gln Glu Ala Ala Lys Lys Lys Pro Gly
   1 5 10 15
<210> 842
   <211> 9
   <212> PRT
   <213> Homo sapiens
<400> 842
   Leu Phe Ala Leu Thr Leu Pro Ile Trp
   1 5
<210> 843
   <211> 11
   <212> PRT
   <213> Homo sapiens
<400> 843
   Leu Gly Ser Val Ala Gly Ile Phe Asn Tyr Tyr
   1               5                   10
<210> 844
   <211> 10
   <212> PRT
   <213> Homo sapiens
<400> 844
   Leu Gly Val Leu Glu Ser Asp Leu Pro Ser
   1               5                   10
<210> 845
   <211> 12
   <212> PRT
   <213> Homo sapiens
<400> 845
   Leu Ile Leu Pro Glu Ala Val Gly Gly Thr Val Phe
   1               5                   10
<210> 846
   <211> 10
   <212> PRT
   <213> Homo sapiens
<400> 846
   Leu Leu Pro Pro Pro Pro Pro Val Ala Arg
   1               5                   10
<210> 847
   <211> 13
   <212> PRT
   <213> Homo sapiens
<400> 847
   Leu Leu Val Ile Val Glu Leu Ile Pro Ser Thr Ser Ser
   1               5                   10
<210> 848
   <211> 14
   <212> PRT
   <213> Homo sapiens
<400> 848
   Leu Leu Val Pro Thr Lys Ala Arg Phe Pro Ala Phe Glu Leu
   1               5                   10
<210> 849
   <211> 13
   <212> PRT
   <213> Homo sapiens
<400> 849
   Leu Asn Glu Thr Gly Asp Glu Pro Phe Gln Tyr Lys Asn
   1               5                   10
<210> 850
   <211> 14
   <212> PRT
   <213> Homo sapiens
<400> 850
   Leu Asn Thr Ile Leu Pro Asp Ala Arg Asp Pro Ala Phe Lys
   1               5                   10
<210> 851
   <211> 11
   <212> PRT
   <213> Homo sapiens
<400> 851
   Leu Arg Pro Ile Gln Ala Leu Gly Thr Glu Tyr
   1               5                   10
<210> 852
   <211> 14
   <212> PRT
   <213> Homo sapiens
<400> 852
   Leu Ser Ala Leu Glu Glu Tyr Thr Lys Lys Leu Asn Thr Gln
   1               5                   10
<210> 853
   <211> 13
   <212> PRT
   <213> Homo sapiens
<400> 853
   Leu Ser Phe Pro Thr Thr Lys Thr Tyr Phe Pro His Phe
   1               5                   10
<210> 854
   <211> 9
   <212> PRT
   <213> Homo sapiens
<400> 854
   Leu Thr Asp Arg Glu Leu Glu Glu Tyr
   1               5
<210> 855
   <211> 9
   <212> PRT
   <213> Homo sapiens
<400> 855
   Leu Val Glu Ala Lys Gln Tyr Ile Ile
   1 5
<210> 856
   <211> 10
   <212> PRT
   <213> Homo sapiens
<400> 856
   Leu Val Ser Ala Ser Gln Asp Gly Lys Leu
   1 5 10
<210> 857
   <211> 10
   <212> PRT
   <213> Homo sapiens
<400> 857
   Leu Val Val Tyr Phe Phe Arg Val Arg Asn
   1 5 10
<210> 858
   <211> 10
   <212> PRT
   <213> Homo sapiens
<400> 858
   Met Leu Ser Asn Pro Asp Leu Met Arg Gln
   1 5 10
<210> 859
   <211> 9
   <212> PRT
   <213> Homo sapiens
<400> 859
   Met Pro Ile Leu Gly Leu Gly Thr Trp
   1 5
<210> 860
   <211> 15
   <212> PRT
   <213> Homo sapiens
<400> 860
   Met Ser Ile Leu Gly Met Thr Pro Gly Phe Gly Asp Lys Thr Phe
   1 5 10 15
<210> 861
   <211> 10
   <212> PRT
   <213> Homo sapiens
<400> 861
   Met Ser Leu Lys Gln Thr Pro Leu Ser Arg
   1 5 10
<210> 862
   <211> 10
   <212> PRT
   <213> Homo sapiens
<400> 862
   Asn Glu Ile Glu Asp Thr Phe Arg Gln Phe
   1 5 10
<210> 863
   <211> 12
   <212> PRT
   <213> Homo sapiens
<400> 863
   Asn Glu Thr Gly Asp Glu Pro Phe Gln Tyr Lys Asn
   1 5 10
<210> 864
   <211> 15
   <212> PRT
   <213> Homo sapiens
<400> 864
   Asn Gln Glu Leu Arg Ala Asp Gly Thr Val Asn Gln Ile Glu Gly
   1 5 10 15
<210> 865
   <211> 10
   <212> PRT
   <213> Homo sapiens
<400> 865
   Asn Arg Ile Thr Ile Pro Val Gln Thr Phe
   1 5 10
<210> 866
   <211> 8
   <212> PRT
   <213> Homo sapiens
<400> 866
   Asn Thr Asp Ser Pro Leu Arg Tyr
   1 5
<210> 867
   <211> 9
   <212> PRT
   <213> Homo sapiens
<400> 867
   Asn Thr Glu Glu Glu Gly Leu Lys Tyr
   1 5
<210> 868
   <211> 18
   <212> PRT
   <213> Homo sapiens
<400> 868
   Asn Trp Val Arg Lys Val Phe Ile Asp Thr Ile Pro Asn Ile Met Phe
   1 5 10 15
   Phe Ser
<210> 869
   <211> 11
   <212> PRT
   <213> Homo sapiens
<400> 869
   Pro Glu Val Leu Asn Arg Ala Leu Ser Asn Leu
   1 5 10
<210> 870
   <211> 11
   <212> PRT
   <213> Homo sapiens
<400> 870
   Pro Lys Phe Glu Val Ile Glu Lys Pro Gln Ala
   1 5 10
<210> 871
   <211> 15
   <212> PRT
   <213> Homo sapiens
<400> 871
   Pro Gln Arg Phe Val Val Ile Gln Asn Glu Asp Leu Gly Pro Ala
   1 5 10 15
<210> 872
   <211> 11
   <212> PRT
   <213> Homo sapiens
<400> 872
   Pro Ser Ile His Ile Trp Asp Thr Glu Thr Ile
   1               5                   10
<210> 873
   <211> 13
   <212> PRT
   <213> Homo sapiens
<400> 873
   Pro Val Asp Leu Ser Lys Trp Ser Gly Pro Leu Ser Leu
   1               5                   10
<210> 874
   <211> 14
   <212> PRT
   <213> Homo sapiens
<400> 874
   Gln Glu Glu Leu Pro Ser Leu Trp Asn Ser Arg Val Gly Phe
   1               5                   10
<210> 875
   <211> 11
   <212> PRT
   <213> Homo sapiens
<400> 875
   Gln Ile Arg Glu Thr Ser Leu Asp Thr Lys Ser
   1               5                   10
<210> 876
   <211> 9
   <212> PRT
   <213> Homo sapiens
<400> 876
   Gln Leu Tyr Met Ile Gln Lys Lys Ser
   1               5
<210> 877
   <211> 9
   <212> PRT
   <213> Homo sapiens
<400> 877
   Gln Leu Tyr Trp Ser His Pro Arg Lys
   1 5
<210> 878
   <211> 11
   <212> PRT
   <213> Homo sapiens
<400> 878
   Gln Ser Met Asp Ser Lys Leu Ser Gly Arg Tyr
   1 5 10
<210> 879
   <211> 19
   <212> PRT
   <213> Homo sapiens
<400> 879
   Gln Thr Pro Gln His Pro Ser Phe Leu Phe Gln Asp Lys Arg Val Ser
   1 5 10 15
   Trp Ser Leu
<210> 880
   <211> 10
   <212> PRT
   <213> Homo sapiens
<400> 880
   Gln Val Ala Asp Val Val Val Ser Arg Trp
   1 5 10
<210> 881
   <211> 10
   <212> PRT
   <213> Homo sapiens
<400> 881
   Gln Val Leu Gly Val Val Ala Arg Ala Ala
   1 5 10
<210> 882
   <211> 9
   <212> PRT
   <213> Homo sapiens
<400> 882
   Arg Ala Ile Glu Gln Leu Ala Met Met
   1 5
<210> 883
   <211> 10
   <212> PRT
   <213> Homo sapiens
<400> 883
   Arg His Gly Ser Lys Tyr Leu Ala Thr Ala
   1 5 10
<210> 884
   <211> 18
   <212> PRT
   <213> Homo sapiens
<400> 884
   Arg His Gly Ser Lys Tyr Leu Ala Thr Ala Ser Thr Met Asp His Ala
   1 5 10 15
   Arg His
<210> 885
   <211> 10
   <212> PRT
   <213> Homo sapiens
<400> 885
   Arg Leu Tyr Pro Trp Gly Val Val Glu Val
   1 5 10
<210> 886
   <211> 10
   <212> PRT
   <213> Homo sapiens
<400> 886
   Arg Gln Gln Pro Pro Asp Leu Val Glu Phe
   1 5 10
<210> 887
   <211> 11
   <212> PRT
   <213> Homo sapiens
<400> 887
   Ser Cys Gly Asp Leu His Ser Val Met Leu Glu
   1 5 10
<210> 888
   <211> 15
   <212> PRT
   <213> Homo sapiens
<400> 888
   Ser Glu His Glu Thr Arg Leu Val Ala Lys Leu Phe Lys Asp Tyr
   1 5 10 15
<210> 889
   <211> 11
   <212> PRT
   <213> Homo sapiens
<400> 889
   Ser Gly Cys Asp Leu Glu Glu Ile Pro Leu Asp
   1 5 10
<210> 890
   <211> 14
   <212> PRT
   <213> Homo sapiens
<400> 890
   Ser His Gly Arg Thr Gln Asp Glu Asn Pro Val Val His Phe
   1 5 10
<210> 891
   <211> 10
   <212> PRT
   <213> Homo sapiens
<400> 891
   Ser His Pro Pro Val Glu Gly Ser Tyr Ala
   1 5 10
<210> 892
   <211> 9
   <212> PRT
   <213> Homo sapiens
<400> 892
   Ser Ile Phe Lys Gln Pro Val Thr Lys
   1 5
<210> 893
   <211> 9
   <212> PRT
   <213> Homo sapiens
<400> 893
   Ser Ile Thr Ser Val Phe Ile Thr Lys
   1 5
<210> 894
   <211> 14
   <212> PRT
   <213> Homo sapiens
<400> 894
   Ser Asn Ala Ile Ile Asn Asp Gly Lys Tyr His Val Val Arg
   1 5 10
<210> 895
   <211> 12
   <212> PRT
   <213> Homo sapiens
<400> 895
   Ser Ser Lys Phe Gln Val Asp Asn Asn Asn Arg Leu
   1 5 10
<210> 896
   <211> 10
   <212> PRT
   <213> Homo sapiens
<400> 896
   Ser Thr Asp Pro Ser Val Leu Gly Lys Tyr
   1 5 10
<210> 897
   <211> 9
   <212> PRT
   <213> Homo sapiens
<400> 897
   Ser Thr Ile Glu Tyr Val Ile Gln Arg
   1 5
<210> 898
   <211> 11
   <212> PRT
   <213> Homo sapiens
<400> 898
   Ser Thr Ile Gly Pro Glu Gly Val His Gln Lys
   1 5 10
<210> 899
   <211> 12
   <212> PRT
   <213> Homo sapiens
<400> 899
   Ser Val Ala Ala Leu Ser Cys Leu Met Leu Val Ala
   1 5 10
<210> 900
   <211> 10
   <212> PRT
   <213> Homo sapiens
<400> 900
   Ser Val Phe Gln Gln Gly Met Lys Asn Lys
   1               5                   10
<210> 901
   <211> 11
   <212> PRT
   <213> Homo sapiens
<400> 901
   Ser Val Pro Glu Phe Pro Leu Ser Pro Pro Lys
   1               5                   10
<210> 902
   <211> 9
   <212> PRT
   <213> Homo sapiens
<400> 902
   Ser Val Ser Pro Val Val His Val Arg
   1               5
<210> 903
   <211> 8
   <212> PRT
   <213> Homo sapiens
<400> 903
   Thr Ala Ile Arg His His Val Leu
   1               5
<210> 904
   <211> 10
   <212> PRT
   <213> Homo sapiens
<400> 904
   Thr Gly Ile Leu Asp Ser Ile Gly Arg Phe
   1               5                   10
<210> 905
   <211> 11
   <212> PRT
   <213> Homo sapiens
<400> 905
   Thr Ile Ile Asp Leu Pro Gly Ile Thr Arg Val
   1 5 10
<210> 906
   <211> 10
   <212> PRT
   <213> Homo sapiens
<400> 906
   Thr Ile Gln Lys Ser Ser Leu Asp Gln Leu
   1 5 10
<210> 907
   <211> 9
   <212> PRT
   <213> Homo sapiens
<400> 907
   Thr Leu Leu Gly Asp Phe Ile Tyr Trp
   1 5
<210> 908
   <211> 10
   <212> PRT
   <213> Homo sapiens
<400> 908
   Thr Met Asp His Ala Arg His Gly Phe Leu
   1 5 10
<210> 909
   <211> 16
   <212> PRT
   <213> Homo sapiens
<400> 909
   Thr Asn Val Pro Arg Ala Ser Val Pro Asp Gly Phe Leu Ser Glu Leu
   1 5 10 15
<210> 910
   <211> 14
   <212> PRT
   <213> Homo sapiens
<400> 910
   Thr Pro Asp Ala Met Lys Ala Ala Met Ala Leu Glu Lys Lys
   1 5 10
<210> 911
   <211> 16
   <212> PRT
   <213> Homo sapiens
<400> 911
   Thr Ser Pro Ala Lys Gln Gln Ala Pro Pro Val Arg Asn Leu His Gln
   1 5 10 15
<210> 912
   <211> 10
   <212> PRT
   <213> Homo sapiens
<400> 912
   Thr Thr Gly Ile Leu Ser Ile Leu Phe Tyr
   1 5 10
<210> 913
   <211> 15
   <212> PRT
   <213> Homo sapiens
<400> 913
   Thr Thr Met Glu Lys Ala Gly Ala His Leu Gln Gly Gly Ala Lys
   1 5 10 15
<210> 914
   <211> 9
   <212> PRT
   <213> Homo sapiens
<400> 914
   Thr Thr Tyr Asn Tyr Ala Val Leu Lys
   1 5
<210> 915
   <211> 8
   <212> PRT
   <213> Homo sapiens
<400> 915
   Thr Val Phe Ser Thr His Phe Met
   1 5
<210> 916
   <211> 12
   <212> PRT
   <213> Homo sapiens
<400> 916
   Val Asp Leu Glu Pro Thr Val Ile Asp Glu Val Arg
   1 5 10
<210> 917
   <211> 13
   <212> PRT
   <213> Homo sapiens
<400> 917
   Val Asp Pro Glu Asn Phe Arg Leu Leu Gly Asn Val Leu
   1 5 10
<210> 918
   <211> 12
   <212> PRT
   <213> Homo sapiens
<400> 918
   Val Asp Trp Ile Ile Leu Gln Cys Ala Glu Asp Ile
   1 5 10
<210> 919
   <211> 12
   <212> PRT
   <213> Homo sapiens
<400> 919
   Val Glu Glu Ser Lys Asp Ala Gly Ile Arg Thr Leu
   1 5 10
<210> 920
   <211> 10
   <212> PRT
   <213> Homo sapiens
<400> 920
   Val Gly Gly Glu Ala Leu Gly Arg Leu Leu
   1 5 10
<210> 921
   <211> 13
   <212> PRT
   <213> Homo sapiens
<400> 921
   Val His Leu Lys Asn Ala Ser Arg Gly Ser Ala Gly Asn
   1 5 10
<210> 922
   <211> 12
   <212> PRT
   <213> Homo sapiens
<400> 922
   Val His Leu Thr Pro Glu Glu Lys Ser Ala Val Thr
   1 5 10
<210> 923
   <211> 31
   <212> PRT
   <213> Homo sapiens
<400> 923
   Val His Leu Thr Pro Glu Glu Lys Ser Ala Val Thr Ala Leu Trp Gly
   1 5 10 15
   Lys Val Asn Val Asp Glu Val Gly Gly Glu Ala Leu Gly Arg Leu
   20 25 30
<210> 924
   <211> 10
   <212> PRT
   <213> Homo sapiens
<400> 924
   Val Ile Arg Leu Leu Leu Ala Ser Gly Ala
   1 5 10
<210> 925
   <211> 9
   <212> PRT
   <213> Homo sapiens
<400> 925
   Val Leu Gly Asp Leu Phe Val Arg Asp
   1 5
<210> 926
   <211> 11
   <212> PRT
   <213> Homo sapiens
<400> 926
   Val Leu Ser Pro Ala Asp Lys Thr Asn Val Lys
   1 5 10
<210> 927
   <211> 25
   <212> PRT
   <213> Homo sapiens
<400> 927
   Val Leu Ser Pro Ala Asp Lys Thr Asn Val Lys Ala Ala Trp Gly Lys
   1 5 10 15
   Val Gly Ala His Ala Gly Glu Tyr Gly
   20 25
<210> 928
   <211> 31
   <212> PRT
   <213> Homo sapiens
<400> 928
   Val Leu Ser Pro Ala Asp Lys Thr Asn Val Lys Ala Ala Trp Gly Lys
   1 5 10 15
   Val Gly Ala His Ala Gly Glu Tyr Gly Ala Glu Ala Leu Glu Arg
   20 25 30
<210> 929
   <211> 7
   <212> PRT
   <213> Homo sapiens
<400> 929
   Val Gln Met Met Thr Ala Lys
   1 5
<210> 930
   <211> 15
   <212> PRT
   <213> Homo sapiens
<400> 930
   Val Ser His Phe Phe Arg Glu Leu Ala Glu Glu Lys Arg Glu Gly
   1 5 10 15
<210> 931
   <211> 10
   <212> PRT
   <213> Homo sapiens
<400> 931
   Val Thr Ala Ser Lys Ala Ile Ile Glu Lys
   1 5 10
<210> 932
   <211> 9
   <212> PRT
   <213> Homo sapiens
<400> 932
   Val Thr Met Thr Ala Ser Arg Met Leu
   1 5
<210> 933
   <211> 9
   <212> PRT
   <213> Homo sapiens
<400> 933
   Val Thr Asn Pro Asp Thr Gly Arg Tyr
   1 5
<210> 934
   <211> 16
   <212> PRT
   <213> Homo sapiens
<400> 934
   Val Thr Pro Arg Thr Pro Pro Pro Ser Gln Gly Lys Gly Arg Gly Leu
   1 5 10 15
<210> 935
   <211> 9
   <212> PRT
   <213> Homo sapiens
<400> 935
   Val Thr Ser Pro Ile Arg Ala Ser Tyr
   1 5
<210> 936
   <211> 12
   <212> PRT
   <213> Homo sapiens
<400> 936
   Val Thr Val Phe Pro Ile Gly Ile Gly Asp Arg Tyr
   1 5 10
<210> 937
   <211> 14
   <212> PRT
   <213> Homo sapiens
<400> 937
   Val Val Ala Gly Val Ala Asn Ala Leu Ala His Lys Tyr His
   1 5 10
<210> 938
   <211> 11
   <212> PRT
   <213> Homo sapiens
<400> 938
   Val Val Ile Arg Gly Asn Ser Ile Ile Met Leu
   1 5 10
<210> 939
   <211> 11
   <212> PRT
   <213> Homo sapiens
<400> 939
   Val Val Tyr Pro Ser Ser Ser Gln Glu Ser Glu
   1               5                   10
<210> 940
   <211> 8
   <212> PRT
   <213> Homo sapiens
<400> 940
   Val Tyr Arg Val Asn Ser Tyr Trp
   1               5
<210> 941
   <211> 17
   <212> PRT
   <213> Homo sapiens
<400> 941
   Trp Ile Ser Lys Gln Glu Tyr Asp Glu Ser Gly Pro Ser Ile Val His
   1               5                   10                  15
   Arg
<210> 942
   <211> 18
   <212> PRT
   <213> Homo sapiens
<400> 942
   Trp Ile Ser Lys Gln Glu Tyr Asp Glu Ser Gly Pro Ser Ile Val His
   1               5                   10                  15
   Arg Lys
<210> 943
   <211> 20
   <212> PRT
   <213> Homo sapiens
<400> 943
   Trp Ile Ser Lys Gln Glu Tyr Asp Glu Ser Gly Pro Ser Ile Val His
   1 5 10 15
   Arg Lys Cys Phe
   20
<210> 944
   <211> 10
   <212> PRT
   <213> Homo sapiens
<400> 944
   Trp Ser Thr Val Leu Ser Thr Tyr His Phe
   1 5 10
<210> 945
   <211> 11
   <212> PRT
   <213> Homo sapiens
<400> 945
   Trp Thr Thr Gly Thr Ile Ser Ser Gln Arg Phe
   1 5 10
<210> 946
   <211> 13
   <212> PRT
   <213> Homo sapiens
<400> 946
   Trp Val Pro Tyr Ile Asn Gly Lys Phe Lys Lys Asp Asn
   1 5 10
<210> 947
   <211> 15
   <212> PRT
   <213> Homo sapiens
<400> 947
   Trp Val Ser Gln Arg Thr Ala Thr Ala Gly Thr Ala Ser Pro Pro
   1 5 10 15
<210> 948
   <211> 11
   <212> PRT
   <213> Homo sapiens
<400> 948
   Tyr Ala Val Ala Val Val Lys Lys Asp Ser Gly
   1 5 10
<210> 949
   <211> 11
   <212> PRT
   <213> Homo sapiens
<400> 949
   Tyr Asp Glu Ser Gly Pro Ser Ile Val His Arg
   1 5 10
<210> 950
   <211> 12
   <212> PRT
   <213> Homo sapiens
<400> 950
   Tyr Asp Glu Ser Gly Pro Ser Ile Val His Arg Lys
   1 5 10
<210> 951
   <211> 14
   <212> PRT
   <213> Homo sapiens
<400> 951
   Tyr Asp Asn Glu Phe Gly Tyr Ser Asn Arg Val Val Asp Leu
   1 5 10
<210> 952
   <211> 11
   <212> PRT
   <213> Homo sapiens
<400> 952
   Tyr Glu Met Pro Ser Glu Glu Gly Tyr Gln Asp
   1 5 10
<210> 953
   <211> 8
   <212> PRT
   <213> Homo sapiens
<400> 953
   Tyr Glu Val Ser Gln Leu Lys Asp
   1 5
<210> 954
   <211> 8
   <212> PRT
   <213> Homo sapiens
<400> 954
   Tyr Gly Met Pro Arg Gln Ile Leu
   1 5
<210> 955
   <211> 9
   <212> PRT
   <213> Homo sapiens
<400> 955
   Tyr Ile Asp Glu Gln Phe Glu Arg Tyr
   1               5
<210> 956
   <211> 10
   <212> PRT
   <213> Homo sapiens
<400> 956
   Tyr Ile Lys Glu Asp Glu Ala Leu Pro Ser
   1               5                   10
<210> 957
   <211> 9
   <212> PRT
   <213> Homo sapiens
<400> 957
   Tyr Leu Leu Leu Leu Leu Leu Leu Ser
   1               5
<210> 958
   <211> 13
   <212> PRT
   <213> Homo sapiens
<400> 958
   Tyr Gln Lys Val Val Ala Gly Val Ala Asn Ala Leu Ala
   1               5                   10
<210> 959
   <211> 17
   <212> PRT
   <213> Homo sapiens
<400> 959
   Tyr Gln Lys Val Val Ala Gly Val Ala Asn Ala Leu Ala His Lys Tyr
   1               5                   10                  15
   His
<210> 960
   <211> 10
   <212> PRT
   <213> Homo sapiens
<400> 960
   Tyr Thr Asp Val Pro Phe His Asn Gln Tyr
   1               5                   10
<210> 961
   <211> 11
   <212> PRT
   <213> Homo sapiens
<400> 961
   Tyr Thr Glu Arg Asp Ala Ser Gly Met Leu Tyr
   1               5                   10
<210> 962
   <211> 9
   <212> PRT
   <213> Homo sapiens
<400> 962
   Ala Pro Ala His Ala Ile Thr Leu Thr
   1               5
<210> 963
   <211> 9
   <212> PRT
   <213> Multiple sclerosis associated retrovirus
<400> 963
   Ala Thr Leu Lys Pro Ala Thr Phe Leu
   1               5
<210> 964
   <211> 9
   <212> PRT
   <213> Human endogenous retrovirus W
<400> 964
   Ala Thr Leu Asn Pro Ala Thr Phe Leu
   1               5
<210> 965
   <211> 9
   <212> PRT
   <213> Homo sapiens
<400> 965
   Cys Pro Arg Gln Arg Ser Ser Arg Ala
   1               5
<210> 966
   <211> 9
   <212> PRT
   <213> Multiple sclerosis associated retrovirus
<400> 966
   Asp Pro Leu Asn Pro Thr Ser Gln Leu
   1 5
<210> 967
   <211> 9
   <212> PRT
   <213> Multiple sclerosis associated retrovirus
<400> 967
   Phe Pro Tyr Gln Arg Lys Tyr Pro Leu
   1 5
<210> 968
   <211> 9
   <212> PRT
   <213> Homo sapiens
<400> 968
   Gly Pro Ala Arg Val Pro Val Pro Phe
   1 5
<210> 969
   <211> 9
   <212> PRT
   <213> Multiple sclerosis associated retrovirus
<400> 969
   Gly Pro Cys Phe Leu Trp Leu Lys Leu
   1 5
<210> 970
   <211> 9
   <212> PRT
   <213> Multiple sclerosis associated retrovirus
<400> 970
   Ile Pro Glu Glu Ala Glu Trp Phe Thr
   1 5
<210> 971
   <211> 9
   <212> PRT
   <213> Multiple sclerosis associated retrovirus
<400> 971
   Ile Pro Phe Leu Gly Ile Arg Glu Thr
   1 5
<210> 972
   <211> 9
   <212> PRT
   <213> Homo sapiens
<400> 972
   Ile Pro Pro Lys Ala Lys Thr Pro Leu
   1               5
<210> 973
   <211> 9
   <212> PRT
   <213> Homo sapiens
<400> 973
   Ile Pro Arg Leu Cys Pro Leu Gln Ala
   1               5
<210> 974
   <211> 12
   <212> PRT
   <213> Multiple sclerosis associated retrovirus
<400> 974
   Ile Pro Val Arg Pro Asp Ser Gln Phe Leu Phe Ala
   1               5                   10
<210> 975
   <211> 12
   <212> PRT
   <213> Homo sapiens
<400> 975
   Lys Glu Glu Asn Pro Thr Ala Phe Leu Asp Arg Leu
   1               5                   10
<210> 976
   <211> 9
   <212> PRT
   <213> Multiple sclerosis associated retrovirus
<400> 976
   Lys Pro Asn Gly Gln Trp Arg Leu Val
   1               5
<210> 977
   <211> 9
   <212> PRT
   <213> Multiple sclerosis associated retrovirus
<400> 977
   Lys Pro Pro Pro Cys Pro Cys Pro Leu
   1               5
<210> 978
   <211> 9
   <212> PRT
   <213> Multiple sclerosis associated retrovirus
<400> 978
   Lys Val Ser Lys Pro Lys Ala Arg Leu
   1 5
<210> 979
   <211> 9
   <212> PRT
   <213> Multiple sclerosis associated retrovirus
<400> 979
   Leu Pro Leu Gly Ser Glu Gln Asn Leu
   1 5
<210> 980
   <211> 9
   <212> PRT
   <213> Multiple sclerosis associated retrovirus
<400> 980
   Leu Pro Thr Gly Gln Asp Phe Ser Leu
   1 5
<210> 981
   <211> 14
   <212> PRT
   <213> Multiple sclerosis associated retrovirus
<400> 981
   Met Pro Glu Ser Pro Thr Pro Leu Leu Gly Arg Asp Ile Leu
   1 5 10
<210> 982
   <211> 11
   <212> PRT
   <213> Multiple sclerosis associated retrovirus
<400> 982
   Met Pro Ser Pro Ser Glu Pro Arg Val Cys Leu
   1 5 10
<210> 983
   <211> 9
   <212> PRT
   <213> Homo sapiens
<400> 983
   Asn Pro Asp Gly Tyr Ile Asp Val Leu
   1 5
<210> 984
   <211> 12
   <212> PRT
   <213> Human endogenous retrovirus W
<400> 984
   Asn Pro His Thr Pro Gly Thr Ser Thr Gln Leu Ala
   1 5 10
<210> 985
   <211> 9
   <212> PRT
   <213> Multiple sclerosis associated retrovirus
<400> 985
   Asn Pro Arg Ser Glu Asn Thr Arg Leu
   1 5
<210> 986
   <211> 15
   <212> PRT
   <213> Multiple sclerosis associated retrovirus
<400> 986
   Pro Pro Pro Tyr Pro Ser Val Pro Ser Pro Thr Pro Ser Ser Thr
   1 5 10 15
<210> 987
   <211> 9
   <212> PRT
   <213> Homo sapiens
<400> 987
   Gln Pro Leu Ser Cys Asp Trp Gly Thr
   1 5
<210> 988
   <211> 9
   <212> PRT
   <213> Multiple sclerosis associated retrovirus
<400> 988
   Arg Pro Ala Ala Asp Phe His Pro Phe
   1 5
<210> 989
   <211> 11
   <212> PRT
   <213> Multiple sclerosis associated retrovirus
<400> 989
   Arg Pro Leu Pro Leu Pro Ile Gly Leu Lys Ala
   1               5                   10
<210> 990
   <211> 9
   <212> PRT
   <213> Homo sapiens
<400> 990
   Ser Pro Asp Ser Ile Glu Gly Gln Leu
   1               5
<210> 991
   <211> 9
   <212> PRT
   <213> Multiple sclerosis associated retrovirus
<400> 991
   Ser Pro His Leu Phe Gly Gln Ala Leu
   1               5
<210> 992
   <211> 9
   <212> PRT
   <213> Multiple sclerosis associated retrovirus
<400> 992
   Thr Pro Ile Ala Arg Pro Leu Tyr Thr
   1               5
<210> 993
   <211> 9
   <212> PRT
   <213> Homo sapiens
<400> 993
   Thr Pro Asn Glu Arg Ser Ala Ala Val
   1               5
<210> 994
   <211> 9
   <212> PRT
   <213> Homo sapiens
<400> 994
   Val Pro Cys Leu Arg Lys Glu Leu Ile
   1               5
<210> 995
   <211> 9
   <212> PRT
   <213> Multiple sclerosis associated retrovirus
<400> 995
   Val Pro Pro Glu Ala Lys Met Pro Leu
   1               5
<210> 996
   <211> 9
   <212> PRT
   <213> Multiple sclerosis associated retrovirus
<400> 996
   Trp Pro His Cys Leu Trp Val Met Ala
   1               5
<210> 997
   <211> 11
   <212> PRT
   <213> Multiple sclerosis associated retrovirus
<400> 997
   Tyr Pro Ala Val Ser Ser Pro Tyr Thr Leu Leu
   1               5                   10
<210> 998
   <211> 21
   <212> PRT
   <213> Human T-lymphotropic virus
<400> 998
   Pro Pro Pro Pro Ser Ser Pro Thr His Asp Pro Pro Asp Ser Asp Pro
   1               5                   10                  15
   Gln Ile Pro Pro Pro
               20
<210> 999
   <211> 21
   <212> PRT
   <213> Homo sapiens
<400> 999
   Pro Thr Arg Ala Pro Ser Gly Pro Arg Pro Pro Ser Arg Ser Gln Ala
   1               5                   10                  15
   Gln Thr Pro Pro Arg
               20
<210> 1000
   <211> 12
   <212> PRT
   <213> Homo sapiens
<400> 1000
   Ala Leu Thr Ile Tyr Glu Met Leu Gln Asn Ile Phe
   1 5 10
<210> 1001
   <211> 12
   <212> PRT
   <213> Homo sapiens
<400> 1001
   Cys Ala Trp Thr Ile Val Arg Val Glu Ile Leu Arg
   1 5 10
<210> 1002
   <211> 12
   <212> PRT
   <213> Homo sapiens
<400> 1002
   Cys Leu Lys Asp Arg Met Asn Phe Asp Ile Pro Glu
   1 5 10
<210> 1003
   <211> 15
   <212> PRT
   <213> Helicobacter pylori
<400> 1003
   Glu Glu Phe Ile Phe His Phe Ile Lys Asn Thr Arg Asp Gly Leu
   1 5 10 15
<210> 1004
   <211> 12
   <212> PRT
   <213> Homo sapiens
<400> 1004
   Glu Met Leu Gln Asn Ile Phe Ala Ile Phe Arg Gln
   1 5 10
<210> 1005
   <211> 12
   <212> PRT
   <213> Homo sapiens
<400> 1005
   Glu Thr Ile Val Glu Asn Leu Leu Ala Asn Val Tyr
   1 5 10
<210> 1006
   <211> 12
   <212> PRT
   <213> Homo sapiens
<400> 1006
   Phe Thr Arg Gly Lys Leu Met Ser Ser Leu His Leu
   1               5                   10
<210> 1007
   <211> 12
   <212> PRT
   <213> Homo sapiens
<400> 1007
   Gly Arg Leu Glu Tyr Cys Leu Lys Asp Arg Met Asn
   1               5                   10
<210> 1008
   <211> 12
   <212> PRT
   <213> Homo sapiens
<400> 1008
   His Leu Lys Arg Tyr Tyr Gly Arg Ile Leu His Tyr
   1               5                   10
<210> 1009
   <211> 12
   <212> PRT
   <213> Homo sapiens
<400> 1009
   His Tyr Leu Lys Ala Lys Glu Tyr Ser His Cys Ala
   1               5                   10
<210> 1010
   <211> 12
   <212> PRT
   <213> Homo sapiens
<400> 1010
   Lys Glu Tyr Ser His Cys Ala Trp Thr Ile Val Arg
   1               5                   10
<210> 1011
   <211> 12
   <212> PRT
   <213> Homo sapiens
<400> 1011
   Leu Glu Lys Glu Asp Phe Thr Arg Gly Lys Leu Met
   1               5                   10
<210> 1012
   <211> 12
   <212> PRT
   <213> Homo sapiens
<400> 1012
   Leu Gly Phe Leu Gln Arg Ser Ser Asn Phe Gln Cys
   1 5 10
<210> 1013
   <211> 12
   <212> PRT
   <213> Homo sapiens
<400> 1013
   Leu Met Ser Ser Leu His Leu Lys Arg Tyr Tyr Gly
   1 5 10
<210> 1014
   <211> 12
   <212> PRT
   <213> Homo sapiens
<400> 1014
   Leu Arg Asn Phe Tyr Phe Ile Asn Arg Leu Thr Gly
   1 5 10
<210> 1015
   <211> 12
   <212> PRT
   <213> Homo sapiens
<400> 1015
   Leu Trp Gln Leu Asn Gly Arg Leu Glu Tyr Cys Leu
   1 5 10
<210> 1016
   <211> 12
   <212> PRT
   <213> Homo sapiens
<400> 1016
   Met Asn Phe Asp Ile Pro Glu Glu Ile Lys Gln Leu
   1 5 10
<210> 1017
   <211> 12
   <212> PRT
   <213> Homo sapiens
<400> 1017
   Met Ser Tyr Asn Leu Leu Gly Phe Leu Gln Arg Ser
   1 5 10
<210> 1018
   <211> 12
   <212> PRT
   <213> Homo sapiens
<400> 1018
   Asn His Leu Lys Thr Val Leu Glu Glu Lys Leu Glu
   1 5 10
<210> 1019
   <211> 12
   <212> PRT
   <213> Homo sapiens
<400> 1019
   Asn Leu Leu Ala Asn Val Tyr His Gln Ile Asn His
   1 5 10
<210> 1020
   <211> 12
   <212> PRT
   <213> Homo sapiens
<400> 1020
   Pro Glu Glu Ile Lys Gln Leu Gln Gln Phe Gln Lys
   1 5 10
<210> 1021
   <211> 12
   <212> PRT
   <213> Homo sapiens
<400> 1021
   Gln Cys Gln Lys Leu Leu Trp Gln Leu Asn Gly Arg
   1 5 10
<210> 1022
   <211> 12
   <212> PRT
   <213> Homo sapiens
<400> 1022
   Gln Lys Glu Asp Ala Ala Leu Thr Ile Tyr Glu Met
   1 5 10
<210> 1023
   <211> 12
   <212> PRT
   <213> Homo sapiens
<400> 1023
   Gln Leu Gln Gln Phe Gln Lys Glu Asp Ala Ala Leu
   1 5 10
<210> 1024
   <211> 12
   <212> PRT
   <213> Homo sapiens
<400> 1024
   Arg Gln Asp Ser Ser Ser Thr Gly Trp Asn Glu Thr
   1 5 10
<210> 1025
   <211> 12
   <212> PRT
   <213> Homo sapiens
<400> 1025
   Arg Ser Ser Asn Phe Gln Cys Gln Lys Leu Leu Trp
   1 5 10
<210> 1026
   <211> 12
   <212> PRT
   <213> Homo sapiens
<400> 1026
   Ser Thr Gly Trp Asn Glu Thr Ile Val Glu Asn Leu
   1 5 10
<210> 1027
   <211> 12
   <212> PRT
   <213> Homo sapiens
<400> 1027
   Val Leu Glu Glu Lys Leu Glu Lys Glu Asp Phe Thr
   1 5 10
<210> 1028
   <211> 12
   <212> PRT
   <213> Homo sapiens
<400> 1028
   Val Arg Val Glu Ile Leu Arg Asn Phe Tyr Phe Ile
   1 5 10
<210> 1029
   <211> 12
   <212> PRT
   <213> Homo sapiens
<400> 1029
   Val Tyr His Gln Ile Asn His Leu Lys Thr Val Leu
   1 5 10
<210> 1030
   <211> 12
   <212> PRT
   <213> Homo sapiens
<400> 1030
   Tyr Phe Ile Asn Arg Leu Thr Gly Tyr Leu Arg Asn
   1 5 10
<210> 1031
   <211> 12
   <212> PRT
   <213> Homo sapiens
<400> 1031
   Tyr Gly Arg Ile Leu His Tyr Leu Lys Ala Lys Glu
   1 5 10
<210> 1032
   <211> 3
   <212> PRT
   <213> Unknown
<220>
   <223> epitope involved in multiple sclerosis
<400> 1032
   Phe Phe Lys
   1
<210> 1033
   <211> 10
   <212> PRT
   <213> Unknown
<220>
   <223> epitope involved in multiple sclerosis
<400> 1033
   Leu Ile Met Phe Lys Asn Val Val Ile Lys
   1 5 10
<210> 1034
   <211> 11
   <212> PRT
   <213> Unknown
<220>
   <223> epitope involved in multiple sclerosis
<400> 1034
   Val Ile Ile Phe Phe Lys Asn Val Val Ile Lys
   1 5 10
<210> 1035
   <211> 11
   <212> PRT
   <213> Homo sapiens
<400> 1035
   Val Val His Phe Phe Lys Asn Ile Val Thr Pro
   1 5 10
<210> 1036
   <211> 11
   <212> PRT
   <213> Unknown
<220>
   <223> epitope involved in multiple sclerosis
<400> 1036
   Val Val His Phe Phe Lys Asn Val Val Ile Lys
   1 5 10
<210> 1037
   <211> 11
   <212> PRT
   <213> Unknown
<220>
   <223> epitope involved in multiple sclerosis
<400> 1037
   Val Val His Phe Phe Lys Asn Val Val Ile Pro
   1 5 10
<210> 1038
   <211> 11
   <212> PRT
   <213> Unknown
<220>
   <223> epitope involved in multiple sclerosis
<400> 1038
   Val Val His Phe Phe Lys Asn Val Val Thr Pro
   1 5 10
<210> 1039
   <211> 3
   <212> PRT
   <213> Unknown
<220>
   <223> epitope involved in multiple sclerosis
<400> 1039
   Val Val Ile
   1
<210> 1040
   <211> 11
   <212> PRT
   <213> Unknown
<220>
   <223> epitope involved in multiple sclerosis
<400> 1040
   Val Val Ile Phe Phe Lys Asn Val Val Ile Lys
   1 5 10
<210> 1041
   <211> 7
   <212> PRT
   <213> Unknown
<220>
   <223> epitope involved in multiple sclerosis
<400> 1041
   Glu Arg Pro Asn His Thr Val
   1 5
<210> 1042
   <211> 5
   <212> PRT
   <213> Unknown
<220>
   <223> epitope involved in multiple sclerosis
<400> 1042
   Arg Pro Asn His Thr
   1 5
<210> 1043
   <211> 21
   <212> PRT
   <213> Unknown
<220>
   <223> epitope involved in multiple sclerosis
<400> 1043
   Thr Pro Arg Val Glu Arg Gly Asn His Thr Val Phe Leu Ala Pro Tyr
   1               5                   10                  15
   Gly Trp Met Val Lys
               20
<210> 1044
   <211> 21
   <212> PRT
   <213> Unknown
<220>
   <223> epitope involved in multiple sclerosis
<400> 1044
   Thr Pro Arg Val Glu Arg Asn Gly His Ser Val Phe Leu Ala Pro Tyr
   1               5                   10                  15
   Gly Trp Met Val Lys
               20
<210> 1045
   <211> 21
   <212> PRT
   <213> Unknown
<220>
   <223> epitope involved in multiple sclerosis
<400> 1045
   Thr Pro Arg Val Glu Arg Pro Asn His Thr Val Phe Leu Ala Pro Tyr
   1               5                   10                  15
   Gly Trp Met Val Lys
               20
<210> 1046
   <211> 19
   <212> PRT
   <213> Homo sapiens
<400> 1046
   Ala Asp Asp Leu Lys Lys Leu Lys Pro Gly Leu Glu Lys Asp Phe Leu
   1 5 10 15
   Pro Leu Tyr
<210> 1047
   <211> 19
   <212> PRT
   <213> Homo sapiens
<400> 1047
   Ala Val Gln Thr Gly Leu Asp Leu Leu Glu Ile Leu Arg Gln Glu Lys
   1 5 10 15
   Gly Gly Ser
<210> 1048
   <211> 19
   <212> PRT
   <213> Homo sapiens
<400> 1048
   Asp Asn Leu Pro Arg Gly Ser Arg Ala His Ile Thr Leu Gly Cys Ala
   1 5 10 15
   Ala Asp Val
<210> 1049
   <211> 21
   <212> PRT
   <213> Homo sapiens
<400> 1049 20
<210> 1050
   <211> 18
   <212> PRT
   <213> Homo sapiens
<400> 1050
   Asp Thr Val Ala Thr Leu Leu Glu Cys Lys Thr Leu Phe Ile Leu Arg
   1 5 10 15
   Gly Leu
<210> 1051
   <211> 34
   <212> PRT
   <213> Homo sapiens
<400> 1051
   Asp Val Leu Lys Lys Ser Tyr Ser Lys Ala Phe Thr Leu Thr Ile Ser
   1 5 10 15
   Ala Leu Phe Val Thr Pro Lys Thr Thr Gly Ala Arg Val Glu Leu Ser
   20 25 30
   Glu Gln
<210> 1052
   <211> 19
   <212> PRT
   <213> Homo sapiens
<400> 1052
   Glu Glu Leu Gly Asn His Lys Ala Phe Lys Lys Glu Leu Arg Gln Phe
   1 5 10 15
   Val Pro Gly
<210> 1053
   <211> 18
   <212> PRT
   <213> Homo sapiens
<400> 1053
   Glu Leu Gln Phe Pro Phe Leu Gln Asp Glu Asp Thr Val Ala Thr Leu
   1 5 10 15
   Leu Glu
<210> 1054
   <211> 18
   <212> PRT
   <213> Homo sapiens
<400> 1054
   Glu Leu Ser Glu Gln Gln Leu Gln Leu Trp Pro Ser Asp Val Asp Lys
   1               5                   10                  15
   Leu Ser
<210> 1055
   <211> 19
   <212> PRT
   <213> Homo sapiens
<400> 1055
   Glu Pro Arg Glu Lys Met Asp Leu Val Thr Tyr Phe Gly Lys Arg Pro
   1               5                   10                  15
   Pro Gly Val
<210> 1056
   <211> 39
   <212> PRT
   <213> Homo sapiens
<400> 1056
   Phe Pro Phe Leu Gln Asp Glu Asp Thr Val Ala Thr Leu Leu Glu Cys
   1               5                   10                  15
   Lys Thr Leu Phe Ile Leu Arg Gly Leu Pro Gly Ser Gly Lys Ser Thr
               20                  25                  30
   Leu Ala Arg Val Ile Val Asp
           35
<210> 1057
   <211> 19
   <212> PRT
   <213> Homo sapiens
<400> 1057
   Gly Ala Arg Gly Ala Phe Ser Glu Glu Tyr Lys Arg Leu Asp Glu Asp
   1               5                   10                  15
   Leu Ala Ala
<210> 1058
   <211> 19
   <212> PRT
   <213> Homo sapiens
<400> 1058
   Gly Glu Glu Val Gly Glu Leu Ser Arg Gly Lys Leu Tyr Ser Leu Gly
   1               5                   10                  15
   Asn Gly Arg
<210> 1059
   <211> 21
   <212> PRT
   <213> Homo sapiens
<400> 1059
   Gly Lys Pro Val Pro Thr Gln Gly Ser Arg Lys Gly Gly Ala Leu Gln
   1               5                   10                  15
   Ser Cys Thr Ile Ile
               20
<210> 1060
   <211> 19
   <212> PRT
   <213> Homo sapiens
<400> 1060
   Gly Trp Phe Leu Thr Lys Lys Ser Ser Glu Thr Leu Arg Lys Ala Gly
   1               5                   10                  15
   Gln Val Phe
<210> 1061
   <211> 19
   <212> PRT
   <213> Homo sapiens
<400> 1061
   His Cys Thr Thr Lys Phe Cys Asp Tyr Gly Lys Ala Pro Gly Ala Glu
   1               5                   10                  15
   Glu Tyr Ala
<210> 1062
   <211> 19
   <212> PRT
   <213> Homo sapiens
<400> 1062
   Ile Leu Arg Gln Glu Lys Gly Gly Ser Arg Gly Glu Glu Val Gly Glu
   1               5                   10                  15
   Leu Ser Arg
<210> 1063
   <211> 19
   <212> PRT
   <213> Homo sapiens
<400> 1063
   Ile Thr Leu Gly Cys Ala Ala Asp Val Glu Ala Val Gln Thr Gly Leu
   1 5 10 15
   Asp Leu Leu
<210> 1064
   <211> 20
   <212> PRT
   <213> Homo sapiens
<400> 1064
   Lys Ala Phe Thr Leu Thr Ile Ser Ala Leu Phe Val Thr Pro Lys Thr
   1 5 10 15
   Thr Gly Ala Arg
   20
<210> 1065
   <211> 19
   <212> PRT
   <213> Homo sapiens
<400> 1065
   Lys Ala Pro Gly Ala Glu Glu Tyr Ala Gln Gln Asp Val Leu Lys Lys
   1 5 10 15
   Ser Tyr Ser
<210> 1066
   <211> 19
   <212> PRT
   <213> Mus musculus
<400> 1066
   Lys Glu Lys Arg Gln Phe Val Pro Gly Asp Glu Pro Arg Glu Lys Met
   1 5 10 15
   Asp Leu Val
<210> 1067
   <211> 32
   <212> PRT
   <213> Homo sapiens
<400> 1067
   Lys Lys Leu Lys Pro Gly Leu Glu Lys Asp Phe Leu Pro Leu Tyr Phe
   1 5 10 15
   Gly Trp Phe Leu Thr Lys Lys Ser Ser Glu Thr Leu Arg Lys Ala Gly
   20 25 30
<210> 1068
   <211> 19
   <212> PRT
   <213> Homo sapiens
<400> 1068
   Lys Leu Tyr Ser Leu Gly Asn Gly Arg Trp Met Leu Thr Leu Ala Lys
   1 5 10 15
   Asn Met Glu
<210> 1069
   <211> 19
   <212> PRT
   <213> Homo sapiens
<400> 1069
   Lys Arg Leu Asp Glu Asp Leu Ala Ala Tyr Cys Arg Arg Arg Asp Ile
   1 5 10 15
   Arg Ile Leu
<210> 1070
   <211> 19
   <212> PRT
   <213> Homo sapiens
<400> 1070
   Lys Thr Ala Trp Arg Leu Asp Cys Ala Gln Leu Lys Glu Lys Asn Gln
   1 5 10 15
   Trp Gln Leu
<210> 1071
   <211> 19
   <212> PRT
   <213> Homo sapiens
<400> 1071
   Leu Asp Asp Thr Asn His Glu Arg Glu Arg Leu Glu Gln Leu Phe Glu
   1 5 10 15
   Met Ala Asp
<210> 1072
   <211> 19
   <212> PRT
   <213> Homo sapiens
<400> 1072
   Leu Glu Lys Asp Phe Leu Pro Leu Tyr Phe Gly Trp Phe Leu Thr Lys
   1 5 10 15
   Lys Ser Ser
<210> 1073
   <211> 18
   <212> PRT
   <213> Mus musculus
<400> 1073
   Leu Glu Gln Leu Phe Glu Met Ala Asp Gln Tyr Gln Gln Val Val Leu
   1 5 10 15
   Val Glu
<210> 1074
   <211> 18
   <212> PRT
   <213> Homo sapiens
<400> 1074
   Leu Lys Glu Lys Asn Gln Trp Gln Leu Ser Ala Asp Asp Leu Lys Lys
   1 5 10 15
   Leu Lys
<210> 1075
   <211> 19
   <212> PRT
   <213> Homo sapiens
<400> 1075
   Met Leu Thr Leu Ala Lys Asn Met Glu Val Arg Ala Ile Phe Thr Gly
   1 5 10 15
   Tyr Tyr Gly
<210> 1076
   <211> 19
   <212> PRT
   <213> Homo sapiens
<400> 1076
   Met Ser Ser Ser Gly Ala Lys Asp Lys Pro Glu Leu Gln Phe Pro Phe
   1 5 10 15
   Leu Gln Asp
<210> 1077
   <211> 19
   <212> PRT
   <213> Homo sapiens
<400> 1077
   Pro Ser Asp Val Asp Lys Leu Ser Pro Thr Asp Asn Leu Pro Arg Gly
   1 5 10 15
   Ser Arg Ala
<210> 1078
   <211> 19
   <212> PRT
   <213> Homo sapiens
<400> 1078
   Gln Asp Val Leu Lys Lys Ser Tyr Ser Lys Ala Phe Thr Leu Thr Ile
   1 5 10 15
   Ser Ala Leu
<210> 1079
   <211> 19
   <212> PRT
   <213> Homo sapiens
<400> 1079
   Arg Ala Ile Phe Thr Gly Tyr Tyr Gly Lys Gly Lys Pro Val Pro Thr
   1 5 10 15
   Gln Gly Ser
<210> 1080
   <211> 18
   <212> PRT
   <213> Homo sapiens
<400> 1080
   Arg Arg Arg Asp Ile Arg Ile Leu Val Leu Asp Asp Thr Asn His Glu
   1 5 10 15
   Arg Glu
<210> 1081
   <211> 19
   <212> PRT
   <213> Homo sapiens
<400> 1081
   Ser Gly Lys Ser Thr Leu Ala Arg Val Ile Val Asp Lys Tyr Arg Asp
   1 5 10 15
   Gly Thr Lys
<210> 1082
   <211> 19
   <212> PRT
   <213> Homo sapiens
<400> 1082
   Thr Leu Phe Ile Leu Arg Gly Leu Pro Gly Ser Gly Lys Ser Thr Leu
   1 5 10 15
   Ala Arg Val
<210> 1083
   <211> 19
   <212> PRT
   <213> Homo sapiens
<400> 1083
   Thr Leu Arg Lys Ala Gly Gln Val Phe Leu Glu Glu Leu Gly Asn His
   1 5 10 15
   Lys Ala Phe
<210> 1084
   <211> 19
   <212> PRT
   <213> Homo sapiens
<400> 1084
   Val Asp Lys Tyr Arg Asp Gly Thr Lys Met Val Ser Ala Asp Ala Tyr
   1 5 10 15
   Lys Ile Thr
<210> 1085
   <211> 19
   <212> PRT
   <213> Homo sapiens
<400> 1085
   Val Ser Ala Asp Ala Tyr Lys Ile Thr Pro Gly Ala Arg Gly Ala Phe
   1 5 10 15
   Ser Glu Glu
<210> 1086
   <211> 19
   <212> PRT
   <213> Homo sapiens
<400> 1086
   Val Thr Pro Lys Thr Thr Gly Ala Arg Val Glu Leu Ser Glu Gln Gln
   1 5 10 15
   Leu Gln Leu
<210> 1087
   <211> 19
   <212> PRT
   <213> Homo sapiens
<400> 1087
   Tyr Phe Gly Lys Arg Pro Pro Gly Val Leu His Cys Thr Thr Lys Phe
   1 5 10 15
   Cys Asp Tyr
<210> 1088
   <211> 16
   <212> PRT
   <213> Homo sapiens
<400> 1088
   Ala Ala Met Glu Leu Lys Val Glu Asp Pro Phe Tyr Trp Val Ser Pro
   1 5 10 15
<210> 1089
   <211> 9
   <212> PRT
   <213> Homo sapiens
<400> 1089
   Cys Leu Gln Tyr Arg Leu Arg Gly Lys
   1 5
<210> 1090
   <211> 16
   <212> PRT
   <213> Homo sapiens
<400> 1090
   Cys Trp Lys Ile Thr Leu Phe Val Ile Val Pro Val Leu Gly Pro Leu
   1               5                   10                  15
<210> 1091
   <211> 16
   <212> PRT
   <213> Homo sapiens
<400> 1091
   Asp Pro Phe Tyr Trp Val Ser Pro Gly Val Leu Val Leu Leu Ala Val
   1               5                   10                  15
<210> 1092
   <211> 16
   <212> PRT
   <213> Homo sapiens
<400> 1092
   Asp Pro His Phe Leu Arg Val Pro Cys Trp Lys Ile Thr Leu Phe Val
   1               5                   10                  15
<210> 1093
   <211> 16
   <212> PRT
   <213> Homo sapiens
<400> 1093
   Glu Gly Gly Phe Thr Cys Phe Phe Arg Asp His Ser Tyr Gln Glu Glu
   1               5                   10                  15
<210> 1094
   <211> 16
   <212> PRT
   <213> Homo sapiens
<400> 1094
   Glu Leu Pro Cys Arg Ile Ser Pro Gly Lys Asn Ala Thr Gly Met Glu
   1               5                   10                  15
<210> 1095
   <211> 17
   <212> PRT
   <213> Homo sapiens
<400> 1095
   Glu Asn Pro Val Val His Phe Phe Lys Asn Ile Val Thr Pro Arg Thr
   1 5 10 15
   Pro
<210> 1096
   <211> 9
   <212> PRT
   <213> Homo sapiens
<400> 1096
   Phe Leu Cys Leu Gln Tyr Arg Leu Arg
   1 5
<210> 1097
   <211> 16
   <212> PRT
   <213> Homo sapiens
<400> 1097
   Gly Asp Glu Val Glu Leu Pro Cys Arg Ile Ser Pro Gly Lys Asn Ala
   1 5 10 15
<210> 1098
   <211> 16
   <212> PRT
   <213> Homo sapiens
<400> 1098
   Gly Glu Gly Lys Val Thr Leu Arg Ile Arg Asn Val Arg Phe Ser Asp
   1 5 10 15
<210> 1099
   <211> 16
   <212> PRT
   <213> Homo sapiens
<400> 1099
   Gly Lys Asn Ala Thr Gly Met Glu Val Gly Trp Tyr Arg Pro Pro Phe
   1 5 10 15
<210> 1100
   <211> 9
   <212> PRT
   <213> Homo sapiens
<400> 1100
   Gly Leu Val Phe Leu Cys Leu Gln Tyr
   1 5
<210> 1101
   <211> 16
   <212> PRT
   <213> Homo sapiens
<400> 1101
   Gly Gln Phe Arg Val Ile Gly Pro Arg His Pro Ile Arg Ala Leu Val
   1 5 10 15
<210> 1102
   <211> 16
   <212> PRT
   <213> Homo sapiens
<400> 1102
   Gly Val Leu Val Leu Leu Ala Val Leu Pro Val Leu Leu Leu Gln Ile
   1 5 10 15
<210> 1103
   <211> 16
   <212> PRT
   <213> Homo sapiens
<400> 1103
   His Leu Tyr Arg Asn Gly Lys Asp Gln Asp Gly Asp Gln Ala Pro Glu
   1 5 10 15
<210> 1104
   <211> 16
   <212> PRT
   <213> Homo sapiens
<400> 1104
   His Arg Thr Phe Asp Pro His Phe Leu Arg Val Pro Cys Trp Lys Ile
   1 5 10 15
<210> 1105
   <211> 16
   <212> PRT
   <213> Homo sapiens
<400> 1105
   Ile Cys Tyr Asn Trp Leu His Arg Arg Leu Ala Gly Gln Phe Leu Glu
   1 5 10 15
<210> 1106
   <211> 16
   <212> PRT
   <213> Homo sapiens
<400> 1106
   Ile Glu Asn Leu His Arg Thr Phe Asp Pro His Phe Leu Arg Val Pro
   1 5 10 15
<210> 1107
   <211> 16
   <212> PRT
   <213> Homo sapiens
<400> 1107
   Ile Arg Asn Val Arg Phe Ser Asp Glu Gly Gly Phe Thr Cys Phe Phe
   1               5                   10                  15
<210> 1108
   <211> 16
   <212> PRT
   <213> Homo sapiens
<400> 1108
   Ile Val Pro Val Leu Gly Pro Leu Val Ala Leu Ile Ile Cys Tyr Asn
   1               5                   10                  15
<210> 1109
   <211> 16
   <212> PRT
   <213> Homo sapiens
<400> 1109
   Lys Asp Ala Ile Gly Glu Gly Lys Val Thr Leu Arg Ile Arg Asn Val
   1               5                   10                  15
<210> 1110
   <211> 21
   <212> PRT
   <213> Homo sapiens
<400> 1110
   Lys Asn Ala Thr Gly Met Glu Val Gly Trp Tyr Arg Pro Pro Phe Ser
   1               5                   10                  15
   Arg Val Val His Leu
               20
<210> 1111
   <211> 21
   <212> PRT
   <213> Homo sapiens
<400> 1111
   Lys Asn Ala Thr Gly Met Glu Val Gly Trp Tyr Arg Pro Pro Phe Ser
   1               5                   10                  15
   Arg Val Val His Leu
               20
<210> 1112
   <211> 9
   <212> PRT
   <213> Homo sapiens
<400> 1112
   Leu Cys Leu Gln Tyr Arg Leu Arg Gly
   1               5
<210> 1113
   <211> 16
   <212> PRT
   <213> Homo sapiens
<400> 1113
   Leu Gly Pro Leu Val Ala Leu Ile Ile Cys Tyr Asn Trp Leu His Arg
   1               5                   10                  15
<210> 1114
   <211> 16
   <212> PRT
   <213> Homo sapiens
<400> 1114
   Leu Lys Val Glu Asp Pro Phe Tyr Trp Val Ser Pro Gly Val Leu Val
   1               5                   10                  15
<210> 1115
   <211> 16
   <212> PRT
   <213> Homo sapiens
<400> 1115
   Leu Leu Ala Val Leu Pro Val Leu Leu Leu Gln Ile Thr Val Gly Leu
   1               5                   10                  15
<210> 1116
   <211> 16
   <212> PRT
   <213> Homo sapiens
<400> 1116
   Leu Leu Gln Ile Thr Val Gly Leu Val Phe Leu Cys Leu Gln Tyr Arg
   1               5                   10                  15
<210> 1117
   <211> 16
   <212> PRT
   <213> Homo sapiens
<400> 1117
   Leu Pro Val Leu Leu Leu Gln Ile Thr Val Gly Leu Val Phe Leu Cys
   1 5 10 15
<210> 1118
   <211> 16
   <212> PRT
   <213> Homo sapiens
<400> 1118
   Leu Gln Tyr Arg Leu Arg Gly Lys Leu Arg Ala Glu Ile Glu Asn Leu
   1 5 10 15
<210> 1119
   <211> 16
   <212> PRT
   <213> Homo sapiens
<400> 1119
   Leu Arg Ala Glu Ile Glu Asn Leu His Arg Thr Phe Asp Pro His Phe
   1 5 10 15
<210> 1120
   <211> 16
   <212> PRT
   <213> Homo sapiens
<400> 1120
   Leu Arg Gly Lys Leu Arg Ala Glu Ile Glu Asn Leu His Arg Thr Phe
   1 5 10 15
<210> 1121
   <211> 16
   <212> PRT
   <213> Homo sapiens
<400> 1121
   Leu Arg Val Pro Cys Trp Lys Ile Thr Leu Phe Val Ile Val Pro Val
   1 5 10 15
<210> 1122
   <211> 9
   <212> PRT
   <213> Homo sapiens
<400> 1122
   Leu Val Phe Leu Cys Leu Gln Tyr Arg
   1 5
<210> 1123
   <211> 16
   <212> PRT
   <213> Homo sapiens
<400> 1123
   Asn Gly Lys Asp Gln Asp Gly Asp Gln Ala Pro Glu Tyr Arg Gly Arg
   1 5 10 15
<210> 1124
   <211> 16
   <212> PRT
   <213> Homo sapiens
<400> 1124
   Gln Ala Pro Glu Tyr Arg Gly Arg Thr Glu Leu Leu Lys Asp Ala Ile
   1 5 10 15
<210> 1125
   <211> 16
   <212> PRT
   <213> Homo sapiens
<400> 1125
   Gln Asp Gly Asp Gln Ala Pro Glu Tyr Arg Gly Arg Thr Glu Leu Leu
   1 5 10 15
<210> 1126
   <211> 9
   <212> PRT
   <213> Homo sapiens
<400> 1126
   Gln Tyr Arg Leu Arg Gly Lys Leu Arg
   1 5
<210> 1127
   <211> 16
   <212> PRT
   <213> Homo sapiens
<400> 1127
   Arg Ala Leu Val Gly Asp Glu Val Glu Leu Pro Cys Arg Ile Ser Pro
   1 5 10 15
<210> 1128
   <211> 16
   <212> PRT
   <213> Homo sapiens
<400> 1128
   Arg Asp His Ser Tyr Gln Glu Glu Ala Ala Met Glu Leu Lys Val Glu
   1 5 10 15
<210> 1129
   <211> 16
   <212> PRT
   <213> Homo sapiens
<400> 1129
   Arg Phe Ser Asp Glu Gly Gly Phe Thr Cys Phe Phe Arg Asp His Ser
   1 5 10 15
<210> 1130
   <211> 16
   <212> PRT
   <213> Homo sapiens
<400> 1130
   Arg His Pro Ile Arg Ala Leu Val Gly Asp Glu Val Glu Leu Pro Cys
   1 5 10 15
<210> 1131
   <211> 16
   <212> PRT
   <213> Homo sapiens
<400> 1131
   Arg Ile Ser Pro Gly Lys Asn Ala Thr Gly Met Glu Val Gly Trp Tyr
   1 5 10 15
<210> 1132
   <211> 14
   <212> PRT
   <213> Homo sapiens
<400> 1132
   Arg Leu Ala Gly Gln Phe Leu Glu Glu Leu Arg Asn Pro Phe
   1 5 10
<210> 1133
   <211> 9
   <212> PRT
   <213> Homo sapiens
<400> 1133
   Arg Leu Arg Gly Lys Leu Arg Ala Glu
   1 5
<210> 1134
   <211> 16
   <212> PRT
   <213> Homo sapiens
<400> 1134
   Arg Pro Pro Phe Ser Arg Val Val His Leu Tyr Arg Asn Gly Lys Asp
   1               5                   10                  15
<210> 1135
   <211> 16
   <212> PRT
   <213> Homo sapiens
<400> 1135
   Ser Arg Val Val His Leu Tyr Arg Asn Gly Lys Asp Gln Asp Gly Asp
   1               5                   10                  15
<210> 1136
   <211> 16
   <212> PRT
   <213> Homo sapiens
<400> 1136
   Thr Cys Phe Phe Arg Asp His Ser Tyr Gln Glu Glu Ala Ala Met Glu
   1               5                   10                  15
<210> 1137
   <211> 16
   <212> PRT
   <213> Homo sapiens
<400> 1137
   Thr Glu Leu Leu Lys Asp Ala Ile Gly Glu Gly Lys Val Thr Leu Arg
   1               5                   10                  15
<210> 1138
   <211> 16
   <212> PRT
   <213> Homo sapiens
<400> 1138
   Thr Gly Met Glu Val Gly Trp Tyr Arg Pro Pro Phe Ser Arg Val Val
   1               5                   10                  15
<210> 1139
   <211> 16
   <212> PRT
   <213> Homo sapiens
<400> 1139
   Thr Leu Phe Val Ile Val Pro Val Leu Gly Pro Leu Val Ala Leu Ile
   1               5                   10                  15
<210> 1140
   <211> 21
   <212> PRT
   <213> Unknown
<220>
   <223> epitope involved in multiple sclerosis
<400> 1140
   Thr Pro Arg Val Glu Arg Asn Gly His Ser Val Phe Leu Ala Pro Tyr
   1 5 10 15
   Gly Trp Met Val Lys
   20
<210> 1141
   <211> 21
   <212> PRT
   <213> Unknown
<220>
   <223> epitope involved in multiple sclerosis
<400> 1141
   Thr Pro Arg Val Glu Arg Asn Gly His Ser Val Phe Leu Ala Pro Tyr
   1 5 10 15
   Gly Trp Met Val Lys
   20
<210> 1142
   <211> 16
   <212> PRT
   <213> Homo sapiens
<400> 1142
   Thr Val Gly Leu Val Phe Leu Cys Leu Gln Tyr Arg Leu Arg Gly Lys
   1 5 10 15
<210> 1143
   <211> 16
   <212> PRT
   <213> Homo sapiens
<400> 1143
   Val Ala Leu Ile Ile Cys Tyr Asn Trp Leu His Arg Arg Leu Ala Gly
   1 5 10 15
<210> 1144
   <211> 9
   <212> PRT
   <213> Homo sapiens
<400> 1144
   Val Phe Leu Cys Leu Gln Tyr Arg Leu
   1 5
<210> 1145
   <211> 9
   <212> PRT
   <213> Homo sapiens
<400> 1145
   Val Gly Leu Val Phe Leu Cys Leu Gln
   1 5
<210> 1146
   <211> 16
   <212> PRT
   <213> Homo sapiens
<400> 1146
   Val Gly Trp Tyr Arg Pro Pro Phe Ser Arg Val Val His Leu Tyr Arg
   1 5 10 15
<210> 1147
   <211> 16
   <212> PRT
   <213> Homo sapiens
<400> 1147
   Val Ile Gly Pro Arg His Pro Ile Arg Ala Leu Val Gly Asp Glu Val
   1 5 10 15
<210> 1148
   <211> 16
   <212> PRT
   <213> Homo sapiens
<400> 1148
   Val Thr Leu Arg Ile Arg Asn Val Arg Phe Ser Asp Glu Gly Gly Phe
   1 5 10 15
<210> 1149
   <211> 16
   <212> PRT
   <213> Homo sapiens
<400> 1149
   Trp Leu His Arg Arg Leu Ala Gly Gln Phe Leu Glu Glu Leu Arg Asn
   1 5 10 15
<210> 1150
   <211> 16
   <212> PRT
   <213> Homo sapiens
<400> 1150
   Trp Val Ser Pro Gly Val Leu Val Leu Leu Ala Val Leu Pro Val Leu
   1 5 10 15
<210> 1151
   <211> 16
   <212> PRT
   <213> Homo sapiens
<400> 1151
   Tyr Gln Glu Glu Ala Ala Met Glu Leu Lys Val Glu Asp Pro Phe Tyr
   1 5 10 15
<210> 1152
   <211> 16
   <212> PRT
   <213> Homo sapiens
<400> 1152
   Tyr Arg Gly Arg Thr Glu Leu Leu Lys Asp Ala Ile Gly Glu Gly Lys
   1 5 10 15
<210> 1153
   <211> 9
   <212> PRT
   <213> Homo sapiens
<400> 1153
   Tyr Arg Leu Arg Gly Lys Leu Arg Ala
   1 5
<210> 1154
   <211> 21
   <212> PRT
   <213> Human herpesvirus
<400> 1154
   Ala Gly Ala Gly Gly Gly Ala Gly Gly Ala Gly Ala Gly Gly Gly Ala
   1 5 10 15
   Gly Gly Ala Gly Gly
   20
<210> 1155
   <211> 28
   <212> PRT
   <213> Human herpesvirus
<400> 1155
   Asp Gly Glu Pro Asp Val Pro Pro Gly Ala Ile Glu Gln Gly Pro Ala
   1               5                   10                  15
   Asp Asp Pro Gly Glu Gly Pro Ser Thr Gly Pro Arg
               20                  25
<210> 1156
   <211> 22
   <212> PRT
   <213> Human herpesvirus
<400> 1156
   Gly Gly Ser Gly Gly Arg Arg Gly Arg Gly Arg Glu Arg Ala Arg Gly
   1               5                   10                  15
   Gly Ser Arg Glu Arg Ala
               20
<210> 1157
   <211> 26
   <212> PRT
   <213> Human herpesvirus
<400> 1157
   Gly Val Phe Val Tyr Gly Gly Ser Lys Thr Ser Leu Tyr Asn Leu Arg
   1               5                   10                  15
   Arg Gly Thr Ala Leu Ala Ile Pro Gln Cys
               20                  25
<210> 1158
   <211> 26
   <212> PRT
   <213> Human herpesvirus
<400> 1158
   Lys Phe Glu Asn Ile Ala Glu Gly Leu Arg Ala Leu Leu Ala Arg Ser
   1               5                   10                  15
   His Val Glu Arg Thr Thr Asp Glu Gly Thr
               20                  25
<210> 1159
   <211> 27
   <212> PRT
   <213> Human herpesvirus
<400> 1159
   Lys Lys Gly Gly Trp Phe Gly Lys His Arg Gly Gln Gly Gly Ser Asn
   1               5                   10                  15
   Pro Lys Phe Glu Asn Ile Ala Glu Gly Leu Arg
               20                  25
<210> 1160
   <211> 44
   <212> PRT
   <213> Human herpesvirus
<400> 1160
   Met Ser Asp Glu Gly Pro Gly Thr Gly Pro Gly Asn Gly Leu Gly Glu
   1               5                   10                  15
   Lys Gly Asp Thr Ser Gly Pro Glu Gly Ser Gly Gly Ser Gly Pro Gln
               20                  25                  30
   Arg Arg Gly Gly Asp Asn His Gly Arg Gly Arg Gly
           35                  40
<210> 1161
   <211> 27
   <212> PRT
   <213> Human herpesvirus
<400> 1161
   Pro Pro Arg Arg Pro Pro Pro Gly Arg Arg Pro Phe Phe His Pro Val
   1               5                   10                  15
   Gly Glu Ala Asp Tyr Phe Glu Tyr His Gln Glu
               20                  25
<210> 1162
   <211> 28
   <212> PRT
   <213> Human herpesvirus
<400> 1162
   Pro Arg Gly Gln Gly Asp Gly Gly Arg Arg Lys Lys Gly Gly Trp Phe
   1               5                   10                  15
   Gly Lys His Arg Gly Gln Gly Gly Ser Asn Pro Lys
               20                  25
<210> 1163
   <211> 26
   <212> PRT
   <213> Human herpesvirus
<400> 1163
   Gln Gly Pro Ala Asp Asp Pro Gly Glu Gly Pro Ser Thr Gly Pro Arg
   1               5                   10                  15
   Gly Gln Gly Asp Gly Gly Arg Arg Lys Lys
               20                  25
<210> 1164
   <211> 20
   <212> PRT
   <213> Human herpesvirus
<400> 1164
   Arg Glu Arg Ala Arg Gly Gly Ser Arg Glu Arg Ala Arg Gly Arg Gly
   1               5                   10                  15
   Arg Gly Arg Gly
               20
<210> 1165
   <211> 149
   <212> PRT
   <213> Human herpesvirus
<400> 1165
   Arg Lys Lys Gly Gly Trp Phe Gly Lys His Arg Gly Gln Gly Gly Ser
   1               5                   10                  15
   Asn Pro Lys Phe Glu Asn Ile Ala Glu Gly Leu Arg Ala Leu Leu Ala
   20 25 30
   Arg Ser His Val Glu Arg Thr Thr Asp Glu Gly Thr Trp Val Ala Gly
   35 40 45
   Val Phe Val Tyr Gly Gly Ser Lys Thr Ser Leu Tyr Asn Leu Arg Arg
   50 55 60
   Gly Thr Ala Leu Ala Ile Pro Gln Cys Arg Leu Thr Pro Leu Ser Arg
   65 70 75 80
   Leu Pro Phe Gly Met Ala Pro Gly Pro Gly Pro Gln Pro Gly Pro Leu
   85 90 95
   Arg Glu Ser Ile Val Cys Tyr Phe Met Val Phe Leu Gln Thr His Ile
   100 105 110
   Phe Ala Glu Val Leu Lys Asp Ala Ile Lys Asp Leu Val Met Thr Lys
   115 120 125
   Pro Ala Pro Thr Cys Asn Ile Arg Val Thr Val Cys Ser Phe Asp Asp
   130 135 140
   Gly Val Asp Leu Pro
   145
<210> 1166
   <211> 28
   <212> PRT
   <213> Human herpesvirus
<400> 1166
   Val Glu Gly Ala Ala Ala Glu Gly Asp Asp Gly Asp Asp Gly Asp Glu
   1 5 10 15
   Gly Gly Asp Gly Asp Glu Gly Glu Glu Gly Gln Glu
   20 25
<210> 1167
   <211> 20
   <212> PRT
   <213> Homo sapiens
<400> 1167
   Ala Val Ile Met Gly Asn Trp Glu Asn His Trp Ile Tyr Trp Val Gly
   1 5 10 15
   Pro Ile Ile Gly
   20
<210> 1168
   <211> 20
   <212> PRT
   <213> Homo sapiens
<400> 1168
   Ala Val Leu Ala Gly Gly Leu Tyr Glu Tyr Val Phe Cys Pro Asp Val
   1 5 10 15
   Glu Phe Lys Arg
   20
<210> 1169
   <211> 20
   <212> PRT
   <213> Homo sapiens
<400> 1169
   Asp Leu Ile Leu Lys Pro Gly Val Val His Val Ile Asp Val Asp Arg
   1 5 10 15
   Gly Glu Glu Lys
   20
<210> 1170
   <211> 20
   <212> PRT
   <213> Homo sapiens
<400> 1170
   Glu Asp Asn Arg Ser Gln Val Glu Thr Asp Asp Leu Ile Leu Lys Pro
   1 5 10 15
   Gly Val Val His
   20
<210> 1171
   <211> 20
   <212> PRT
   <213> Homo sapiens
<400> 1171
   Gly Ile Leu Tyr Leu Val Thr Pro Pro Ser Val Val Gly Gly Leu Gly
   1               5                   10                  15
   Val Thr Met Val
               20
<210> 1172
   <211> 20
   <212> PRT
   <213> Homo sapiens
<400> 1172
   Gly Lys Cys Gly Pro Leu Cys Thr Arg Glu Asn Ile Met Val Ala Phe
   1               5                   10                  15
   Lys Gly Val Trp
               20
<210> 1173
   <211> 20
   <212> PRT
   <213> Homo sapiens
<400> 1173
   Gly Thr Glu Lys Pro Leu Pro Val Asp Met Val Leu Ile Ser Leu Cys
   1               5                   10                  15
   Phe Gly Leu Ser
               20
<210> 1174
   <211> 20
   <212> PRT
   <213> Homo sapiens
<400> 1174
   His Gly Asn Leu Thr Ala Gly His Gly Leu Leu Val Glu Leu Ile Ile
   1               5                   10                  15
   Thr Phe Gln Leu
               20
<210> 1175
   <211> 20
   <212> PRT
   <213> Homo sapiens
<400> 1175
   His Leu Phe Ala Ile Asn Tyr Thr Gly Ala Ser Met Asn Pro Ala Arg
   1               5                   10                  15
   Ser Phe Gly Pro
               20
<210> 1176
   <211> 20
   <212> PRT
   <213> Homo sapiens
<400> 1176
   Ile Ala Thr Met Val Gln Cys Phe Gly His Ile Ser Gly Gly His Ile
   1               5                   10                  15
   Asn Pro Ala Val
               20
<210> 1177
   <211> 20
   <212> PRT
   <213> Homo sapiens
<400> 1177
   Ile Ser Gly Gly His Ile Asn Pro Ala Val Thr Val Ala Met Val Cys
   1               5                   10                  15
   Thr Arg Lys Ile
               20
<210> 1178
   <211> 20
   <212> PRT
   <213> Homo sapiens
<400> 1178
   Lys Arg Thr Asp Val Thr Gly Ser Ile Ala Leu Ala Ile Gly Phe Ser
   1               5                   10                  15
   Val Ala Ile Gly
               20
<210> 1179
   <211> 20
   <212> PRT
   <213> Homo sapiens
<400> 1179
   Leu Ser Leu Gly Ser Thr Ile Asn Trp Gly Gly Thr Glu Lys Pro Leu
   1               5                   10                  15
   Pro Val Asp Met
               20
<210> 1180
   <211> 20
   <212> PRT
   <213> Homo sapiens
<400> 1180
   Met Ser Asp Arg Pro Thr Ala Arg Arg Trp Gly Lys Cys Gly Pro Leu
   1               5                   10                  15
   Cys Thr Arg Glu
               20
<210> 1181
   <211> 20
   <212> PRT
   <213> Homo sapiens
<400> 1181
   Asn Ile Met Val Ala Phe Lys Gly Val Trp Thr Gln Ala Phe Trp Lys
   1               5                   10                  15
   Ala Val Thr Ala
               20
<210> 1182
   <211> 20
   <212> PRT
   <213> Homo sapiens
<400> 1182
   Gln Gln Thr Lys Gly Ser Tyr Met Glu Val Glu Asp Asn Arg Ser Gln
   1               5                   10                  15
   Val Glu Thr Asp
               20
<210> 1183
   <211> 20
   <212> PRT
   <213> Homo sapiens
<400> 1183
   Arg Phe Lys Glu Ala Phe Ser Lys Ala Ala Gln Gln Thr Lys Gly Ser
   1               5                   10                  15
   Tyr Met Glu Val
               20
<210> 1184
   <211> 20
   <212> PRT
   <213> Homo sapiens
<400> 1184
   Ser Ile Ala Lys Ser Val Phe Tyr Ile Ala Ala Gln Cys Leu Gly Ala
   1               5                   10                  15
   Ile Ile Gly Ala
               20
<210> 1185
   <211> 20
   <212> PRT
   <213> Homo sapiens
<400> 1185
   Ser Met Asn Pro Ala Arg Ser Phe Gly Pro Ala Val Ile Met Gly Asn
   1               5                   10                  15
   Trp Glu Asn His
               20
<210> 1186
   <211> 20
   <212> PRT
   <213> Homo sapiens
<400> 1186
   Thr Gln Ala Phe Trp Lys Ala Val Thr Ala Glu Phe Leu Ala Met Leu
   1               5                   10                  15
   Ile Phe Val Leu
               20
<210> 1187
   <211> 20
   <212> PRT
   <213> Homo sapiens
<400> 1187
   Thr Val Ala Met Val Cys Thr Arg Lys Ile Ser Ile Ala Lys Ser Val
   1 5 10 15
   Phe Tyr Ile Ala
   20
<210> 1188
   <211> 20
   <212> PRT
   <213> Homo sapiens
<400> 1188
   Val Phe Cys Pro Asp Val Glu Phe Lys Arg Arg Phe Lys Glu Ala Phe
   1 5 10 15
   Ser Lys Ala Ala
   20
<210> 1189
   <211> 20
   <212> PRT
   <213> Homo sapiens
<400> 1189
   Val Phe Thr Ile Phe Ala Ser Cys Asp Ser Lys Arg Thr Asp Val Thr
   1 5 10 15
   Gly Ser Ile Ala
   20
<210> 1190
   <211> 20
   <212> PRT
   <213> Homo sapiens
<400> 1190
   Val Val Gly Gly Leu Gly Val Thr Met Val His Gly Asn Leu Thr Ala
   1 5 10 15
   Gly His Gly Leu
   20
<210> 1191
   <211> 20
   <212> PRT
   <213> Homo sapiens
<400> 1191
   Trp Ile Tyr Trp Val Gly Pro Ile Ile Gly Ala Val Leu Ala Gly Gly
   1 5 10 15
   Leu Tyr Glu Tyr
   20
<210> 1192
   <211> 19
   <212> PRT
   <213> Homo sapiens
<400> 1192
   Asn Ala Thr Gly Met Glu Val Gly Trp Tyr Arg Pro Pro Phe Ser Arg
   1 5 10 15
   Val Val His
<210> 1193
   <211> 12
   <212> PRT
   <213> Homo sapiens
<400> 1193
   Arg Ala Leu Val Gly Asp Glu Val Glu Leu Pro Cys
   1 5 10
<210> 1194
   <211> 21
   <212> PRT
   <213> Unknown
<220>
   <223> epitope involved in multiple sclerosis
<400> 1194
   Thr Pro Arg Val Glu Arg Phe Gly His Ser Val Phe Leu Ala Pro Tyr
   1 5 10 15
   Gly Trp Met Val Lys
   20
<210> 1195
   <211> 21
   <212> PRT
   <213> Unknown
<220>
   <223> epitope involved in multiple sclerosis
<400> 1195
   Thr Pro Arg Val Glu Arg Gly Gly His Ser Val Phe Leu Ala Pro Tyr
   1 5 10 15
   Gly Trp Met Val Lys
   20
<210> 1196
   <211> 21
   <212> PRT
   <213> Unknown
<220>
   <223> epitope involved in multiple sclerosis
<400> 1196
   Thr Pro Arg Val Glu Arg Gln Gly His Ser Val Phe Leu Ala Pro Tyr
   1 5 10 15
   Gly Trp Met Val Lys
   20
<210> 1197
   <211> 15
   <212> PRT
   <213> Homo sapiens
<400> 1197
   Glu Lys Lys Gly Lys Asp Gln Ser Gly Glu Val Leu Ser Ser Val
   1 5 10 15
<210> 1198
   <211> 24
   <212> PRT
   <213> Homo sapiens
<400> 1198
   Phe Cys Pro Asp Val Glu Phe Lys Arg Arg Phe Lys Glu Ala Phe Ser
   1 5 10 15
   Lys Ala Ala Gln Gln Thr Lys Gly
   20
<210> 1199
   <211> 12
   <212> PRT
   <213> Unknown
<220>
   <223> epitope involved in multiple sclerosis
<400> 1199
   Asp Pro Gly Gln Leu Arg Leu Trp Arg Leu Ala Ser
   1 5 10
<210> 1200
   <211> 12
   <212> PRT
   <213> Unknown
<220>
   <223> epitope involved in multiple sclerosis
<400> 1200
   Phe His Gln Pro Thr Tyr Asp Tyr Thr Val Ser Arg
   1 5 10
<210> 1201
   <211> 12
   <212> PRT
   <213> Unknown
<220>
   <223> epitope involved in multiple sclerosis
<400> 1201
   Phe Ser Lys Thr Glu Pro Leu Ser Pro Ser Trp Phe
   1 5 10
<210> 1202
   <211> 12
   <212> PRT
   <213> Unknown
<220>
   <223> epitope involved in multiple sclerosis
<400> 1202
   Gly Thr Trp Ile Cys Asp Phe Lys Ser Cys Ser Trp
   1 5 10
<210> 1203
   <211> 12
   <212> PRT
   <213> Unknown
<220>
   <223> epitope involved in multiple sclerosis
<400> 1203
   His Ile Asp Val Ser Arg Pro Trp Arg Val Thr Gly
   1 5 10
<210> 1204
   <211> 12
   <212> PRT
   <213> Unknown
<220>
   <223> epitope involved in multiple sclerosis
<400> 1204
   His Trp Arg His Trp Leu Ala Asp Thr Ala Phe Pro
   1 5 10
<210> 1205
   <211> 12
   <212> PRT
   <213> Unknown
<220>
   <223> epitope involved in multiple sclerosis
<400> 1205
   His Tyr Asn Pro Trp Glu Gln Glu Pro Met Met Tyr
   1 5 10
<210> 1206
   <211> 12
   <212> PRT
   <213> Unknown
<220>
   <223> epitope involved in multiple sclerosis
<400> 1206
   Asn Ala Leu Thr Glu Ser Lys Tyr Val Lys Leu Leu
   1 5 10
<210> 1207
   <211> 12
   <212> PRT
   <213> Unknown
<220>
   <223> epitope involved in multiple sclerosis
<400> 1207
   Asn Cys Ala Phe Trp Leu Gly Lys Leu Tyr Cys Arg
   1 5 10
<210> 1208
   <211> 12
   <212> PRT
   <213> Homo sapiens
<400> 1208
   Asn Cys Ser Trp Cys His Val Leu Gln Arg Cys Ser
   1 5 10
<210> 1209
   <211> 10
   <212> PRT
   <213> Prochlorococcus marinus
<400> 1209
   Asn Cys Trp Lys Pro Gln Val Leu His Cys
   1 5 10
<210> 1210
   <211> 12
   <212> PRT
   <213> Unknown
<220>
   <223> epitope involved in multiple sclerosis
<400> 1210
   Asn Asn Leu Thr Gln Ser Lys Phe Leu Arg Leu Gln
   1 5 10
<210> 1211
   <211> 12
   <212> PRT
   <213> Unknown
<220>
   <223> epitope involved in multiple sclerosis
<400> 1211
   Asn Asn Pro Ala Pro Val Tyr Thr His Arg Val Leu
   1 5 10
<210> 1212
   <211> 12
   <212> PRT
   <213> Unknown
<220>
   <223> epitope involved in multiple sclerosis
<400> 1212
   Asn Pro Val Glu His Trp Leu Ala Val Leu Pro Thr
   1 5 10
<210> 1213
   <211> 12
   <212> PRT
   <213> Unknown
<220>
   <223> epitope involved in multiple sclerosis
<400> 1213
   Asn Tyr Pro Ser Trp Leu His Ala Tyr Arg Leu Ala
   1               5                   10
<210> 1214
   <211> 12
   <212> PRT
   <213> Unknown
<220>
   <223> epitope involved in multiple sclerosis
<400> 1214
   Gln His Asn Val Ser Arg Pro Trp Val Leu Phe Thr
   1               5                   10
<210> 1215
   <211> 12
   <212> PRT
   <213> Unknown
<220>
   <223> epitope involved in multiple sclerosis
<400> 1215
   Ser Cys Asn Tyr His Gly Arg Thr Leu Thr Cys Trp
   1               5                   10
<210> 1216
   <211> 12
   <212> PRT
   <213> Unknown
<220>
   <223> epitope involved in multiple sclerosis
<400> 1216
   Ser Cys Asn Tyr His His Thr Tyr Leu Thr Cys Trp
   1               5                   10
<210> 1217
   <211> 12
   <212> PRT
   <213> Unknown
<220>
   <223> epitope involved in multiple sclerosis
<400> 1217
   Ser Cys Gln Leu Val Arg Thr Ile Trp Ser Cys Ser
   1               5                   10
<210> 1218
   <211> 12
   <212> PRT
   <213> Unknown
<220>
   <223> epitope involved in multiple sclerosis
<400> 1218
   Ser Leu Gly Ser Lys Met Asp Ile Ser Arg Pro Trp
   1 5 10
<210> 1219
   <211> 12
   <212> PRT
   <213> Unknown
<220>
   <223> epitope involved in multiple sclerosis
<400> 1219
   Ser Thr Leu Ser Glu Ser Lys Val Asn Arg Leu Leu
   1 5 10
<210> 1220
   <211> 12
   <212> PRT
   <213> Unknown
<220>
   <223> epitope involved in multiple sclerosis
<400> 1220
   Thr Ala Gln Asp Ile Ser Arg Pro Trp Trp Phe Pro
   1 5 10
<210> 1221
   <211> 12
   <212> PRT
   <213> Unknown
<220>
   <223> epitope involved in multiple sclerosis
<400> 1221
   Thr Ile Met Asp Ile Ser Arg Thr Trp Thr Lys Val
   1 5 10
<210> 1222
   <211> 12
   <212> PRT
   <213> Unknown
<220>
   <223> epitope involved in multiple sclerosis
<400> 1222
   Thr Asn Thr Leu Thr Pro His Lys Leu Gln Met Leu
   1 5 10
<210> 1223
   <211> 12
   <212> PRT
   <213> Unknown
<220>
   <223> epitope involved in multiple sclerosis
<400> 1223
   Thr Pro Gln Gln Asp Leu Tyr Asp Ile Met Tyr Asp
   1 5 10
<210> 1224
   <211> 12
   <212> PRT
   <213> Unknown
<220>
   <223> epitope involved in multiple sclerosis
<400> 1224
   Thr Gln Gly Leu Lys Tyr Trp Tyr Pro Val Trp Ser
   1 5 10
<210> 1225
   <211> 12
   <212> PRT
   <213> Unknown
<220>
   <223> epitope involved in multiple sclerosis
<400> 1225
   Val Pro Gln Lys Tyr Trp Trp Leu Ser Asp His Thr
   1 5 10
<210> 1226
   <211> 12
   <212> PRT
   <213> Unknown
<220>
   <223> epitope involved in multiple sclerosis
<400> 1226
   Val Val Pro Lys Tyr Trp Trp Ser Ala Glu Leu Pro
   1 5 10
<210> 1227
   <211> 12
   <212> PRT
   <213> Unknown
<220>
   <223> epitope involved in multiple sclerosis
<400> 1227
   Trp His Trp Arg Gln Pro Thr Trp Leu Gln Val Ile
   1               5                   10
<210> 1228
   <211> 11
   <212> PRT
   <213> Homo sapiens
<400> 1228
   Phe Phe Arg Asp His Ser Tyr Gln Glu Glu Ala
   1               5                   10
<210> 1229
   <211> 11
   <212> PRT
   <213> Unknown
<220>
   <223> epitope involved in multiple sclerosis
<400> 1229
   Phe Phe Arg Asp His Ser Tyr Gln Ser Glu Ala
   1               5                   10
<210> 1230
   <211> 7
   <212> PRT
   <213> Unknown
<220>
   <223> epitope involved in multiple sclerosis
<400> 1230
   Ala Pro Ala His His Pro Pro
   1               5
<210> 1231
   <211> 7
   <212> PRT
   <213> Unknown
<220>
   <223> epitope involved in multiple sclerosis
<400> 1231
   Ala Pro Ala His Gln Ile Pro
   1 5
<210> 1232
   <211> 7
   <212> PRT
   <213> Unknown
<220>
   <223> epitope involved in multiple sclerosis
<400> 1232
   Ala Pro Pro His Leu Pro Pro
   1 5
<210> 1233
   <211> 7
   <212> PRT
   <213> Unknown
<220>
   <223> epitope involved in multiple sclerosis
<400> 1233
   Ala Pro Pro His Gln Met Pro
   1 5
<210> 1234
   <211> 7
   <212> PRT
   <213> Unknown
<220>
   <223> epitope involved in multiple sclerosis
<400> 1234
   Ala Pro Pro His Val Met Pro
   1 5
<210> 1235
   <211> 7
   <212> PRT
   <213> Unknown
<220>
   <223> epitope involved in multiple sclerosis
<400> 1235
   Ala Pro Ser His Pro Pro Pro
   1 5
<210> 1236
   <211> 7
   <212> PRT
   <213> Unknown
<220>
   <223> epitope involved in multiple sclerosis
<400> 1236
   Glu Phe Gly Thr Phe Leu Trp
   1               5
<210> 1237
   <211> 6
   <212> PRT
   <213> Unknown
<220>
   <223> epitope involved in multiple sclerosis
<400> 1237
   Phe Gly Thr Tyr Leu Trp
   1               5
<210> 1238
   <211> 7
   <212> PRT
   <213> Unknown
<220>
   <223> epitope involved in multiple sclerosis
<400> 1238
   Phe His Leu Pro Trp Met Gln
   1               5
<210> 1239
   <211> 7
   <212> PRT
   <213> Unknown
<220>
   <223> epitope involved in multiple sclerosis
<400> 1239
   Gly Pro Val Asn Met Asn Leu
   1               5
<210> 1240
   <211> 7
   <212> PRT
   <213> Unknown
<220>
   <223> epitope involved in multiple sclerosis
<400> 1240
   Gly Arg Gln Ala Gln Tyr Tyr
   1 5
<210> 1241
   <211> 11
   <212> PRT
   <213> Human herpesvirus
<400> 1241
   Gly Arg Arg Pro Phe Phe His Pro Val Gly Glu
   1 5 10
<210> 1242
   <211> 7
   <212> PRT
   <213> Unknown
<220>
   <223> epitope involved in multiple sclerosis
<400> 1242
   Ile Leu Asn Trp His Gln Phe
   1 5
<210> 1243
   <211> 7
   <212> PRT
   <213> Unknown
<220>
   <223> epitope involved in multiple sclerosis
<400> 1243
   Lys Phe Gly Thr Ala Leu Trp
   1 5
<210> 1244
   <211> 7
   <212> PRT
   <213> Unknown
<220>
   <223> epitope involved in multiple sclerosis
<400> 1244
   Lys Trp Pro Ile Ile Asp Thr
   1 5
<210> 1245
   <211> 6
   <212> PRT
   <213> Unknown
<220>
   <223> epitope involved in multiple sclerosis
<400> 1245
   Leu Asn Trp Asn Pro Phe
   1 5
<210> 1246
   <211> 7
   <212> PRT
   <213> Unknown
<220>
   <223> epitope involved in multiple sclerosis
<400> 1246
   Met Phe Asn Trp His Pro Phe
   1 5
<210> 1247
   <211> 7
   <212> PRT
   <213> Unknown
<220>
   <223> epitope involved in multiple sclerosis
<400> 1247
   Met Gln Ala His Gln Thr Lys
   1 5
<210> 1248
   <211> 7
   <212> PRT
   <213> Unknown
<220>
   <223> epitope involved in multiple sclerosis
<400> 1248
   Pro Leu Glu Leu Ser Val Tyr
   1 5
<210> 1249
   <211> 7
   <212> PRT
   <213> Unknown
<220>
   <223> epitope involved in multiple sclerosis
<400> 1249
   Pro Val Glu Leu Gln Leu Tyr
   1 5
<210> 1250
   <211> 7
   <212> PRT
   <213> Unknown
<220>
   <223> epitope involved in multiple sclerosis
<400> 1250
   Pro Val Gly Pro Trp Thr Asn
   1 5
<210> 1251
   <211> 7
   <212> PRT
   <213> Unknown
<220>
   <223> epitope involved in multiple sclerosis
<400> 1251
   Gln Phe Gly Thr Phe Leu Trp
   1 5
<210> 1252
   <211> 8
   <212> PRT
   <213> Homo sapiens
<400> 1252
   Ser Phe Asp Gly Thr Phe Leu Trp
   1 5
<210> 1253
   <211> 7
   <212> PRT
   <213> Unknown
<220>
   <223> epitope involved in multiple sclerosis
<400> 1253
   Ser Phe Gly Thr Ala Leu Trp
   1 5
<210> 1254
   <211> 7
   <212> PRT
   <213> Unknown
<220>
   <223> epitope involved in multiple sclerosis
<400> 1254
   Ser Phe Gly Thr Phe Leu Trp
   1 5
<210> 1255
   <211> 7
   <212> PRT
   <213> Unknown
<220>
   <223> epitope involved in multiple sclerosis
<400> 1255
   Ser Phe Gly Thr Ile Leu Trp
   1 5
<210> 1256
   <211> 7
   <212> PRT
   <213> Unknown
<220>
   <223> epitope involved in multiple sclerosis
<400> 1256
   Ser His Arg Pro Thr Thr Gln
   1 5
<210> 1257
   <211> 7
   <212> PRT
   <213> Unknown
<220>
   <223> epitope involved in multiple sclerosis
<400> 1257
   Ser Gln Ser Val Ala Ala Ser
   1 5
<210> 1258
   <211> 7
   <212> PRT
   <213> Unknown
<220>
   <223> epitope involved in multiple sclerosis
<400> 1258
   Ser Thr Glu Gly Arg His Asn
   1 5
<210> 1259
   <211> 7
   <212> PRT
   <213> Unknown
<220>
   <223> epitope involved in multiple sclerosis
<400> 1259
   Thr Val Gln Pro Gln Phe His
   1 5
<210> 1260
   <211> 7
   <212> PRT
   <213> Unknown
<220>
   <223> epitope involved in multiple sclerosis
<400> 1260
   Thr Tyr Met Val Pro Ala Pro
   1 5
<210> 1261
   <211> 8
   <212> PRT
   <213> Unknown
<220>
   <223> epitope involved in multiple sclerosis
<400> 1261
   Val Leu Asn Arg Trp Asn Pro Phe
   1 5
<210> 1262
   <211> 7
   <212> PRT
   <213> Unknown
<220>
   <223> epitope involved in multiple sclerosis
<400> 1262
   Val Leu Asn Trp His Pro Phe
   1 5
<210> 1263
   <211> 7
   <212> PRT
   <213> Unknown
<220>
   <223> epitope involved in multiple sclerosis
<400> 1263
   Trp Gly Leu Asp Asn Pro Pro
   1               5
<210> 1264
   <211> 7
   <212> PRT
   <213> Unknown
<220>
   <223> epitope involved in multiple sclerosis
<400> 1264
   Trp Tyr Tyr Lys Pro Pro Ala
   1               5
<210> 1265
   <211> 9
   <212> PRT
   <213> Human herpesvirus
<400> 1265
   Arg Pro Arg Thr Pro Pro Pro Ser Tyr
   1               5
<210> 1266
   <211> 8
   <212> PRT
   <213> Homo sapiens
<400> 1266
   Thr Pro Arg Thr Pro Pro Pro Gln
   1               5
<210> 1267
   <211> 13
   <212> PRT
   <213> Homo sapiens
<400> 1267
   Phe Ser Trp Gly Ala Glu Gly Gln Arg Pro Gly Phe Gly
   1               5                   10
<210> 1268
   <211> 25
   <212> PRT
   <213> Homo sapiens
<400> 1268
   Ser Ile Ala Phe Pro Ser Lys Thr Ser Ala Ser Ile Gly Ser Leu Cys
   1 5 10 15
   Ala Asp Ala Arg Met Tyr Gly Val Leu
   20 25
<210> 1269
   <211> 17
   <212> PRT
   <213> Homo sapiens
<400> 1269
   Ser Leu Cys Phe Gly Leu Ser Ile Ala Thr Met Val Gln Cys Phe Gly
   1 5 10 15
   His
<210> 1270
   <211> 18
   <212> PRT
   <213> Homo sapiens
<400> 1270
   Val Ile Asp Val Asp Arg Gly Glu Glu Lys Lys Gly Lys Asp Gln Ser
   1 5 10 15
   Gly Glu
<210> 1271
   <211> 11
   <212> PRT
   <213> Homo sapiens
<400> 1271
   Gly Asp Glu Val Glu Leu Pro Cys Arg Ile Ser
   1 5 10
<210> 1272
   <211> 14
   <212> PRT
   <213> Homo sapiens
<400> 1272
   Leu Thr Val Gly Leu Thr Arg Arg Gly Gln Pro Arg Gln Tyr
   1 5 10
<210> 1273
   <211> 14
   <212> PRT
   <213> Homo sapiens
<400> 1273
   Leu Thr Val Gly Leu Thr Arg Arg Gly Gln Pro Arg Gln Tyr
   1               5                   10
<210> 1274
   <211> 13
   <212> PRT
   <213> Homo sapiens
<400> 1274
   Tyr Asn Gln Tyr Ser Gln Arg Tyr His Gln Arg Thr Asn
   1               5                   10
<210> 1275
   <211> 13
   <212> PRT
   <213> Homo sapiens
<400> 1275
   Tyr Asn Gln Tyr Ser Gln Arg Tyr His Gln Arg Thr Asn
   1               5                   10
<210> 1276
   <211> 21
   <212> PRT
   <213> Homo sapiens
<400> 1276
   Gly Ile Thr Val Ser Arg Asn Gly Ser Ser Val Phe Thr Thr Ser Gln
   1               5                   10                  15
   Asp Ser Thr Leu Lys
               20
<210> 1277
   <211> 21
   <212> PRT
   <213> Unknown
<220>
   <223> epitope involved in multiple sclerosis
<400> 1277
   Thr Cys Phe Phe Arg Asp Gly Asn Tyr Gln Glu Glu Ala Ala Met Glu
   1               5                   10                  15
   Leu Lys Val Glu Asp
               20
<210> 1278
   <211> 19
   <212> PRT
   <213> Homo sapiens
<400> 1278
   Thr Phe Arg Asp Leu Gly Asn Leu Thr His Leu Phe Leu His Gly Asn
   1               5                   10                  15
   Arg Ile Ser
<210> 1279
   <211> 21
   <212> PRT
   <213> Unknown
<220>
   <223> epitope involved in multiple sclerosis
<400> 1279
   Thr Phe Arg Val Leu Arg Asn Gly Thr Ser Val Phe Leu His Pro Asn
   1               5                   10                  15
   Lys Trp Thr Val Lys
                                           20
<210> 1280
   <211> 19
   <212> PRT
   <213> Homo sapiens
<400> 1280
   Thr Leu Ile Asn Leu Thr Asn Leu Thr His Leu Tyr Leu His Asn Asn
   1               5                   10                  15
   Lys Phe Thr
<210> 1281
   <211> 21
   <212> PRT
   <213> Homo sapiens
<400> 1281
   Val Val His Leu Tyr Arg Asn Gly Lys Asp Gln Asp Gly Asp Gln Ala
   1               5                   10                  15
   Pro Glu Tyr Arg Gly
               20
<210> 1282
   <211> 11
   <212> PRT
   <213> Homo sapiens
<400> 1282
   Glu Arg Leu Gly Pro Leu Val Glu Gln Gly Arg
   1 5 10
<210> 1283
   <211> 15
   <212> PRT
   <213> Homo sapiens
<400> 1283
   Lys Leu Thr Ser Glu Ala Arg Gly Arg Ile Pro Val Ala Gln Lys
   1 5 10 15
<210> 1284
   <211> 27
   <212> PRT
   <213> Homo sapiens
<400> 1284
   Met Lys Ile Leu Thr Glu Arg Gly Tyr Ser Phe Thr Thr Thr Ala Glu
   1 5 10 15
   Arg Glu Ile Val Arg Asp Ile Lys Glu Lys Leu
   20 25
<210> 1285
   <211> 15
   <212> PRT
   <213> Homo sapiens
<400> 1285
   Ala His Tyr Gly Ser Leu Pro Gln Lys Ser His Gly Arg Thr Gln
   1 5 10 15
<210> 1286
   <211> 15
   <212> PRT
   <213> Homo sapiens
<400> 1286
   Asp Pro Val Val His Phe Phe Lys Asn Ile Val Thr Pro Arg Thr
   1 5 10 15
<210> 1287
   <211> 16
   <212> PRT
   <213> Homo sapiens
<400> 1287
   Gly Lys Gly Arg Gly Leu Ser Leu Ser Arg Phe Ser Trp Gly Ala Glu
   1               5                   10                  15
<210> 1288
   <211> 7
   <212> PRT
   <213> Homo sapiens
<400> 1288
   Gly Leu Gln Leu Tyr Phe Ser
   1               5
<210> 1289
   <211> 15
   <212> PRT
   <213> Homo sapiens
<400> 1289
   Gly Gln Arg Pro Gly Phe Gly Tyr Gly Gly Arg Ala Ser Asp Tyr
   1               5                   10                  15
<210> 1290
   <211> 15
   <212> PRT
   <213> Homo sapiens
<400> 1290
   Ile Leu Asp Ser Ile Gly Arg Phe Phe Gly Gly Asp Arg Gly Ala
   1               5                   10                  15
<210> 1291
   <211> 15
   <212> PRT
   <213> Homo sapiens
<400> 1291
   Pro Lys Arg Gly Ser Gly Lys Asp Ser His His Pro Ala Arg Thr
   1               5                   10                  15
<210> 1292
   <211> 21
   <212> PRT
   <213> Homo sapiens
<400> 1292
   Tyr Leu Ala Ser Ala Ser Thr Met Asp His Ala Arg His Gly Phe Leu
   1               5                   10                  15
   Pro Arg Arg His Arg
               20
<210> 1293
   <211> 26
   <212> PRT
   <213> Zea mays
<400> 1293
   Phe Thr Gly Ala Ser Met Asn Pro Ala Arg Ser Phe Gly Pro Ala Leu
   1               5                   10                  15
   Ala Thr Gly Asp Trp Thr Asn His Trp Val
               20                  25
<210> 1294
   <211> 26
   <212> PRT
   <213> Homo sapiens
<400> 1294
   Tyr Thr Gly Ala Ser Met Asn Pro Ala Arg Ser Phe Gly Pro Ala Val
   1               5                   10                  15
   Ile Met Gly Asn Trp Glu Asn His Trp Ile
   20                  25
<210> 1295
   <211> 20
   <212> PRT
   <213> Triticum aestivum
<400> 1295
   Ala Met Cys Asn Val Tyr Ile Pro Pro Tyr Cys Ala Met Ala Pro Phe
   1               5                   10                  15
   Gly Ile Phe Gly
               20
<210> 1296
   <211> 8
   <212> PRT
   <213> Triticum aestivum
<400> 1296
   Glu Leu Pro Tyr Pro Gln Pro Gln
   1 5
<210> 1297
   <211> 8
   <212> PRT
   <213> Triticum aestivum
<400> 1297
   Phe Pro Gln Pro Glu Leu Pro Tyr
   1 5
<210> 1298
   <211> 10
   <212> PRT
   <213> Triticum aestivum
<400> 1298
   Phe Pro Gln Pro Gln Pro Phe Pro Pro Gln
   1 5 10
<210> 1299
   <211> 10
   <212> PRT
   <213> Triticum aestivum
<400> 1299
   Phe Pro Gln Pro Gln Gln Gln Phe Pro Gln
   1 5 10
<210> 1300
   <211> 13
   <212> PRT
   <213> Triticum aestivum
<400> 1300
   Phe Pro Gln Gln Pro Gln Gln Pro Phe Pro Gln Gln Pro
   1 5 10
<210> 1301
   <211> 12
   <212> PRT
   <213> Triticum aestivum
<400> 1301
   Phe Pro Gln Gln Pro Gln Gln Pro Tyr Pro Gln Gln
   1 5 10
<210> 1302
   <211> 13
   <212> PRT
   <213> Triticum aestivum
<400> 1302
   Phe Pro Gln Gln Pro Gln Gln Pro Tyr Pro Gln Gln Pro
   1 5 10
<210> 1303
   <211> 13
   <212> PRT
   <213> Triticum aestivum
<400> 1303
   Phe Pro Gln Gln Pro Gln Gln Pro Tyr Pro Gln Gln Pro
   1 5 10
<210> 1304
   <211> 15
   <212> PRT
   <213> Triticum aestivum
<400> 1304
   Phe Pro Gln Gln Pro Gln Gln Pro Tyr Pro Gln Gln Pro Gln Gln
   1 5 10 15
<210> 1305
   <211> 10
   <212> PRT
   <213> Triticum aestivum
<400> 1305
   Phe Pro Ser Gln Gln Pro Tyr Leu Gln Leu
   1 5 10
<210> 1306
   <211> 12
   <212> PRT
   <213> Unknown
<220>
   <223> epitope involved in celiac disease
<400> 1306
   Phe Ser Gln Pro Glu Gln Glu Phe Pro Gln Pro Gln
   1 5 10
<210> 1307
   <211> 12
   <212> PRT
   <213> Triticum aestivum
<400> 1307
   Phe Ser Gln Pro Gln Gln Gln Phe Pro Gln Pro Gln
   1 5 10
<210> 1308
   <211> 11
   <212> PRT
   <213> Unknown
<220>
   <223> eptiope involved in celiac disease
<400> 1308
   Gly Ile Ile Gln Pro Glu Gln Pro Ala Gln Leu
   1 5 10
<210> 1309
   <211> 11
   <212> PRT
   <213> Triticum aestivum
<400> 1309
   Gly Ile Ile Gln Pro Gln Gln Pro Ala Gln Leu
   1 5 10
<210> 1310
   <211> 15
   <212> PRT
   <213> Triticum aestivum
<400> 1310
   Gly Ser Phe Gln Pro Ser Gln Gln Asn Pro Gln Ala Gln Gly Ser
   1 5 10 15
<210> 1311
   <211> 20
   <212> PRT
   <213> Triticum aestivum
<400> 1311
   His Gln Pro Gln Gln Thr Phe Pro Gln Pro Gln Gln Thr Tyr Pro His
   1 5 10 15
   Gln Pro Gln Gln
   20
<210> 1312
   <211> 20
   <212> PRT
   <213> Triticum aestivum
<400> 1312
   Ile His Ser Val Ala His Ser Ile Ile Met Gln Gln Glu Gln Gln Gln
   1               5                   10                  15
   Gly Val Pro Ile
               20
<210> 1313
   <211> 10
   <212> PRT
   <213> Unknown
<220>
   <223> epitope involved in celiac disease
<400> 1313
   Ile Ile Gln Pro Glu Gln Pro Ala Gln Leu
   1               5                   10
<210> 1314
   <211> 9
   <212> PRT
   <213> Triticum aestivum
<400> 1314
   Ile Ile Gln Pro Gln Gln Pro Ala Gln
   1               5
<210> 1315
   <211> 10
   <212> PRT
   <213> Triticum aestivum
<400> 1315
   Ile Ile Gln Pro Gln Gln Pro Ala Gln Leu
   1               5                   10
<210> 1316
   <211> 20
   <212> PRT
   <213> Triticum aestivum
<400> 1316
   Leu Gly Ile Ile Gln Pro Gln Gln Pro Ala Gln Leu Glu Gly Ile Arg
   1               5                   10                  15
   Ser Leu Val Leu
               20
<210> 1317
   <211> 12
   <212> PRT
   <213> Triticum aestivum
<400> 1317
   Leu Gly Gln Gly Ser Phe Arg Pro Ser Gln Gln Asn
   1               5                   10
<210> 1318
   <211> 17
   <212> PRT
   <213> Triticum aestivum
<400> 1318
   Leu Gly Gln Gln Gln Pro Phe Pro Pro Gln Gln Pro Tyr Pro Gln Pro
   1               5                   10                  15
   Gln
<210> 1319
   <211> 19
   <212> PRT
   <213> Triticum aestivum
<400> 1319
   Leu Gly Gln Gln Gln Pro Phe Pro Pro Gln Gln Pro Tyr Pro Gln Pro
   1               5                   10                  15
   Gln Pro Phe
<210> 1320
   <211> 20
   <212> PRT
   <213> Unknown
<220>
   <223> epitope involved in celiac disease
<400> 1320
   Leu Gln Leu Gln Pro Phe Pro Gln Pro Glu Lys Pro Tyr Pro Gln Pro
   1               5                   10                  15
   Glu Lys Pro Tyr
               20
<210> 1321
   <211> 20
   <212> PRT
   <213> Unknown
<220>
   <223> epitope involved in celiac disease
<400> 1321
   Leu Gln Leu Gln Pro Phe Pro Gln Pro Glu Lys Pro Tyr Pro Gln Pro
   1               5                   10                  15
   Glu Leu Pro Tyr
               20
<210> 1322
   <211> 20
   <212> PRT
   <213> Unknown
<220>
   <223> epitope involved in celiac disease
<400> 1322
   Leu Gln Leu Gln Pro Phe Pro Gln Pro Glu Lys Pro Tyr Pro Gln Pro
   1               5                   10                  15
   Glu Leu Pro Tyr
               20
<210> 1323
   <211> 20
   <212> PRT
   <213> Unknown
<220>
   <223> epitope involved in celiac disease
<400> 1323
   Leu Gln Leu Gln Pro Phe Pro Gln Pro Glu Leu Pro Tyr Pro Gln Pro
   1               5                   10                  15
   Glu Lys Pro Tyr
               20
<210> 1324
   <211> 20
   <212> PRT
   <213> Unknown
<220>
   <223> epitope involved in celiac disease
<400> 1324
   Leu Gln Leu Gln Pro Phe Pro Gln Pro Glu Leu Pro Tyr Pro Gln Pro
   1               5                   10                  15
   Glu Leu Pro Tyr
               20
<210> 1325
   <211> 33
   <212> PRT
   <213> Unknown
<220>
   <223> epitope involved in celiac disease
<400> 1325
   Leu Gln Leu Gln Pro Phe Pro Gln Pro Glu Leu Pro Tyr Pro Gln Pro
   1               5                   10                  15
   Glu Leu Pro Tyr Pro Gln Pro Glu Leu Pro Tyr Pro Gln Pro Gln Pro
               20                  25                  30
   Phe
<210> 1326
   <211> 13
   <212> PRT
   <213> Triticum aestivum
<400> 1326
   Leu Gln Leu Gln Pro Phe Pro Gln Pro Gln Leu Pro Tyr
   1               5                   10
<210> 1327
   <211> 13
   <212> PRT
   <213> Triticum aestivum
<400> 1327
   Leu Gln Leu Gln Pro Phe Pro Gln Pro Gln Leu Pro Tyr
   1               5                   10
<210> 1328
   <211> 20
   <212> PRT
   <213> Triticum aestivum
<400> 1328
   Leu Gln Leu Gln Pro Phe Pro Gln Pro Gln Leu Pro Tyr Pro Gln Pro
   1               5                   10                  15
   Gln Leu Pro Tyr
               20
<210> 1329
   <211> 20
   <212> PRT
   <213> Triticum aestivum
<400> 1329
   Leu Gln Leu Gln Pro Phe Pro Gln Pro Gln Leu Pro Tyr Pro Gln Pro
   1               5                   10                  15
   Gln Leu Pro Tyr
               20
<210> 1330
   <211> 32
   <212> PRT
   <213> Unknown
<220>
   <223> epitope involved in celiac disease
<400> 1330
   Leu Gln Leu Gln Pro Phe Pro Gln Pro Gln Leu Pro Tyr Pro Gln Pro
   1               5                   10                  15
   Gln Leu Pro Tyr Pro Gln Pro Gln Leu Pro Tyr Pro Gln Pro Pro Phe
               20                  25                  30
<210> 1331
   <211> 33
   <212> PRT
   <213> Triticum aestivum
<400> 1331
   Leu Gln Leu Gln Pro Phe Pro Gln Pro Gln Leu Pro Tyr Pro Gln Pro
   1               5                   10                  15
   Gln Leu Pro Tyr Pro Gln Pro Gln Leu Pro Tyr Pro Gln Pro Gln Pro
               20                  25                  30
   Phe
<210> 1332
   <211> 8
   <212> PRT
   <213> Triticum aestivum
<400> 1332
   Leu Gln Pro Phe Pro Gln Pro Glu
   1 5
<210> 1333
   <211> 11
   <212> PRT
   <213> Unknown
<220>
   <223> epitope involved in celiac disease
<400> 1333
   Leu Gln Pro Phe Pro Gln Pro Glu Leu Pro Tyr
   1 5 10
<210> 1334
   <211> 11
   <212> PRT
   <213> Triticum aestivum
<400> 1334
   Leu Gln Pro Phe Pro Gln Pro Gln Leu Pro Tyr
   1 5 10
<210> 1335
   <211> 20
   <212> PRT
   <213> Triticum aestivum
<400> 1335
   Leu Gln Pro Gln Gln Pro Phe Pro Gln Gln Pro Gln Gln Pro Tyr Pro
   1 5 10 15
   Gln Gln Pro Gln
   20
<210> 1336
   <211> 20
   <212> PRT
   <213> Triticum aestivum
<400> 1336
   Leu Arg Pro Leu Phe Gln Leu Ala Gln Gly Leu Gly Ile Ile Gln Pro
   1 5 10 15
   Gln Gln Pro Ala
   20
<210> 1337
   <211> 12
   <212> PRT
   <213> Human adenovirus 12
<400> 1337
   Leu Arg Arg Gly Met Phe Arg Pro Ser Gln Cys Asn
   1 5 10
<210> 1338
   <211> 8
   <212> PRT
   <213> Triticum aestivum
<400> 1338
   Pro Glu Leu Pro Tyr Pro Gln Pro
   1 5
<210> 1339
   <211> 14
   <212> PRT
   <213> Unknown
<220>
   <223> epitope involved in celiac disease
<400> 1339
   Pro Glu Gln Pro Gln Gln Ser Phe Pro Glu Gln Glu Arg Pro
   1 5 10
<210> 1340
   <211> 8
   <212> PRT
   <213> Triticum aestivum
<400> 1340
   Pro Phe Pro Gln Pro Glu Leu Pro
   1 5
<210> 1341
   <211> 9
   <212> PRT
   <213> Triticum aestivum
<400> 1341
   Pro Phe Pro Gln Pro Gln Leu Pro Tyr
   1 5
<210> 1342
   <211> 15
   <212> PRT
   <213> Triticum aestivum
<400> 1342
   Pro Phe Pro Gln Pro Gln Leu Pro Tyr Pro Gln Pro Gln Leu Pro
   1 5 10 15
<210> 1343
   <211> 20
   <212> PRT
   <213> Triticum aestivum
<400> 1343
   Pro Phe Pro Gln Pro Gln Gln Thr Phe Pro Gln Gln Pro Gln Leu Pro
   1 5 10 15
   Phe Pro Gln Gln
   20
<210> 1344
   <211> 20
   <212> PRT
   <213> Triticum aestivum
<400> 1344
   Pro Phe Pro Gln Gln Pro Gln Gln Pro Phe Pro Gln Gln Pro Gln Gln
   1 5 10 15
   Ser Phe Pro Gln
   20
<210> 1345
   <211> 14
   <212> PRT
   <213> Triticum aestivum
<400> 1345
   Pro Phe Arg Pro Gln Gln Pro Tyr Pro Gln Pro Gln Pro Gln
   1 5 10
<210> 1346
   <211> 8
   <212> PRT
   <213> Triticum aestivum
<400> 1346
   Pro Phe Ser Gln Gln Gln Gln Gln
   1 5
<210> 1347
   <211> 10
   <212> PRT
   <213> Triticum aestivum
<400> 1347
   Pro Phe Ser Gln Gln Gln Gln Ser Pro Phe
   1 5 10
<210> 1348
   <211> 20
   <212> PRT
   <213> Triticum aestivum
<400> 1348
   Pro Phe Thr Gln Pro Gln Gln Pro Thr Pro Ile Gln Pro Gln Gln Pro
   1 5 10 15
   Phe Pro Gln Gln
   20
<210> 1349
   <211> 8
   <212> PRT
   <213> Triticum aestivum
<400> 1349
   Pro Gln Pro Glu Leu Pro Tyr Pro
   1 5
<210> 1350
   <211> 9
   <212> PRT
   <213> Unknown
<220>
   <223> epitope involved in celiac disease
<400> 1350
   Pro Gln Pro Glu Leu Pro Tyr Pro Gln
   1 5
<210> 1351
   <211> 11
   <212> PRT
   <213> Unknown
<220>
   <223> epitope involved in celiac disease
<400> 1351
   Pro Gln Pro Glu Leu Pro Tyr Pro Gln Pro Glu
   1 5 10
<210> 1352
   <211> 14
   <212> PRT
   <213> Unknown
<220>
   <223> epitope involved in celiac disease
<400> 1352
   Pro Gln Pro Glu Leu Pro Tyr Pro Gln Pro Glu Leu Pro Tyr
   1 5 10
<210> 1353
   <211> 14
   <212> PRT
   <213> Unknown
<220>
   <223> epitope involved in celiac disease
<400> 1353
   Pro Gln Pro Glu Leu Pro Tyr Pro Gln Pro Gln Leu Pro Tyr
   1 5 10
<210> 1354
   <211> 9
   <212> PRT
   <213> Triticum aestivum
<400> 1354
   Pro Gln Pro Gln Leu Pro Tyr Pro Gln
   1 5
<210> 1355
   <211> 12
   <212> PRT
   <213> Triticum aestivum
<400> 1355
   Pro Gln Pro Gln Leu Pro Tyr Pro Gln Pro Gln Leu
   1 5 10
<210> 1356
   <211> 14
   <212> PRT
   <213> Triticum aestivum
<400> 1356
   Pro Gln Pro Gln Leu Pro Tyr Pro Gln Pro Gln Leu Pro Tyr
   1 5 10
<210> 1357
   <211> 14
   <212> PRT
   <213> Triticum aestivum
<400> 1357
   Pro Gln Pro Gln Leu Pro Tyr Pro Gln Pro Gln Leu Pro Tyr
   1               5                   10
<210> 1358
   <211> 14
   <212> PRT
   <213> Triticum aestivum
<400> 1358
   Pro Gln Pro Gln Leu Pro Tyr Pro Gln Pro Gln Leu Pro Tyr
   1               5                   10
<210> 1359
   <211> 20
   <212> PRT
   <213> Triticum aestivum
<400> 1359
   Pro Gln Pro Gln Gln Pro Gln Leu Pro Phe Pro Gln Gln Pro Gln Gln
   1               5                   10                  15
   Pro Phe Pro Gln
               20
<210> 1360
   <211> 5
   <212> PRT
   <213> Triticum aestivum
<400> 1360
   Pro Gln Gln Pro Phe
   1               5
<210> 1361
   <211> 20
   <212> PRT
   <213> Triticum aestivum
<400> 1361
   Pro Gln Gln Pro Phe Pro Gln Pro Gln Gln Pro Gln Gln Pro Phe Pro
   1               5                   10                  15
   Gln Ser Gln Gln
               20
<210> 1362
   <211> 20
   <212> PRT
   <213> Triticum aestivum
<400> 1362
   Pro Gln Gln Pro Phe Pro Gln Pro Gln Gln Gln Phe Pro Gln Pro Gln
   1 5 10 15
   Gln Pro Gln Gln
   20
<210> 1363
   <211> 11
   <212> PRT
   <213> Triticum aestivum
<400> 1363
   Pro Gln Gln Pro Phe Pro Gln Gln Pro Gln Gln
   1 5 10
<210> 1364
   <211> 11
   <212> PRT
   <213> Triticum aestivum
<400> 1364
   Pro Gln Gln Pro Phe Pro Gln Gln Pro Gln Gln
   1 5 10
<210> 1365
   <211> 17
   <212> PRT
   <213> Triticum aestivum
<400> 1365
   Pro Gln Gln Pro Phe Pro Gln Gln Pro Gln Gln Pro Tyr Pro Gln Gln
   1 5 10 15
   Pro
<210> 1366
   <211> 20
   <212> PRT
   <213> Triticum aestivum
<400> 1366
   Pro Gln Gln Pro Phe Pro Gln Ser Gln Gln Pro Gln Gln Pro Phe Pro
   1 5 10 15
   Gln Pro Gln Gln
   20
<210> 1367
   <211> 20
   <212> PRT
   <213> Triticum aestivum
<400> 1367
   Pro Gln Gln Pro Gln Gln Pro Phe Pro Leu Gln Pro Gln Gln Pro Phe
   1 5 10 15
   Pro Gln Gln Pro
   20
<210> 1368
   <211> 14
   <212> PRT
   <213> Triticum aestivum
<400> 1368
   Pro Gln Gln Pro Gln Gln Ser Phe Pro Gln Gln Gln Arg Pro
   1 5 10
<210> 1369
   <211> 14
   <212> PRT
   <213> Triticum aestivum
<400> 1369
   Pro Gln Gln Pro Gln Gln Ser Phe Pro Gln Gln Gln Arg Pro
   1 5 10
<210> 1370
   <211> 9
   <212> PRT
   <213> Triticum aestivum
<400> 1370
   Pro Gln Gln Ser Phe Pro Glu Gln Glu
   1 5
<210> 1371
   <211> 9
   <212> PRT
   <213> Triticum aestivum
<400> 1371
   Pro Gln Gln Ser Phe Pro Gln Gln Gln
   1 5
<210> 1372
   <211> 14
   <212> PRT
   <213> Triticum aestivum
<400> 1372
   Pro Gln Thr Gln Gln Pro Gln Gln Pro Phe Pro Gln Pro Gln
   1 5 10
<210> 1373
   <211> 15
   <212> PRT
   <213> Triticum aestivum
<400> 1373
   Pro Ser Gly Gln Gly Ser Phe Gln Pro Ser Gln Gln Asn Pro Gln
   1               5                   10                  15
<210> 1374
   <211> 9
   <212> PRT
   <213> Triticum aestivum
<400> 1374
   Pro Tyr Pro Gln Pro Gln Leu Pro Tyr
   1               5
<210> 1375
   <211> 9
   <212> PRT
   <213> Triticum aestivum
<400> 1375
   Pro Tyr Pro Gln Pro Gln Leu Pro Tyr
   1               5
<210> 1376
   <211> 15
   <212> PRT
   <213> Triticum aestivum
<400> 1376
   Gln Ala Gln Gly Ser Val Gln Pro Gln Gln Leu Pro Gln Phe Glu
   1               5                   10                  15
<210> 1377
   <211> 20
   <212> PRT
   <213> Triticum aestivum
<400> 1377
   Gln Phe Pro Gln Pro Gln Gln Pro Gln Gln Ser Phe Pro Gln Gln Gln
   1               5                   10                  15
   Gln Pro Ala Ile
               20
<210> 1378
   <211> 20
   <212> PRT
   <213> Triticum aestivum
<400> 1378
   Gln Phe Pro Gln Thr Gln Gln Pro Gln Gln Pro Phe Pro Gln Pro Gln
   1 5 10 15
   Gln Thr Phe Pro
   20
<210> 1379
   <211> 10
   <212> PRT
   <213> Triticum aestivum
<400> 1379
   Gln Phe Gln Gly Gln Gln Gln Pro Phe Pro
   1 5 10
<210> 1380
   <211> 15
   <212> PRT
   <213> Aegilops markgrafii
<400> 1380
   Gln Gly Gln Gln Gly Tyr Tyr Pro Thr Ser Pro Gln Gln Ser Gly
   1 5 10 15
<210> 1381
   <211> 10
   <212> PRT
   <213> Triticum aestivum
<400> 1381
   Gln Gly Gln Gln Gln Pro Phe Pro Pro Gln
   1 5 10
<210> 1382
   <211> 10
   <212> PRT
   <213> Triticum aestivum
<400> 1382
   Gln Leu Ile Pro Cys Met Asp Val Val Leu
   1 5 10
<210> 1383
   <211> 13
   <212> PRT
   <213> Triticum aestivum
<400> 1383
   Gln Leu Pro Tyr Pro Gln Pro Gln Leu Pro Tyr Pro Gln
   1 5 10
<210> 1384
   <211> 15
   <212> PRT
   <213> Triticum aestivum
<400> 1384
   Gln Leu Pro Tyr Pro Gln Pro Gln Leu Pro Tyr Pro Gln Pro Gln
   1 5 10 15
<210> 1385
   <211> 8
   <212> PRT
   <213> Triticum aestivum
<400> 1385
   Gln Leu Gln Pro Phe Pro Gln Pro
   1 5
<210> 1386
   <211> 12
   <212> PRT
   <213> Triticum aestivum
<400> 1386
   Gln Leu Gln Pro Phe Pro Gln Pro Glu Leu Pro Tyr
   1 5 10
<210> 1387
   <211> 17
   <212> PRT
   <213> Triticum aestivum
<400> 1387
   Gln Leu Gln Pro Phe Pro Gln Pro Glu Leu Pro Tyr Pro Gln Pro Gln
   1 5 10 15
   Ser
<210> 1388
   <211> 17
   <212> PRT
   <213> Triticum aestivum
<400> 1388
   Gln Leu Gln Pro Phe Pro Gln Pro Glu Leu Pro Tyr Pro Gln Pro Gln
   1 5 10 15
   Ser
<210> 1389
   <211> 12
   <212> PRT
   <213> Triticum aestivum
<400> 1389
   Gln Leu Gln Pro Phe Pro Gln Pro Gln Leu Pro Tyr
   1 5 10
<210> 1390
   <211> 12
   <212> PRT
   <213> Triticum aestivum
<400> 1390
   Gln Leu Gln Pro Phe Pro Gln Pro Gln Leu Pro Tyr
   1 5 10
<210> 1391
   <211> 16
   <212> PRT
   <213> Triticum aestivum
<400> 1391
   Gln Leu Gln Pro Phe Pro Gln Pro Gln Leu Pro Tyr Pro Gln Pro Gln
   1 5 10 15
<210> 1392
   <211> 17
   <212> PRT
   <213> Triticum aestivum
<400> 1392
   Gln Leu Gln Pro Phe Pro Gln Pro Gln Leu Pro Tyr Pro Gln Pro Gln
   1 5 10 15
   Leu
<210> 1393
   <211> 17
   <212> PRT
   <213> Triticum aestivum
<400> 1393
   Gln Leu Gln Pro Phe Pro Gln Pro Gln Leu Pro Tyr Pro Gln Pro Gln
   1 5 10 15
   Pro
<210> 1394
   <211> 17
   <212> PRT
   <213> Triticum aestivum
<400> 1394
   Gln Leu Gln Pro Phe Pro Gln Pro Gln Leu Pro Tyr Pro Gln Pro Gln
   1               5                   10                  15
   Ser
<210> 1395
   <211> 8
   <212> PRT
   <213> Triticum aestivum
<400> 1395
   Gln Pro Glu Leu Pro Tyr Pro Gln
   1               5
<210> 1396
   <211> 8
   <212> PRT
   <213> Triticum aestivum
<400> 1396
   Gln Pro Phe Pro Gln Pro Glu Leu
   1               5
<210> 1397
   <211> 10
   <212> PRT
   <213> Unknown
<220>
   <223> epitope involved in celiac disease
<400> 1397
   Gln Pro Phe Pro Gln Pro Glu Leu Pro Tyr
   1               5                   10
<210> 1398
   <211> 10
   <212> PRT
   <213> Triticum aestivum
<400> 1398
   Gln Pro Phe Pro Gln Pro Gln Leu Pro Tyr
   1               5                   10
<210> 1399
   <211> 10
   <212> PRT
   <213> Triticum aestivum
<400> 1399
   Gln Pro Phe Pro Ser Gln Gln Pro Tyr Leu
   1               5                   10
<210> 1400
   <211> 13
   <212> PRT
   <213> Triticum aestivum
<400> 1400
   Gln Pro Gln Leu Pro Phe Pro Gln Gln Pro Gln Gln Pro
   1               5                   10
<210> 1401
   <211> 15
   <212> PRT
   <213> Triticum aestivum
<400> 1401
   Gln Pro Gln Leu Pro Tyr Pro Gln Pro Gln Leu Pro Tyr Pro Gln
   1               5                   10                  15
<210> 1402
   <211> 10
   <212> PRT
   <213> Triticum aestivum
<400> 1402
   Gln Pro Gln Pro Phe Pro Pro Gln Leu Pro
   1               5                   10
<210> 1403
   <211> 21
   <212> PRT
   <213> Triticum aestivum
<400> 1403
   Gln Pro Gln Pro Phe Pro Gln Gln Ser Glu Gln Ser Gln Gln Pro Phe
   1               5                   10                  15
   Gln Pro Gln Pro Phe
               20
<210> 1404
   <211> 10
   <212> PRT
   <213> Triticum aestivum
<400> 1404
   Gln Pro Gln Pro Phe Pro Ser Gln Gln Pro
   1               5                   10
<210> 1405
   <211> 20
   <212> PRT
   <213> Triticum aestivum
<400> 1405
   Gln Pro Gln Pro Phe Arg Pro Gln Gln Pro Tyr Pro Gln Pro Gln Pro
   1               5                   10                  15
   Gln Tyr Ser Gln
               20
<210> 1406
   <211> 10
   <212> PRT
   <213> Unknown
<220>
   <223> epitope involved in celiac disease
<400> 1406
   Gln Pro Gln Pro Gln Phe Pro Glu Gln Gln
   1               5                   10
<210> 1407
   <211> 19
   <212> PRT
   <213> Triticum aestivum
<400> 1407
   Gln Pro Gln Gln Pro Phe Pro Gln Gln Pro Gln Gln Pro Tyr Pro Gln
     1               5                   10                  15
   Gln Pro Gln
<210> 1408
   <211> 13
   <212> PRT
   <213> Triticum aestivum
<400> 1408
   Gln Pro Gln Gln Pro Ile Pro Val Gln Pro Gln Gln Ser
   1               5                   10
<210> 1409
   <211> 20
   <212> PRT
   <213> Triticum aestivum
<400> 1409
   Gln Pro Gln Gln Pro Ile Pro Val Gln Pro Gln Gln Ser Phe Pro Gln
   1 5 10 15
   Gln Ser Gln Gln
   20
<210> 1410
   <211> 20
   <212> PRT
   <213> Triticum aestivum
<400> 1410
   Gln Pro Gln Gln Pro Gln Gln Pro Phe Pro Gln Gln Gln Gln Pro Leu
   1 5 10 15
   Ile Gln Pro Tyr
   20
<210> 1411
   <211> 16
   <212> PRT
   <213> Triticum aestivum
<400> 1411
   Gln Pro Gln Gln Pro Gln Gln Ser Phe Pro Gln Gln Gln Arg Pro Phe
   1 5 10 15
<210> 1412
   <211> 20
   <212> PRT
   <213> Triticum aestivum
<400> 1412
   Gln Pro Gln Gln Gln Tyr Pro Ser Gly Gln Gly Ser Phe Gln Pro Ser
   1 5 10 15
   Gln Gln Asn Pro
   20
<210> 1413
   <211> 10
   <212> PRT
   <213> Triticum aestivum
<400> 1413
   Gln Pro Gln Gln Ser Phe Pro Gln Gln Gln
   1 5 10
<210> 1414
   <211> 12
   <212> PRT
   <213> Triticum aestivum
<400> 1414
   Gln Pro Gln Gln Ser Phe Pro Gln Gln Gln Arg Pro
   1               5                   10
<210> 1415
   <211> 10
   <212> PRT
   <213> Unknown
<220>
   <223> epitope involved in celiac disease
<400> 1415
   Gln Pro Gln Gln Thr Phe Pro Glu Gln Ala
   1               5                   10
<210> 1416
   <211> 10
   <212> PRT
   <213> Triticum aestivum
<400> 1416
   Gln Pro Tyr Pro Gln Pro Gln Pro Gln Tyr
   1               5                   10
<210> 1417
   <211> 20
   <212> PRT
   <213> Triticum aestivum
<400> 1417
   Gln Gln Phe Leu Gln Pro Gln Gln Pro Phe Pro Gln Gln Pro Gln Gln
   1               5                   10                  15
   Pro Tyr Pro Gln
               20
<210> 1418
   <211> 13
   <212> PRT
   <213> Triticum aestivum
<400> 1418
   Gln Gln Gly Tyr Tyr Pro Thr Ser Pro Gln Gln Ser Gly
   1               5                   10
<210> 1419
   <211> 20
   <212> PRT
   <213> Triticum aestivum
<400> 1419
   Gln Gln Leu Pro Gln Pro Gln Gln Pro Gln Gln Ser Phe Pro Gln Gln
   1               5                   10                  15
   Gln Arg Pro Phe
               20
<210> 1420
   <211> 20
   <212> PRT
   <213> Triticum aestivum
<400> 1420
   Gln Gln Pro Phe Cys Gln Gln Pro Gln Arg Thr Ile Pro Gln Pro His
   1               5                   10                  15
   Gln Thr Phe His
               20
<210> 1421
   <211> 5
   <212> PRT
   <213> Triticum aestivum
<400> 1421
   Gln Gln Pro Phe Pro
   1               5
<210> 1422
   <211> 20
   <212> PRT
   <213> Triticum aestivum
<400> 1422
   Gln Gln Pro Phe Pro Gln Gln Pro Gln Gln Pro Phe Pro Gln Pro Gln
   1               5                   10                  15
   Gln Pro Ile Pro
               20
<210> 1423
   <211> 20
   <212> PRT
   <213> Triticum aestivum
<400> 1423
   Gln Gln Pro Leu Pro Gln Pro Gln Gln Pro Gln Gln Pro Phe Pro Gln
   1 5 10 15
   Ser Gln Gln Pro
   20
<210> 1424
   <211> 15
   <212> PRT
   <213> Triticum aestivum
<400> 1424
   Gln Gln Pro Pro Phe Ser Gln Gln Gln Gln Gln Pro Leu Pro Gln
   1 5 10 15
<210> 1425
   <211> 10
   <212> PRT
   <213> Triticum durum
<400> 1425
   Gln Gln Pro Gln Asp Ala Val Gln Pro Phe
   1 5 10
<210> 1426
   <211> 20
   <212> PRT
   <213> Triticum aestivum
<400> 1426
   Gln Gln Pro Gln Leu Pro Phe Pro Gln Gln Pro Gln Gln Pro Phe Pro
   1 5 10 15
   Gln Pro Gln Gln
   20
<210> 1427
   <211> 20
   <212> PRT
   <213> Triticum aestivum
<400> 1427
   Gln Gln Pro Tyr Pro Gln Pro Gln Pro Phe Pro Ser Gln Gln Pro Tyr
   1 5 10 15
   Leu Gln Leu Gln
   20
<210> 1428
   <211> 20
   <212> PRT
   <213> Triticum aestivum
<400> 1428
   Gln Gln Gln Phe Ile Gln Pro Gln Gln Pro Phe Pro Gln Gln Pro Gln
   1               5                   10                  15
   Gln Thr Tyr Pro
               20
<210> 1429
   <211> 10
   <212> PRT
   <213> Triticum aestivum
<400> 1429
   Gln Gln Gln Pro Phe Pro Pro Gln Gln Pro
   1               5                   10
<210> 1430
   <211> 20
   <212> PRT
   <213> Triticum aestivum
<400> 1430
   Gln Gln Gln Gln Pro Pro Phe Ser Gln Gln Gln Gln Ser Pro Phe Ser
   1               5                   10                  15
   Gln Gln Gln Gln
               20
<210> 1431
   <211> 20
   <212> PRT
   <213> Triticum aestivum
<400> 1431
   Gln Gln Thr Tyr Pro His Gln Pro Gln Gln Gln Phe Pro Gln Thr Gln
   1               5                   10                  15
   Gln Pro Gln Gln
               20
<210> 1432
   <211> 21
   <212> PRT
   <213> Triticum aestivum
<220>
   <221> misc_feature
   <222> (3)..(3)
   <223> Xaa can be any naturally occurring amino acid
<220>
   <221> misc_feature
   <222> (8)..(8)
   <223> Xaa can be any naturally occurring amino acid
<220>
   <221> misc_feature
   <222> (14)..(14)
   <223> Xaa can be any naturally occurring amino acid
<400> 1432
   Gln Gln Xaa Ser Gln Pro Gln Xaa Pro Gln Gln Gln Gln Xaa Pro Gln
   1               5                   10                  15
   Gln Pro Gln Gln Phe
               20
<210> 1433
   <211> 19
   <212> PRT
   <213> Triticum aestivum
<400> 1433
   Gln Gln Tyr Pro Leu Gly Gln Gly Ser Phe Arg Pro Ser Gln Gln Asn
   1               5                   10                  15
   Pro Gln Ala
<210> 1434
   <211> 12
   <212> PRT
   <213> Triticum aestivum
<400> 1434
   Gln Ser Glu Gln Ser Gln Gln Pro Phe Gln Pro Gln
   1               5                   10
<210> 1435
   <211> 20
   <212> PRT
   <213> Triticum aestivum
<400> 1435
   Gln Ser Phe Leu Gln Gln Gln Met Asn Pro Cys Lys Asn Phe Leu Leu
   1 5 10 15
   Gln Gln Cys Asn
   20
<210> 1436
   <211> 20
   <212> PRT
   <213> Triticum aestivum
<400> 1436
   Gln Thr Gln Gln Pro Gln Gln Pro Phe Pro Gln Gln Pro Gln Gln Pro
   1 5 10 15
   Phe Pro Gln Thr
   20
<210> 1437
   <211> 20
   <212> PRT
   <213> Triticum aestivum
<400> 1437
   Gln Val Pro Leu Val Gln Gln Gln Gln Phe Leu Gly Gln Gln Gln Pro
   1 5 10 15
   Phe Pro Pro Gln
   20
<210> 1438
   <211> 9
   <212> PRT
   <213> Triticum aestivum
<220>
   <221> misc_feature
   <222> (2)..(2)
   <223> Xaa can be any naturally occurring amino acid
<400> 1438
   Gln Xaa Pro Gln Gln Pro Gln Gln Phe
   1 5
<210> 1439
   <211> 20
   <212> PRT
   <213> Triticum aestivum
<400> 1439
   Ser Phe Pro Gln Gln Gln Gln Pro Ala Ile Gln Ser Phe Leu Gln Gln
   1 5 10 15
   Gln Met Asn Pro
   20
<210> 1440
   <211> 10
   <212> PRT
   <213> Triticum aestivum
<400> 1440
   Ser Phe Gln Gln Pro Gln Gln Gln Tyr Pro
   1 5 10
<210> 1441
   <211> 12
   <212> PRT
   <213> Triticum aestivum
<400> 1441
   Ser Gly Gln Gly Ser Phe Gln Pro Ser Gln Gln Asn
   1 5 10
<210> 1442
   <211> 14
   <212> PRT
   <213> Triticum aestivum
<400> 1442
   Ser Gly Gln Gly Ser Phe Gln Pro Ser Gln Gln Asn Pro Gln
   1 5 10
<210> 1443
   <211> 20
   <212> PRT
   <213> Triticum aestivum
<400> 1443
   Ser Gln Gln Pro Tyr Leu Gln Leu Gln Pro Phe Pro Gln Pro Gln Leu
   1 5 10 15
   Pro Tyr Ser Gln
   20
<210> 1444
   <211> 20
   <212> PRT
   <213> Triticum aestivum
<400> 1444
   Thr Ile Pro Gln Pro His Gln Thr Phe His His Gln Pro Gln Gln Thr
   1 5 10 15
   Phe Pro Gln Pro
   20
<210> 1445
   <211> 20
   <212> PRT
   <213> Triticum aestivum
<400> 1445
   Thr Pro Ile Gln Pro Gln Gln Pro Phe Pro Gln Gln Pro Gln Gln Pro
   1 5 10 15
   Gln Gln Pro Phe
   20
<210> 1446
   <211> 15
   <212> PRT
   <213> Triticum aestivum
<400> 1446
   Val Gln Gly Gln Gly Ile Ile Gln Pro Gln Gln Pro Ala Gln Leu
   1 5 10 15
<210> 1447
   <211> 20
   <212> PRT
   <213> Triticum aestivum
<400> 1447
   Val Tyr Ile Pro Pro Tyr Cys Thr Ile Ala Pro Phe Gly Ile Phe Gly
   1 5 10 15
   Thr Asn Tyr Arg
   20
<210> 1448
   <211> 20
   <212> PRT
   <213> Triticum aestivum
<400> 1448
   Trp Pro Gln Gln Gln Pro Phe Pro Gln Pro Gln Gln Pro Phe Cys Gln
   1 5 10 15
   Gln Pro Gln Arg
   20
<210> 1449
   <211> 15
   <212> PRT
   <213> Triticum aestivum
<400> 1449
   Tyr Leu Gln Leu Gln Pro Phe Pro Gln Pro Gln Leu Pro Tyr Pro
   1               5                   10                  15
<210> 1450
   <211> 15
   <212> PRT
   <213> Triticum aestivum
<400> 1450
   Tyr Pro Gln Pro Gln Leu Pro Tyr Pro Gln Pro Gln Pro Phe Arg
   1               5                   10                  15
<210> 1451
   <211> 10
   <212> PRT
   <213> Triticum aestivum
<400> 1451
   Tyr Pro Gln Pro Gln Pro Phe Pro Ser Gln
   1               5                   10
<210> 1452
   <211> 15
   <212> PRT
   <213> Triticum aestivum
<400> 1452
   Tyr Pro Ser Gly Gln Gly Ser Phe Gln Pro Ser Gln Gln Asn Pro
   1               5                   10                  15
<210> 1453
   <211> 21
   <212> PRT
   <213> Triticum aestivum
<400> 1453
   Tyr Gln Gln Leu Pro Gln Pro Gln Gln Pro Gln Gln Ser Phe Pro Gln
   1               5                   10                  15
   Gln Gln Arg Pro Phe
               20
<210> 1454
   <211> 14
   <212> PRT
   <213> Unknown
<220>
   <223> epitope involved in celiac disease
<400> 1454
   Gln Leu Gln Pro Phe Pro Gln Pro Glu Leu Pro Tyr Pro Gln
   1 5 10
<210> 1455
   <211> 11
   <212> PRT
   <213> Triticum aestivum
<400> 1455
   Gly Ile Ile Gln Pro Gln Gln Pro Ala Gln Leu
   1 5 10
<210> 1456
   <211> 9
   <212> PRT
   <213> Triticum aestivum
<400> 1456
   Ile Gln Pro Gln Gln Pro Ala Gln Leu
   1 5
<210> 1457
   <211> 15
   <212> PRT
   <213> Triticum aestivum
<400> 1457
   Gln Pro Gln Gln Pro Gln Gln Ser Phe Pro Gln Gln Gln Arg Pro
   1 5 10 15
<210> 1458
   <211> 9
   <212> PRT
   <213> Triticum aestivum
<400> 1458
   Gln Gln Pro Phe Pro Gln Gln Pro Gln
   1 5
<210> 1459
   <211> 9
   <212> PRT
   <213> Triticum aestivum
<400> 1459
   Gln Gln Pro Gln Gln Pro Tyr Pro Gln
   1 5
<210> 1460
   <211> 9
   <212> PRT
   <213> Triticum aestivum
<400> 1460
   Ser Gln Pro Gln Gln Gln Phe Pro Gln
   1 5
<210> 1461
   <211> 18
   <212> PRT
   <213> Triticum aestivum
<400> 1461
   Phe Leu Gly Gln Gln Gln Pro Phe Pro Pro Gln Gln Pro Tyr Pro Gln
   1 5 10 15
   Pro Gln
<210> 1462
   <211> 20
   <212> PRT
   <213> Triticum aestivum
<400> 1462
   Phe Pro Gly Gln Gln Gln Pro Phe Pro Pro Gln Gln Pro Tyr Pro Gln
   1 5 10 15
   Pro Gln Pro Phe
   20
<210> 1463
   <211> 12
   <212> PRT
   <213> Triticum aestivum
<400> 1463
   Leu Gly Gln Gln Gln Pro Phe Pro Pro Gln Gln Pro
   1 5 10
<210> 1464
   <211> 19
   <212> PRT
   <213> Triticum aestivum
<400> 1464
   Gln Phe Leu Gly Gln Gln Gln Pro Phe Pro Pro Gln Gln Pro Tyr Pro
   1 5 10 15
   Gln Pro Gln
<210> 1465
   <211> 18
   <212> PRT
   <213> Triticum aestivum
<400> 1465
   Gln Asn Pro Ser Gln Gln Gln Pro Gln Glu Gln Val Pro Leu Val Gln
   1 5 10 15
   Gln Gln
<210> 1466
   <211> 19
   <212> PRT
   <213> Triticum aestivum
<400> 1466
   Val Arg Val Pro Val Pro Gln Leu Gln Pro Gln Asn Pro Ser Gln Gln
   1 5 10 15
   Gln Pro Gln
<210> 1467
   <211> 15
   <212> PRT
   <213> Triticum aestivum
<400> 1467
   Gln Pro Gln Gln Pro Gln Gln Ser Phe Pro Gln Gln Gln Arg Pro
   1 5 10 15
<210> 1468
   <211> 8
   <212> PRT
   <213> Triticum aestivum
<400> 1468
   Val Arg Val Pro Val Pro Gln Leu
   1 5
<210> 1469
   <211> 20
   <212> PRT
   <213> Unknown
<220>
   <223> epitope involved in celiac disease
<400> 1469
   Leu Gln Leu Gln Pro Phe Pro Gln Pro Glu Leu Pro Tyr Pro Gln Pro
   1 5 10 15
   Glu Leu Pro Tyr
   20
<210> 1470
   <211> 11
   <212> PRT
   <213> Unknown
<220>
   <223> epitope involved in celiac disease
<400> 1470
   Leu Gln Pro Phe Pro Gln Pro Glu Leu Pro Tyr
   1 5 10
<210> 1471
   <211> 14
   <212> PRT
   <213> Avena sativa
<400> 1471
   Gln Tyr Gln Pro Tyr Pro Glu Gln Gln Gln Pro Phe Val Gln
   1 5 10
<210> 1472
   <211> 19
   <212> PRT
   <213> Avena sativa
<400> 1472
   Ser Glu Gln Tyr Gln Pro Tyr Pro Glu Gln Gln Glu Pro Phe Val Gln
   1 5 10 15
   Gln Gln Gln
<210> 1473
   <211> 22
   <212> PRT
   <213> Avena nuda
<400> 1473
   Thr Thr Thr Val Gln Tyr Asp Pro Ser Glu Gln Tyr Gln Pro Tyr Pro
   1 5 10 15
   Glu Gln Gln Glu Pro Phe
   20
<210> 1474
   <211> 22
   <212> PRT
   <213> Avena sativa
<400> 1474
   Thr Thr Thr Val Gln Tyr Asn Pro Ser Glu Gln Tyr Gln Pro Tyr Pro
   1 5 10 15
   Glu Gln Gln Glu Pro Phe
   20
<210> 1475
   <211> 12
   <212> PRT
   <213> Avena sativa
<400> 1475
   Tyr Gln Pro Tyr Pro Glu Gln Gln Glu Pro Phe Val
   1 5 10
<210> 1476
   <211> 17
   <212> PRT
   <213> Triticum aestivum
<400> 1476
   Phe Pro Gln Pro Gln Leu Pro Tyr Pro Gln Pro Gln Ser Gln Val Leu
   1 5 10 15
   Gln
<210> 1477
   <211> 15
   <212> PRT
   <213> Triticum aestivum
<400> 1477
   Leu Gln Leu Gln Pro Phe Pro Gln Pro Gln Leu Pro Tyr Pro Gln
   1 5 10 15
<210> 1478
   <211> 10
   <212> PRT
   <213> Homo sapiens
<400> 1478
   Ala Ala Glu Lys Gln Met Lys Asp Lys Gln
   1               5                   10
<210> 1479
   <211> 10
   <212> PRT
   <213> Unknown
<220>
   <223> epitope involved in celiac disease
<400> 1479
   Ala Glu Asp Lys Glu Asp Asp Ala Glu Asp
   1               5                   10
<210> 1480
   <211> 10
   <212> PRT
   <213> Homo sapiens
<400> 1480
   Ala Lys Ile Asp Asp Pro Thr Asp Ser Lys
   1               5                   10
<210> 1481
   <211> 10
   <212> PRT
   <213> Unknown
<220>
   <223> epitope involved in celiac disease
<400> 1481
   Ala Ser Phe Ala Glu Ala Pro Phe Ser Asn
   1               5                   10
<210> 1482
   <211> 10
   <212> PRT
   <213> Unknown
<220>
   <223> epitope involved in celiac disease
<400> 1482
   Asp Ala Glu Asp Lys Asp Glu Asp Glu Glu
   1               5                   10
<210> 1483
   <211> 10
   <212> PRT
   <213> Homo sapiens
<400> 1483
   Asp Glu Glu Asp Lys Glu Glu Asp Glu Glu
   1 5 10
<210> 1484
   <211> 10
   <212> PRT
   <213> Unknown
<220>
   <223> epitope involved in celiac disease
<400> 1484
   Glu Asp Asp Ala Glu Asp Lys Asp Glu Asp
   1 5 10
<210> 1485
   <211> 10
   <212> PRT
   <213> Homo sapiens
<400> 1485
   Glu Asp Glu Glu Asp Glu Glu Asp Lys Glu
   1 5 10
<210> 1486
   <211> 10
   <212> PRT
   <213> Homo sapiens
<400> 1486
   Glu Asp Lys Asp Glu Asp Glu Glu Asp Glu
   1 5 10
<210> 1487
   <211> 10
   <212> PRT
   <213> Homo sapiens
<400> 1487
   Glu Asp Lys Glu Glu Asp Glu Glu Glu Asp
   1 5 10
<210> 1488
   <211> 10
   <212> PRT
   <213> Homo sapiens
<400> 1488
   Glu Asp Lys Lys Arg Lys Glu Glu Glu Glu
   1 5 10
<210> 1489
   <211> 10
   <212> PRT
   <213> Unknown
<220>
   <223> epitope involved in celiac disease
<400> 1489
   Glu Glu Ala Glu Asp Lys Glu Asp Asp Ala
   1 5 10
<210> 1490
   <211> 10
   <212> PRT
   <213> Homo sapiens
<400> 1490
   Glu Glu Asp Glu Glu Asp Lys Glu Glu Asp
   1 5 10
<210> 1491
   <211> 10
   <212> PRT
   <213> Homo sapiens
<400> 1491
   Glu Glu Glu Asp Lys Lys Arg Lys Glu Glu
   1 5 10
<210> 1492
   <211> 10
   <212> PRT
   <213> Homo sapiens
<400> 1492
   Glu Glu Glu Glu Ala Glu Asp Lys Glu Asp
   1 5 10
<210> 1493
   <211> 10
   <212> PRT
   <213> Homo sapiens
<400> 1493
   Glu Lys Asp Lys Gly Leu Gln Thr Ser Gln
   1 5 10
<210> 1494
   <211> 10
   <212> PRT
   <213> Homo sapiens
<400> 1494
   Glu Lys Gln Met Lys Asp Lys Gln Asp Glu
   1 5 10
<210> 1495
   <211> 10
   <212> PRT
   <213> Homo sapiens
<400> 1495
   Glu Gln Arg Leu Lys Glu Glu Glu Glu Asp
   1 5 10
<210> 1496
   <211> 10
   <212> PRT
   <213> Unknown
<220>
   <223> epitope involved in celiac disease
<400> 1496
   Phe Ala Glu Ala Pro Phe Ser Asn Lys Gly
   1 5 10
<210> 1497
   <211> 10
   <212> PRT
   <213> Homo sapiens
<400> 1497
   Gly Leu Gln Thr Ser Gln Asp Ala Arg Phe
   1 5 10
<210> 1498
   <211> 10
   <212> PRT
   <213> Homo sapiens
<400> 1498
   Gly Val Thr Lys Ala Ala Glu Lys Gln Met
   1 5 10
<210> 1499
   <211> 10
   <212> PRT
   <213> Homo sapiens
<400> 1499
   Ile Phe Asp Asn Phe Leu Ile Thr Asn Asp
   1 5 10
<210> 1500
   <211> 10
   <212> PRT
   <213> Homo sapiens
<400> 1500
   Lys Asp Glu Asp Glu Glu Asp Glu Glu Asp
   1 5 10
<210> 1501
   <211> 10
   <212> PRT
   <213> Homo sapiens
<400> 1501
   Lys Glu Glu Glu Glu Asp Lys Lys Arg Lys
   1 5 10
<210> 1502
   <211> 10
   <212> PRT
   <213> Homo sapiens
<400> 1502
   Lys Gly Lys Asn Val Leu Ile Asn Lys Asp
   1 5 10
<210> 1503
   <211> 10
   <212> PRT
   <213> Homo sapiens
<400> 1503
   Lys Ile Lys Asp Pro Asp Ala Ser Lys Pro
   1 5 10
<210> 1504
   <211> 10
   <212> PRT
   <213> Homo sapiens
<400> 1504
   Lys Leu Phe Pro Asn Ser Leu Asp Gln Thr
   1 5 10
<210> 1505
   <211> 10
   <212> PRT
   <213> Homo sapiens
<400> 1505
   Lys Gln Asp Glu Glu Gln Arg Leu Lys Glu
   1 5 10
<210> 1506
   <211> 10
   <212> PRT
   <213> Homo sapiens
<400> 1506
   Lys Ser Gly Thr Ile Phe Asp Asn Phe Leu
   1 5 10
<210> 1507
   <211> 10
   <212> PRT
   <213> Homo sapiens
<400> 1507
   Leu Pro Pro Lys Lys Ile Lys Asp Pro Asp
   1 5 10
<210> 1508
   <211> 10
   <212> PRT
   <213> Homo sapiens
<400> 1508
   Gln Val Lys Ser Gly Thr Ile Phe Asp Asn
   1 5 10
<210> 1509
   <211> 10
   <212> PRT
   <213> Homo sapiens
<400> 1509
   Arg Lys Glu Glu Glu Glu Ala Glu Asp Lys
   1 5 10
<210> 1510
   <211> 10
   <212> PRT
   <213> Homo sapiens
<400> 1510
   Arg Leu Lys Glu Glu Glu Glu Asp Lys Lys
   1 5 10
<210> 1511
   <211> 10
   <212> PRT
   <213> Homo sapiens
<400> 1511
   Thr Lys Ala Ala Glu Lys Gln Met Lys Asp
   1 5 10
<210> 1512
   <211> 21
   <212> PRT
   <213> Triticum aestivum
<400> 1512
   Ala His Gly Arg Ser Gln Val Leu Gln Gln Ser Thr Tyr Gln Leu Leu
   1 5 10 15
   Gln Glu Leu Cys Cys
   20
<210> 1513
   <211> 21
   <212> PRT
   <213> Triticum aestivum
<400> 1513
   Ala Gln Gly Ser Val Gln Pro Gln Gln Leu Pro Gln Phe Glu Glu Ile
   1 5 10 15
   Arg Asn Leu Ala Leu
   20
<210> 1514
   <211> 21
   <212> PRT
   <213> Triticum aestivum
<400> 1514
   Phe Cys Met Asp Val Val Leu Gln Gln His Asn Ile Ala His Gly Arg
   1 5 10 15
   Ser Gln Val Leu Gln
   20
<210> 1515
   <211> 18
   <212> PRT
   <213> Triticum aestivum
<400> 1515
   His Asn Ile Ala His Gly Arg Ser Gln Val Leu Gln Gln Ser Thr Tyr
   1               5                   10                  15
   Gln Leu
<210> 1516
   <211> 21
   <212> PRT
   <213> Triticum aestivum
<400> 1516
   Leu Cys Cys Gln His Leu Trp Gln Ile Pro Glu Gln Ser Gln Cys Gln
   1               5                   10                  15
   Ala Ile His Asn Val
               20
<210> 1517
   <211> 15
   <212> PRT
   <213> Triticum aestivum
<400> 1517
   Leu Pro Tyr Ser Gln Pro Gln Pro Phe Arg Pro Gln Gln Pro Tyr
   1               5                   10                  15
<210> 1518
   <211> 21
   <212> PRT
   <213> Triticum aestivum
<400> 1518
   Leu Pro Tyr Ser Gln Pro Gln Pro Phe Arg Pro Gln Gln Pro Tyr Pro
   1               5                   10                  15
   Gln Pro Gln Pro Gln
               20
<210> 1519
   <211> 21
   <212> PRT
   <213> Triticum aestivum
<400> 1519
   Leu Gln Gln His Asn Ile Ala His Gly Arg Ser Gln Val Leu Gln Gln
   1               5                   10                  15
   Ser Thr Tyr Gln Leu
               20
<210> 1520
   <211> 21
   <212> PRT
   <213> Triticum aestivum
<400> 1520
   Leu Gln Gln Gln Leu Ile Phe Cys Met Asp Val Val Leu Gln Gln His
   1               5                   10                  15
   Asn Ile Ala His Gly
               20
<210> 1521
   <211> 21
   <212> PRT
   <213> Triticum aestivum
<400> 1521
   Met Lys Thr Phe Leu Ile Leu Ala Leu Leu Ala Ile Val Ala Thr Thr
   1               5                   10                  15
   Ala Thr Thr Ala Val
               20
<210> 1522
   <211> 21
   <212> PRT
   <213> Triticum aestivum
<400> 1522
   Pro Leu Val Gln Gln Gln Gln Phe Leu Gly Gln Gln Gln Pro Phe Pro
   1               5                   10                  15
   Pro Gln Gln Pro Tyr
               20
<210> 1523
   <211> 18
   <212> PRT
   <213> Triticum aestivum
<400> 1523
   Pro Pro Gln Gln Pro Tyr Pro Gln Pro Gln Pro Phe Pro Ser Gln Gln
   1               5                   10                  15
   Pro Tyr
<210> 1524
   <211> 21
   <212> PRT
   <213> Triticum aestivum
<400> 1524
   Pro Gln Gln Pro Ile Ser Gln Gln Gln Gln Gln Gln Gln Gln Gln Gln
   1               5                   10                  15
   Gln Gln Gln Gln Gln
               20
<210> 1525
   <211> 21
   <212> PRT
   <213> Triticum aestivum
<400> 1525
   Gln Phe Leu Gly Gln Gln Gln Pro Phe Pro Pro Gln Gln Pro Tyr Pro
   1               5                   10                  15
   Gln Pro Gln Pro Phe
               20
<210> 1526
   <211> 21
   <212> PRT
   <213> Triticum aestivum
<400> 1526
   Gln Gly Ser Phe Arg Pro Ser Gln Gln Asn Pro Gln Ala Gln Gly Ser
   1               5                   10                  15
   Val Gln Pro Gln Gln
               20
<210> 1527
   <211> 21
   <212> PRT
   <213> Triticum aestivum
<400> 1527
   Gln Asn Pro Ser Gln Gln Gln Pro Gln Glu Gln Val Pro Leu Val Gln
   1               5                   10                  15
   Gln Gln Gln Phe Leu
               20
<210> 1528
   <211> 21
   <212> PRT
   <213> Triticum aestivum
<400> 1528
   Gln Pro Phe Pro Pro Gln Leu Pro Tyr Ser Gln Pro Gln Pro Phe Arg
   1               5                   10                  15
   Pro Gln Gln Pro Tyr
               20
<210> 1529
   <211> 21
   <212> PRT
   <213> Triticum aestivum
<400> 1529
   Gln Pro Phe Pro Pro Gln Gln Pro Tyr Pro Gln Pro Gln Pro Phe Pro
   1               5                   10                  15
   Ser Gln Gln Pro Tyr
               20
<210> 1530
   <211> 11
   <212> PRT
   <213> Triticum aestivum
<400> 1530
   Gln Pro Phe Pro Gln Pro Gln Leu Pro Tyr Pro
   1               5                   10
<210> 1531
   <211> 21
   <212> PRT
   <213> Triticum aestivum
<400> 1531
   Gln Pro Phe Pro Gln Pro Gln Leu Pro Tyr Ser Gln Pro Gln Pro Phe
   1               5                   10                  15
   Arg Pro Gln Gln Pro
               20
<210> 1532
   <211> 21
   <212> PRT
   <213> Triticum aestivum
<400> 1532
   Gln Pro Phe Pro Ser Gln Gln Pro Tyr Leu Gln Leu Gln Pro Phe Pro
   1 5 10 15
   Gln Pro Gln Leu Pro
   20
<210> 1533
   <211> 21
   <212> PRT
   <213> Triticum aestivum
<400> 1533
   Gln Pro Phe Arg Pro Gln Gln Pro Tyr Pro Gln Pro Gln Pro Gln Tyr
   1 5 10 15
   Ser Gln Pro Gln Gln
   20
<210> 1534
   <211> 21
   <212> PRT
   <213> Triticum aestivum
<400> 1534
   Gln Pro Gln Glu Gln Val Pro Leu Val Gln Gln Gln Gln Phe Leu Gly
   1 5 10 15
   Gln Gln Gln Pro Phe
   20
<210> 1535
   <211> 21
   <212> PRT
   <213> Triticum aestivum
<400> 1535
   Gln Pro Gln Tyr Ser Gln Pro Gln Gln Pro Ile Ser Gln Gln Gln Gln
   1 5 10 15
   Gln Gln Gln Gln Gln
   20
<210> 1536
   <211> 21
   <212> PRT
   <213> Triticum aestivum
<400> 1536
   Gln Pro Ser Ser Gln Val Ser Phe Gln Gln Pro Leu Gln Gln Tyr Pro
   1 5 10 15
   Leu Gly Gln Gly Ser
   20
<210> 1537
   <211> 21
   <212> PRT
   <213> Triticum aestivum
<400> 1537
   Gln Pro Tyr Leu Gln Leu Gln Pro Phe Pro Gln Pro Gln Leu Pro Tyr
   1 5 10 15
   Ser Gln Pro Gln Pro
   20
<210> 1538
   <211> 21
   <212> PRT
   <213> Triticum aestivum
<400> 1538
   Gln Pro Tyr Pro Gln Pro Gln Pro Phe Pro Ser Gln Gln Pro Tyr Leu
   1 5 10 15
   Gln Leu Gln Pro Phe
   20
<210> 1539
   <211> 21
   <212> PRT
   <213> Triticum aestivum
<400> 1539
   Gln Pro Tyr Pro Gln Pro Gln Pro Gln Tyr Ser Gln Pro Gln Gln Pro
   1 5 10 15
   Ile Ser Gln Gln Gln
   20
<210> 1540
   <211> 21
   <212> PRT
   <213> Triticum aestivum
<400> 1540
   Gln Gln Lys Gln Gln Gln Gln Pro Ser Ser Gln Val Ser Phe Gln Gln
   1 5 10 15
   Pro Leu Gln Gln Tyr
   20
<210> 1541
   <211> 18
   <212> PRT
   <213> Triticum aestivum
<400> 1541
   Gln Gln Gln Gln Phe Leu Gly Gln Gln Gln Pro Phe Pro Pro Gln Gln
   1 5 10 15
   Pro Tyr
<210> 1542
   <211> 18
   <212> PRT
   <213> Triticum aestivum
<400> 1542
   Gln Gln Gln Gln Pro Ser Ser Gln Val Ser Phe Gln Gln Pro Leu Gln
   1 5 10 15
   Gln Tyr
<210> 1543
   <211> 21
   <212> PRT
   <213> Triticum aestivum
<400> 1543
   Gln Gln Gln Gln Gln Gln Gln Gln Gln Gln Gln Gln Gln Ile Leu Gln
   1 5 10 15
   Gln Ile Leu Gln Gln
   20
<210> 1544
   <211> 21
   <212> PRT
   <213> Triticum aestivum
<400> 1544
   Gln Gln Gln Gln Gln Gln Gln Gln Gln Gln Gln Gln Gln Gln Gln Gln
   1 5 10 15
   Gln Gln Gln Ile Leu
   20
<210> 1545
   <211> 10
   <212> PRT
   <213> Triticum aestivum
<400> 1545
   Gln Gln Arg Pro Phe Ile Gln Pro Ser Leu
   1 5 10
<210> 1546
   <211> 21
   <212> PRT
   <213> Triticum aestivum
<400> 1546
   Gln Gln Tyr Pro Leu Gly Gln Gly Ser Phe Arg Pro Ser Gln Gln Asn
   1 5 10 15
   Pro Gln Ala Gln Gly
   20
<210> 1547
   <211> 21
   <212> PRT
   <213> Triticum aestivum
<400> 1547
   Ser Phe Gln Gln Pro Leu Gln Gln Tyr Pro Leu Gly Gln Gly Ser Phe
   1 5 10 15
   Arg Pro Ser Gln Gln
   20
<210> 1548
   <211> 21
   <212> PRT
   <213> Triticum aestivum
<400> 1548
   Ser Gln Cys Gln Ala Ile His Asn Val Val His Ala Ile Ile Leu His
   1 5 10 15
   Gln Gln Gln Gln Gln
   20
<210> 1549
   <211> 21
   <212> PRT
   <213> Triticum aestivum
<400> 1549
   Ser Gln Gln Asn Pro Gln Ala Gln Gly Ser Val Gln Pro Gln Gln Leu
   1 5 10 15
   Pro Gln Phe Glu Glu
   20
<210> 1550
   <211> 21
   <212> PRT
   <213> Triticum aestivum
<400> 1550
   Thr Ala Val Arg Val Pro Val Pro Gln Leu Gln Pro Gln Asn Pro Ser
   1 5 10 15
   Gln Gln Gln Pro Gln
   20
<210> 1551
   <211> 21
   <212> PRT
   <213> Triticum aestivum
<400> 1551
   Val Leu Gln Gln Ser Thr Tyr Gln Leu Leu Gln Glu Leu Cys Cys Gln
   1 5 10 15
   His Leu Trp Gln Ile
   20
<210> 1552
   <211> 21
   <212> PRT
   <213> Triticum aestivum
<400> 1552
   Val Pro Gln Leu Gln Pro Gln Asn Pro Ser Gln Gln Gln Pro Gln Glu
   1 5 10 15
   Gln Val Pro Leu Val
   20
<210> 1553
   <211> 21
   <212> PRT
   <213> Triticum aestivum
<400> 1553
   Tyr Gln Leu Leu Gln Glu Leu Cys Cys Gln His Leu Trp Gln Ile Pro
   1               5                   10                  15
   Glu Gln Ser Gln Cys
               20
<210> 1554
   <211> 12
   <212> PRT
   <213> Triticum aestivum
<400> 1554
   Ala His Gly Arg Ser Gln Val Leu Gln Gln Ser Thr
   1               5                   10
<210> 1555
   <211> 12
   <212> PRT
   <213> Triticum aestivum
<400> 1555
   Ala Leu Gln Thr Leu Pro Ala Met Cys Asn Val Tyr
   1               5                   10
<210> 1556
   <211> 12
   <212> PRT
   <213> Triticum aestivum
<400> 1556
   Ala Met Cys Asn Val Tyr Ile Pro Pro Tyr Cys Thr
   1               5                   10
<210> 1557
   <211> 12
   <212> PRT
   <213> Triticum aestivum
<400> 1557
   Glu Glu Ile Arg Asn Leu Ala Leu Gln Thr Leu Pro
   1               5                   10
<210> 1558
   <211> 12
   <212> PRT
   <213> Triticum aestivum
<400> 1558
   Phe Pro Pro Gln Gln Pro Tyr Pro Gln Pro Gln Pro
   1               5                   10
<210> 1559
   <211> 12
   <212> PRT
   <213> Triticum aestivum
<400> 1559
   Phe Pro Gln Pro Gln Leu Pro Tyr Ser Gln Pro Gln
   1 5 10
<210> 1560
   <211> 12
   <212> PRT
   <213> Triticum aestivum
<400> 1560
   Phe Pro Ser Gln Gln Pro Tyr Leu Gln Leu Gln Pro
   1 5 10
<210> 1561
   <211> 12
   <212> PRT
   <213> Triticum aestivum
<400> 1561
   Phe Gln Gln Pro Leu Gln Gln Tyr Pro Leu Gly Gln
   1 5 10
<210> 1562
   <211> 12
   <212> PRT
   <213> Triticum aestivum
<400> 1562
   Gly Ser Phe Arg Pro Ser Gln Gln Asn Pro Gln Ala
   1 5 10
<210> 1563
   <211> 12
   <212> PRT
   <213> Triticum aestivum
<400> 1563
   His Asn Val Val His Ala Ile Ile Leu His Gln Gln
   1 5 10
<210> 1564
   <211> 12
   <212> PRT
   <213> Triticum aestivum
<400> 1564
   Ile Ile Leu His Gln Gln Gln Lys Gln Gln Gln Gln
   1 5 10
<210> 1565
   <211> 12
   <212> PRT
   <213> Triticum aestivum
<400> 1565
   Ile Pro Pro Tyr Cys Thr Ile Ala Pro Phe Gly Ile
   1 5 10
<210> 1566
   <211> 12
   <212> PRT
   <213> Triticum aestivum
<400> 1566
   Leu Cys Cys Gln His Leu Trp Gln Ile Pro Glu Gln
   1 5 10
<210> 1567
   <211> 12
   <212> PRT
   <213> Triticum aestivum
<400> 1567
   Leu Gln Gln His Asn Ile Ala His Gly Arg Ser Gln
   1 5 10
<210> 1568
   <211> 12
   <212> PRT
   <213> Triticum aestivum
<400> 1568
   Leu Gln Gln Gln Leu Ile Pro Cys Met Asp Val Val
   1 5 10
<210> 1569
   <211> 12
   <212> PRT
   <213> Triticum aestivum
<400> 1569
   Pro Cys Met Asp Val Val Leu Gln Gln His Asn Ile
   1 5 10
<210> 1570
   <211> 12
   <212> PRT
   <213> Triticum aestivum
<400> 1570
   Pro Phe Arg Pro Gln Gln Pro Tyr Pro Gln Pro Gln
   1               5                   10
<210> 1571
   <211> 12
   <212> PRT
   <213> Triticum aestivum
<400> 1571
   Pro Gln Tyr Ser Gln Pro Gln Gln Pro Ile Ser Gln
   1               5                   10
<210> 1572
   <211> 12
   <212> PRT
   <213> Triticum aestivum
<400> 1572
   Pro Ser Gln Gln Gln Pro Gln Glu Gln Val Pro Leu
   1               5                   10
<210> 1573
   <211> 12
   <212> PRT
   <213> Triticum aestivum
<400> 1573
   Pro Ser Ser Gln Val Ser Phe Gln Gln Pro Leu Gln
   1               5                   10
<210> 1574
   <211> 12
   <212> PRT
   <213> Triticum aestivum
<400> 1574
   Pro Tyr Ser Gln Pro Gln Pro Phe Arg Pro Gln Gln
   1               5                   10
<210> 1575
   <211> 12
   <212> PRT
   <213> Triticum aestivum
<400> 1575
   Gln Glu Gln Val Pro Leu Val Gln Gln Gln Gln Phe
   1               5                   10
<210> 1576
   <211> 12
   <212> PRT
   <213> Triticum aestivum
<400> 1576
   Gln Gly Ser Val Gln Pro Gln Gln Leu Pro Gln Phe
   1               5                   10
<210> 1577
   <211> 12
   <212> PRT
   <213> Triticum aestivum
<400> 1577
   Gln Ile Leu Gln Gln Ile Leu Gln Gln Gln Leu Ile
   1               5                   10
<210> 1578
   <211> 12
   <212> PRT
   <213> Triticum aestivum
<400> 1578
   Gln Lys Gln Gln Gln Gln Pro Ser Ser Gln Val Ser
   1               5                   10
<210> 1579
   <211> 12
   <212> PRT
   <213> Triticum aestivum
<400> 1579
   Gln Leu Gln Pro Gln Asn Pro Ser Gln Gln Gln Pro
   1               5                   10
<210> 1580
   <211> 12
   <212> PRT
   <213> Triticum aestivum
<400> 1580
   Gln Gln Asn Pro Gln Ala Gln Gly Ser Val Gln Pro
   1               5                   10
<210> 1581
   <211> 12
   <212> PRT
   <213> Triticum aestivum
<400> 1581
   Gln Gln Pro Ile Ser Gln Gln Ile Leu Gln Gln Ile
   1 5 10
<210> 1582
   <211> 12
   <212> PRT
   <213> Triticum aestivum
<400> 1582
   Gln Tyr Pro Leu Gly Gln Gly Ser Phe Arg Pro Ser
   1 5 10
<210> 1583
   <211> 12
   <212> PRT
   <213> Triticum aestivum
<400> 1583
   Ser Gln Cys Gln Ala Ile His Asn Val Val His Ala
   1 5 10
<210> 1584
   <211> 11
   <212> PRT
   <213> Triticum aestivum
<400> 1584
   Thr Ile Ala Pro Phe Gly Ile Phe Gly Thr Asn
   1 5 10
<210> 1585
   <211> 12
   <212> PRT
   <213> Triticum aestivum
<400> 1585
   Val Leu Gln Gln Ser Thr Tyr Gln Leu Leu Gln Glu
   1 5 10
<210> 1586
   <211> 12
   <212> PRT
   <213> Triticum aestivum
<400> 1586
   Val Gln Gln Gln Gln Phe Leu Gly Gln Gln Gln Pro
   1 5 10
<210> 1587
   <211> 12
   <212> PRT
   <213> Triticum aestivum
<400> 1587
   Trp Gln Ile Pro Glu Gln Ser Gln Cys Gln Ala Ile
   1 5 10
<210> 1588
   <211> 12
   <212> PRT
   <213> Triticum aestivum
<400> 1588
   Tyr Leu Gln Leu Gln Pro Phe Pro Gln Pro Gln Leu
   1 5 10
<210> 1589
   <211> 12
   <212> PRT
   <213> Triticum aestivum
<400> 1589
   Tyr Pro Gln Pro Gln Pro Phe Pro Ser Gln Gln Pro
   1 5 10
<210> 1590
   <211> 12
   <212> PRT
   <213> Triticum aestivum
<400> 1590
   Tyr Gln Leu Leu Gln Glu Leu Cys Cys Gln His Leu
   1 5 10
<210> 1591
   <211> 15
   <212> PRT
   <213> Triticum aestivum
<400> 1591
   Gln Leu Gln Pro Phe Pro Gln Pro Gln Leu Pro Tyr Pro Gln Pro
   1 5 10 15
<210> 1592
   <211> 12
   <212> PRT
   <213> Unknown
<220>
   <223> epitope involved in celiac disease
<400> 1592
   Phe Pro Gln Gln Pro Glu Gln Pro Tyr Pro Gln Gln
   1 5 10
<210> 1593
   <211> 15
   <212> PRT
   <213> Triticum aestivum
<400> 1593
   Gly Gln Gly Ile Ile Gln Pro Gln Gln Pro Ala Gln Leu Ile Arg
   1               5                   10                  15
<210> 1594
   <211> 33
   <212> PRT
   <213> Triticum aestivum
<400> 1594
   Leu Gln Leu Gln Pro Phe Pro Gln Pro Gln Leu Pro Tyr Pro Gln Pro
   1               5                   10                  15
   Gln Leu Pro Tyr Pro Gln Pro Gln Leu Pro Tyr Pro Gln Pro Gln Pro
               20                  25                  30
   Phe
<210> 1595
   <211> 9
   <212> PRT
   <213> Triticum aestivum
<400> 1595
   Pro Phe Pro Gln Pro Gln Leu Pro Tyr
   1               5
<210> 1596
   <211> 15
   <212> PRT
   <213> Triticum aestivum
<400> 1596
   Pro Phe Pro Gln Gln Pro Gln Gln Pro Tyr Pro Gln Gln Pro Gln
   1               5                   10                  15
<210> 1597
   <211> 25
   <212> PRT
   <213> Triticum aestivum
<400> 1597
   Pro Gln Leu Pro Gln Phe Leu Gln Pro Gln Pro Tyr Pro Gln Pro Gln
   1               5                   10                  15
   Leu Pro Tyr Pro Gln Pro Gln Pro Phe
                       20                  25
<210> 1598
   <211> 9
   <212> PRT
   <213> Triticum aestivum
<400> 1598
   Pro Gln Pro Gln Leu Pro Gln Pro Gln
   1               5
<210> 1599
   <211> 17
   <212> PRT
   <213> Triticum aestivum
<400> 1599
   Pro Gln Pro Gln Pro Phe Leu Pro Gln Leu Pro Tyr Pro Gln Pro Gln
   1               5                   10                  15
   Ser
<210> 1600
   <211> 17
   <212> PRT
   <213> Triticum aestivum
<400> 1600
   Pro Gln Pro Gln Pro Phe Pro Pro Gln Leu Pro Tyr Pro Gln Pro Gln
   1               5                   10                  15
   Ser
<210> 1601
   <211> 14
   <212> PRT
   <213> Triticum aestivum
<400> 1601
   Pro Gln Gln Pro Phe Pro Ser Gln Gln Gln Gln Pro Leu Ile
   1               5                   10
<210> 1602
   <211> 15
   <212> PRT
   <213> Triticum aestivum
<400> 1602
   Pro Gln Gln Pro Gln Gln Ser Phe Pro Gln Gln Gln Gln Pro Ala
   1               5                   10                  15
<210> 1603
   <211> 14
   <212> PRT
   <213> Triticum aestivum
<400> 1603
   Pro Gln Gln Gln Thr Leu Gln Pro Gln Gln Pro Ala Gln Leu
   1               5                   10
<210> 1604
   <211> 15
   <212> PRT
   <213> Triticum aestivum
<400> 1604
   Gln Ile Pro Gln Gln Gln Gln Ile Pro Gln Gln Pro Gln Gln Phe
   1               5                   10                  15
<210> 1605
   <211> 17
   <212> PRT
   <213> Triticum aestivum
<400> 1605
   Gln Leu Gln Pro Phe Pro Gln Pro Thr Leu Pro Tyr Pro Gln Pro Gln
   1               5                   10                  15
   Ser
<210> 1606
   <211> 18
   <212> PRT
   <213> Triticum aestivum
<400> 1606
   Gln Pro Gln Pro Tyr Pro Gln Pro Gln Leu Pro Tyr Pro Gln Pro Gln
   1               5                   10                  15
   Pro Phe
<210> 1607
   <211> 17
   <212> PRT
   <213> Triticum aestivum
<400> 1607
   Gln Pro Gln Gln Pro Phe Pro Gln Pro Gln Gln Pro Phe Pro Trp Gln
   1 5 10 15
   Pro
<210> 1608
   <211> 15
   <212> PRT
   <213> Triticum aestivum
<400> 1608
   Gln Gln Phe Ser Gln Pro Gln Gln Gln Phe Pro Gln Pro Gln Gln
   1 5 10 15
<210> 1609
   <211> 18
   <212> PRT
   <213> Unknown
<220>
   <223> epitope involved in celiac disease
<400> 1609
   Gln Tyr Pro Ser Gly Glu Gly Ser Phe Gln Pro Ser Gln Glu Asn Pro
   1 5 10 15
   Gln Ala
<210> 1610
   <211> 6
   <212> PRT
   <213> Triticum aestivum
<400> 1610
   Gln Gln Gln Pro Phe Pro
   1 5
<210> 1611
   <211> 8
   <212> PRT
   <213> Homo sapiens
<400> 1611
   Ala Ser Phe Glu Pro Phe Ser Asn
   1 5
<210> 1612
   <211> 8
   <212> PRT
   <213> Homo sapiens
<400> 1612
   Phe Glu Pro Phe Ser Asn Lys Gly
   1               5
<210> 1613
   <211> 9
   <212> PRT
   <213> Triticum aestivum
<400> 1613
   Pro Gln Pro Gln Leu Pro Tyr Pro Gln
   1               5
<210> 1614
   <211> 9
   <212> PRT
   <213> Triticum aestivum
<400> 1614
   Gln Gly Ser Phe Gln Pro Ser Gln Gln
   1               5
<210> 1615
   <211> 18
   <212> PRT
   <213> Triticum aestivum
<400> 1615
   Gln Pro Gln Gln Pro Phe Pro Gln Pro Gln Gln Pro Phe Pro Trp Gln
   1               5                   10                  15
   Pro Gln
<210> 1616
   <211> 9
   <212> PRT
   <213> Triticum aestivum
<400> 1616
   Gln Gln Pro Gln Gln Pro Phe Pro Gln
   1               5
<210> 1617
   <211> 9
   <212> PRT
   <213> Triticum aestivum
<400> 1617
   Gln Gln Pro Gln Gln Pro Tyr Pro Gln
   1               5
<210> 1618
   <211> 13
   <212> PRT
   <213> Triticum aestivum
<400> 1618
   Leu Gly Glu Gly Ser Phe Gln Pro Ser Gln Glu Asn Pro
   1               5                   10
<210> 1619
   <211> 15
   <212> PRT
   <213> Triticum aestivum
<400> 1619
   Leu Gln Pro Phe Pro Gln Pro Gln Leu Pro Tyr Pro Gln Pro Gln
   1               5                   10                  15
<210> 1620
   <211> 15
   <212> PRT
   <213> Triticum aestivum
<400> 1620
   Leu Gln Pro Phe Pro Gln Pro Gln Leu Pro Tyr Ser Gln Pro Gln
   1               5                   10                  15
<210> 1621
   <211> 16
   <212> PRT
   <213> Triticum aestivum
<400> 1621
   Gln Leu Gln Pro Phe Pro His Pro Gln Leu Pro Tyr Pro His Pro Gln
   1               5                   10                  15
<210> 1622
   <211> 16
   <212> PRT
   <213> Triticum aestivum
<400> 1622
   Gln Leu Gln Pro Phe Pro His Pro Gln Leu Pro Tyr Pro Gln Pro Gln
   1               5                   10                  15
<210> 1623
   <211> 16
   <212> PRT
   <213> Triticum aestivum
<400> 1623
   Gln Leu Gln Pro Phe Pro Gln Ala Gln Leu Pro Tyr Ser Gln Pro Gln
   1 5 10 15
<210> 1624
   <211> 16
   <212> PRT
   <213> Triticum aestivum
<400> 1624
   Gln Leu Gln Pro Phe Pro Gln Pro Gln Leu Pro Tyr Pro Gln Pro Glu
   1 5 10 15
<210> 1625
   <211> 16
   <212> PRT
   <213> Triticum aestivum
<400> 1625
   Gln Leu Gln Pro Phe Pro Gln Pro Gln Leu Pro Tyr Pro Gln Pro Gln
   1 5 10 15
<210> 1626
   <211> 16
   <212> PRT
   <213> Triticum aestivum
<400> 1626
   Gln Leu Gln Pro Phe Pro Gln Pro Gln Leu Pro Tyr Ser Gln Pro Glu
   1 5 10 15
<210> 1627
   <211> 16
   <212> PRT
   <213> Triticum aestivum
<400> 1627
   Gln Leu Gln Pro Phe Pro Gln Pro Gln Leu Pro Tyr Ser Gln Pro Gln
   1 5 10 15
<210> 1628
   <211> 14
   <212> PRT
   <213> Aegilops tauschii
<400> 1628
   Gln Leu Gln Pro Phe Pro Gln Pro Gln Leu Pro Tyr Thr His
   1 5 10
<210> 1629
   <211> 16
   <212> PRT
   <213> Triticum aestivum
<400> 1629
   Gln Leu Gln Pro Phe Ser Gln Pro Gln Leu Pro Tyr Ser Gln Pro Gln
   1               5                   10                  15
<210> 1630
   <211> 17
   <212> PRT
   <213> Triticum aestivum
<400> 1630
   Gln Leu Gln Arg Pro Phe Pro Gln Pro Gln Leu Pro Tyr Pro Gln Pro
   1               5                   10                  15
   Glu
<210> 1631
   <211> 15
   <212> PRT
   <213> Triticum aestivum
<400> 1631
   Gln Pro Phe Arg Pro Gln Gln Pro Tyr Pro Gln Pro Gln Pro Gln
   1               5                   10                  15
<210> 1632
   <211> 15
   <212> PRT
   <213> Triticum aestivum
<400> 1632
   Gln Pro Gln Pro Phe Leu Pro Gln Leu Pro Tyr Pro Gln Pro Glu
   1               5                   10                  15
<210> 1633
   <211> 15
   <212> PRT
   <213> Triticum aestivum
<400> 1633
   Gln Pro Gln Pro Phe Gln Pro Gln Leu Pro Tyr Pro Gln Pro Glu
   1               5                   10                  15
<210> 1634
   <211> 14
   <212> PRT
   <213> Triticum aestivum
<400> 1634
   Pro Gln Thr Gln Gln Pro Gln Gln Pro Phe Pro Gln Pro Gln
   1               5                   10
<210> 1635
   <211> 20
   <212> PRT
   <213> Triticum aestivum
<400> 1635
   Phe Glu Glu Ile Arg Asn Leu Ala Leu Gln Thr Leu Pro Ala Met Cys
   1               5                   10                  15
   Asn Val Tyr Ile
               20
<210> 1636
   <211> 20
   <212> PRT
   <213> Triticum aestivum
<400> 1636
   Phe Leu Gly Gln Gln Gln Ser Phe Pro Pro Gln Gln Pro Tyr Pro Gln
   1               5                   10                  15
   Pro Gln Pro Phe
               20
<210> 1637
   <211> 17
   <212> PRT
   <213> Triticum aestivum
<400> 1637
   Pro Pro Tyr Cys Thr Ile Val Pro Phe Gly Ile Phe Gly Thr Asn Tyr
   1               5                   10                  15
   Arg
<210> 1638
   <211> 20
   <212> PRT
   <213> Triticum aestivum
<400> 1638
   Gln Leu Ile Pro Cys Met Asp Val Val Leu Gln Gln His Asn Ile Ala
   1               5                   10                  15
   His Gly Arg Ser
               20
<210> 1639
   <211> 20
   <212> PRT
   <213> Triticum aestivum
<400> 1639
   Gln Val Leu Gln Gln Ser Thr Tyr Gln Leu Leu Gln Glu Leu Cys Cys
   1               5                   10                  15
   Gln His Leu Trp
               20
<210> 1640
   <211> 4
   <212> PRT
   <213> Triticum aestivum
<400> 1640
   Phe Phe Gln Pro
   1
<210> 1641
   <211> 8
   <212> PRT
   <213> Triticum aestivum
<400> 1641
   Ile Ile Leu His Gln Gln His His
   1               5
<210> 1642
   <211> 4
   <212> PRT
   <213> Triticum aestivum
<400> 1642
   Ile Pro Glu Gln
   1
<210> 1643
   <211> 8
   <212> PRT
   <213> Triticum aestivum
<400> 1643
   Pro Leu Val Gln Glu Gln Gln Phe
   1               5
<210> 1644
   <211> 6
   <212> PRT
   <213> Triticum aestivum
<400> 1644
   Pro Gln Gln Leu Pro Gln
   1               5
<210> 1645
   <211> 6
   <212> PRT
   <213> Triticum aestivum
<400> 1645
   Gln Gly Phe Phe Gln Pro
   1               5
<210> 1646
   <211> 6
   <212> PRT
   <213> Triticum aestivum
<400> 1646
   Gln Gly Ser Phe Gln Pro
   1               5
<210> 1647
   <211> 6
   <212> PRT
   <213> Triticum aestivum
<400> 1647
   Gln Leu Gln Pro Phe Pro
   1               5
<210> 1648
   <211> 6
   <212> PRT
   <213> Triticum aestivum
<400> 1648
   Gln Pro Gln Pro Phe Pro
   1               5
<210> 1649
   <211> 6
   <212> PRT
   <213> Triticum aestivum
<400> 1649
   Gln Pro Gln Gln Pro Phe
   1               5
<210> 1650
   <211> 8
   <212> PRT
   <213> Triticum aestivum
<400> 1650
   Gln Val Pro Leu Val Gln Glu Gln
   1 5
<210> 1651
   <211> 6
   <212> PRT
   <213> Triticum aestivum
<400> 1651
   Trp Gln Ile Pro Glu Gln
   1 5
<210> 1652
   <211> 11
   <212> PRT
   <213> Triticum aestivum
<400> 1652
   Pro Gln Pro Gln Leu Pro Tyr Pro Gln Pro Gln
   1 5 10
<210> 1653
   <211> 10
   <212> PRT
   <213> Triticum aestivum
<400> 1653
   Phe Pro Pro Gln Gln Pro Tyr Pro Gln Pro
   1 5 10
<210> 1654
   <211> 10
   <212> PRT
   <213> Triticum aestivum
<400> 1654
   Leu Gln Gln Gln Gln Gln Gln Gln Gln Gln
   1 5 10
<210> 1655
   <211> 10
   <212> PRT
   <213> Triticum aestivum
<400> 1655
   Pro Gln Gln Pro Tyr Pro Gln Pro Gln Pro
   1 5 10
<210> 1656
   <211> 10
   <212> PRT
   <213> Triticum aestivum
<400> 1656
   Gln Lys Gln Gln Leu Gln Gln Gln Gln Gln
   1 5 10
<210> 1657
   <211> 10
   <212> PRT
   <213> Triticum aestivum
<400> 1657
   Gln Leu Gln Pro Phe Pro Gln Pro Gln Pro
   1 5 10
<210> 1658
   <211> 10
   <212> PRT
   <213> Triticum aestivum
<400> 1658
   Gln Pro Phe Pro Gln Pro Gln Pro Phe Pro
   1 5 10
<210> 1659
   <211> 10
   <212> PRT
   <213> Triticum aestivum
<400> 1659
   Gln Pro Tyr Leu Gln Leu Gln Pro Phe Pro
   1 5 10
<210> 1660
   <211> 10
   <212> PRT
   <213> Triticum aestivum
<400> 1660
   Gln Pro Tyr Pro Gln Pro Gln Pro Phe Pro
   1 5 10
<210> 1661
   <211> 10
   <212> PRT
   <213> Triticum aestivum
<400> 1661
   Gln Gln Gln Gln Gln Ile Leu Gln Gln Ile
   1 5 10
<210> 1662
   <211> 10
   <212> PRT
   <213> Triticum aestivum
<400> 1662
   Gln Gln Gln Gln Gln Gln Leu Gln Gln Gln
   1 5 10
<210> 1663
   <211> 10
   <212> PRT
   <213> Triticum aestivum
<400> 1663
   Gln Gln Gln Gln Gln Gln Gln Ile Leu Gln
   1 5 10
<210> 1664
   <211> 10
   <212> PRT
   <213> Triticum aestivum
<400> 1664
   Gln Gln Gln Gln Gln Gln Gln Gln Leu Gln
   1 5 10
<210> 1665
   <211> 10
   <212> PRT
   <213> Triticum aestivum
<400> 1665
   Gln Gln Gln Gln Gln Gln Gln Gln Gln Ile
   1 5 10
<210> 1666
   <211> 10
   <212> PRT
   <213> Triticum aestivum
<400> 1666
   Gln Val Ser Phe Gln Gln Pro Gln Gln Gln
   1 5 10
<210> 1667
   <211> 10
   <212> PRT
   <213> Triticum aestivum
<400> 1667
   Arg Asp Val Val Leu Gln Gln His Asn Ile
   1 5 10
<210> 1668
   <211> 10
   <212> PRT
   <213> Triticum aestivum
<400> 1668
   Arg Asn Leu Ala Leu Gln Thr Leu Pro Ala
   1 5 10
<210> 1669
   <211> 10
   <212> PRT
   <213> Triticum aestivum
<400> 1669
   Ser Gln Gln Pro Tyr Leu Gln Leu Gln Pro
   1 5 10
<210> 1670
   <211> 10
   <212> PRT
   <213> Triticum aestivum
<400> 1670
   Tyr Leu Gln Leu Gln Pro Phe Pro Gln Pro
   1 5 10
<210> 1671
   <211> 10
   <212> PRT
   <213> Triticum aestivum
<400> 1671
   Cys Gln Gln Leu Trp Gln Ile Pro Glu Gln
   1 5 10
<210> 1672
   <211> 10
   <212> PRT
   <213> Triticum aestivum
<400> 1672
   Phe Glu Glu Ile Arg Asn Leu Ala Leu Gln
   1 5 10
<210> 1673
   <211> 10
   <212> PRT
   <213> Triticum aestivum
<400> 1673
   Phe Leu Pro Gln Gln Pro Tyr Pro Gln Pro
   1 5 10
<210> 1674
   <211> 10
   <212> PRT
   <213> Triticum aestivum
<400> 1674
   Phe Gln Pro Gln Gln Leu Pro Gln Phe Glu
   1 5 10
<210> 1675
   <211> 10
   <212> PRT
   <213> Triticum aestivum
<400> 1675
   Gly Ser Phe Gln Pro Gln Gln Leu Pro Gln
   1 5 10
<210> 1676
   <211> 10
   <212> PRT
   <213> Triticum aestivum
<400> 1676
   Ile Ala Pro Phe Gly Ile Phe Gly Thr Asn
   1 5 10
<210> 1677
   <211> 10
   <212> PRT
   <213> Triticum aestivum
<400> 1677
   Leu Pro Tyr Pro Gln Pro Gln Pro Phe Leu
   1 5 10
<210> 1678
   <211> 10
   <212> PRT
   <213> Triticum aestivum
<400> 1678
   Leu Ser Gln Val Ser Phe Gln Gln Pro Gln
   1 5 10
<210> 1679
   <211> 10
   <212> PRT
   <213> Triticum aestivum
<400> 1679
   Pro Leu Val Gln Glu Gln Gln Phe Gln Gly
   1 5 10
<210> 1680
   <211> 10
   <212> PRT
   <213> Triticum aestivum
<400> 1680
   Pro Gln Phe Glu Glu Ile Arg Asn Leu Ala
   1 5 10
<210> 1681
   <211> 10
   <212> PRT
   <213> Triticum aestivum
<400> 1681
   Pro Gln Gln Leu Pro Gln Phe Glu Glu Ile
   1 5 10
<210> 1682
   <211> 10
   <212> PRT
   <213> Triticum aestivum
<400> 1682
   Pro Gln Gln Pro Ile Ser Gln Gln Gln Gln
   1 5 10
<210> 1683
   <211> 10
   <212> PRT
   <213> Triticum aestivum
<400> 1683
   Pro Gln Gln Gln Tyr Pro Ser Gly Gln Gly
   1 5 10
<210> 1684
   <211> 10
   <212> PRT
   <213> Triticum aestivum
<400> 1684
   Gln Glu Gln Val Pro Leu Val Gln Glu Gln
   1 5 10
<210> 1685
   <211> 10
   <212> PRT
   <213> Triticum aestivum
<400> 1685
   Gln Glu Ser Thr Tyr Gln Leu Val Gln Gln
   1 5 10
<210> 1686
   <211> 10
   <212> PRT
   <213> Triticum aestivum
<400> 1686
   Gln Gly Phe Phe Gln Pro Ser Gln Gln Asn
   1 5 10
<210> 1687
   <211> 10
   <212> PRT
   <213> Triticum aestivum
<400> 1687
   Gln Leu Pro Gln Phe Glu Glu Ile Arg Asn
   1 5 10
<210> 1688
   <211> 10
   <212> PRT
   <213> Triticum aestivum
<400> 1688
   Gln Leu Gln Leu Gln Asn Pro Ser Gln Gln
   1 5 10
<210> 1689
   <211> 10
   <212> PRT
   <213> Triticum aestivum
<400> 1689
   Gln Leu Trp Gln Ile Pro Glu Gln Ser Arg
   1 5 10
<210> 1690
   <211> 10
   <212> PRT
   <213> Triticum aestivum
<400> 1690
   Gln Pro Phe Leu Pro Gln Gln Pro Tyr Pro
   1 5 10
<210> 1691
   <211> 10
   <212> PRT
   <213> Triticum aestivum
<400> 1691
   Gln Pro Phe Pro Pro Gln Gln Pro Tyr Pro
   1 5 10
<210> 1692
   <211> 10
   <212> PRT
   <213> Triticum aestivum
<400> 1692
   Gln Pro Ile Ser Gln Gln Gln Gln Gln Gln
   1 5 10
<210> 1693
   <211> 10
   <212> PRT
   <213> Triticum aestivum
<400> 1693
   Gln Pro Leu Ser Gln Val Ser Phe Gln Gln
   1 5 10
<210> 1694
   <211> 10
   <212> PRT
   <213> Triticum aestivum
<400> 1694
   Gln Pro Gln Pro Phe Leu Pro Gln Gln Pro
   1 5 10
<210> 1695
   <211> 10
   <212> PRT
   <213> Triticum aestivum
<400> 1695
   Gln Pro Gln Tyr Ser Gln Pro Gln Gln Pro
   1 5 10
<210> 1696
   <211> 10
   <212> PRT
   <213> Triticum aestivum
<400> 1696
   Gln Gln His His His His Gln Glu Gln Lys
   1 5 10
<210> 1697
   <211> 10
   <212> PRT
   <213> Triticum aestivum
<400> 1697
   Gln Gln Pro Gln Gln Gln Tyr Pro Ser Gly
   1 5 10
<210> 1698
   <211> 10
   <212> PRT
   <213> Triticum aestivum
<400> 1698
   Gln Gln Gln Pro Leu Ser Gln Val Ser Phe
   1 5 10
<210> 1699
   <211> 10
   <212> PRT
   <213> Triticum aestivum
<400> 1699
   Gln Gln Gln Gln Gln Pro Leu Ser Gln Val
   1 5 10
<210> 1700
   <211> 10
   <212> PRT
   <213> Triticum aestivum
<400> 1700
   Gln Gln Gln Gln Gln Gln Gln Pro Leu Ser
   1 5 10
<210> 1701
   <211> 10
   <212> PRT
   <213> Triticum aestivum
<400> 1701
   Gln Gln Tyr Pro Ser Gly Gln Gly Phe Phe
   1 5 10
<210> 1702
   <211> 10
   <212> PRT
   <213> Triticum aestivum
<400> 1702
   Gln Val Pro Leu Val Gln Glu Gln Gln Phe
   1 5 10
<210> 1703
   <211> 10
   <212> PRT
   <213> Triticum aestivum
<400> 1703
   Gln Tyr Ser Gln Pro Gln Gln Pro Ile Ser
   1 5 10
<210> 1704
   <211> 10
   <212> PRT
   <213> Triticum aestivum
<400> 1704
   Ser Gly Gln Gly Phe Phe Gln Pro Ser Gln
   1 5 10
<210> 1705
   <211> 10
   <212> PRT
   <213> Triticum aestivum
<400> 1705
   Ser Gln Pro Gln Gln Pro Ile Ser Gln Gln
   1 5 10
<210> 1706
   <211> 10
   <212> PRT
   <213> Triticum aestivum
<400> 1706
   Ser Thr Thr Ile Ala Pro Phe Gly Ile Phe
   1 5 10
<210> 1707
   <211> 10
   <212> PRT
   <213> Triticum aestivum
<400> 1707
   Thr Ile Ala Pro Phe Gly Ile Phe Gly Thr
   1 5 10
<210> 1708
   <211> 10
   <212> PRT
   <213> Triticum aestivum
<400> 1708
   Val Leu Gln Glu Ser Thr Tyr Gln Leu Val
   1               5                   10
<210> 1709
   <211> 10
   <212> PRT
   <213> Triticum aestivum
<400> 1709
   Val Pro Gln Leu Gln Leu Gln Asn Pro Ser
   1               5                   10
<210> 1710
   <211> 10
   <212> PRT
   <213> Triticum aestivum
<400> 1710
   Val Pro Val Pro Gln Leu Gln Leu Gln Asn
   1               5                   10
<210> 1711
   <211> 10
   <212> PRT
   <213> Triticum aestivum
<400> 1711
   Val Arg Val Pro Val Pro Gln Leu Gln Leu
   1               5                   10
<210> 1712
   <211> 10
   <212> PRT
   <213> Triticum aestivum
<400> 1712
   Trp Gln Ile Pro Glu Gln Ser Arg Cys Gln
   1               5                   10
<210> 1713
   <211> 10
   <212> PRT
   <213> Triticum aestivum
<400> 1713
   Tyr Pro Gln Pro Gln Pro Phe Leu Pro Gln
   1               5                   10
<210> 1714
   <211> 10
   <212> PRT
   <213> Triticum aestivum
<400> 1714
   Tyr Pro Ser Gly Gln Gly Phe Phe Gln Pro
   1               5                   10
<210> 1715
   <211> 12
   <212> PRT
   <213> Triticum aestivum
<400> 1715
   Phe Pro Gln Pro Gln Gln Gln Phe Pro Gln Pro Gln
   1               5                   10
<210> 1716
   <211> 12
   <212> PRT
   <213> Triticum aestivum
<400> 1716
   Phe Pro Gln Gln Pro Gln Gln Pro Tyr Pro Gln Gln
   1               5                   10
<210> 1717
   <211> 20
   <212> PRT
   <213> Triticum aestivum
<400> 1717
   Leu Gln Pro Gln Gln Pro Phe Pro Gln Gln Pro Gln Gln Pro Tyr Pro
   1               5                   10                  15
   Gln Gln Pro Gln
               20
<210> 1718
   <211> 18
   <212> PRT
   <213> Triticum aestivum
<400> 1718
   Pro His Gln Pro Gln Gln Gln Val Pro Gln Pro Gln Gln Pro Gln Gln
   1 5 10 15
   Pro Phe
<210> 1719
   <211> 14
   <212> PRT
   <213> Triticum aestivum
<400> 1719
   Pro Gln Gln Pro Gln Gln Ser Phe Pro Gln Gln Gln Arg Pro
   1 5 10
<210> 1720
   <211> 17
   <212> PRT
   <213> Triticum aestivum
<400> 1720
   Gln Pro Gln Gln Pro Phe Pro Gln Pro Gln Gln Pro Phe Pro Trp Gln
   1 5 10 15
   Pro
<210> 1721
   <211> 23
   <212> PRT
   <213> Secale strictum
<400> 1721
   Gln Gln Pro Phe Pro Gln Gln Pro Gln Gln Pro Tyr Pro Gln Gln Pro
   1 5 10 15
   Gln Gln Pro Phe Pro Gln Pro
   20
<210> 1722
   <211> 19
   <212> PRT
   <213> Triticum aestivum
<400> 1722
   Ser His Gln Gln Gln Pro Phe Pro Gln Gln Pro Tyr Pro Gln Gln Pro
   1 5 10 15
   Tyr Pro Ser
<210> 1723
   <211> 9
   <212> PRT
   <213> Triticum aestivum
<400> 1723
   Glu Gly Ser Phe Gln Pro Ser Gln Glu
   1               5
<210> 1724
   <211> 9
   <212> PRT
   <213> Triticum aestivum
<400> 1724
   Gln Gly Tyr Tyr Pro Thr Ser Pro Gln
   1               5
<210> 1725
   <211> 687
   <212> PRT
   <213> Homo sapiens
<400> 1725
   Met Ala Glu Glu Leu Val Leu Glu Arg Cys Asp Leu Glu Leu Glu Thr
   1               5                   10                  15
   Asn Gly Arg Asp His His Thr Ala Asp Leu Cys Arg Glu Lys Leu Val
               20                  25                  30
   Val Arg Arg Gly Gln Pro Phe Trp Leu Thr Leu His Phe Glu Gly Arg
           35                  40                  45
   Asn Tyr Glu Ala Ser Val Asp Ser Leu Thr Phe Ser Val Val Thr Gly
       50                  55                  60
   Pro Ala Pro Ser Gln Glu Ala Gly Thr Lys Ala Arg Phe Pro Leu Arg
   65                  70                  75                  80
   Asp Ala Val Glu Glu Gly Asp Trp Thr Ala Thr Val Val Asp Gln Gln
                   85                  90                  95
   Asp Cys Thr Leu Ser Leu Gln Leu Thr Thr Pro Ala Asn Ala Pro Ile
               100                 105                 110
   Gly Leu Tyr Arg Leu Ser Leu Glu Ala Ser Thr Gly Tyr Gln Gly Ser
           115                 120                 125
   Ser Phe Val Leu Gly His Phe Ile Leu Leu Phe Asn Ala Trp Cys Pro
       130                 135                 140
   Ala Asp Ala Val Tyr Leu Asp Ser Glu Glu Glu Arg Gln Glu Tyr Val
   145                 150                 155                 160
   Leu Thr Gln Gln Gly Phe Ile Tyr Gln Gly Ser Ala Lys Phe Ile Lys
                   165                 170                 175
   Asn Ile Pro Trp Asn Phe Gly Gln Phe Glu Asp Gly Ile Leu Asp Ile
               180                 185                 190
   Cys Leu Ile Leu Leu Asp Val Asn Pro Lys Phe Leu Lys Asn Ala Gly
           195                 200                 205
   Arg Asp Cys Ser Arg Arg Ser Ser Pro Val Tyr Val Gly Arg Val Val
       210                 215                 220
   Ser Gly Met Val Asn Cys Asn Asp Asp Gln Gly Val Leu Leu Gly Arg
   225                 230                 235                 240
   Trp Asp Asn Asn Tyr Gly Asp Gly Val Ser Pro Met Ser Trp Ile Gly
                   245                 250                 255
   Ser Val Asp Ile Leu Arg Arg Trp Lys Asn His Gly Cys Gln Arg Val
               260                 265                 270
   Lys Tyr Gly Gln Cys Trp Val Phe Ala Ala Val Ala Cys Thr Val Leu
           275                 280                 285
   Arg Cys Leu Gly Ile Pro Thr Arg Val Val Thr Asn Tyr Asn Ser Ala
       290                 295                 300
   His Asp Gln Asn Ser Asn Leu Leu Ile Glu Tyr Phe Arg Asn Glu Phe
   305                 310                 315                 320
   Gly Glu Ile Gln Gly Asp Lys Ser Glu Met Ile Trp Asn Phe His Cys
                   325                 330                 335
   Trp Val Glu Ser Trp Met Thr Arg Pro Asp Leu Gln Pro Gly Tyr Glu
               340                 345                 350
   Gly Trp Gln Ala Leu Asp Pro Thr Pro Gln Glu Lys Ser Glu Gly Thr
           355                 360                 365
   Tyr Cys Cys Gly Pro Val Pro Val Arg Ala Ile Lys Glu Gly Asp Leu
   370 375 380
   Ser Thr Lys Tyr Asp Ala Pro Phe Val Phe Ala Glu Val Asn Ala Asp
   385 390 395 400
   Val Val Asp Trp Ile Gln Gln Asp Asp Gly Ser Val His Lys Ser Ile
   405 410 415
   Asn Arg Ser Leu Ile Val Gly Leu Lys Ile Ser Thr Lys Ser Val Gly
   420 425 430
   Arg Asp Glu Arg Glu Asp Ile Thr His Thr Tyr Lys Tyr Pro Glu Gly
   435 440 445
   Ser Ser Glu Glu Arg Glu Ala Phe Thr Arg Ala Asn His Leu Asn Lys
   450 455 460
   Leu Ala Glu Lys Glu Glu Thr Gly Met Ala Met Arg Ile Arg Val Gly
   465 470 475 480
   Gln Ser Met Asn Met Gly Ser Asp Phe Asp Val Phe Ala His Ile Thr
   485 490 495
   Asn Asn Thr Ala Glu Glu Tyr Val Cys Arg Leu Leu Leu Cys Ala Arg
   500 505 510
   Thr Val Ser Tyr Asn Gly Ile Leu Gly Pro Glu Cys Gly Thr Lys Tyr
   515 520 525
   Leu Leu Asn Leu Asn Leu Glu Pro Phe Ser Glu Lys Ser Val Pro Leu
   530 535 540
   Cys Ile Leu Tyr Glu Lys Tyr Arg Asp Cys Leu Thr Glu Ser Asn Leu
   545 550 555 560
   Ile Lys Val Arg Ala Leu Leu Val Glu Pro Val Ile Asn Ser Tyr Leu
   565 570 575
   Leu Ala Glu Arg Asp Leu Tyr Leu Glu Asn Pro Glu Ile Lys Ile Arg
   580 585 590
   Ile Leu Gly Glu Pro Lys Gln Lys Arg Lys Leu Val Ala Glu Val Ser
   595 600 605
   Leu Gln Asn Pro Leu Pro Val Ala Leu Glu Gly Cys Thr Phe Thr Val
   610 615 620
   Glu Gly Ala Gly Leu Thr Glu Glu Gln Lys Thr Val Glu Ile Pro Asp
   625 630 635 640
   Pro Val Glu Ala Gly Glu Glu Val Lys Val Arg Met Asp Leu Leu Pro
   645 650 655
   Leu His Met Gly Leu His Lys Leu Val Val Asn Phe Glu Ser Asp Lys
   660 665 670
   Leu Lys Ala Val Lys Gly Phe Arg Asn Val Ile Ile Gly Pro Ala
   675 680 685
<210> 1726
   <211> 15
   <212> PRT
   <213> Unknown
<220>
   <223> epitope involved in celiac disease
<400> 1726
   Phe Pro Glu Gln Pro Gln Gln Pro Tyr Pro Gln Gln Pro Gln Gln
   1 5 10 15
<210> 1727
   <211> 14
   <212> PRT
   <213> Unknown
<220>
   <223> epitope involved in celiac disease
<400> 1727
   Pro Gln Thr Glu Gln Pro Gln Gln Pro Phe Pro Gln Pro Gln
   1 5 10
<210> 1728
   <211> 13
   <212> PRT
   <213> Unknown
<220>
   <223> epitope involved in celiac disease
<400> 1728
   Gln Glu Gly Tyr Tyr Pro Thr Ser Pro Glu Gln Ser Gly
   1 5 10
<210> 1729
   <211> 13
   <212> PRT
   <213> Unknown
<220>
   <223> epitope involved in celiac disease
<400> 1729
   Gln Glu Gly Tyr Tyr Pro Thr Ser Pro Gln Gln Ser Gly
   1 5 10
<210> 1730
   <211> 13
   <212> PRT
   <213> Unknown
<220>
   <223> epitope involved in celiac disease
<400> 1730
   Gln Gln Gly Tyr Tyr Pro Thr Ser Pro Glu Gln Ser Gly
   1 5 10
<210> 1731
   <211> 14
   <212> PRT
   <213> Unknown
<220>
   <223> epitope involved in celiac disease
<400> 1731
   Ser Gly Glu Gly Ser Phe Gln Pro Ser Gln Gln Asn Pro Gln
   1 5 10
<210> 1732
   <211> 12
   <212> PRT
   <213> Unknown
<220>
   <223> epitope involved in celiac disease
<400> 1732
   Gln Leu Gln Pro Phe Ser Gln Pro Gln Leu Pro Tyr
   1 5 10
<210> 1733
   <211> 15
   <212> PRT
   <213> Unknown
<220>
   <223> epitope involved in celiac disease
<400> 1733
   Gln Gln Pro Pro Phe Ala Glu Gln Glu Gln Pro Val Leu Pro Gln
   1 5 10 15
<210> 1734
   <211> 15
   <212> PRT
   <213> Unknown
<220>
   <223> epitope involved in celiac disease
<400> 1734
   Gln Gln Pro Pro Phe Gly Glu Gln Glu Gln Pro Val Leu Pro Gln
   1 5 10 15
<210> 1735
   <211> 15
   <212> PRT
   <213> Triticum aestivum
<400> 1735
   Gln Gln Pro Pro Phe Ser Gln Gln Gln Gln Pro Val Leu Pro Gln
   1 5 10 15
<210> 1736
   <211> 15
   <212> PRT
   <213> Triticum aestivum
<400> 1736
   Gln Gln Pro Pro Phe Ser Gln Gln Gln Gln Gln Pro Leu Pro Gln
   1 5 10 15
<210> 1737
   <211> 20
   <212> PRT
   <213> Triticum aestivum
<400> 1737
   Gln Gln Gln Gln Pro Pro Phe Ser Gln Gln Gln Gln Ser Pro Phe Ser
   1 5 10 15
   Gln Gln Gln Gln
   20
<210> 1738
   <211> 14
   <212> PRT
   <213> Unknown
<220>
   <223> epitope involved in celiac disease
<400> 1738
   Ala Gln Pro Glu Gln Pro Glu Gln Pro Phe Pro Gln Pro Gln
   1               5                   10
<210> 1739
   <211> 14
   <212> PRT
   <213> Unknown
<220>
   <223> epitope involved in celiac disease
<400> 1739
   Lys Gln Pro Glu Gln Pro Glu Gln Pro Phe Pro Gln Pro Gln
   1               5                   10
<210> 1740
   <211> 14
   <212> PRT
   <213> Unknown
<220>
   <223> epitope involved in celiac disease
<400> 1740
   Pro Ala Pro Glu Gln Pro Glu Gln Pro Phe Pro Gln Pro Gln
   1               5                   10
<210> 1741
   <211> 14
   <212> PRT
   <213> Unknown
<220>
   <223> epitope involved in celiac disease
<400> 1741
   Pro Lys Pro Glu Gln Pro Glu Gln Pro Phe Pro Gln Pro Gln
   1               5                   10
<210> 1742
   <211> 14
   <212> PRT
   <213> Unknown
<220>
   <223> epitope involved in celiac disease
<400> 1742
   Pro Gln Ala Glu Gln Pro Glu Gln Pro Phe Pro Gln Pro Gln
   1               5                   10
<210> 1743
   <211> 14
   <212> PRT
   <213> Unknown
<220>
   <223> epitope involved in celiac disease
<400> 1743
   Pro Gln Lys Glu Gln Pro Glu Gln Pro Phe Pro Gln Pro Gln
   1               5                   10
<210> 1744
   <211> 14
   <212> PRT
   <213> Unknown
<220>
   <223> epitope involved in celiac disease
<400> 1744
   Pro Gln Pro Ala Gln Pro Glu Gln Pro Phe Pro Gln Pro Gln
   1               5                   10
<210> 1745
   <211> 14
   <212> PRT
   <213> Unknown
<220>
   <223> epitope involved in celiac disease
<400> 1745
   Pro Gln Pro Glu Ala Pro Glu Gln Pro Phe Pro Gln Pro Gln
   1               5                   10
<210> 1746
   <211> 14
   <212> PRT
   <213> Unknown
<220>
   <223> epitope involved in celiac disease
<400> 1746
   Pro Gln Pro Glu Gln Ala Glu Gln Pro Phe Pro Gln Pro Gln
   1               5                   10
<210> 1747
   <211> 14
   <212> PRT
   <213> Unknown
<220>
   <223> epitope involved in celiac disease
<400> 1747
   Pro Gln Pro Glu Gln Pro Ala Gln Pro Phe Pro Gln Pro Gln
   1 5 10
<210> 1748
   <211> 14
   <212> PRT
   <213> Unknown
<220>
   <223> epitope involved in celiac disease
<400> 1748
   Pro Gln Pro Glu Gln Pro Glu Ala Pro Phe Pro Gln Pro Gln
   1 5 10
<210> 1749
   <211> 14
   <212> PRT
   <213> Unknown
<220>
   <223> epitope involved in celiac disease
<400> 1749
   Pro Gln Pro Glu Gln Pro Glu Gln Ala Phe Pro Gln Pro Gln
   1 5 10
<210> 1750
   <211> 14
   <212> PRT
   <213> Unknown
<220>
   <223> epitope involved in celiac disease
<400> 1750
   Pro Gln Pro Glu Gln Pro Glu Gln Pro Ala Pro Gln Pro Gln
   1 5 10
<210> 1751
   <211> 14
   <212> PRT
   <213> Unknown
<220>
   <223> epitope involved in celiac disease
<400> 1751
   Pro Gln Pro Glu Gln Pro Glu Gln Pro Phe Ala Gln Pro Gln
   1 5 10
<210> 1752
   <211> 14
   <212> PRT
   <213> Unknown
<220>
   <223> epitope involved in celiac disease
<400> 1752
   Pro Gln Pro Glu Gln Pro Glu Gln Pro Phe Pro Ala Pro Gln
   1 5 10
<210> 1753
   <211> 14
   <212> PRT
   <213> Unknown
<220>
   <223> epitope involved in celiac disease
<400> 1753
   Pro Gln Pro Glu Gln Pro Glu Gln Pro Phe Pro Lys Pro Gln
   1 5 10
<210> 1754
   <211> 14
   <212> PRT
   <213> Unknown
<220>
   <223> epitope involved in celiac disease
<400> 1754
   Pro Gln Pro Glu Gln Pro Glu Gln Pro Phe Pro Gln Ala Gln
   1 5 10
<210> 1755
   <211> 14
   <212> PRT
   <213> Unknown
<220>
   <223> epitope involved in celiac disease
<400> 1755
   Pro Gln Pro Glu Gln Pro Glu Gln Pro Phe Pro Gln Lys Gln
   1 5 10
<210> 1756
   <211> 14
   <212> PRT
   <213> Unknown
<220>
   <223> epitope involved in celiac disease
<400> 1756
   Pro Gln Pro Glu Gln Pro Glu Gln Pro Phe Pro Gln Pro Ala
   1 5 10
<210> 1757
   <211> 14
   <212> PRT
   <213> Unknown
<220>
   <223> epitope involved in celiac disease
<400> 1757
   Pro Gln Pro Glu Gln Pro Glu Gln Pro Phe Pro Gln Pro Lys
   1 5 10
<210> 1758
   <211> 14
   <212> PRT
   <213> Unknown
<220>
   <223> epitope involved in celiac disease
<400> 1758
   Pro Gln Pro Glu Gln Pro Glu Gln Pro Phe Pro Gln Pro Gln
   1 5 10
<210> 1759
   <211> 14
   <212> PRT
   <213> Unknown
<220>
   <223> epitope involved in celiac disease
<400> 1759
   Pro Gln Pro Glu Gln Pro Gln Gln Pro Phe Pro Gln Pro Gln
   1 5 10
<210> 1760
   <211> 14
   <212> PRT
   <213> Unknown
<220>
   <223> epitope involved in celiac disease
<400> 1760
   Pro Gln Pro Gln Gln Pro Glu Gln Pro Phe Pro Gln Pro Gln
   1 5 10
<210> 1761
   <211> 17
   <212> PRT
   <213> Triticum aestivum
<400> 1761
   Gln Pro Gln Gln Pro Gln Gln Pro Phe Pro Gln Gln Gln Gln Pro Leu
   1               5                   10                  15
   Ile
<210> 1762
   <211> 17
   <212> PRT
   <213> Triticum aestivum
<400> 1762
   Gln Pro Gln Gln Pro Gln Gln Ser Phe Pro Gln Gln Gln Gln Leu Met
   1               5                   10                  15
   Ile
<210> 1763
   <211> 17
   <212> PRT
   <213> Triticum aestivum
<400> 1763
   Gln Pro Gln Gln Pro Gln Gln Ser Phe Pro Gln Gln Gln Gln Pro Ala
   1               5                   10                  15
   Ile
<210> 1764
   <211> 17
   <212> PRT
   <213> Triticum aestivum
<400> 1764
   Gln Pro Gln Gln Pro Gln Gln Ser Phe Pro Gln Gln Gln Gln Pro Leu
   1               5                   10                  15
   Ile
<210> 1765
   <211> 18
   <212> PRT
   <213> Triticum aestivum
<400> 1765
   Gln Gln Tyr Pro Ser Gly Gln Gly Ser Phe Gln Pro Ser Gln Gln Asn
   1 5 10 15
   Pro Gln
<210> 1766
   <211> 13
   <212> PRT
   <213> Triticum aestivum
<400> 1766
   Ser Gly Gln Gly Ser Phe Gln Pro Ser Gln Gln Asn Pro
   1 5 10
<210> 1767
   <211> 15
   <212> PRT
   <213> Homo sapiens
<400> 1767
   Cys Asp Gly Glu Arg Pro Thr Leu Ala Phe Leu Gln Asp Val Met
   1 5 10 15
<210> 1768
   <211> 9
   <212> PRT
   <213> Influenza A virus
<400> 1768
   Cys Thr Glu Leu Lys Leu Ser Asp Tyr
   1 5
<210> 1769
   <211> 10
   <212> PRT
   <213> Homo sapiens
<400> 1769
   Asp Gly Val Ala Val Leu Lys Val Gly Gly
   1 5 10
<210> 1770
   <211> 17
   <212> PRT
   <213> Human coxsackievirus
<400> 1770
   Glu Ala Ile Pro Ala Leu Thr Ala Val Glu Thr Gly His Thr Ser Gln
   1 5 10 15
   Val
<210> 1771
   <211> 17
   <212> PRT
   <213> Mycobacterium tuberculosis
<400> 1771
   Glu Gly Asp Glu Ala Thr Gly Ala Asn Ile Val Lys Val Ala Leu Glu
   1 5 10 15
   Ala
<210> 1772
   <211> 9
   <212> PRT
   <213> Influenza A virus
<400> 1772
   Glu Leu Arg Ser Arg Tyr Trp Ala Ile
   1 5
<210> 1773
   <211> 15
   <212> PRT
   <213> Homo sapiens
<400> 1773
   Phe Val Asn Gln His Leu Cys Gly Ser His Leu Val Glu Ala Leu
   1 5 10 15
<210> 1774
   <211> 20
   <212> PRT
   <213> Coxsackievirus B4
<400> 1774
   Gly Ala His Glu Thr Ser Leu Ser Ala Ser Gly Asn Ser Ile Ile His
   1 5 10 15
   Tyr Thr Asn Ile
   20
<210> 1775
   <211> 10
   <212> PRT
   <213> Homo sapiens
<400> 1775
   Gly Cys Ala Leu Leu Arg Cys Ile Pro Ala
   1               5                   10
<210> 1776
   <211> 10
   <212> PRT
   <213> Mycobacterium bovis
<400> 1776
   Gly Gly Val Ala Val Ile Lys Ala Gly Ala
   1               5                   10
<210> 1777
   <211> 9
   <212> PRT
   <213> Influenza A virus
<400> 1777
   Gly Ile Leu Gly Phe Val Phe Thr Leu
   1               5
<210> 1778
   <211> 20
   <212> PRT
   <213> Coxsackievirus B4
<400> 1778
   Gly Asn Ser Ile Ile His Tyr Thr Asn Ile Asn Tyr Tyr Lys Asp Ala
   1               5                   10                  15
   Ala Ser Asn Ser
               20
<210> 1779
   <211> 10
   <212> PRT
   <213> Mycobacterium bovis
<400> 1779
   Gly Val Thr Leu Leu Gln Ala Ala Pro Thr
   1               5                   10
<210> 1780
   <211> 13
   <212> PRT
   <213> Homo sapiens
<400> 1780
   Ile Ala Phe Thr Ser Glu His Ser His Phe Ser Leu Lys
   1               5                   10
<210> 1781
   <211> 9
   <212> PRT
   <213> Influenza A virus
<400> 1781
   Ile Leu Arg Gly Ser Val Ala His Lys
   1               5
<210> 1782
   <211> 10
   <212> PRT
   <213> Mycobacterium bovis
<400> 1782
   Ile Val Ala Gly Gly Gly Val Thr Leu Leu
   1               5                   10
<210> 1783
   <211> 10
   <212> PRT
   <213> Homo sapiens
<400> 1783
   Ile Val Leu Gly Gly Gly Cys Ala Leu Leu
   1               5                   10
<210> 1784
   <211> 9
   <212> PRT
   <213> Influenza A virus
<400> 1784
   Ile Tyr Ser Thr Val Ala Ser Ser Leu
   1               5
<210> 1785
   <211> 9
   <212> PRT
   <213> Lymphocytic choriomeningitis virus
<400> 1785
   Lys Ala Val Tyr Asn Phe Ala Thr Cys
   1               5
<210> 1786
   <211> 14
   <212> PRT
   <213> Human poliovirus
<400> 1786
   Lys Glu Val Pro Ala Leu Thr Ala Val Glu Thr Gly Ala Thr
   1               5                   10
<210> 1787
   <211> 12
   <212> PRT
   <213> Coxsackievirus B4
<400> 1787
   Lys Ile Leu Pro Glu Val Lys Glu Lys His Glu Phe
   1 5 10
<210> 1788
   <211> 10
   <212> PRT
   <213> Mycobacterium bovis
<400> 1788
   Leu Ala Lys Leu Ala Gly Gly Val Ala Val
   1 5 10
<210> 1789
   <211> 10
   <212> PRT
   <213> Homo sapiens
<400> 1789
   Leu Ala Lys Leu Ser Asp Gly Val Ala Val
   1 5 10
<210> 1790
   <211> 20
   <212> PRT
   <213> Mus musculus
<400> 1790
   Leu Val Leu Asn Arg Leu Lys Val Gly Leu Gln Val Val Ala Val Lys
   1 5 10 15
   Ala Pro Gly Phe
   20
<210> 1791
   <211> 20
   <212> PRT
   <213> Coxsackievirus B4
<400> 1791
   Met Gly Ala Gln Val Ser Thr Gln Lys Thr Gly Ala His Glu Thr Ser
   1 5 10 15
   Leu Ser Ala Ser
   20
<210> 1792
   <211> 20
   <212> PRT
   <213> Homo sapiens
<400> 1792
   Asn Met Tyr Ala Met Met Ile Ala Arg Phe Lys Met Phe Pro Glu Val
   1 5 10 15
   Lys Glu Lys Gly
   20
<210> 1793
   <211> 30
   <212> PRT
   <213> Coxsackievirus B4
<400> 1793
   Asn Tyr Tyr Lys Asp Ala Ala Ser Asn Ser Ala Asn Arg Gln Asp Phe
   1 5 10 15
   Thr Gln Asp Pro Ser Lys Phe Thr Glu Pro Val Lys Asp Val
   20 25 30
<210> 1794
   <211> 13
   <212> PRT
   <213> Influenza A virus
<400> 1794
   Pro Lys Tyr Val Lys Gln Asn Thr Leu Lys Leu Ala Thr
   1 5 10
<210> 1795
   <211> 15
   <212> PRT
   <213> Mycobacterium sp.
<400> 1795
   Pro Tyr Ile Leu Leu Val Ser Ser Lys Val Ser Thr Val Lys Asp
   1 5 10 15
<210> 1796
   <211> 14
   <212> PRT
   <213> Clostridium tetani
<400> 1796
   Gln Tyr Ile Lys Ala Asn Ser Lys Phe Ile Gly Ile Thr Glu
   1 5 10
<210> 1797
   <211> 11
   <212> PRT
   <213> Influenza A virus
<400> 1797
   Ser Phe Glu Arg Phe Glu Ile Phe Pro Lys Glu
   1 5 10
<210> 1798
   <211> 15
   <212> PRT
   <213> Mus musculus
<400> 1798
   Ser His Leu Val Glu Ala Leu Tyr Leu Val Cys Gly Glu Arg Gly
   1 5 10 15
<210> 1799
   <211> 8
   <212> PRT
   <213> Gallus gallus
<400> 1799
   Ser Ile Ile Asn Phe Glu Lys Leu
   1 5
<210> 1800
   <211> 19
   <212> PRT
   <213> Coxsackievirus B4
<400> 1800
   Ser Lys Phe Thr Glu Pro Val Lys Asp Val Met Ile Lys Ser Leu Pro
   1 5 10 15
   Ala Leu Asn
<210> 1801
   <211> 15
   <212> PRT
   <213> Mus musculus
<400> 1801
   Thr Tyr Glu Ile Ala Pro Val Phe Val Leu Leu Glu Tyr Val Thr
   1 5 10 15
<210> 1802
   <211> 24
   <212> PRT
   <213> Mycobacterium bovis
<400> 1802
   Val Ala Gly Gly Gly Val Thr Leu Leu Gln Ala Ala Pro Thr Leu Asp
   1 5 10 15
   Glu Leu Lys Leu Glu Gly Asp Glu
   20
<210> 1803
   <211> 24
   <212> PRT
   <213> Homo sapiens
<400> 1803
   Val Leu Gly Gly Gly Val Ala Leu Leu Arg Val Ile Pro Ala Leu Asp
   1 5 10 15
   Ser Leu Thr Pro Ala Asn Glu Asp
   20
<210> 1804
   <211> 20
   <212> PRT
   <213> Rubella virus
<400> 1804
   Ala Phe Gly His Ser Asp Ala Ala Cys Trp Gly Phe Pro Thr Asp Thr
   1 5 10 15
   Val Met Ser Val
   20
<210> 1805
   <211> 20
   <212> PRT
   <213> Homo sapiens
<400> 1805
   Glu Ile Ile Lys Arg Thr Leu Lys Ile Pro Ala Met Thr Ile Ala Lys
   1 5 10 15
   Asn Ala Gly Val
   20
<210> 1806
   <211> 20
   <212> PRT
   <213> Homo sapiens
<400> 1806
   Gly Glu Val Ile Val Thr Lys Asp Asp Ala Met Leu Leu Lys Gly Lys
   1 5 10 15
   Gly Asp Lys Ala
   20
<210> 1807
   <211> 20
   <212> PRT
   <213> Homo sapiens
<400> 1807
   Ile Val Leu Gly Gly Gly Cys Ala Leu Leu Arg Cys Ile Pro Ala Leu
   1 5 10 15
   Asp Ser Leu Thr
   20
<210> 1808
   <211> 20
   <212> PRT
   <213> Homo sapiens
<400> 1808
   Lys Phe Gly Ala Asp Ala Arg Ala Leu Met Leu Gln Gly Val Asp Leu
   1 5 10 15
   Leu Ala Asp Ala
   20
<210> 1809
   <211> 20
   <212> PRT
   <213> Homo sapiens
<400> 1809
   Leu Val Ile Ile Ala Glu Asp Val Asp Gly Glu Ala Leu Ser Thr Leu
   1 5 10 15
   Val Leu Asn Arg
   20
<210> 1810
   <211> 21
   <212> PRT
   <213> Rubella virus
<400> 1810
   Pro Thr Asp Val Ser Cys Glu Gly Leu Gly Ala Trp Val Pro Thr Ala
   1 5 10 15
   Pro Cys Ala Arg Ile
   20
<210> 1811
   <211> 9
   <212> PRT
   <213> Homo sapiens
<400> 1811
   Val Met Ala Pro Arg Thr Val Leu Leu
   1 5
<210> 1812
   <211> 19
   <212> PRT
   <213> Homo sapiens
<400> 1812
   Pro Gly Ser Gly Phe Trp Ser Phe Gly Ser Glu Asp Gly Ser Gly Asp
   1 5 10 15
   Ser Glu Asn
<210> 1813
   <211> 9
   <212> PRT
   <213> Homo sapiens
<400> 1813
   Ala Leu Trp Met Arg Leu Leu Pro Leu
   1 5
<210> 1814
   <211> 9
   <212> PRT
   <213> Homo sapiens
<400> 1814
   Phe Leu Phe Ala Val Gly Phe Tyr Leu
   1 5
<210> 1815
   <211> 9
   <212> PRT
   <213> Homo sapiens
<400> 1815
   Phe Leu Ile Val Leu Ser Val Ala Leu
   1 5
<210> 1816
   <211> 9
   <212> PRT
   <213> Homo sapiens
<400> 1816
   Phe Leu Trp Ser Val Phe Met Leu Ile
   1 5
<210> 1817
   <211> 26
   <212> PRT
   <213> Homo sapiens
<400> 1817
   Phe Trp Tyr Ile Pro Pro Ser Leu Arg Thr Leu Glu Asp Asn Glu Glu
   1 5 10 15
   Arg Met Ser Arg Leu Ser Lys Val Ala Pro
   20 25
<210> 1818
   <211> 13
   <212> PRT
   <213> Homo sapiens
<400> 1818
   Gly Ala His Gly Asp Thr Thr Pro Glu Tyr Gln Asp Leu
   1 5 10
<210> 1819
   <211> 10
   <212> PRT
   <213> Homo sapiens
<400> 1819
   Gly Ile Val Glu Gln Cys Cys Thr Ser Ile
   1 5 10
<210> 1820
   <211> 9
   <212> PRT
   <213> Homo sapiens
<400> 1820
   His Leu Val Glu Ala Leu Tyr Leu Val
   1 5
<210> 1821
   <211> 34
   <212> PRT
   <213> Homo sapiens
<400> 1821
   His Thr Asn Val Cys Phe Trp Tyr Ile Pro Pro Ser Leu Arg Thr Leu
   1               5                   10                  15
   Glu Asp Asn Glu Glu Arg Met Ser Arg Leu Ser Lys Val Ala Pro Val
               20                  25                  30
   Ile Lys
<210> 1822
   <211> 13
   <212> PRT
   <213> Homo sapiens
<400> 1822
   Lys Glu Arg Leu Ala Ala Leu Gly Pro Glu Gly Ala His
   1               5                   10
<210> 1823
   <211> 23
   <212> PRT
   <213> Homo sapiens
<400> 1823
   Lys Glu Arg Leu Ala Ala Leu Gly Pro Glu Gly Ala His Gly Asp Thr
   1               5                   10                  15
   Thr Pro Glu Tyr Gln Asp Leu
               20
<210> 1824
   <211> 9
   <212> PRT
   <213> Homo sapiens
<400> 1824
   Lys Leu Gln Val Phe Leu Ile Val Leu
   1               5
<210> 1825
   <211> 9
   <212> PRT
   <213> Homo sapiens
<400> 1825
   Leu Leu Pro Pro Leu Leu Glu His Leu
   1 5
<210> 1826
   <211> 6
   <212> PRT
   <213> Homo sapiens
<400> 1826
   Met Ala Ser Pro Gly Ser
   1 5
<210> 1827
   <211> 20
   <212> PRT
   <213> Homo sapiens
<400> 1827
   Met Ala Ser Pro Gly Ser Gly Phe Trp Ser Phe Gly Ser Glu Asp Gly
   1 5 10 15
   Ser Gly Asp Ser
   20
<210> 1828
   <211> 13
   <212> PRT
   <213> Homo sapiens
<400> 1828
   Asn Phe Phe Arg Met Val Ile Ser Asn Pro Ala Ala Thr
   1 5 10
<210> 1829
   <211> 13
   <212> PRT
   <213> Unknown
<220>
   <223> epitope involved in diabetes
<400> 1829
   Asn Phe Ile Arg Met Val Ile Ser Asn Pro Ala Ala Thr
   1 5 10
<210> 1830
   <211> 13
   <212> PRT
   <213> Unknown
<220>
   <223> epitope involved in diabetes
<400> 1830
   Gln Pro Leu Ala Leu Glu Gly Ser Leu Gln Ser Arg Gly
   1 5 10
<210> 1831
   <211> 9
   <212> PRT
   <213> Homo sapiens
<400> 1831
   Arg Leu Leu Cys Ala Leu Thr Ser Leu
   1 5
<210> 1832
   <211> 15
   <212> PRT
   <213> Homo sapiens
<400> 1832
   Arg Thr Leu Glu Asp Asn Glu Glu Arg Met Ser Arg Leu Ser Lys
   1 5 10 15
<210> 1833
   <211> 9
   <212> PRT
   <213> Homo sapiens
<400> 1833
   Ser Leu Ala Ala Gly Val Lys Leu Leu
   1 5
<210> 1834
   <211> 10
   <212> PRT
   <213> Homo sapiens
<400> 1834
   Ser Leu Gln Lys Arg Gly Ile Val Glu Gln
   1 5 10
<210> 1835
   <211> 9
   <212> PRT
   <213> Homo sapiens
<400> 1835
   Ser Leu Gln Pro Leu Ala Leu Glu Gly
   1 5
<210> 1836
   <211> 15
   <212> PRT
   <213> Homo sapiens
<400> 1836
   Ser Leu Gln Pro Leu Ala Leu Glu Gly Ser Leu Gln Lys Arg Gly
   1               5                   10                  15
<210> 1837
   <211> 15
   <212> PRT
   <213> Unknown
<220>
   <223> epitope involved in diabetes
<400> 1837
   Ser Leu Gln Pro Leu Ala Leu Glu Gly Ser Leu Gln Ser Arg Gly
   1               5                   10                  15
<210> 1838
   <211> 9
   <212> PRT
   <213> Homo sapiens
<400> 1838
   Ser Leu Ser Pro Leu Gln Ala Glu Leu
   1               5
<210> 1839
   <211> 9
   <212> PRT
   <213> Homo sapiens
<400> 1839
   Ser Leu Tyr Gln Leu Glu Asn Tyr Cys
   1               5
<210> 1840
   <211> 3
   <212> PRT
   <213> Homo sapiens
<400> 1840
   Thr Ser Ile
   1
<210> 1841
   <211> 585
   <212> PRT
   <213> Homo sapiens
<400> 1841
   Met Ala Ser Pro Gly Ser Gly Phe Trp Ser Phe Gly Ser Glu Asp Gly
   1               5                   10                  15
   Ser Gly Asp Ser Glu Asn Pro Gly Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ala Glu Lys Pro Ala Glu Ser Gly Gly Ser Gln Pro Pro
       50                  55                  60
   Arg Ala Ala Ala Arg Lys Ala Ala Cys Ala Cys Asp Gln Lys Pro Cys
   65                  70                  75                  80
   Ser Cys Ser Lys Val Asp Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Asn Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
               180                 185                 190
   Ala Asp Trp Leu Thr Ser Thr Ala Asn Thr Asn Met Phe Thr Tyr Glu
           195                 200                 205
   Ile Ala Pro Val Phe Val Leu Leu Glu Tyr Val Thr Leu Lys Lys Met
       210                 215                 220
   Arg Glu Ile Ile Gly Trp Pro Gly Gly Ser Gly Asp Gly Ile Phe Ser
   225                 230                 235                 240
   Pro Gly Gly Ala Ile Ser Asn Met Tyr Ala Met Met Ile Ala Arg Phe
                   245                 250                 255
   Lys Met Phe Pro Glu Val Lys Glu Lys Gly Met Ala Ala Leu Pro Arg
               260                 265                 270
   Leu Ile Ala Phe Thr Ser Glu His Ser His Phe Ser Leu Lys Lys Gly
           275                 280                 285
   Ala Ala Ala Leu Gly Ile Gly Thr Asp Ser Val Ile Leu Ile Lys Cys
       290                 295                 300
   Asp Glu Arg Gly Lys Met Ile Pro Ser Asp Leu Glu Arg Arg Ile Leu
   305                 310                 315                 320
   Glu Ala Lys Gln Lys Gly Phe Val Pro Phe Leu Val Ser Ala Thr Ala
                   325                 330                 335
   Gly Thr Thr Val Tyr Gly Ala Phe Asp Pro Leu Leu Ala Val Ala Asp
               340                 345                 350
   Ile Cys Lys Lys Tyr Lys Ile Trp Met His Val Asp Ala Ala Trp Gly
           355                 360                 365
   Gly Gly Leu Leu Met Ser Arg Lys His Lys Trp Lys Leu Ser Gly Val
       370                 375                 380
   Glu Arg Ala Asn Ser Val Thr Trp Asn Pro His Lys Met Met Gly Val
   385                 390                 395                 400
   Pro Leu Gln Cys Ser Ala Leu Leu Val Arg Glu Glu Gly Leu Met Gln
                   405                 410                 415
   Asn Cys Asn Gln Met His Ala Ser Tyr Leu Phe Gln Gln Asp Lys His
               420                 425                 430
   Tyr Asp Leu Ser Tyr Asp Thr Gly Asp Lys Ala Leu Gln Cys Gly Arg
           435                 440                 445
   His Val Asp Val Phe Lys Leu Trp Leu Met Trp Arg Ala Lys Gly Thr
       450                 455                 460
   Thr Gly Phe Glu Ala His Val Asp Lys Cys Leu Glu Leu Ala Glu Tyr
   465                 470                 475                 480
   Leu Tyr Asn Ile Ile Lys Asn Arg Glu Gly Tyr Glu Met Val Phe Asp
                   485                 490                 495
   Gly Lys Pro Gln His Thr Asn Val Cys Phe Trp Tyr Ile Pro Pro Ser
   500 505 510
   Leu Arg Thr Leu Glu Asp Asn Glu Glu Arg Met Ser Arg Leu Ser Lys
   515 520 525
   Val Ala Pro Val Ile Lys Ala Arg Met Met Glu Tyr Gly Thr Thr Met
   530 535 540
   Val Ser Tyr Gln Pro Leu Gly Asp Lys Val Asn Phe Phe Arg Met Val
   545 550 555 560
   Ile Ser Asn Pro Ala Ala Thr His Gln Asp Ile Asp Phe Leu Ile Glu
   565 570 575
   Glu Ile Glu Arg Leu Gly Gln Asp Leu
   580 585
<210> 1842
   <211> 14
   <212> PRT
   <213> Homo sapiens
<400> 1842
   Ala Phe Ile Glu Phe Lys Ala Asp Glu Lys Lys Glu Asp Glu
   1 5 10
<210> 1843
   <211> 15
   <212> PRT
   <213> Homo sapiens
<400> 1843
   Cys Phe Trp Tyr Ile Pro Pro Ser Leu Arg Thr Leu Glu Asp Asn
   1 5 10 15
<210> 1844
   <211> 15
   <212> PRT
   <213> Homo sapiens
<400> 1844
   Glu Arg Met Ser Arg Leu Ser Lys Val Ala Pro Val Ile Lys Ala
   1 5 10 15
<210> 1845
   <211> 18
   <212> PRT
   <213> Bos taurus
<400> 1845
   Phe Lys Ala Asp Glu Lys Lys Phe Trp Gly Lys Tyr Leu Tyr Glu Ile
   1               5                   10                  15
   Ala Arg
<210> 1846
   <211> 33
   <212> PRT
   <213> Homo sapiens
<400> 1846
   Asn Met Tyr Ala Met Met Ile Ala Arg Phe Lys Met Phe Pro Glu Val
   1               5                   10                  15
   Lys Glu Lys Gly Met Ala Ala Leu Pro Arg Leu Ile Ala Phe Thr Ser
               20                  25                  30
   Glu
<210> 1847
   <211> 20
   <212> PRT
   <213> Homo sapiens
<400> 1847
   Ser Arg Leu Ser Lys Val Ala Pro Val Ile Lys Ala Arg Met Met Glu
   1               5                   10                  15
   Tyr Gly Thr Thr
               20
<210> 1848
   <211> 41
   <212> PRT
   <213> Homo sapiens
<400> 1848
   Val Ser Tyr Gln Pro Leu Gly Asp Lys Val Asn Phe Phe Arg Met Val
   1               5                   10                  15
   Ile Ser Asn Pro Ala Ala Thr His Gln Asp Ile Asp Phe Leu Ile Glu
               20                  25                  30
   Glu Ile Glu Arg Leu Gly Gln Asp Leu
           35                  40
<210> 1849
   <211> 20
   <212> PRT
   <213> Homo sapiens
<400> 1849
   Ala Glu Asp Glu Val Gln Arg Glu Arg Val Ser Ala Lys Asn Ala Leu
   1 5 10 15
   Glu Ser Tyr Ala
   20
<210> 1850
   <211> 20
   <212> PRT
   <213> Homo sapiens
<400> 1850
   Ala Glu Lys Asp Glu Phe Glu His Lys Arg Lys Glu Leu Glu Gln Val
   1 5 10 15
   Cys Asn Pro Ile
   20
<210> 1851
   <211> 20
   <212> PRT
   <213> Homo sapiens
<400> 1851
   Ala Gly Gly Val Met Thr Ala Leu Ile Lys Arg Asn Ser Thr Ile Pro
   1 5 10 15
   Thr Lys Gln Thr
   20
<210> 1852
   <211> 10
   <212> PRT
   <213> Homo sapiens
<400> 1852
   Ala Lys Ser Lys Asn His Thr Thr His Asn
   1 5 10
<210> 1853
   <211> 9
   <212> PRT
   <213> Homo sapiens
<400> 1853
   Ala Leu Thr Ala Val Ala Glu Glu Val
   1 5
<210> 1854
   <211> 12
   <212> PRT
   <213> Homo sapiens
<400> 1854
   Ala Gln Val Ala Thr Ile Ser Ala Asn Gly Asp Lys
   1               5                   10
<210> 1855
   <211> 12
   <212> PRT
   <213> Homo sapiens
<400> 1855
   Ala Val Ala Asp Ile Cys Lys Lys Tyr Lys Ile Trp
   1               5                   10
<210> 1856
   <211> 20
   <212> PRT
   <213> Homo sapiens
<400> 1856
   Asp Gly Val Thr Val Ala Lys Ser Ile Asp Leu Lys Asp Lys Tyr Lys
   1               5                   10                  15
   Asn Ile Gly Ala
               20
<210> 1857
   <211> 31
   <212> PRT
   <213> Homo sapiens
<400> 1857
   Asp Lys Val Asn Phe Phe Arg Met Val Ile Ser Asn Pro Ala Ala Thr
   1               5                   10                  15
   His Gln Asp Ile Asp Phe Leu Ile Glu Glu Ile Glu Arg Leu Gly
               20                  25                  30
<210> 1858
   <211> 20
   <212> PRT
   <213> Homo sapiens
<400> 1858
   Asp Thr Glu Arg Leu Ile Gly Asp Ala Ala Lys Asn Gln Val Ala Leu
   1               5                   10                  15
   Asn Pro Gln Asn
   20
<210> 1859
   <211> 16
   <212> PRT
   <213> Homo sapiens
<400> 1859
   Glu Asp Leu Gln Val Gly Gln Val Glu Leu Gly Gly Gly Pro Gly Ala
   1 5 10 15
<210> 1860
   <211> 20
   <212> PRT
   <213> Mus musculus
<400> 1860
   Glu Glu Ile Ala Gln Val Ala Thr Ile Ser Ala Asn Gly Asp Lys Asp
   1 5 10 15
   Ile Gly Asn Ile
   20
<210> 1861
   <211> 20
   <212> PRT
   <213> Homo sapiens
<400> 1861
   Glu Glu Ile Ala Gln Val Ala Thr Ile Ser Ala Asn Gly Asp Lys Glu
   1 5 10 15
   Ile Gly Asn Ile
   20
<210> 1862
   <211> 9
   <212> PRT
   <213> Homo sapiens
<400> 1862
   Phe Leu Gln Asp Val Met Asn Ile Leu
   1 5
<210> 1863
   <211> 16
   <212> PRT
   <213> Homo sapiens
<400> 1863
   Phe Tyr Thr Pro Lys Thr Arg Arg Glu Ala Glu Asp Leu Gln Val Gly
   1 5 10 15
<210> 1864
   <211> 18
   <212> PRT
   <213> Homo sapiens
<400> 1864
   Gly Ala Gly Ser Leu Gln Pro Leu Ala Leu Glu Gly Ser Leu Gln Lys
   1 5 10 15
   Arg Gly
<210> 1865
   <211> 20
   <212> PRT
   <213> Homo sapiens
<400> 1865
   Gly Leu Asn Val Leu Arg Ile Ile Asn Glu Pro Thr Ala Ala Ala Ile
   1 5 10 15
   Ala Tyr Gly Leu
   20
<210> 1866
   <211> 10
   <212> PRT
   <213> Homo sapiens
<400> 1866
   Gly Ser Gly Pro Thr Ile Glu Glu Val Asp
   1 5 10
<210> 1867
   <211> 10
   <212> PRT
   <213> Homo sapiens
<400> 1867
   His Leu Cys Gly Ser His Leu Val Glu Ala
   1 5 10
<210> 1868
   <211> 16
   <212> PRT
   <213> Homo sapiens
<400> 1868
   His Leu Val Glu Ala Leu Tyr Leu Val Cys Gly Glu Arg Gly Phe Phe
   1 5 10 15
<210> 1869
   <211> 20
   <212> PRT
   <213> Homo sapiens
<400> 1869
   Ile Ala Tyr Gly Leu Asp Arg Thr Gly Lys Gly Glu Arg Asn Val Leu
   1 5 10 15
   Ile Phe Asp Leu
   20
<210> 1870
   <211> 15
   <212> PRT
   <213> Homo sapiens
<400> 1870
   Ile Asp Phe Leu Ile Glu Glu Ile Glu Arg Leu Gly Gln Asp Leu
   1 5 10 15
<210> 1871
   <211> 20
   <212> PRT
   <213> Homo sapiens
<400> 1871
   Ile Pro Pro Ser Leu Arg Thr Leu Glu Asp Asn Glu Glu Arg Met Ser
   1 5 10 15
   Arg Leu Ser Lys
   20
<210> 1872
   <211> 20
   <212> PRT
   <213> Homo sapiens
<400> 1872
   Lys Ala Asn Lys Ile Thr Ile Thr Asn Asp Lys Gly Arg Leu Ser Lys
   1 5 10 15
   Glu Glu Ile Glu
   20
<210> 1873
   <211> 20
   <212> PRT
   <213> Homo sapiens
<400> 1873
   Lys Ala Pro Gly Phe Gly Asp Asn Arg Lys Asn Gln Leu Lys Asp Met
   1               5                   10                  15
   Ala Ile Ala Thr
               20
<210> 1874
   <211> 20
   <212> PRT
   <213> Homo sapiens
<400> 1874
   Lys Glu Glu Ile Glu Arg Met Val Gln Glu Ala Glu Lys Tyr Lys Ala
   1               5                   10                  15
   Glu Asp Glu Val
               20
<210> 1875
   <211> 15
   <212> PRT
   <213> Homo sapiens
<400> 1875
   Lys Lys Gly Ala Ala Ala Leu Gly Ile Gly Thr Asp Ser Val Ile
   1               5                   10                  15
<210> 1876
   <211> 12
   <212> PRT
   <213> Homo sapiens
<400> 1876
   Lys Lys Tyr Lys Ile Trp Met His Val Asp Ala Ala
   1               5                   10
<210> 1877
   <211> 12
   <212> PRT
   <213> Homo sapiens
<400> 1877
   Lys Leu Cys Ala Leu Leu Tyr Gly Asp Ala Glu Lys
   1               5                   10
<210> 1878
   <211> 20
   <212> PRT
   <213> Homo sapiens
<400> 1878
   Lys Arg Thr Leu Ser Ser Ser Thr Gln Ala Ser Leu Glu Ile Asp Ser
   1 5 10 15
   Leu Phe Glu Gly
   20
<210> 1879
   <211> 18
   <212> PRT
   <213> Homo sapiens
<400> 1879
   Lys Val Asn Phe Phe Arg Met Val Ile Ser Asn Pro Ala Ala Thr His
   1 5 10 15
   Gln Asp
<210> 1880
   <211> 20
   <212> PRT
   <213> Homo sapiens
<400> 1880
   Leu Glu Ser Tyr Ala Phe Asn Met Lys Ser Ala Val Glu Asp Glu Gly
   1 5 10 15
   Leu Lys Gly Lys
   20
<210> 1881
   <211> 15
   <212> PRT
   <213> Homo sapiens
<400> 1881
   Leu Lys Lys Met Arg Glu Ile Ile Gly Trp Pro Gly Gly Ser Gly
   1 5 10 15
<210> 1882
   <211> 20
   <212> PRT
   <213> Homo sapiens
<400> 1882
   Leu Leu Ala Asp Ala Val Ala Val Thr Met Gly Pro Lys Gly Arg Thr
   1 5 10 15
   Val Ile Ile Glu
   20
<210> 1883
   <211> 20
   <212> PRT
   <213> Homo sapiens
<400> 1883
   Leu Leu Leu Leu Asp Val Ala Pro Leu Ser Leu Gly Leu Glu Thr Ala
   1 5 10 15
   Gly Gly Val Met
   20
<210> 1884
   <211> 9
   <212> PRT
   <213> Homo sapiens
<400> 1884
   Leu Leu Gln Glu Tyr Asn Trp Glu Leu
   1 5
<210> 1885
   <211> 9
   <212> PRT
   <213> Mus musculus
<400> 1885
   Leu Tyr Leu Val Cys Gly Glu Arg Gly
   1 5
<210> 1886
   <211> 20
   <212> PRT
   <213> Homo sapiens
<400> 1886
   Met Ala Lys Ala Ala Ala Val Gly Ile Asp Leu Gly Thr Thr Tyr Ser
   1 5 10 15
   Cys Val Gly Val
   20
<210> 1887
   <211> 12
   <212> PRT
   <213> Homo sapiens
<400> 1887
   Met Ala Ser Pro Gly Ser Gly Phe Trp Ser Phe Gly
   1               5                   10
<210> 1888
   <211> 20
   <212> PRT
   <213> Homo sapiens
<400> 1888
   Met Leu Arg Leu Pro Thr Val Phe Arg Gln Met Arg Pro Val Ser Arg
   1               5                   10                  15
   Val Leu Ala Pro
               20
<210> 1889
   <211> 20
   <212> PRT
   <213> Homo sapiens
<400> 1889
   Asn Asp Gln Gly Asn Arg Thr Thr Pro Ser Tyr Val Ala Phe Thr Asp
   1               5                   10                  15
   Thr Glu Arg Leu
               20
<210> 1890
   <211> 20
   <212> PRT
   <213> Homo sapiens
<400> 1890
   Asn Glu Glu Ala Gly Asp Gly Thr Thr Thr Ala Thr Val Leu Ala Arg
   1               5                   10                  15
   Ser Ile Ala Lys
               20
<210> 1891
   <211> 14
   <212> PRT
   <213> Homo sapiens
<400> 1891
   Asn Ile Leu Leu Gln Tyr Val Val Lys Ser Phe Asp Arg Ser
   1               5                   10
<210> 1892
   <211> 13
   <212> PRT
   <213> Homo sapiens
<400> 1892
   Asn Pro Ala Ala Thr His Gln Asp Ile Asp Phe Leu Ile
   1               5                   10
<210> 1893
   <211> 20
   <212> PRT
   <213> Homo sapiens
<400> 1893
   Asn Pro Val Glu Ile Arg Arg Gly Val Met Leu Ala Val Asp Ala Val
   1               5                   10                  15
   Ile Ala Glu Leu
               20
<210> 1894
   <211> 12
   <212> PRT
   <213> Homo sapiens
<400> 1894
   Pro Ala Glu Ser Gly Gly Ser Gln Pro Pro Arg Ala
   1               5                   10
<210> 1895
   <211> 20
   <212> PRT
   <213> Homo sapiens
<400> 1895
   Pro Ala Pro Gly Val Pro Gln Ile Glu Val Thr Phe Asp Ile Asp Ala
   1               5                   10                  15
   Asn Gly Ile Leu
               20
<210> 1896
   <211> 20
   <212> PRT
   <213> Homo sapiens
<400> 1896
   Pro Phe Gln Val Ile Asn Asp Gly Asp Lys Pro Lys Val Gln Val Ser
   1               5                   10                  15
   Tyr Lys Gly Glu
   20
<210> 1897
   <211> 20
   <212> PRT
   <213> Homo sapiens
<400> 1897
   Pro Gly Pro Gly Gly Phe Gly Ala Gln Gly Pro Lys Gly Gly Ser Gly
   1 5 10 15
   Ser Gly Pro Thr
   20
<210> 1898
   <211> 20
   <212> PRT
   <213> Homo sapiens
<400> 1898
   Pro Thr Lys Gln Thr Gln Ile Phe Thr Thr Tyr Ser Asp Asn Gln Pro
   1 5 10 15
   Gly Val Leu Ile
   20
<210> 1899
   <211> 13
   <212> PRT
   <213> Unknown
<220>
   <223> epitope involved in diabetes
<400> 1899
   Pro Val Gln Leu Ser Asn Gln Trp His Val Val Gly Ala
   1 5 10
<210> 1900
   <211> 20
   <212> PRT
   <213> Homo sapiens
<400> 1900
   Gln Asp Ala Tyr Val Leu Leu Ser Glu Lys Lys Ile Ser Ser Ile Gln
   1 5 10 15
   Ser Ile Val Pro
   20
<210> 1901
   <211> 21
   <212> PRT
   <213> Homo sapiens
<400> 1901
   Gln Ser Ile Val Pro Ala Leu Glu Ile Ala Asn Ala His Arg Lys Pro
   1               5                   10                  15
   Leu Val Ile Ile Ala
               20
<210> 1902
   <211> 20
   <212> PRT
   <213> Homo sapiens

<400> 1902
   Arg Phe Glu Glu Leu Cys Ser Asp Leu Phe Arg Ser Thr Leu Glu Pro
   1               5                   10                  15
   Val Glu Lys Ala
               20
<210> 1903
   <211> 20
   <212> PRT
   <213> Homo sapiens
<400> 1903
   Arg Lys Gly Val Ile Thr Val Lys Asp Gly Lys Thr Leu Asn Asp Glu
   1               5                   10                  15
   Leu Glu Ile Ile
               20
<210> 1904
   <211> 10
   <212> PRT
   <213> Homo sapiens
<400> 1904
   Arg Met Met Glu Tyr Gly Thr Thr Met Val
   1               5                   10
<210> 1905
   <211> 20
   <212> PRT
   <213> Homo sapiens
<400> 1905
   Arg Ser Ile Ala Lys Glu Gly Phe Glu Lys Ile Ser Lys Gly Ala Asn
   1               5                   10                  15
   Pro Val Glu Ile
               20
<210> 1906
   <211> 20
   <212> PRT
   <213> Homo sapiens
<400> 1906
   Arg Val Leu Ala Pro His Leu Thr Arg Ala Tyr Ala Lys Asp Val Lys
   1               5                   10                  15
   Phe Gly Ala Asp
               20
<210> 1907
   <211> 20
   <212> PRT
   <213> Homo sapiens
<400> 1907
   Ser Cys Val Gly Val Phe Gln His Gly Lys Val Glu Ile Ile Ala Asn
   1               5                   10                  15
   Asp Gln Gly Asn
               20
<210> 1908
   <211> 10
   <212> PRT
   <213> Homo sapiens
<400> 1908
   Ser Leu Tyr His Val Tyr Glu Val Asn Leu
   1               5                   10
<210> 1909
   <211> 12
   <212> PRT
   <213> Homo sapiens
<400> 1909
   Thr Ala Arg Ala Trp Cys Gln Val Ala Gln Lys Phe
   1               5                   10
<210> 1910
   <211> 20
   <212> PRT
   <213> Homo sapiens
<400> 1910
   Thr His Leu Gly Gly Glu Asp Phe Asp Asn Arg Leu Val Asn His Phe
   1               5                   10                  15
   Val Glu Glu Phe
               20
<210> 1911
   <211> 9
   <212> PRT
   <213> Homo sapiens
<400> 1911
   Thr Ile Ala Asp Phe Trp Gln Met Val
   1               5
<210> 1912
   <211> 20
   <212> PRT
   <213> Homo sapiens
<400> 1912
   Thr Ile Asp Asp Gly Ile Phe Glu Val Lys Ala Thr Ala Gly Asp Thr
   1               5                   10                  15
   His Leu Gly Gly
               20
<210> 1913
   <211> 20
   <212> PRT
   <213> Homo sapiens
<400> 1913
   Thr Val Ile Ile Glu Gln Ser Trp Gly Ser Pro Lys Val Thr Lys Asp
   1               5                   10                  15
   Gly Val Thr Val
               20
<210> 1914
   <211> 10
   <212> PRT
   <213> Homo sapiens
<400> 1914
   Val Cys Gly Glu Arg Gly Phe Phe Tyr Thr
   1               5                   10
<210> 1915
   <211> 20
   <212> PRT
   <213> Homo sapiens
<400> 1915
   Val Cys Asn Pro Ile Ile Ser Gly Leu Tyr Gln Gly Ala Gly Gly Pro
   1               5                   10                  15
   Gly Pro Gly Gly
               20
<210> 1916
   <211> 20
   <212> PRT
   <213> Homo sapiens
<400> 1916
   Val Asp Lys Cys Leu Glu Leu Ala Glu Tyr Leu Tyr Asn Ile Ile Lys
   1               5                   10                  15
   Asn Arg Glu Gly
               20
<210> 1917
   <211> 13
   <212> PRT
   <213> Homo sapiens
<400> 1917
   Val Glu Gln Cys Cys Thr Ser Ile Cys Ser Leu Tyr Gln
   1               5                   10
<210> 1918
   <211> 20
   <212> PRT
   <213> Homo sapiens
<400> 1918
   Val Ile Ala Glu Leu Lys Lys Gln Ser Lys Pro Val Thr Thr Pro Glu
   1               5                   10                  15
   Glu Ile Ala Gln
               20
<210> 1919
   <211> 9
   <212> PRT
   <213> Homo sapiens
<400> 1919
   Val Ile Val Met Leu Thr Pro Leu Val
   1 5
<210> 1920
   <211> 24
   <212> PRT
   <213> Unknown
<220>
   <223> epitope involved in diabetes
<400> 1920
   Val Leu Gly Gly Gly Val Leu Leu Leu Arg Val Ile Pro Ala Leu Asp
   1 5 10 15
   Ser Leu Thr Pro Ala Asn Glu Asp
   20
<210> 1921
   <211> 10
   <212> PRT
   <213> Homo sapiens
<400> 1921
   Val Met Asn Ile Leu Leu Gln Tyr Val Val
   1 5 10
<210> 1922
   <211> 20
   <212> PRT
   <213> Homo sapiens
<400> 1922
   Val Asn Met Val Glu Lys Gly Ile Ile Asp Pro Thr Lys Val Val Arg
   1 5 10 15
   Thr Ala Leu Leu
   20
<210> 1923
   <211> 20
   <212> PRT
   <213> Homo sapiens
<400> 1923
   Val Gln Lys Leu Leu Gln Asp Phe Phe Asn Gly Arg Asp Leu Asn Lys
   1 5 10 15
   Ser Ile Asn Pro
   20
<210> 1924
   <211> 20
   <212> PRT
   <213> Homo sapiens
<400> 1924
   Val Thr Asp Ala Leu Asn Ala Thr Arg Ala Ala Val Glu Glu Gly Ile
   1 5 10 15
   Val Leu Gly Gly
   20
<210> 1925
   <211> 9
   <212> PRT
   <213> Mus musculus
<400> 1925
   Val Tyr Leu Lys Thr Asn Val Phe Leu
   1 5
<210> 1926
   <211> 13
   <212> PRT
   <213> Homo sapiens
<400> 1926
   Ala Lys Gly Thr Thr Gly Phe Glu Ala His Val Asp Lys
   1 5 10
<210> 1927
   <211> 10
   <212> PRT
   <213> Homo sapiens
<400> 1927
   Ala Leu Trp Gly Pro Asp Pro Ala Ala Ala
   1 5 10
<210> 1928
   <211> 15
   <212> PRT
   <213> Homo sapiens
<400> 1928
   Phe Asp Arg Ser Thr Lys Val Ile Asp Phe His Tyr Pro Asn Glu
   1 5 10 15
<210> 1929
   <211> 20
   <212> PRT
   <213> Homo sapiens
<400> 1929
   Phe Phe Arg Met Val Ile Ser Asn Pro Ala Ala Thr His Gln Asp Ile
   1               5                   10                  15
   Asp Phe Leu Ile
               20
<210> 1930
   <211> 13
   <212> PRT
   <213> Homo sapiens
<400> 1930
   Phe Phe Tyr Thr Pro Lys Thr Arg Arg Glu Ala Glu Asp
   1               5                   10
<210> 1931
   <211> 17
   <212> PRT
   <213> Homo sapiens
<400> 1931
   Phe Tyr Thr Pro Lys Thr Arg Arg Glu Ala Glu Asp Leu Gln Val Gly
   1               5                   10                  15
   Gln
<210> 1932
   <211> 17
   <212> PRT
   <213> Homo sapiens
<400> 1932
   Gly Gly Pro Gly Ala Gly Ser Leu Gln Pro Leu Ala Leu Glu Gly Ser
   1               5                   10                  15
   Leu
<210> 1933
   <211> 12
   <212> PRT
   <213> Homo sapiens
<400> 1933
   Gly His Pro Arg Tyr Phe Asn Gln Leu Ser Thr Gly
   1               5                   10
<210> 1934
   <211> 20
   <212> PRT
   <213> Homo sapiens
<400> 1934
   Ile Pro Pro Ser Leu Arg Tyr Leu Glu Asp Asn Glu Glu Arg Met Ser
   1               5                   10                  15
   Arg Leu Ser Lys
               20
<210> 1935
   <211> 20
   <212> PRT
   <213> Mus musculus
<400> 1935
   Lys Gly Asp Lys Ala His Ile Glu Lys Arg Ile Gln Glu Ile Thr Glu
   1               5                   10                  15
   Gln Leu Asp Ile
               20
<210> 1936
   <211> 15
   <212> PRT
   <213> Homo sapiens
<400> 1936
   Lys His Tyr Asp Leu Ser Tyr Asp Thr Gly Asp Lys Ala Leu Gln
   1               5                   10                  15
<210> 1937
   <211> 15
   <212> PRT
   <213> Homo sapiens
<400> 1937
   Lys Arg Gly Ile Val Glu Gln Cys Cys Thr Ser Ile Cys Ser Leu
   1               5                   10                  15
<210> 1938
   <211> 9
   <212> PRT
   <213> Homo sapiens
<400> 1938
   Leu Ala Leu Glu Gly Ser Leu Gln Lys
   1               5
<210> 1939
   <211> 17
   <212> PRT
   <213> Homo sapiens
<400> 1939
   Leu Ala Leu Trp Gly Pro Asp Pro Ala Ala Ala Phe Val Asn Gln His
   1               5                   10                  15
   Leu
<210> 1940
   <211> 17
   <212> PRT
   <213> Homo sapiens
<400> 1940
   Leu Leu Pro Leu Leu Ala Leu Leu Ala Leu Trp Gly Pro Asp Pro Ala
   1               5                   10                  15
   Ala
<210> 1941
   <211> 9
   <212> PRT
   <213> Homo sapiens
<400> 1941
   Leu Asn Ile Asp Leu Leu Trp Ser Val
   1               5
<210> 1942
   <211> 14
   <212> PRT
   <213> Homo sapiens
<400> 1942
   Leu Pro Arg Leu Ile Ala Phe Thr Ser Glu His Ser His Phe
   1               5                   10
<210> 1943
   <211> 15
   <212> PRT
   <213> Homo sapiens
<400> 1943
   Leu Pro Arg Leu Ile Ala Phe Thr Ser Glu His Ser His Phe Ser
   1               5                   10                  15
<210> 1944
   <211> 17
   <212> PRT
   <213> Homo sapiens
<400> 1944
   Leu Val Glu Ala Leu Tyr Leu Val Cys Gly Glu Arg Gly Phe Phe Tyr
   1 5 10 15
   Thr
<210> 1945
   <211> 10
   <212> PRT
   <213> Unknown
<220>
   <223> epitope involved in diabetes
<400> 1945
   Leu Trp Glu Ile Glu Phe Ala Lys Gln Leu
   1 5 10
<210> 1946
   <211> 16
   <212> PRT
   <213> Homo sapiens
<400> 1946
   Met Ala Leu Trp Met Arg Leu Leu Pro Leu Leu Ala Leu Leu Ala Leu
   1 5 10 15
<210> 1947
   <211> 20
   <212> PRT
   <213> Homo sapiens
<400> 1947
   Asn Trp Glu Leu Ala Asp Gln Pro Gln Asn Leu Glu Glu Ile Leu Met
   1 5 10 15
   His Cys Gln Thr
   20
<210> 1948
   <211> 17
   <212> PRT
   <213> Homo sapiens
<400> 1948
   Pro Ala Ala Ala Phe Val Asn Gln His Leu Cys Gly Ser His Leu Val
   1 5 10 15
   Glu
<210> 1949
   <211> 14
   <212> PRT
   <213> Homo sapiens
<400> 1949
   Pro Gly Ser Gly Phe Trp Ser Phe Gly Ser Glu Asp Gly Ser
   1               5                   10
<210> 1950
   <211> 17
   <212> PRT
   <213> Homo sapiens
<400> 1950
   Gln His Leu Cys Gly Ser His Leu Val Glu Ala Leu Tyr Leu Val Cys
   1               5                   10                  15
   Gly
<210> 1951
   <211> 17
   <212> PRT
   <213> Homo sapiens
<400> 1951
   Arg Glu Ala Glu Asp Leu Gln Val Gly Gln Val Glu Leu Gly Gly Gly
   1               5                   10                  15
   Pro
<210> 1952
   <211> 12
   <212> PRT
   <213> Homo sapiens
<400> 1952
   Arg Leu Ile Ala Phe Thr Ser Glu His Ser His Phe
   1               5                   10
<210> 1953
   <211> 9
   <212> PRT
   <213> Homo sapiens
<400> 1953
   Arg Leu Leu Pro Leu Leu Ala Leu Leu
   1               5
<210> 1954
   <211> 15
   <212> PRT
   <213> Homo sapiens
<400> 1954
   Arg Met Met Glu Tyr Gly Thr Thr Met Val Ser Tyr Gln Pro Leu
   1               5                   10                  15
<210> 1955
   <211> 20
   <212> PRT
   <213> Mus musculus
<400> 1955
   Arg Thr Ala Leu Leu Asp Ala Ala Gly Val Ala Ser Leu Leu Thr Thr
   1               5                   10                  15
   Ala Glu Ala Val
               20
<210> 1956
   <211> 20
   <212> PRT
   <213> Mus musculus
<400> 1956
   Thr Ala Glu Ala Val Val Thr Glu Ile Pro Lys Glu Glu Lys Asp Pro
   1               5                   10                  15
   Gly Met Gly Ala
               20
<210> 1957
   <211> 20
   <212> PRT
   <213> Homo sapiens
<400> 1957
<210> 1958
   <211> 17
   <212> PRT
   <213> Homo sapiens
<400> 1958
   Val Cys Gly Glu Arg Gly Phe Phe Tyr Thr Pro Lys Thr Arg Arg Glu
   1 5 10 15
   Ala
<210> 1959
   <211> 17
   <212> PRT
   <213> Homo sapiens
<400> 1959
   Val Gly Gln Val Glu Leu Gly Gly Gly Pro Gly Ala Gly Ser Leu Gln
   1 5 10 15
   Pro
<210> 1960
   <211> 9
   <212> PRT
   <213> Homo sapiens
<400> 1960
   Val Leu Phe Gly Leu Gly Phe Ala Ile
   1 5
<210> 1961
   <211> 24
   <212> PRT
   <213> Homo sapiens
<400> 1961
   Val Leu Gly Gly Gly Cys Ala Leu Leu Arg Cys Ile Pro Ala Leu Asp
   1 5 10 15
   Ser Leu Thr Pro Ala Asn Glu Asp
   20
<210> 1962
   <211> 8
   <212> PRT
   <213> Homo sapiens
<400> 1962
   Trp Gly Pro Asp Pro Ala Ala Ala
   1 5
<210> 1963
   <211> 582
   <212> PRT
   <213> Homo sapiens
<400> 1963
   Pro Gly Ser Gly Phe Trp Ser Phe Gly Ser Glu Asp Gly Ser Gly Asp
   1               5                   10                  15
   Ser Glu Asn Pro Gly Thr Ala Arg Ala Trp Cys Gln Val Ala Gln Lys
               20                  25                  30
   Phe Thr Gly Gly Ile Gly Asn Lys Leu Cys Ala Leu Leu Tyr Gly Asp
           35                  40                  45
   Ala Glu Lys Pro Ala Glu Ser Gly Gly Ser Gln Pro Pro Arg Ala Ala
       50                  55                  60
   Ala Arg Lys Ala Ala Cys Ala Cys Asp Gln Lys Pro Cys Ser Cys Ser
   65                  70                  75                  80
   Lys Val Asp Val Asn Tyr Ala Phe Leu His Ala Thr Asp Leu Leu Pro
                   85                  90                  95
   Ala Cys Asp Gly Glu Arg Pro Thr Leu Ala Phe Leu Gln Asp Val Met
               100                 105                 110
   Asn Ile Leu Leu Gln Tyr Val Val Lys Ser Phe Asp Arg Ser Thr Lys
           115                 120                 125
   Val Ile Asp Phe His Tyr Pro Asn Glu Leu Leu Gln Glu Tyr Asn Trp
       130                 135                 140
   Glu Leu Ala Asp Gln Pro Gln Asn Leu Glu Glu Ile Leu Met His Cys
   145                 150                 155                 160
   Gln Thr Thr Leu Lys Tyr Ala Ile Lys Thr Gly His Pro Arg Tyr Phe
                   165                 170                 175
   Asn Gln Leu Ser Thr Gly Leu Asp Met Val Gly Leu Ala Ala Asp Trp
               180                 185                 190
   Leu Thr Ser Thr Ala Asn Thr Asn Met Phe Thr Tyr Glu Ile Ala Pro
           195                 200                 205
   Val Phe Val Leu Leu Glu Tyr Val Thr Leu Lys Lys Met Arg Glu Ile
       210                 215                 220
   Ile Gly Trp Pro Gly Gly Ser Gly Asp Gly Ile Phe Ser Pro Gly Gly
   225                 230                 235                 240
   Ala Ile Ser Asn Met Tyr Ala Met Met Ile Ala Arg Phe Lys Met Phe
                   245                 250                 255
   Pro Glu Val Lys Glu Lys Gly Met Ala Ala Leu Pro Arg Leu Ile Ala
               260                 265                 270
   Phe Thr Ser Glu His Ser His Phe Ser Leu Lys Lys Gly Ala Ala Ala
           275                 280                 285
   Leu Gly Ile Gly Thr Asp Ser Val Ile Leu Ile Lys Cys Asp Glu Arg
       290                 295                 300
   Gly Lys Met Ile Pro Ser Asp Leu Glu Arg Arg Ile Leu Glu Ala Lys
   305                 310                 315                 320
   Gln Lys Gly Phe Val Pro Phe Leu Val Ser Ala Thr Ala Gly Thr Thr
                   325                 330                 335
   Val Tyr Gly Ala Phe Asp Pro Leu Leu Ala Val Ala Asp Ile Cys Lys
               340                 345                 350
   Lys Tyr Lys Ile Trp Met His Val Asp Ala Ala Trp Gly Gly Gly Leu
           355                 360                 365
   Leu Met Ser Arg Lys His Lys Trp Lys Leu Ser Gly Val Glu Arg Ala
       370                 375                 380
   Asn Ser Val Thr Trp Asn Pro His Lys Met Met Gly Val Pro Leu Gln
   385                 390                 395                 400
   Cys Ser Ala Leu Leu Val Arg Glu Glu Gly Leu Met Gln Asn Cys Asn
                   405                 410                 415
   Gln Met His Ala Ser Tyr Leu Phe Gln Gln Asp Lys His Tyr Asp Leu
               420                 425                 430
   Ser Tyr Asp Thr Gly Asp Lys Ala Leu Gln Cys Gly Arg His Val Asp
           435                 440                 445
   Val Phe Lys Leu Trp Leu Met Trp Arg Ala Lys Gly Thr Thr Gly Phe
       450                 455                 460
   Glu Ala His Val Asp Lys Cys Leu Glu Leu Ala Glu Tyr Leu Tyr Asn
   465 470 475 480
   Ile Ile Lys Asn Arg Glu Gly Tyr Glu Met Val Phe Asp Gly Lys Pro
   485 490 495
   Gln His Thr Asn Val Cys Phe Trp Tyr Ile Pro Pro Ser Leu Arg Thr
   500 505 510
   Leu Glu Asp Asn Glu Glu Arg Met Ser Arg Leu Ser Lys Val Ala Pro
   515 520 525
   Val Ile Lys Ala Arg Met Met Glu Tyr Gly Thr Thr Met Val Ser Tyr
   530 535 540
   Gln Pro Leu Gly Asp Lys Val Asn Phe Phe Arg Met Val Ile Ser Asn
   545 550 555 560
   Pro Ala Ala Thr His Gln Asp Ile Asp Phe Leu Ile Glu Glu Ile Glu
   565 570 575
   Arg Leu Gly Gln Asp Leu
   580
<210> 1964
   <211> 11
   <212> PRT
   <213> Homo sapiens
<400> 1964
   Ala Phe Thr Ser Glu His Ser His Phe Ser Leu
   1 5 10
<210> 1965
   <211> 12
   <212> PRT
   <213> Homo sapiens
<400> 1965
   Ala Phe Thr Ser Glu His Ser His Phe Ser Leu Lys
   1 5 10
<210> 1966
   <211> 17
   <212> PRT
   <213> Homo sapiens
<400> 1966
   Ala Gly Ser Leu Gln Pro Leu Ala Leu Glu Gly Ser Leu Gln Lys Arg
   1 5 10 15
   Gly
<210> 1967
   <211> 9
   <212> PRT
   <213> Homo sapiens
<400> 1967
   Ala Leu Tyr Leu Val Cys Gly Glu Arg
   1 5
<210> 1968
   <211> 18
   <212> PRT
   <213> Homo sapiens
<400> 1968
   Ala Asn Thr Asn Met Phe Thr Tyr Glu Ile Ala Pro Val Phe Val Leu
   1 5 10 15
   Leu Glu
<210> 1969
   <211> 20
   <212> PRT
   <213> Homo sapiens
<400> 1969
   Ala Thr His Gln Asp Ile Asp Phe Leu Ile Glu Glu Ile Glu Arg Leu
   1 5 10 15
   Gly Gln Asp Leu
   20
<210> 1970
   <211> 9
   <212> PRT
   <213> Unknown
<220>
   <223> epitope involved in diabetes
<400> 1970
   Ala Tyr Asn Lys Ala Asn Trp Phe Leu
   1 5
<210> 1971
   <211> 16
   <212> PRT
   <213> Homo sapiens
<400> 1971
   Cys Cys Thr Ser Ile Cys Ser Leu Tyr Gln Leu Glu Asn Tyr Cys Asn
   1               5                   10                  15
<210> 1972
   <211> 11
   <212> PRT
   <213> Unknown
<220>
   <223> epitope involved in diabetes
<400> 1972
   Cys His Ala Val Lys Val Gly Asn Pro Ser Cys
   1               5                   10
<210> 1973
   <211> 11
   <212> PRT
   <213> Unknown
<220>
   <223> epitope involved in diabetes
<400> 1973
   Cys Pro Arg Asp Leu Asn Gly Lys Ser Gly Cys
   1               5                   10
<210> 1974
   <211> 20
   <212> PRT
   <213> Homo sapiens
<400> 1974
   Glu Ala Lys Gln Lys Gly Phe Val Pro Phe Leu Val Ser Ala Thr Ala
   1               5                   10                  15
   Gly Thr Thr Val
               20
<210> 1975
   <211> 20
   <212> PRT
   <213> Homo sapiens
<400> 1975
   Glu Arg Met Ser Arg Leu Ser Lys Val Ala Pro Val Ile Lys Ala Arg
   1               5                   10                  15
   Met Met Glu Tyr
   20
<210> 1976
   <211> 20
   <212> PRT
   <213> Homo sapiens
<400> 1976
   Glu Val Lys Glu Lys Gly Met Ala Ala Leu Pro Arg Leu Ile Ala Phe
   1 5 10 15
   Thr Ser Glu His
   20
<210> 1977
   <211> 11
   <212> PRT
   <213> Homo sapiens
<400> 1977
   Phe Lys Met Phe Pro Glu Val Lys Glu Lys Gly
   1 5 10
<210> 1978
   <211> 10
   <212> PRT
   <213> Homo sapiens
<400> 1978
   Phe Thr Ser Glu His Ser His Phe Ser Leu
   1 5 10
<210> 1979
   <211> 17
   <212> PRT
   <213> Homo sapiens
<400> 1979
   Phe Val Asn Gln His Leu Cys Gly Ser His Leu Val Glu Ala Leu Tyr
   1 5 10 15
   Leu
<210> 1980
   <211> 13
   <212> PRT
   <213> Homo sapiens
<400> 1980
   Phe Trp Tyr Ile Pro Pro Ser Leu Arg Thr Leu Glu Asp
   1 5 10
<210> 1981
   <211> 20
   <212> PRT
   <213> Homo sapiens
<400> 1981
   Gly Asp Lys Val Asn Phe Phe Arg Met Val Ile Ser Asn Pro Ala Ala
   1 5 10 15
   Thr His Gln Asp
   20
<210> 1982
   <211> 17
   <212> PRT
   <213> Homo sapiens
<400> 1982
   Gly Glu Arg Gly Phe Phe Tyr Thr Pro Lys Thr Arg Arg Glu Ala Glu
   1 5 10 15
   Asp
<210> 1983
   <211> 21
   <212> PRT
   <213> Homo sapiens
<400> 1983
   Gly Gly Gly Leu Leu Met Ser Arg Lys His Lys Trp Lys Leu Ser Gly
   1 5 10 15
   Val Glu Arg Ala Asn
   20
<210> 1984
   <211> 15
   <212> PRT
   <213> Homo sapiens
<400> 1984
   Gly Ile Val Glu Gln Cys Cys Thr Ser Ile Cys Ser Leu Tyr Gln
   1 5 10 15
<210> 1985
   <211> 19
   <212> PRT
   <213> Homo sapiens
<400> 1985
   Gly Leu Met Gln Asn Cys Asn Gln Met His Ala Ser Tyr Leu Phe Gln
   1               5                   10                  15
   Gln Asp Lys
<210> 1986
   <211> 17
   <212> PRT
   <213> Homo sapiens
<400> 1986
   Gly Gln Val Glu Leu Gly Gly Gly Pro Gly Ala Gly Ser Leu Gln Pro
   1               5                   10                  15
   Leu
<210> 1987
   <211> 15
   <212> PRT
   <213> Homo sapiens
<400> 1987
   Gly Ser His Leu Val Glu Ala Leu Tyr Leu Val Cys Gly Glu Arg
   1               5                   10                  15
<210> 1988
   <211> 17
   <212> PRT
   <213> Homo sapiens
<400> 1988
   Gly Ser Leu Gln Lys Arg Gly Ile Val Glu Gln Cys Cys Thr Ser Ile
   1               5                   10                  15
   Cys
<210> 1989
   <211> 20
   <212> PRT
   <213> Homo sapiens
<400> 1989
   Gly Thr Thr Met Val Ser Tyr Gln Pro Leu Gly Asp Lys Val Asn Phe
   1 5 10 15
   Phe Arg Met Val
   20
<210> 1990
   <211> 15
   <212> PRT
   <213> Homo sapiens
<400> 1990
   His Leu Cys Gly Ser His Leu Val Glu Ala Leu Tyr Leu Val Cys
   1 5 10 15
<210> 1991
   <211> 15
   <212> PRT
   <213> Homo sapiens
<400> 1991
   His Leu Val Glu Ala Leu Tyr Leu Val Cys Gly Glu Arg Gly Phe
   1 5 10 15
<210> 1992
   <211> 20
   <212> PRT
   <213> Homo sapiens
<400> 1992
   His Thr Asn Val Cys Phe Trp Tyr Ile Pro Pro Ser Leu Arg Thr Leu
   1 5 10 15
   Glu Asp Asn Glu
   20
<210> 1993
   <211> 14
   <212> PRT
   <213> Unknown
<220>
   <223> epitope involved in diabetes
<400> 1993
   Ile Met Glu Gln Cys Phe Glu Ile Gly Cys Pro Ala Thr Asn
   1 5 10
<210> 1994
   <211> 16
   <212> PRT
   <213> Homo sapiens
<400> 1994
   Ile Pro Pro Ser Leu Arg Thr Leu Glu Asp Asn Glu Glu Arg Met Ser
   1               5                   10                  15
<210> 1995
   <211> 20
   <212> PRT
   <213> Homo sapiens
<400> 1995
   Ile Ser Asn Pro Ala Ala Thr His Gln Asp Ile Asp Phe Leu Ile Glu
   1               5                   10                  15
   Glu Ile Glu Arg
               20
<210> 1996
   <211> 9
   <212> PRT
   <213> Unknown
<220>
   <223> epitope involved in diabetes
<400> 1996
   Lys Tyr Asn Lys Ala Ala Trp Phe Leu
   1               5
<210> 1997
   <211> 9
   <212> PRT
   <213> Unknown
<220>
   <223> epitope involved in diabetes
<400> 1997
   Lys Tyr Asn Lys Ala Asn Ala Phe Leu
   1               5
<210> 1998
   <211> 9
   <212> PRT
   <213> Unknown
<220>
   <223> epitope involved in diabetes
<400> 1998
   Lys Tyr Asn Lys Ala Asn Trp Ala Leu
   1               5
<210> 1999
   <211> 9
   <212> PRT
   <213> Unknown
<220>
   <223> epitope involved in diabetes
<400> 1999
   Lys Tyr Asn Lys Ala Asn Trp Phe Leu
   1               5
<210> 2000
   <211> 17
   <212> PRT
   <213> Homo sapiens
<400> 2000
   Leu Cys Gly Ser His Leu Val Glu Ala Leu Tyr Leu Val Cys Gly Glu
   1               5                   10                  15
   Arg
<210> 2001
   <211> 14
   <212> PRT
   <213> Unknown
<220>
   <223> epitope involved in diabetes
<400> 2001
   Leu Met Glu Pro Cys Tyr Ala Trp Gly Cys Thr Gly Pro Lys
   1               5                   10
<210> 2002
   <211> 15
   <212> PRT
   <213> Homo sapiens
<400> 2002
   Leu Val Glu Ala Leu Tyr Leu Val Cys Gly Glu Arg Gly Phe Phe
   1               5                   10                  15
<210> 2003
   <211> 15
   <212> PRT
   <213> Homo sapiens
<400> 2003
   Leu Tyr Leu Val Cys Gly Glu Arg Gly Phe Phe Tyr Thr Pro Lys
   1               5                   10                  15
<210> 2004
   <211> 15
   <212> PRT
   <213> Homo sapiens
<400> 2004
   Met Ile Ala Arg Phe Lys Met Phe Pro Glu Val Lys Glu Lys Gly
   1 5 10 15
<210> 2005
   <211> 20
   <212> PRT
   <213> Homo sapiens
<400> 2005
   Met Met Ile Ala Arg Phe Lys Met Phe Pro Glu Val Lys Glu Lys Gly
   1 5 10 15
   Met Ala Ala Leu
   20
<210> 2006
   <211> 10
   <212> PRT
   <213> Homo sapiens
<400> 2006
   Met Tyr Ala Met Met Ile Ala Arg Phe Lys
   1 5 10
<210> 2007
   <211> 12
   <212> PRT
   <213> Homo sapiens
<400> 2007
   Met Tyr Ala Met Met Ile Ala Arg Phe Lys Met Phe
   1 5 10
<210> 2008
   <211> 14
   <212> PRT
   <213> Unknown
<220>
   <223> epitope involved in diabetes
<400> 2008
   Asn Asp Ser Gln Cys Trp Lys Leu Gly Cys Ile Ala Glu Lys
   1 5 10
<210> 2009
   <211> 12
   <212> PRT
   <213> Homo sapiens
<400> 2009
   Asn Ile Leu Leu Gln Tyr Val Val Lys Ser Phe Asp
   1               5                   10
<210> 2010
   <211> 14
   <212> PRT
   <213> Unknown
<220>
   <223> epitope involved in diabetes
<400> 2010
   Asn Pro Glu Asp Cys Phe Lys Thr Gly Cys Asn Ser Pro Thr
   1               5                   10
<210> 2011
   <211> 12
   <212> PRT
   <213> Homo sapiens
<400> 2011
   Asn Tyr Ala Phe Leu His Ala Thr Asp Leu Leu Pro
   1               5                   10
<210> 2012
   <211> 20
   <212> PRT
   <213> Homo sapiens
<400> 2012
   Pro Ser Leu Arg Thr Leu Glu Asp Asn Glu Glu Arg Met Ser Arg Leu
   1               5                   10                  15
   Ser Lys Val Ala
               20
<210> 2013
   <211> 20
   <212> PRT
   <213> Homo sapiens
<400> 2013
   Pro Val Ile Lys Ala Arg Met Met Glu Tyr Gly Thr Thr Met Val Ser
   1               5                   10                  15
   Tyr Gln Pro Leu
               20
<210> 2014
   <211> 15
   <212> PRT
   <213> Homo sapiens
<400> 2014
   Gln His Leu Cys Gly Ser His Leu Val Glu Ala Leu Tyr Leu Val
   1 5 10 15
<210> 2015
   <211> 9
   <212> PRT
   <213> Homo sapiens
<400> 2015
   Arg Phe Lys Met Phe Pro Glu Val Lys
   1 5
<210> 2016
   <211> 10
   <212> PRT
   <213> Homo sapiens
<400> 2016
   Arg Phe Lys Met Phe Pro Glu Val Lys Glu
   1 5 10
<210> 2017
   <211> 11
   <212> PRT
   <213> Homo sapiens
<400> 2017
   Arg Phe Lys Met Phe Pro Glu Val Lys Glu Lys
   1 5 10
<210> 2018
   <211> 10
   <212> PRT
   <213> Homo sapiens
<400> 2018
   Ser His Leu Val Glu Ala Leu Tyr Leu Val
   1 5 10
<210> 2019
   <211> 14
   <212> PRT
   <213> Unknown
<220>
   <223> epitope involved in diabetes
<400> 2019
   Ser Asn Val Glu Cys Asp Gly Leu Asn Cys Asp Trp Ile Ile
   1 5 10
<210> 2020
   <211> 16
   <212> PRT
   <213> Homo sapiens
<400> 2020
   Ser Pro Ser Leu Trp Glu Ile Glu Phe Ala Lys Gln Leu Ala Ser Val
   1 5 10 15
<210> 2021
   <211> 16
   <212> PRT
   <213> Unknown
<220>
   <223> epitope involved in diabetes
<400> 2021
   Ser Pro Ser Leu Trp Glu Ile Glu Ile Ala Lys Gln Leu Ala Ser Val
   1 5 10 15
<210> 2022
   <211> 17
   <212> PRT
   <213> Homo sapiens
<400> 2022
   Thr Arg Arg Glu Ala Glu Asp Leu Gln Val Gly Gln Val Glu Leu Gly
   1 5 10 15
   Gly
<210> 2023
   <211> 9
   <212> PRT
   <213> Homo sapiens
<400> 2023
   Thr Ser Glu His Ser His Phe Ser Leu
   1 5
<210> 2024
   <211> 15
   <212> PRT
   <213> Homo sapiens
<400> 2024
   Val Glu Ala Leu Tyr Leu Val Cys Gly Glu Arg Gly Phe Phe Tyr
   1               5                   10                  15
<210> 2025
   <211> 9
   <212> PRT
   <213> Homo sapiens
<400> 2025
   Val Met Asn Ile Leu Leu Gln Tyr Val
   1               5
<210> 2026
   <211> 20
   <212> PRT
   <213> Homo sapiens
<400> 2026
   Tyr Glu Met Val Phe Asp Gly Lys Pro Gln His Thr Asn Val Cys Phe
   1               5                   10                  15
   Trp Tyr Ile Pro
               20
<210> 2027
   <211> 15
   <212> PRT
   <213> Homo sapiens
<400> 2027
   Tyr Leu Val Cys Gly Glu Arg Gly Phe Phe Tyr Thr Pro Lys Thr
   1               5                   10                  15
<210> 2028
   <211> 979
   <212> PRT
   <213> Homo sapiens
<400> 2028
   Met Arg Arg Pro Arg Arg Pro Gly Gly Leu Gly Gly Ser Gly Gly Leu
   1               5                   10                  15
   Arg Leu Leu Leu Cys Leu Leu Leu Leu Ser Ser Arg Pro Gly Gly Cys
               20                  25                  30
   Ser Ala Val Ser Ala His Gly Cys Leu Phe Asp Arg Arg Leu Cys Ser
           35                  40                  45
   His Leu Glu Val Cys Ile Gln Asp Gly Leu Phe Gly Gln Cys Gln Val
       50                  55                  60
   Gly Val Gly Gln Ala Arg Pro Leu Leu Gln Val Thr Ser Pro Val Leu
   65                  70                  75                  80
   Gln Arg Leu Gln Gly Val Leu Arg Gln Leu Met Ser Gln Gly Leu Ser
                   85                  90                  95
   Trp His Asp Asp Leu Thr Gln Tyr Val Ile Ser Gln Glu Met Glu Arg
               100                 105                 110
   Ile Pro Arg Leu Arg Pro Pro Glu Pro Arg Pro Arg Asp Arg Ser Gly
           115                 120                 125
   Leu Ala Pro Lys Arg Pro Gly Pro Ala Gly Glu Leu Leu Leu Gln Asp
       130                 135                 140
   Ile Pro Thr Gly Ser Ala Pro Ala Ala Gln His Arg Leu Pro Gln Pro
   145                 150                 155                 160
   Pro Val Gly Lys Gly Gly Ala Gly Ala Ser Ser Ser Leu Ser Pro Leu
                   165                 170                 175
   Gln Ala Glu Leu Leu Pro Pro Leu Leu Glu His Leu Leu Leu Pro Pro
               180                 185                 190
   Gln Pro Pro His Pro Ser Leu Ser Tyr Glu Pro Ala Leu Leu Gln Pro
           195                 200                 205
   Tyr Leu Phe His Gln Phe Gly Ser Arg Asp Gly Ser Arg Val Ser Glu
       210                 215                 220
   Gly Ser Pro Gly Met Val Ser Val Gly Pro Leu Pro Lys Ala Glu Ala
   225                 230                 235                 240
   Pro Ala Leu Phe Ser Arg Thr Ala Ser Lys Gly Ile Phe Gly Asp His
                   245                 250                 255
   Pro Gly His Ser Tyr Gly Asp Leu Pro Gly Pro Ser Pro Ala Gln Leu
               260                 265                 270
   Phe Gln Asp Ser Gly Leu Leu Tyr Leu Ala Gln Glu Leu Pro Ala Pro
           275                 280                 285
   Ser Arg Ala Arg Val Pro Arg Leu Pro Glu Gln Gly Ser Ser Ser Arg
   290 295 300
   Ala Glu Asp Ser Pro Glu Gly Tyr Glu Lys Glu Gly Leu Gly Asp Arg
   305 310 315 320
   Gly Glu Lys Pro Ala Ser Pro Ala Val Gln Pro Asp Ala Ala Leu Gln
   325 330 335
   Arg Leu Ala Ala Val Leu Ala Gly Tyr Gly Val Glu Leu Arg Gln Leu
   340 345 350
   Thr Pro Glu Gln Leu Ser Thr Leu Leu Thr Leu Leu Gln Leu Leu Pro
   355 360 365
   Lys Gly Ala Gly Arg Asn Pro Gly Gly Val Val Asn Val Gly Ala Asp
   370 375 380
   Ile Lys Lys Thr Met Glu Gly Pro Val Glu Gly Arg Asp Thr Ala Glu
   385 390 395 400
   Leu Pro Ala Arg Thr Ser Pro Met Pro Gly His Pro Thr Ala Ser Pro
   405 410 415
   Thr Ser Ser Glu Val Gln Gln Val Pro Ser Pro Val Ser Ser Glu Pro
   420 425 430
   Pro Lys Ala Ala Arg Pro Pro Val Thr Pro Val Leu Leu Glu Lys Lys
   435 440 445
   Ser Pro Leu Gly Gln Ser Gln Pro Thr Val Ala Gly Gln Pro Ser Ala
   450 455 460
   Arg Pro Ala Ala Glu Glu Tyr Gly Tyr Ile Val Thr Asp Gln Lys Pro
   465 470 475 480
   Leu Ser Leu Ala Ala Gly Val Lys Leu Leu Glu Ile Leu Ala Glu His
   485 490 495
   Val His Met Ser Ser Gly Ser Phe Ile Asn Ile Ser Val Val Gly Pro
   500 505 510
   Ala Leu Thr Phe Arg Ile Arg His Asn Glu Gln Asn Leu Ser Leu Ala
   515 520 525
   Asp Val Thr Gln Gln Ala Gly Leu Val Lys Ser Glu Leu Glu Ala Gln
   530 535 540
   Thr Gly Leu Gln Ile Leu Gln Thr Gly Val Gly Gln Arg Glu Glu Ala
   545 550 555 560
   Ala Ala Val Leu Pro Gln Thr Ala His Ser Thr Ser Pro Met Arg Ser
   565 570 575
   Val Leu Leu Thr Leu Val Ala Leu Ala Gly Val Ala Gly Leu Leu Val
   580 585 590
   Ala Leu Ala Val Ala Leu Cys Val Arg Gln His Ala Arg Gln Gln Asp
   595 600 605
   Lys Glu Arg Leu Ala Ala Leu Gly Pro Glu Gly Ala His Gly Asp Thr
   610 615 620
   Thr Phe Glu Tyr Gln Asp Leu Cys Arg Gln His Met Ala Thr Lys Ser
   625 630 635 640
   Leu Phe Asn Arg Ala Glu Gly Pro Pro Glu Pro Ser Arg Val Ser Ser
   645 650 655
   Val Ser Ser Gln Phe Ser Asp Ala Ala Gln Ala Ser Pro Ser Ser His
   660 665 670
   Ser Ser Thr Pro Ser Trp Cys Glu Glu Pro Ala Gln Ala Asn Met Asp
   675 680 685
   Ile Ser Thr Gly His Met Ile Leu Ala Tyr Met Glu Asp His Leu Arg
   690 695 700
   Asn Arg Asp Arg Leu Ala Lys Glu Trp Gln Ala Leu Cys Ala Tyr Gln
   705 710 715 720
   Ala Glu Pro Asn Thr Cys Ala Thr Ala Gln Gly Glu Gly Asn Ile Lys
   725 730 735
   Lys Asn Arg His Pro Asp Phe Leu Pro Tyr Asp His Ala Arg Ile Lys
   740 745 750
   Leu Lys Val Glu Ser Ser Pro Ser Arg Ser Asp Tyr Ile Asn Ala Ser
   755 760 765
   Pro Ile Ile Glu His Asp Pro Arg Met Pro Ala Tyr Ile Ala Thr Gln
   770 775 780
   Gly Pro Leu Ser His Thr Ile Ala Asp Phe Trp Gln Met Val Trp Glu
   785 790 795 800
   Ser Gly Cys Thr Val Ile Val Met Leu Thr Pro Leu Val Glu Asp Gly
   805 810 815
   Val Lys Gln Cys Asp Arg Tyr Trp Pro Asp Glu Gly Ala Ser Leu Tyr
   820 825 830
   His Val Tyr Glu Val Asn Leu Val Ser Glu His Ile Trp Cys Glu Asp
   835 840 845
   Phe Leu Val Arg Ser Phe Tyr Leu Lys Asn Val Gln Thr Gln Glu Thr
   850 855 860
   Arg Thr Leu Thr Gln Phe His Phe Leu Ser Trp Pro Ala Glu Gly Thr
   865 870 875 880
   Pro Ala Ser Thr Arg Pro Leu Leu Asp Phe Arg Arg Lys Val Asn Lys
   885 890 895
   Cys Tyr Arg Gly Arg Ser Cys Pro Ile Ile Val His Cys Ser Asp Gly
   900 905 910
   Ala Gly Arg Thr Gly Thr Tyr Ile Leu Ile Asp Met Val Leu Asn Arg
   915 920 925
   Met Ala Lys Gly Val Lys Glu Ile Asp Ile Ala Ala Thr Leu Glu His
   930 935 940
   Val Arg Asp Gln Arg Pro Gly Leu Val Arg Ser Lys Asp Gln Phe Glu
   945 950 955 960
   Phe Ala Leu Thr Ala Val Ala Glu Glu Val Asn Ala Ile Leu Lys Ala
   965 970 975
   Leu Pro Gln
<210> 2029
   <211> 20
   <212> PRT
   <213> Homo sapiens
<400> 2029
   Ala Ala Leu Gly Ile Gly Thr Asp Ser Val Ile Leu Ile Lys Cys Asp
   1 5 10 15
   Glu Arg Gly Lys
   20
<210> 2030
   <211> 12
   <212> PRT
   <213> Homo sapiens
<400> 2030
   Ala Phe Ile Lys Ala Thr Gly Lys Lys Glu Asp Glu
   1               5                   10
<210> 2031
   <211> 11
   <212> PRT
   <213> Mus musculus
<400> 2031
   Ala Pro Val Ile Lys Ala Arg Met Met Glu Tyr
   1               5                   10
<210> 2032
   <211> 13
   <212> PRT
   <213> Homo sapiens
<400> 2032
   Cys Ala Tyr Gln Ala Glu Pro Asn Thr Cys Ala Thr Ala
   1               5                   10
<210> 2033
   <211> 13
   <212> PRT
   <213> Homo sapiens
<400> 2033
   Cys Thr Val Ile Val Met Leu Thr Pro Leu Val Glu Asp
   1               5                   10
<210> 2034
   <211> 13
   <212> PRT
   <213> Homo sapiens
<400> 2034
   Asp Gln Phe Glu Phe Ala Leu Thr Ala Val Ala Glu Glu
   1               5                   10
<210> 2035
   <211> 15
   <212> PRT
   <213> Unknown
<220>
   <223> epitope involved in diabetes
<400> 2035
   Glu Lys Ala His Arg Pro Ile Trp Ala Arg Met Asp Ala Lys Lys
   1               5                   10                  15
<210> 2036
   <211> 20
   <212> PRT
   <213> Homo sapiens
<400> 2036
   Glu Tyr Val Thr Leu Lys Lys Met Arg Glu Ile Ile Gly Trp Pro Gly
   1               5                   10                  15
   Gly Ser Gly Asp
               20
<210> 2037
   <211> 15
   <212> PRT
   <213> Bos taurus
<400> 2037
   Phe Lys Ala Asp Glu Lys Lys Phe Trp Gly Lys Tyr Leu Tyr Glu
   1               5                   10                  15
<210> 2038
   <211> 19
   <212> PRT
   <213> Homo sapiens
<400> 2038
   Phe Tyr Leu Lys Asn Val Gln Thr Gln Glu Thr Arg Thr Leu Thr Gln
   1               5                   10                  15
   Phe His Phe
<210> 2039
   <211> 20
   <212> PRT
   <213> Homo sapiens
<400> 2039
   Gly Met Ala Ala Leu Pro Arg Leu Ile Ala Phe Thr Ser Glu His Ser
   1               5                   10                  15
   His Phe Ser Leu
               20
<210> 2040
   <211> 13
   <212> PRT
   <213> Homo sapiens
<400> 2040
   Gly Ser Phe Ile Asn Ile Ser Val Val Gly Pro Ala Leu
   1               5                   10
<210> 2041
   <211> 18
   <212> PRT
   <213> Homo sapiens
<400> 2041
   Gly Ser Leu Gln Pro Leu Ala Leu Glu Gly Ser Leu Gln Lys Arg Gly
   1               5                   10                  15
   Ile Val
<210> 2042
   <211> 20
   <212> PRT
   <213> Mus musculus
<400> 2042
   His Thr Pro Gly Val His Met Ala Ser Leu Ser Val Tyr Leu Lys Thr
   1               5                   10                  15
   Asn Val Phe Leu
               20
<210> 2043
   <211> 15
   <212> PRT
   <213> Homo sapiens
<400> 2043
   Ile Lys Ala Arg Met Met Glu Tyr Gly Thr Thr Met Val Ser Tyr
   1               5                   10                  15
<210> 2044
   <211> 20
   <212> PRT
   <213> Homo sapiens
<400> 2044
   Ile Lys Leu Lys Val Glu Ser Ser Pro Ser Arg Ser Asp Tyr Ile Asn
   1               5                   10                  15
   Ala Ser Pro Ile
               20
<210> 2045
   <211> 9
   <212> PRT
   <213> Unknown
<220>
   <223> epitope involved in diabetes
<400> 2045
   Lys Tyr Asn Lys Ala Asn Val Phe Leu
   1               5
<210> 2046
   <211> 23
   <212> PRT
   <213> Homo sapiens
<400> 2046
   Leu Ala Lys Glu Trp Gln Ala Leu Cys Ala Tyr Gln Ala Glu Pro Asn
   1               5                   10                  15
   Thr Cys Ala Thr Ala Gln Gly
               20
<210> 2047
   <211> 15
   <212> PRT
   <213> Homo sapiens
<400> 2047
   Leu Ala Lys Leu Ser Asp Gly Val Ala Val Leu Lys Val Gly Gly
   1               5                   10                  15
<210> 2048
   <211> 13
   <212> PRT
   <213> Homo sapiens
<400> 2048
   Leu Glu Ile Leu Ala Glu His Val His Met Ser Ser Gly
   1               5                   10
<210> 2049
   <211> 19
   <212> PRT
   <213> Mus musculus
<400> 2049
   Leu His Arg Ser Gly Val Leu Ile Ile His His Leu Gln Glu Asp Tyr
   1 5 10 15
   Arg Thr Tyr
<210> 2050
   <211> 20
   <212> PRT
   <213> Homo sapiens
<400> 2050
   Leu Tyr His Val Tyr Glu Val Asn Leu Val Ser Glu His Ile Trp Cys
   1 5 10 15
   Glu Asp Phe Leu
   20
<210> 2051
   <211> 17
   <212> PRT
   <213> Homo sapiens
<400> 2051
   Met Ala Ser Pro Gly Ser Gly Phe Trp Ser Phe Gly Ser Glu Asp Gly
   1 5 10 15
   Ser
<210> 2052
   <211> 37
   <212> PRT
   <213> Homo sapiens
<400> 2052
   Met Gly Ile Leu Lys Leu Gln Val Phe Leu Ile Val Leu Ser Val Ala
   1 5 10 15
   Leu Asn His Leu Lys Ala Thr Pro Ile Glu Ser His Gln Val Glu Lys
   20 25 30
   Arg Lys Cys Asn Thr
   35
<210> 2053
   <211> 15
   <212> PRT
   <213> Homo sapiens
<400> 2053
   Met Val Phe Asp Gly Lys Pro Gln His Thr Asn Val Cys Phe Trp
   1 5 10 15
<210> 2054
   <211> 21
   <212> PRT
   <213> Homo sapiens
<400> 2054
   Met Val Trp Glu Ser Gly Cys Thr Val Ile Val Met Leu Thr Pro Leu
   1 5 10 15
   Val Glu Asp Gly Val
   20
<210> 2055
   <211> 9
   <212> PRT
   <213> Unknown
<220>
   <223> epitope involved in diabetes
<400> 2055
   Asn Arg Val Ala Pro Asn Val Arg Gln
   1 5
<210> 2056
   <211> 13
   <212> PRT
   <213> Homo sapiens
<400> 2056
   Pro Ala Tyr Ile Ala Thr Gln Gly Pro Leu Ser His Thr
   1 5 10
<210> 2057
   <211> 15
   <212> PRT
   <213> Mus musculus
<400> 2057
   Pro His Leu Val Glu Ala Leu Tyr Leu Val Cys Gly Glu Arg Gly
   1 5 10 15
<210> 2058
   <211> 13
   <212> PRT
   <213> Homo sapiens
<400> 2058
   Pro Ser Leu Ser Tyr Glu Pro Ala Leu Leu Gln Pro Tyr
   1 5 10
<210> 2059
   <211> 15
   <212> PRT
   <213> Mus musculus
<400> 2059
   Pro Val Ile Lys Ala Arg Met Met Glu Tyr Gly Thr Thr Met Val
   1 5 10 15
<210> 2060
   <211> 13
   <212> PRT
   <213> Homo sapiens
<400> 2060
   Arg Ser Val Leu Leu Thr Leu Val Ala Leu Ala Gly Val
   1 5 10
<210> 2061
   <211> 20
   <212> PRT
   <213> Homo sapiens
<400> 2061
   Ser Glu His Ile Trp Cys Glu Asp Phe Leu Val Arg Ser Phe Tyr Leu
   1 5 10 15
   Lys Asn Val Gln
   20
<210> 2062
   <211> 15
   <212> PRT
   <213> Mus musculus
<400> 2062
   Ser His Leu Val Glu Ala Leu Tyr Leu Val Ala Gly Glu Arg Gly
   1 5 10 15
<210> 2063
   <211> 21
   <212> PRT
   <213> Homo sapiens
<400> 2063
   Ser Lys Asp Gln Phe Glu Phe Ala Leu Thr Ala Val Ala Glu Glu Val
   1 5 10 15
   Asn Ala Ile Leu Lys
   20
<210> 2064
   <211> 13
   <212> PRT
   <213> Homo sapiens
<400> 2064
   Ser Leu Tyr His Val Tyr Glu Val Asn Leu Val Ser Glu
   1 5 10
<210> 2065
   <211> 10
   <212> PRT
   <213> Mus musculus
<400> 2065
   Ser Leu Tyr Gln Leu Glu Asn Tyr Cys Asn
   1 5 10
<210> 2066
   <211> 18
   <212> PRT
   <213> Mus musculus
<400> 2066
   Thr Ala Ala Leu Ser Tyr Thr Ile Ser Arg Met Glu Glu Ser Ser Val
   1 5 10 15
   Thr Leu
<210> 2067
   <211> 15
   <212> PRT
   <213> Homo sapiens
<400> 2067
   Thr Gly His Pro Arg Tyr Phe Asn Gln Leu Ser Thr Gly Leu Asp
   1 5 10 15
<210> 2068
   <211> 13
   <212> PRT
   <213> Mus musculus
<400> 2068
   Thr Gln Tyr Glu Ala Val Ala Thr Ser Asn Met Gln Glu
   1 5 10
<210> 2069
   <211> 13
   <212> PRT
   <213> Homo sapiens
<400> 2069
   Thr Tyr Ile Leu Ile Asp Met Val Leu Asn Arg Met Ala
   1 5 10
<210> 2070
   <211> 16
   <212> PRT
   <213> Mus musculus
<400> 2070
   Val Pro Pro Ser Leu Arg Thr Leu Glu Asp Asn Glu Glu Arg Met Ser
   1 5 10 15
<210> 2071
   <211> 16
   <212> PRT
   <213> Mus musculus
<400> 2071
   Val Pro Thr Ser Leu Arg Arg Leu Glu Gln Pro Asn Val Ala Ile Ser
   1 5 10 15
<210> 2072
   <211> 18
   <212> PRT
   <213> Homo sapiens
<400> 2072
   Val Ser Ser Gln Phe Ser Asp Ala Ala Gln Ala Ser Pro Ser Ser His
   1 5 10 15
   Ser Ser
<210> 2073
   <211> 23
   <212> PRT
   <213> Mus musculus
<400> 2073
   Trp Tyr Val Met Val Thr Ala Ala Leu Ser Tyr Thr Ile Ser Arg Met
   1               5                   10                  15
   Glu Glu Ser Ser Val Thr Leu
               20
<210> 2074
   <211> 979
   <212> PRT
   <213> Homo sapiens
<400> 2074
   Met Arg Arg Pro Arg Arg Pro Gly Gly Leu Gly Gly Ser Gly Gly Leu
   1               5                   10                  15
   Arg Leu Leu Leu Cys Leu Leu Leu Leu Ser Ser Arg Pro Gly Gly Cys
               20                  25                  30
   Ser Ala Val Ser Ala His Gly Cys Leu Phe Asp Arg Arg Leu Cys Ser
           35                  40                  45
   His Leu Glu Val Cys Ile Gln Asp Gly Leu Phe Gly Gln Cys Gln Val
       50                  55                  60
   Gly Val Gly Gln Ala Arg Pro Leu Leu Gln Val Thr Ser Pro Val Leu
   65                  70                  75                  80
   Gln Arg Leu Gln Gly Val Leu Arg Gln Leu Met Ser Gln Gly Leu Ser
                   85                  90                  95
   Trp His Asp Asp Leu Thr Gln Tyr Val Ile Ser Gln Glu Met Glu Arg
               100                 105                 110
   Ile Pro Arg Leu Arg Pro Pro Glu Pro Arg Pro Arg Asp Arg Ser Gly
           115                 120                 125
   Leu Ala Pro Lys Arg Pro Gly Pro Ala Gly Glu Leu Leu Leu Gln Asp
       130                 135                 140
   Ile Pro Thr Gly Ser Ala Pro Ala Ala Gln His Arg Leu Pro Gln Pro
   145                 150                 155                 160
   Pro Val Gly Lys Gly Gly Ala Gly Ala Ser Ser Ser Leu Ser Pro Leu
                   165                 170                 175
   Gln Ala Glu Leu Leu Pro Pro Leu Leu Glu His Leu Leu Leu Pro Pro
               180                 185                 190
   Gln Pro Pro His Pro Ser Leu Ser Tyr Glu Pro Ala Leu Leu Gln Pro
           195                 200                 205
   Tyr Leu Phe His Gln Phe Gly Ser Arg Asp Gly Ser Arg Val Ser Glu
       210                 215                 220
   Gly Ser Pro Gly Met Val Ser Val Gly Pro Leu Pro Lys Ala Glu Ala
   225 230 235 240
   Pro Ala Leu Phe Ser Arg Thr Ala Ser Lys Gly Ile Phe Gly Asp His
   245 250 255
   Pro Gly His Ser Tyr Gly Asp Leu Pro Gly Pro Ser Pro Ala Gln Leu
   260 265 270
   Phe Gln Asp Ser Gly Leu Leu Tyr Leu Ala Gln Glu Leu Pro Ala Pro
   275 280 285
   Ser Arg Ala Arg Val Pro Arg Leu Pro Glu Gln Gly Ser Ser Ser Arg
   290 295 300
   Ala Glu Asp Ser Pro Glu Gly Tyr Glu Lys Glu Gly Leu Gly Asp Arg
   305 310 315 320
   Gly Glu Lys Pro Ala Ser Pro Ala Val Gln Pro Asp Ala Ala Leu Gln
   325 330 335
   Arg Leu Ala Ala Val Leu Ala Gly Tyr Gly Val Glu Leu Arg Gln Leu
   340 345 350
   Thr Pro Glu Gln Leu Ser Thr Leu Leu Thr Leu Leu Gln Leu Leu Pro
   355 360 365
   Lys Gly Ala Gly Arg Asn Pro Gly Gly Val Val Asn Val Gly Ala Asp
   370 375 380
   Ile Lys Lys Thr Met Glu Gly Pro Val Glu Gly Arg Asp Thr Ala Glu
   385 390 395 400
   Leu Pro Ala Arg Thr Ser Pro Met Pro Gly His Pro Thr Ala Ser Pro
   405 410 415
   Thr Ser Ser Glu Val Gln Gln Val Pro Ser Pro Val Ser Ser Glu Pro
   420 425 430
   Pro Lys Ala Ala Arg Pro Pro Val Thr Pro Val Leu Leu Glu Lys Lys
   435 440 445
   Ser Pro Leu Gly Gln Ser Gln Pro Thr Val Ala Gly Gln Pro Ser Ala
   450 455 460
   Arg Pro Ala Ala Glu Glu Tyr Gly Tyr Ile Val Thr Asp Gln Lys Pro
   465 470 475 480
   Leu Ser Leu Ala Ala Gly Val Lys Leu Leu Glu Ile Leu Ala Glu His
                   485                 490                 495
   Val His Met Ser Ser Gly Ser Phe Ile Asn Ile Ser Val Val Gly Pro
               500                 505                 510
   Ala Leu Thr Phe Arg Ile Arg His Asn Glu Gln Asn Leu Ser Leu Ala
           515                 520                 525
   Asp Val Thr Gln Gln Ala Gly Leu Val Lys Ser Glu Leu Glu Ala Gln
       530                 535                 540
   Thr Gly Leu Gln Ile Leu Gln Thr Gly Val Gly Gln Arg Glu Glu Ala
   545                 550                 555                 560
   Ala Ala Val Leu Pro Gln Thr Ala His Ser Thr Ser Pro Met Arg Ser
                   565                 570                 575
   Val Leu Leu Thr Leu Val Ala Leu Ala Gly Val Ala Gly Leu Leu Val
               580                 585                 590
   Ala Leu Ala Val Ala Leu Cys Val Arg Gln His Ala Arg Gln Gln Asp
           595                 600                 605
   Lys Glu Arg Leu Ala Ala Leu Gly Pro Glu Gly Ala His Gly Asp Thr
       610                 615                 620
   Thr Phe Glu Tyr Gln Asp Leu Cys Arg Gln His Met Ala Thr Lys Ser
   625                 630                 635                 640
   Leu Phe Asn Arg Ala Glu Gly Pro Pro Glu Pro Ser Arg Val Ser Ser
                   645                 650                 655
   Val Ser Ser Gln Phe Ser Asp Ala Ala Gln Ala Ser Pro Ser Ser His
               660                 665                 670
   Ser Ser Thr Pro Ser Trp Cys Glu Glu Pro Ala Gln Ala Asn Met Asp
           675                 680                 685
   Ile Ser Thr Gly His Met Ile Leu Ala Tyr Met Glu Asp His Leu Arg
       690                 695                 700
   Asn Arg Asp Arg Leu Ala Lys Glu Trp Gln Ala Leu Cys Ala Tyr Gln
   705                 710                 715                 720
   Ala Glu Pro Asn Thr Cys Ala Thr Ala Gln Gly Glu Gly Asn Ile Lys
                   725                 730                 735
   Lys Asn Arg His Pro Asp Phe Leu Pro Tyr Asp His Ala Arg Ile Lys
   740 745 750
   Leu Lys Val Glu Ser Ser Pro Ser Arg Ser Asp Tyr Ile Asn Ala Ser
   755 760 765
   Pro Ile Ile Glu His Asp Pro Arg Met Pro Ala Tyr Ile Ala Thr Gln
   770 775 780
   Gly Pro Leu Ser His Thr Ile Ala Asp Phe Trp Gln Met Val Trp Glu
   785 790 795 800
   Ser Gly Cys Thr Val Ile Val Met Leu Thr Pro Leu Val Glu Asp Gly
   805 810 815
   Val Lys Gln Cys Asp Arg Tyr Trp Pro Asp Glu Gly Ala Ser Leu Tyr
   820 825 830
   His Val Tyr Glu Val Asn Leu Val Ser Glu His Ile Trp Cys Glu Asp
   835 840 845
   Phe Leu Val Arg Ser Phe Tyr Leu Lys Asn Val Gln Thr Gln Glu Thr
   850 855 860
   Arg Thr Leu Thr Gln Phe His Phe Leu Ser Trp Pro Ala Glu Gly Thr
   865 870 875 880
   Pro Ala Ser Thr Arg Pro Leu Leu Asp Phe Arg Arg Lys Val Asn Lys
   885 890 895
   Cys Tyr Arg Gly Arg Ser Cys Pro Ile Ile Val His Cys Ser Asp Gly
   900 905 910
   Ala Gly Arg Thr Gly Thr Tyr Ile Leu Ile Asp Met Val Leu Asn Arg
   915 920 925
   Met Ala Lys Gly Val Lys Glu Ile Asp Ile Ala Ala Thr Leu Glu His
   930 935 940
   Val Arg Asp Gln Arg Pro Gly Leu Val Arg Ser Lys Asp Gln Phe Glu
   945 950 955 960
   Phe Ala Leu Thr Ala Val Ala Glu Glu Val Asn Ala Ile Leu Lys Ala
   965 970 975
   Leu Pro Gln
<210> 2075
   <211> 11
   <212> PRT
   <213> Unknown
<220>
   <223> epitope involved in diabetes
<400> 2075
   Ala Val Arg Pro Leu Trp Val Arg Met Glu Ala
   1 5 10
<210> 2076
   <211> 10
   <212> PRT
   <213> Homo sapiens
<400> 2076
   Asp Thr Thr Phe Glu Tyr Gln Asp Leu Cys
   1 5 10
<210> 2077
   <211> 11
   <212> PRT
   <213> Mus musculus
<400> 2077
   Glu Ala Leu Tyr Leu Val Cys Gly Glu Arg Gly
   1 5 10
<210> 2078
   <211> 9
   <212> PRT
   <213> Homo sapiens
<400> 2078
   Glu Leu Ala Glu Tyr Leu Tyr Asn Ile
   1 5
<210> 2079
   <211> 10
   <212> PRT
   <213> Homo sapiens
<400> 2079
   Gly Ala His Gly Asp Thr Thr Phe Glu Tyr
   1 5 10
<210> 2080
   <211> 7
   <212> PRT
   <213> Unknown
<220>
   <223> epitope involved in diabetes
<400> 2080
   Gly Glu Arg Ala Lys Arg Ser
   1               5
<210> 2081
   <211> 12
   <212> PRT
   <213> Bos taurus
<400> 2081
   Gly Ile Val Glu Gln Cys Cys Ala Ser Val Cys Ser
   1               5                   10
<210> 2082
   <211> 15
   <212> PRT
   <213> Bos taurus
<400> 2082
   Gly Ile Val Glu Gln Cys Cys Ala Ser Val Cys Ser Leu Tyr Gln
   1               5                   10                  15
<210> 2083
   <211> 12
   <212> PRT
   <213> Homo sapiens
<400> 2083
   Gly Ile Val Glu Gln Cys Cys Thr Ser Ile Cys Ser
   1               5                   10
<210> 2084
   <211> 23
   <212> PRT
   <213> Mus musculus
<400> 2084
   Gly Arg His Ser Ala Glu Tyr Tyr Asn Lys Gln Tyr Leu Glu Arg Thr
   1               5                   10                  15
   Arg Ala Glu Leu Asp Thr Ala
               20
<210> 2085
   <211> 9
   <212> PRT
   <213> Homo sapiens
<400> 2085
   Ile Leu Met His Cys Gln Thr Thr Leu
   1 5
<210> 2086
   <211> 7
   <212> PRT
   <213> Unknown
<220>
   <223> epitope involved in diabetes
<400> 2086
   Leu Asp Gln Arg Ser Ser Lys
   1 5
<210> 2087
   <211> 7
   <212> PRT
   <213> Unknown
<220>
   <223> epitope involved in diabetes
<400> 2087
   Leu Asp Gln Ser Arg Leu Lys
   1 5
<210> 2088
   <211> 19
   <212> PRT
   <213> Mus musculus
<400> 2088
   Leu Lys Ile Cys Gly Phe Leu Gln Arg Thr Asn Ser Leu Glu Glu Lys
   1 5 10 15
   Ser Arg Leu
<210> 2089
   <211> 7
   <212> PRT
   <213> Unknown
<220>
   <223> epitope involved in diabetes
<400> 2089
   Leu Pro Gly Glu Arg Gln Ile
   1 5
<210> 2090
   <211> 7
   <212> PRT
   <213> Unknown
<220>
   <223> epitope involved in diabetes
<400> 2090
   Leu Pro Asn Arg Ser Gln Ile
   1 5
<210> 2091
   <211> 7
   <212> PRT
   <213> Unknown
<220>
   <223> epitope involved in diabetes
<400> 2091
   Asn Ile Lys Ser Ser Leu Pro
   1 5
<210> 2092
   <211> 7
   <212> PRT
   <213> Unknown
<220>
   <223> epitope involved in diabetes
<400> 2092
   Asn Pro Gly Ser Ser Leu Gly
   1 5
<210> 2093
   <211> 17
   <212> PRT
   <213> Mus musculus
<400> 2093
   Asn Ser Gln Pro Glu Ile Leu Glu Arg Thr Arg Ala Glu Val Asp Thr
   1 5 10 15
   Ala
<210> 2094
   <211> 7
   <212> PRT
   <213> Unknown
<220>
   <223> epitope involved in diabetes
<400> 2094
   Pro Asp Gly Ser Arg Leu Val
   1 5
<210> 2095
   <211> 7
   <212> PRT
   <213> Unknown
<220>
   <223> epitope involved in diabetes
<400> 2095
   Pro Gly Ser Arg Leu Gln Pro
   1 5
<210> 2096
   <211> 7
   <212> PRT
   <213> Unknown
<220>
   <223> epitope involved in diabetes
<400> 2096
   Pro Gly Thr Arg Leu Gln Pro
   1 5
<210> 2097
   <211> 7
   <212> PRT
   <213> Unknown
<220>
   <223> epitope involved in diabetes
<400> 2097
   Pro Ser Arg Leu Val Asn Gln
   1 5
<210> 2098
   <211> 12
   <212> PRT
   <213> Bos taurus
<400> 2098
   Gln Cys Cys Ala Ser Val Cys Ser Leu Tyr Gln Leu
   1 5 10
<210> 2099
   <211> 12
   <212> PRT
   <213> Homo sapiens
<400> 2099
   Gln Cys Cys Thr Ser Ile Cys Ser Leu Tyr Gln Leu
   1 5 10
<210> 2100
   <211> 7
   <212> PRT
   <213> Unknown
<220>
   <223> epitope involved in diabetes
<400> 2100
   Gln Gly Glu Arg Ser Gly Pro
   1 5
<210> 2101
   <211> 7
   <212> PRT
   <213> Unknown
<220>
   <223> epitope involved in diabetes
<400> 2101
   Gln Arg Ser Gly Arg Gly Pro
   1 5
<210> 2102
   <211> 15
   <212> PRT
   <213> Unknown
<220>
   <223> epitope involved in diabetes
<400> 2102
   Ser His Leu Val Glu Ala Leu Ala Leu Val Ala Gly Glu Arg Gly
   1 5 10 15
<210> 2103
   <211> 8
   <212> PRT
   <213> Mus musculus
<400> 2103
   Ser His Leu Val Glu Ala Leu Tyr
   1 5
<210> 2104
   <211> 7
   <212> PRT
   <213> Unknown
<220>
   <223> epitope involved in diabetes
<400> 2104
   Ser Thr Gly Glu Arg Ile Gln
   1 5
<210> 2105
   <211> 20
   <212> PRT
   <213> Homo sapiens
<400> 2105
   Thr Asn Met Phe Thr Tyr Glu Ile Ala Pro Val Phe Val Leu Leu Glu
   1 5 10 15
   Tyr Val Thr Leu
   20
<210> 2106
   <211> 13
   <212> PRT
   <213> Bos taurus
<400> 2106
   Val Cys Gly Glu Arg Gly Phe Phe Tyr Thr Pro Lys Ala
   1 5 10
<210> 2107
   <211> 7
   <212> PRT
   <213> Unknown
<220>
   <223> epitope involved in diabetes
<400> 2107
   Val Gly Glu Arg Gly Ser Lys
   1 5
<210> 2108
   <211> 7
   <212> PRT
   <213> Unknown
<220>
   <223> epitope involved in diabetes
<400> 2108
   Val Ile Gln Gly Ser Gln Leu
   1 5
<210> 2109
   <211> 7
   <212> PRT
   <213> Unknown
<220>
   <223> epitope involved in diabetes
<400> 2109
   Val Ile Gln Gly Ser Arg Leu
   1 5
<210> 2110
   <211> 24
   <212> PRT
   <213> Mus musculus
<400> 2110
   Val Leu Gly Gly Gly Cys Ala Leu Leu Arg Cys Ile Pro Ala Leu Asp
   1 5 10 15
   Ser Leu Lys Pro Ala Asn Glu Asp
   20
<210> 2111
   <211> 7
   <212> PRT
   <213> Unknown
<220>
   <223> epitope involved in diabetes
<400> 2111
   Val Pro Gln Gln Ser Ile Pro
   1 5
<210> 2112
   <211> 7
   <212> PRT
   <213> Unknown
<220>
   <223> epitope involved in diabetes
<400> 2112
   Val Arg Gly Arg Gly Ser Lys
   1 5
<210> 2113
   <211> 7
   <212> PRT
   <213> Unknown
<220>
   <223> epitope involved in diabetes
<400> 2113
   Val Ser Arg Ser Leu Leu Asp
   1               5
<210> 2114
   <211> 585
   <212> PRT
   <213> Homo sapiens
<400> 2114
   Met Ala Ser Pro Gly Ser Gly Phe Trp Ser Phe Gly Ser Glu Asp Gly
   1               5                   10                  15
   Ser Gly Asp Ser Glu Asn Pro Gly Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ala Glu Lys Pro Ala Glu Ser Gly Gly Ser Gln Pro Pro
       50                  55                  60
   Arg Ala Ala Ala Arg Lys Ala Ala Cys Ala Cys Asp Gln Lys Pro Cys
   65                  70                  75                  80
   Ser Cys Ser Lys Val Asp Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Asn Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
   180 185 190
   Ala Asp Trp Leu Thr Ser Thr Ala Asn Thr Asn Met Phe Thr Tyr Glu
   195 200 205
   Ile Ala Pro Val Phe Val Leu Leu Glu Tyr Val Thr Leu Lys Lys Met
   210 215 220
   Arg Glu Ile Ile Gly Trp Pro Gly Gly Ser Gly Asp Gly Ile Phe Ser
   225 230 235 240
   Pro Gly Gly Ala Ile Ser Asn Met Tyr Ala Met Met Ile Ala Arg Phe
   245 250 255
   Lys Met Phe Pro Glu Val Lys Glu Lys Gly Met Ala Ala Leu Pro Arg
   260 265 270
   Leu Ile Ala Phe Thr Ser Glu His Ser His Phe Ser Leu Lys Lys Gly
   275 280 285
   Ala Ala Ala Leu Gly Ile Gly Thr Asp Ser Val Ile Leu Ile Lys Cys
   290 295 300
   Asp Glu Arg Gly Lys Met Ile Pro Ser Asp Leu Glu Arg Arg Ile Leu
   305 310 315 320
   Glu Ala Lys Gln Lys Gly Phe Val Pro Phe Leu Val Ser Ala Thr Ala
   325 330 335
   Gly Thr Thr Val Tyr Gly Ala Phe Asp Pro Leu Leu Ala Val Ala Asp
   340 345 350
   Ile Cys Lys Lys Tyr Lys Ile Trp Met His Val Asp Ala Ala Trp Gly
   355 360 365
   Gly Gly Leu Leu Met Ser Arg Lys His Lys Trp Lys Leu Ser Gly Val
   370 375 380
   Glu Arg Ala Asn Ser Val Thr Trp Asn Pro His Lys Met Met Gly Val
   385 390 395 400
   Pro Leu Gln Cys Ser Ala Leu Leu Val Arg Glu Glu Gly Leu Met Gln
   405 410 415
   Asn Cys Asn Gln Met His Ala Ser Tyr Leu Phe Gln Gln Asp Lys His
   420 425 430
   Tyr Asp Leu Ser Tyr Asp Thr Gly Asp Lys Ala Leu Gln Cys Gly Arg
   435 440 445
   His Val Asp Val Phe Lys Leu Trp Leu Met Trp Arg Ala Lys Gly Thr
   450 455 460
   Thr Gly Phe Glu Ala His Val Asp Lys Cys Leu Glu Leu Ala Glu Tyr
   465 470 475 480
   Leu Tyr Asn Ile Ile Lys Asn Arg Glu Gly Tyr Glu Met Val Phe Asp
   485 490 495
   Gly Lys Pro Gln His Thr Asn Val Cys Phe Trp Tyr Ile Pro Pro Ser
   500 505 510
   Leu Arg Thr Leu Glu Asp Asn Glu Glu Arg Met Ser Arg Leu Ser Lys
   515 520 525
   Val Ala Pro Val Ile Lys Ala Arg Met Met Glu Tyr Gly Thr Thr Met
   530 535 540
   Val Ser Tyr Gln Pro Leu Gly Asp Lys Val Asn Phe Phe Arg Met Val
   545 550 555 560
   Ile Ser Asn Pro Ala Ala Thr His Gln Asp Ile Asp Phe Leu Ile Glu
   565 570 575
   Glu Ile Glu Arg Leu Gly Gln Asp Leu
   580 585
<210> 2115
   <211> 30
   <212> PRT
   <213> Homo sapiens
<400> 2115
   Ala Ala Ala Phe Val Asn Gln His Leu Cys Gly Ser His Leu Val Glu
   1 5 10 15
   Ala Leu Tyr Leu Val Cys Gly Glu Arg Gly Phe Phe Tyr Thr
   20 25 30
<210> 2116
   <211> 9
   <212> PRT
   <213> Mus musculus
<400> 2116
   Ala Leu Ile Pro Tyr Cys Val His Met
   1 5
<210> 2117
   <211> 9
   <212> PRT
   <213> Mus musculus
<400> 2117
   Ala Leu Ser Tyr Thr Ile Ser Arg Met
   1 5
<210> 2118
   <211> 9
   <212> PRT
   <213> Mus musculus
<400> 2118
   Ala Thr His Phe Pro His Gln Val Ile
   1 5
<210> 2119
   <211> 9
   <212> PRT
   <213> Mus musculus
<400> 2119
   Cys Ala Leu Thr Ser Leu Thr Thr Met
   1 5
<210> 2120
   <211> 9
   <212> PRT
   <213> Mus musculus
<400> 2120
   Cys Ala Asn Pro Asp Trp Ile His Ile
   1 5
<210> 2121
   <211> 21
   <212> PRT
   <213> Homo sapiens
<400> 2121
   Cys Gly Arg His Val Asp Val Phe Lys Leu Trp Leu Met Trp Arg Ala
   1 5 10 15
   Lys Gly Thr Thr Gly
   20
<210> 2122
   <211> 9
   <212> PRT
   <213> Mus musculus
<400> 2122
   Cys Lys Ser Ala Ser Ile Pro Leu Met
   1 5
<210> 2123
   <211> 9
   <212> PRT
   <213> Mus musculus
<400> 2123
   Cys Gln Gly Glu Asn Gly Thr Lys Pro
   1 5
<210> 2124
   <211> 20
   <212> PRT
   <213> Homo sapiens
<400> 2124
   Asp Glu Arg Gly Lys Met Ile Pro Ser Asp Leu Glu Arg Arg Ile Leu
   1 5 10 15
   Glu Ala Lys Gln
   20
<210> 2125
   <211> 9
   <212> PRT
   <213> Mus musculus
<400> 2125
   Asp Phe Leu His Arg Ser Gly Val Leu
   1 5
<210> 2126
   <211> 12
   <212> PRT
   <213> Unknown
<220>
   <223> epitope involved in diabetes
<400> 2126
   Asp Ser Tyr Glu Ile Met Gln Gly Arg Ala Val Val
   1 5 10
<210> 2127
   <211> 9
   <212> PRT
   <213> Mus musculus
<400> 2127
   Asp Tyr Arg Thr Tyr Tyr Gly Phe Leu
   1 5
<210> 2128
   <211> 20
   <212> PRT
   <213> Homo sapiens
<400> 2128
   Glu Glu Ile Leu Met His Cys Gln Thr Thr Leu Lys Tyr Ala Ile Lys
   1 5 10 15
   Thr Gly His Pro
   20
<210> 2129
   <211> 20
   <212> PRT
   <213> Homo sapiens
<400> 2129
   Glu Arg Ala Asn Ser Val Thr Trp Asn Pro His Lys Met Met Gly Val
   1 5 10 15
   Pro Leu Gln Cys
   20
<210> 2130
   <211> 9
   <212> PRT
   <213> Mus musculus
<400> 2130
   Glu Ser Ser Val Thr Leu His Arg Leu
   1 5
<210> 2131
   <211> 20
   <212> PRT
   <213> Homo sapiens
<400> 2131
   Glu Tyr Gly Thr Thr Met Val Ser Tyr Gln Pro Leu Gly Asp Lys Val
   1 5 10 15
   Asn Phe Phe Arg
   20
<210> 2132
   <211> 20
   <212> PRT
   <213> Homo sapiens
<400> 2132
   Glu Tyr Leu Tyr Asn Ile Ile Lys Asn Arg Glu Gly Tyr Glu Met Val
   1               5                   10                  15
   Phe Asp Gly Lys
               20
<210> 2133
   <211> 9
   <212> PRT
   <213> Mus musculus
<400> 2133
   Phe Ala Ile Asn Ser Glu Met Phe Leu
   1               5
<210> 2134
   <211> 9
   <212> PRT
   <213> Mus musculus
<400> 2134
   Phe Glu His Thr Pro Gly Val His Met
   1               5
<210> 2135
   <211> 9
   <212> PRT
   <213> Mus musculus
<400> 2135
   Phe Gly Ile Asp Leu Leu Trp Ser Val
   1               5
<210> 2136
   <211> 9
   <212> PRT
   <213> Mus musculus
<400> 2136
   Phe Leu His Arg Ser Gly Val Leu Ile
   1               5
<210> 2137
   <211> 9
   <212> PRT
   <213> Homo sapiens
<400> 2137
   Phe Leu Trp Ser Val Phe Trp Leu Ile
   1 5
<210> 2138
   <211> 9
   <212> PRT
   <213> Mus musculus
<400> 2138
   Gly Asp Pro Arg Asn Ile Phe Ser Ile
   1 5
<210> 2139
   <211> 9
   <212> PRT
   <213> Mus musculus
<400> 2139
   Gly Phe Ala Ile Asn Ser Glu Met Phe
   1 5
<210> 2140
   <211> 20
   <212> PRT
   <213> Homo sapiens
<400> 2140
   Gly Gly Ser Gly Asp Gly Ile Phe Ser Pro Gly Gly Ala Ile Ser Asn
   1 5 10 15
   Met Tyr Ala Met
   20
<210> 2141
   <211> 9
   <212> PRT
   <213> Mus musculus
<400> 2141
   Gly His Ala Met Gly Ser Ser Cys Val
   1 5
<210> 2142
   <211> 9
   <212> PRT
   <213> Mus musculus
<400> 2142
   Gly Leu Val Arg Asn Leu Gly Val Leu
   1 5
<210> 2143
   <211> 9
   <212> PRT
   <213> Mus musculus
<400> 2143
   Gly Thr Lys Pro Ser Phe Arg Leu Leu
   1 5
<210> 2144
   <211> 9
   <212> PRT
   <213> Mus musculus
<400> 2144
   His Ala Glu Pro Leu Phe Tyr Leu Leu
   1 5
<210> 2145
   <211> 9
   <212> PRT
   <213> Mus musculus
<400> 2145
   His Met Ala Ser Leu Ser Val Tyr Leu
   1 5
<210> 2146
   <211> 9
   <212> PRT
   <213> Mus musculus
<400> 2146
   Ile Tyr Phe Pro Leu Trp Phe Gln Leu
   1 5
<210> 2147
   <211> 9
   <212> PRT
   <213> Mus musculus
<400> 2147
   Ile Tyr Pro Asn His Ser Ser Pro Cys
   1 5
<210> 2148
   <211> 22
   <212> PRT
   <213> Homo sapiens
<400> 2148
   Lys Gly Thr Thr Gly Phe Glu Ala His Val Asp Lys Cys Leu Glu Leu
   1 5 10 15
   Ala Glu Tyr Leu Tyr Asn
   20
<210> 2149
   <211> 9
   <212> PRT
   <213> Mus musculus
<400> 2149
   Lys Ile Pro Thr His Ala Glu Pro Leu
   1 5
<210> 2150
   <211> 20
   <212> PRT
   <213> Homo sapiens
<400> 2150
   Lys Thr Gly His Pro Arg Tyr Phe Asn Gln Leu Ser Thr Gly Leu Asp
   1 5 10 15
   Met Val Gly Leu
   20
<210> 2151
   <211> 9
   <212> PRT
   <213> Mus musculus
<400> 2151
   Lys Trp Cys Ala Asn Pro Asp Trp Ile
   1 5
<210> 2152
   <211> 22
   <212> PRT
   <213> Homo sapiens
<400> 2152
   Leu Ala Phe Leu Gln Asp Val Met Asn Ile Leu Leu Gln Tyr Val Val
   1 5 10 15
   Lys Ser Phe Asp Arg Ser
   20
<210> 2153
   <211> 20
   <212> PRT
   <213> Homo sapiens
<400> 2153
   Leu Glu Ala Lys Gln Lys Gly Phe Val Pro Phe Leu Val Ser Ala Thr
   1               5                   10                  15
   Ala Gly Thr Thr
               20
<210> 2154
   <211> 9
   <212> PRT
   <213> Mus musculus
<400> 2154
   Leu Gly Phe Ala Ile Asn Ser Glu Met
   1               5
<210> 2155
   <211> 20
   <212> PRT
   <213> Homo sapiens
<400> 2155
   Leu Leu Tyr Gly Asp Ala Glu Lys Pro Ala Glu Ser Gly Gly Ser Gln
   1               5                   10                  15
   Pro Pro Arg Ala
               20
<210> 2156
   <211> 9
   <212> PRT
   <213> Mus musculus
<400> 2156
   Leu Arg Leu Phe Gly Ile Asp Leu Leu
   1               5
<210> 2157
   <211> 9
   <212> PRT
   <213> Mus musculus
<400> 2157
   Leu Ser Phe Cys Lys Ser Ala Ser Ile
   1 5
<210> 2158
   <211> 9
   <212> PRT
   <213> Mus musculus
<400> 2158
   Leu Ser Val Tyr Leu Lys Thr Asn Val
   1 5
<210> 2159
   <211> 9
   <212> PRT
   <213> Mus musculus
<400> 2159
   Leu Thr Ser Leu Thr Thr Met Gln Leu
   1 5
<210> 2160
   <211> 9
   <212> PRT
   <213> Mus musculus
<400> 2160
   Met Gln Leu Tyr Arg Phe Ile Lys Ile
   1 5
<210> 2161
   <211> 11
   <212> PRT
   <213> Unknown
<220>
   <223> epitope involved in diabetes
<400> 2161
   Met Tyr Glu Leu Leu Gln Pro Ala Ala Ile Val
   1 5 10
<210> 2162
   <211> 9
   <212> PRT
   <213> Homo sapiens
<400> 2162
   Asn Leu Phe Leu Phe Leu Phe Ala Val
   1 5
<210> 2163
   <211> 9
   <212> PRT
   <213> Mus musculus
<400> 2163
   Asn Gln Asn Val Gly Thr Lys Met Ile
   1 5
<210> 2164
   <211> 20
   <212> PRT
   <213> Homo sapiens
<400> 2164
   Pro Glu Val Lys Glu Lys Gly Met Ala Ala Leu Pro Arg Leu Ile Ala
   1 5 10 15
   Phe Thr Ser Glu
   20
<210> 2165
   <211> 14
   <212> PRT
   <213> Mus musculus
<400> 2165
   Pro Ser Leu Trp Glu Ile Glu Phe Ala Lys Gln Leu Ala Ser
   1 5 10
<210> 2166
   <211> 9
   <212> PRT
   <213> Mus musculus
<400> 2166
   Gln Leu Asn Gln Asn Val Gly Thr Lys
   1 5
<210> 2167
   <211> 20
   <212> PRT
   <213> Homo sapiens
<400> 2167
   Gln Asn Cys Asn Gln Met His Ala Ser Tyr Leu Phe Gln Gln Asp Lys
   1 5 10 15
   His Tyr Asp Leu
   20
<210> 2168
   <211> 9
   <212> PRT
   <213> Mus musculus
<400> 2168
   Arg Phe Ile Lys Ile Pro Thr His Ala
   1 5
<210> 2169
   <211> 9
   <212> PRT
   <213> Mus musculus
<400> 2169
   Arg Met Glu Glu Ser Ser Val Thr Leu
   1 5
<210> 2170
   <211> 9
   <212> PRT
   <213> Mus musculus
<400> 2170
   Ser Phe Cys Lys Ser Ala Ser Ile Pro
   1 5
<210> 2171
   <211> 9
   <212> PRT
   <213> Mus musculus
<400> 2171
   Ser Phe Leu Trp Ser Val Phe Trp Leu
   1 5
<210> 2172
   <211> 9
   <212> PRT
   <213> Mus musculus
<400> 2172
   Ser Arg Met Glu Glu Ser Ser Val Thr
   1 5
<210> 2173
   <211> 9
   <212> PRT
   <213> Mus musculus
<400> 2173
   Ser Arg Val Phe Ile Ala Thr His Phe
   1 5
<210> 2174
   <211> 9
   <212> PRT
   <213> Mus musculus
<400> 2174
   Thr His Ala Glu Pro Leu Phe Tyr Leu
   1               5
<210> 2175
   <211> 9
   <212> PRT
   <213> Mus musculus
<400> 2175
   Thr His Phe Pro His Gln Val Ile Leu
   1               5
<210> 2176
   <211> 9
   <212> PRT
   <213> Mus musculus
<400> 2176
   Thr Ser Leu Thr Thr Met Gln Leu Tyr
   1               5
<210> 2177
   <211> 9
   <212> PRT
   <213> Mus musculus
<400> 2177
   Thr Tyr Tyr Gly Phe Leu Asn Phe Met
   1               5
<210> 2178
   <211> 30
   <212> PRT
   <213> Homo sapiens
<400> 2178
   Val Cys Gly Glu Arg Gly Phe Phe Tyr Thr Pro Lys Thr Arg Arg Glu
   1               5                   10                  15
   Ala Glu Asp Leu Gln Val Gly Gln Val Glu Leu Gly Gly Gly
               20                  25                  30
<210> 2179
   <211> 9
   <212> PRT
   <213> Mus musculus
<400> 2179
   Val Phe Trp Leu Ile Gln Ile Ser Val
   1 5
<210> 2180
   <211> 9
   <212> PRT
   <213> Mus musculus
<400> 2180
   Tyr Leu Lys Thr Asn Val Phe Leu Phe
   1 5
<210> 2181
   <211> 9
   <212> PRT
   <213> Homo sapiens
<400> 2181
   Tyr Leu Leu Leu Arg Val Leu Asn Ile
   1 5
<210> 2182
   <211> 9
   <212> PRT
   <213> Mus musculus
<400> 2182
   Tyr Pro Asn His Ser Ser Pro Cys Leu
   1 5
<210> 2183
   <211> 9
   <212> PRT
   <213> Mus musculus
<400> 2183
   Tyr Thr Ile Ser Arg Met Glu Glu Ser
   1 5
<210> 2184
   <211> 10
   <212> PRT
   <213> Unknown
<220>
   <223> epitope involved in diabetes
<400> 2184
   Tyr Val Arg Pro Leu Trp Val Arg Met Glu
   1 5 10
<210> 2185
   <211> 10
   <212> PRT
   <213> Homo sapiens
<400> 2185
   Ala Leu Trp Met Arg Leu Leu Pro Leu Leu
   1 5 10
<210> 2186
   <211> 13
   <212> PRT
   <213> Homo sapiens
<400> 2186
   Asp Trp Ile His Ile Asp Thr Thr Pro Phe Ala Gly Leu
   1 5 10
<210> 2187
   <211> 13
   <212> PRT
   <213> Homo sapiens
<400> 2187
   Glu Trp Val His Ile Asp Thr Thr Pro Phe Ala Ser Leu
   1 5 10
<210> 2188
   <211> 9
   <212> PRT
   <213> Homo sapiens
<400> 2188
   Phe Tyr Thr Pro Lys Thr Arg Arg Glu
   1 5
<210> 2189
   <211> 8
   <212> PRT
   <213> Homo sapiens
<400> 2189
   Gly Glu Arg Gly Phe Phe Tyr Thr
   1 5
<210> 2190
   <211> 9
   <212> PRT
   <213> Homo sapiens
<400> 2190
   Leu Pro Leu Leu Ala Leu Leu Ala Leu
   1 5
<210> 2191
   <211> 9
   <212> PRT
   <213> Homo sapiens
<400> 2191
   Leu Trp Met Arg Leu Leu Pro Leu Leu
   1 5
<210> 2192
   <211> 5
   <212> PRT
   <213> Homo sapiens
<400> 2192
   Pro Ala Glu Gly Thr
   1 5
<210> 2193
   <211> 13
   <212> PRT
   <213> Homo sapiens
<400> 2193
   Gln His Leu Gln Lys Asp Tyr Arg Ala Tyr Tyr Thr Phe
   1 5 10
<210> 2194
   <211> 13
   <212> PRT
   <213> Homo sapiens
<400> 2194
   Arg Val Leu Asn Ile Asp Leu Leu Trp Ser Val Pro Ile
   1 5 10
<210> 2195
   <211> 31
   <212> PRT
   <213> Homo sapiens
<400> 2195
   Trp Cys Glu Asp Phe Leu Val Arg Ser Phe Tyr Leu Lys Asn Val Gln
   1 5 10 15
   Thr Gln Glu Thr Arg Thr Leu Thr Gln Phe His Phe Leu Ser Trp
   20 25 30
<210> 2196
   <211> 10
   <212> PRT
   <213> Homo sapiens
<400> 2196
   Trp Met Arg Leu Leu Pro Leu Leu Ala Leu
   1               5                   10
<210> 2197
   <211> 13
   <212> PRT
   <213> Homo sapiens
<400> 2197
   Tyr Thr Phe Leu Asn Phe Met Ser Asn Val Gly Asp Pro
   1               5                   10
<210> 2198
   <211> 979
   <212> PRT
   <213> Homo sapiens
<400> 2198
   Met Arg Arg Pro Arg Arg Pro Gly Gly Leu Gly Gly Ser Gly Gly Leu
   1               5                   10                  15
   Arg Leu Leu Leu Cys Leu Leu Leu Leu Ser Ser Arg Pro Gly Gly Cys
               20                  25                  30
   Ser Ala Val Ser Ala His Gly Cys Leu Phe Asp Arg Arg Leu Cys Ser
           35                  40                  45
   His Leu Glu Val Cys Ile Gln Asp Gly Leu Phe Gly Gln Cys Gln Val
       50                  55                  60
   Gly Val Gly Gln Ala Arg Pro Leu Leu Gln Val Thr Ser Pro Val Leu
   65                  70                  75                  80
   Gln Arg Leu Gln Gly Val Leu Arg Gln Leu Met Ser Gln Gly Leu Ser
                   85                  90                  95
   Trp His Asp Asp Leu Thr Gln Tyr Val Ile Ser Gln Glu Met Glu Arg
               100                 105                 110
   Ile Pro Arg Leu Arg Pro Pro Glu Pro Arg Pro Arg Asp Arg Ser Gly
           115                 120                 125
   Leu Ala Pro Lys Arg Pro Gly Pro Ala Gly Glu Leu Leu Leu Gln Asp
       130                 135                 140
   Ile Pro Thr Gly Ser Ala Pro Ala Ala Gln His Arg Leu Pro Gln Pro
   145                 150                 155                 160
   Pro Val Gly Lys Gly Gly Ala Gly Ala Ser Ser Ser Leu Ser Pro Leu
                   165                 170                 175
   Gln Ala Glu Leu Leu Pro Pro Leu Leu Glu His Leu Leu Leu Pro Pro
               180                 185                 190
   Gln Pro Pro His Pro Ser Leu Ser Tyr Glu Pro Ala Leu Leu Gln Pro
           195                 200                 205
   Tyr Leu Phe His Gln Phe Gly Ser Arg Asp Gly Ser Arg Val Ser Glu
       210                 215                 220
   Gly Ser Pro Gly Met Val Ser Val Gly Pro Leu Pro Lys Ala Glu Ala
   225                 230                 235                 240
   Pro Ala Leu Phe Ser Arg Thr Ala Ser Lys Gly Ile Phe Gly Asp His
                   245                 250                 255
   Pro Gly His Ser Tyr Gly Asp Leu Pro Gly Pro Ser Pro Ala Gln Leu
               260                 265                 270
   Phe Gln Asp Ser Gly Leu Leu Tyr Leu Ala Gln Glu Leu Pro Ala Pro
           275                 280                 285
   Ser Arg Ala Arg Val Pro Arg Leu Pro Glu Gln Gly Ser Ser Ser Arg
       290                 295                 300
   Ala Glu Asp Ser Pro Glu Gly Tyr Glu Lys Glu Gly Leu Gly Asp Arg
   305                 310                 315                 320
   Gly Glu Lys Pro Ala Ser Pro Ala Val Gln Pro Asp Ala Ala Leu Gln
                   325                 330                 335
   Arg Leu Ala Ala Val Leu Ala Gly Tyr Gly Val Glu Leu Arg Gln Leu
               340                 345                 350
   Thr Pro Glu Gln Leu Ser Thr Leu Leu Thr Leu Leu Gln Leu Leu Pro
           355                 360                 365
   Lys Gly Ala Gly Arg Asn Pro Gly Gly Val Val Asn Val Gly Ala Asp
       370                 375                 380
   Ile Lys Lys Thr Met Glu Gly Pro Val Glu Gly Arg Asp Thr Ala Glu
   385                 390                 395                 400
   Leu Pro Ala Arg Thr Ser Pro Met Pro Gly His Pro Thr Ala Ser Pro
                   405                 410                 415
   Thr Ser Ser Glu Val Gln Gln Val Pro Ser Pro Val Ser Ser Glu Pro
               420                 425                 430
   Pro Lys Ala Ala Arg Pro Pro Val Thr Pro Val Leu Leu Glu Lys Lys
           435                 440                 445
   Ser Pro Leu Gly Gln Ser Gln Pro Thr Val Ala Gly Gln Pro Ser Ala
       450                 455                 460
   Arg Pro Ala Ala Glu Glu Tyr Gly Tyr Ile Val Thr Asp Gln Lys Pro
   465                 470                 475                 480
   Leu Ser Leu Ala Ala Gly Val Lys Leu Leu Glu Ile Leu Ala Glu His
                   485                 490                 495
   Val His Met Ser Ser Gly Ser Phe Ile Asn Ile Ser Val Val Gly Pro
               500                 505                 510
   Ala Leu Thr Phe Arg Ile Arg His Asn Glu Gln Asn Leu Ser Leu Ala
           515                 520                 525
   Asp Val Thr Gln Gln Ala Gly Leu Val Lys Ser Glu Leu Glu Ala Gln
       530                 535                 540
   Thr Gly Leu Gln Ile Leu Gln Thr Gly Val Gly Gln Arg Glu Glu Ala
   545                 550                 555                 560
   Ala Ala Val Leu Pro Gln Thr Ala His Ser Thr Ser Pro Met Arg Ser
                   565                 570                 575
   Val Leu Leu Thr Leu Val Ala Leu Ala Gly Val Ala Gly Leu Leu Val
               580                 585                 590
   Ala Leu Ala Val Ala Leu Cys Val Arg Gln His Ala Arg Gln Gln Asp
           595                 600                 605
   Lys Glu Arg Leu Ala Ala Leu Gly Pro Glu Gly Ala His Gly Asp Thr
       610                 615                 620
   Thr Phe Glu Tyr Gln Asp Leu Cys Arg Gln His Met Ala Thr Lys Ser
   625                 630                 635                 640
   Leu Phe Asn Arg Ala Glu Gly Pro Pro Glu Pro Ser Arg Val Ser Ser
                   645                 650                 655
   Val Ser Ser Gln Phe Ser Asp Ala Ala Gln Ala Ser Pro Ser Ser His
               660                 665                 670
   Ser Ser Thr Pro Ser Trp Cys Glu Glu Pro Ala Gln Ala Asn Met Asp
           675                 680                 685
   Ile Ser Thr Gly His Met Ile Leu Ala Tyr Met Glu Asp His Leu Arg
       690                 695                 700
   Asn Arg Asp Arg Leu Ala Lys Glu Trp Gln Ala Leu Cys Ala Tyr Gln
   705                 710                 715                 720
   Ala Glu Pro Asn Thr Cys Ala Thr Ala Gln Gly Glu Gly Asn Ile Lys
                   725                 730                 735
   Lys Asn Arg His Pro Asp Phe Leu Pro Tyr Asp His Ala Arg Ile Lys
               740                 745                 750
   Leu Lys Val Glu Ser Ser Pro Ser Arg Ser Asp Tyr Ile Asn Ala Ser
           755                 760                 765
   Pro Ile Ile Glu His Asp Pro Arg Met Pro Ala Tyr Ile Ala Thr Gln
       770                 775                 780
   Gly Pro Leu Ser His Thr Ile Ala Asp Phe Trp Gln Met Val Trp Glu
   785                 790                 795                 800
   Ser Gly Cys Thr Val Ile Val Met Leu Thr Pro Leu Val Glu Asp Gly
                   805                 810                 815
   Val Lys Gln Cys Asp Arg Tyr Trp Pro Asp Glu Gly Ala Ser Leu Tyr
               820                 825                 830
   His Val Tyr Glu Val Asn Leu Val Ser Glu His Ile Trp Cys Glu Asp
           835                 840                 845
   Phe Leu Val Arg Ser Phe Tyr Leu Lys Asn Val Gln Thr Gln Glu Thr
       850                 855                 860
   Arg Thr Leu Thr Gln Phe His Phe Leu Ser Trp Pro Ala Glu Gly Thr
   865                 870                 875                 880
   Pro Ala Ser Thr Arg Pro Leu Leu Asp Phe Arg Arg Lys Val Asn Lys
   885 890 895
   Cys Tyr Arg Gly Arg Ser Cys Pro Ile Ile Val His Cys Ser Asp Gly
   900 905 910
   Ala Gly Arg Thr Gly Thr Tyr Ile Leu Ile Asp Met Val Leu Asn Arg
   915 920 925
   Met Ala Lys Gly Val Lys Glu Ile Asp Ile Ala Ala Thr Leu Glu His
   930 935 940
   Val Arg Asp Gln Arg Pro Gly Leu Val Arg Ser Lys Asp Gln Phe Glu
   945 950 955 960
   Phe Ala Leu Thr Ala Val Ala Glu Glu Val Asn Ala Ile Leu Lys Ala
   965 970 975
   Leu Pro Gln
<210> 2199
   <211> 15
   <212> PRT
   <213> Mus musculus
<400> 2199
   Ala Glu Leu Asn Gln Leu Arg Ala Lys Glu Pro Thr Lys Leu Ala
   1 5 10 15
<210> 2200
   <211> 15
   <212> PRT
   <213> Mus musculus
<400> 2200
   Glu Leu Arg Glu Leu Arg Leu Arg Leu Asp Gln Leu Thr Ala Asn
   1 5 10 15
<210> 2201
   <211> 9
   <212> PRT
   <213> Homo sapiens
<400> 2201
   Glu Arg Gly Phe Phe Tyr Thr Pro Lys
   1 5
<210> 2202
   <211> 7
   <212> PRT
   <213> Mus musculus
<400> 2202
   Gly Leu Ala Pro Ser Gly Ala
   1 5
<210> 2203
   <211> 10
   <212> PRT
   <213> Homo sapiens
<400> 2203
   Leu Cys Gly Ser His Leu Val Glu Ala Leu
   1 5 10
<210> 2204
   <211> 10
   <212> PRT
   <213> Homo sapiens
<400> 2204
   Leu Val Cys Gly Glu Arg Gly Phe Phe Tyr
   1 5 10
<210> 2205
   <211> 10
   <212> PRT
   <213> Homo sapiens
<400> 2205
   Leu Tyr Leu Val Cys Gly Glu Arg Gly Phe
   1 5 10
<210> 2206
   <211> 15
   <212> PRT
   <213> Mus musculus
<400> 2206
   Gln Val His Val Glu Met Asp Val Ala Lys Pro Asp Leu Thr Ala
   1 5 10 15
<210> 2207
   <211> 9
   <212> PRT
   <213> Mus musculus
<400> 2207
   Trp Tyr Arg Ser Lys Phe Ala Asp Leu
   1 5
<210> 2208
   <211> 13
   <212> PRT
   <213> Bos taurus
<400> 2208
   Phe Lys Ala Asp Glu Lys Lys Phe Trp Gly Lys Tyr Leu
   1               5                   10
<210> 2209
   <211> 9
   <212> PRT
   <213> Homo sapiens
<400> 2209
   Gln Met Arg Pro Val Ser Arg Val Leu
   1               5
<210> 2210
   <211> 5
   <212> PRT
   <213> Bos taurus
<400> 2210
   Ala Asp Glu Lys Lys
   1               5
<210> 2211
   <211> 16
   <212> PRT
   <213> Bos taurus
<400> 2211
   Ala Asp Glu Lys Lys Phe Trp Gly Lys Tyr Leu Tyr Glu Ile Ala Arg
   1               5                   10                  15
<210> 2212
   <211> 5
   <212> PRT
   <213> Bos taurus
<400> 2212
   Glu Phe Lys Ala Asp
   1               5
<210> 2213
   <211> 6
   <212> PRT
   <213> Bos taurus
<400> 2213
   Glu Phe Lys Ala Asp Glu
   1 5
<210> 2214
   <211> 8
   <212> PRT
   <213> Bos taurus
<400> 2214
   Glu Phe Lys Ala Asp Glu Lys Lys
   1 5
<210> 2215
   <211> 6
   <212> PRT
   <213> Bos taurus
<400> 2215
   Phe Lys Ala Asp Glu Lys
   1 5
<210> 2216
   <211> 9
   <212> PRT
   <213> Simian immunodeficiency virus
<400> 2216
   Phe Tyr Cys Lys Met Asn Trp Phe Leu
   1 5
<210> 2217
   <211> 9
   <212> PRT
   <213> Aquifex aeolicus
<400> 2217
   Gly Phe Ile Lys Asn Phe Trp Phe Ile
   1 5
<210> 2218
   <211> 9
   <212> PRT
   <213> Helicobacter pylori
<400> 2218
   Gly Tyr Asn Lys Ala Met Gly Phe Leu
   1 5
<210> 2219
   <211> 9
   <212> PRT
   <213> Helicobacter pylori
<400> 2219
   Ile Phe Phe Lys Thr Ser Ser Phe Leu
   1 5
<210> 2220
   <211> 9
   <212> PRT
   <213> Saccharomyces cerevisiae
<400> 2220
   Ile Tyr Gln Lys Ala Asn Thr Phe Leu
   1 5
<210> 2221
   <211> 9
   <212> PRT
   <213> Helicobacter pylori
<400> 2221
   Ile Tyr Val Lys Thr Ser Ser Phe Leu
   1 5
<210> 2222
   <211> 6
   <212> PRT
   <213> Bos taurus
<400> 2222
   Lys Ala Asp Glu Lys Lys
   1 5
<210> 2223
   <211> 9
   <212> PRT
   <213> Borrelia burgdorferi
<400> 2223
   Lys Tyr Asn Lys Asn Gly Asn Phe Leu
   1 5
<210> 2224
   <211> 9
   <212> PRT
   <213> Haemophilus influenzae
<400> 2224
   Lys Tyr Tyr Lys Asn Ser Gln Phe Leu
   1 5
<210> 2225
   <211> 9
   <212> PRT
   <213> Saccharomyces cerevisiae
<400> 2225
   Lys Tyr Tyr Lys Thr Ser Asn Phe Leu
   1 5
<210> 2226
   <211> 18
   <212> PRT
   <213> Bos taurus
<400> 2226
   Leu Pro Lys Leu Lys Pro Asp Pro Asn Thr Leu Cys Asp Glu Phe Lys
   1 5 10 15
   Ala Asp
<210> 2227
   <211> 9
   <212> PRT
   <213> Borrelia burgdorferi 6
<400> 2227
   Leu Tyr Ser Lys Asn Leu Val Phe Leu
   1 5
<210> 2228
   <211> 9
   <212> PRT
   <213> Methanothermobacter thermautotrophicus
<400> 2228
   Asn Phe Met Lys Thr Ala Gly Phe Ile
   1 5
<210> 2229
   <211> 9
   <212> PRT
   <213> Rickettsia prowazekii
<400> 2229
   Gln Tyr Asn Lys Thr Gly Tyr Phe Ile
   1 5
<210> 2230
   <211> 9
   <212> PRT
   <213> Langat virus
<400> 2230
   Arg Phe Ser Lys Ala Leu Tyr Phe Leu
   1               5
<210> 2231
   <211> 9
   <212> PRT
   <213> Escherichia coli
<400> 2231
   Arg Tyr Gln Lys Ala Gly Val Met Leu
   1               5
<210> 2232
   <211> 9
   <212> PRT
   <213> Aquifex aeolicus
<400> 2232
   Thr Tyr Leu Lys Asn Ala Gln Phe Val
   1               5
<210> 2233
   <211> 9
   <212> PRT
   <213> Drosophila melanogaster
<400> 2233
   Val Tyr Val Lys Thr Ser Ser Phe Ile
   1               5
<210> 2234
   <211> 9
   <212> PRT
   <213> Haemophilus influenzae
<400> 2234
   Tyr Phe Leu Lys Cys Gly Tyr Phe Leu
   1               5
<210> 2235
   <211> 9
   <212> PRT
   <213> Sonchus yellow net virus
<400> 2235
   Tyr Tyr Asn Lys Ala Leu Ser Phe Ile
   1               5
<210> 2236
   <211> 21
   <212> PRT
   <213> Homo sapiens
<400> 2236
   His Gly Asp Thr Thr Phe Glu Tyr Gln Asp Leu Cys Arg Gln His Met
   1               5                   10                  15
   Ala Thr Lys Ser Leu
               20
<210> 2237
   <211> 21
   <212> PRT
   <213> Homo sapiens
<400> 2237
   Arg Leu Ala Ala Leu Gly Pro Glu Gly Ala His Gly Asp Thr Thr Phe
   1               5                   10                  15
   Glu Tyr Gln Asp Leu
               20
<210> 2238
   <211> 20
   <212> PRT
   <213> Homo sapiens
<400> 2238
   Arg Gln His Ala Arg Gln Gln Asp Lys Glu Arg Leu Ala Ala Leu Gly
   1               5                   10                  15
   Pro Glu Gly Ala
               20
<210> 2239
   <211> 42
   <212> PRT
   <213> Homo sapiens
<400> 2239
   Arg Gln His Ala Arg Gln Gln Asp Lys Glu Arg Leu Ala Ala Leu Gly
   1               5                   10                  15
   Pro Glu Gly Ala His Gly Asp Thr Thr Phe Glu Tyr Gln Asp Leu Cys
               20                  25                  30
   Arg Gln His Met Ala Thr Lys Ser Leu Phe
           35                  40
<210> 2240
   <211> 16
   <212> PRT
   <213> Mus musculus
<400> 2240
   Gln Cys Cys Thr Ser Ile Cys Ser Leu Tyr Gln Leu Glu Asn Tyr Cys
   1               5                   10                  15
<210> 2241
   <211> 12
   <212> PRT
   <213> Bos taurus
<400> 2241
   Glu Asp Lys Gly Ala Cys Leu Leu Pro Lys Ile Glu
   1               5                   10
<210> 2242
   <211> 30
   <212> PRT
   <213> Homo sapiens
<400> 2242
   Phe Phe Arg Met Val Ile Ser Asn Pro Ala Ala Thr His Gln Asp Ile
   1               5                   10                  15
   Asp Phe Leu Ile Glu Glu Ile Glu Arg Leu Gly Gln Asp Leu
               20                  25                  30
<210> 2243
   <211> 41
   <212> PRT
   <213> Homo sapiens
<400> 2243
   Gly Gly Ala Ile Ser Asn Met Tyr Ala Met Met Ile Ala Arg Phe Lys
   1               5                   10                  15
   Met Phe Pro Glu Val Lys Glu Lys Gly Met Ala Ala Leu Pro Arg Leu
               20                  25                  30
   Ile Ala Phe Thr Ser Glu His Ser His
           35                  40
<210> 2244
   <211> 9
   <212> PRT
   <213> Unknown
<220>
   <223> epitope involved in diabetes
<400> 2244
   Leu Tyr Leu Val Cys Gly Glu Arg Leu
   1 5
<210> 2245
   <211> 17
   <212> PRT
   <213> Homo sapiens
<400> 2245
   Asn Ile Ile Lys Asn Arg Glu Gly Tyr Glu Met Val Phe Asp Gly Lys
   1 5 10 15
   Pro
<210> 2246
   <211> 4
   <212> PRT
   <213> Homo sapiens
<400> 2246
   Pro Gly Gly Ser
   1
<210> 2247
   <211> 9
   <212> PRT
   <213> Homo sapiens
<400> 2247
   Val Ile Lys Ala Arg Met Met Glu Tyr
   1 5
<210> 2248
   <211> 9
   <212> PRT
   <213> Unknown
<220>
   <223> epitope involved in diabetes
<400> 2248
   Tyr Ala Ile Glu Asn Tyr Leu Glu Leu
   1 5
<210> 2249
   <211> 585
   <212> PRT
   <213> Homo sapiens
<400> 2249
   Met Ala Ser Pro Gly Ser Gly Phe Trp Ser Phe Gly Ser Glu Asp Gly
   1               5                   10                  15
   Ser Gly Asp Ser Glu Asn Pro Gly Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ala Glu Lys Pro Ala Glu Ser Gly Gly Ser Gln Pro Pro
       50                  55                  60
   Arg Ala Ala Ala Arg Lys Ala Ala Cys Ala Cys Asp Gln Lys Pro Cys
   65                  70                  75                  80
   Ser Cys Ser Lys Val Asp Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Asn Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
               180                 185                 190
   Ala Asp Trp Leu Thr Ser Thr Ala Asn Thr Asn Met Phe Thr Tyr Glu
           195                 200                 205
   Ile Ala Pro Val Phe Val Leu Leu Glu Tyr Val Thr Leu Lys Lys Met
       210                 215                 220
   Arg Glu Ile Ile Gly Trp Pro Gly Gly Ser Gly Asp Gly Ile Phe Ser
   225                 230                 235                 240
   Pro Gly Gly Ala Ile Ser Asn Met Tyr Ala Met Met Ile Ala Arg Phe
                   245                 250                 255
   Lys Met Phe Pro Glu Val Lys Glu Lys Gly Met Ala Ala Leu Pro Arg
   260 265 270
   Leu Ile Ala Phe Thr Ser Glu His Ser His Phe Ser Leu Lys Lys Gly
   275 280 285
   Ala Ala Ala Leu Gly Ile Gly Thr Asp Ser Val Ile Leu Ile Lys Cys
   290 295 300
   Asp Glu Arg Gly Lys Met Ile Pro Ser Asp Leu Glu Arg Arg Ile Leu
   305 310 315 320
   Glu Ala Lys Gln Lys Gly Phe Val Pro Phe Leu Val Ser Ala Thr Ala
   325 330 335
   Gly Thr Thr Val Tyr Gly Ala Phe Asp Pro Leu Leu Ala Val Ala Asp
   340 345 350
   Ile Cys Lys Lys Tyr Lys Ile Trp Met His Val Asp Ala Ala Trp Gly
   355 360 365
   Gly Gly Leu Leu Met Ser Arg Lys His Lys Trp Lys Leu Ser Gly Val
   370 375 380
   Glu Arg Ala Asn Ser Val Thr Trp Asn Pro His Lys Met Met Gly Val
   385 390 395 400
   Pro Leu Gln Cys Ser Ala Leu Leu Val Arg Glu Glu Gly Leu Met Gln
   405 410 415
   Asn Cys Asn Gln Met His Ala Ser Tyr Leu Phe Gln Gln Asp Lys His
   420 425 430
   Tyr Asp Leu Ser Tyr Asp Thr Gly Asp Lys Ala Leu Gln Cys Gly Arg
   435 440 445
   His Val Asp Val Phe Lys Leu Trp Leu Met Trp Arg Ala Lys Gly Thr
   450 455 460
   Thr Gly Phe Glu Ala His Val Asp Lys Cys Leu Glu Leu Ala Glu Tyr
   465 470 475 480
   Leu Tyr Asn Ile Ile Lys Asn Arg Glu Gly Tyr Glu Met Val Phe Asp
   485 490 495
   Gly Lys Pro Gln His Thr Asn Val Cys Phe Trp Tyr Ile Pro Pro Ser
   500 505 510
   Leu Arg Thr Leu Glu Asp Asn Glu Glu Arg Met Ser Arg Leu Ser Lys
   515 520 525
   Val Ala Pro Val Ile Lys Ala Arg Met Met Glu Tyr Gly Thr Thr Met
   530 535 540
   Val Ser Tyr Gln Pro Leu Gly Asp Lys Val Asn Phe Phe Arg Met Val
   545 550 555 560
   Ile Ser Asn Pro Ala Ala Thr His Gln Asp Ile Asp Phe Leu Ile Glu
   565 570 575
   Glu Ile Glu Arg Leu Gly Gln Asp Leu
   580 585
<210> 2250
   <211> 12
   <212> PRT
   <213> Mus musculus
<400> 2250
   Leu Tyr Phe Cys Ala Ala Arg Ser Asn Tyr Gln Leu
   1 5 10
<210> 2251
   <211> 15
   <212> PRT
   <213> Homo sapiens
<400> 2251
   Ala Tyr Val Leu Leu Ser Glu Lys Lys Ile Ser Ser Ile Gln Ser
   1 5 10 15
<210> 2252
   <211> 15
   <212> PRT
   <213> Homo sapiens
<400> 2252
   Gly Glu Ala Leu Ser Thr Leu Val Leu Asn Arg Leu Lys Val Gly
   1 5 10 15
<210> 2253
   <211> 15
   <212> PRT
   <213> Mycobacterium
<400> 2253
   Gly Glu Ala Leu Ser Thr Leu Val Val Asn Lys Ile Arg Gly Thr
   1 5 10 15
<210> 2254
   <211> 11
   <212> PRT
   <213> Homo sapiens
<400> 2254
   Ala Leu Trp Gly Pro Asp Pro Ala Ala Ala Phe
   1 5 10
<210> 2255
   <211> 10
   <212> PRT
   <213> Homo sapiens
<400> 2255
   Leu Ala Leu Trp Gly Pro Asp Pro Ala Ala
   1 5 10
<210> 2256
   <211> 11
   <212> PRT
   <213> Homo sapiens
<400> 2256
   Arg Leu Leu Pro Leu Leu Ala Leu Leu Ala Leu
   1 5 10
<210> 2257
   <211> 42
   <212> PRT
   <213> Homo sapiens
<400> 2257
   Ile Ala Ala Thr Leu Glu His Val Arg Asp Gln Arg Pro Gly Leu Val
   1 5 10 15
   Arg Ser Lys Asp Gln Phe Glu Phe Ala Leu Thr Ala Val Ala Glu Glu
   20 25 30
   Val Asn Ala Ile Leu Lys Ala Leu Pro Gln
   35 40
<210> 2258
   <211> 9
   <212> PRT
   <213> Homo sapiens
<400> 2258
   Asn Leu Ala Gln Asp Leu Ala Thr Val
   1 5
<210> 2259
   <211> 9
   <212> PRT
   <213> Homo sapiens
<400> 2259
   Gln Leu Ala Arg Gln Gln Val His Val
   1 5
<210> 2260
   <211> 979
   <212> PRT
   <213> Homo sapiens
<400> 2260
   Met Arg Arg Pro Arg Arg Pro Gly Gly Leu Gly Gly Ser Gly Gly Leu
   1               5                   10                  15
   Arg Leu Leu Leu Cys Leu Leu Leu Leu Ser Ser Arg Pro Gly Gly Cys
               20                  25                  30
   Ser Ala Val Ser Ala His Gly Cys Leu Phe Asp Arg Arg Leu Cys Ser
           35                  40                  45
   His Leu Glu Val Cys Ile Gln Asp Gly Leu Phe Gly Gln Cys Gln Val
       50                  55                  60
   Gly Val Gly Gln Ala Arg Pro Leu Leu Gln Val Thr Ser Pro Val Leu
   65                  70                  75                  80
   Gln Arg Leu Gln Gly Val Leu Arg Gln Leu Met Ser Gln Gly Leu Ser
                   85                  90                  95
   Trp His Asp Asp Leu Thr Gln Tyr Val Ile Ser Gln Glu Met Glu Arg
               100                 105                 110
   Ile Pro Arg Leu Arg Pro Pro Glu Pro Arg Pro Arg Asp Arg Ser Gly
           115                 120                 125
   Leu Ala Pro Lys Arg Pro Gly Pro Ala Gly Glu Leu Leu Leu Gln Asp
       130                 135                 140
   Ile Pro Thr Gly Ser Ala Pro Ala Ala Gln His Arg Leu Pro Gln Pro
   145                 150                 155                 160
   Pro Val Gly Lys Gly Gly Ala Gly Ala Ser Ser Ser Leu Ser Pro Leu
                   165                 170                 175
   Gln Ala Glu Leu Leu Pro Pro Leu Leu Glu His Leu Leu Leu Pro Pro
   180 185 190
   Gln Pro Pro His Pro Ser Leu Ser Tyr Glu Pro Ala Leu Leu Gln Pro
   195 200 205
   Tyr Leu Phe His Gln Phe Gly Ser Arg Asp Gly Ser Arg Val Ser Glu
   210 215 220
   Gly Ser Pro Gly Met Val Ser Val Gly Pro Leu Pro Lys Ala Glu Ala
   225 230 235 240
   Pro Ala Leu Phe Ser Arg Thr Ala Ser Lys Gly Ile Phe Gly Asp His
   245 250 255
   Pro Gly His Ser Tyr Gly Asp Leu Pro Gly Pro Ser Pro Ala Gln Leu
   260 265 270
   Phe Gln Asp Ser Gly Leu Leu Tyr Leu Ala Gln Glu Leu Pro Ala Pro
   275 280 285
   Ser Arg Ala Arg Val Pro Arg Leu Pro Glu Gln Gly Ser Ser Ser Arg
   290 295 300
   Ala Glu Asp Ser Pro Glu Gly Tyr Glu Lys Glu Gly Leu Gly Asp Arg
   305 310 315 320
   Gly Glu Lys Pro Ala Ser Pro Ala Val Gln Pro Asp Ala Ala Leu Gln
   325 330 335
   Arg Leu Ala Ala Val Leu Ala Gly Tyr Gly Val Glu Leu Arg Gln Leu
   340 345 350
   Thr Pro Glu Gln Leu Ser Thr Leu Leu Thr Leu Leu Gln Leu Leu Pro
   355 360 365
   Lys Gly Ala Gly Arg Asn Pro Gly Gly Val Val Asn Val Gly Ala Asp
   370 375 380
   Ile Lys Lys Thr Met Glu Gly Pro Val Glu Gly Arg Asp Thr Ala Glu
   385 390 395 400
   Leu Pro Ala Arg Thr Ser Pro Met Pro Gly His Pro Thr Ala Ser Pro
   405 410 415
   Thr Ser Ser Glu Val Gln Gln Val Pro Ser Pro Val Ser Ser Glu Pro
   420 425 430
   Pro Lys Ala Ala Arg Pro Pro Val Thr Pro Val Leu Leu Glu Lys Lys
   435 440 445
   Ser Pro Leu Gly Gln Ser Gln Pro Thr Val Ala Gly Gln Pro Ser Ala
   450 455 460
   Arg Pro Ala Ala Glu Glu Tyr Gly Tyr Ile Val Thr Asp Gln Lys Pro
   465 470 475 480
   Leu Ser Leu Ala Ala Gly Val Lys Leu Leu Glu Ile Leu Ala Glu His
   485 490 495
   Val His Met Ser Ser Gly Ser Phe Ile Asn Ile Ser Val Val Gly Pro
   500 505 510
   Ala Leu Thr Phe Arg Ile Arg His Asn Glu Gln Asn Leu Ser Leu Ala
   515 520 525
   Asp Val Thr Gln Gln Ala Gly Leu Val Lys Ser Glu Leu Glu Ala Gln
   530 535 540
   Thr Gly Leu Gln Ile Leu Gln Thr Gly Val Gly Gln Arg Glu Glu Ala
   545 550 555 560
   Ala Ala Val Leu Pro Gln Thr Ala His Ser Thr Ser Pro Met Arg Ser
   565 570 575
   Val Leu Leu Thr Leu Val Ala Leu Ala Gly Val Ala Gly Leu Leu Val
   580 585 590
   Ala Leu Ala Val Ala Leu Cys Val Arg Gln His Ala Arg Gln Gln Asp
   595 600 605
   Lys Glu Arg Leu Ala Ala Leu Gly Pro Glu Gly Ala His Gly Asp Thr
   610 615 620
   Thr Phe Glu Tyr Gln Asp Leu Cys Arg Gln His Met Ala Thr Lys Ser
   625 630 635 640
   Leu Phe Asn Arg Ala Glu Gly Pro Pro Glu Pro Ser Arg Val Ser Ser
   645 650 655
   Val Ser Ser Gln Phe Ser Asp Ala Ala Gln Ala Ser Pro Ser Ser His
   660 665 670
   Ser Ser Thr Pro Ser Trp Cys Glu Glu Pro Ala Gln Ala Asn Met Asp
           675                 680                 685
   Ile Ser Thr Gly His Met Ile Leu Ala Tyr Met Glu Asp His Leu Arg
       690                 695                 700
   Asn Arg Asp Arg Leu Ala Lys Glu Trp Gln Ala Leu Cys Ala Tyr Gln
   705                 710                 715                 720
   Ala Glu Pro Asn Thr Cys Ala Thr Ala Gln Gly Glu Gly Asn Ile Lys
                   725                 730                 735
   Lys Asn Arg His Pro Asp Phe Leu Pro Tyr Asp His Ala Arg Ile Lys
               740                 745                 750
   Leu Lys Val Glu Ser Ser Pro Ser Arg Ser Asp Tyr Ile Asn Ala Ser
           755                 760                 765
   Pro Ile Ile Glu His Asp Pro Arg Met Pro Ala Tyr Ile Ala Thr Gln
       770                 775                 780
   Gly Pro Leu Ser His Thr Ile Ala Asp Phe Trp Gln Met Val Trp Glu
   785                 790                 795                 800
   Ser Gly Cys Thr Val Ile Val Met Leu Thr Pro Leu Val Glu Asp Gly
                   805                 810                 815
   Val Lys Gln Cys Asp Arg Tyr Trp Pro Asp Glu Gly Ala Ser Leu Tyr
               820                 825                 830
   His Val Tyr Glu Val Asn Leu Val Ser Glu His Ile Trp Cys Glu Asp
           835                 840                 845
   Phe Leu Val Arg Ser Phe Tyr Leu Lys Asn Val Gln Thr Gln Glu Thr
       850                 855                 860
   Arg Thr Leu Thr Gln Phe His Phe Leu Ser Trp Pro Ala Glu Gly Thr
   865                 870                 875                 880
   Pro Ala Ser Thr Arg Pro Leu Leu Asp Phe Arg Arg Lys Val Asn Lys
                   885                 890                 895
   Cys Tyr Arg Gly Arg Ser Cys Pro Ile Ile Val His Cys Ser Asp Gly
               900                 905                 910
   Ala Gly Arg Thr Gly Thr Tyr Ile Leu Ile Asp Met Val Leu Asn Arg
           915                 920                 925
   Met Ala Lys Gly Val Lys Glu Ile Asp Ile Ala Ala Thr Leu Glu His
   930 935 940
   Val Arg Asp Gln Arg Pro Gly Leu Val Arg Ser Lys Asp Gln Phe Glu
   945 950 955 960
   Phe Ala Leu Thr Ala Val Ala Glu Glu Val Asn Ala Ile Leu Lys Ala
   965 970 975
   Leu Pro Gln
<210> 2261
   <211> 12
   <212> PRT
   <213> Human coxsackievirus
<400> 2261
   Glu Trp Leu Lys Val Lys Ile Leu Pro Glu Val Lys
   1 5 10
<210> 2262
   <211> 20
   <212> PRT
   <213> Enterovirus A
<400> 2262
   Trp Leu Lys Val Lys Ile Leu Pro Glu Val Lys Glu Lys His Glu Phe
   1 5 10 15
   Leu Asn Arg Leu
   20
<210> 2263
   <211> 15
   <212> PRT
   <213> Human Endogenous Retrovirus
<400> 2263
   Gly Lys Thr Cys Pro Lys Glu Ile Pro Lys Gly Ser Lys Asn Thr
   1 5 10 15
<210> 2264
   <211> 23
   <212> PRT
   <213> Homo sapiens
<400> 2264
   Ala Arg Phe Lys Met Phe Pro Glu Val Lys Glu Lys Gly Met Ala Ala
   1 5 10 15
   Leu Pro Arg Leu Ile Ala Phe
   20
<210> 2265
   <211> 23
   <212> PRT
   <213> Human coxsackievirus
<400> 2265
   Leu Lys Val Lys Ile Leu Pro Glu Val Lys Glu Lys His Glu Phe Leu
   1 5 10 15
   Ser Arg Leu Lys Gln Leu Pro
   20
<210> 2266
   <211> 10
   <212> PRT
   <213> Unknown
<220>
   <223> epitope involved in diabetes
<400> 2266
   Ser Leu Tyr Gln Leu Glu Asn Tyr Cys Ala
   1 5 10
<210> 2267
   <211> 13
   <212> PRT
   <213> Mus musculus
<400> 2267
   Tyr Lys Met Phe Pro Glu Val Lys Glu Lys Gly Met Ala
   1 5 10
<210> 2268
   <211> 13
   <212> PRT
   <213> Mus musculus
<400> 2268
   Tyr Lys Tyr Phe Pro Glu Val Lys Thr Lys Gly Met Ala
   1 5 10
<210> 2269
   <211> 9
   <212> PRT
   <213> Unknown
<220>
   <223> epitope involved in diabetes
<400> 2269
   Ala Arg Lys Val Lys Gly Ala Ala Gly
   1               5
<210> 2270
   <211> 9
   <212> PRT
   <213> Unknown
<220>
   <223> epitope involved in diabetes
<400> 2270
   Ala Arg Leu Ser Val Val Arg Asn Gly
   1               5
<210> 2271
   <211> 21
   <212> PRT
   <213> Homo sapiens
<400> 2271
   Asp Lys Val Asn Phe Phe Arg Met Val Ile Ser Asn Pro Ala Ala Thr
   1               5                   10                  15
   His Gln Asp Ile Asp
               20
<210> 2272
   <211> 9
   <212> PRT
   <213> Unknown
<220>
   <223> epitope involved in diabetes
<400> 2272
   Phe Arg Lys Ala Asp Lys Phe Pro Met
   1               5
<210> 2273
   <211> 9
   <212> PRT
   <213> Unknown
<220>
   <223> epitope involved in diabetes
<400> 2273
   Phe Arg Lys Thr Ser Ile Ser Gly Ala
   1               5
<210> 2274
   <211> 9
   <212> PRT
   <213> Unknown
<220>
   <223> epitope involved in diabetes
<400> 2274
   Phe Arg Lys Thr Ser Lys Gly Gly Ser
   1 5
<210> 2275
   <211> 9
   <212> PRT
   <213> Unknown
<220>
   <223> epitope involved in diabetes
<400> 2275
   Gly Gly Thr Thr Thr Pro Arg Thr Thr
   1 5
<210> 2276
   <211> 9
   <212> PRT
   <213> Unknown
<220>
   <223> epitope involved in diabetes
<400> 2276
   Gly Arg Lys Ser Ala Val Ser Pro Ala
   1 5
<210> 2277
   <211> 9
   <212> PRT
   <213> Unknown
<220>
   <223> epitope involved in diabetes
<400> 2277
   His Lys Lys Ser Leu Ser Ser Pro Ser
   1 5
<210> 2278
   <211> 9
   <212> PRT
   <213> Unknown
<220>
   <223> epitope involved in diabetes
<400> 2278
   His Arg Lys Ser Thr Val Ala Gly Ser
   1 5
<210> 2279
   <211> 9
   <212> PRT
   <213> Unknown
<220>
   <223> epitope involved in diabetes
<400> 2279
   Lys Gly Lys Lys Ala Leu Gln Ser Ala
   1 5
<210> 2280
   <211> 9
   <212> PRT
   <213> Unknown
<220>
   <223> epitope involved in diabetes
<400> 2280
   Lys Lys Lys Lys Tyr Ala Glu Ala Val
   1 5
<210> 2281
   <211> 9
   <212> PRT
   <213> Unknown
<220>
   <223> epitope involved in diabetes
<400> 2281
   Lys Lys Thr Lys Gly Leu Val Thr Thr
   1 5
<210> 2282
   <211> 9
   <212> PRT
   <213> Unknown
<220>
   <223> epitope involved in diabetes
<400> 2282
   Lys Arg Arg Ala Ser Ala Gly Gly Pro
   1 5
<210> 2283
   <211> 9
   <212> PRT
   <213> Unknown
<220>
   <223> epitope involved in diabetes
<400> 2283
   Lys Ser Val Lys Pro Arg Gln Ala Thr
   1 5
<210> 2284
   <211> 9
   <212> PRT
   <213> Unknown
<220>
   <223> epitope involved in diabetes
<400> 2284
   Leu Arg Lys Lys Gly Tyr Asp Pro Gly
   1 5
<210> 2285
   <211> 9
   <212> PRT
   <213> Unknown
<220>
   <223> epitope involved in diabetes
<400> 2285
   Leu Ser Arg Lys Lys Thr Leu Thr Thr
   1 5
<210> 2286
   <211> 13
   <212> PRT
   <213> Homo sapiens
<400> 2286
   Met Phe Pro Glu Val Lys Glu Lys Gly Met Ala Ala Leu
   1 5 10
<210> 2287
   <211> 9
   <212> PRT
   <213> Unknown
<220>
   <223> epitope involved in diabetes
<400> 2287
   Met Arg Lys Ala Asn Ser Pro Pro Thr
   1 5
<210> 2288
   <211> 9
   <212> PRT
   <213> Unknown
<220>
   <223> epitope involved in diabetes
<400> 2288
   Met Arg Lys Ser Thr Gly Thr Ala Ser
   1 5
<210> 2289
   <211> 9
   <212> PRT
   <213> Unknown
<220>
   <223> epitope involved in diabetes
<400> 2289
   Asn Arg Lys Leu Lys Ser Asn Met Met
   1 5
<210> 2290
   <211> 9
   <212> PRT
   <213> Unknown
<220>
   <223> epitope involved in diabetes
<400> 2290
   Asn Arg Arg Leu Leu Pro Met Pro Glu
   1 5
<210> 2291
   <211> 9
   <212> PRT
   <213> Unknown
<220>
   <223> epitope involved in diabetes
<400> 2291
   Gln Lys Lys Met Val Ala Leu Ser Gly
   1 5
<210> 2292
   <211> 9
   <212> PRT
   <213> Unknown
<220>
   <223> epitope involved in diabetes
<400> 2292
   Gln Lys Arg Ala Lys Gly Leu Ser Ala
   1 5
<210> 2293
   <211> 9
   <212> PRT
   <213> Unknown
<220>
   <223> epitope involved in diabetes
<400> 2293
   Arg Gly Leu Lys Ser Lys Ile Ala Met
   1 5
<210> 2294
   <211> 9
   <212> PRT
   <213> Unknown
<220>
   <223> epitope involved in diabetes
<400> 2294
   Arg Lys Lys Lys Pro Pro Gly Leu Ala
   1 5
<210> 2295
   <211> 9
   <212> PRT
   <213> Unknown
<220>
   <223> epitope involved in diabetes
<400> 2295
   Arg Lys Leu Ala His Pro Gly Thr Ser
   1 5
<210> 2296
   <211> 9
   <212> PRT
   <213> Unknown
<220>
   <223> epitope involved in diabetes
<400> 2296
   Arg Lys Arg Thr Arg His Leu Glu Gly
   1 5

<210> 2297
   <211> 9
   <212> PRT
   <213> Unknown
<220>
   <223> epitope involved in diabetes
<400> 2297
   Arg Lys Thr Ala Leu Gly Thr Arg Gln
   1 5
<210> 2298
   <211> 9
   <212> PRT
   <213> Unknown
<220>
   <223> epitope involved in diabetes
<400> 2298
   Arg Lys Val Val Gln Thr Ser Leu Glu
   1 5
<210> 2299
   <211> 9
   <212> PRT
   <213> Unknown
<220>
   <223> epitope involved in diabetes
<400> 2299
   Arg Arg Lys Leu Arg Thr Asn Ala Gly
   1 5
<210> 2300
   <211> 9
   <212> PRT
   <213> Unknown
<220>
   <223> epitope involved in diabetes
<400> 2300
   Arg Arg Lys Pro Gly Thr Glu Gln Leu
   1 5
<210> 2301
   <211> 9
   <212> PRT
   <213> Unknown
<220>
   <223> epitope involved in diabetes
<400> 2301
   Arg Ser Leu Lys Thr Lys Leu Pro Ser
   1               5
<210> 2302
   <211> 9
   <212> PRT
   <213> Unknown
<220>
   <223> epitope involved in diabetes
<400> 2302
   Ser Arg Lys Ala Thr Ala Ser Leu Pro
   1               5
<210> 2303
   <211> 9
   <212> PRT
   <213> Unknown
<220>
   <223> epitope involved in diabetes
<400> 2303
   Ser Arg Lys Lys Thr Phe Thr Gly Ala
   1               5
<210> 2304
   <211> 9
   <212> PRT
   <213> Unknown
<220>
   <223> epitope involved in diabetes
<400> 2304
   Ser Arg Lys Ser Gly Ser Leu Thr Lys
   1               5
<210> 2305
   <211> 9
   <212> PRT
   <213> Unknown
<220>
   <223> epitope involved in diabetes
<400> 2305
   Ser Arg Lys Val Ala Leu Gln Gly Gly
   1 5
<210> 2306
   <211> 9
   <212> PRT
   <213> Unknown
<220>
   <223> epitope involved in diabetes
<400> 2306
   Thr Lys Leu Ser Asn Thr Pro Ser Pro
   1 5
<210> 2307
   <211> 9
   <212> PRT
   <213> Unknown
<220>
   <223> epitope involved in diabetes
<400> 2307
   Thr Gln Asp Leu Gly Tyr Ser Arg Val
   1 5
<210> 2308
   <211> 9
   <212> PRT
   <213> Unknown
<220>
   <223> epitope involved in diabetes
<400> 2308
   Thr Arg Gly Leu Lys Gly Ala Pro Gln
   1 5
<210> 2309
   <211> 9
   <212> PRT
   <213> Unknown
<220>
   <223> epitope involved in diabetes
<400> 2309
   Thr Arg Lys Ala Leu Thr Gln Asn Thr
   1 5
<210> 2310
   <211> 20
   <212> PRT
   <213> Homo sapiens
<400> 2310
   Val Phe Asp Gly Lys Pro Gln His Thr Asn Val Cys Phe Trp Tyr Ile
   1 5 10 15
   Pro Pro Ser Leu
   20
<210> 2311
   <211> 20
   <212> PRT
   <213> Homo sapiens
<400> 2311
   Val Asn Phe Phe Arg Met Val Ile Ser Asn Pro Ala Ala Thr His Gln
   1 5 10 15
   Asp Ile Asp Phe
   20
<210> 2312
   <211> 9
   <212> PRT
   <213> Unknown
<220>
   <223> epitope involved in diabetes
<400> 2312
   Val Arg Lys Leu Thr Val Thr Ser Ala
   1 5
<210> 2313
   <211> 9
   <212> PRT
   <213> Unknown
<220>
   <223> epitope involved in diabetes
<400> 2313
   Tyr Lys Lys Gly Ala Arg Pro Ile Gln
   1 5
<210> 2314
   <211> 9
   <212> PRT
   <213> Unknown
<220>
   <223> epitope involved in diabetes
<400> 2314
   Tyr Arg Lys Lys Ser Ser Ala Glu Leu
   1 5
<210> 2315
   <211> 9
   <212> PRT
   <213> Unknown
<220>
   <223> epitope involved in diabetes
<400> 2315
   Tyr Arg Lys Thr Ala Lys Glu Gly Thr
   1 5
<210> 2316
   <211> 29
   <212> PRT
   <213> Rubella virus
<400> 2316
   Glu Ala Cys Val Thr Ser Trp Leu Trp Ser Glu Gly Glu Gly Ala Val
   1 5 10 15
   Phe Tyr Arg Val Asp Leu His Phe Ile Asn Leu Gly Thr
   20 25
<210> 2317
   <211> 21
   <212> PRT
   <213> Rubella virus
<400> 2317
   Met Asp Phe Trp Cys Val Glu His Asp Arg Pro Pro Pro Ala Thr Pro
   1 5 10 15
   Thr Ser Leu Thr Thr
   20
<210> 2318
   <211> 15
   <212> PRT
   <213> Homo sapiens
<400> 2318
   Asn Met Phe Thr Tyr Glu Ile Ala Pro Val Phe Val Leu Met Glu
   1 5 10 15
<210> 2319
   <211> 25
   <212> PRT
   <213> Rubella virus
<400> 2319
   Pro Phe Leu Gly His Asp Gly His His Gly Gly Thr Leu Arg Val Gly
   1               5                   10                  15
   Gln His His Arg Asn Ala Ser Asp Val
               20                  25
<210> 2320
   <211> 21
   <212> PRT
   <213> Rubella virus
<400> 2320
   Arg Val Lys Phe His Thr Glu Thr Arg Thr Val Trp Gln Leu Ser Val
   1               5                   10                  15
   Ala Gly Val Ser Cys
               20
<210> 2321
   <211> 16
   <212> PRT
   <213> Human coxsackievirus
<400> 2321
   Lys Ile Leu Pro Glu Val Lys Glu Lys His Glu Phe Leu Ser Arg Leu
   1               5                   10                  15
<210> 2322
   <211> 17
   <212> PRT
   <213> Homo sapiens
<400> 2322
   Lys Met Phe Pro Glu Val Lys Glu Lys Gly Met Ala Ala Leu Pro Arg
   1               5                   10                  15
   Leu
<210> 2323
   <211> 17
   <212> PRT
   <213> Homo sapiens
<400> 2323
   Lys Tyr Phe Pro Glu Val Lys Thr Lys Gly Met Ala Ala Val Pro Lys
   1 5 10 15
   Leu
<210> 2324
   <211> 7
   <212> PRT
   <213> Unknown
<220>
   <223> epitope involved in diabetes
<400> 2324
   Lys Arg Ser Arg Leu Asp Val
   1 5
<210> 2325
   <211> 7
   <212> PRT
   <213> Unknown
<220>
   <223> epitope involved in diabetes
<400> 2325
   Leu Gly Arg Gly Gly Ser Lys
   1 5
<210> 2326
   <211> 7
   <212> PRT
   <213> Unknown
<220>
   <223> epitope involved in diabetes
<400> 2326
   Arg Leu Gly Lys Arg Asp Ser
   1 5
<210> 2327
   <211> 7
   <212> PRT
   <213> Unknown
<220>
   <223> epitope involved in diabetes
<400> 2327
   Arg Gln Leu Gly Arg Gly Ser
   1 5
<210> 2328
   <211> 7
   <212> PRT
   <213> Unknown
<220>
   <223> epitope involved in diabetes
<400> 2328
   Ser Thr Val Gln Asp Cys Val
   1 5
<210> 2329
   <211> 7
   <212> PRT
   <213> Unknown
<220>
   <223> epitope involved in diabetes
<400> 2329
   Tyr Leu Gly Arg Gly Asp Lys
   1 5
<210> 2330
   <211> 12
   <212> PRT
   <213> Photobacterium phosphoreum
<400> 2330
   Ala Lys Val Ala Ile Val Ile Ala Arg Phe Asn Ser
   1 5 10
<210> 2331
   <211> 12
   <212> PRT
   <213> Human herpesvirus
<400> 2331
   Gly Gly Val Ala Val Val Ile Gly Arg Phe Phe Gly
   1 5 10
<210> 2332
   <211> 12
   <212> PRT
   <213> Synechocystis sp.
<400> 2332
   Ser Ala Ile Gly Ile Tyr Leu Gly Arg Phe Leu Arg
   1 5 10
<210> 2333
   <211> 10
   <212> PRT
   <213> Mus musculus
<400> 2333
   His Leu Cys Gly Pro His Leu Val Glu Ala
   1 5 10
<210> 2334
   <211> 9
   <212> PRT
   <213> Mus musculus
<400> 2334
   Ile Val Asp Gln Cys Cys Thr Ser Ile
   1 5
<210> 2335
   <211> 9
   <212> PRT
   <213> Mus musculus
<400> 2335
   Leu Leu Val His Phe Leu Pro Leu Leu
   1 5
<210> 2336
   <211> 13
   <212> PRT
   <213> Streptococcus pneumoniae
<400> 2336
   Asp Asp Ile Met Met Ser Tyr Ala Ala Lys Gly Phe Val
   1 5 10
<210> 2337
   <211> 13
   <212> PRT
   <213> Rickettsia prowazekii
<400> 2337
   Glu Leu Val Leu Met Ser Tyr Arg Asp Lys Leu Val Leu
   1 5 10
<210> 2338
   <211> 13
   <212> PRT
   <213> Human herpesvirus
<400> 2338
   Lys Glu Leu Leu Gln Ser Tyr Val Ser Lys Asn Asn Asn
   1 5 10
<210> 2339
   <211> 13
   <212> PRT
   <213> Legionella pneumophila
<400> 2339
   Lys Met Ser Leu Leu Gln Tyr Ile Val Lys Asn Lys Ile
   1 5 10
<210> 2340
   <211> 13
   <212> PRT
   <213> Lactococcus lactis
<400> 2340
   Leu Val Ile Leu Pro Met Phe His Val Ser Gly Leu Ser
   1 5 10
<210> 2341
   <211> 13
   <212> PRT
   <213> Homo sapiens
<400> 2341
   Pro Ile Ile Leu Pro Leu Trp His Val Gly Glu Pro Gly
   1 5 10
<210> 2342
   <211> 13
   <212> PRT
   <213> Homo sapiens
<400> 2342
   Pro Ile Ile Leu Pro Leu Trp His Val Gly Met Asn Asp
   1 5 10
<210> 2343
   <211> 13
   <212> PRT
   <213> Neisseria meningitidis
<400> 2343
   Pro Val Ile Leu Pro Gln Trp Gln Ser Leu Gly Asn Arg
   1 5 10
<210> 2344
   <211> 13
   <212> PRT
   <213> Staphylococcus aureus
<400> 2344
   Pro Tyr Val Leu Leu Ala Tyr Ile Val Lys Ser Val Gly
   1 5 10
<210> 2345
   <211> 13
   <212> PRT
   <213> Chlamydia pneumoniae
<400> 2345
   Gln Ala Met Asn Ile Leu Tyr Gln Thr Val Gln Ala Phe
   1 5 10
<210> 2346
   <211> 13
   <212> PRT
   <213> Streptococcus pyogenes
<400> 2346
   Arg Glu Ile Leu Pro Gln Tyr Gln Leu Val Ile Leu Ala
   1 5 10
<210> 2347
   <211> 13
   <212> PRT
   <213> Homo sapiens
<400> 2347
   Thr Thr Leu Leu Pro Gln Trp Arg Val Ser Ala Phe Val
   1 5 10
<210> 2348
   <211> 12
   <212> PRT
   <213> Homo sapiens
<400> 2348
   Ala Tyr Ala Lys Asp Val Lys Phe Gly Ala Asp Ala
   1 5 10
<210> 2349
   <211> 13
   <212> PRT
   <213> Mus musculus
<400> 2349
   Leu Ser Lys Val Ala Pro Val Ile Lys Ala Arg Met Met
   1 5 10
<210> 2350
   <211> 10
   <212> PRT
   <213> Unknown
<220>
   <223> epitope involved in diabetes
<400> 2350
   Ser Arg Leu Gly Leu Trp Val Arg Met Glu
   1 5 10
<210> 2351
   <211> 14
   <212> PRT
   <213> Mus musculus
<400> 2351
   Trp Ser Arg Met Asp Gln Leu Ala Lys Glu Leu Thr Ala Glu
   1 5 10
<210> 2352
   <211> 18
   <212> PRT
   <213> Mus musculus
<400> 2352
   Phe Leu Pro Leu Leu Ala Leu Leu Ala Leu Trp Glu Pro Lys Pro Thr
   1 5 10 15
   Gln Ala
<210> 2353
   <211> 20
   <212> PRT
   <213> Homo sapiens
<400> 2353
   Gly Gly Gly Pro Gly Ala Gly Ser Leu Gln Pro Leu Ala Leu Glu Gly
   1 5 10 15
   Ser Leu Gln Lys
   20
<210> 2354
   <211> 24
   <212> PRT
   <213> Homo sapiens
<400> 2354
   Lys Leu Lys Val Glu Ser Ser Pro Ser Arg Ser Asp Tyr Ile Asn Ala
   1 5 10 15
   Ser Pro Ile Ile Glu His Asp Pro
   20
<210> 2355
   <211> 28
   <212> PRT
   <213> Homo sapiens
<400> 2355
   Leu Ala Lys Glu Trp Gln Ala Leu Cys Ala Tyr Gln Ala Glu Pro Asn
   1               5                   10                  15
   Thr Cys Ala Thr Ala Gln Gly Glu Gly Asn Ile Lys
               20                  25
<210> 2356
   <211> 20
   <212> PRT
   <213> Homo sapiens
<400> 2356
   Ser Phe Tyr Leu Lys Asn Val Gln Thr Gln Glu Thr Arg Thr Leu Thr
   1               5                   10                  15
   Gln Phe His Phe
               20
<210> 2357
   <211> 22
   <212> PRT
   <213> Homo sapiens
<400> 2357
   Ser Lys Asp Gln Phe Glu Phe Ala Leu Thr Ala Val Ala Glu Glu Val
   1               5                   10                  15
   Asn Ala Ile Leu Lys Ala
               20
<210> 2358
   <211> 29
   <212> PRT
   <213> Homo sapiens
<400> 2358
   Ser Arg Val Ser Ser Val Ser Ser Gln Phe Ser Asp Ala Ala Gln Ala
   1               5                   10                  15
   Ser Pro Ser Ser His Ser Ser Thr Pro Ser Trp Cys Glu
               20                  25
<210> 2359
   <211> 12
   <212> PRT
   <213> Homo sapiens
<400> 2359
   Ala Phe Leu Gln Asp Val Met Asn Ile Leu Leu Gln
   1 5 10
<210> 2360
   <211> 19
   <212> PRT
   <213> Homo sapiens
<400> 2360
   Ala Leu Leu Arg Cys Ile Pro Ala Leu Asp Ser Leu Thr Pro Ala Asn
   1 5 10 15
   Glu Asp Cys
<210> 2361
   <211> 12
   <212> PRT
   <213> Homo sapiens
<400> 2361
   Ala Pro Lys Arg Pro Gly Pro Ala Gly Glu Leu Leu
   1 5 10
<210> 2362
   <211> 12
   <212> PRT
   <213> Human coxsackievirus
<400> 2362
   Ala Thr Cys Arg Phe Tyr Thr Leu Asp Ser Ile Lys
   1 5 10
<210> 2363
   <211> 12
   <212> PRT
   <213> Human coxsackievirus
<400> 2363
   Asp Ile Asn Thr Val Thr Thr Val Ala Gln Ser Arg
   1 5 10
<210> 2364
   <211> 12
   <212> PRT
   <213> Human coxsackievirus
<400> 2364
   Asp Arg Val Arg Ser Ile Thr Leu Gly Asn Ser Thr
   1 5 10
<210> 2365
   <211> 12
   <212> PRT
   <213> Homo sapiens
<400> 2365
   Glu Glu Ile Ala Gln Val Ala Thr Ile Ser Ala Asn
   1 5 10
<210> 2366
   <211> 12
   <212> PRT
   <213> Human coxsackievirus
<400> 2366
   Glu Thr Ser Leu Ser Ala Ala Gly Asn Ser Ile Ile
   1 5 10
<210> 2367
   <211> 12
   <212> PRT
   <213> Homo sapiens
<400> 2367
   Phe Phe Arg Met Val Ile Ser Asn Pro Ala Ala Thr
   1 5 10
<210> 2368
   <211> 12
   <212> PRT
   <213> Homo sapiens
<400> 2368
   Gly Ala Asp Ile Lys Lys Thr Met Glu Gly Pro Val
   1 5 10
<210> 2369
   <211> 12
   <212> PRT
   <213> Homo sapiens
<400> 2369
   Gly Val Glu Leu Arg Gln Leu Thr Pro Glu Gln Leu
   1 5 10
<210> 2370
   <211> 12
   <212> PRT
   <213> Homo sapiens
<400> 2370
   Lys Asp Val Lys Phe Gly Ala Asp Ala Arg Ala Leu
   1 5 10
<210> 2371
   <211> 16
   <212> PRT
   <213> Homo sapiens
<400> 2371
   Lys Glu Gln Phe Glu Phe Ala Leu Thr Ala Val Ala Glu Glu Val Cys
   1 5 10 15
<210> 2372
   <211> 12
   <212> PRT
   <213> Homo sapiens
<400> 2372
   Lys Phe Gly Ala Asp Ala Arg Ala Leu Met Leu Gln
   1 5 10
<210> 2373
   <211> 12
   <212> PRT
   <213> Homo sapiens
<400> 2373
   Lys Thr Gly His Pro Arg Tyr Phe Asn Gln Leu Ser
   1 5 10
<210> 2374
   <211> 12
   <212> PRT
   <213> Human coxsackievirus
<400> 2374
   Leu Leu Glu Ser Gln Ile Ala Thr Ile Glu Gln Thr
   1 5 10
<210> 2375
   <211> 12
   <212> PRT
   <213> Homo sapiens
<400> 2375
   Leu Leu Gln Tyr Val Val Lys Ser Phe Asp Arg Ser
   1 5 10
<210> 2376
   <211> 12
   <212> PRT
   <213> Homo sapiens
<400> 2376
   Leu Arg Leu Leu Leu Cys Leu Leu Leu Leu Ser Ser
   1 5 10
<210> 2377
   <211> 12
   <212> PRT
   <213> Homo sapiens
<400> 2377
   Leu Arg Gln Leu Thr Pro Glu Gln Leu Ser Thr Leu
   1 5 10
<210> 2378
   <211> 12
   <212> PRT
   <213> Human coxsackievirus
<400> 2378
   Leu Thr Ala Val Glu Thr Gly His Thr Ser Gln Val
   1 5 10
<210> 2379
   <211> 12
   <212> PRT
   <213> Homo sapiens
<400> 2379
   Leu Thr Arg Ala Tyr Ala Lys Asp Val Lys Phe Gly
   1 5 10
<210> 2380
   <211> 12
   <212> PRT
   <213> Homo sapiens
<400> 2380
   Pro Pro Glu Pro Arg Pro Arg Asp Arg Ser Gly Leu
   1 5 10
<210> 2381
   <211> 12
   <212> PRT
   <213> Homo sapiens
<400> 2381
   Pro Arg Pro Arg Asp Arg Ser Gly Leu Ala Pro Lys
   1 5 10
<210> 2382
   <211> 12
   <212> PRT
   <213> Human coxsackievirus
<400> 2382
   Pro Ser Asn Ser Ala Ser Val Pro Ala Leu Thr Ala
   1 5 10
<210> 2383
   <211> 12
   <212> PRT
   <213> Homo sapiens
<400> 2383
   Pro Thr Leu Ala Phe Leu Gln Asp Val Met Asn Ile
   1 5 10
<210> 2384
   <211> 23
   <212> PRT
   <213> Human coxsackievirus
<400> 2384
   Pro Val Lys Asp Val Met Ile Lys Ser Leu Pro Ala Pro Ala Leu Asn
   1 5 10 15
   Ser Pro Thr Val Glu Glu Cys
   20
<210> 2385
   <211> 12
   <212> PRT
   <213> Homo sapiens
<400> 2385
   Arg Asp Arg Ser Gly Leu Ala Pro Lys Arg Pro Gly
   1 5 10
<210> 2386
   <211> 12
   <212> PRT
   <213> Homo sapiens
<400> 2386
   Arg Leu Arg Pro Pro Glu Pro Arg Pro Arg Asp Arg
   1 5 10
<210> 2387
   <211> 12
   <212> PRT
   <213> Human coxsackievirus
<400> 2387
   Arg Ser Gly Pro Ser Asn Ser Ala Ser Val Pro Ala
   1 5 10
<210> 2388
   <211> 12
   <212> PRT
   <213> Human coxsackievirus
<400> 2388
   Arg Ser Ile Thr Leu Gly Asn Ser Thr Ile Thr Thr
   1 5 10
<210> 2389
   <211> 12
   <212> PRT
   <213> Homo sapiens
<400> 2389
   Arg Thr Ala Leu Leu Asp Ala Ala Gly Val Ala Ser
   1 5 10
<210> 2390
   <211> 12
   <212> PRT
   <213> Human coxsackievirus
<400> 2390
   Ser Ala Ser Val Pro Ala Leu Thr Ala Val Glu Thr
   1 5 10
<210> 2391
   <211> 12
   <212> PRT
   <213> Homo sapiens
<400> 2391
   Ser Gly Gly Leu Arg Leu Leu Leu Cys Leu Leu Leu
   1 5 10
<210> 2392
   <211> 12
   <212> PRT
   <213> Homo sapiens
<400> 2392
   Ser Gly Leu Ala Pro Lys Arg Pro Gly Pro Ala Gly
   1 5 10
<210> 2393
   <211> 12
   <212> PRT
   <213> Human coxsackievirus
<400> 2393
   Ser Leu Glu Lys Lys Met Ser Asn Tyr Ile Gln Phe
   1 5 10
<210> 2394
   <211> 12
   <212> PRT
   <213> Human coxsackievirus
<400> 2394
   Ser Gln Ile Ala Thr Ile Glu Gln Thr Ala Pro Ser
   1 5 10
<210> 2395
   <211> 12
   <212> PRT
   <213> Human coxsackievirus
<400> 2395
   Thr Lys Gln Met Val Gln Met Arg Arg Lys Leu Glu
   1 5 10
<210> 2396
   <211> 12
   <212> PRT
   <213> Human coxsackievirus
<400> 2396
   Thr Arg Lys Asp Ile Asn Thr Val Thr Thr Val Ala
   1 5 10
<210> 2397
   <211> 12
   <212> PRT
   <213> Human coxsackievirus
<400> 2397
   Val Glu Thr Gly His Thr Ser Gln Val Thr Pro Ser
   1 5 10
<210> 2398
   <211> 12
   <212> PRT
   <213> Homo sapiens
<400> 2398
   Val Lys Glu Lys Gly Met Ala Ala Leu Pro Arg Leu
   1 5 10
<210> 2399
   <211> 17
   <212> PRT
   <213> Human coxsackievirus
<400> 2399
   Val Met Ile Lys Ser Leu Pro Ala Leu Asn Ser Pro Thr Val Glu Glu
   1 5 10 15
   Cys
<210> 2400
   <211> 12
   <212> PRT
   <213> Human coxsackievirus
<400> 2400
   Val Pro Ala Leu Thr Ala Val Glu Thr Gly His Thr
   1 5 10
<210> 2401
   <211> 14
   <212> PRT
   <213> Mus musculus
<400> 2401
   Glu Tyr Val Thr Leu Lys Lys Met Arg Glu Ile Ile Gly Trp
   1 5 10
<210> 2402
   <211> 20
   <212> PRT
   <213> Homo sapiens
<400> 2402
   Asp Val Met Asn Ile Leu Leu Gln Tyr Val Val Lys Ser Phe Asp Arg
   1 5 10 15
   Ser Thr Lys Val
   20
<210> 2403
   <211> 20
   <212> PRT
   <213> Homo sapiens
<400> 2403
   Gly Ala Gly Ser Leu Gln Pro Leu Ala Leu Glu Gly Ser Leu Gln Lys
   1 5 10 15
   Arg Gly Ile Val
   20
<210> 2404
   <211> 20
   <212> PRT
   <213> Homo sapiens
<400> 2404
   Lys Asp Tyr Arg Ala Tyr Tyr Thr Phe Leu Asn Phe Met Ser Asn Val
   1 5 10 15
   Gly Asp Pro Arg
   20
<210> 2405
   <211> 20
   <212> PRT
   <213> Homo sapiens
<400> 2405
   Lys Val Asn Phe Phe Arg Met Val Ile Ser Asn Pro Ala Ala Thr His
   1 5 10 15
   Gln Asp Ile Asp
   20
<210> 2406
   <211> 20
   <212> PRT
   <213> Homo sapiens
<400> 2406
   Leu Ile Ala Phe Thr Ser Glu His Ser His Phe Ser Leu Lys Lys Gly
   1 5 10 15
   Ala Ala Ala Leu
   20
<210> 2407
   <211> 20
   <212> PRT
   <213> Homo sapiens
<400> 2407
   Arg Leu Gln Gln Leu Val Leu Asp Pro Gly Phe Leu Gly Leu Glu Pro
   1 5 10 15
   Leu Leu Asp Leu
   20
<210> 2408
   <211> 20
   <212> PRT
   <213> Porphyromonas gingivalis
<400> 2408
   Thr Leu Val Val Asn Arg Leu Arg Gly Ser Leu Lys Ile Cys Ala Val
   1 5 10 15
   Lys Ala Pro Gly
   20
<210> 2409
   <211> 11
   <212> PRT
   <213> Unknown
<220>
   <223> epitope involved in diabetes
<400> 2409
   Ala His His Pro Ile Trp Ala Arg Met Asp Ala
   1 5 10
<210> 2410
   <211> 14
   <212> PRT
   <213> Mus musculus
<400> 2410
   Asp Thr Lys Val Met Lys Cys Val Leu Glu Val Ile Ser Asp
   1 5 10
<210> 2411
   <211> 20
   <212> PRT
   <213> Homo sapiens
<400> 2411
   Asp Ser Val Ile Leu Ile Lys Cys Asp Glu Arg Gly Lys Met Ile Pro
   1 5 10 15
   Ser Asp Leu Glu
   20
<210> 2412
   <211> 20
   <212> PRT
   <213> Homo sapiens
<400> 2412
   His Lys Trp Lys Leu Ser Gly Val Glu Arg Ala Asn Ser Val Thr Trp
   1 5 10 15
   Asn Pro His Lys
   20
<210> 2413
   <211> 20
   <212> PRT
   <213> Homo sapiens
<400> 2413
   Lys Cys Leu Glu Leu Ala Glu Tyr Leu Tyr Asn Ile Ile Lys Asn Arg
   1 5 10 15
   Glu Gly Tyr Glu
   20
<210> 2414
   <211> 20
   <212> PRT
   <213> Homo sapiens
<400> 2414
   Lys Gly Met Ala Ala Leu Pro Arg Leu Ile Ala Phe Thr Ser Glu His
   1 5 10 15
   Ser His Phe Ser
   20
<210> 2415
   <211> 20
   <212> PRT
   <213> Homo sapiens
<400> 2415
   Arg Pro Thr Leu Ala Phe Leu Gln Asp Val Met Asn Ile Leu Leu Gln
   1 5 10 15
   Tyr Val Val Lys
   20
<210> 2416
   <211> 20
   <212> PRT
   <213> Homo sapiens
<400> 2416
   Tyr Asp Leu Ser Tyr Asp Thr Gly Asp Lys Ala Leu Gln Cys Gly Arg
   1 5 10 15
   His Val Asp Val
   20
<210> 2417
   <211> 13
   <212> PRT
   <213> Homo sapiens
<400> 2417
   Ile Pro Ala Leu Asp Ser Leu Thr Pro Ala Asn Glu Asp
   1 5 10
<210> 2418
   <211> 20
   <212> PRT
   <213> Homo sapiens
<400> 2418
   Pro Thr Ile Ala Phe Leu Gln Asp Val Met Asn Ile Leu Leu Gln Tyr
   1 5 10 15
   Val Val Lys Ser
   20
<210> 2419
   <211> 15
   <212> PRT
   <213> Unknown
<220>
   <223> epitope involved in diabetes
<400> 2419
   Ser His Leu Val Glu Ala Leu Tyr Leu Val Cys Gly Glu Glu Gly
   1 5 10 15
<210> 2420
   <211> 15
   <212> PRT
   <213> Unknown
<220>
   <223> epitope involved in diabetes
<400> 2420
   Ser His Leu Val Glu Ala Leu Tyr Leu Val Cys Gly Gly Glu Gly
   1 5 10 15
<210> 2421
   <211> 369
   <212> PRT
   <213> Homo sapiens
<400> 2421
   Met Glu Phe Leu Glu Arg Thr Tyr Leu Val Asn Asp Lys Ala Ala Lys
   1 5 10 15
   Met Tyr Ala Phe Thr Leu Glu Ser Val Glu Leu Gln Gln Lys Pro Val
   20 25 30
   Asn Lys Asp Gln Cys Pro Arg Glu Arg Pro Glu Glu Leu Glu Ser Gly
   35 40 45
   Gly Met Tyr His Cys His Ser Gly Ser Lys Pro Thr Glu Lys Gly Ala
   50 55 60
   Asn Glu Tyr Ala Tyr Ala Lys Trp Lys Leu Cys Ser Ala Ser Ala Ile
   65 70 75 80
   Cys Phe Ile Phe Met Ile Ala Glu Val Val Gly Gly His Ile Ala Gly
   85 90 95
   Ser Leu Ala Val Val Thr Asp Ala Ala His Leu Leu Ile Asp Leu Thr
   100 105 110
   Ser Phe Leu Leu Ser Leu Phe Ser Leu Trp Leu Ser Ser Lys Pro Pro
   115 120 125
   Ser Lys Arg Leu Thr Phe Gly Trp His Arg Ala Glu Ile Leu Gly Ala
   130 135 140
   Leu Leu Ser Ile Leu Cys Ile Trp Val Val Thr Gly Val Leu Val Tyr
   145 150 155 160
   Leu Ala Cys Glu Arg Leu Leu Tyr Pro Asp Tyr Gln Ile Gln Ala Thr
   165 170 175
   Val Met Ile Ile Val Ser Ser Cys Ala Val Ala Ala Asn Ile Val Leu
   180 185 190
   Thr Val Val Leu His Gln Arg Cys Leu Gly His Asn His Lys Glu Val
   195 200 205
   Gln Ala Asn Ala Ser Val Arg Ala Ala Phe Val His Ala Leu Gly Asp
       210                 215                 220
   Leu Phe Gln Ser Ile Ser Val Leu Ile Ser Ala Leu Ile Ile Tyr Phe
   225                 230                 235                 240
   Lys Pro Glu Tyr Lys Ile Ala Asp Pro Ile Cys Thr Phe Ile Phe Ser
                   245                 250                 255
   Ile Leu Val Leu Ala Ser Thr Ile Thr Ile Leu Lys Asp Phe Ser Ile
               260                 265                 270
   Leu Leu Met Glu Gly Val Pro Lys Ser Leu Asn Tyr Ser Gly Val Lys
           275                 280                 285
   Glu Leu Ile Leu Ala Val Asp Gly Val Leu Ser Val His Ser Leu His
       290                 295                 300
   Ile Trp Ser Leu Thr Met Asn Gln Val Ile Leu Ser Ala His Val Ala
   305                 310                 315                 320
   Thr Ala Ala Ser Arg Asp Ser Gln Val Val Arg Arg Glu Ile Ala Lys
                   325                 330                 335
   Ala Leu Ser Lys Ser Phe Thr Met His Ser Leu Thr Ile Gln Met Glu
               340                 345                 350
   Ser Pro Val Asp Gln Asp Pro Asp Cys Leu Phe Cys Glu Asp Pro Cys
           355                 360                 365
   Asp
<210> 2422
   <211> 18
   <212> PRT
   <213> Homo sapiens
<400> 2422
   Leu Pro Arg Leu Ile Ala Phe Thr Ser Glu His Ser His Phe Ser Leu
   1               5                   10                  15
   Lys Lys
<210> 2423
   <211> 14
   <212> PRT
   <213> Homo sapiens
<400> 2423
   Val Asn Phe Phe Arg Met Val Ile Ser Asn Pro Ala Ala Thr
   1 5 10
<210> 2424
   <211> 10
   <212> PRT
   <213> Homo sapiens
<400> 2424
   Pro Leu Ala Leu Glu Gly Ser Leu Gln Lys
   1 5 10
<210> 2425
   <211> 10
   <212> PRT
   <213> Homo sapiens
<400> 2425
   Pro Leu Leu Ala Leu Leu Ala Leu Trp Gly
   1 5 10
<210> 2426
   <211> 12
   <212> PRT
   <213> Mus musculus
<400> 2426
   Ala Ser Ser Leu Gly Gly Asn Gln Asp Thr Gln Tyr
   1 5 10
<210> 2427
   <211> 9
   <212> PRT
   <213> Homo sapiens
<400> 2427
   Ala Leu Gly Asp Leu Phe Gln Ser Ile
   1 5
<210> 2428
   <211> 10
   <212> PRT
   <213> Homo sapiens
<400> 2428
   Ala Val Ala Ala Asn Ile Val Leu Thr Val
   1 5 10
<210> 2429
   <211> 10
   <212> PRT
   <213> Homo sapiens
<400> 2429
   Phe Leu Leu Ser Leu Phe Ser Leu Trp Leu
   1 5 10
<210> 2430
   <211> 9
   <212> PRT
   <213> Homo sapiens
<400> 2430
   His Ile Ala Gly Ser Leu Ala Val Val
   1 5
<210> 2431
   <211> 10
   <212> PRT
   <213> Homo sapiens
<400> 2431
   Ile Leu Ala Val Asp Gly Val Leu Ser Val
   1 5 10
<210> 2432
   <211> 9
   <212> PRT
   <213> Homo sapiens
<400> 2432
   Ile Leu Lys Asp Phe Ser Ile Leu Leu
   1 5
<210> 2433
   <211> 10
   <212> PRT
   <213> Homo sapiens
<400> 2433
   Ile Leu Val Leu Ala Ser Thr Ile Thr Ile
   1 5 10
<210> 2434
   <211> 9
   <212> PRT
   <213> Homo sapiens
<400> 2434
   Leu Leu Ile Asp Leu Thr Ser Phe Leu
   1 5
<210> 2435
   <211> 9
   <212> PRT
   <213> Homo sapiens
<400> 2435
   Ser Ile Ser Val Leu Ile Ser Ala Leu
   1 5
<210> 2436
   <211> 9
   <212> PRT
   <213> Homo sapiens
<400> 2436
   Val Val Thr Gly Val Leu Val Tyr Leu
   1 5
<210> 2437
   <211> 9
   <212> PRT
   <213> Homo sapiens
<400> 2437
   Phe Ile Phe Ser Ile Leu Val Leu Ala
   1 5
<210> 2438
   <211> 9
   <212> PRT
   <213> Homo sapiens
<400> 2438
   Ile Gln Ala Thr Val Met Ile Ile Val
   1 5
<210> 2439
   <211> 9
   <212> PRT
   <213> Homo sapiens
<400> 2439
   Lys Met Tyr Ala Phe Thr Leu Glu Ser
   1 5
<210> 2440
   <211> 9
   <212> PRT
   <213> Homo sapiens
<400> 2440
   Lys Ser Leu Asn Tyr Ser Gly Val Lys
   1 5
<210> 2441
   <211> 9
   <212> PRT
   <213> Homo sapiens
<400> 2441
   Leu Leu Ser Ile Leu Cys Ile Trp Val
   1               5
<210> 2442
   <211> 9
   <212> PRT
   <213> Homo sapiens
<400> 2442
   Leu Leu Ser Leu Phe Ser Leu Trp Leu
   1               5
<210> 2443
   <211> 9
   <212> PRT
   <213> Homo sapiens
<400> 2443
   Met Ile Ile Val Ser Ser Cys Ala Val
   1               5
<210> 2444
   <211> 9
   <212> PRT
   <213> Homo sapiens
<400> 2444
   Arg Leu Leu Tyr Pro Asp Tyr Gln Ile
   1               5
<210> 2445
   <211> 9
   <212> PRT
   <213> Homo sapiens
<400> 2445
   Thr Met His Ser Leu Thr Ile Gln Met
   1               5
<210> 2446
   <211> 9
   <212> PRT
   <213> Homo sapiens
<400> 2446
   Val Ala Ala Asn Ile Val Leu Thr Val
   1 5
<210> 2447
   <211> 27
   <212> PRT
   <213> Homo sapiens
<400> 2447
   Ala Cys Glu Arg Leu Leu Tyr Pro Asp Tyr Gln Ile Gln Ala Thr Val
   1 5 10 15
   Met Ile Ile Val Ser Ser Cys Ala Val Ala Ala
   20 25
<210> 2448
   <211> 27
   <212> PRT
   <213> Homo sapiens
<400> 2448
   Ala Lys Met His Ala Phe Thr Leu Glu Ser Val Glu Leu Gln Gln Lys
   1 5 10 15
   Pro Val Asn Lys Asp Gln Cys Pro Arg Glu Arg
   20 25
<210> 2449
   <211> 27
   <212> PRT
   <213> Homo sapiens
<400> 2449
   Ala Asn Glu Tyr Ala Tyr Ala Lys Trp Lys Leu Cys Ser Ala Ser Ala
   1 5 10 15
   Ile Cys Phe Ile Phe Met Ile Ala Glu Val Val
   20 25
<210> 2450
   <211> 34
   <212> PRT
   <213> Homo sapiens
<400> 2450
   Ala Ser Arg Asp Ser Gln Val Val Arg Arg Glu Ile Ala Lys Ala Leu
   1 5 10 15
   Ser Lys Ser Phe Thr Met His Ser Leu Thr Ile Gln Met Glu Ser Pro
               20                  25                  30
   Val Asp
<210> 2451
   <211> 27
   <212> PRT
   <213> Homo sapiens
<400> 2451
   Asp Gly Val Leu Ser Val His Ser Leu His Ile Trp Ser Leu Thr Met
   1               5                   10                  15
   Asn Gln Val Ile Leu Ser Ala His Val Ala Thr
               20                  25
<210> 2452
   <211> 27
   <212> PRT
   <213> Homo sapiens
<400> 2452
   Glu Glu Leu Glu Ser Gly Gly Met Tyr His Cys His Ser Gly Ser Lys
   1               5                   10                  15
   Pro Thr Glu Lys Gly Ala Asn Glu Tyr Ala Tyr
               20                  25
<210> 2453
   <211> 41
   <212> PRT
   <213> Homo sapiens
<400> 2453
   Phe Gly Trp His Arg Ala Glu Ile Leu Gly Ala Leu Leu Ser Ile Leu
   1               5                   10                  15
   Cys Ile Trp Val Val Thr Gly Val Leu Val Tyr Leu Ala Cys Glu Arg
               20                  25                  30
   Leu Leu Tyr Pro Asp Tyr Gln Ile Gln
           35                  40
<210> 2454
   <211> 27
   <212> PRT
   <213> Homo sapiens
<400> 2454
   Phe Ile Phe Ser Ile Leu Val Leu Ala Ser Thr Ile Thr Ile Leu Lys
   1               5                   10                  15
   Asp Phe Ser Ile Leu Leu Met Glu Gly Val Pro
               20                  25
<210> 2455
   <211> 27
   <212> PRT
   <213> Homo sapiens
<400> 2455
   Gly His Ile Ala Gly Ser Leu Ala Val Val Thr Asp Ala Ala His Leu
   1               5                   10                  15
   Leu Ile Asp Leu Thr Ser Phe Leu Leu Ser Leu
               20                  25
<210> 2456
   <211> 27
   <212> PRT
   <213> Homo sapiens
<400> 2456
   His Leu Leu Ile Asp Leu Thr Ser Phe Leu Leu Ser Leu Phe Ser Leu
   1               5                   10                  15
   Trp Leu Ser Ser Lys Pro Pro Ser Lys Arg Leu
               20                  25
<210> 2457
   <211> 27
   <212> PRT
   <213> Homo sapiens
<400> 2457
   His Gln Arg Cys Leu Gly His Asn His Lys Glu Val Gln Ala Asn Ala
   1               5                   10                  15
   Ser Val Arg Ala Ala Phe Val His Ala Leu Gly
               20                  25
<210> 2458
   <211> 27
   <212> PRT
   <213> Homo sapiens
<400> 2458
   Leu Phe Gln Ser Ile Ser Val Leu Ile Ser Ala Leu Ile Ile Tyr Phe
   1 5 10 15
   Lys Pro Glu Tyr Lys Ile Ala Asp Pro Ile Cys
   20 25
<210> 2459
   <211> 27
   <212> PRT
   <213> Homo sapiens
<400> 2459
   Leu Lys Asp Phe Ser Ile Leu Leu Met Glu Gly Val Pro Lys Ser Leu
   1 5 10 15
   Asn Tyr Ser Gly Val Lys Glu Leu Ile Leu Ala
   20 25
<210> 2460
   <211> 27
   <212> PRT
   <213> Homo sapiens
<400> 2460
   Met Glu Phe Leu Glu Arg Thr Tyr Leu Val Asn Asp Lys Ala Ala Lys
   1 5 10 15
   Met His Ala Phe Thr Leu Glu Ser Val Glu Leu
   20 25
<210> 2461
   <211> 26
   <212> PRT
   <213> Homo sapiens
<400> 2461
   Met His Ser Leu Thr Ile Gln Met Glu Ser Pro Val Asp Gln Asp Pro
   1 5 10 15
   Asp Cys Leu Phe Cys Glu Asp Pro Cys Asp
   20 25
<210> 2462
   <211> 27
   <212> PRT
   <213> Homo sapiens
<400> 2462
   Asn Ala Ser Val Arg Ala Ala Phe Val His Ala Leu Gly Asp Leu Phe
   1 5 10 15
   Gln Ser Ile Ser Val Leu Ile Ser Ala Leu Ile
   20 25
<210> 2463
   <211> 27
   <212> PRT
   <213> Homo sapiens
<400> 2463
   Gln Lys Pro Val Asn Lys Asp Gln Cys Pro Arg Glu Arg Pro Glu Glu
   1 5 10 15
   Leu Glu Ser Gly Gly Met Tyr His Cys His Ser
   20 25
<210> 2464
   <211> 27
   <212> PRT
   <213> Homo sapiens
<400> 2464
   Ser Ala Ile Cys Phe Ile Phe Met Ile Ala Glu Val Val Gly Gly His
   1 5 10 15
   Ile Ala Gly Ser Leu Ala Val Val Thr Asp Ala
   20 25
<210> 2465
   <211> 27
   <212> PRT
   <213> Homo sapiens
<400> 2465
   Ser Cys Ala Val Ala Ala Asn Ile Val Leu Thr Val Val Leu His Gln
   1 5 10 15
   Arg Cys Leu Gly His Asn His Lys Glu Val Gln
   20 25
<210> 2466
   <211> 27
   <212> PRT
   <213> Homo sapiens
<400> 2466
   Ser Leu Asn Tyr Ser Gly Val Lys Glu Leu Ile Leu Ala Val Asp Gly
   1 5 10 15
   Val Leu Ser Val His Ser Leu His Ile Trp Ser
   20 25
<210> 2467
   <211> 27
   <212> PRT
   <213> Homo sapiens
<400> 2467
   Ser Leu Trp Leu Ser Ser Lys Pro Pro Ser Lys Arg Leu Thr Phe Gly
   1               5                   10                  15
   Trp His Arg Ala Glu Ile Leu Gly Ala Leu Leu
               20                  25
<210> 2468
   <211> 27
   <212> PRT
   <213> Homo sapiens
<400> 2468
   Thr Met Asn Gln Val Ile Leu Ser Ala His Val Ala Thr Ala Ala Ser
   1               5                   10                  15
   Arg Asp Ser Gln Val Val Arg Arg Glu Ile Ala
               20                  25
<210> 2469
   <211> 27
   <212> PRT
   <213> Homo sapiens
<400> 2469
   Tyr Phe Lys Pro Glu Tyr Lys Ile Ala Asp Pro Ile Cys Thr Phe Ile
   1               5                   10                  15
   Phe Ser Ile Leu Val Leu Ala Ser Thr Ile Thr
               20                  25
<210> 2470
   <211> 9
   <212> PRT
   <213> Mus musculus
<400> 2470
   Ala Ile Asn Ser Glu Met Phe Leu Arg
   1               5
<210> 2471
   <211> 10
   <212> PRT
   <213> Mus musculus
<400> 2471
   Leu Ala Leu Glu Val Ala Arg Gln Lys Arg
   1 5 10
<210> 2472
   <211> 10
   <212> PRT
   <213> Mus musculus
<400> 2472
   Thr Leu Ala Leu Glu Val Ala Gln Gln Lys
   1 5 10
<210> 2473
   <211> 10
   <212> PRT
   <213> Mus musculus
<400> 2473
   Thr Leu Ala Leu Glu Val Ala Arg Gln Lys
   1 5 10
<210> 2474
   <211> 20
   <212> PRT
   <213> Mycobacterium tuberculosis
<400> 2474
   Thr Leu Val Val Asn Lys Ile Arg Gly Thr Phe Lys Ser Val Ala Val
   1 5 10 15
   Lys Ala Pro Gly
   20
<210> 2475
   <211> 20
   <212> PRT
   <213> Chlamydia pneumoniae
<400> 2475
   Thr Leu Val Val Asn Lys Leu Gln Gly Leu Leu Gln Val Thr Val Val
   1 5 10 15
   Thr Ile Pro Gln
   20
<210> 2476
   <211> 14
   <212> PRT
   <213> Homo sapiens
<400> 2476
   Leu Thr Val Gly Leu Thr Arg Arg Gly Gln Pro Arg Gln Tyr
   1 5 10
<210> 2477
   <211> 13
   <212> PRT
   <213> Homo sapiens
<400> 2477
   Tyr Asn Gln Tyr Ser Gln Arg Tyr His Gln Arg Thr Asn
   1 5 10
<210> 2478
   <211> 9
   <212> PRT
   <213> Homo sapiens
<400> 2478
   Ala Cys Asp Gly Glu Arg Pro Thr Leu
   1 5
<210> 2479
   <211> 9
   <212> PRT
   <213> Homo sapiens
<400> 2479
   Ala His Val Asp Lys Cys Leu Glu Leu
   1 5
<210> 2480
   <211> 10
   <212> PRT
   <213> Unknown
<220>
   <223> epitope involved in diabetes
<400> 2480
   Ala Leu Trp Gly Pro Asp Pro Ala Ala Val
   1 5 10
<210> 2481
   <211> 9
   <212> PRT
   <213> Homo sapiens
<400> 2481
   Ala Pro Val Ile Lys Ala Arg Met Met
   1 5
<210> 2482
   <211> 10
   <212> PRT
   <213> Unknown
<220>
   <223> epitope involved in diabetes
<400> 2482
   Ala Gln Trp Gly Pro Asp Pro Ala Ala Val
   1 5 10
<210> 2483
   <211> 10
   <212> PRT
   <213> Homo sapiens
<400> 2483
   His Pro Arg Tyr Phe Asn Gln Leu Ser Thr
   1 5 10
<210> 2484
   <211> 10
   <212> PRT
   <213> Homo sapiens
<400> 2484
   Ile Pro Ser Asp Leu Glu Arg Arg Ile Leu
   1 5 10
<210> 2485
   <211> 10
   <212> PRT
   <213> Unknown
<220>
   <223> epitope involved in diabetes
<400> 2485
   Arg Gln Trp Gly Pro Asp Pro Ala Ala Val
   1 5 10
<210> 2486
   <211> 9
   <212> PRT
   <213> Homo sapiens
<400> 2486
   Ser Pro Gly Ser Gly Phe Trp Ser Phe
   1 5
<210> 2487
   <211> 1015
   <212> PRT
   <213> Homo sapiens
<400> 2487
   Met Gly Pro Pro Leu Pro Leu Leu Leu Leu Leu Leu Leu Leu Leu Pro
   1               5                   10                  15
   Pro Arg Val Leu Pro Ala Ala Pro Ser Ser Val Pro Arg Gly Arg Gln
               20                  25                  30
   Leu Pro Gly Arg Leu Gly Cys Leu Leu Glu Glu Gly Leu Cys Gly Ala
           35                  40                  45
   Ser Glu Ala Cys Val Asn Asp Gly Val Phe Gly Arg Cys Gln Lys Val
       50                  55                  60
   Pro Ala Met Asp Phe Tyr Arg Tyr Glu Val Ser Pro Val Ala Leu Gln
   65                  70                  75                  80
   Arg Leu Arg Val Ala Leu Gln Lys Leu Ser Gly Thr Gly Phe Thr Trp
                   85                  90                  95
   Gln Asp Asp Tyr Thr Gln Tyr Val Met Asp Gln Glu Leu Ala Asp Leu
               100                 105                 110
   Pro Lys Thr Tyr Leu Arg Arg Pro Glu Ala Ser Ser Pro Ala Arg Pro
           115                 120                 125
   Ser Lys His Ser Val Gly Ser Glu Arg Arg Tyr Ser Arg Glu Gly Gly
       130                 135                 140
   Ala Ala Leu Ala Asn Ala Leu Arg Arg His Leu Pro Phe Leu Glu Ala
   145                 150                 155                 160
   Leu Ser Gln Ala Pro Ala Ser Asp Val Leu Ala Arg Thr His Thr Ala
                   165                 170                 175
   Gln Asp Arg Pro Pro Ala Glu Gly Asp Asp Arg Phe Ser Glu Ser Ile
               180                 185                 190
   Leu Thr Tyr Val Ala His Thr Ser Ala Leu Thr Tyr Pro Pro Gly Ser
           195                 200                 205
   Arg Thr Gln Leu Arg Glu Asp Leu Leu Pro Arg Thr Leu Gly Gln Leu
       210                 215                 220
   Gln Pro Asp Glu Leu Ser Pro Lys Val Asp Ser Gly Val Asp Arg His
   225                 230                 235                 240
   His Leu Met Ala Ala Leu Ser Ala Tyr Ala Ala Gln Arg Pro Pro Ala
   245 250 255
   Pro Pro Gly Glu Gly Ser Leu Glu Pro Gln Tyr Leu Leu Arg Ala Pro
   260 265 270
   Ser Arg Met Pro Arg Pro Leu Leu Ala Pro Ala Ala Pro Gln Lys Trp
   275 280 285
   Pro Ser Pro Leu Gly Asp Ser Glu Asp Pro Ser Ser Thr Gly Asp Gly
   290 295 300
   Ala Arg Ile His Thr Leu Leu Lys Asp Leu Gln Arg Gln Pro Ala Glu
   305 310 315 320
   Val Arg Gly Leu Ser Gly Leu Glu Leu Asp Gly Met Ala Glu Leu Met
   325 330 335
   Ala Gly Leu Met Gln Gly Val Asp His Gly Val Ala Arg Gly Ser Pro
   340 345 350
   Gly Arg Ala Ala Leu Gly Glu Ser Gly Glu Gln Ala Asp Gly Pro Lys
   355 360 365
   Ala Thr Leu Arg Gly Asp Ser Phe Pro Asp Asp Gly Val Gln Asp Asp
   370 375 380
   Asp Asp Arg Leu Tyr Gln Glu Val His Arg Leu Ser Ala Thr Leu Gly
   385 390 395 400
   Gly Leu Leu Gln Asp His Gly Ser Arg Leu Leu Pro Gly Ala Leu Pro
   405 410 415
   Phe Ala Arg Pro Leu Asp Met Glu Arg Lys Lys Ser Glu His Pro Glu
   420 425 430
   Ser Ser Leu Ser Ser Glu Glu Glu Thr Ala Gly Val Glu Asn Val Lys
   435 440 445
   Ser Gln Thr Tyr Ser Lys Asp Leu Leu Gly Gln Gln Pro His Ser Glu
   450 455 460
   Pro Gly Ala Ala Ala Phe Gly Glu Leu Gln Asn Gln Met Pro Gly Pro
   465 470 475 480
   Ser Lys Glu Glu Gln Ser Leu Pro Ala Gly Ala Gln Glu Ala Leu Ser
                   485                 490                 495
   Asp Gly Leu Gln Leu Glu Val Gln Pro Ser Glu Glu Glu Ala Arg Gly
               500                 505                 510
   Tyr Ile Val Thr Asp Arg Asp Pro Leu Arg Pro Glu Glu Gly Arg Arg
           515                 520                 525
   Leu Val Glu Asp Val Ala Arg Leu Leu Gln Val Pro Ser Ser Ala Phe
       530                 535                 540
   Ala Asp Val Glu Val Leu Gly Pro Ala Val Thr Phe Lys Val Ser Ala
   545                 550                 555                 560
   Asn Val Gln Asn Val Thr Thr Glu Asp Val Glu Lys Ala Thr Val Asp
                   565                 570                 575
   Asn Lys Asp Lys Leu Glu Glu Thr Ser Gly Leu Lys Ile Leu Gln Thr
               580                 585                 590
   Gly Val Gly Ser Lys Ser Lys Leu Lys Phe Leu Pro Pro Gln Ala Glu
           595                 600                 605
   Gln Glu Asp Ser Thr Lys Phe Ile Ala Leu Thr Leu Val Ser Leu Ala
       610                 615                 620
   Cys Ile Leu Gly Val Leu Leu Ala Ser Gly Leu Ile Tyr Cys Leu Arg
   625                 630                 635                 640
   His Ser Ser Gln His Arg Leu Lys Glu Lys Leu Ser Gly Leu Gly Gly
                   645                 650                 655
   Asp Pro Gly Ala Asp Ala Thr Ala Ala Tyr Gln Glu Leu Cys Arg Gln
               660                 665                 670
   Arg Met Ala Thr Arg Pro Pro Asp Arg Pro Glu Gly Pro His Thr Ser
           675                 680                 685
   Arg Ile Ser Ser Val Ser Ser Gln Phe Ser Asp Gly Pro Ile Pro Ser
       690                 695                 700
   Pro Ser Ala Arg Ser Ser Ala Ser Ser Trp Ser Glu Glu Pro Val Gln
   705                 710                 715                 720
   Ser Asn Met Asp Ile Ser Thr Gly His Met Ile Leu Ser Tyr Met Glu
                   725                 730                 735
   Asp His Leu Lys Asn Lys Asn Arg Leu Glu Lys Glu Trp Glu Ala Leu
               740                 745                 750
   Cys Ala Tyr Gln Ala Glu Pro Asn Ser Ser Phe Val Ala Gln Arg Glu
           755                 760                 765
   Glu Asn Val Pro Lys Asn Arg Ser Leu Ala Val Leu Thr Tyr Asp His
       770                 775                 780
   Ser Arg Val Leu Leu Lys Ala Glu Asn Ser His Ser His Ser Asp Tyr
   785                 790                 795                 800
   Ile Asn Ala Ser Pro Ile Met Asp His Asp Pro Arg Asn Pro Ala Tyr
                   805                 810                 815
   Ile Ala Thr Gln Gly Pro Leu Pro Ala Thr Val Ala Asp Phe Trp Gln
               820                 825                 830
   Met Val Trp Glu Ser Gly Cys Val Val Ile Val Met Leu Thr Pro Leu
           835                 840                 845
   Ala Glu Asn Gly Val Arg Gln Cys Tyr His Tyr Trp Pro Asp Glu Gly
       850                 855                 860
   Ser Asn Leu Tyr His Ile Tyr Glu Val Asn Leu Val Ser Glu His Ile
   865                 870                 875                 880
   Trp Cys Glu Asp Phe Leu Val Arg Ser Phe Tyr Leu Lys Asn Leu Gln
                   885                 890                 895
   Thr Asn Glu Thr Arg Thr Val Thr Gln Phe His Phe Leu Ser Trp Tyr
               900                 905                 910
   Asp Arg Gly Val Pro Ser Ser Ser Arg Ser Leu Leu Asp Phe Arg Arg
           915                 920                 925
   Lys Val Asn Lys Cys Tyr Arg Gly Arg Ser Cys Pro Ile Ile Val His
       930                 935                 940
   Cys Ser Asp Gly Ala Gly Arg Ser Gly Thr Tyr Val Leu Ile Asp Met
   945                 950                 955                 960
   Val Leu Asn Lys Met Ala Lys Gly Ala Lys Glu Ile Asp Ile Ala Ala
                   965                 970                 975
   Thr Leu Glu His Leu Arg Asp Gln Arg Pro Gly Met Val Gln Thr Lys
               980                 985                 990
   Glu Gln Phe Glu Phe Ala Leu Thr Ala Val Ala Glu Glu Val Asn Ala
   995 1000 1005
   Ile Leu Lys Ala Leu Pro Gln
   1010 1015
<210> 2488
   <211> 979
   <212> PRT
   <213> Homo sapiens
<400> 2488
   Met Arg Arg Pro Arg Arg Pro Gly Gly Leu Gly Gly Ser Gly Gly Leu
   1 5 10 15
   Arg Leu Leu Leu Cys Leu Leu Leu Leu Ser Ser Arg Pro Gly Gly Cys
   20 25 30
   Ser Ala Val Ser Ala His Gly Cys Leu Phe Asp Arg Arg Leu Cys Ser
   35 40 45
   His Leu Glu Val Cys Ile Gln Asp Gly Leu Phe Gly Gln Cys Gln Val
   50 55 60
   Gly Val Gly Gln Ala Arg Pro Leu Leu Gln Val Thr Ser Pro Val Leu
   65 70 75 80
   Gln Arg Leu Gln Gly Val Leu Arg Gln Leu Met Ser Gln Gly Leu Ser
   85 90 95
   Trp His Asp Asp Leu Thr Gln Tyr Val Ile Ser Gln Glu Met Glu Arg
   100 105 110
   Ile Pro Arg Leu Arg Pro Pro Glu Pro Arg Pro Arg Asp Arg Ser Gly
   115 120 125
   Leu Ala Pro Lys Arg Pro Gly Pro Ala Gly Glu Leu Leu Leu Gln Asp
   130 135 140
   Ile Pro Thr Gly Ser Ala Pro Ala Ala Gln His Arg Leu Pro Gln Pro
   145 150 155 160
   Pro Val Gly Lys Gly Gly Ala Gly Ala Ser Ser Ser Leu Ser Pro Leu
   165 170 175
   Gln Ala Glu Leu Leu Pro Pro Leu Leu Glu His Leu Leu Leu Pro Pro
   180 185 190
   Gln Pro Pro His Pro Ser Leu Ser Tyr Glu Pro Ala Leu Leu Gln Pro
   195 200 205
   Tyr Leu Phe His Gln Phe Gly Ser Arg Asp Gly Ser Arg Val Ser Glu
   210 215 220
   Gly Ser Pro Gly Met Val Ser Val Gly Pro Leu Pro Lys Ala Glu Ala
   225 230 235 240
   Pro Ala Leu Phe Ser Arg Thr Ala Ser Lys Gly Ile Phe Gly Asp His
   245 250 255
   Pro Gly His Ser Tyr Gly Asp Leu Pro Gly Pro Ser Pro Ala Gln Leu
   260 265 270
   Phe Gln Asp Ser Gly Leu Leu Tyr Leu Ala Gln Glu Leu Pro Ala Pro
   275 280 285
   Ser Arg Ala Arg Val Pro Arg Leu Pro Glu Gln Gly Ser Ser Ser Arg
   290 295 300
   Ala Glu Asp Ser Pro Glu Gly Tyr Glu Lys Glu Gly Leu Gly Asp Arg
   305 310 315 320
   Gly Glu Lys Pro Ala Ser Pro Ala Val Gln Pro Asp Ala Ala Leu Gln
   325 330 335
   Arg Leu Ala Ala Val Leu Ala Gly Tyr Gly Val Glu Leu Arg Gln Leu
   340 345 350
   Thr Pro Glu Gln Leu Ser Thr Leu Leu Thr Leu Leu Gln Leu Leu Pro
   355 360 365
   Lys Gly Ala Gly Arg Asn Pro Gly Gly Val Val Asn Val Gly Ala Asp
   370 375 380
   Ile Lys Lys Thr Met Glu Gly Pro Val Glu Gly Arg Asp Thr Ala Glu
   385 390 395 400
   Leu Pro Ala Arg Thr Ser Pro Met Pro Gly His Pro Thr Ala Ser Pro
   405 410 415
   Thr Ser Ser Glu Val Gln Gln Val Pro Ser Pro Val Ser Ser Glu Pro
   420 425 430
   Pro Lys Ala Ala Arg Pro Pro Val Thr Pro Val Leu Leu Glu Lys Lys
   435 440 445
   Ser Pro Leu Gly Gln Ser Gln Pro Thr Val Ala Gly Gln Pro Ser Ala
   450 455 460
   Arg Pro Ala Ala Glu Glu Tyr Gly Tyr Ile Val Thr Asp Gln Lys Pro
   465 470 475 480
   Leu Ser Leu Ala Ala Gly Val Lys Leu Leu Glu Ile Leu Ala Glu His
   485 490 495
   Val His Met Ser Ser Gly Ser Phe Ile Asn Ile Ser Val Val Gly Pro
   500 505 510
   Ala Leu Thr Phe Arg Ile Arg His Asn Glu Gln Asn Leu Ser Leu Ala
   515 520 525
   Asp Val Thr Gln Gln Ala Gly Leu Val Lys Ser Glu Leu Glu Ala Gln
   530 535 540
   Thr Gly Leu Gln Ile Leu Gln Thr Gly Val Gly Gln Arg Glu Glu Ala
   545 550 555 560
   Ala Ala Val Leu Pro Gln Thr Ala His Ser Thr Ser Pro Met Arg Ser
   565 570 575
   Val Leu Leu Thr Leu Val Ala Leu Ala Gly Val Ala Gly Leu Leu Val
   580 585 590
   Ala Leu Ala Val Ala Leu Cys Val Arg Gln His Ala Arg Gln Gln Asp
   595 600 605
   Lys Glu Arg Leu Ala Ala Leu Gly Pro Glu Gly Ala His Gly Asp Thr
   610 615 620
   Thr Phe Glu Tyr Gln Asp Leu Cys Arg Gln His Met Ala Thr Lys Ser
   625 630 635 640
   Leu Phe Asn Arg Ala Glu Gly Pro Pro Glu Pro Ser Arg Val Ser Ser
   645 650 655
   Val Ser Ser Gln Phe Ser Asp Ala Ala Gln Ala Ser Pro Ser Ser His
   660 665 670
   Ser Ser Thr Pro Ser Trp Cys Glu Glu Pro Ala Gln Ala Asn Met Asp
   675 680 685
   Ile Ser Thr Gly His Met Ile Leu Ala Tyr Met Glu Asp His Leu Arg
       690                 695                 700
   Asn Arg Asp Arg Leu Ala Lys Glu Trp Gln Ala Leu Cys Ala Tyr Gln
   705                 710                 715                 720
   Ala Glu Pro Asn Thr Cys Ala Thr Ala Gln Gly Glu Gly Asn Ile Lys
                   725                 730                 735
   Lys Asn Arg His Pro Asp Phe Leu Pro Tyr Asp His Ala Arg Ile Lys
               740                 745                 750
   Leu Lys Val Glu Ser Ser Pro Ser Arg Ser Asp Tyr Ile Asn Ala Ser
           755                 760                 765
   Pro Ile Ile Glu His Asp Pro Arg Met Pro Ala Tyr Ile Ala Thr Gln
       770                 775                 780
   Gly Pro Leu Ser His Thr Ile Ala Asp Phe Trp Gln Met Val Trp Glu
   785                 790                 795                 800
   Ser Gly Cys Thr Val Ile Val Met Leu Thr Pro Leu Val Glu Asp Gly
                   805                 810                 815
   Val Lys Gln Cys Asp Arg Tyr Trp Pro Asp Glu Gly Ala Ser Leu Tyr
               820                 825                 830
   His Val Tyr Glu Val Asn Leu Val Ser Glu His Ile Trp Cys Glu Asp
           835                 840                 845
   Phe Leu Val Arg Ser Phe Tyr Leu Lys Asn Val Gln Thr Gln Glu Thr
       850                 855                 860
   Arg Thr Leu Thr Gln Phe His Phe Leu Ser Trp Pro Ala Glu Gly Thr
   865                 870                 875                 880
   Pro Ala Ser Thr Arg Pro Leu Leu Asp Phe Arg Arg Lys Val Asn Lys
                   885                 890                 895
   Cys Tyr Arg Gly Arg Ser Cys Pro Ile Ile Val His Cys Ser Asp Gly
               900                 905                 910
   Ala Gly Arg Thr Gly Thr Tyr Ile Leu Ile Asp Met Val Leu Asn Arg
           915                 920                 925
   Met Ala Lys Gly Val Lys Glu Ile Asp Ile Ala Ala Thr Leu Glu His
       930                 935                 940
   Val Arg Asp Gln Arg Pro Gly Leu Val Arg Ser Lys Asp Gln Phe Glu
   945 950 955 960
   Phe Ala Leu Thr Ala Val Ala Glu Glu Val Asn Ala Ile Leu Lys Ala
   965 970 975
   Leu Pro Gln
<210> 2489
   <211> 11
   <212> PRT
   <213> Homo sapiens
<400> 2489
   Glu Arg Leu Gly Pro Leu Val Glu Gln Gly Arg
   1 5 10
<210> 2490
   <211> 20
   <212> PRT
   <213> Mus musculus
<400> 2490
   Lys Cys Asn Thr Ala Thr Cys Ala Thr Gln Arg Ala Leu Asn Phe Leu
   1 5 10 15
   Val Arg Ser Ser
   20
<210> 2491
   <211> 18
   <212> PRT
   <213> Mus musculus
<400> 2491
   Phe Ala Ile Asn Ser Glu Met Phe Leu Arg Ser Cys Gln Gly Glu Asn
   1 5 10 15
   Gly Thr
<210> 2492
   <211> 18
   <212> PRT
   <213> Mus musculus
<400> 2492
   Phe Ile Lys Ile Pro Thr His Ala Glu Pro Leu Phe Tyr Leu Leu Ser
   1 5 10 15
   Phe Cys
<210> 2493
   <211> 18
   <212> PRT
   <213> Mus musculus
<400> 2493
   Leu Thr Thr Met Gln Leu Tyr Arg Phe Ile Lys Ile Pro Thr His Ala
   1               5                   10                  15
   Glu Pro
<210> 2494
   <211> 18
   <212> PRT
   <213> Mus musculus
<400> 2494
   Thr Lys Met Ile Trp Val Ala Val Ile Gly Asp Trp Phe Asn Leu Ile
   1               5                   10                  15
   Phe Lys
<210> 2495
   <211> 7
   <212> PRT
   <213> Mycobacterium avium
<400> 2495
   Gly Asp Ser Ala Arg Val Leu
   1               5
<210> 2496
   <211> 7
   <212> PRT
   <213> Mycobacterium avium
<400> 2496
   His Ala Thr Val Gln Ile Asp
   1               5
<210> 2497
   <211> 7
   <212> PRT
   <213> Mycobacterium avium
<400> 2497
   His Leu Ile Ser Thr Gly Asp
   1               5
<210> 2498
   <211> 9
   <212> PRT
   <213> Mycobacterium avium
<400> 2498
   Leu Ala Ala Asn Phe Val Val Ala Leu
   1 5
<210> 2499
   <211> 7
   <212> PRT
   <213> Mycobacterium avium
<400> 2499
   Leu Ser Pro Gly Lys Asp Met
   1 5
<210> 2500
   <211> 9
   <212> PRT
   <213> Mycobacterium avium
<400> 2500
   Met Ile Ala Val Ala Leu Ala Gly Leu
   1 5
<210> 2501
   <211> 15
   <212> PRT
   <213> Homo sapiens
<400> 2501
   Glu Arg Thr Tyr Leu Val Asn Asp Lys Ala Ala Lys Met His Ala
   1 5 10 15
<210> 2502
   <211> 15
   <212> PRT
   <213> Homo sapiens
<400> 2502
   Ile Phe Ser Ile Leu Val Leu Ala Ser Thr Ile Thr Ile Leu Lys
   1 5 10 15
<210> 2503
   <211> 23
   <212> PRT
   <213> Homo sapiens
<400> 2503
   Lys Glu Arg Leu Ala Ala Leu Gly Pro Glu Gly Ala His Gly Asp Thr
   1 5 10 15
   Thr Phe Glu Tyr Gln Asp Leu
   20
<210> 2504
   <211> 14
   <212> PRT
   <213> Homo sapiens
<400> 2504
   Tyr Ala Tyr Ala Lys Trp Lys Leu Cys Ser Ala Ser Ala Ile
   1 5 10
<210> 2505
   <211> 15
   <212> PRT
   <213> Homo sapiens
<400> 2505
   Tyr Lys Ile Ala Asp Pro Ile Cys Thr Phe Ile Phe Ser Ile Leu
   1 5 10 15
<210> 2506
   <211> 20
   <212> PRT
   <213> Mycobacterium tuberculosis
<400> 2506
   Ala Ala Ser Gly Pro Lys Val Val Ile Asp Gly Lys Asp Gln Asn Val
   1 5 10 15
   Thr Gly Ser Val
   20
<210> 2507
   <211> 20
   <212> PRT
   <213> Human herpesvirus
<400> 2507
   Ala Gly Ala Gly Gly Gly Ala Gly Gly Ala Gly Ala Gly Gly Gly Ala
   1 5 10 15
   Gly Gly Ala Gly
   20
<210> 2508
   <211> 21
   <212> PRT
   <213> Human herpesvirus
<400> 2508
   Ala Gly Ala Gly Gly Gly Ala Gly Gly Ala Gly Ala Gly Gly Gly Ala
   1               5                   10                  15
   Gly Gly Ala Gly Ala
               20
<210> 2509
   <211> 15
   <212> PRT
   <213> Mycobacterium tuberculosis
<400> 2509
   Ala Gly Lys Pro Leu Leu Ile Ile Ala Glu Asp Val Glu Gly Glu
   1               5                   10                  15
<210> 2510
   <211> 13
   <212> PRT
   <213> Homo sapiens
<400> 2510
   Ala Gly Trp Leu Ala Asp Arg Ser Val Arg Tyr Pro Ile
   1               5                   10
<210> 2511
   <211> 10
   <212> PRT
   <213> Mycobacterium tuberculosis
<400> 2511
   Ala Leu Ser Thr Leu Val Val Asn Lys Ile
   1               5                   10
<210> 2512
   <211> 15
   <212> PRT
   <213> Mycobacterium tuberculosis
<400> 2512
   Ala Leu Ser Thr Leu Val Val Asn Lys Ile Arg Gly Thr Phe Lys
   1               5                   10                  15
<210> 2513
   <211> 20
   <212> PRT
   <213> Mycobacterium tuberculosis
<400> 2513
   Ala Ser Ala Thr Lys Asp Gly Ser His Tyr Lys Ile Thr Gly Thr Ala
   1               5                   10                  15
   Thr Gly Val Asp
               20
<210> 2514
   <211> 20
   <212> PRT
   <213> Mycobacterium tuberculosis
<400> 2514
   Ala Thr Gly Val Asp Met Ala Asn Pro Met Ser Pro Val Asn Lys Ser
   1               5                   10                  15
   Phe Glu Ile Glu
               20
<210> 2515
   <211> 10
   <212> PRT
   <213> Human herpesvirus
<400> 2515
   Ala Val Phe Asp Arg Lys Ser Asp Ala Lys
   1               5                   10
<210> 2516
   <211> 9
   <212> PRT
   <213> Human herpesvirus
<400> 2516
   Ala Tyr Arg Arg Arg Trp Arg Arg Leu
   1               5
<210> 2517
   <211> 9
   <212> PRT
   <213> Human herpesvirus
<400> 2517
   Cys Leu Gly Gly Leu Leu Thr Met Val
   1               5
<210> 2518
   <211> 19
   <212> PRT
   <213> Mycobacterium leprae
<400> 2518
   Asp Ala Trp Arg Glu Gly Glu Glu Phe Val Val Glu Phe Asp Leu Pro
   1 5 10 15
   Gly Ile Lys
<210> 2519
   <211> 20
   <212> PRT
   <213> Mycobacterium tuberculosis
<400> 2519
   Asp Gly Asn Pro Pro Glu Val Lys Ser Val Gly Leu Gly Asn Val Asn
   1 5 10 15
   Gly Val Thr Leu
   20
<210> 2520
   <211> 16
   <212> PRT
   <213> Mycobacterium tuberculosis
<400> 2520
   Asp Leu Ser Leu Leu Gly Lys Ala Arg Lys Val Val Val Thr Lys Asp
   1 5 10 15
<210> 2521
   <211> 12
   <212> PRT
   <213> Human herpesvirus
<400> 2521
   Asp Ser Glu Leu Glu Ile Lys Arg Tyr Lys Asn Arg
   1 5 10
<210> 2522
   <211> 15
   <212> PRT
   <213> Homo sapiens
<400> 2522
   Asp Thr Gln Phe Val Arg Phe Asp Ser Asp Ala Ala Ser Gln Arg
   1 5 10 15
<210> 2523
   <211> 15
   <212> PRT
   <213> Mycobacterium bovis
<400> 2523
   Asp Val Glu Gly Glu Ala Leu Ser Thr Leu Val Val Asn Lys Ile
   1 5 10 15
<210> 2524
   <211> 15
   <212> PRT
   <213> Mycobacterium tuberculosis
<400> 2524
   Glu Glu Ser Asn Thr Phe Gly Leu Gln Leu Glu Leu Thr Glu Gly
   1 5 10 15
<210> 2525
   <211> 9
   <212> PRT
   <213> Human herpesvirus
<400> 2525
   Glu Leu Lys Arg Lys Met Ile Tyr Met
   1 5
<210> 2526
   <211> 15
   <212> PRT
   <213> Homo sapiens
<400> 2526
   Glu Asn Pro Val Val His Phe Phe Lys Asn Ile Val Thr Pro Arg
   1 5 10 15
<210> 2527
   <211> 11
   <212> PRT
   <213> Human herpesvirus
<400> 2527
   Glu Pro Leu Pro Gln Gly Gln Leu Thr Ala Tyr
   1 5 10
<210> 2528
   <211> 6
   <212> PRT
   <213> Proteus mirabilis
<400> 2528
   Glu Ser Arg Arg Ala Leu
   1 5
<210> 2529
   <211> 16
   <212> PRT
   <213> Homo sapiens
<400> 2529
   Phe Asp Gln Lys Ile Val Glu Trp Asp Ser Arg Lys Ser Lys Tyr Phe
   1 5 10 15
<210> 2530
   <211> 9
   <212> PRT
   <213> Human herpesvirus
<400> 2530
   Phe Leu Arg Gly Arg Ala Tyr Gly Leu
   1 5
<210> 2531
   <211> 18
   <212> PRT
   <213> Homo sapiens
<400> 2531
   Phe Pro Lys Pro Pro Ser Tyr Asn Val Ala Thr Thr Leu Pro Ser Tyr
   1 5 10 15
   Asp Glu
<210> 2532
   <211> 20
   <212> PRT
   <213> Human herpesvirus
<400> 2532
   Gly Ala Gly Gly Gly Ala Gly Gly Ala Gly Ala Gly Gly Gly Ala Gly
   1 5 10 15
   Gly Ala Gly Ala
   20
<210> 2533
   <211> 24
   <212> PRT
   <213> Human herpesvirus
<400> 2533
   Gly Gly Asp Asn His Gly Arg Gly Arg Gly Arg Gly Arg Gly Arg Gly
   1 5 10 15
   Gly Gly Arg Pro Gly Ala Pro Gly
   20
<210> 2534
   <211> 24
   <212> PRT
   <213> Human herpesvirus
<400> 2534
   Gly Gly Asp Asn His Gly Arg Gly Arg Gly Arg Gly Arg Gly Arg Gly
   1 5 10 15
   Gly Gly Arg Pro Gly Ala Pro Gly
   20
<210> 2535
   <211> 24
   <212> PRT
   <213> Human herpesvirus
<400> 2535
   Gly Gly Asp Asn His Gly Arg Gly Arg Gly Arg Gly Arg Gly Arg Gly
   1 5 10 15
   Gly Gly Arg Pro Gly Ala Pro Gly
   20
<210> 2536
   <211> 24
   <212> PRT
   <213> Human herpesvirus
<400> 2536
   Gly Gly Asp Asn His Gly Arg Gly Arg Gly Arg Gly Arg Gly Arg Gly
   1 5 10 15
   Gly Gly Arg Pro Gly Ala Pro Gly
   20
<210> 2537
   <211> 15
   <212> PRT
   <213> Phaseolus vulgaris
<400> 2537
   Gly Gly Tyr Gly Asp Gly Gly Ala His Gly Gly Gly Tyr Gly Gly
   1 5 10 15
<210> 2538
   <211> 9
   <212> PRT
   <213> Human herpesvirus
<400> 2538
   Gly Leu Cys Thr Leu Val Ala Met Leu
   1 5
<210> 2539
   <211> 10
   <212> PRT
   <213> Mycobacterium tuberculosis
<400> 2539
   Gly Leu Glu Pro Gly Val Val Ala Glu Lys
   1               5                   10
<210> 2540
   <211> 11
   <212> PRT
   <213> Mycobacterium tuberculosis
<400> 2540
   Gly Leu Glu Pro Gly Val Val Ala Glu Lys Val
   1               5                   10
<210> 2541
   <211> 16
   <212> PRT
   <213> Mycobacterium leprae
<400> 2541
   Gly Met Glu Pro Gly Val Val Ala Glu Lys Val Arg Asn Leu Ser Val
   1               5                   10                  15
<210> 2542
   <211> 15
   <212> PRT
   <213> Mycobacterium tuberculosis
<400> 2542
   Ile Ala Phe Asn Ser Gly Leu Glu Pro Gly Val Val Ala Glu Lys
   1               5                   10                  15
<210> 2543
   <211> 16
   <212> PRT
   <213> Mycobacterium leprae
<400> 2543
   Ile Ala Phe Asn Ser Gly Met Glu Pro Gly Val Val Ala Glu Lys Val
   1               5                   10                  15
<210> 2544
   <211> 20
   <212> PRT
   <213> Mycobacterium tuberculosis
<400> 2544
   Ile Ala Ile Gly Gly Ala Ala Thr Gly Ile Ala Ala Val Leu Thr Asp
   1               5                   10                  15
   Gly Asn Pro Pro
               20
<210> 2545
   <211> 15
   <212> PRT
   <213> Homo sapiens
<400> 2545
   Lys Asp Leu Leu Glu Gln Lys Arg Ala Ala Val Asp Thr Tyr Cys
   1               5                   10                  15
<210> 2546
   <211> 14
   <212> PRT
   <213> Human herpesvirus
<400> 2546
   Lys Gly Gly Trp Phe Gly Lys His Arg Gly Gln Gly Gly Ser
   1               5                   10
<210> 2547
   <211> 22
   <212> PRT
   <213> Artemia franciscana
<400> 2547
   Lys Lys Glu Glu Lys Lys Glu Glu Ser Glu Glu Glu Asp Glu Asp Met
   1               5                   10                  15
   Gly Phe Gly Leu Phe Asp
               20
<210> 2548
   <211> 9
   <212> PRT
   <213> Human herpesvirus
<400> 2548
   Lys Arg Lys Met Ile Tyr Met Cys Tyr
   1               5
<210> 2549
   <211> 9
   <212> PRT
   <213> Human herpesvirus
<400> 2549
   Lys Arg Pro Pro Ile Phe Ile Arg Arg
   1 5
<210> 2550
   <211> 9
   <212> PRT
   <213> Human herpesvirus
<400> 2550
   Leu Leu Trp Thr Leu Val Val Leu Leu
   1 5
<210> 2551
   <211> 15
   <212> PRT
   <213> Mycobacterium tuberculosis
<400> 2551
   Met Ala Lys Thr Ile Ala Tyr Asp Glu Glu Ala Arg Arg Gly Leu
   1 5 10 15
<210> 2552
   <211> 15
   <212> PRT
   <213> Mycobacterium tuberculosis
<400> 2552
   Met Ser Pro Val Asn Lys Ser Phe Glu Ile Glu Val Thr Cys Ser
   1 5 10 15
<210> 2553
   <211> 9
   <212> PRT
   <213> Human herpesvirus
<400> 2553
   Asn Leu Val Pro Met Val Ala Thr Val
   1 5
<210> 2554
   <211> 13
   <212> PRT
   <213> Influenza A virus
<400> 2554
   Pro Lys Tyr Val Lys Gln Asn Thr Leu Lys Leu Ala Thr
   1 5 10
<210> 2555
   <211> 13
   <212> PRT
   <213> Unknown
<220>
   <223> epitope involved in rheumatoid arthritis
<400> 2555
   Pro Lys Tyr Val Lys Arg Asn Thr Leu Lys Leu Ala Thr
   1 5 10
<210> 2556
   <211> 12
   <212> PRT
   <213> Clostridium tetani
<400> 2556
   Pro Leu Tyr Lys Lys Met Glu Ala Val Lys Leu Arg
   1 5 10
<210> 2557
   <211> 15
   <212> PRT
   <213> Mycobacterium sp.
<400> 2557
   Pro Tyr Ile Leu Leu Val Ser Ser Lys Val Ser Thr Val Lys Asp
   1 5 10 15
<210> 2558
   <211> 16
   <212> PRT
   <213> Homo sapiens
<400> 2558
   Gln Pro His Asp Leu Gly Lys Val Gly Glu Val Ile Val Thr Lys Asp
   1 5 10 15
<210> 2559
   <211> 8
   <212> PRT
   <213> Human herpesvirus
<400> 2559
   Arg Ala Lys Phe Lys Gln Leu Leu
   1 5
<210> 2560
   <211> 14
   <212> PRT
   <213> Human herpesvirus
<400> 2560
   Arg Ala Arg Gly Arg Gly Arg Gly Arg Gly Glu Lys Arg Pro
   1 5 10
<210> 2561
   <211> 15
   <212> PRT
   <213> Human herpesvirus
<400> 2561
   Arg Arg Gly Arg Gly Arg Glu Arg Ala Arg Gly Gly Ser Arg Glu
   1 5 10 15
<210> 2562
   <211> 9
   <212> PRT
   <213> Human herpesvirus
<400> 2562
   Arg Arg Ile Glu Glu Ile Cys Met Lys
   1 5
<210> 2563
   <211> 9
   <212> PRT
   <213> Human herpesvirus
<400> 2563
   Arg Arg Leu Val Val Thr Leu Gln Cys
   1 5
<210> 2564
   <211> 9
   <212> PRT
   <213> Human herpesvirus
<400> 2564
   Ser Glu Asn Asp Arg Leu Arg Leu Leu
   1 5
<210> 2565
   <211> 20
   <212> PRT
   <213> Mycobacterium tuberculosis
<400> 2565
   Ser Gly Glu Thr Thr Thr Ala Ala Gly Thr Thr Ala Ser Pro Gly Ala
   1 5 10 15
   Ala Ser Gly Pro
   20
<210> 2566
   <211> 13
   <212> PRT
   <213> Influenza A virus
<400> 2566
   Ser Gly Pro Leu Lys Ala Glu Ile Ala Gln Arg Leu Glu
   1 5 10
<210> 2567
   <211> 7
   <212> PRT
   <213> Mus musculus
<400> 2567
   Ser Leu Ala Tyr Gly Leu Arg
   1 5
<210> 2568
   <211> 16
   <212> PRT
   <213> Mycobacterium tuberculosis
<400> 2568
   Ser Val Ala Val Lys Ala Pro Gly Phe Gly Asp Arg Arg Lys Ala Met
   1 5 10 15
<210> 2569
   <211> 9
   <212> PRT
   <213> Human herpesvirus
<400> 2569
   Ser Val Arg Asp Arg Leu Ala Arg Leu
   1 5
<210> 2570
   <211> 9
   <212> PRT
   <213> Unknown
<220>
   <223> epitope involved in rheumatoid arthritis
<400> 2570
   Thr Phe Gly Ala Gln Leu Glu Leu Thr
   1 5
<210> 2571
   <211> 9
   <212> PRT
   <213> Mycobacterium tuberculosis
<400> 2571
   Thr Phe Gly Leu Gln Leu Glu Leu Thr
   1 5
<210> 2572
   <211> 15
   <212> PRT
   <213> Human T-cell lymphotrophic virus
<400> 2572
   Thr Asn Ser His Val Pro Ile Leu Gln Glu Arg Pro Pro Leu Glu
   1 5 10 15
<210> 2573
   <211> 20
   <212> PRT
   <213> Mycobacterium tuberculosis
<400> 2573
   Val Ala Gly Leu Ser Gly Cys Ser Ser Asn Lys Ser Thr Thr Gly Ser
   1 5 10 15
   Gly Glu Thr Thr
   20
<210> 2574
   <211> 17
   <212> PRT
   <213> Homo sapiens
<400> 2574
   Val Asp Asp Thr Gln Phe Val Arg Phe Asp Ser Asp Ala Ala Ser Gln
   1 5 10 15
   Arg
<210> 2575
   <211> 19
   <212> PRT
   <213> Homo sapiens
<400> 2575
   Val Asp Asp Thr Gln Phe Val Arg Phe Asp Ser Asp Ala Ala Ser Gln
   1 5 10 15
   Arg Met Glu
<210> 2576
   <211> 20
   <212> PRT
   <213> Homo sapiens
<400> 2576
   Val Asp Asp Thr Gln Phe Val Arg Phe Asp Ser Asp Ala Ala Ser Gln
   1 5 10 15
   Arg Met Glu Pro
   20
<210> 2577
   <211> 21
   <212> PRT
   <213> Homo sapiens
<400> 2577
   Val Asp Asp Thr Gln Phe Val Arg Phe Asp Ser Asp Ala Ala Ser Gln
   1 5 10 15
   Arg Met Glu Pro Arg
   20
<210> 2578
   <211> 20
   <212> PRT
   <213> Mycobacterium tuberculosis
<400> 2578
   Val Thr Gly Ser Val Val Cys Thr Thr Ala Ala Gly Asn Val Asn Ile
   1 5 10 15
   Ala Ile Gly Gly
   20
<210> 2579
   <211> 15
   <212> PRT
   <213> Mycobacterium bovis
<400> 2579
   Val Val Ala Glu Lys Val Arg Asn Leu Pro Ala Gly His Gly Leu
   1 5 10 15
<210> 2580
   <211> 9
   <212> PRT
   <213> Human herpesvirus
<400> 2580
   Tyr Val Leu Asp His Leu Ile Val Val
   1 5
<210> 2581
   <211> 20
   <212> PRT
   <213> Homo sapiens
<400> 2581
   Lys Phe Gly Ala Asp Ala Arg Ala Leu Met Leu Gln Gly Val Asp Leu
   1               5                   10                  15
   Leu Ala Asp Ala
               20
<210> 2582
   <211> 22
   <212> PRT
   <213> Human herpesvirus
<400> 2582
   Gly Gly Ala Gly Ala Gly Gly Gly Ala Gly Gly Ala Gly Ala Gly Gly
   1               5                   10                  15
   Gly Ala Gly Gly Ala Gly
               20
<210> 2583
   <211> 11
   <212> PRT
   <213> Homo sapiens
<400> 2583
   Gly Leu Lys Gly Glu Gln Gly Glu Pro Gly Ala
   1               5                   10
<210> 2584
   <211> 11
   <212> PRT
   <213> Gallus gallus
<400> 2584
   Ile Ala Gly Phe Lys Gly Glu Gln Gly Pro Lys
   1               5                   10
<210> 2585
   <211> 20
   <212> PRT
   <213> Mycobacterium tuberculosis
<400> 2585
   Met Lys Arg Gly Leu Thr Val Ala Val Ala Gly Ala Ala Ile Leu Val
   1 5 10 15
   Ala Gly Leu Ser
   20
<210> 2586
   <211> 20
   <212> PRT
   <213> Mycobacterium tuberculosis
<400> 2586
   Asn Gly Val Thr Leu Gly Tyr Thr Ser Gly Thr Cys Gln Gly Asn Ala
   1 5 10 15
   Ser Ala Thr Lys
   20
<210> 2587
   <211> 26
   <212> PRT
   <213> Gallus gallus
<400> 2587
   Pro Thr Gly Pro Leu Gly Pro Lys Gly Gln Thr Gly Glu Leu Gly Ile
   1 5 10 15
   Ala Gly Phe Lys Gly Glu Gln Gly Pro Lys
   20 25
<210> 2588
   <211> 9
   <212> PRT
   <213> Unknown
<220>
   <223> epitope involved in rheumatoid arthritis
<400> 2588
   Ala Asp Gly Gly Ala Gln Gly Thr Ala
   1 5
<210> 2589
   <211> 12
   <212> PRT
   <213> Unknown
<220>
   <223> epitope involved in rheumatoid arthritis
<400> 2589
   Ala Asp Ile Trp Leu Thr Pro Asp Pro Thr Thr Pro
   1 5 10
<210> 2590
   <211> 15
   <212> PRT
   <213> Homo sapiens
<400> 2590
   Cys Val Asp Thr Arg Ser Gly Asn Cys Tyr Leu Asp Ile Arg Pro
   1 5 10 15
<210> 2591
   <211> 12
   <212> PRT
   <213> Unknown
<220>
   <223> epitope involved in rheumatoid arthritis
<400> 2591
   Asp Val Tyr Trp Leu Met Pro Asp Pro Pro Thr Ser
   1 5 10
<210> 2592
   <211> 15
   <212> PRT
   <213> Homo sapiens
<400> 2592
   Glu Pro Val Ser Gly Ser Phe Thr Thr Ala Leu Asp Gly Pro Ser
   1 5 10 15
<210> 2593
   <211> 15
   <212> PRT
   <213> Homo sapiens
<400> 2593
   Phe Phe Tyr Phe Phe Thr Gly Ser Ser Gln Leu Glu Phe Asp Pro
   1 5 10 15
<210> 2594
   <211> 15
   <212> PRT
   <213> Rattus norvegicus
<400> 2594
   Gly Ile Ala Gly Phe Lys Gly Glu Gln Gly Pro Lys Gly Glu Thr
   1 5 10 15
<210> 2595
   <211> 21
   <212> PRT
   <213> Homo sapiens
<400> 2595
   Gly Pro Lys Gly Gln Thr Gly Glu Pro Gly Ile Ala Gly Phe Lys Gly
   1               5                   10                  15
   Glu Gln Gly Pro Lys
               20
<210> 2596
   <211> 21
   <212> PRT
   <213> Homo sapiens
<400> 2596
   Gly Pro Lys Gly Gln Thr Gly Lys Pro Gly Ile Ala Gly Phe Lys Gly
   1               5                   10                  15
   Glu Gln Gly Pro Lys
               20
<210> 2597
   <211> 12
   <212> PRT
   <213> Unknown
<220>
   <223> epitope involved in rheumatoid arthritis
<400> 2597
   Gly Thr Ser Thr Glu Val Trp Leu Ala Pro Asp Pro
   1               5                   10
<210> 2598
   <211> 15
   <212> PRT
   <213> Homo sapiens
<400> 2598
   Gly Val Val Ser His Ser Phe Pro Ala Thr Leu Glu Thr Gln Glu
   1               5                   10                  15
<210> 2599
   <211> 12
   <212> PRT
   <213> Unknown
<220>
   <223> epitope involved in rheumatoid arthritis
<400> 2599
   His Leu Pro Ser Phe Leu Arg Pro Asp Pro Pro Leu
   1 5 10
<210> 2600
   <211> 12
   <212> PRT
   <213> Unknown
<220>
   <223> epitope involved in rheumatoid arthritis
<400> 2600
   Ile Phe Pro Ser Phe Leu Arg Pro Asp Pro Pro Leu
   1 5 10
<210> 2601
   <211> 15
   <212> PRT
   <213> Homo sapiens
<400> 2601
   Ile Ser Gly Leu Pro Ser Gly Gly Asp Asp Leu Glu Thr Ser Thr
   1 5 10 15
<210> 2602
   <211> 15
   <212> PRT
   <213> Homo sapiens
<400> 2602
   Ile Trp Tyr Ile Asn Cys Phe Gly Cys Glu Thr His Ala Met Leu
   1 5 10 15
<210> 2603
   <211> 15
   <212> PRT
   <213> Homo sapiens
<400> 2603
   Leu Ser Gly Leu Pro Ser Gly Gly Glu Val Leu Glu Ile Ser Val
   1 5 10 15
<210> 2604
   <211> 9
   <212> PRT
   <213> Unknown
<220>
   <223> epitope involved in rheumatoid arthritis
<400> 2604
   Leu Ser Ser Arg Glu Pro Gln Ala Arg
   1 5
<210> 2605
   <211> 11
   <212> PRT
   <213> Rattus norvegicus
<400> 2605
   Leu Val Gly Pro Arg Gly Glu Arg Gly Phe Pro
   1               5                   10
<210> 2606
   <211> 17
   <212> PRT
   <213> Homo sapiens
<400> 2606
   Asn Glu Glu Gly Phe Phe Ser Ala Arg Gly His Arg Pro Leu Asp Lys
   1               5                   10                  15
   Lys
<210> 2607
   <211> 12
   <212> PRT
   <213> Unknown
<220>
   <223> epitope involved in rheumatoid arthritis
<400> 2607
   Asn Ser Asp His Phe Leu Gln Pro Asp Pro Pro Leu
   1               5                   10
<210> 2608
   <211> 9
   <212> PRT
   <213> Unknown
<220>
   <223> epitope involved in rheumatoid arthritis
<400> 2608
   Pro Gly Pro Ser Arg Ala His Phe Leu
   1               5
<210> 2609
   <211> 15
   <212> PRT
   <213> Escherichia coli
<400> 2609
   Gln Lys Arg Ala Ala Tyr Asp Gln Tyr Gly His Ala Ala Phe Glu
   1               5                   10                  15
<210> 2610
   <211> 9
   <212> PRT
   <213> Unknown
<220>
   <223> epitope involved in rheumatoid arthritis
<400> 2610
   Arg Leu Thr Arg Glu Leu Tyr Ala Gln
   1               5
<210> 2611
   <211> 13
   <212> PRT
   <213> Homo sapiens
<400> 2611
   Arg Ser Phe Thr Leu Ala Ser Ser Glu Thr Gly Val Gly
   1               5                   10
<210> 2612
   <211> 15
   <212> PRT
   <213> Homo sapiens
<400> 2612
   Val Lys Glu Gly His Ser Pro Pro Asp Asp Val Asp Ile Val Ile
   1               5                   10                  15
<210> 2613
   <211> 16
   <212> PRT
   <213> Homo sapiens
<400> 2613
   Val Tyr Ala Thr Arg Ser Ser Ala Val Arg Leu Arg Ser Ser Val Pro
   1               5                   10                  15
<210> 2614
   <211> 9
   <212> PRT
   <213> Unknown
<220>
   <223> epitope involved in rheumatoid arthritis
<400> 2614
   Tyr Thr Gln Lys His Lys His Gln Ala
   1               5
<210> 2615
   <211> 13
   <212> PRT
   <213> Bos taurus
<400> 2615
   Ala Gly Phe Lys Gly Glu Gln Gly Pro Lys Gly Glu Pro
   1               5                   10
<210> 2616
   <211> 11
   <212> PRT
   <213> Homo sapiens
<400> 2616
   Ala Arg Gly Leu Thr Gly Arg Pro Gly Asp Ala
   1               5                   10
<210> 2617
   <211> 15
   <212> PRT
   <213> Mus musculus
<400> 2617
   Ala Thr Glu Gly Gln Val Arg Val Asn Ser Ile Tyr Gln Asp Lys
   1               5                   10                  15
<210> 2618
   <211> 15
   <212> PRT
   <213> Bos taurus
<400> 2618
   Ala Thr Tyr Lys Arg Arg Leu Gln Lys Arg Ser Ser Arg Pro Leu
   1               5                   10                  15
<210> 2619
   <211> 21
   <212> PRT
   <213> Unknown
<220>
   <223> epitope involved in rheumatoid arthritis
<400> 2619
   Gly Glu Pro Gly Ile Ala Gly Phe Lys Gly Asp Gln Gly Pro Lys Gly
   1               5                   10                  15
   Glu Pro Gly Pro Ala
               20
<210> 2620
   <211> 12
   <212> PRT
   <213> Rattus norvegicus
<400> 2620
   Gly Ile Ala Gly Phe Lys Gly Glu Gln Gly Pro Lys
   1 5 10
<210> 2621
   <211> 15
   <212> PRT
   <213> Rattus norvegicus
<400> 2621
   Gly Ile Ala Gly Phe Lys Gly Glu Gln Gly Pro Lys Gly Glu Thr
   1 5 10 15
<210> 2622
   <211> 8
   <212> PRT
   <213> Homo sapiens
<400> 2622
   Leu Lys Phe Val Glu Gln Asn Glu
   1 5
<210> 2623
   <211> 15
   <212> PRT
   <213> Mus musculus
<400> 2623
   Asn Thr Tyr Lys His Arg Leu Gln Lys Arg Thr Met Arg Pro Thr
   1 5 10 15
<210> 2624
   <211> 13
   <212> PRT
   <213> Homo sapiens
<400> 2624
   Pro Thr Phe Gly Arg Ser Phe Thr Leu Ala Ser Ser Glu
   1 5 10
<210> 2625
   <211> 26
   <212> PRT
   <213> Unknown
<220>
   <223> epitope involved in rheumatoid arthritis
<400> 2625
   Pro Thr Gly Pro Leu Gly Pro Lys Gly Gln Thr Gly Glu Leu Gly Ala
   1 5 10 15
   Pro Gly Asn Lys Gly Glu Gln Gly Pro Lys
   20 25
<210> 2626
   <211> 14
   <212> PRT
   <213> Homo sapiens
<400> 2626
   Ser Gln Arg Phe Ser Lys Ile Ala Ser Asn Thr Gln Ser Arg
   1 5 10
<210> 2627
   <211> 15
   <212> PRT
   <213> Canis lupus
<400> 2627
   Ser Thr Tyr Lys Arg Arg Leu Gln Lys Arg Ser Ser Arg Ala Pro
   1 5 10 15
<210> 2628
   <211> 20
   <212> PRT
   <213> Homo sapiens
<400> 2628
   Thr Gly Glu Pro Gly Ile Ala Gly Phe Lys Gly Glu Gln Gly Pro Lys
   1 5 10 15
   Gly Glu Pro Gly
   20
<210> 2629
   <211> 15
   <212> PRT
   <213> Unknown
<220>
   <223> epitope involved in rheumatoid arthritis
<400> 2629
   Thr Thr Tyr Lys Arg Arg Leu Gln Lys Arg Ala Ala Arg His Pro
   1 5 10 15
<210> 2630
   <211> 9
   <212> PRT
   <213> Homo sapiens
<400> 2630
   Thr Tyr Lys Arg Arg Leu Gln Lys Arg
   1 5
<210> 2631
   <211> 14
   <212> PRT
   <213> Sus scrofa
<400> 2631
   Thr Tyr Lys Arg Arg Leu Gln Lys Arg Ser Ser Arg Ser Leu
   1 5 10
<210> 2632
   <211> 13
   <212> PRT
   <213> Homo sapiens
<400> 2632
   Val Gly Tyr Asp Asp Gln Glu Ser Val Lys Ser Lys Val
   1 5 10
<210> 2633
   <211> 18
   <212> PRT
   <213> Homo sapiens
<400> 2633
   Ala Gly Met Asp Met Cys Ser Ala Gly Trp Leu Ala Asp Arg Ser Val
   1 5 10 15
   Arg Tyr
<210> 2634
   <211> 7
   <212> PRT
   <213> Homo sapiens
<400> 2634
   Ala Leu Pro Ala Pro Ile Glu
   1 5
<210> 2635
   <211> 7
   <212> PRT
   <213> Homo sapiens
<400> 2635
   Ala Pro Ile Glu Lys Thr Ile
   1 5
<210> 2636
   <211> 7
   <212> PRT
   <213> Homo sapiens
<400> 2636
   Cys Lys Val Ser Asn Lys Ala
   1 5
<210> 2637
   <211> 7
   <212> PRT
   <213> Homo sapiens
<400> 2637
   Asp Gly Lys Glu Tyr Lys Cys
   1 5
<210> 2638
   <211> 7
   <212> PRT
   <213> Homo sapiens
<400> 2638
   Asp Val Ser His Glu Asp Pro
   1 5
<210> 2639
   <211> 7
   <212> PRT
   <213> Homo sapiens
<400> 2639
   Glu Asp Pro Gln Val Lys Phe
   1 5
<210> 2640
   <211> 7
   <212> PRT
   <213> Homo sapiens
<400> 2640
   Glu Lys Thr Ile Ser Lys Ala
   1 5
<210> 2641
   <211> 6
   <212> PRT
   <213> Homo sapiens
<400> 2641
   Glu Gln Arg Arg Ala Ala
   1 5
<210> 2642
   <211> 7
   <212> PRT
   <213> Homo sapiens
<400> 2642
   Glu Tyr Lys Cys Lys Val Ser
   1 5
<210> 2643
   <211> 7
   <212> PRT
   <213> Homo sapiens
<400> 2643
   Phe Leu Phe Pro Pro Lys Pro
   1 5
<210> 2644
   <211> 7
   <212> PRT
   <213> Homo sapiens
<400> 2644
   Phe Asn Trp Tyr Val Asp Gly
   1 5
<210> 2645
   <211> 7
   <212> PRT
   <213> Homo sapiens
<400> 2645
   Phe Pro Pro Lys Pro Lys Asp
   1 5
<210> 2646
   <211> 16
   <212> PRT
   <213> Bos taurus
<400> 2646
   Gly Ala Arg Gly Pro Glu Gly Ala Gln Gly Pro Arg Gly Glu Pro Gly
   1 5 10 15
<210> 2647
   <211> 15
   <212> PRT
   <213> Bos taurus
<400> 2647
   Gly Glu Pro Gly Ile Ala Gly Phe Lys Gly Glu Gln Gly Pro Lys
   1 5 10 15
<210> 2648
   <211> 15
   <212> PRT
   <213> Rattus norvegicus
<400> 2648
   Gly Glu Pro Gly Ile Ala Gly Phe Lys Gly Glu Gln Gly Pro Lys
   1 5 10 15
<210> 2649
   <211> 15
   <212> PRT
   <213> Rattus norvegicus
<400> 2649
   Gly Glu Pro Gly Ile Ala Gly Phe Lys Gly Glu Gln Gly Pro Lys
   1 5 10 15
<210> 2650
   <211> 7
   <212> PRT
   <213> Homo sapiens
<400> 2650
   Gly Lys Glu Tyr Lys Cys Lys
   1 5
<210> 2651
   <211> 16
   <212> PRT
   <213> Homo sapiens
<400> 2651
   Gly Lys Pro Gly Ile Ala Gly Phe Lys Gly Glu Gln Gly Pro Lys Gly
   1 5 10 15
<210> 2652
   <211> 16
   <212> PRT
   <213> Homo sapiens
<400> 2652
   Gly Asn Gln Trp Val Gly Tyr Asp Asp Gln Glu Ser Val Lys Ser Lys
   1 5 10 15
<210> 2653
   <211> 7
   <212> PRT
   <213> Homo sapiens
<400> 2653
   Gly Pro Ser Val Phe Leu Phe
   1               5
<210> 2654
   <211> 11
   <212> PRT
   <213> Homo sapiens
<400> 2654
   Gly Arg Gly Asp Ser Val Val Tyr Gly Leu Arg
   1               5                   10
<210> 2655
   <211> 16
   <212> PRT
   <213> Homo sapiens
<400> 2655
   Gly Arg Ser Phe Thr Leu Ala Ser Ser Glu Thr Gly Val Gly Ala Pro
   1               5                   10                  15
<210> 2656
   <211> 15
   <212> PRT
   <213> Homo sapiens
<400> 2656
   Gly Thr Ser Leu Thr Ile Pro Cys Tyr Phe Ile Asp Pro Met His
   1               5                   10                  15
<210> 2657
   <211> 7
   <212> PRT
   <213> Homo sapiens
<400> 2657
   Gly Val Gln Val His Asn Ala
   1               5
<210> 2658
   <211> 7
   <212> PRT
   <213> Homo sapiens
<400> 2658
   His Gln Asn Trp Leu Asp Gly
   1               5
<210> 2659
   <211> 7
   <212> PRT
   <213> Homo sapiens
<400> 2659
   Ile Glu Lys Thr Ile Ser Lys
   1 5
<210> 2660
   <211> 7
   <212> PRT
   <213> Homo sapiens
<400> 2660
   Ile Ser Lys Ala Lys Gly Gln
   1 5
<210> 2661
   <211> 7
   <212> PRT
   <213> Homo sapiens
<400> 2661
   Lys Ala Leu Pro Ala Pro Ile
   1 5
<210> 2662
   <211> 7
   <212> PRT
   <213> Homo sapiens
<400> 2662
   Lys Cys Lys Val Ser Asn Lys
   1 5
<210> 2663
   <211> 7
   <212> PRT
   <213> Homo sapiens
<400> 2663
   Lys Glu Tyr Lys Cys Lys Val
   1 5
<210> 2664
   <211> 7
   <212> PRT
   <213> Homo sapiens
<400> 2664
   Lys Phe Asn Trp Tyr Val Asp
   1 5
<210> 2665
   <211> 15
   <212> PRT
   <213> Gallus gallus
<400> 2665
   Lys Gly Gln Thr Gly Glu Leu Gly Ile Ala Gly Phe Lys Gly Glu
   1 5 10 15
<210> 2666
   <211> 7
   <212> PRT
   <213> Homo sapiens
<400> 2666
   Lys Pro Arg Glu Gln Gln Tyr
   1 5
<210> 2667
   <211> 7
   <212> PRT
   <213> Homo sapiens
<400> 2667
   Lys Thr Ile Ser Lys Ala Lys
   1 5
<210> 2668
   <211> 7
   <212> PRT
   <213> Homo sapiens
<400> 2668
   Lys Thr Lys Pro Arg Glu Gln
   1 5
<210> 2669
   <211> 7
   <212> PRT
   <213> Homo sapiens
<400> 2669
   Lys Val Ser Asn Lys Ala Leu
   1 5
<210> 2670
   <211> 7
   <212> PRT
   <213> Homo sapiens
<400> 2670
   Leu Asp Gly Lys Glu Tyr Lys
   1 5
<210> 2671
   <211> 7
   <212> PRT
   <213> Homo sapiens
<400> 2671
   Leu Phe Pro Pro Lys Pro Lys
   1 5
<210> 2672
   <211> 33
   <212> PRT
   <213> Homo sapiens
<400> 2672
   Leu Gly Pro Lys Gly Gln Thr Gly Glu Pro Gly Ile Ala Gly Phe Lys
   1 5 10 15
   Gly Glu Gln Gly Pro Lys Gly Glu Pro Gly Pro Ala Gly Pro Gln Gly
   20 25 30
   Ala
<210> 2673
   <211> 7
   <212> PRT
   <213> Homo sapiens
<400> 2673
   Leu His Gln Asn Trp Leu Asp
   1 5
<210> 2674
   <211> 7
   <212> PRT
   <213> Homo sapiens
<400> 2674
   Leu Pro Ala Pro Ile Glu Lys
   1 5
<210> 2675
   <211> 7
   <212> PRT
   <213> Homo sapiens
<400> 2675
   Leu Thr Val Leu His Gln Asn
   1 5
<210> 2676
   <211> 16
   <212> PRT
   <213> Homo sapiens
<400> 2676
   Met Gly Ile Pro Thr Phe Gly Arg Ser Phe Thr Leu Ala Ser Ser Glu
   1 5 10 15
<210> 2677
   <211> 7
   <212> PRT
   <213> Homo sapiens
<400> 2677
   Asn Ala Lys Thr Lys Pro Arg
   1 5
<210> 2678
   <211> 16
   <212> PRT
   <213> Homo sapiens
<400> 2678
   Asn Phe Gly Ser Gln Arg Phe Ser Lys Ile Ala Ser Asn Thr Gln Ser
   1 5 10 15
<210> 2679
   <211> 7
   <212> PRT
   <213> Homo sapiens
<400> 2679
   Asn Lys Ala Leu Pro Ala Pro
   1 5
<210> 2680
   <211> 7
   <212> PRT
   <213> Homo sapiens
<400> 2680
   Asn Ser Thr Tyr Arg Val Val
   1 5
<210> 2681
   <211> 7
   <212> PRT
   <213> Homo sapiens
<400> 2681
   Asn Trp Leu Asp Gly Lys Glu
   1               5
<210> 2682
   <211> 7
   <212> PRT
   <213> Homo sapiens
<400> 2682
   Asn Trp Tyr Val Asp Gly Val
   1               5
<210> 2683
   <211> 7
   <212> PRT
   <213> Homo sapiens
<400> 2683
   Pro Ala Pro Ile Glu Lys Thr
   1               5
<210> 2684
   <211> 7
   <212> PRT
   <213> Homo sapiens
<400> 2684
   Pro Ile Glu Lys Thr Ile Ser
   1               5
<210> 2685
   <211> 7
   <212> PRT
   <213> Homo sapiens
<400> 2685
   Pro Gln Val Lys Phe Asn Trp
   1               5
<210> 2686
   <211> 7
   <212> PRT
   <213> Homo sapiens
<400> 2686
   Pro Arg Glu Gln Gln Tyr Asn
   1               5
<210> 2687
   <211> 7
   <212> PRT
   <213> Homo sapiens
<400> 2687
   Pro Ser Val Phe Leu Phe Pro
   1 5
<210> 2688
   <211> 16
   <212> PRT
   <213> Homo sapiens
<400> 2688
   Gln His Leu Asp Phe Ile Ser Ile Met Thr Tyr Asp Phe His Gly Ala
   1 5 10 15
<210> 2689
   <211> 7
   <212> PRT
   <213> Homo sapiens
<400> 2689
   Gln Asn Trp Leu Asp Gly Lys
   1 5
<210> 2690
   <211> 7
   <212> PRT
   <213> Homo sapiens
<400> 2690
   Gln Gln Tyr Asn Ser Thr Tyr
   1 5
<210> 2691
   <211> 7
   <212> PRT
   <213> Homo sapiens
<400> 2691
   Gln Val Lys Phe Asn Trp Tyr
   1 5
<210> 2692
   <211> 16
   <212> PRT
   <213> Homo sapiens
<400> 2692
   Gln Trp Val Gly Tyr Asp Asp Gln Glu Ser Val Lys Ser Lys Val Gln
   1 5 10 15
<210> 2693
   <211> 7
   <212> PRT
   <213> Homo sapiens
<400> 2693
   Gln Tyr Asn Ser Thr Tyr Arg
   1 5
<210> 2694
   <211> 7
   <212> PRT
   <213> Homo sapiens
<400> 2694
   Arg Glu Gln Gln Tyr Asn Ser
   1 5
<210> 2695
   <211> 7
   <212> PRT
   <213> Homo sapiens
<400> 2695
   Arg Val Val Ser Val Leu Thr
   1 5
<210> 2696
   <211> 16
   <212> PRT
   <213> Homo sapiens
<400> 2696
   Ser Cys Phe Pro Asp Ala Leu Asp Arg Phe Leu Cys Thr His Ile Ile
   1 5 10 15
<210> 2697
   <211> 7
   <212> PRT
   <213> Homo sapiens
<400> 2697
   Ser Asn Lys Ala Leu Pro Ala
   1 5
<210> 2698
   <211> 7
   <212> PRT
   <213> Homo sapiens
<400> 2698
   Ser Val Phe Leu Phe Pro Pro
   1 5
<210> 2699
   <211> 7
   <212> PRT
   <213> Homo sapiens
<400> 2699
   Ser Val Leu Thr Val Leu His
   1 5
<210> 2700
   <211> 7
   <212> PRT
   <213> Homo sapiens
<400> 2700
   Thr Ile Ser Lys Ala Lys Gly
   1 5
<210> 2701
   <211> 7
   <212> PRT
   <213> Homo sapiens
<400> 2701
   Thr Val Leu His Gln Asn Trp
   1 5
<210> 2702
   <211> 7
   <212> PRT
   <213> Homo sapiens
<400> 2702
   Thr Tyr Arg Val Val Ser Val
   1 5
<210> 2703
   <211> 7
   <212> PRT
   <213> Homo sapiens
<400> 2703
   Val Asp Gly Val Gln Val His
   1 5
<210> 2704
   <211> 7
   <212> PRT
   <213> Homo sapiens
<400> 2704
   Val Phe Leu Phe Pro Pro Lys
   1 5
<210> 2705
   <211> 7
   <212> PRT
   <213> Homo sapiens
<400> 2705
   Val His Asn Ala Lys Thr Lys
   1 5
<210> 2706
   <211> 7
   <212> PRT
   <213> Homo sapiens
<400> 2706
   Val Lys Phe Asn Trp Tyr Val
   1 5
<210> 2707
   <211> 7
   <212> PRT
   <213> Homo sapiens
<400> 2707
   Val Leu His Gln Asn Trp Leu
   1 5
<210> 2708
   <211> 7
   <212> PRT
   <213> Homo sapiens
<400> 2708
   Val Ser Asn Lys Ala Leu Pro
   1 5
<210> 2709
   <211> 7
   <212> PRT
   <213> Homo sapiens
<400> 2709
   Val Ser Val Leu Thr Val Leu
   1 5
<210> 2710
   <211> 18
   <212> PRT
   <213> Homo sapiens
<400> 2710
   Val Val Leu Leu Val Ala Thr Glu Gly Arg Val Arg Val Asn Ser Ala
   1               5                   10                  15
   Tyr Gln
<210> 2711
   <211> 7
   <212> PRT
   <213> Homo sapiens
<400> 2711
   Val Val Ser Val Leu Thr Val
   1               5
<210> 2712
   <211> 18
   <212> PRT
   <213> Homo sapiens
<400> 2712
   Val Val Val Lys Gly Ile Val Phe His Tyr Arg Ala Ile Ser Thr Arg
   1               5                   10                  15
   Tyr Thr
<210> 2713
   <211> 7
   <212> PRT
   <213> Homo sapiens
<400> 2713
   Trp Leu Asp Gly Lys Glu Tyr
   1               5
<210> 2714
   <211> 7
   <212> PRT
   <213> Homo sapiens
<400> 2714
   Trp Tyr Val Asp Gly Val Gln
   1               5
<210> 2715
   <211> 7
   <212> PRT
   <213> Homo sapiens
<400> 2715
   Tyr Lys Cys Lys Val Ser Asn
   1 5
<210> 2716
   <211> 15
   <212> PRT
   <213> Homo sapiens
<400> 2716
   Tyr Leu Ala Trp Gln Ala Gly Met Asp Met Cys Ser Ala Gly Trp
   1 5 10 15
<210> 2717
   <211> 7
   <212> PRT
   <213> Homo sapiens
<400> 2717
   Tyr Asn Ser Thr Tyr Arg Val
   1 5
<210> 2718
   <211> 7
   <212> PRT
   <213> Homo sapiens
<400> 2718
   Tyr Arg Val Val Ser Val Leu
   1 5
<210> 2719
   <211> 7
   <212> PRT
   <213> Homo sapiens
<400> 2719
   Tyr Val Asp Gly Val Gln Val
   1 5
<210> 2720
   <211> 9
   <212> PRT
   <213> Human herpesvirus
<400> 2720
   Ala Ala Arg Gln Arg Leu Gln Asp Ile
   1 5
<210> 2721
   <211> 13
   <212> PRT
   <213> Homo sapiens
<400> 2721
   Ala Gly Trp Leu Ala Asp Gly Ser Leu Arg Tyr Pro Ile
   1 5 10
<210> 2722
   <211> 13
   <212> PRT
   <213> Homo sapiens
<400> 2722
   Ala Gly Trp Leu Ala Asp Gln Thr Val Arg Tyr Pro Ile
   1 5 10
<210> 2723
   <211> 14
   <212> PRT
   <213> Homo sapiens
<400> 2723
   Ala Arg Gly Ala Gln Gly Pro Pro Gly Ala Thr Gly Phe Pro
   1 5 10
<210> 2724
   <211> 18
   <212> PRT
   <213> Homo sapiens
<400> 2724
   Ala Thr Leu Glu Val Gln Ser Leu Arg Ser Asn Asp Ser Gly Val Tyr
   1 5 10 15
   Arg Cys
<210> 2725
   <211> 10
   <212> PRT
   <213> Unknown
<220>
   <223> epitope involved in rheumatoid arthritis
<400> 2725
   Ala Trp Asn Cys Cys Val Lys Gln Thr Pro
   1 5 10
<210> 2726
   <211> 20
   <212> PRT
   <213> Mycobacterium tuberculosis
<400> 2726
   Ala Tyr Asp Glu Glu Ala Arg Arg Gly Leu Glu Arg Gly Leu Asn Ala
   1               5                   10                  15
   Leu Ala Asp Ala
               20
<210> 2727
   <211> 9
   <212> PRT
   <213> Human herpesvirus
<400> 2727
   Cys Leu Gly Gly Leu Leu Thr Met Asn
   1               5
<210> 2728
   <211> 7
   <212> PRT
   <213> Homo sapiens
<400> 2728
   Cys Ser Val Met His Glu Gly
   1               5
<210> 2729
   <211> 11
   <212> PRT
   <213> Homo sapiens
<400> 2729
   Asp Ala Gly Pro Gln Gly Lys Val Gly Pro Ser
   1               5                   10
<210> 2730
   <211> 7
   <212> PRT
   <213> Homo sapiens
<400> 2730
   Asp Gly Ser Phe Phe Leu Tyr
   1               5
<210> 2731
   <211> 18
   <212> PRT
   <213> Homo sapiens
<400> 2731
   Asp Ser Gly His Arg Gly Tyr Ser Gly Ser Gln Ala Ser Asp Asn Glu
   1 5 10 15
   Gly His
<210> 2732
   <211> 9
   <212> PRT
   <213> Porphyromonas gingivalis
<400> 2732
   Asp Ser Arg Gly Asn Pro Thr Val Glu
   1 5
<210> 2733
   <211> 7
   <212> PRT
   <213> Homo sapiens
<400> 2733
   Glu Gly Leu His Asn His Tyr
   1 5
<210> 2734
   <211> 11
   <212> PRT
   <213> Homo sapiens
<400> 2734
   Glu Arg Gly Leu Lys Gly His Arg Gly Phe Thr
   1 5 10
<210> 2735
   <211> 11
   <212> PRT
   <213> Homo sapiens
<400> 2735
   Gly Gly Gln Gly Ser Arg His Gln Gln Ala Arg
   1 5 10
<210> 2736
   <211> 14
   <212> PRT
   <213> Homo sapiens
<400> 2736
   Gly Gly Val Gly Pro Ile Gly Pro Pro Gly Glu Arg Gly Ala
   1 5 10
<210> 2737
   <211> 9
   <212> PRT
   <213> Bos taurus
<400> 2737
   Gly Pro Ala Gly Pro Ala Gly Glu Arg
   1 5
<210> 2738
   <211> 9
   <212> PRT
   <213> Human herpesvirus
<400> 2738
   Gly Pro Lys Val Lys Arg Pro Pro Ile
   1 5
<210> 2739
   <211> 11
   <212> PRT
   <213> Unknown
<220>
   <223> epitope involved in rheumatoid arthritis
<400> 2739
   Gly Trp Val Ala Met His Pro Gln Gly Asp Leu
   1 5 10
<210> 2740
   <211> 21
   <212> PRT
   <213> Homo sapiens
<400> 2740
   His Gln Cys His Gln Glu Ser Thr Arg Gly Arg Ser Arg Gly Arg Cys
   1 5 10 15
   Gly Arg Ser Gly Ser
   20
<210> 2741
   <211> 18
   <212> PRT
   <213> Homo sapiens
<400> 2741
   His Ser Ala Ser Gln Asp Gly Gln Asp Thr Ile Arg Gly His Pro Gly
   1 5 10 15
   Ser Ser
<210> 2742
   <211> 22
   <212> PRT
   <213> Homo sapiens
<400> 2742
   His Ser Gly Ile Gly His Gly Gln Ala Ser Ser Ala Val Arg Asp Ser
   1               5                   10                  15
   Gly His Arg Gly Tyr Ser
               20
<210> 2743
   <211> 17
   <212> PRT
   <213> Homo sapiens
<400> 2743
   His Ser Thr Ser Gln Glu Gly Gln Asp Thr Ile His Gly His Arg Gly
   1               5                   10                  15
   Ser
<210> 2744
   <211> 7
   <212> PRT
   <213> Homo sapiens
<400> 2744
   Lys Ser Leu Ser Leu Ser Pro
   1               5
<210> 2745
   <211> 20
   <212> PRT
   <213> Homo sapiens
<400> 2745
   Leu Gly Leu Arg Leu Ile Tyr Phe Ser Tyr Asp Val Lys Met Lys Glu
   1               5                   10                  15
   Lys Gly Asp Ile
               20
<210> 2746
   <211> 10
   <212> PRT
   <213> Unknown
<220>
   <223> epitope involved in rheumatoid arthritis
<400> 2746
   Leu Gly Trp Met Arg Tyr His Pro Gln Ala
   1               5                   10
<210> 2747
   <211> 10
   <212> PRT
   <213> Human herpesvirus
<400> 2747
   Leu Leu Asp Phe Val Arg Phe Met Gly Tyr
   1               5                   10
<210> 2748
   <211> 10
   <212> PRT
   <213> Unknown
<220>
   <223> epitope involved in rheumatoid arthritis
<400> 2748
   Leu Leu Pro Trp Leu Asp Gly His Pro Met
   1               5                   10
<210> 2749
   <211> 10
   <212> PRT
   <213> Homo sapiens
<400> 2749
   Leu Leu Arg Gly Tyr His Gln Asp Ala Tyr
   1               5                   10
<210> 2750
   <211> 11
   <212> PRT
   <213> Homo sapiens
<400> 2750
   Leu Val Gly Pro Arg Gly Glu Arg Gly Phe Pro
   1               5                   10
<210> 2751
   <211> 10
   <212> PRT
   <213> Unknown
<220>
   <223> epitope involved in rheumatoid arthritis
<400> 2751
   Asn Trp Leu Ser Leu Ile Thr Ala Ala Arg
   1 5 10
<210> 2752
   <211> 13
   <212> PRT
   <213> Homo sapiens
<400> 2752
   Pro Glu Phe Ser Gly Leu Pro Ser Gly Ile Ala Glu Val
   1 5 10
<210> 2753
   <211> 15
   <212> PRT
   <213> Mus musculus
<400> 2753
   Pro Pro Gly Ala Asn Gly Asn Pro Gly Pro Ala Gly Pro Pro Gly
   1 5 10 15
<210> 2754
   <211> 9
   <212> PRT
   <213> Homo sapiens
<400> 2754
   Pro Pro Gly Pro Thr Gly Ala Ser Gly
   1 5
<210> 2755
   <211> 7
   <212> PRT
   <213> Homo sapiens
<400> 2755
   Pro Gln Val Tyr Thr Leu Pro
   1 5
<210> 2756
   <211> 7
   <212> PRT
   <213> Homo sapiens
<400> 2756
   Pro Arg Glu Pro Gln Val Tyr
   1 5
<210> 2757
   <211> 11
   <212> PRT
   <213> Homo sapiens
<400> 2757
   Gln Gly Lys Val Gly Pro Ser Gly Ala Pro Gly
   1 5 10
<210> 2758
   <211> 9
   <212> PRT
   <213> Human herpesvirus
<400> 2758
   Gln Leu Leu Gln His Tyr Arg Glu Val
   1 5
<210> 2759
   <211> 18
   <212> PRT
   <213> Homo sapiens
<400> 2759
   Arg Gly Phe Thr Gly Leu Gln Gly Leu Pro Gly Pro Pro Gly Pro Ser
   1 5 10 15
   Gly Asp
<210> 2760
   <211> 9
   <212> PRT
   <213> Unknown
<220>
   <223> epitope involved in rheumatoid arthritis
<400> 2760
   Arg Arg Leu Pro Phe Gly Ser Gln Met
   1 5
<210> 2761
   <211> 15
   <212> PRT
   <213> Homo sapiens
<400> 2761
   Arg Val Ala Glu Gln Leu Arg Ala Tyr Leu Glu Gly Glu Cys Val
   1 5 10 15
<210> 2762
   <211> 19
   <212> PRT
   <213> Homo sapiens
<400> 2762
   Ser Gly Phe Gln Gly Leu Pro Gly Pro Pro Gly Pro Pro Gly Glu Gly
   1 5 10 15
   Gly Gly Lys
<210> 2763
   <211> 19
   <212> PRT
   <213> Homo sapiens
<400> 2763
   Ser His Gln Glu Ser Thr Arg Gly Arg Ser Arg Gly Arg Ser Gly Arg
   1 5 10 15
   Ser Gly Ser
<210> 2764
   <211> 10
   <212> PRT
   <213> Unknown
<220>
   <223> epitope involved in rheumatoid arthritis
<400> 2764
   Ser Ile Phe Phe Ala Val Val Leu Glu Leu
   1 5 10
<210> 2765
   <211> 10
   <212> PRT
   <213> Unknown
<220>
   <223> epitope involved in rheumatoid arthritis
<400> 2765
   Ser Leu Cys Ile Asn Leu Arg Ala Ala Arg
   1 5 10
<210> 2766
   <211> 11
   <212> PRT
   <213> Unknown
<220>
   <223> epitope involved in rheumatoid arthritis
<400> 2766
   Ser Leu Ile Thr Ala Ala Arg Gly Ala Pro Ser
   1 5 10
<210> 2767
   <211> 17
   <212> PRT
   <213> Homo sapiens
<400> 2767
   Ser Met Asn Val Glu Val Thr Asp Lys Gly Asp Val Pro Glu Gly Tyr
   1 5 10 15
   Lys
<210> 2768
   <211> 17
   <212> PRT
   <213> Homo sapiens
<400> 2768
   Ser Gln Ile Val Asn Asp Phe Gln Lys Gly Asp Ile Ala Glu Gly Tyr
   1 5 10 15
   Ser
<210> 2769
   <211> 10
   <212> PRT
   <213> Unknown
<220>
   <223> epitope involved in rheumatoid arthritis
<400> 2769
   Val Asp Leu Met Thr Ile Arg Ser Cys Arg
   1 5 10
<210> 2770
   <211> 9
   <212> PRT
   <213> Human herpesvirus
<400> 2770
   Val Met Ala Thr Leu Leu Pro Pro Val
   1 5
<210> 2771
   <211> 13
   <212> PRT
   <213> Homo sapiens
<400> 2771
   Trp Asp Arg Glu Thr Gln Ile Cys Lys Ala Lys Ala Gln
   1 5 10
<210> 2772
   <211> 10
   <212> PRT
   <213> Unknown
<220>
   <223> epitope involved in rheumatoid arthritis
<400> 2772
   Trp Phe Thr Ser Trp Glu Arg Asp Trp Trp
   1 5 10
<210> 2773
   <211> 10
   <212> PRT
   <213> Unknown
<220>
   <223> epitope involved in rheumatoid arthritis
<400> 2773
   Trp Pro Pro Ser Phe Trp Tyr His Pro Gln
   1 5 10
<210> 2774
   <211> 10
   <212> PRT
   <213> Unknown
<220>
   <223> epitope involved in rheumatoid arthritis
<400> 2774
   Trp Val Ala Met His Pro Gln Gly Asp Leu
   1 5 10
<210> 2775
   <211> 10
   <212> PRT
   <213> Unknown
<220>
   <223> epitope involved in rheumatoid arthritis
<400> 2775
   Trp Trp Ala Thr Phe Phe Glu Ala Thr Arg
   1 5 10
<210> 2776
   <211> 10
   <212> PRT
   <213> Unknown
<220>
   <223> epitope involved in rheumatoid arthritis
<400> 2776
   Trp Trp Asn Met Phe Gln Met Gly Val Val
   1 5 10
<210> 2777
   <211> 9
   <212> PRT
   <213> Unknown
<220>
   <223> epitope involved in rheumatoid arthritis
<400> 2777
   Ala Ala Phe Ser Glu Leu Pro Leu Gly
   1 5
<210> 2778
   <211> 9
   <212> PRT
   <213> Unknown
<220>
   <223> epitope involved in rheumatoid arthritis
<400> 2778
   Ala Asp Ala Ser Val Pro Val Met Pro
   1 5
<210> 2779
   <211> 15
   <212> PRT
   <213> Bos taurus
<400> 2779
   Ala Gly Phe Lys Gly Glu Gln Gly Pro Lys Gly Glu Pro Gly Pro
   1 5 10 15
<210> 2780
   <211> 18
   <212> PRT
   <213> Homo sapiens
<400> 2780
   Ala Gly Phe Lys Gly Glu Gln Gly Pro Lys Gly Glu Pro Gly Pro Ala
   1 5 10 15
   Gly Pro
<210> 2781
   <211> 18
   <212> PRT
   <213> Homo sapiens
<400> 2781
   Ala Gly Phe Lys Gly Glu Gln Gly Pro Lys Gly Glu Pro Gly Pro Ala
   1               5                   10                  15
   Gly Pro
<210> 2782
   <211> 9
   <212> PRT
   <213> Unknown
<220>
   <223> epitope involved in rheumatoid arthritis
<400> 2782
   Ala Lys Tyr Ser Asn Gln Arg Ala Gln
   1               5
<210> 2783
   <211> 9
   <212> PRT
   <213> Unknown
<220>
   <223> epitope involved in rheumatoid arthritis
<400> 2783
   Ala Leu Ala Arg Leu Met Pro Gly Ser
   1               5
<210> 2784
   <211> 9
   <212> PRT
   <213> Unknown
<220>
   <223> epitope involved in rheumatoid arthritis
<400> 2784
   Ala Pro Arg Arg Leu Phe Asp Ile Ala
   1               5
<210> 2785
   <211> 9
   <212> PRT
   <213> Unknown
<220>
   <223> epitope involved in rheumatoid arthritis
<400> 2785
   Ala Pro Ser Ser Leu Ala Gly Asn Gly
   1 5
<210> 2786
   <211> 9
   <212> PRT
   <213> Unknown
<220>
   <223> epitope involved in rheumatoid arthritis
<400> 2786
   Ala Gln Ala Tyr Asp Leu Ser Pro Ser
   1 5
<210> 2787
   <211> 9
   <212> PRT
   <213> Unknown
<220>
   <223> epitope involved in rheumatoid arthritis
<400> 2787
   Ala Gln Ser Ala Ile Thr Ala Cys Trp
   1 5
<210> 2788
   <211> 9
   <212> PRT
   <213> Unknown
<220>
   <223> epitope involved in rheumatoid arthritis
<400> 2788
   Ala Ser Lys Ala Lys Ala Arg Pro Val
   1 5
<210> 2789
   <211> 9
   <212> PRT
   <213> Unknown
<220>
   <223> epitope involved in rheumatoid arthritis
<400> 2789
   Ala Thr Ala Asp Tyr Pro Gln Lys Ser
   1 5
<210> 2790
   <211> 9
   <212> PRT
   <213> Unknown
<220>
   <223> epitope involved in rheumatoid arthritis
<400> 2790
   Ala Thr Asn Ile Phe Ile Glu Gly Thr
   1 5
<210> 2791
   <211> 9
   <212> PRT
   <213> Unknown
<220>
   <223> epitope involved in rheumatoid arthritis
<400> 2791
   Cys Thr Arg His Pro Asp Cys Gln Ala
   1 5
<210> 2792
   <211> 16
   <212> PRT
   <213> Homo sapiens
<400> 2792
   Asp Asp Thr Gln Phe Val Arg Phe Asp Ser Asp Ala Ala Ser Pro Arg
   1 5 10 15
<210> 2793
   <211> 16
   <212> PRT
   <213> Homo sapiens
<400> 2793
   Asp Leu Arg Ser Trp Thr Ala Ala Asp Thr Ala Ala Gln Ile Thr Gln
   1 5 10 15
<210> 2794
   <211> 17
   <212> PRT
   <213> Homo sapiens
<400> 2794
   Asp Leu Ser Ser Trp Thr Ala Ala Asp Thr Ala Ala Gln Ile Thr Gln
   1 5 10 15
   Arg
<210> 2795
   <211> 15
   <212> PRT
   <213> Homo sapiens
<400> 2795
   Asp Asn Pro Glu Tyr Ser Pro Asp Pro Ser Ile Tyr Ala Tyr Asp
   1 5 10 15
<210> 2796
   <211> 18
   <212> PRT
   <213> Bos taurus
<400> 2796
   Glu Pro Gly Ile Ala Gly Phe Lys Gly Glu Gln Gly Pro Lys Gly Glu
   1 5 10 15
   Pro Gly
<210> 2797
   <211> 9
   <212> PRT
   <213> Unknown
<220>
   <223> epitope involved in rheumatoid arthritis
<400> 2797
   Glu Gln Ala Gln Ser Gln Thr Leu Pro
   1 5
<210> 2798
   <211> 9
   <212> PRT
   <213> Unknown
<220>
   <223> epitope involved in rheumatoid arthritis
<400> 2798
   Glu Val Ala His Pro Ser Val Pro Leu
   1 5
<210> 2799
   <211> 15
   <212> PRT
   <213> Homo sapiens
<400> 2799
   Phe Glu Asp Met Ala Lys Ser Asp Lys Ala Arg Tyr Asp Arg Glu
   1 5 10 15
<210> 2800
   <211> 17
   <212> PRT
   <213> Homo sapiens
<400> 2800
   Phe Leu Ala Glu Gly Gly Gly Val Arg Gly Pro Arg Val Val Glu Arg
   1 5 10 15
   His
<210> 2801
   <211> 12
   <212> PRT
   <213> Homo sapiens
<400> 2801
   Phe Val Arg Phe Asp Ser Asp Ala Ala Ser Pro Arg
   1 5 10
<210> 2802
   <211> 11
   <212> PRT
   <213> Homo sapiens
<400> 2802
   Phe Val Arg Phe Asp Ser Asp Ala Ala Ser Gln
   1 5 10
<210> 2803
   <211> 13
   <212> PRT
   <213> Homo sapiens
<400> 2803
   Phe Val Arg Phe Asp Ser Asp Ala Ala Ser Gln Arg Met
   1 5 10
<210> 2804
   <211> 9
   <212> PRT
   <213> Unknown
<220>
   <223> epitope involved in rheumatoid arthritis
<400> 2804
   Gly Ala Ser Gly Asp Ile Ala Lys Gln
   1 5
<210> 2805
   <211> 9
   <212> PRT
   <213> Unknown
<220>
   <223> epitope involved in rheumatoid arthritis
<400> 2805
   Gly Gly Glu Thr Leu Leu His Met Ala
   1 5
<210> 2806
   <211> 15
   <212> PRT
   <213> Homo sapiens
<400> 2806
   Gly Ile Ala Gly Phe Lys Gly Glu Gln Gly Pro Lys Gly Glu Pro
   1 5 10 15
<210> 2807
   <211> 15
   <212> PRT
   <213> Homo sapiens
<400> 2807
   Gly Ile Ala Gly Phe Lys Gly Glu Gln Gly Pro Lys Gly Glu Pro
   1 5 10 15
<210> 2808
   <211> 15
   <212> PRT
   <213> Rattus norvegicus
<400> 2808
   Gly Ile Ala Gly Phe Lys Gly Glu Gln Gly Pro Lys Gly Glu Thr
   1 5 10 15
<210> 2809
   <211> 15
   <212> PRT
   <213> Bos taurus
<400> 2809
   Gly Pro Glu Gly Ala Gln Gly Pro Arg Gly Glu Pro Gly Thr Pro
   1 5 10 15
<210> 2810
   <211> 9
   <212> PRT
   <213> Unknown
<220>
   <223> epitope involved in rheumatoid arthritis
<400> 2810
   Gly Pro Pro Arg Arg Leu Ile Asp Thr
   1               5
<210> 2811
   <211> 9
   <212> PRT
   <213> Unknown
<220>
   <223> epitope involved in rheumatoid arthritis
<400> 2811
   Gly Arg Thr Pro Glu Asp Asn Thr Leu
   1               5
<210> 2812
   <211> 9
   <212> PRT
   <213> Unknown
<220>
   <223> epitope involved in rheumatoid arthritis
<400> 2812
   Gly Trp Pro Val Ala Pro Asp Pro Ala
   1               5
<210> 2813
   <211> 9
   <212> PRT
   <213> Unknown
<220>
   <223> epitope involved in rheumatoid arthritis
<400> 2813
   His Ala Lys Lys Thr Pro Pro Ala Ser
   1               5
<210> 2814
   <211> 9
   <212> PRT
   <213> Unknown
<220>
   <223> epitope involved in rheumatoid arthritis
<400> 2814
   His Glu Leu Gly Asp Ile Ala Ile Ala
   1               5
<210> 2815
   <211> 9
   <212> PRT
   <213> Unknown
<220>
   <223> epitope involved in rheumatoid arthritis
<400> 2815
   His Leu Arg Gln Gly Arg Asn Thr Ala
   1 5
<210> 2816
   <211> 21
   <212> PRT
   <213> Unknown
<220>
   <223> epitope involved in rheumatoid arthritis
<400> 2816
   His Gln Cys His Gln Glu Ser Thr Arg Gly Arg Ser Arg Gly Arg Cys
   1 5 10 15
   Gly Arg Ser Gly Ser
   20
<210> 2817
   <211> 9
   <212> PRT
   <213> Unknown
<220>
   <223> epitope involved in rheumatoid arthritis
<400> 2817
   Ile Arg Arg Ser Glu Thr Pro Arg Ala
   1 5
<210> 2818
   <211> 9
   <212> PRT
   <213> Unknown
<220>
   <223> epitope involved in rheumatoid arthritis
<400> 2818
   Ile Arg Tyr His Thr Asn Trp Ser Ser
   1 5
<210> 2819
   <211> 19
   <212> PRT
   <213> Homo sapiens
<400> 2819
   Ile Tyr Phe Arg Asn Gln Lys Gly His Ser Gly Leu Gln Pro Thr Gly
   1 5 10 15
   Phe Leu Ser
<210> 2820
   <211> 19
   <212> PRT
   <213> Homo sapiens
<400> 2820
   Ile Tyr Phe Arg Asn Gln Lys Gly His Ser Gly Leu Gln Pro Thr Gly
   1 5 10 15
   Leu Leu Ser
<210> 2821
   <211> 15
   <212> PRT
   <213> Homo sapiens
<400> 2821
   Lys Glu Lys Ser Lys Phe Glu Asp Met Ala Lys Ser Asp Lys Ala
   1 5 10 15
<210> 2822
   <211> 1 5
   <212> PRT
   <213> Homo sapiens
<400> 2822
<210> 2823
   <211> 16
   <212> PRT
   <213> Homo sapiens
<400> 2823
   Lys Ile His Ala Arg Glu Ile Phe Asp Ser Arg Gly Asn Pro Thr Val
   1 5 10 15
<210> 2824
   <211> 15
   <212> PRT
   <213> Homo sapiens
<400> 2824
   Lys Lys Gly Lys Lys Lys Asp Pro Asn Ala Pro Lys Arg Pro Pro
   1 5 10 15
<210> 2825
   <211> 9
   <212> PRT
   <213> Unknown
<220>
   <223> epitope involved in rheumatoid arthritis
<400> 2825
   Lys Met Pro Ile Glu Ala Asp Pro Leu
   1 5
<210> 2826
   <211> 15
   <212> PRT
   <213> Homo sapiens
<400> 2826
   Lys Ser Asp Lys Ala Arg Tyr Asp Arg Glu Met Lys Asn Tyr Val
   1 5 10 15
<210> 2827
   <211> 9
   <212> PRT
   <213> Unknown
<220>
   <223> epitope involved in rheumatoid arthritis
<400> 2827
   Lys Ser Gly Pro Ile Asp Ile Ile Gly
   1 5
<210> 2828
   <211> 15
   <212> PRT
   <213> Homo sapiens
<400> 2828
   Lys Thr Met Ser Ala Lys Glu Lys Ser Lys Phe Glu Asp Met Ala
   1 5 10 15
<210> 2829
   <211> 9
   <212> PRT
   <213> Unknown
<220>
   <223> epitope involved in rheumatoid arthritis
<400> 2829
   Leu Gly Leu His Ala Leu Gln Ser Pro
   1 5
<210> 2830
   <211> 9
   <212> PRT
   <213> Unknown
<220>
   <223> epitope involved in rheumatoid arthritis
<400> 2830
   Leu Gly Arg Asp Ala Asn Ala Trp Lys
   1 5
<210> 2831
   <211> 9
   <212> PRT
   <213> Unknown
<220>
   <223> epitope involved in rheumatoid arthritis
<400> 2831
   Leu Leu Arg Pro Val Ala Arg Asp Thr
   1 5
<210> 2832
   <211> 9
   <212> PRT
   <213> Unknown
<220>
   <223> epitope involved in rheumatoid arthritis
<400> 2832
   Leu Asn Thr Ala Lys His Arg Glu Ser
   1 5
<210> 2833
   <211> 13
   <212> PRT
   <213> Homo sapiens
<400> 2833
   Leu Pro Ser Tyr Glu Glu Ala Leu Ser Leu Pro Ser Lys
   1 5 10
<210> 2834
   <211> 9
   <212> PRT
   <213> Unknown
<220>
   <223> epitope involved in rheumatoid arthritis
<400> 2834
   Leu Ser Arg Ala Ser Leu Lys Thr Leu
   1 5
<210> 2835
   <211> 15
   <212> PRT
   <213> Homo sapiens
<400> 2835
   Leu Ser Ser Trp Thr Ala Ala Asp Thr Ala Ala Gln Ile Thr Gln
   1 5 10 15
<210> 2836
   <211> 9
   <212> PRT
   <213> Unknown
<220>
   <223> epitope involved in rheumatoid arthritis
<400> 2836
   Leu Val Gly Thr Pro Gln Lys Thr Lys
   1 5
<210> 2837
   <211> 15
   <212> PRT
   <213> Homo sapiens
<400> 2837
   Met Lys Asn Tyr Val Pro Pro Lys Gly Asp Lys Lys Gly Lys Lys
   1 5 10 15
<210> 2838
   <211> 9
   <212> PRT
   <213> Unknown
<220>
   <223> epitope involved in rheumatoid arthritis
<400> 2838
   Asn Lys Ala Leu Thr Phe Phe Leu Pro
   1 5
<210> 2839
   <211> 9
   <212> PRT
   <213> Unknown
<220>
   <223> epitope involved in rheumatoid arthritis
<400> 2839
   Asn Leu Phe Thr Gln Thr Gly Thr Pro
   1               5
<210> 2840
   <211> 9
   <212> PRT
   <213> Unknown
<220>
   <223> epitope involved in rheumatoid arthritis
<400> 2840
   Asn Asn Ser Phe Glu Lys Val Lys Gln
   1               5
<210> 2841
   <211> 9
   <212> PRT
   <213> Unknown
<220>
   <223> epitope involved in rheumatoid arthritis
<400> 2841
   Asn Ser Ser Pro Tyr Leu Asn Thr Lys
   1               5
<210> 2842
   <211> 9
   <212> PRT
   <213> Unknown
<220>
   <223> epitope involved in rheumatoid arthritis
<400> 2842
   Pro Asp His Leu Pro Arg His Val Ser
   1               5
<210> 2843
   <211> 9
   <212> PRT
   <213> Unknown
<220>
   <223> epitope involved in rheumatoid arthritis
<400> 2843
   Pro Glu Ile Ile Glu Asn Phe Asp Pro
   1 5
<210> 2844
   <211> 9
   <212> PRT
   <213> Unknown
<220>
   <223> epitope involved in rheumatoid arthritis
<400> 2844
   Pro Leu Thr Asn Arg Arg Leu Arg Gly
   1 5
<210> 2845
   <211> 15
   <212> PRT
   <213> Homo sapiens
<400> 2845
   Pro Pro Lys Gly Asp Lys Lys Gly Lys Lys Lys Asp Pro Asn Ala
   1 5 10 15
<210> 2846
   <211> 9
   <212> PRT
   <213> Unknown
<220>
   <223> epitope involved in rheumatoid arthritis
<400> 2846
   Pro Pro Leu Leu Leu Thr Asp Val Thr
   1 5
<210> 2847
   <211> 9
   <212> PRT
   <213> Unknown
<220>
   <223> epitope involved in rheumatoid arthritis
<400> 2847
   Pro Gln Ser Pro Gly Ser Ser Phe Pro
   1 5
<210> 2848
   <211> 9
   <212> PRT
   <213> Unknown
<220>
   <223> epitope involved in rheumatoid arthritis
<400> 2848
   Pro Ser Phe Pro Pro Ala Leu Arg Leu
   1 5
<210> 2849
   <211> 9
   <212> PRT
   <213> Unknown
<220>
   <223> epitope involved in rheumatoid arthritis
<400> 2849
   Pro Val Ser Ser Thr Arg Gly Asn Met
   1 5
<210> 2850
   <211> 9
   <212> PRT
   <213> Unknown
<220>
   <223> epitope involved in rheumatoid arthritis
<400> 2850
   Pro Tyr Pro Phe Ala Gln Lys Arg Leu
   1 5
<210> 2851
   <211> 9
   <212> PRT
   <213> Unknown
<220>
   <223> epitope involved in rheumatoid arthritis
<400> 2851
   Gln Ser Pro Arg Leu Tyr Pro Gly Ser
   1 5
<210> 2852
   <211> 9
   <212> PRT
   <213> Unknown
<220>
   <223> epitope involved in rheumatoid arthritis
<400> 2852
   Arg Gly Gly Ile Arg Arg Gln Ser Leu
   1               5
<210> 2853
   <211> 9
   <212> PRT
   <213> Unknown
<220>
   <223> epitope involved in rheumatoid arthritis
<400> 2853
   Arg Lys Ala Leu Arg Leu Ser Ala Ser
   1               5
<210> 2854
   <211> 9
   <212> PRT
   <213> Unknown
<220>
   <223> epitope involved in rheumatoid arthritis
<400> 2854
   Arg Pro Pro His Ser Ala Ser Trp Arg
   1               5
<210> 2855
   <211> 9
   <212> PRT
   <213> Unknown
<220>
   <223> epitope involved in rheumatoid arthritis
<400> 2855
   Arg Arg Gly Pro Glu Ala His Gln Ala
   1               5
<210> 2856
   <211> 9
   <212> PRT
   <213> Unknown
<220>
   <223> epitope involved in rheumatoid arthritis
<400> 2856
   Arg Arg Arg Val Arg Asn Ser Asp Thr
   1               5
<210> 2857
   <211> 15
   <212> PRT
   <213> Homo sapiens
<400> 2857
   Arg Tyr Asp Arg Glu Met Lys Asn Tyr Val Pro Pro Lys Gly Asp
   1 5 10 15
<210> 2858
   <211> 9
   <212> PRT
   <213> Unknown
<220>
   <223> epitope involved in rheumatoid arthritis
<400> 2858
   Ser Glu Ser Thr Ile Gln Asn Leu Arg
   1 5
<210> 2859
   <211> 14
   <212> PRT
   <213> Homo sapiens
<400> 2859
   Ser His Gln Glu Ser Thr Arg Gly Arg Ser Arg Gly Arg Ser
   1 5 10
<210> 2860
   <211> 19
   <212> PRT
   <213> Homo sapiens
<400> 2860
   Ser His Gln Glu Ser Thr Arg Gly Arg Ser Arg Gly Arg Ser Gly Arg
   1 5 10 15
   Ser Gly Ser
<210> 2861
   <211> 19
   <212> PRT
   <213> Homo sapiens
<400> 2861
   Ser His Gln Glu Ser Thr Arg Gly Arg Ser Arg Gly Arg Ser Gly Arg
   1 5 10 15
   Ser Gly Ser
<210> 2862
   <211> 19
   <212> PRT
   <213> Homo sapiens
<400> 2862
   Ser His Gln Glu Ser Thr Arg Gly Arg Ser Arg Gly Arg Ser Gly Arg
   1 5 10 15
   Ser Gly Ser
<210> 2863
   <211> 19
   <212> PRT
   <213> Homo sapiens
<400> 2863
   Ser His Gln Glu Ser Thr Arg Gly Arg Ser Arg Gly Arg Ser Gly Arg
   1 5 10 15
   Ser Gly Ser
<210> 2864
   <211> 19
   <212> PRT
   <213> Homo sapiens
<400> 2864
   Ser His Gln Glu Ser Thr Arg Gly Arg Ser Arg Gly Arg Ser Gly Arg
   1 5 10 15
   Ser Gly Ser
<210> 2865
   <211> 19
   <212> PRT
   <213> Homo sapiens
<400> 2865
   Ser His Gln Glu Ser Thr Arg Gly Arg Ser Arg Gly Arg Ser Gly Arg
   1 5 10 15
   Ser Gly Ser
<210> 2866
   <211> 19
   <212> PRT
   <213> Homo sapiens
<400> 2866
   Ser His Gln Glu Ser Thr Arg Gly Arg Ser Arg Gly Arg Ser Gly Arg
   1               5                   10                  15
   Ser Gly Ser
<210> 2867
   <211> 19
   <212> PRT
   <213> Homo sapiens
<400> 2867
   Ser His Gln Glu Ser Thr Arg Gly Arg Ser Arg Gly Arg Ser Gly Arg
   1               5                   10                  15
   Ser Gly Ser
<210> 2868
   <211> 9
   <212> PRT
   <213> Unknown
<220>
   <223> epitope involved in rheumatoid arthritis
<400> 2868
   Ser Thr Leu Val Pro Asn Lys Ala Pro
   1               5
<210> 2869
   <211> 16
   <212> PRT
   <213> Homo sapiens
<400> 2869
   Ser Thr Arg Ser Val Ser Ser Ser Ser Tyr Arg Arg Met Phe Gly Gly
   1               5                   10                  15
<210> 2870
   <211> 9
   <212> PRT
   <213> Unknown
<220>
   <223> epitope involved in rheumatoid arthritis
<400> 2870
   Thr Phe Arg Asp Leu His Gln Gln Gln
   1 5
<210> 2871
   <211> 14
   <212> PRT
   <213> Homo sapiens
<400> 2871
   Thr Gly Pro Ser Thr Arg Gly Arg Gln Gly Ser Arg His Glu
   1 5 10
<210> 2872
   <211> 17
   <212> PRT
   <213> Homo sapiens
<400> 2872
   Thr Gly Pro Ser Thr Arg Gly Arg Gln Gly Ser Arg His Glu Gln Ala
   1 5 10 15
   Gln
<210> 2873
   <211> 17
   <212> PRT
   <213> Homo sapiens
<400> 2873
   Thr Gly Pro Ser Thr Arg Gly Arg Gln Gly Ser Arg His Glu Gln Ala
   1 5 10 15
   Gln
<210> 2874
   <211> 18
   <212> PRT
   <213> Homo sapiens
<400> 2874
   Thr Ile His Ala His Pro Gly Ser Arg Arg Gly Gly Arg His Gly Tyr
   1 5 10 15
   His His
<210> 2875
   <211> 10
   <212> PRT
   <213> Unknown
<220>
   <223> epitope involved in rheumatoid arthritis
<400> 2875
   Thr Leu Thr Phe Thr Leu Asn Lys Leu Arg
   1               5                   10
<210> 2876
   <211> 9
   <212> PRT
   <213> Unknown
<220>
   <223> epitope involved in rheumatoid arthritis
<400> 2876
   Thr Pro Asp Ser Leu Gly Arg Pro Ser
   1               5
<210> 2877
   <211> 17
   <212> PRT
   <213> Homo sapiens
<400> 2877
   Val Asp Asp Thr Gln Phe Val Arg Phe Asp Ser Asp Ala Ala Ser Pro
   1               5                   10                  15
   Arg
<210> 2878
   <211> 21
   <212> PRT
   <213> Homo sapiens
<400> 2878
   Val Asp Asp Thr Gln Phe Val Arg Phe Asp Ser Asp Ala Ala Ser Pro
   1               5                   10                  15
   Arg Gly Glu Pro Arg
               20
<210> 2879
   <211> 9
   <212> PRT
   <213> Unknown
<220>
   <223> epitope involved in rheumatoid arthritis
<400> 2879
   Val Pro Val Asp Ala Gly Thr Ala Lys
   1 5
<210> 2880
   <211> 9
   <212> PRT
   <213> Unknown
<220>
   <223> epitope involved in rheumatoid arthritis
<400> 2880
   Tyr Lys Leu Arg Arg Ala Tyr Tyr Ala
   1 5
<210> 2881
   <211> 9
   <212> PRT
   <213> Unknown
<220>
   <223> epitope involved in rheumatoid arthritis
<400> 2881
   Tyr Thr Leu Gln Lys Ala Val Leu Ser
   1 5
<210> 2882
   <211> 11
   <212> PRT
   <213> Rattus norvegicus
<400> 2882
   Ala Arg Gly Leu Thr Gly Arg Pro Gly Asp Ala
   1 5 10
<210> 2883
   <211> 16
   <212> PRT
   <213> Homo sapiens
<400> 2883
   Cys Lys Pro Ile Ser Gly His Asn Ser Leu Phe Trp Tyr Arg Gln Thr
   1 5 10 15
<210> 2884
   <211> 16
   <212> PRT
   <213> Homo sapiens
<400> 2884
   Asp Ala Gly Val Ile Gln Ser Pro Arg His Glu Val Thr Glu Met Gly
   1 5 10 15
<210> 2885
   <211> 7
   <212> PRT
   <213> Homo sapiens
<400> 2885
   Asp Glu Leu Thr Lys Asn Gln
   1 5
<210> 2886
   <211> 7
   <212> PRT
   <213> Homo sapiens
<400> 2886
   Asp Lys Ser Arg Trp Gln Glu
   1 5
<210> 2887
   <211> 10
   <212> PRT
   <213> Homo sapiens
<400> 2887
   Asp Pro Ile Ser Gly His Val Ser Leu Phe
   1 5 10
<210> 2888
   <211> 7
   <212> PRT
   <213> Homo sapiens
<400> 2888
   Asp Ser Asp Gly Ser Phe Phe
   1 5
<210> 2889
   <211> 10
   <212> PRT
   <213> Homo sapiens
<400> 2889
   Glu Ala Leu His Asn His Tyr Gln Lys Ser
   1 5 10
<210> 2890
   <211> 7
   <212> PRT
   <213> Homo sapiens
<400> 2890
   Glu Gly Asn Val Phe Ser Cys
   1               5
<210> 2891
   <211> 7
   <212> PRT
   <213> Homo sapiens
<400> 2891
   Glu Asn Tyr Lys Thr Thr Pro
   1               5
<210> 2892
   <211> 7
   <212> PRT
   <213> Homo sapiens
<400> 2892
   Glu Pro Gln Val Tyr Thr Leu
   1               5
<210> 2893
   <211> 7
   <212> PRT
   <213> Homo sapiens
<400> 2893
   Glu Trp Gly Ser Asn Asp Gly
   1               5
<210> 2894
   <211> 7
   <212> PRT
   <213> Homo sapiens
<400> 2894
   Phe Phe Leu Tyr Ser Lys Leu
   1               5
<210> 2895
   <211> 7
   <212> PRT
   <213> Homo sapiens
<400> 2895
   Phe Leu Tyr Ser Lys Leu Thr
   1               5
<210> 2896
   <211> 15
   <212> PRT
   <213> Bos taurus
<400> 2896
   Gly Glu Arg Gly Ser Pro Gly Ala Val Gly Pro Lys Gly Ser Pro
   1 5 10 15
<210> 2897
   <211> 7
   <212> PRT
   <213> Homo sapiens
<400> 2897
   Gly Leu His Asn His Tyr Thr
   1 5
<210> 2898
   <211> 7
   <212> PRT
   <213> Homo sapiens
<400> 2898
   Gly Asn Val Phe Ser Cys Ser
   1 5
<210> 2899
   <211> 15
   <212> PRT
   <213> Bos taurus
<400> 2899
   Gly Pro Thr Gly Ala Arg Gly Pro Glu Gly Ala Gln Gly Pro Arg
   1 5 10 15
<210> 2900
   <211> 7
   <212> PRT
   <213> Homo sapiens
<400> 2900
   Gly Gln Pro Arg Glu Pro Gln
   1 5
<210> 2901
   <211> 15
   <212> PRT
   <213> Bos taurus
<400> 2901
   Gly Ser Ala Gly Ala Pro Gly Ile Ala Gly Ala Pro Gly Phe Pro
   1 5 10 15
<210> 2902
   <211> 7
   <212> PRT
   <213> Homo sapiens
<400> 2902
   Gly Ser Phe Phe Leu Tyr Ser
   1               5
<210> 2903
   <211> 7
   <212> PRT
   <213> Homo sapiens
<400> 2903
   His Glu Gly Leu His Asn His
   1               5
<210> 2904
   <211> 8
   <212> PRT
   <213> Rattus norvegicus
<400> 2904
   Ile Ala Gly Phe Lys Gly Glu Gln
   1               5
<210> 2905
   <211> 16
   <212> PRT
   <213> Homo sapiens
<400> 2905
   Lys Ile Gln Pro Ser Glu Pro Arg Asp Ser Ala Val Tyr Phe Cys Ala
   1               5                   10                  15
<210> 2906
   <211> 7
   <212> PRT
   <213> Homo sapiens
<400> 2906
   Lys Asn Gln Val Ser Leu Thr
   1               5
<210> 2907
   <211> 10
   <212> PRT
   <213> Homo sapiens
<400> 2907
   Lys Pro Ile Ser Gly His Asn Ser Leu Phe
   1               5                   10
<210> 2908
   <211> 7
   <212> PRT
   <213> Homo sapiens
<400> 2908
   Lys Ser Arg Trp Gln Glu Gly
   1 5
<210> 2909
   <211> 7
   <212> PRT
   <213> Homo sapiens
<400> 2909
   Leu Asp Ser Asp Gly Ser Phe
   1 5
<210> 2910
   <211> 7
   <212> PRT
   <213> Homo sapiens
<400> 2910
   Leu His Asn His Tyr Thr Gln
   1 5
<210> 2911
   <211> 7
   <212> PRT
   <213> Homo sapiens
<400> 2911
   Leu Thr Lys Asn Gln Val Ser
   1 5
<210> 2912
   <211> 11
   <212> PRT
   <213> Homo sapiens
<400> 2912
   Leu Val Gly Pro Arg Gly Glu Arg Gly Phe Pro
   1 5 10
<210> 2913
   <211> 7
   <212> PRT
   <213> Homo sapiens
<400> 2913
   Met His Glu Gly Leu His Asn
   1 5
<210> 2914
   <211> 14
   <212> PRT
   <213> Rattus norvegicus
<400> 2914
   Met Pro Gly Glu Arg Gly Ala Ala Gly Ile Ala Gly Pro Lys
   1 5 10
<210> 2915
   <211> 7
   <212> PRT
   <213> Homo sapiens
<400> 2915
   Asn Gln Val Ser Leu Thr Cys
   1 5
<210> 2916
   <211> 7
   <212> PRT
   <213> Homo sapiens
<400> 2916
   Asn Val Phe Ser Cys Ser Val
   1 5
<210> 2917
   <211> 7
   <212> PRT
   <213> Homo sapiens
<400> 2917
   Asn Tyr Lys Thr Thr Pro Pro
   1 5
<210> 2918
   <211> 7
   <212> PRT
   <213> Homo sapiens
<400> 2918
   Pro Pro Ser Arg Asp Glu Leu
   1 5
<210> 2919
   <211> 7
   <212> PRT
   <213> Homo sapiens
<400> 2919
   Pro Pro Ser Arg Glu Glu Met
   1 5
<210> 2920
   <211> 7
   <212> PRT
   <213> Homo sapiens
<400> 2920
   Pro Ser Arg Asp Glu Leu Thr
   1 5
<210> 2921
   <211> 7
   <212> PRT
   <213> Homo sapiens
<400> 2921
   Pro Ser Arg Glu Glu Met Thr
   1 5
<210> 2922
   <211> 7
   <212> PRT
   <213> Homo sapiens
<400> 2922
   Gln Lys Ser Leu Ser Leu Ser
   1 5
<210> 2923
   <211> 16
   <212> PRT
   <213> Homo sapiens
<400> 2923
   Gln Pro Leu Lys Glu Gln Pro Ala Leu Asn Asp Ser Arg Tyr Cys Leu
   1 5 10 15
<210> 2924
   <211> 7
   <212> PRT
   <213> Homo sapiens
<400> 2924
   Gln Pro Arg Glu Pro Gln Val
   1 5
<210> 2925
   <211> 7
   <212> PRT
   <213> Homo sapiens
<400> 2925
   Gln Val Ser Leu Thr Cys Leu
   1 5
<210> 2926
   <211> 7
   <212> PRT
   <213> Homo sapiens
<400> 2926
   Gln Val Tyr Thr Leu Pro Pro
   1 5
<210> 2927
   <211> 7
   <212> PRT
   <213> Homo sapiens
<400> 2927
   Arg Glu Pro Gln Val Tyr Thr
   1 5
<210> 2928
   <211> 21
   <212> PRT
   <213> Mus musculus
<400> 2928
   Arg Gly Phe Gly Phe Val Thr Phe Ser Ser Met Ala Glu Val Asp Ala
   1 5 10 15
   Ala Met Ala Ala Arg
   20
<210> 2929
   <211> 11
   <212> PRT
   <213> Homo sapiens
<400> 2929
   Arg Ser Leu Asp Phe Gln Ala Thr Thr Met Phe
   1 5 10
<210> 2930
   <211> 7
   <212> PRT
   <213> Homo sapiens
<400> 2930
   Arg Trp Gln Glu Gly Asn Val
   1               5
<210> 2931
   <211> 16
   <212> PRT
   <213> Homo sapiens
<400> 2931
   Ser Ala Thr Phe Trp Gln Asn Pro Arg Asn His Phe Arg Cys Gln Val
   1               5                   10                  15
<210> 2932
   <211> 7
   <212> PRT
   <213> Homo sapiens
<400> 2932
   Ser Cys Ser Val Met His Glu
   1               5
<210> 2933
   <211> 7
   <212> PRT
   <213> Homo sapiens
<400> 2933
   Ser Asp Gly Ser Phe Phe Leu
   1               5
<210> 2934
   <211> 7
   <212> PRT
   <213> Homo sapiens
<400> 2934
   Ser Phe Phe Leu Tyr Ser Lys
   1               5
<210> 2935
   <211> 27
   <212> PRT
   <213> Mus musculus
<400> 2935
   Ser Gly Lys Pro Gly Ala His Val Thr Val Lys Lys Leu Phe Val Gly
   1               5                   10                  15
   Gly Ile Lys Glu Asp Thr Glu Glu His His Leu
               20                  25
<210> 2936
   <211> 19
   <212> PRT
   <213> Homo sapiens
<400> 2936
   Ser His Gln Glu Ser Thr Arg Gly Arg Ser Arg Gly Arg Ser Gly Arg
   1               5                   10                  15
   Ser Gly Ser
<210> 2937
   <211> 7
   <212> PRT
   <213> Homo sapiens
<400> 2937
   Ser Leu Ser Leu Ser Pro Gly
   1               5
<210> 2938
   <211> 7
   <212> PRT
   <213> Homo sapiens
<400> 2938
   Ser Leu Thr Cys Leu Val Lys
   1               5
<210> 2939
   <211> 10
   <212> PRT
   <213> Homo sapiens
<400> 2939
   Ser Pro Arg Ser Gly Asp Leu Ser Val Tyr
   1               5                   10
<210> 2940
   <211> 7
   <212> PRT
   <213> Homo sapiens
<400> 2940
   Ser Arg Glu Glu Met Thr Lys
   1               5
<210> 2941
   <211> 7
   <212> PRT
   <213> Homo sapiens
<400> 2941
   Ser Arg Trp Gln Glu Gly Asn
   1 5
<210> 2942
   <211> 16
   <212> PRT
   <213> Homo sapiens
<400> 2942
   Ser Arg Tyr Cys Leu Ser Ser Arg Leu Arg Val Ser Ala Thr Phe Trp
   1 5 10 15
<210> 2943
   <211> 7
   <212> PRT
   <213> Homo sapiens
<400> 2943
   Ser Val Met His Glu Gly Leu
   1 5
<210> 2944
   <211> 7
   <212> PRT
   <213> Homo sapiens
<400> 2944
   Thr Lys Asn Gln Val Ser Leu
   1 5
<210> 2945
   <211> 7
   <212> PRT
   <213> Homo sapiens
<400> 2945
   Thr Leu Pro Pro Ser Arg Glu
   1 5
<210> 2946
   <211> 7
   <212> PRT
   <213> Homo sapiens
<400> 2946
   Val Glu Trp Gly Ser Asn Asp
   1 5
<210> 2947
   <211> 7
   <212> PRT
   <213> Homo sapiens
<400> 2947
   Val Phe Ser Cys Ser Val Met
   1 5
<210> 2948
   <211> 7
   <212> PRT
   <213> Homo sapiens
<400> 2948
   Val Met His Glu Gly Leu His
   1 5
<210> 2949
   <211> 7
   <212> PRT
   <213> Homo sapiens
<400> 2949
   Val Ser Leu Thr Cys Leu Val
   1 5
<210> 2950
   <211> 16
   <212> PRT
   <213> Homo sapiens
<400> 2950
   Val Tyr Phe Cys Ala Ser Ser Phe Ser Thr Cys Ser Ala Asn Tyr Gly
   1 5 10 15
<210> 2951
   <211> 7
   <212> PRT
   <213> Homo sapiens
<400> 2951
   Val Tyr Thr Leu Pro Pro Ser
   1 5
<210> 2952
   <211> 7
   <212> PRT
   <213> Homo sapiens
<400> 2952
   Trp Gln Glu Gly Asn Val Phe
   1 5
<210> 2953
   <211> 15
   <212> PRT
   <213> Homo sapiens
<400> 2953
   Gly Ile Ala Gly Ala Pro Gly Phe Pro Gly Pro Arg Gly Pro Pro
   1 5 10 15
<210> 2954
   <211> 15
   <212> PRT
   <213> Homo sapiens
<400> 2954
   Gly Ile Pro Gly Ala Lys Gly Ser Ala Gly Ala Pro Gly Ile Ala
   1 5 10 15
<210> 2955
   <211> 20
   <212> PRT
   <213> Homo sapiens
<400> 2955
   Ala Arg Gly His Arg Pro Leu Asp Lys Lys Arg Glu Glu Ala Pro Ser
   1 5 10 15
   Leu Arg Pro Ala
   20
<210> 2956
   <211> 20
   <212> PRT
   <213> Homo sapiens
<400> 2956
   Ala Arg Gly His Arg Pro Leu Asp Lys Lys Arg Glu Glu Ala Pro Ser
   1 5 10 15
   Leu Arg Pro Ala
   20
<210> 2957
   <211> 20
   <212> PRT
   <213> Homo sapiens
<400> 2957
   Ala Arg Gly His Arg Pro Leu Asp Lys Lys Arg Glu Glu Ala Pro Ser
   1 5 10 15
   Leu Arg Pro Ala
   20
<210> 2958
   <211> 20
   <212> PRT
   <213> Homo sapiens
<400> 2958
   Ala Arg Gly His Arg Pro Leu Asp Lys Lys Arg Glu Glu Ala Pro Ser
   1               5                   10                  15
   Leu Arg Pro Ala
               20
<210> 2959
   <211> 20
   <212> PRT
   <213> Homo sapiens
<400> 2959
   Ala Arg Gly His Arg Pro Leu Asp Lys Lys Arg Glu Glu Ala Pro Ser
   1               5                   10                  15
   Leu Arg Pro Ala
               20
<210> 2960
   <211> 20
   <212> PRT
   <213> Homo sapiens
<400> 2960
   Ala Arg Gly His Arg Pro Leu Asp Lys Lys Arg Glu Glu Ala Pro Ser
   1               5                   10                  15
   Leu Arg Pro Ala
               20
<210> 2961
   <211> 20
   <212> PRT
   <213> Homo sapiens
<400> 2961
   Ala Arg Gly His Arg Pro Leu Asp Lys Lys Arg Glu Glu Ala Pro Ser
   1               5                   10                  15
   Leu Arg Pro Ala
               20
<210> 2962
   <211> 20
   <212> PRT
   <213> Homo sapiens
<400> 2962
   Ala Arg Gly His Arg Pro Leu Asp Lys Lys Arg Glu Glu Ala Pro Ser
   1               5                   10                  15
   Leu Arg Pro Ala
               20
<210> 2963
   <211> 20
   <212> PRT
   <213> Homo sapiens
<400> 2963
   Ala Arg Gly His Arg Pro Leu Asp Lys Lys Arg Glu Glu Ala Pro Ser
   1               5                   10                  15
   Leu Arg Pro Ala
               20
<210> 2964
   <211> 20
   <212> PRT
   <213> Homo sapiens
<400> 2964
   Ala Arg Gly His Arg Pro Leu Asp Lys Lys Arg Glu Glu Ala Pro Ser
   1               5                   10                  15
   Leu Arg Pro Ala
               20
<210> 2965
   <211> 20
   <212> PRT
   <213> Homo sapiens
<400> 2965
   Ala Arg Gly His Arg Pro Leu Asp Lys Lys Arg Glu Glu Ala Pro Ser
   1               5                   10                  15
   Leu Arg Pro Ala
               20
<210> 2966
   <211> 20
   <212> PRT
   <213> Homo sapiens
<400> 2966
   Ala Arg Gly His Arg Pro Leu Asp Lys Lys Arg Glu Glu Ala Pro Ser
   1               5                   10                  15
   Leu Arg Pro Ala
               20
<210> 2967
   <211> 20
   <212> PRT
   <213> Homo sapiens
<400> 2967
   Ala Arg Gly His Arg Pro Leu Asp Lys Lys Arg Glu Glu Ala Pro Ser
   1               5                   10                  15
   Leu Arg Pro Ala
               20
<210> 2968
   <211> 20
   <212> PRT
   <213> Homo sapiens
<400> 2968
   Ala Arg Gly His Arg Pro Leu Asp Lys Lys Arg Glu Glu Ala Pro Ser
   1               5                   10                  15
   Leu Arg Pro Ala
               20
<210> 2969
   <211> 20
   <212> PRT
   <213> Homo sapiens
<400> 2969
   Ala Arg Gly His Arg Pro Leu Asp Lys Lys Arg Glu Glu Ala Pro Ser
   1               5                   10                  15
   Leu Arg Pro Ala
               20
<210> 2970
   <211> 20
   <212> PRT
   <213> Homo sapiens
<400> 2970
   Ala Arg Gly His Arg Pro Leu Asp Lys Lys Arg Glu Glu Ala Pro Ser
   1               5                   10                  15
   Leu Arg Pro Ala
               20
<210> 2971
   <211> 21
   <212> PRT
   <213> Unknown
<220>
   <223> epitope involved in rheumatoid arthritis
<400> 2971
   Ala Arg Gly His Arg Pro Leu Asp Lys Lys Arg Glu Glu Ala Pro Ser
   1               5                   10                  15
   Leu Arg Pro Ala Asp
               20
<210> 2972
   <211> 38
   <212> PRT
   <213> Unknown
<220>
   <223> epitope involved in rheumatoid arthritis
<400> 2972
   Ala Arg Gly His Arg Pro Leu Asp Lys Lys Arg Glu Glu Ala Pro Ser
   1               5                   10                  15
   Leu Arg Pro Ala Gly Gly Gly His Ser Thr Lys Arg Gly His Ala Lys
               20                  25                  30
   Ser Arg Pro Val Arg Gly
           35
<210> 2973
   <211> 18
   <212> PRT
   <213> Homo sapiens
<400> 2973
   Phe Phe Ser Ala Arg Gly His Arg Pro Leu Asp Lys Lys Arg Glu Glu
   1               5                   10                  15
   Ala Pro
<210> 2974
   <211> 18
   <212> PRT
   <213> Homo sapiens
<400> 2974
   Phe Phe Ser Ala Arg Gly His Arg Pro Leu Asp Lys Lys Arg Glu Glu
   1 5 10 15
   Ala Pro
<210> 2975
   <211> 18
   <212> PRT
   <213> Homo sapiens
<400> 2975
   Phe Phe Ser Ala Arg Gly His Arg Pro Leu Asp Lys Lys Arg Glu Glu
   1 5 10 15
   Ala Pro
<210> 2976
   <211> 18
   <212> PRT
   <213> Homo sapiens
<400> 2976
   Phe Phe Ser Ala Arg Gly His Arg Pro Leu Asp Lys Lys Arg Glu Glu
   1 5 10 15
   Ala Pro
<210> 2977
   <211> 18
   <212> PRT
   <213> Homo sapiens
<400> 2977
   Phe Phe Ser Ala Arg Gly His Arg Pro Leu Asp Lys Lys Arg Glu Glu
   1 5 10 15
   Ala Pro
<210> 2978
   <211> 18
   <212> PRT
   <213> Homo sapiens
<400> 2978
   Phe Phe Ser Ala Arg Gly His Arg Pro Leu Asp Lys Lys Arg Glu Glu
   1 5 10 15
   Ala Pro
<210> 2979
   <211> 18
   <212> PRT
   <213> Homo sapiens
<400> 2979
   Phe Phe Ser Ala Arg Gly His Arg Pro Leu Asp Lys Lys Arg Glu Glu
   1 5 10 15
   Ala Pro
<210> 2980
   <211> 18
   <212> PRT
   <213> Homo sapiens
<400> 2980
   Phe Phe Ser Ala Arg Gly His Arg Pro Leu Asp Lys Lys Arg Glu Glu
   1 5 10 15
   Ala Pro
<210> 2981
   <211> 24
   <212> PRT
   <213> Unknown
<220>
   <223> epitope involved in rheumatoid arthritis
<400> 2981
   Gly Phe Phe Cys Ala Arg Gly His Arg Pro Leu Asp Lys Lys Arg Glu
   1 5 10 15
   Glu Ala Pro Cys Leu Arg Pro Ala
   20
<210> 2982
   <211> 18
   <212> PRT
   <213> Homo sapiens
<400> 2982
   Gly Asn Val Ser Pro Gly Thr Arg Arg Glu Tyr His Thr Glu Lys Leu
   1 5 10 15
   Val Thr
<210> 2983
   <211> 18
   <212> PRT
   <213> Homo sapiens
<400> 2983
   Gly Asn Val Ser Pro Gly Thr Arg Arg Glu Tyr His Thr Glu Lys Leu
   1 5 10 15
   Val Thr
<210> 2984
   <211> 18
   <212> PRT
   <213> Homo sapiens
<400> 2984
   Gly Asn Val Ser Pro Gly Thr Arg Arg Glu Tyr His Thr Glu Lys Leu
   1 5 10 15
   Val Thr
<210> 2985
   <211> 21
   <212> PRT
   <213> Unknown
<220>
   <223> epitope involved in rheumatoid arthritis
<400> 2985
<210> 2986
   <211> 15
   <212> PRT
   <213> Homo sapiens
<400> 2986
   His Ser Thr Lys Arg Gly His Ala Lys Ser Arg Pro Val Arg Gly
   1 5 10 15
<210> 2987
   <211> 15
   <212> PRT
   <213> Homo sapiens
<400> 2987
   His Ser Thr Lys Arg Gly His Ala Lys Ser Arg Pro Val Arg Gly
   1 5 10 15
<210> 2988
   <211> 15
   <212> PRT
   <213> Homo sapiens
<400> 2988
   His Ser Thr Lys Arg Gly His Ala Lys Ser Arg Pro Val Arg Gly
   1 5 10 15
<210> 2989
   <211> 15
   <212> PRT
   <213> Homo sapiens
<400> 2989
   His Ser Thr Lys Arg Gly His Ala Lys Ser Arg Pro Val Arg Gly
   1 5 10 15
<210> 2990
   <211> 15
   <212> PRT
   <213> Homo sapiens
<400> 2990
   His Ser Thr Lys Arg Gly His Ala Lys Ser Arg Pro Val Arg Gly
   1 5 10 15
<210> 2991
   <211> 15
   <212> PRT
   <213> Homo sapiens
<400> 2991
   His Ser Thr Lys Arg Gly His Ala Lys Ser Arg Pro Val Arg Gly
   1 5 10 15
<210> 2992
   <211> 15
   <212> PRT
   <213> Homo sapiens
<400> 2992
   His Ser Thr Lys Arg Gly His Ala Lys Ser Arg Pro Val Arg Gly
   1 5 10 15
<210> 2993
   <211> 15
   <212> PRT
   <213> Homo sapiens
<400> 2993
   His Ser Thr Lys Arg Gly His Ala Lys Ser Arg Pro Val Arg Gly
   1 5 10 15
<210> 2994
   <211> 36
   <212> PRT
   <213> Unknown
<220>
   <223> epitope involved in rheumatoid arthritis
<400> 2994
   His Ser Thr Lys Arg Gly His Ala Lys Ser Arg Pro Val Arg Gly His
   1 5 10 15
   Gln Cys His Gln Glu Ser Thr Arg Gly Arg Ser Arg Gly Arg Cys Gly
   20 25 30
   Arg Ser Gly Ser
   35
<210> 2995
   <211> 18
   <212> PRT
   <213> Homo sapiens
<400> 2995
   Ile Ala Lys Asp Leu Leu Pro Ser Arg Asp Arg Gln His Leu Pro Leu
   1 5 10 15
   Ile Lys
<210> 2996
   <211> 18
   <212> PRT
   <213> Homo sapiens
<400> 2996
   Ile Ala Lys Asp Leu Leu Pro Ser Arg Asp Arg Gln His Leu Pro Leu
   1 5 10 15
   Ile Lys
<210> 2997
   <211> 18
   <212> PRT
   <213> Homo sapiens
<400> 2997
   Ile Ala Lys Asp Leu Leu Pro Ser Arg Asp Arg Gln His Leu Pro Leu
   1 5 10 15
   Ile Lys
<210> 2998
   <211> 18
   <212> PRT
   <213> Homo sapiens
<400> 2998
   Ile Ala Lys Asp Leu Leu Pro Ser Arg Asp Arg Gln His Leu Pro Leu
   1 5 10 15
   Ile Lys
<210> 2999
   <211> 22
   <212> PRT
   <213> Mus musculus
<400> 2999
   Gln Thr Leu Asp His Asn Thr Met Tyr Trp Tyr Lys Gln Asp Ser Lys
   1 5 10 15
   Lys Leu Leu Lys Ile Met
   20
<210> 3000
   <211> 18
   <212> PRT
   <213> Homo sapiens
<400> 3000
   Arg Ala Arg Pro Ala Lys Ala Ala Ala Thr Gln Lys Lys Val Glu Arg
   1 5 10 15
   Lys Ala
<210> 3001
   <211> 18
   <212> PRT
   <213> Homo sapiens
<400> 3001
   Arg Ala Arg Pro Ala Lys Ala Ala Ala Thr Gln Lys Lys Val Glu Arg
   1 5 10 15
   Lys Ala
<210> 3002
   <211> 18
   <212> PRT
   <213> Homo sapiens
<400> 3002
   Arg Ala Arg Pro Ala Lys Ala Ala Ala Thr Gln Lys Lys Val Glu Arg
   1 5 10 15
   Lys Ala
<210> 3003
   <211> 18
   <212> PRT
   <213> Homo sapiens
<400> 3003
   Arg Ala Arg Pro Ala Lys Ala Ala Ala Thr Gln Lys Lys Val Glu Arg
   1 5 10 15
   Lys Ala
<210> 3004
   <211> 18
   <212> PRT
   <213> Homo sapiens
<400> 3004
   Arg Ala Arg Pro Ala Lys Ala Ala Ala Thr Gln Lys Lys Val Glu Arg
   1 5 10 15
   Lys Ala
<210> 3005
   <211> 18
   <212> PRT
   <213> Homo sapiens
<400> 3005
   Arg Ala Arg Pro Ala Lys Ala Ala Ala Thr Gln Lys Lys Val Glu Arg
   1 5 10 15
   Lys Ala
<210> 3006
   <211> 18
   <212> PRT
   <213> Homo sapiens
<400> 3006
   Ser Gly Ile Gly Thr Leu Asp Gly Phe Arg His Arg His Pro Asp Glu
   1 5 10 15
   Ala Ala
<210> 3007
   <211> 18
   <212> PRT
   <213> Homo sapiens
<400> 3007
   Ser Gly Ile Gly Thr Leu Asp Gly Phe Arg His Arg His Pro Asp Glu
   1 5 10 15
   Ala Ala
<210> 3008
   <211> 18
   <212> PRT
   <213> Homo sapiens
<400> 3008
   Ser Arg Ala Leu Ala Arg Glu Val Asp Leu Lys Asp Tyr Glu Asp Gln
   1 5 10 15
   Gln Lys
<210> 3009
   <211> 18
   <212> PRT
   <213> Homo sapiens
<400> 3009
   Ser Arg Ala Leu Ala Arg Glu Val Asp Leu Lys Asp Tyr Glu Asp Gln
   1 5 10 15
   Gln Lys
<210> 3010
   <211> 18
   <212> PRT
   <213> Homo sapiens
<400> 3010
   Ser Arg Ala Leu Ala Arg Glu Val Asp Leu Lys Asp Tyr Glu Asp Gln
   1               5                   10                  15
   Gln Lys
<210> 3011
   <211> 15
   <212> PRT
   <213> Homo sapiens
<400> 3011
   Gly Asn Pro Gly Thr Asp Gly Ile Pro Gly Ala Lys Gly Ser Ala
   1               5                   10                  15
<210> 3012
   <211> 15
   <212> PRT
   <213> Homo sapiens
<400> 3012
   Gly Pro Ala Gly Ala Ser Gly Asn Pro Gly Thr Asp Gly Ile Pro
   1               5                   10                  15
<210> 3013
   <211> 15
   <212> PRT
   <213> Homo sapiens
<400> 3013
   Gly Pro Arg Gly Glu Pro Gly Thr Pro Gly Ser Pro Gly Pro Ala
   1               5                   10                  15
<210> 3014
   <211> 15
   <212> PRT
   <213> Homo sapiens
<400> 3014
   Gly Thr Pro Gly Ser Pro Gly Pro Ala Gly Ala Ser Gly Asn Pro
   1               5                   10                  15
<210> 3015
   <211> 7
   <212> PRT
   <213> Homo sapiens
<400> 3015
   Ala Gly Glu Lys Val Thr Met
   1 5
<210> 3016
   <211> 7
   <212> PRT
   <213> Homo sapiens
<400> 3016
   Ala His Ile Trp Trp Asn Asp
   1 5
<210> 3017
   <211> 7
   <212> PRT
   <213> Homo sapiens
<400> 3017
   Ala Arg Met Arg Asp Gly Ala
   1 5
<210> 3018
   <211> 7
   <212> PRT
   <213> Homo sapiens
<400> 3018
   Ala Ser Thr Arg Glu Ser Gly
   1 5
<210> 3019
   <211> 7
   <212> PRT
   <213> Homo sapiens
<400> 3019
   Ala Val Tyr Phe Cys His Gln
   1 5
<210> 3020
   <211> 7
   <212> PRT
   <213> Homo sapiens
<400> 3020
   Ala Trp Phe Ala Tyr Trp Gly
   1 5
<210> 3021
   <211> 7
   <212> PRT
   <213> Homo sapiens
<400> 3021
   Ala Tyr Trp Gly Gln Gly Thr
   1               5
<210> 3022
   <211> 7
   <212> PRT
   <213> Homo sapiens
<400> 3022
   Cys Ala Arg Met Arg Asp Gly
   1               5
<210> 3023
   <211> 7
   <212> PRT
   <213> Homo sapiens
<400> 3023
   Cys His Gln Tyr Leu Ser Ser
   1               5
<210> 3024
   <211> 7
   <212> PRT
   <213> Homo sapiens
<400> 3024
   Cys Ser Phe Ser Gly Phe Ser
   1               5
<210> 3025
   <211> 7
   <212> PRT
   <213> Homo sapiens
<400> 3025
   Asp Gly Ala Trp Phe Ala Tyr
   1               5
<210> 3026
   <211> 7
   <212> PRT
   <213> Homo sapiens
<400> 3026
   Asp His Lys Asn Tyr Leu Ala
   1 5
<210> 3027
   <211> 7
   <212> PRT
   <213> Homo sapiens
<400> 3027
   Asp Leu Ala Val Tyr Phe Cys
   1 5
<210> 3028
   <211> 7
   <212> PRT
   <213> Homo sapiens
<400> 3028
   Glu Asp Leu Ala Val Tyr Phe
   1 5
<210> 3029
   <211> 7
   <212> PRT
   <213> Homo sapiens
<400> 3029
   Glu Ser Gly Val Pro Asp Arg
   1 5
<210> 3030
   <211> 7
   <212> PRT
   <213> Homo sapiens
<400> 3030
   Glu Trp Leu Ala His Ile Trp
   1 5
<210> 3031
   <211> 7
   <212> PRT
   <213> Homo sapiens
<400> 3031
   Phe Ala Tyr Trp Gly Gln Gly
   1 5
<210> 3032
   <211> 7
   <212> PRT
   <213> Homo sapiens
<400> 3032
   Phe Cys His Gln Tyr Leu Ser
   1 5
<210> 3033
   <211> 7
   <212> PRT
   <213> Homo sapiens
<400> 3033
   Phe Ser Gly Phe Ser Leu Thr
   1 5
<210> 3034
   <211> 7
   <212> PRT
   <213> Homo sapiens
<400> 3034
   Phe Ser Leu Thr Thr Tyr Gly
   1 5
<210> 3035
   <211> 7
   <212> PRT
   <213> Homo sapiens
<400> 3035
   Phe Tyr Asn Thr Val Leu Lys
   1 5
<210> 3036
   <211> 7
   <212> PRT
   <213> Homo sapiens
<400> 3036
   Gly Ala Trp Phe Ala Tyr Trp
   1 5
<210> 3037
   <211> 7
   <212> PRT
   <213> Homo sapiens
<400> 3037
   Gly Phe Ser Leu Thr Thr Tyr
   1 5
<210> 3038
   <211> 7
   <212> PRT
   <213> Homo sapiens
<400> 3038
   Gly Ile Gly Val Gly Trp Ile
   1 5
<210> 3039
   <211> 7
   <212> PRT
   <213> Homo sapiens
<400> 3039
   Gly Lys Gly Leu Glu Trp Leu
   1 5
<210> 3040
   <211> 7
   <212> PRT
   <213> Homo sapiens
<400> 3040
   Gly Leu Glu Trp Leu Ala His
   1 5
<210> 3041
   <211> 15
   <212> PRT
   <213> Bos taurus
<400> 3041
   Gly Pro Ala Gly Pro Ala Gly Thr Ala Gly Ala Arg Gly Ala Pro
   1 5 10 15
<210> 3042
   <211> 7
   <212> PRT
   <213> Homo sapiens
<400> 3042
   Gly Gln Ser Pro Lys Leu Leu
   1 5
<210> 3043
   <211> 7
   <212> PRT
   <213> Homo sapiens
<400> 3043
   Gly Ser Gly Ser Gly Thr Asp
   1 5
<210> 3044
   <211> 7
   <212> PRT
   <213> Homo sapiens
<400> 3044
   Gly Ser Gly Thr Asp Phe Thr
   1               5
<210> 3045
   <211> 7
   <212> PRT
   <213> Homo sapiens
<400> 3045
   Gly Val Gly Trp Ile Arg Gln
   1               5
<210> 3046
   <211> 7
   <212> PRT
   <213> Homo sapiens
<400> 3046
   Gly Val Pro Asp Arg Phe Thr
   1               5
<210> 3047
   <211> 7
   <212> PRT
   <213> Homo sapiens
<400> 3047
   Gly Trp Ile Arg Gln Arg Ser
   1               5
<210> 3048
   <211> 7
   <212> PRT
   <213> Homo sapiens
<400> 3048
   His Ile Trp Trp Asn Asp Asn
   1               5
<210> 3049
   <211> 7
   <212> PRT
   <213> Homo sapiens
<400> 3049
   His Gln Tyr Leu Ser Ser Phe
   1               5
<210> 3050
   <211> 7
   <212> PRT
   <213> Homo sapiens
<400> 3050
   Ile Gly Val Gly Trp Ile Arg
   1               5
<210> 3051
   <211> 7
   <212> PRT
   <213> Homo sapiens
<400> 3051
   Ile Arg Gln Arg Ser Gly Lys
   1               5
<210> 3052
   <211> 7
   <212> PRT
   <213> Homo sapiens
<400> 3052
   Ile Trp Trp Asn Asp Asn Lys
   1               5
<210> 3053
   <211> 7
   <212> PRT
   <213> Homo sapiens
<400> 3053
   Lys Gly Leu Glu Trp Leu Ala
   1               5
<210> 3054
   <211> 7
   <212> PRT
   <213> Homo sapiens
<400> 3054
   Lys Leu Leu Ile Tyr Trp Ala
   1               5
<210> 3055
   <211> 7
   <212> PRT
   <213> Homo sapiens
<400> 3055
   Lys Asn Tyr Leu Ala Trp Tyr
   1 5
<210> 3056
   <211> 7
   <212> PRT
   <213> Homo sapiens
<400> 3056
   Lys Val Thr Met Thr Cys Lys
   1 5
<210> 3057
   <211> 7
   <212> PRT
   <213> Homo sapiens
<400> 3057
   Leu Ala His Ile Trp Trp Asn
   1 5
<210> 3058
   <211> 7
   <212> PRT
   <213> Homo sapiens
<400> 3058
   Leu Ala Val Tyr Phe Cys His
   1 5
<210> 3059
   <211> 7
   <212> PRT
   <213> Homo sapiens
<400> 3059
   Leu Glu Trp Leu Ala His Ile
   1 5
<210> 3060
   <211> 7
   <212> PRT
   <213> Homo sapiens
<400> 3060
   Leu Ile Tyr Trp Ala Ser Thr
   1 5
<210> 3061
   <211> 7
   <212> PRT
   <213> Homo sapiens
<400> 3061
   Leu Leu Ile Tyr Trp Ala Ser
   1 5
<210> 3062
   <211> 7
   <212> PRT
   <213> Homo sapiens
<400> 3062
   Leu Thr Cys Ser Phe Ser Gly
   1 5
<210> 3063
   <211> 7
   <212> PRT
   <213> Homo sapiens
<400> 3063
   Leu Tyr Ser Ser Asp His Lys
   1 5
<210> 3064
   <211> 7
   <212> PRT
   <213> Homo sapiens
<400> 3064
   Met Arg Asp Gly Ala Trp Phe
   1 5
<210> 3065
   <211> 7
   <212> PRT
   <213> Homo sapiens
<400> 3065
   Asn Tyr Leu Ala Trp Tyr Gln
   1 5
<210> 3066
   <211> 7
   <212> PRT
   <213> Homo sapiens
<400> 3066
   Pro Gly Gln Ser Pro Lys Leu
   1 5
<210> 3067
   <211> 7
   <212> PRT
   <213> Homo sapiens
<400> 3067
   Pro Lys Leu Leu Ile Tyr Trp
   1 5
<210> 3068
   <211> 7
   <212> PRT
   <213> Homo sapiens
<400> 3068
   Gln Ser Pro Lys Leu Leu Ile
   1 5
<210> 3069
   <211> 7
   <212> PRT
   <213> Homo sapiens
<400> 3069
   Gln Ser Val Leu Tyr Ser Ser
   1 5
<210> 3070
   <211> 7
   <212> PRT
   <213> Homo sapiens
<400> 3070
   Gln Thr Glu Asp Leu Ala Val
   1 5
<210> 3071
   <211> 7
   <212> PRT
   <213> Homo sapiens
<400> 3071
   Gln Tyr Leu Ser Ser Phe Thr
   1 5
<210> 3072
   <211> 7
   <212> PRT
   <213> Homo sapiens
<400> 3072
   Arg Asp Gly Ala Trp Phe Ala
   1 5
<210> 3073
   <211> 7
   <212> PRT
   <213> Homo sapiens
<400> 3073
   Arg Glu Ser Gly Val Pro Asp
   1 5
<210> 3074
   <211> 7
   <212> PRT
   <213> Homo sapiens
<400> 3074
   Arg Met Arg Asp Gly Ala Trp
   1 5
<210> 3075
   <211> 7
   <212> PRT
   <213> Homo sapiens
<400> 3075
   Ser Ala Gly Glu Lys Val Thr
   1 5
<210> 3076
   <211> 7
   <212> PRT
   <213> Homo sapiens
<400> 3076
   Ser Asp His Lys Asn Tyr Leu
   1 5
<210> 3077
   <211> 7
   <212> PRT
   <213> Homo sapiens
<400> 3077
   Ser Phe Ser Gly Phe Ser Leu
   1 5
<210> 3078
   <211> 7
   <212> PRT
   <213> Homo sapiens
<400> 3078
   Ser Gly Phe Ser Leu Thr Thr
   1 5
<210> 3079
   <211> 7
   <212> PRT
   <213> Homo sapiens
<400> 3079
   Ser Gly Ser Gly Thr Asp Phe
   1 5
<210> 3080
   <211> 7
   <212> PRT
   <213> Homo sapiens
<400> 3080
   Ser Gly Val Pro Asp Arg Phe
   1 5
<210> 3081
   <211> 7
   <212> PRT
   <213> Homo sapiens
<400> 3081
   Ser Leu Thr Thr Tyr Gly Ile
   1 5
<210> 3082
   <211> 7
   <212> PRT
   <213> Homo sapiens
<400> 3082
   Ser Pro Lys Leu Leu Ile Tyr
   1 5
<210> 3083
   <211> 7
   <212> PRT
   <213> Homo sapiens
<400> 3083
   Ser Gln Ser Val Leu Tyr Ser
   1 5
<210> 3084
   <211> 7
   <212> PRT
   <213> Homo sapiens
<400> 3084
   Ser Ser Gln Ser Val Leu Tyr
   1 5
<210> 3085
   <211> 7
   <212> PRT
   <213> Homo sapiens
<400> 3085
   Ser Thr Arg Glu Ser Gly Val
   1 5
<210> 3086
   <211> 7
   <212> PRT
   <213> Homo sapiens
<400> 3086
   Ser Val Leu Tyr Ser Ser Asp
   1 5
<210> 3087
   <211> 7
   <212> PRT
   <213> Homo sapiens
<400> 3087
   Thr Cys Ser Phe Ser Gly Phe
   1 5
<210> 3088
   <211> 7
   <212> PRT
   <213> Homo sapiens
<400> 3088
   Thr Glu Asp Leu Ala Val Tyr
   1 5
<210> 3089
   <211> 7
   <212> PRT
   <213> Homo sapiens
<400> 3089
   Thr Gly Ser Gly Ser Gly Thr
   1 5
<210> 3090
   <211> 7
   <212> PRT
   <213> Homo sapiens
<400> 3090
   Thr Met Thr Cys Lys Ser Ser
   1 5
<210> 3091
   <211> 7
   <212> PRT
   <213> Homo sapiens
<400> 3091
   Thr Arg Glu Ser Gly Val Pro
   1 5
<210> 3092
   <211> 7
   <212> PRT
   <213> Homo sapiens
<400> 3092
   Thr Thr Tyr Gly Ile Gly Val
   1 5
<210> 3093
   <211> 7
   <212> PRT
   <213> Homo sapiens
<400> 3093
   Thr Tyr Gly Ile Gly Val Gly
   1 5
<210> 3094
   <211> 7
   <212> PRT
   <213> Homo sapiens
<400> 3094
   Thr Tyr Tyr Cys Ala Arg Met
   1 5
<210> 3095
   <211> 7
   <212> PRT
   <213> Homo sapiens
<400> 3095
   Val Gly Trp Ile Arg Gln Arg
   1 5
<210> 3096
   <211> 7
   <212> PRT
   <213> Homo sapiens
<400> 3096
   Val Leu Tyr Ser Ser Asp His
   1               5
<210> 3097
   <211> 7
   <212> PRT
   <213> Homo sapiens
<400> 3097
   Val Pro Asp Arg Phe Thr Gly
   1               5
<210> 3098
   <211> 7
   <212> PRT
   <213> Homo sapiens
<400> 3098
   Val Thr Met Thr Cys Lys Ser
   1               5
<210> 3099
   <211> 7
   <212> PRT
   <213> Homo sapiens
<400> 3099
   Val Tyr Phe Cys His Gln Tyr
   1               5
<210> 3100
   <211> 7
   <212> PRT
   <213> Homo sapiens
<400> 3100
   Trp Ala Ser Thr Arg Glu Ser
   1               5
<210> 3101
   <211> 7
   <212> PRT
   <213> Homo sapiens
<400> 3101
   Trp Phe Ala Tyr Trp Gly Gln
   1               5
<210> 3102
   <211> 7
   <212> PRT
   <213> Homo sapiens
<400> 3102
   Trp Ile Arg Gln Arg Ser Gly
   1 5
<210> 3103
   <211> 7
   <212> PRT
   <213> Homo sapiens
<400> 3103
   Trp Leu Ala His Ile Trp Trp
   1 5
<210> 3104
   <211> 7
   <212> PRT
   <213> Homo sapiens
<400> 3104
   Trp Asn Asp Asn Lys Phe Tyr
   1 5
<210> 3105
   <211> 7
   <212> PRT
   <213> Homo sapiens
<400> 3105
   Trp Trp Asn Asp Asn Lys Phe
   1 5
<210> 3106
   <211> 7
   <212> PRT
   <213> Homo sapiens
<400> 3106
   Tyr Cys Ala Arg Met Arg Asp
   1 5
<210> 3107
   <211> 7
   <212> PRT
   <213> Homo sapiens
<400> 3107
   Tyr Phe Cys His Gln Tyr Leu
   1 5
<210> 3108
   <211> 7
   <212> PRT
   <213> Homo sapiens
<400> 3108
   Tyr Gly Ile Gly Val Gly Trp
   1 5
<210> 3109
   <211> 7
   <212> PRT
   <213> Homo sapiens
<400> 3109
   Tyr Leu Ala Trp Tyr Gln Gln
   1 5
<210> 3110
   <211> 7
   <212> PRT
   <213> Homo sapiens
<400> 3110
   Tyr Leu Ser Ser Phe Thr Phe
   1 5
<210> 3111
   <211> 7
   <212> PRT
   <213> Homo sapiens
<400> 3111
   Tyr Ser Ser Asp His Lys Asn
   1 5
<210> 3112
   <211> 7
   <212> PRT
   <213> Homo sapiens
<400> 3112
   Tyr Trp Ala Ser Thr Arg Glu
   1 5
<210> 3113
   <211> 7
   <212> PRT
   <213> Homo sapiens
<400> 3113
   Tyr Tyr Cys Ala Arg Met Arg
   1               5
<210> 3114
   <211> 16
   <212> PRT
   <213> Mycobacterium tuberculosis
<400> 3114
   Ala Ile Leu Thr Gly Gly Gln Val Ile Ser Glu Glu Gly Val Gly Leu
   1               5                   10                  15
<210> 3115
   <211> 16
   <212> PRT
   <213> Mycobacterium tuberculosis
<400> 3115
   Asp Arg Arg Lys Ala Met Leu Gln Asp Met Ala Ile Leu Thr Gly Gly
   1               5                   10                  15
<210> 3116
   <211> 16
   <212> PRT
   <213> Mycobacterium tuberculosis
<400> 3116
   Asp Val Glu Gly Glu Ala Leu Ser Thr Leu Val Val Asn Lys Ile Arg
   1               5                   10                  15
<210> 3117
   <211> 15
   <212> PRT
   <213> Mycobacterium tuberculosis
<400> 3117
   Glu Glu Gly Val Gly Leu Thr Leu Glu Asn Ala Asp Leu Ser Leu
   1               5                   10                  15
<210> 3118
   <211> 11
   <212> PRT
   <213> Mycobacterium tuberculosis
<400> 3118
   Val Val Asn Lys Ile Arg Gly Thr Phe Lys Ser
   1               5                   10
<210> 3119
   <211> 16
   <212> PRT
   <213> Mycobacterium tuberculosis
<400> 3119
   Val Val Asn Lys Ile Arg Gly Thr Phe Lys Ser Val Ala Val Lys Ala
   1               5                   10                  15
<210> 3120
   <211> 13
   <212> PRT
   <213> Homo sapiens
<400> 3120
   Gln Asp Phe Thr Asn Arg Ile Asn Lys Leu Lys Asn Ser
   1               5                   10
<210> 3121
   <211> 8
   <212> PRT
   <213> Streptococcus pyogenes
<400> 3121
   Leu Thr Ser Arg Pro Ala His Gly
   1               5
<210> 3122
   <211> 12
   <212> PRT
   <213> Mus musculus
<400> 3122
   Leu Tyr Phe Cys Ala Ala Arg Ser Asn Tyr Gln Leu
   1               5                   10
<210> 3123
   <211> 15
   <212> PRT
   <213> Rattus norvegicus
<400> 3123
   Ala Leu Ser Thr Leu Val Leu Asn Arg Leu Lys Val Gly Leu Gln
   1               5                   10                  15
<210> 3124
   <211> 15
   <212> PRT
   <213> Homo sapiens
<400> 3124
   Ala Tyr Val Leu Leu Ser Glu Lys Lys Ile Ser Ser Ile Gln Ser
   1               5                   10                  15
<210> 3125
   <211> 15
   <212> PRT
   <213> Homo sapiens
<400> 3125
   Gly Glu Ala Leu Ser Thr Leu Val Leu Asn Arg Leu Lys Val Gly
   1 5 10 15
<210> 3126
   <211> 15
   <212> PRT
   <213> Mycobacterium
<400> 3126
   Gly Glu Ala Leu Ser Thr Leu Val Val Asn Lys Ile Arg Gly Thr
   1 5 10 15
<210> 3127
   <211> 15
   <212> PRT
   <213> Homo sapiens
<400> 3127
   Lys Cys Glu Phe Gln Asp Ala Tyr Val Leu Leu Ser Glu Lys Lys
   1 5 10 15
<210> 3128
   <211> 15
   <212> PRT
   <213> Homo sapiens
<400> 3128
   Val Ala Ser Leu Leu Thr Thr Ala Glu Val Val Val Thr Glu Ile
   1 5 10 15
<210> 3129
   <211> 15
   <212> PRT
   <213> Rattus norvegicus
<400> 3129
   Lys Glu Arg Tyr Ser Thr Leu Tyr Ile Ser Asn Ala Gln Val Glu
   1 5 10 15
<210> 3130
   <211> 15
   <212> PRT
   <213> Homo sapiens
<400> 3130
   Ala Val Cys Arg Thr Ser Met Cys Ser Ile Gln Ser Ala Pro Pro
   1 5 10 15
<210> 3131
   <211> 15
   <212> PRT
   <213> Mus musculus
<400> 3131
   Gly Glu Pro Gly Ile Ala Gly Phe Lys Gly Asp Gln Gly Pro Lys
   1 5 10 15
<210> 3132
   <211> 15
   <212> PRT
   <213> Rattus norvegicus
<400> 3132
   Gly Glu Pro Gly Ile Ala Gly Phe Lys Gly Glu Gln Gly Pro Lys
   1 5 10 15
<210> 3133
   <211> 8
   <212> PRT
   <213> Rattus norvegicus
<400> 3133
   Ile Ala Gly Phe Lys Gly Glu Gln
   1 5
<210> 3134
   <211> 15
   <212> PRT
   <213> Bos taurus
<400> 3134
   Gly Glu Pro Gly Ile Ala Gly Phe Lys Gly Glu Gln Gly Pro Lys
   1 5 10 15
<210> 3135
   <211> 15
   <212> PRT
   <213> Bos taurus
<400> 3135
   Gly Glu Pro Gly Ile Ala Gly Phe Lys Gly Glu Gln Gly Pro Lys
   1 5 10 15
<210> 3136
   <211> 26
   <212> PRT
   <213> Homo sapiens
<400> 3136
   Ala Gly Ala Ala Arg Asp Ala Leu Gln Gly Phe Lys Ile Thr Gln Asn
   1 5 10 15
   Asn Ala Met Lys Ile Ser Phe Ala Lys Lys
   20 25
<210> 3137
   <211> 24
   <212> PRT
   <213> Homo sapiens
<400> 3137
   Glu Pro Ala Val Tyr Phe Lys Glu Gln Phe Leu Asp Gly Asp Gly Trp
   1 5 10 15
   Thr Ser Arg Trp Ile Glu Ser Lys
   20
<210> 3138
   <211> 23
   <212> PRT
   <213> Homo sapiens
<400> 3138
   Gly Arg Gly Arg Gly Arg Gly Arg Gly Arg Gly Arg Gly Arg Gly Arg
   1 5 10 15
   Gly Arg Gly Gly Pro Arg Arg
   20
<210> 3139
   <211> 17
   <212> PRT
   <213> Homo sapiens
<400> 3139
   Lys Ala Leu Ser Val Glu Thr Glu Lys Leu Leu Lys Tyr Leu Glu Ala
   1 5 10 15
   Val
<210> 3140
   <211> 24
   <212> PRT
   <213> Homo sapiens
<400> 3140
   Lys Met Thr Leu Lys Asn Arg Glu Pro Val Gln Leu Glu Thr Leu Ser
   1               5                   10                  15
   Ile Arg Gly Asn Arg Ile Arg Tyr
               20
<210> 3141
   <211> 15
   <212> PRT
   <213> Homo sapiens
<400> 3141
   Lys Pro Lys Ala Ala Lys Pro Lys Lys Ala Ala Ala Lys Lys Lys
   1               5                   10                  15
<210> 3142
   <211> 11
   <212> PRT
   <213> Homo sapiens
<400> 3142
   Met Ala Val Pro Glu Thr Arg Pro Asn His Thr
   1               5                   10
<210> 3143
   <211> 20
   <212> PRT
   <213> Homo sapiens
<400> 3143
   Met Lys Leu Val Arg Phe Leu Met Lys Leu Ser His Glu Thr Val Thr
   1               5                   10                  15
   Ile Glu Leu Lys
               20
<210> 3144
   <211> 17
   <212> PRT
   <213> Enterovirus A
<400> 3144
   Met Val Thr Ser Thr Ile Thr Glu Lys Leu Leu Lys Asn Leu Val Lys
   1               5                   10                  15
   Ile
<210> 3145
   <211> 21
   <212> PRT
   <213> Mus musculus
<400> 3145
   Arg Ile His Met Val Tyr Ser Lys Arg Ser Gly Lys Pro Arg Gly Tyr
   1               5                   10                  15
   Ala Phe Ile Glu Tyr
               20
<210> 3146
   <211> 24
   <212> PRT
   <213> Homo sapiens
<400> 3146
   Ser Gln Phe Gly Gln Ile Leu Asp Ile Leu Val Ser Arg Ser Leu Lys
   1               5                   10                  15
   Met Arg Gly Gln Ala Phe Val Ile
               20
<210> 3147
   <211> 22
   <212> PRT
   <213> Homo sapiens
<400> 3147
   Thr Lys Tyr Lys Gln Arg Asn Gly Trp Ser His Lys Asp Leu Leu Arg
   1               5                   10                  15
   Leu Ser His Leu Lys Pro
               20
<210> 3148
   <211> 20
   <212> PRT
   <213> Homo sapiens
<400> 3148
   Thr Leu His Lys Ala Phe Lys Gly Ser Ile Phe Val Val Phe Asp Ser
   1               5                   10                  15
   Ile Glu Ser Ala
               20
<210> 3149
   <211> 37
   <212> PRT
   <213> Homo sapiens
<400> 3149
   Val Glu Pro Lys Val Lys Ser Lys Lys Arg Glu Ala Val Ala Gly Arg
   1               5                   10                  15
   Gly Arg Gly Arg Gly Arg Gly Arg Gly Arg Gly Arg Gly Arg Gly Arg
               20                  25                  30
   Gly Gly Pro Arg Arg
           35
<210> 3150
   <211> 15
   <212> PRT
   <213> Homo sapiens
<400> 3150
   Ala Ala Arg Gly Arg Gly Arg Gly Met Gly Arg Gly Asn Ile Phe
   1               5                   10                  15
<210> 3151
   <211> 22
   <212> PRT
   <213> Homo sapiens
<400> 3151
   Thr Lys Tyr Lys Gln Arg Asn Gly Trp Ser His Lys Asp Leu Leu Arg
   1               5                   10                  15
   Leu Ser His Leu Lys Pro
               20
<210> 3152
   <211> 22
   <212> PRT
   <213> Homo sapiens
<400> 3152
   Thr Lys Tyr Lys Gln Arg Asn Gly Trp Ser His Lys Asp Leu Leu Arg
   1               5                   10                  15
   Leu Ser His Leu Lys Pro
               20
<210> 3153
   <211> 25
   <212> PRT
   <213> Homo sapiens
<400> 3153
   Val Ala Gly Arg Gly Arg Gly Arg Gly Arg Gly Arg Gly Arg Gly Arg
   1               5                   10                  15
   Gly Arg Gly Arg Gly Gly Pro Arg Arg
               20                  25
<210> 3154
   <211> 18
   <212> PRT
   <213> Homo sapiens
<400> 3154
   Arg Lys Gly His Phe Leu Leu Ser Ser Lys Ser Gly Phe Trp Thr Ile
   1               5                   10                  15
   Trp Leu
<210> 3155
   <211> 22
   <212> PRT
   <213> Mus musculus
<400> 3155
   Ala Gly Arg Gly Arg Gly Arg Gly Arg Gly Arg Gly Arg Gly Arg Gly
   1               5                   10                  15
   Arg Gly Arg Gly Ser Leu
               20
<210> 3156
   <211> 16
   <212> PRT
   <213> Homo sapiens
<400> 3156
   Gly Lys Ala Leu Cys Trp Val Cys Ala Gln Ser Arg Lys His Arg Asp
   1               5                   10                  15
<210> 3157
   <211> 15
   <212> PRT
   <213> Human Endogenous Retrovirus
<400> 3157
   Gly Lys Thr Cys Pro Lys Glu Ile Pro Lys Gly Ser Lys Asn Thr
   1               5                   10                  15
<210> 3158
   <211> 16
   <212> PRT
   <213> Homo sapiens
<400> 3158
   Gly Leu Lys Lys Met Leu Arg Thr Cys Ala Val His Ile Thr Leu Asp
   1               5                   10                  15
<210> 3159
   <211> 49
   <212> PRT
   <213> Mus musculus
<400> 3159
   Pro Leu Phe Pro Ala Gly Arg Gly Arg Gly Arg Gly Arg Gly Arg Gly
   1               5                   10                  15
   Arg Gly Arg Gly Arg Gly Arg Gly Arg Gly Arg Gly Arg Gly Arg Gly
               20                  25                  30
   Arg Gly Arg Gly Arg Gly Arg Gly Arg Gly Arg Gly Ser Leu Ile Tyr
           35                  40                  45
   Ile
<210> 3160
   <211> 9
   <212> PRT
   <213> Mycobacterium bovis
<400> 3160
   Lys Pro Leu Leu Ile Ile Ala Glu Asp
   1               5
<210> 3161
   <211> 10
   <212> PRT
   <213> Human adenovirus
<400> 3161
   Phe Trp Arg Phe Leu Trp Gly Ser Ser Gln
   1               5                   10
<210> 3162
   <211> 20
   <212> PRT
   <213> Unknown
<220>
   <223> epitope involved in rheumatoid arthritis
<400> 3162
   Met Ser Lys Thr Glu Trp Asn Ala Ser Gln Leu Pro Arg Leu Gly Gly
   1 5 10 15
   Lys Glu Asp Arg
   20
<210> 3163
   <211> 20
   <212> PRT
   <213> Unknown
<220>
   <223> epitope involved in rheumatoid arthritis
<400> 3163
   Met Ser Lys Thr Glu Trp Asn Ala Ser Gln Ser Arg Phe Gly Trp Phe
   1 5 10 15
   Glu Asn Lys Glu
   20
<210> 3164
   <211> 11
   <212> PRT
   <213> Influenza A virus
<400> 3164
   Thr Leu Gln Arg Phe Ala Trp Gly Ser Ser Asn
   1 5 10
<210> 3165
   <211> 50
   <212> PRT
   <213> Homo sapiens
<400> 3165
   Gly Tyr Gly Lys Ile Asn Lys Lys Arg Ile Ala Leu Thr Asp Asn Ala
   1 5 10 15
   Leu Ile Ala Arg Ser Leu Gly Lys Tyr Gly Ile Ile Cys Met Glu Asp
   20 25 30
   Leu Ile His Glu Ile Tyr Thr Val Gly Lys Arg Phe Lys Glu Ala Asn
   35 40 45
   Asn Phe
   50
<210> 3166
   <211> 19
   <212> PRT
   <213> Homo sapiens
<400> 3166
   Arg Met Ala Arg Met Ala Arg Lys Ala Gly Asn Phe Tyr Val Pro Ala
   1               5                   10                  15
   Glu Pro Lys
<210> 3167
   <211> 15
   <212> PRT
   <213> Homo sapiens
<400> 3167
   Ala Ala Val Gly Thr Ser Ala Ala Pro Val Pro Ser Asp Asn His
   1               5                   10                  15
<210> 3168
   <211> 15
   <212> PRT
   <213> Proteus mirabilis
<400> 3168
   Phe Ala Glu Ser Arg Ile Arg Arg Glu Thr Ile Ala Ala Glu Asp
   1               5                   10                  15
<210> 3169
   <211> 15
   <212> PRT
   <213> Homo sapiens
<400> 3169
   Gly Ser Leu Asp Ser Leu Arg Arg Glu Ile Glu Gln Met Arg Arg
   1               5                   10                  15
<210> 3170
   <211> 15
   <212> PRT
   <213> Proteus mirabilis
<400> 3170
   Leu Gly Ser Ser Glu Ser Arg Arg Ala Leu Gln Asp Ser Gln Arg
   1               5                   10                  15
<210> 3171
   <211> 5
   <212> PRT
   <213> Homo sapiens
<400> 3171
   Leu Arg Arg Glu Ile
   1 5
<210> 3172
   <211> 16
   <212> PRT
   <213> Homo sapiens
<400> 3172
   Ser Gln Lys Asp Leu Leu Glu Gln Arg Arg Ala Ala Val Asp Thr Tyr
   1 5 10 15
<210> 3173
   <211> 21
   <212> PRT
   <213> Bos taurus
<400> 3173
   Ala Arg Thr Lys Gln Thr Ala Arg Lys Ser Thr Gly Gly Lys Ala Pro
   1 5 10 15
   Arg Lys Gln Leu Ala
   20
<210> 3174
   <211> 15
   <212> PRT
   <213> Homo sapiens
<400> 3174
   Glu Gly Asn Arg Pro Thr Asn Ser Ile Val Phe Thr Lys Leu Thr
   1 5 10 15
<210> 3175
   <211> 15
   <212> PRT
   <213> Homo sapiens
<400> 3175
   Phe Asn Gly Glu Lys Ile Asn Tyr Thr Glu Gly Arg Ala Val Leu
   1 5 10 15
<210> 3176
   <211> 15
   <212> PRT
   <213> Homo sapiens
<400> 3176
   Lys Ile Phe Val Gln Gly Ile Ile Trp Asp Ile Asn Ser Phe Asp
   1 5 10 15
<210> 3177
   <211> 21
   <212> PRT
   <213> Bos taurus
<400> 3177
   Leu Ala Gly Asn Ala Ala Arg Asp Asn Lys Lys Thr Arg Ile Ile Pro
   1 5 10 15
   Arg His Leu Gln Leu
   20
<210> 3178
   <211> 15
   <212> PRT
   <213> Homo sapiens
<400> 3178
   Leu Gly Lys Gln Leu Ala Lys Lys Ile Glu Pro Glu Leu Asp Gly
   1 5 10 15
<210> 3179
   <211> 15
   <212> PRT
   <213> Homo sapiens
<400> 3179
   Met Arg Met Leu Val Asp Leu Ala Lys Ser Arg Gly Val Glu Ala
   1 5 10 15
<210> 3180
   <211> 16
   <212> PRT
   <213> Bos taurus
<400> 3180
   Pro Ala Thr Gly Gly Val Lys Lys Pro His Arg Tyr Arg Pro Gly Thr
   1 5 10 15
<210> 3181
   <211> 13
   <212> PRT
   <213> Homo sapiens
<400> 3181
   Val Glu Ile Arg Ala Glu Gly Asn Ser Arg Phe Thr Tyr
   1 5 10
<210> 3182
   <211> 10
   <212> PRT
   <213> Homo sapiens
<400> 3182
   Ala Ser Ala Ala Arg Leu Thr Met Met Trp
   1 5 10
<210> 3183
   <211> 16
   <212> PRT
   <213> Homo sapiens
<400> 3183
   Leu Phe Lys Pro Pro Lys Tyr His Pro Asp Val Pro Tyr Val Lys Arg
   1 5 10 15
<210> 3184
   <211> 15
   <212> PRT
   <213> Homo sapiens
<400> 3184
   Leu Arg Lys Phe Lys Asn Ile Ser Tyr Phe Pro Gly Lys Leu Arg
   1 5 10 15
<210> 3185
   <211> 17
   <212> PRT
   <213> Homo sapiens
<400> 3185
   Ser Lys Leu Ile Lys Ile Phe Gln Asp His Pro Leu Gln Lys Thr Tyr
   1 5 10 15
   Asn
<210> 3186
   <211> 15
   <212> PRT
   <213> Proteus mirabilis
<400> 3186
   Ala Ser Arg Glu Thr Lys Glu Leu Arg Gln Glu Glu Arg Gln Pro
   1 5 10 15
<210> 3187
   <211> 15
   <212> PRT
   <213> Unknown
<220>
   <223> epitope involved in rheumatoid arthritis
<400> 3187
   Asn Gly Ser Ser Glu Ser Arg Arg Ala Leu Gln Asp Ser Gln Arg
   1 5 10 15
<210> 3188
   <211> 16
   <212> PRT
   <213> Rattus norvegicus
<400> 3188
   Tyr Tyr Val Phe Phe Glu Glu Gln Glu Asp Glu Ile Ile Gly Phe Gly
   1 5 10 15
<210> 3189
   <211> 15
   <212> PRT
   <213> Human herpesvirus
<400> 3189
   Gln Lys Arg Ala Ala Gln Arg Ala Ala Gly Pro Ser Val Ala Ser
   1 5 10 15
<210> 3190
   <211> 15
   <212> PRT
   <213> Lactococcus lactis
<400> 3190
   Gln Lys Arg Ala Ala Tyr Asp Gln Tyr Gly Glu Ala Gly Ala Asn
   1 5 10 15
<210> 3191
   <211> 15
   <212> PRT
   <213> Brucella ovis
<400> 3191
   Gln Lys Arg Ala Ala Tyr Asp Arg Phe Gly His Ala Ala Phe Glu
   1 5 10 15
<210> 3192
   <211> 19
   <212> PRT
   <213> Homo sapiens
<400> 3192
   Leu Pro Asn Phe Ser Ser Leu Asn Leu Arg Glu Thr Asn Leu Asp Ser
   1 5 10 15
   Leu Pro Leu
<210> 3193
   <211> 19
   <212> PRT
   <213> Homo sapiens
<400> 3193
   Leu Pro Asn Phe Ser Ser Leu Asn Leu Arg Glu Thr Asn Leu Asp Ser
   1 5 10 15
   Leu Pro Leu
<210> 3194
   <211> 19
   <212> PRT
   <213> Homo sapiens
<400> 3194
   Ser Ser Arg Ser Tyr Val Thr Thr Ser Thr Arg Thr Tyr Ser Leu Gly
   1 5 10 15
   Ser Ala Leu
<210> 3195
   <211> 7
   <212> PRT
   <213> Unknown
<220>
   <223> epitope involved in rheumatoid arthritis
<400> 3195
   Ala Leu Pro Pro Pro Tyr Met
   1 5
<210> 3196
   <211> 7
   <212> PRT
   <213> Unknown
<220>
   <223> epitope involved in rheumatoid arthritis
<400> 3196
   Glu Ser Met Pro Ala Leu Ser
   1 5
<210> 3197
   <211> 7
   <212> PRT
   <213> Unknown
<220>
   <223> epitope involved in rheumatoid arthritis
<400> 3197
   Gly Thr Asn Thr Ala Gln Lys
   1 5
<210> 3198
   <211> 7
   <212> PRT
   <213> Unknown
<220>
   <223> epitope involved in rheumatoid arthritis
<400> 3198
   Ile Pro Asn Ser Thr His Thr
   1 5
<210> 3199
   <211> 7
   <212> PRT
   <213> Unknown
<220>
   <223> epitope involved in rheumatoid arthritis
<400> 3199
   Lys Trp Ser Phe Thr Pro Pro
   1 5
<210> 3200
   <211> 7
   <212> PRT
   <213> Unknown
<220>
   <223> epitope involved in rheumatoid arthritis
<400> 3200
   Leu His Ser Asp Ala Arg Asp
   1 5
<210> 3201
   <211> 7
   <212> PRT
   <213> Unknown
<220>
   <223> epitope involved in rheumatoid arthritis
<400> 3201
   Leu Asn Thr Thr Leu Lys Val
   1 5
<210> 3202
   <211> 7
   <212> PRT
   <213> Unknown
<220>
   <223> epitope involved in rheumatoid arthritis
<400> 3202
   Leu Pro Tyr Lys Leu Thr Gln
   1 5
<210> 3203
   <211> 7
   <212> PRT
   <213> Unknown
<220>
   <223> epitope involved in rheumatoid arthritis
<400> 3203
   Met Val Pro Glu Leu Thr Glu
   1 5
<210> 3204
   <211> 7
   <212> PRT
   <213> Unknown
<220>
   <223> epitope involved in rheumatoid arthritis
<400> 3204
   Pro Gly Pro Ser Thr Thr Ser
   1 5
<210> 3205
   <211> 16
   <212> PRT
   <213> Homo sapiens
<400> 3205
   Ala Ala Ala Gly Tyr Asp Val Glu Lys Asn Asn Ser Arg Ile Lys Leu
   1 5 10 15
<210> 3206
   <211> 16
   <212> PRT
   <213> Homo sapiens
<400> 3206
   Ala Ala Lys Pro Lys Thr Ala Lys Pro Lys Ala Ala Lys Pro Lys Lys
   1 5 10 15
<210> 3207
   <211> 16
   <212> PRT
   <213> Homo sapiens
<400> 3207
   Ala Gly Ala Ala Lys Arg Lys Ala Ser Gly Pro Pro Val Ser Glu Leu
   1               5                   10                  15
<210> 3208
   <211> 16
   <212> PRT
   <213> Homo sapiens
<400> 3208
   Ala Lys Lys Ala Gly Ala Ala Lys Ala Lys Lys Pro Ala Gly Ala Ala
   1               5                   10                  15
<210> 3209
   <211> 16
   <212> PRT
   <213> Homo sapiens
<400> 3209
   Ala Pro Lys Ser Pro Ala Lys Ala Lys Ala Val Lys Pro Lys Ala Ala
   1               5                   10                  15
<210> 3210
   <211> 16
   <212> PRT
   <213> Homo sapiens
<400> 3210
   Ala Thr Pro Lys Lys Ser Ala Lys Lys Thr Pro Lys Lys Ala Lys Lys
   1               5                   10                  15
<210> 3211
   <211> 16
   <212> PRT
   <213> Homo sapiens
<400> 3211
   Glu Lys Thr Pro Val Lys Lys Lys Ala Arg Lys Ser Ala Gly Ala Ala
   1               5                   10                  15
<210> 3212
   <211> 16
   <212> PRT
   <213> Homo sapiens
<400> 3212
   Ile Lys Leu Gly Leu Lys Ser Leu Val Ser Lys Gly Thr Leu Val Gln
   1 5 10 15
<210> 3213
   <211> 15
   <212> PRT
   <213> Unknown
<220>
   <223> epitope involved in rheumatoid arthritis
<400> 3213
   Ile Trp Tyr Ile Asn Cys Phe Ala Cys Glu Thr His Ala Met Leu
   1 5 10 15
<210> 3214
   <211> 15
   <212> PRT
   <213> Unknown
<220>
   <223> epitope involved in rheumatoid arthritis
<400> 3214
   Ile Trp Tyr Ile Asn Cys Phe Val Cys Glu Thr His Ala Met Leu
   1 5 10 15
<210> 3215
   <211> 15
   <212> PRT
   <213> Unknown
<220>
   <223> epitope involved in rheumatoid arthritis
<400> 3215
   Ile Trp Tyr Ile Ser Cys Phe Gly Cys Glu Thr His Ala Met Leu
   1 5 10 15
<210> 3216
   <211> 15
   <212> PRT
   <213> Unknown
<220>
   <223> epitope involved in rheumatoid arthritis
<400> 3216
   Ile Trp Tyr Ile Thr Cys Phe Gly Cys Glu Thr His Ala Met Leu
   1 5 10 15
<210> 3217
   <211> 16
   <212> PRT
   <213> Homo sapiens
<400> 3217
   Lys Ala Lys Lys Pro Ala Ala Ala Ala Gly Ala Lys Lys Ala Lys Ser
   1               5                   10                  15
<210> 3218
   <211> 17
   <212> PRT
   <213> Homo sapiens
<400> 3218
   Lys Glu Ile Lys Lys Val Ala Thr Pro Lys Lys Ala Ser Lys Pro Lys
   1               5                   10                  15
   Lys
<210> 3219
   <211> 16
   <212> PRT
   <213> Homo sapiens
<400> 3219
   Lys Leu Asn Lys Lys Ala Ala Ser Gly Glu Ala Lys Pro Lys Ala Lys
   1               5                   10                  15
<210> 3220
   <211> 16
   <212> PRT
   <213> Homo sapiens
<400> 3220
   Lys Ser Pro Lys Lys Ala Lys Ala Ala Lys Pro Lys Lys Ala Pro Lys
   1               5                   10                  15
<210> 3221
   <211> 15
   <212> PRT
   <213> Gallus gallus
<400> 3221
   Lys Ser Arg Lys Ala Ser Lys Ala Lys Lys Val Lys Arg Ser Lys
   1               5                   10                  15
<210> 3222
   <211> 16
   <212> PRT
   <213> Homo sapiens
<400> 3222
   Leu Val Gln Thr Lys Gly Thr Gly Ala Ser Gly Ser Phe Lys Leu Asn
   1 5 10 15
<210> 3223
   <211> 16
   <212> PRT
   <213> Homo sapiens
<400> 3223
   Pro Ala Gly Ala Ala Lys Lys Pro Lys Lys Ala Thr Gly Ala Ala Thr
   1 5 10 15
<210> 3224
   <211> 16
   <212> PRT
   <213> Homo sapiens
<400> 3224
   Arg Ser Gly Val Ser Leu Ala Ala Leu Lys Lys Ala Leu Ala Ala Ala
   1 5 10 15
<210> 3225
   <211> 16
   <212> PRT
   <213> Homo sapiens
<400> 3225
   Ser Glu Thr Ala Pro Ala Ala Pro Ala Ala Pro Ala Pro Ala Glu Lys
   1 5 10 15
<210> 3226
   <211> 16
   <212> PRT
   <213> Gallus gallus
<400> 3226
   Ser Lys Pro Arg Ala Lys Ser Gly Ala Arg Lys Ser Pro Lys Lys Lys
   1 5 10 15
<210> 3227
   <211> 31
   <212> PRT
   <213> Gallus gallus
<400> 3227
   Thr Glu Ser Leu Val Leu Ser Pro Ala Pro Ala Lys Pro Lys Arg Val
   1 5 10 15
   Lys Ala Ser Arg Arg Ser Ala Ser His Pro Thr Tyr Ser Glu Met
   20 25 30
<210> 3228
   <211> 16
   <212> PRT
   <213> Homo sapiens
<400> 3228
   Val Ser Glu Leu Ile Thr Lys Ala Val Ala Ala Ser Lys Glu Arg Ser
   1 5 10 15
<210> 3229
   <211> 18
   <212> PRT
   <213> Homo sapiens
<400> 3229
   Cys Ser Ala Gly Trp Leu Ala Asp Arg Ser Val Arg Tyr Pro Ile Ser
   1 5 10 15
   Lys Ala
<210> 3230
   <211> 16
   <212> PRT
   <213> Homo sapiens
<400> 3230
   Ile Pro Cys Tyr Phe Ile Asp Pro Met His Pro Val Thr Thr Ala Pro
   1 5 10 15
<210> 3231
   <211> 16
   <212> PRT
   <213> Homo sapiens
<400> 3231
   Lys Glu Lys Glu Val Val Leu Leu Val Ala Thr Glu Gly Arg Val Arg
   1 5 10 15
<210> 3232
   <211> 18
   <212> PRT
   <213> Homo sapiens
<400> 3232
   Leu Ala Asp Arg Ser Val Arg Tyr Pro Ile Ser Lys Ala Arg Pro Asn
   1 5 10 15
   Cys Gly
<210> 3233
   <211> 16
   <212> PRT
   <213> Homo sapiens
<400> 3233
   Leu Gln Lys Arg Ser Ser Arg His Pro Arg Arg Ser Arg Pro Ser Thr
   1 5 10 15
<210> 3234
   <211> 18
   <212> PRT
   <213> Homo sapiens
<400> 3234
   Gln Ala Gly Met Asp Met Cys Ser Ala Gly Trp Leu Ala Asp Arg Ser
   1 5 10 15
   Val Arg
<210> 3235
   <211> 15
   <212> PRT
   <213> Yersinia enterocolitica
<400> 3235
   Gln Lys Gln Leu Gly Trp Gln Ala Gly Met Asp Glu Ala Arg Thr
   1 5 10 15
<210> 3236
   <211> 16
   <212> PRT
   <213> Homo sapiens
<400> 3236
   Gln Pro Ser Pro Leu Arg Val Leu Leu Gly Thr Ser Leu Thr Ile Pro
   1 5 10 15
<210> 3237
   <211> 16
   <212> PRT
   <213> Homo sapiens
<400> 3237
   Arg Val Leu Leu Gly Thr Ser Leu Thr Ile Pro Cys Tyr Phe Ile Asp
   1 5 10 15
<210> 3238
   <211> 16
   <212> PRT
   <213> Homo sapiens
<400> 3238
   Arg Val Asn Ser Ala Tyr Gln Asp Lys Val Ser Leu Pro Asn Tyr Pro
   1 5 10 15
<210> 3239
   <211> 18
   <212> PRT
   <213> Homo sapiens
<400> 3239
   Thr Thr Gly His Val Tyr Leu Ala Trp Gln Ala Gly Met Asp Met Cys
   1 5 10 15
   Ser Ala
<210> 3240
   <211> 17
   <212> PRT
   <213> Homo sapiens
<400> 3240
   Val Ala Thr Glu Gly Arg Val Arg Val Asn Ser Ala Tyr Gln Asp Lys
   1 5 10 15
   Val
<210> 3241
   <211> 9
   <212> PRT
   <213> Unknown
<220>
   <223> epitope involved in rheumatoid arthritis
<400> 3241
   Ala Ser Ser Leu Leu Ala Ser Ser Pro
   1 5
<210> 3242
   <211> 9
   <212> PRT
   <213> Unknown
<220>
   <223> epitope involved in rheumatoid arthritis
<400> 3242
   Ile Pro Phe Pro Thr Leu Phe Ala Pro
   1 5
<210> 3243
   <211> 9
   <212> PRT
   <213> Unknown
<220>
   <223> epitope involved in rheumatoid arthritis
<400> 3243
   Pro His Glu Ser Asp Ala Thr Val Arg
   1 5
<210> 3244
   <211> 9
   <212> PRT
   <213> Unknown
<220>
   <223> epitope involved in rheumatoid arthritis
<400> 3244
   Pro His Asn Arg Gln Glu Ser Pro Ala
   1 5
<210> 3245
   <211> 9
   <212> PRT
   <213> Unknown
<220>
   <223> epitope involved in rheumatoid arthritis
<400> 3245
   Pro Pro Leu Lys Pro Val Ile Asp Glu
   1 5
<210> 3246
   <211> 9
   <212> PRT
   <213> Unknown
<220>
   <223> epitope involved in rheumatoid arthritis
<400> 3246
   Pro Ser Pro Ser Leu Ser His Pro Leu
   1 5
<210> 3247
   <211> 16
   <212> PRT
   <213> Homo sapiens
<400> 3247
   Lys Ala Thr Leu Val Cys Leu Ile Ser Asp Phe Tyr Pro Gly Ala Val
   1 5 10 15
<210> 3248
   <211> 16
   <212> PRT
   <213> Homo sapiens
<400> 3248
   Ser Gly Leu Gln Ala Glu Asp Glu Ala Asp Tyr Tyr Cys Ser Ser Tyr
   1 5 10 15
<210> 3249
   <211> 16
   <212> PRT
   <213> Homo sapiens
<400> 3249
   Thr Gly Thr Ser Ser Asp Val Gly Gly Tyr Asn Tyr Val Ser Trp Tyr
   1 5 10 15
<210> 3250
   <211> 8
   <212> PRT
   <213> Homo sapiens
<400> 3250
   Glu Val Phe Ile Ser Ala Pro Arg
   1 5
<210> 3251
   <211> 21
   <212> PRT
   <213> Human T-lymphotropic virus
<400> 3251
   Pro Pro Pro Pro Ser Ser Pro Thr His Asp Pro Pro Asp Ser Asp Pro
   1 5 10 15
   Gln Ile Pro Pro Pro
   20
<210> 3252
   <211> 8
   <212> PRT
   <213> Homo sapiens
<400> 3252
   Pro Gln Ile Val Pro Glu Pro Met
   1 5
<210> 3253
   <211> 21
   <212> PRT
   <213> Homo sapiens
<400> 3253
   Pro Thr Arg Ala Pro Ser Gly Pro Arg Pro Pro Ser Arg Ser Gln Ala
   1               5                   10                  15
   Gln Thr Pro Pro Arg
               20
<210> 3254
   <211> 12
   <212> PRT
   <213> Homo sapiens
<400> 3254
   Ala Thr Glu Gly Arg Val Arg Val Asn Ser Ala Tyr
   1               5                   10
<210> 3255
   <211> 6
   <212> PRT
   <213> Homo sapiens
<400> 3255
   Leu Thr Ser Arg Pro Ala
   1               5
<210> 3256
   <211> 20
   <212> PRT
   <213> Homo sapiens
<400> 3256
   Val Val Leu Leu Val Ala Thr Glu Gly Arg Val Arg Val Asn Ser Ala
   1               5                   10                  15
   Tyr Gln Asp Lys
               20
<210> 3257
   <211> 20
   <212> PRT
   <213> Homo sapiens
<400> 3257
   Val Val Leu Leu Val Ala Thr Glu Gly Arg Val Arg Val Asn Ser Ala
   1               5                   10                  15
   Tyr Gln Asp Lys
               20
<210> 3258
   <211> 18
   <212> PRT
   <213> Unknown
<220>
   <223> epitope involved in rheumatoid arthritis
<400> 3258
   Gly Pro Pro Gly Ala Arg Gly Leu Thr Gly Arg Pro Gly Asp Ala Gly
   1               5                   10                  15
   Pro Pro
<210> 3259
   <211> 18
   <212> PRT
   <213> Unknown
<220>
   <223> epitope involved in rheumatoid arthritis
<400> 3259
   Gly Pro Pro Gly Ala Arg Gly Leu Thr Gly Arg Pro Gly Asp Ala Gly
   1               5                   10                  15
   Pro Pro
<210> 3260
   <211> 37
   <212> PRT
   <213> Unknown
<220>
   <223> epitope involved in rheumatoid arthritis
<400> 3260
   Gly Pro Pro Gly Pro Pro Gly Pro Pro Gly Pro Pro Gly Pro Pro Gly
   1               5                   10                  15
   Ala Arg Gly Leu Thr Gly Arg Pro Gly Asp Ala Gly Pro Pro Gly Pro
                   20                  25                  30
   Pro Gly Ala Cys Ala
           35
<210> 3261
   <211> 206
   <212> PRT
   <213> Homo sapiens
<400> 3261
   Pro Ser Val Phe Leu Phe Pro Pro Lys Pro Lys Asp Thr Leu Met Ile
   1               5                   10                  15
   Ser Arg Thr Pro Glu Val Thr Cys Val Val Val Asp Val Ser Gln Glu
               20                  25                  30
   Asp Pro Gln Val Gln Phe Asn Trp Tyr Val Asp Gly Val Gln Val His
           35                  40                  45
   Asn Ala Lys Thr Lys Pro Arg Glu Gln Gln Phe Asn Ser Thr Tyr Arg
       50                  55                  60
   Val Val Ser Val Leu Thr Val Leu His Gln Asn Trp Leu Asp Gly Lys
   65                  70                  75                  80
   Glu Tyr Lys Cys Lys Val Ser Asn Lys Gly Leu Pro Ser Ser Ile Glu
                   85                  90                  95
   Lys Thr Ile Ser Lys Ala Lys Gly Gln Pro Arg Glu Pro Gln Val Tyr
               100                 105                 110
   Thr Leu Pro Pro Ser Gln Glu Glu Met Thr Lys Asn Gln Val Ser Leu
           115                 120                 125
   Thr Cys Leu Val Lys Gly Phe Tyr Pro Ser Asp Ile Ala Val Glu Trp
       130                 135                 140
   Glu Ser Asn Gly Gln Pro Glu Asn Asn Tyr Lys Thr Thr Pro Pro Val
   145                 150                 155                 160
   Leu Asp Ser Asp Gly Ser Phe Phe Leu Tyr Ser Arg Leu Thr Val Asp
                   165                 170                 175
   Lys Ser Arg Trp Gln Glu Gly Asn Val Phe Ser Cys Ser Val Met His
               180                 185                 190
   Glu Ala Leu His Asn His Tyr Thr Gln Lys Ser Leu Ser Leu
             195                 200                 205
<210> 3262
   <211> 9
   <212> PRT
   <213> Burkholderia fungorum
<400> 3262
   Arg Ala Gly Thr Asp Glu Gly Phe Gly
   1               5
<210> 3263
   <211> 20
   <212> PRT
   <213> Homo sapiens
<400> 3263
   Ala Phe Phe Pro Asn Met Val Asn Met Leu Val Leu Gly Lys His Leu
   1               5                   10                  15
   Gly Ile Pro Lys
               20
<210> 3264
   <211> 17
   <212> PRT
   <213> Homo sapiens
<400> 3264
   Ala Lys Tyr Asn Gln Leu Leu Arg Ile Glu Glu Glu Leu Gly Ser Lys
   1               5                   10                  15
   Ala
<210> 3265
   <211> 18
   <212> PRT
   <213> Homo sapiens
<400> 3265
   Ala Asn Gly Trp Gly Val Met Val Ser His Arg Ser Gly Glu Thr Glu
   1               5                   10                  15
   Asp Thr
<210> 3266
   <211> 20
   <212> PRT
   <213> Homo sapiens
<400> 3266
   Ala Pro Asp Arg Lys Gly Phe Arg Leu Leu Leu Ala Ser Pro Arg Ser
   1               5                   10                  15
   Cys Tyr Lys Leu
               20
<210> 3267
   <211> 20
   <212> PRT
   <213> Homo sapiens
<400> 3267
   Cys Leu Glu Glu Lys Val Cys Ser Leu Leu Glu Pro Leu Gly Leu Gln
   1 5 10 15
   Cys Thr Phe Ile
   20
<210> 3268
   <211> 18
   <212> PRT
   <213> Homo sapiens
<400> 3268
   Glu Lys Gly Val Pro Leu Tyr Arg His Ile Ala Asp Leu Ala Gly Asn
   1 5 10 15
   Ser Glu
<210> 3269
   <211> 20
   <212> PRT
   <213> Homo sapiens
<400> 3269
   Glu Pro Leu Gly Leu Gln Cys Thr Phe Ile Asn Asp Phe Phe Thr Tyr
   1 5 10 15
   His Ile Arg His
   20
<210> 3270
   <211> 13
   <212> PRT
   <213> Homo sapiens
<400> 3270
   Glu Thr Thr Glu Glu Ser Leu Arg Asn Tyr Tyr Glu Gln
   1 5 10
<210> 3271
   <211> 14
   <212> PRT
   <213> Homo sapiens
<400> 3271
   Phe Glu Glu Tyr Gly Lys Ile Asp Thr Ile Glu Ile Ile Thr
   1 5 10
<210> 3272
   <211> 20
   <212> PRT
   <213> Homo sapiens
<400> 3272
   Phe Pro Gly Leu Ile Thr Leu Thr Ile Ser Leu Leu Asp Thr Ser Asn
   1 5 10 15
   Leu Glu Leu Pro
   20
<210> 3273
   <211> 20
   <212> PRT
   <213> Homo sapiens
<400> 3273
   Phe Gln Glu Gln Gln Asn Glu Gly His Gly Glu Ala Leu Leu Phe Glu
   1 5 10 15
   Gly Ile Lys Lys
   20
<210> 3274
   <211> 19
   <212> PRT
   <213> Homo sapiens
<400> 3274
   Phe Arg Ser Gly Lys Tyr Asp Leu Asp Phe Lys Ser Pro Asp Asp Pro
   1 5 10 15
   Ser Arg Tyr
<210> 3275
   <211> 12
   <212> PRT
   <213> Homo sapiens
<400> 3275
   Gly Ala Arg Gly Leu Thr Gly Arg Pro Gly Asp Ala
   1 5 10
<210> 3276
   <211> 12
   <212> PRT
   <213> Homo sapiens
<400> 3276
   Gly Ala Arg Gly Leu Thr Gly Arg Pro Gly Asp Ala
   1               5                   10
<210> 3277
   <211> 19
   <212> PRT
   <213> Homo sapiens
<400> 3277
   Ile Gln Val Val Gly Asp Asp Leu Thr Val Thr Asn Pro Lys Arg Ile
   1               5                   10                  15
   Ala Lys Ala
<210> 3278
   <211> 20
   <212> PRT
   <213> Homo sapiens
<400> 3278
   Lys Ala Ala Ser Gly Ser Thr Gly Asp Gln Lys Val Gln Ile Ser Tyr
   1               5                   10                  15
   Tyr Gly Pro Lys
               20
<210> 3279
   <211> 13
   <212> PRT
   <213> Homo sapiens
<400> 3279
   Lys Phe Ala Gly Arg Asn Phe Arg Asn Pro Leu Ala Lys
   1               5                   10
<210> 3280
   <211> 17
   <212> PRT
   <213> Homo sapiens
<400> 3280
   Lys Ile His Ala Arg Glu Ile Phe Asp Ser Arg Gly Asn Pro Thr Val
   1               5                   10                  15
   Glu
<210> 3281
   <211> 17
   <212> PRT
   <213> Homo sapiens
<400> 3281
   Lys Ile His Ala Arg Glu Ile Phe Asp Ser Arg Gly Asn Pro Thr Val
   1               5                   10                  15
   Glu
<210> 3282
   <211> 17
   <212> PRT
   <213> Homo sapiens
<400> 3282
   Lys Ile His Ala Arg Glu Ile Phe Asp Ser Arg Gly Asn Pro Thr Val
   1               5                   10                  15
   Glu
<210> 3283
   <211> 17
   <212> PRT
   <213> Homo sapiens
<400> 3283
   Lys Ile His Ala Arg Glu Ile Phe Asp Ser Arg Gly Asn Pro Thr Val
   1               5                   10                  15
   Glu
<210> 3284
   <211> 17
   <212> PRT
   <213> Porphyromonas gingivalis
<400> 3284
   Lys Ile Ile Gly Arg Glu Ile Leu Asp Ser Arg Gly Asn Pro Thr Val
   1               5                   10                  15
   Glu
<210> 3285
   <211> 20
   <212> PRT
   <213> Homo sapiens
<400> 3285
   Lys Ser Val Thr Thr Leu Ala Met Lys Ala Lys Cys Lys Leu Thr Ile
   1 5 10 15
   Cys Pro Glu Glu
   20
<210> 3286
   <211> 20
   <212> PRT
   <213> Homo sapiens
<400> 3286
   Leu Leu Asp Thr Ser Asn Leu Glu Leu Pro Glu Ala Val Val Phe Gln
   1 5 10 15
   Asp Ser Val Val
   20
<210> 3287
   <211> 20
   <212> PRT
   <213> Homo sapiens
<400> 3287
   Leu Val Asn Cys Asp Arg Asp Asn Leu Glu Ser Ser Ala Met Asp Cys
   1 5 10 15
   Glu Asp Asp Glu
   20
<210> 3288
   <211> 20
   <212> PRT
   <213> Homo sapiens
<400> 3288
   Asn Asp Phe Phe Thr Tyr His Ile Arg His Gly Glu Val His Cys Gly
   1 5 10 15
   Thr Asn Val Arg
   20
<210> 3289
   <211> 18
   <212> PRT
   <213> Homo sapiens
<400> 3289
   Asn Phe Arg Glu Ala Met Arg Ile Gly Ala Glu Val Tyr His Asn Leu
   1               5                   10                  15
   Lys Asn
<210> 3290
   <211> 18
   <212> PRT
   <213> Homo sapiens
<400> 3290
   Asn Pro Lys Arg Ile Ala Lys Ala Val Asn Glu Lys Ser Cys Asn Cys
   1               5                   10                  15
   Leu Leu
<210> 3291
   <211> 20
   <212> PRT
   <213> Homo sapiens
<400> 3291
   Pro Phe Gly Pro Val Ile Asn Gly Arg Cys Cys Leu Glu Glu Lys Val
   1               5                   10                  15
   Cys Ser Leu Leu
               20
<210> 3292
   <211> 20
   <212> PRT
   <213> Homo sapiens
<400> 3292
   Pro Gly Gly Lys His Asn Met Asp Phe Tyr Val Glu Ala Leu Ala Phe
   1               5                   10                  15
   Pro Asp Thr Asp
   20
<210> 3293
   <211> 17
   <212> PRT
   <213> Homo sapiens
<400> 3293
   Arg Asp Tyr Phe Glu Glu Tyr Gly Lys Ile Asp Thr Ile Glu Ile Ile
   1 5 10 15
   Thr
<210> 3294
   <211> 20
   <212> PRT
   <213> Homo sapiens
<400> 3294
   Ser Ile Asn Ala Ser Pro Gly Val Val Val Asp Ile Ala His Ser Pro
   1 5 10 15
   Pro Ala Lys Lys
   20
<210> 3295
   <211> 17
   <212> PRT
   <213> Homo sapiens
<400> 3295
   Val Asp Leu Phe Thr Ser Lys Gly Leu Phe Arg Ala Ala Val Pro Ser
   1 5 10 15
   Gly
<210> 3296
   <211> 20
   <212> PRT
   <213> Homo sapiens
<400> 3296
   Val Glu Ala Leu Ala Phe Pro Asp Thr Asp Phe Pro Gly Leu Ile Thr
   1 5 10 15
   Leu Thr Ile Ser
   20
<210> 3297
   <211> 20
   <212> PRT
   <213> Homo sapiens
<400> 3297
   Val Leu Asp Ser Glu Asp Leu Gln Asp Met Ser Leu Met Thr Leu Ser
   1 5 10 15
   Thr Lys Thr Pro
   20
<210> 3298
   <211> 20
   <212> PRT
   <213> Homo sapiens
<400> 3298
   Val Arg Val Phe Gln Ala Thr Arg Gly Lys Leu Ser Ser Lys Cys Ser
   1 5 10 15
   Val Val Leu Gly
   20
<210> 3299
   <211> 20
   <212> PRT
   <213> Homo sapiens
<400> 3299
   Trp Gly Pro Cys Gly Gln Gly Ala Ile Leu Leu Val Asn Cys Asp Arg
   1 5 10 15
   Asp Asn Leu Glu
   20
<210> 3300
   <211> 21
   <212> PRT
   <213> Homo sapiens
<400> 3300
   Tyr Glu Ala Leu Glu Leu Arg Asp Asn Asp Lys Thr Arg Tyr Met Gly
   1 5 10 15
   Lys Gly Val Ser Lys
   20
<210> 3301
   <211> 25
   <212> PRT
   <213> Homo sapiens
<400> 3301
   Ala Cys Leu Asp Val Gly Ala Val Ile Ala Thr Pro Glu Gln Leu Phe
   1 5 10 15
   Ala Ala Tyr Glu Asp Gly Phe Glu Gln
   20 25
<210> 3302
   <211> 24
   <212> PRT
   <213> Homo sapiens
<400> 3302
   Asp Phe Asp Arg Ala Gln Arg Ala Cys Leu Gln Asn Ser Ala Ile Ile
   1 5 10 15
   Ala Thr Pro Glu Gln Leu Gln Ala
   20
<210> 3303
   <211> 25
   <212> PRT
   <213> Homo sapiens
<400> 3303
   Phe Ala Ala Tyr Glu Asp Gly Phe Glu Gln Cys Asp Ala Gly Trp Leu
   1 5 10 15
   Ala Asp Gln Thr Val Arg Tyr Pro Ile
   20 25
<210> 3304
   <211> 25
   <212> PRT
   <213> Mus musculus
<400> 3304
   Phe Ala Ala Tyr Glu Asp Gly Phe Glu Gln Cys Asp Ala Gly Trp Leu
   1 5 10 15
   Ser Asp Gln Thr Val Arg Tyr Pro Ile
   20 25
<210> 3305
   <211> 25
   <212> PRT
   <213> Homo sapiens
<400> 3305
   Phe Asp Gln Cys Asp Tyr Gly Trp Leu Ser Asp Ala Ser Val Arg His
   1 5 10 15
   Pro Val Thr Val Ala Arg Ala Gln Cys
   20 25
<210> 3306
   <211> 25
   <212> PRT
   <213> Homo sapiens
<400> 3306
   Gly Val Arg Thr Leu Tyr Arg Phe Glu Asn Gln Thr Gly Phe Pro Pro
   1 5 10 15
   Pro Asp Ser Arg Phe Asp Ala Tyr Cys
   20 25
<210> 3307
   <211> 24
   <212> PRT
   <213> Homo sapiens
<400> 3307
   His Gln Cys Asp Ala Gly Trp Leu Ala Asp Gln Thr Val Arg Tyr Pro
   1 5 10 15
   Ile His Thr Pro Arg Glu Gly Cys
   20
<210> 3308
   <211> 23
   <212> PRT
   <213> Mus musculus
<400> 3308
   Ile Ala Ser Pro Glu Gln Leu Phe Ala Ala Tyr Glu Asp Gly Phe Glu
   1 5 10 15
   Gln Cys Asp Ala Gly Trp Leu
   20
<210> 3309
   <211> 23
   <212> PRT
   <213> Mus musculus
<400> 3309
   Ile Ala Thr Pro Glu Gln Leu Gln Ala Ala Tyr Glu Asp Gly Phe His
   1 5 10 15
   Gln Cys Asp Ala Gly Trp Leu
   20
<210> 3310
   <211> 24
   <212> PRT
   <213> Homo sapiens
<400> 3310
   Ile Ala Thr Pro Glu Gln Leu Gln Ala Ala Tyr Glu Asp Gly Phe His
   1 5 10 15
   Gln Cys Asp Ala Gly Trp Leu Ala
   20
<210> 3311
   <211> 24
   <212> PRT
   <213> Homo sapiens
<400> 3311
   Leu Ala Asp Arg Ser Val Arg Tyr Pro Ile Ser Lys Ala Arg Pro Asn
   1 5 10 15
   Cys Gly Gly Asn Leu Leu Gly Val
   20
<210> 3312
   <211> 25
   <212> PRT
   <213> Homo sapiens
<400> 3312
   Leu His Lys Val Lys Val Gly Lys Ser Pro Pro Val Arg Gly Ser Leu
   1 5 10 15
   Ser Gly Lys Val Ser Leu Pro Cys His
   20 25
<210> 3313
   <211> 24
   <212> PRT
   <213> Homo sapiens
<400> 3313
   Pro Ile His Thr Pro Arg Glu Gly Cys Tyr Gly Asp Lys Asp Glu Phe
   1 5 10 15
   Pro Gly Val Arg Thr Tyr Gly Ile
   20
<210> 3314
   <211> 25
   <212> PRT
   <213> Homo sapiens
<400> 3314
   Pro Pro Asp Ser Arg Phe Asp Ala Tyr Cys Phe Lys Arg Arg Met Ser
   1               5                   10                  15
   Asp Leu Ser Val Ile Gly His Pro Ile
               20                  25
<210> 3315
   <211> 25
   <212> PRT
   <213> Mus musculus
<400> 3315
   Gln Ala Ala Tyr Glu Asp Gly Phe His Gln Cys Asp Ala Gly Trp Leu
   1               5                   10                  15
   Ala Asp Gln Thr Val Arg Tyr Pro Ile
               20                  25
<210> 3316
   <211> 25
   <212> PRT
   <213> Homo sapiens
<400> 3316
   Arg Leu Ala Thr Val Gly Glu Leu Gln Ala Ala Trp Arg Asn Gly Phe
   1               5                   10                  15
   Asp Gln Cys Asp Tyr Gly Trp Leu Ser
               20                  25
<210> 3317
   <211> 24
   <212> PRT
   <213> Homo sapiens
<400> 3317
   Arg Val Ser Lys Glu Lys Glu Val Val Leu Leu Val Ala Thr Glu Gly
   1               5                   10                  15
   Arg Val Arg Val Asn Ser Ala Tyr
               20
<210> 3318
   <211> 13
   <212> PRT
   <213> Homo sapiens
<400> 3318
   Ser Asp Leu Ser Val Ile Gly His Pro Ile Asp Ser Glu
   1               5                   10
<210> 3319
   <211> 24
   <212> PRT
   <213> Homo sapiens
<400> 3319
   Tyr Leu Ala Trp Gln Ala Gly Met Asp Met Cys Ser Ala Gly Trp Leu
   1               5                   10                  15
   Ala Asp Arg Ser Val Arg Tyr Pro
               20
<210> 3320
   <211> 20
   <212> PRT
   <213> Mus musculus
<400> 3320
   His Gln Cys His Gln Glu Ser Thr Gly Arg Ser Arg Gly Arg Cys Gly
   1               5                   10                  15
   Arg Ser Gly Ser
               20
<210> 3321
   <211> 21
   <212> PRT
   <213> Mus musculus
<400> 3321
   His Gln Cys His Gln Glu Ser Thr Lys Gly Lys Ser Lys Gly Lys Cys
   1               5                   10                  15
   Gly Lys Ser Gly Ser
               20
<210> 3322
   <211> 21
   <212> PRT
   <213> Mus musculus
<400> 3322
   His Gln Cys His Gln Glu Ser Thr Lys Gly Lys Ser Lys Gly Lys Cys
   1 5 10 15
   Gly Lys Ser Gly Ser
   20
<210> 3323
   <211> 19
   <212> PRT
   <213> Mus musculus
<400> 3323
   Ser His Gln Glu Ser Thr Lys Gly Lys Ser Lys Gly Lys Ser Gly Lys
   1 5 10 15
   Ser Gly Ser
<210> 3324
   <211> 19
   <212> PRT
   <213> Mus musculus
<400> 3324
   Ser His Gln Glu Ser Thr Lys Gly Lys Ser Lys Gly Lys Ser Gly Lys
   1 5 10 15
   Ser Gly Ser
<210> 3325
   <211> 7
   <212> PRT
   <213> Homo sapiens
<400> 3325
   Cys Ser Val Met His Glu Ala
   1 5
<210> 3326
   <211> 7
   <212> PRT
   <213> Unknown
<220>
   <223> epitope involved in rheumatoid arthritis
<400> 3326
   Phe Arg Val Val Ser Val Leu
   1 5
<210> 3327
   <211> 7
   <212> PRT
   <213> Unknown
<220>
   <223> epitope involved in rheumatoid arthritis
<400> 3327
   His Asn Ala Lys Thr Lys Leu
   1 5
<210> 3328
   <211> 7
   <212> PRT
   <213> Homo sapiens
<400> 3328
   Met His Glu Ala Leu His Asn
   1 5
<210> 3329
   <211> 7
   <212> PRT
   <213> Unknown
<220>
   <223> epitope involved in rheumatoid arthritis
<400> 3329
   Pro Arg Glu Gln Gln Phe Asn
   1 5
<210> 3330
   <211> 7
   <212> PRT
   <213> Unknown
<220>
   <223> epitope involved in rheumatoid arthritis
<400> 3330
   Gln Gln Phe Asn Ser Thr Phe
   1 5
<210> 3331
   <211> 7
   <212> PRT
   <213> Homo sapiens
<400> 3331
   Ser Val Met His Glu Ala Leu
   1 5
<210> 3332
   <211> 7
   <212> PRT
   <213> Unknown
<220>
   <223> epitope involved in rheumatoid arthritis
<400> 3332
   Thr Phe Arg Val Val Ser Val
   1 5
<210> 3333
   <211> 7
   <212> PRT
   <213> Homo sapiens
<400> 3333
   Val Leu Thr Val Leu His Gln
   1 5
<210> 3334
   <211> 7
   <212> PRT
   <213> Homo sapiens
<400> 3334
   Val Met His Glu Ala Leu His
   1 5
<210> 3335
   <211> 15
   <212> PRT
   <213> Homo sapiens
<400> 3335
   Asp Lys Lys Arg Glu Glu Ala Pro Ser Leu Arg Pro Ala Pro Pro
   1 5 10 15
<210> 3336
   <211> 15
   <212> PRT
   <213> Homo sapiens
<400> 3336
   Asp Ser Pro Gly Ser Gly Asn Ala Arg Pro Asn Asn Pro Asp Trp
   1 5 10 15
<210> 3337
   <211> 14
   <212> PRT
   <213> Homo sapiens
<400> 3337
   Glu Gln Ser Ala Asp Ser Ser Arg His Ser Gly Ser Gly His
   1 5 10
<210> 3338
   <211> 15
   <212> PRT
   <213> Homo sapiens
<400> 3338
   Glu Arg Gly Ser Ala Gly His Trp Thr Ser Glu Ser Ser Val Ser
   1 5 10 15
<210> 3339
   <211> 14
   <212> PRT
   <213> Homo sapiens
<400> 3339
   Glu Ser Ser Arg Asp Gly Ser Arg His Pro Arg Ser His Asp
   1 5 10
<210> 3340
   <211> 15
   <212> PRT
   <213> Homo sapiens
<400> 3340
   Gly Asp Lys Glu Leu Arg Thr Gly Lys Glu Lys Val Thr Ser Gly
   1 5 10 15
<210> 3341
   <211> 15
   <212> PRT
   <213> Homo sapiens
<400> 3341
   Gly Pro Arg Val Val Glu Arg His Gln Ser Ala Cys Lys Asp Ser
   1 5 10 15
<210> 3342
   <211> 15
   <212> PRT
   <213> Homo sapiens
<400> 3342
   Gly Thr Phe Glu Glu Val Ser Gly Asn Val Ser Pro Gly Thr Arg
   1 5 10 15
<210> 3343
   <211> 15
   <212> PRT
   <213> Homo sapiens
<400> 3343
   Ile Glu Val Leu Lys Arg Lys Val Ile Glu Lys Val Gln His Ile
   1 5 10 15
<210> 3344
   <211> 15
   <212> PRT
   <213> Homo sapiens
<400> 3344
   Leu Glu Asn Leu Arg Ser Lys Ile Gln Lys Leu Glu Ser Asp Val
   1 5 10 15
<210> 3345
   <211> 15
   <212> PRT
   <213> Homo sapiens
<400> 3345
   Leu Leu Gln Gln Glu Arg Pro Ile Arg Asn Ser Val Asp Glu Leu
   1 5 10 15
<210> 3346
   <211> 15
   <212> PRT
   <213> Homo sapiens
<400> 3346
   Met Glu Leu Glu Arg Pro Gly Gly Asn Glu Ile Thr Arg Gly Gly
   1 5 10 15
<210> 3347
   <211> 15
   <212> PRT
   <213> Homo sapiens
<400> 3347
   Asn Arg Cys His Ala Ala Asn Pro Asn Gly Arg Tyr Tyr Trp Gly
   1 5 10 15
<210> 3348
   <211> 15
   <212> PRT
   <213> Homo sapiens
<400> 3348
   Pro Glu Trp Lys Ala Leu Thr Asp Met Pro Gln Met Arg Met Glu
   1 5 10 15
<210> 3349
   <211> 15
   <212> PRT
   <213> Homo sapiens
<400> 3349
   Pro Arg Lys Gln Cys Ser Lys Glu Asp Gly Gly Gly Trp Trp Tyr
   1 5 10 15
<210> 3350
   <211> 15
   <212> PRT
   <213> Homo sapiens
<400> 3350
   Gln Lys Leu Glu Ser Asp Val Ser Ala Gln Met Glu Tyr Cys Arg
   1 5 10 15
<210> 3351
   <211> 15
   <212> PRT
   <213> Homo sapiens
<400> 3351
   Gln Asn Pro Gly Ser Pro Arg Pro Gly Ser Thr Gly Thr Trp Asn
   1 5 10 15
<210> 3352
   <211> 15
   <212> PRT
   <213> Homo sapiens
<400> 3352
   Arg Gly His Ala Lys Ser Arg Pro Val Arg Gly Ile His Thr Ser
   1 5 10 15
<210> 3353
   <211> 15
   <212> PRT
   <213> Homo sapiens
<400> 3353
   Arg Pro Ala Pro Pro Pro Ile Ser Gly Gly Gly Tyr Arg Ala Arg
   1 5 10 15
<210> 3354
   <211> 15
   <212> PRT
   <213> Homo sapiens
<400> 3354
   Ser Cys Ser Arg Ala Leu Ala Arg Glu Val Asp Leu Lys Asp Tyr
   1 5 10 15
<210> 3355
   <211> 15
   <212> PRT
   <213> Homo sapiens
<400> 3355
   Ser Gly Cys Arg Met Lys Gly Leu Ile Asp Glu Val Asn Gln Asp
   1 5 10 15
<210> 3356
   <211> 15
   <212> PRT
   <213> Homo sapiens
<400> 3356
   Ser Gly Ile Gly Thr Leu Asp Gly Phe Arg His Arg His Pro Asp
   1 5 10 15
<210> 3357
   <211> 15
   <212> PRT
   <213> Homo sapiens
<400> 3357
   Ser Arg Gly Ser Glu Ser Gly Ile Phe Thr Asn Thr Lys Glu Ser
   1 5 10 15
<210> 3358
   <211> 15
   <212> PRT
   <213> Homo sapiens
<400> 3358
   Ser Ser His His Pro Gly Ile Ala Glu Phe Pro Ser Arg Gly Lys
   1 5 10 15
<210> 3359
   <211> 15
   <212> PRT
   <213> Homo sapiens
<400> 3359
   Ser Tyr Asn Arg Gly Asp Ser Thr Phe Glu Ser Lys Ser Tyr Lys
   1 5 10 15
<210> 3360
   <211> 14
   <212> PRT
   <213> Homo sapiens
<400> 3360
<210> 3361
   <211> 18
   <212> PRT
   <213> Homo sapiens
<400> 3361
   Thr Ile His Ala His Pro Gly Ser Arg Arg Gly Gly Arg His Gly Tyr
   1 5 10 15
   His His
<210> 3362
   <211> 15
   <212> PRT
   <213> Homo sapiens
<400> 3362
   Thr Thr Arg Arg Ser Cys Ser Lys Thr Val Thr Lys Thr Val Ile
   1 5 10 15
<210> 3363
   <211> 15
   <212> PRT
   <213> Homo sapiens
<400> 3363
   Val Asp Ile Asp Ile Lys Ile Arg Ser Cys Arg Gly Ser Cys Ser
   1 5 10 15
<210> 3364
   <211> 15
   <212> PRT
   <213> Homo sapiens
<400> 3364
   Val Ile Gln Asn Arg Gln Asp Gly Ser Val Asp Phe Gly Arg Lys
   1 5 10 15
<210> 3365
   <211> 15
   <212> PRT
   <213> Homo sapiens
<400> 3365
   Trp Tyr Asn Arg Cys His Ala Ala Asn Pro Asn Gly Arg Tyr Tyr
   1 5 10 15
<210> 3366
   <211> 15
   <212> PRT
   <213> Homo sapiens
<400> 3366
   Trp Tyr Ser Met Arg Lys Met Ser Met Lys Ile Arg Pro Phe Phe
   1 5 10 15
<210> 3367
   <211> 15
   <212> PRT
   <213> Homo sapiens
<400> 3367
   Gly Gly Gly Tyr Arg Ala Arg Pro Ala Lys Ala Ala Ala Thr Gln
   1 5 10 15
<210> 3368
   <211> 15
   <212> PRT
   <213> Homo sapiens
<400> 3368
   Gly Lys Leu Tyr Gly Ile Arg Asp Val Arg Ser Thr Arg Asp Arg
   1 5 10 15
<210> 3369
   <211> 15
   <212> PRT
   <213> Homo sapiens
<400> 3369
   Tyr Ala Glu Tyr His Phe Arg Val Gly Ser Glu Ala Glu Gly Tyr
   1 5 10 15
<210> 3370
   <211> 20
   <212> PRT
   <213> Porphyromonas gingivalis
<400> 3370
   Thr Leu Val Val Asn Arg Leu Arg Gly Ser Leu Lys Ile Cys Ala Val
   1 5 10 15
   Lys Ala Pro Gly
   20
<210> 3371
   <211> 17
   <212> PRT
   <213> Homo sapiens
<400> 3371
   Lys Ile His Ala Arg Glu Ile Phe Asp Ser Arg Gly Asn Pro Thr Val
   1 5 10 15
   Glu
<210> 3372
   <211> 19
   <212> PRT
   <213> Homo sapiens
<400> 3372
   Ala Asn Lys Tyr Gln Ile Ser Val Asn Lys Tyr Arg Gly Thr Ala Gly
   1 5 10 15
   Asn Ala Leu
<210> 3373
   <211> 30
   <212> PRT
   <213> Homo sapiens
<400> 3373
   Lys Tyr Gln Ile Ser Val Asn Lys Tyr Arg Gly Thr Ala Gly Asn Ala
   1 5 10 15
   Leu Met Asp Gly Ala Ser Gln Leu Met Gly Glu Asn Arg Thr
   20 25 30
<210> 3374
   <211> 30
   <212> PRT
   <213> Homo sapiens
<400> 3374
   Lys Tyr Gln Ile Ser Val Asn Lys Tyr Arg Gly Thr Ala Gly Asn Ala
   1 5 10 15
   Leu Met Asp Gly Ala Ser Gln Leu Met Gly Glu Asn Arg Thr
   20 25 30
<210> 3375
   <211> 18
   <212> PRT
   <213> Homo sapiens
<400> 3375
   Asn Lys Tyr Arg Gly Thr Ala Gly Asn Ala Leu Met Asp Gly Ala Ser
   1 5 10 15
   Gln Leu
<210> 3376
   <211> 18
   <212> PRT
   <213> Homo sapiens
<400> 3376
   Asn Lys Tyr Arg Gly Thr Ala Gly Asn Ala Leu Met Asp Gly Ala Ser
   1 5 10 15
   Gln Leu
<210> 3377
   <211> 10
   <212> PRT
   <213> Homo sapiens
<400> 3377
   Leu Val Val Tyr Pro Trp Thr Gln Arg Phe
   1 5 10
<210> 3378
   <211> 18
   <212> PRT
   <213> Homo sapiens
<400> 3378
   Gln Val Thr Gln Pro Thr Val Gly Met Asn Phe Lys Thr Pro Arg Gly
   1 5 10 15
   Pro Val
<210> 3379
   <211> 16
   <212> PRT
   <213> Homo sapiens
<400> 3379
   Arg Leu Pro Ile Ile Asp Val Ala Pro Leu Asp Val Gly Ala Pro Asp
   1 5 10 15
<210> 3380
   <211> 16
   <212> PRT
   <213> Homo sapiens
<400> 3380
   Thr Gln Gly Lys Ile Val Asp Leu Val Lys Glu Leu Asp Arg Asp Thr
   1 5 10 15
<210> 3381
   <211> 9
   <212> PRT
   <213> Human herpesvirus
<400> 3381
   Gly Gln Ser Arg Gly Gln Ser Arg Gly
   1 5
<210> 3382
   <211> 21
   <212> PRT
   <213> Human herpesvirus
<400> 3382
   Gly Gln Ser Arg Gly Gln Ser Arg Gly Arg Gly Arg Gly Arg Gly Arg
   1 5 10 15
   Gly Arg Gly Lys Gly
   20
<210> 3383
   <211> 15
   <212> PRT
   <213> Human herpesvirus
<400> 3383
   Gly Arg Gly Arg Gly Arg Gly Arg Gly Arg Gly Gly Gly Arg Gly
   1 5 10 15
<210> 3384
   <211> 8
   <212> PRT
   <213> Homo sapiens
<400> 3384
   Asp Leu Leu Pro Ser Arg Asp Arg
   1 5
<210> 3385
   <211> 8
   <212> PRT
   <213> Homo sapiens
<400> 3385
   Asp Leu Leu Pro Ser Arg Asp Arg
   1 5
<210> 3386
   <211> 9
   <212> PRT
   <213> Homo sapiens
<400> 3386
   Asp Arg Gln His Leu Pro Leu Ile Lys
   1 5
<210> 3387
   <211> 24
   <212> PRT
   <213> Homo sapiens
<400> 3387
   Glu Glu Ala Pro Ser Leu Arg Pro Ala Pro Pro Pro Ile Ser Gly Gly
   1 5 10 15
   Gly Tyr Arg Ala Arg Pro Ala Lys
   20
<210> 3388
   <211> 24
   <212> PRT
   <213> Homo sapiens
<400> 3388
   Glu Glu Ala Pro Ser Leu Arg Pro Ala Pro Pro Pro Ile Ser Gly Gly
   1 5 10 15
   Gly Tyr Arg Ala Arg Pro Ala Lys
   20
<210> 3389
   <211> 17
   <212> PRT
   <213> Homo sapiens
<400> 3389
   Glu Ser Ser Ser His His Pro Gly Ile Ala Glu Phe Pro Ser Arg Gly
   1 5 10 15
   Lys
<210> 3390
   <211> 15
   <212> PRT
   <213> Homo sapiens
<400> 3390
   Lys Asp Leu Leu Pro Ser Arg Asp Arg Gln His Leu Pro Leu Ile
   1 5 10 15
<210> 3391
   <211> 15
   <212> PRT
   <213> Homo sapiens
<400> 3391
   Lys Asp Leu Leu Pro Ser Arg Asp Arg Gln His Leu Pro Leu Ile
   1 5 10 15
<210> 3392
   <211> 17
   <212> PRT
   <213> Homo sapiens
<400> 3392
   Lys Glu Ser Ser Ser His His Pro Gly Ile Ala Glu Phe Pro Ser Arg
   1 5 10 15
   Gly
<210> 3393
   <211> 18
   <212> PRT
   <213> Homo sapiens
<400> 3393
   Lys Gln Phe Thr Ser Ser Thr Ser Tyr Asn Arg Gly Asp Ser Thr Phe
   1 5 10 15
   Glu Ser
<210> 3394
   <211> 21
   <212> PRT
   <213> Homo sapiens
<400> 3394
   Lys Gln Phe Thr Ser Ser Thr Ser Tyr Asn Arg Gly Asp Ser Thr Phe
   1 5 10 15
   Glu Ser Lys Ser Tyr
   20
<210> 3395
   <211> 25
   <212> PRT
   <213> Homo sapiens
<400> 3395
   Lys Arg Glu Glu Ala Pro Ser Leu Arg Pro Ala Pro Pro Pro Ile Ser
   1 5 10 15
   Gly Gly Gly Tyr Arg Ala Arg Pro Ala
   20 25
<210> 3396
   <211> 18
   <212> PRT
   <213> Homo sapiens
<400> 3396
   Gln Phe Thr Ser Ser Thr Ser Tyr Asn Arg Gly Asp Ser Thr Phe Glu
   1 5 10 15
   Ser Lys
<210> 3397
   <211> 13
   <212> PRT
   <213> Homo sapiens
<400> 3397
   Thr Ser Ser Thr Ser Tyr Asn Arg Gly Asp Ser Thr Phe
   1 5 10
<210> 3398
   <211> 16
   <212> PRT
   <213> Homo sapiens
<400> 3398
   Gly Val Tyr Ala Thr Arg Ser Ser Ala Val Arg Leu Arg Ser Ser Val
   1 5 10 15
<210> 3399
   <211> 16
   <212> PRT
   <213> Homo sapiens
<400> 3399
   Met Ser Thr Arg Ser Val Ser Ser Ser Ser Tyr Arg Arg Met Phe Gly
   1 5 10 15
<210> 3400
   <211> 20
   <212> PRT
   <213> Bos taurus
<400> 3400
   Met Ser Gly Arg Gly Lys Gln Gly Gly Lys Ala Arg Ala Lys Ala Lys
   1 5 10 15
   Thr Arg Ser Ser
   20
<210> 3401
   <211> 3
   <212> PRT
   <213> Unknown
<220>
   <223> epitope involved in rheumatoid arthritis
<400> 3401
   Ala Ala Ala
   1
<210> 3402
   <211> 38
   <212> PRT
   <213> Mus musculus
<400> 3402
   Lys Gly Ser Ile Thr Ser Val Gln Ala Ile Tyr Val Pro Ala Asp Asp
   1 5 10 15
   Leu Thr Asp Pro Ala Pro Ala Thr Thr Phe Ala His Leu Asp Ala Thr
   20 25 30
   Thr Val Leu Ser Arg Ala
   35
<210> 3403
   <211> 16
   <212> PRT
   <213> Mus musculus
<400> 3403
   Lys Leu Val Ile Ile Glu Ser Asp Leu Glu Arg Ala Glu Glu Arg Ala
   1 5 10 15
<210> 3404
   <211> 26
   <212> PRT
   <213> Mus musculus
<400> 3404
   Lys Ser Leu Gln Asp Ile Ile Ala Ile Leu Gly Met Asp Glu Leu Ser
   1 5 10 15
   Glu Glu Asp Lys Leu Thr Val Ser Arg Ala
   20 25
<210> 3405
   <211> 18
   <212> PRT
   <213> Mus musculus
<400> 3405
   Arg Ile Met Asn Val Ile Gly Glu Pro Ile Asp Glu Arg Gly Pro Ile
   1 5 10 15
   Lys Thr
<210> 3406
   <211> 16
   <212> PRT
   <213> Mus musculus
<400> 3406
   Arg Ser Ile Thr Thr Ile Ile Asp Thr Phe His Gln Tyr Ser Arg Lys
   1 5 10 15
<210> 3407
   <211> 32
   <212> PRT
   <213> Mus musculus
<400> 3407
   Arg Thr Thr Gly Ile Val Leu Asp Ser Gly Asp Gly Val Thr His Asn
   1 5 10 15
   Val Pro Ile Tyr Glu Gly Tyr Ala Leu Pro His Ala Ile Met Arg Leu
   20 25 30
<210> 3408
   <211> 16
   <212> PRT
   <213> Mycobacterium tuberculosis
<400> 3408
   Gly Leu Glu Pro Gly Val Val Ala Glu Lys Val Arg Asn Leu Ser Val
   1 5 10 15
<210> 3409
   <211> 18
   <212> PRT
   <213> Homo sapiens
<400> 3409
   Ala Phe Arg Gly Gln Tyr Cys Tyr Glu Leu Asp Glu Lys Ala Val Arg
   1               5                   10                  15
   Pro Gly
<210> 3410
   <211> 16
   <212> PRT
   <213> Homo sapiens
<400> 3410
   Ala Gly Tyr Ala Leu Ala Gln Met Gly Arg Leu Lys Gly Pro Leu Leu
   1               5                   10                  15
<210> 3411
   <211> 17
   <212> PRT
   <213> Homo sapiens
<400> 3411
   Ala Ile Ser Asp Tyr Val Phe Asn Thr Ala Ser Leu Val Tyr His Glu
   1               5                   10                  15
   Glu
<210> 3412
   <211> 17
   <212> PRT
   <213> Homo sapiens
<400> 3412
   Ala Lys Asn Ser Leu Tyr Leu Gln Met Asn Ser Leu Arg Ala Glu Asp
   1               5                   10                  15
   Thr
<210> 3413
   <211> 16
   <212> PRT
   <213> Homo sapiens
<400> 3413
   Ala Leu Ala Thr Ile Ser Thr Leu Glu Ala Val Arg Gly Arg Pro Phe
   1               5                   10                  15
<210> 3414
   <211> 13
   <212> PRT
   <213> Homo sapiens
<400> 3414
   Ala Leu Thr Asn Gly Ile Tyr Pro His Lys Leu Val Phe
   1 5 10
<210> 3415
   <211> 17
   <212> PRT
   <213> Homo sapiens
<400> 3415
   Ala Met Lys Ala Ala Met Ala Leu Glu Lys Lys Leu Asn Gln Ala Leu
   1 5 10 15
   Leu
<210> 3416
   <211> 16
   <212> PRT
   <213> Homo sapiens
<400> 3416
   Ala Asn Gly Val Ile His Met Leu Asp Gly Ile Leu Leu Pro Pro Thr
   1 5 10 15
<210> 3417
   <211> 15
   <212> PRT
   <213> Homo sapiens
<400> 3417
   Ala Asn Leu Glu Leu Ile Asn Thr Trp Val Ala Lys Asn Thr Asn
   1 5 10 15
<210> 3418
   <211> 14
   <212> PRT
   <213> Homo sapiens
<400> 3418
   Ala Pro Glu Leu Leu Phe Phe Ala Lys Arg Tyr Lys Ala Ala
   1 5 10
<210> 3419
   <211> 15
   <212> PRT
   <213> Homo sapiens
<400> 3419
   Ala Pro Arg Leu Leu Ile Tyr Asp Ala Ser Asn Arg Ala Thr Gly
   1 5 10 15
<210> 3420
   <211> 15
   <212> PRT
   <213> Homo sapiens
<400> 3420
   Ala Pro Ser Ala Ile Leu Pro Leu Pro Gly Gln Ser Val Glu Arg
   1 5 10 15
<210> 3421
   <211> 16
   <212> PRT
   <213> Homo sapiens
<400> 3421
   Ala Pro Val Glu Leu Ile Leu Ser Asp Glu Thr Leu Pro Ala Pro Glu
   1 5 10 15
<210> 3422
   <211> 15
   <212> PRT
   <213> Homo sapiens
<400> 3422
   Ala Arg Thr Gln Leu Ser Trp Leu Lys Lys Gln Leu Ala Ala Ala
   1 5 10 15
<210> 3423
   <211> 14
   <212> PRT
   <213> Homo sapiens
<400> 3423
   Ala Ser His Phe Glu Gln Met Ala Ala Ala Ser Met His Arg
   1 5 10
<210> 3424
   <211> 16
   <212> PRT
   <213> Homo sapiens
<400> 3424
   Ala Tyr Pro Arg Ile Ser Val Asn Asn Val Leu Pro Val Phe Asp Asn
   1 5 10 15
<210> 3425
   <211> 24
   <212> PRT
   <213> Homo sapiens
<400> 3425
   Asp Asp Asp Gly Thr Gly Gln Lys Gln Ile Trp Arg Ile Glu Gly Ser
   1 5 10 15
   Asn Lys Val Pro Val Asp Pro Ala
   20
<210> 3426
   <211> 19
   <212> PRT
   <213> Homo sapiens
<400> 3426
   Asp Asp Leu Lys Thr Asp Phe Tyr Lys Asp Leu Thr Ser Leu Gly His
   1 5 10 15
   Asn Glu Asn
<210> 3427
   <211> 16
   <212> PRT
   <213> Homo sapiens
<400> 3427
   Asp Glu Pro Gln Tyr Leu Asp Leu Pro Ser Thr Ala Thr Ser Val Asn
   1 5 10 15
<210> 3428
   <211> 14
   <212> PRT
   <213> Homo sapiens
<400> 3428
   Asp Phe Asn Val Gly Gly Tyr Ile Gln Ala Val Leu Asp Arg
   1 5 10
<210> 3429
   <211> 16
   <212> PRT
   <213> Homo sapiens
<400> 3429
   Asp Gly Lys Arg Ile Gln Tyr Gln Leu Val Asp Ile Ser Gln Asp Asn
   1 5 10 15
<210> 3430
   <211> 17
   <212> PRT
   <213> Homo sapiens
<400> 3430
   Asp Gly Val Arg Glu Val Phe Glu Met Ala Thr Arg Ala Ala Leu Gln
   1 5 10 15
   Ala
<210> 3431
   <211> 15
   <212> PRT
   <213> Homo sapiens
<400> 3431
   Asp His Pro Thr Phe Asn Lys Ile Thr Pro Asn Leu Ala Glu Phe
   1 5 10 15
<210> 3432
   <211> 15
   <212> PRT
   <213> Homo sapiens
<400> 3432
   Asp His Val Lys Leu Val Asn Glu Val Thr Glu Phe Ala Lys Thr
   1 5 10 15
<210> 3433
   <211> 14
   <212> PRT
   <213> Homo sapiens
<400> 3433
   Asp Ile Arg Pro Glu Ile His Glu Asn Tyr Arg Ile Asn Gly
   1 5 10
<210> 3434
   <211> 15
   <212> PRT
   <213> Homo sapiens
<400> 3434
   Asp Lys Val Leu Ile Arg Ile Met Val Ser Arg Ser Glu Val Asp
   1 5 10 15
<210> 3435
   <211> 17
   <212> PRT
   <213> Homo sapiens
<400> 3435
   Glu Glu Val Lys Leu Ile Lys Lys Met Gly Asp His Leu Thr Asn Leu
   1 5 10 15
   His
<210> 3436
   <211> 16
   <212> PRT
   <213> Homo sapiens
<400> 3436
   Glu Gly Ala Arg Asn Ile Val Ala Ala Met Lys Ala His Gly Val Asp
   1 5 10 15

<210> 3437
   <211> 12
   <212> PRT
   <213> Homo sapiens
<400> 3437
   Glu Ile Ile Asn Trp Leu Asp Lys Asn Gln Thr Ala
   1 5 10
<210> 3438
   <211> 15
   <212> PRT
   <213> Homo sapiens
<400> 3438
   Glu Asn Ile Pro Phe Thr Leu Pro Glu Val Ser Thr Asn Lys Thr
   1 5 10 15
<210> 3439
   <211> 19
   <212> PRT
   <213> Homo sapiens
<400> 3439
   Glu Pro His Arg His Ser Ile Phe Thr Pro Glu Thr Asn Pro Arg Ala
   1 5 10 15
   Gly Leu Glu
<210> 3440
   <211> 18
   <212> PRT
   <213> Homo sapiens
<400> 3440
   Glu Gln Leu Gly Glu Tyr Lys Phe Gln Asn Ala Leu Leu Val Arg Tyr
   1 5 10 15
   Thr Lys
<210> 3441
   <211> 14
   <212> PRT
   <213> Homo sapiens
<400> 3441
   Glu Arg Asn Leu Val Ser Trp Glu Ser Gln Thr Gln Pro Gln
   1 5 10
<210> 3442
   <211> 15
   <212> PRT
   <213> Homo sapiens
<400> 3442
   Glu Arg Thr Ile Thr Met His Lys Asp Ser Thr Gly His Val Gly
   1 5 10 15
<210> 3443
   <211> 11
   <212> PRT
   <213> Homo sapiens
<400> 3443
   Glu Thr Phe Leu Lys Asp Leu Pro Ile Met Phe
   1 5 10
<210> 3444
   <211> 13
   <212> PRT
   <213> Homo sapiens
<400> 3444
   Glu Thr Tyr Gly Glu Met Ala Asp Cys Cys Ala Lys Gln
   1 5 10
<210> 3445
   <211> 12
   <212> PRT
   <213> Homo sapiens
<400> 3445
   Glu Val Glu Asn Ala Val Leu Gly Lys Asp Phe Lys
   1 5 10
<210> 3446
   <211> 22
   <212> PRT
   <213> Homo sapiens
<400> 3446
   Phe Glu Lys Ser Lys Glu Gln Leu Thr Pro Leu Ile Lys Lys Ala Gly
   1 5 10 15
   Thr Glu Leu Val Asn Phe
   20
<210> 3447
   <211> 14
   <212> PRT
   <213> Homo sapiens
<400> 3447
   Phe Glu Pro Phe Phe Met Met Ile Ala Thr Pro Ala Pro His
   1 5 10
<210> 3448
   <211> 21
   <212> PRT
   <213> Homo sapiens
<400> 3448
   Phe Leu Glu Gln Gln Asn Lys Ile Leu Leu Ala Glu Leu Glu Gln Leu
   1 5 10 15
   Lys Gly Gln Gly Lys
   20
<210> 3449
   <211> 9
   <212> PRT
   <213> Homo sapiens
<400> 3449
   Phe Leu Leu Phe Pro Asp Met Glu Ala
   1 5
<210> 3450
   <211> 16
   <212> PRT
   <213> Homo sapiens
<400> 3450
   Phe Pro Gly Phe Leu Glu Asn Gln Lys Asp Pro Leu Ala Val Asp Lys
   1 5 10 15
<210> 3451
   <211> 17
   <212> PRT
   <213> Homo sapiens
<400> 3451
   Phe Gln Asn Ala Leu Leu Val Arg Tyr Thr Lys Lys Val Pro Gln Val
   1 5 10 15
   Ser
<210> 3452
   <211> 17
   <212> PRT
   <213> Homo sapiens
<400> 3452
   Phe Arg Gly Gln Tyr Cys Tyr Glu Leu Asp Glu Lys Ala Val Arg Pro
   1 5 10 15
   Gly
<210> 3453
   <211> 17
   <212> PRT
   <213> Homo sapiens
<400> 3453
   Phe Arg Gly Gln Tyr Cys Tyr Glu Leu Asp Glu Lys Ala Val Arg Pro
   1 5 10 15
   Gly
<210> 3454
   <211> 12
   <212> PRT
   <213> Homo sapiens
<400> 3454
   Phe Ser Ile Ser Pro Asn Ala Val Ser Ala Glu Glu
   1 5 10
<210> 3455
   <211> 12
   <212> PRT
   <213> Homo sapiens
<400> 3455
   Phe Ser Ser Glu Glu Ser Asn Leu Gly Ala Asn Asn
   1 5 10
<210> 3456
   <211> 10
   <212> PRT
   <213> Homo sapiens
<400> 3456
   Phe Val Glu Leu Gly Thr Gln Pro Ala Thr
   1 5 10
<210> 3457
   <211> 14
   <212> PRT
   <213> Homo sapiens
<400> 3457
   Gly Ala Tyr Tyr Leu Lys Leu Glu Asn Gln Ser Leu Lys Ser
   1 5 10
<210> 3458
   <211> 17
   <212> PRT
   <213> Homo sapiens
<400> 3458
   Gly Asp Ile Gln Gln Phe Leu Ile Thr Gly Asp Pro Lys Ala Ala Tyr
   1 5 10 15
   Asp
<210> 3459
   <211> 18
   <212> PRT
   <213> Homo sapiens
<400> 3459
   Gly Glu Thr Ser Glu Met Tyr Leu Ile Gln Pro Asp Ser Ser Val Lys
   1 5 10 15
   Pro Tyr
<210> 3460
   <211> 15
   <212> PRT
   <213> Homo sapiens
<400> 3460
   Gly Gly Gly Tyr Val Lys Leu Phe Pro Asn Ser Leu Asp Gln Thr
   1 5 10 15
<210> 3461
   <211> 18
   <212> PRT
   <213> Homo sapiens
<400> 3461
   Gly Gly His Ala Ile Arg Ile Leu Gly Trp Gly Val Glu Asn Gly Thr
   1               5                   10                  15
   Pro Tyr
<210> 3462
   <211> 16
   <212> PRT
   <213> Homo sapiens
<400> 3462
   Gly Gly Gln Phe Leu Arg Ala Val Ala Gln Arg Cys Pro Ser Pro Pro
   1               5                   10                  15
<210> 3463
   <211> 17
   <212> PRT
   <213> Homo sapiens
<400> 3463
   Gly His Leu Ser Phe Gly Phe Met Gln Asp Leu Gln Gln Lys Val Val
   1               5                   10                  15
   Tyr
<210> 3464
   <211> 16
   <212> PRT
   <213> Homo sapiens
<400> 3464
   Gly Ile Lys Ser Leu Val Ser Lys Gly Thr Leu Val Gln Thr Lys Gly
   1               5                   10                  15
<210> 3465
   <211> 16
   <212> PRT
   <213> Homo sapiens
<400> 3465
   Gly Leu Lys Ser Leu Val Ser Lys Gly Thr Leu Val Gln Thr Lys Gly
   1               5                   10                  15
<210> 3466
   <211> 14
   <212> PRT
   <213> Homo sapiens
<400> 3466
   Gly Leu Leu Trp Ile Ala Leu His Gly Asn Gln Ile Thr Ser
   1               5                   10
<210> 3467
   <211> 15
   <212> PRT
   <213> Homo sapiens
<400> 3467
   Gly Asn Gln Phe Trp Ala Ile Arg Gly Asn Glu Val Arg Ala Gly
   1 5 10 15
<210> 3468
   <211> 12
   <212> PRT
   <213> Homo sapiens
<400> 3468
   Gly Asn Arg Ile Ala Gln Trp Gln Ser Phe Gln Leu
   1 5 10
<210> 3469
   <211> 22
   <212> PRT
   <213> Homo sapiens
<400> 3469
   Gly Pro Asp Pro Ser Ser Pro Ala Phe Arg Ile Glu Asp Ala Asn Leu
   1 5 10 15
   Ile Pro Pro Val Pro Asp
   20
<210> 3470
   <211> 19
   <212> PRT
   <213> Homo sapiens
<400> 3470
   Gly Pro Gly Ile Pro Gly Arg Phe Thr Lys Glu Ala Gly Thr Leu Ala
   1 5 10 15
   Tyr Tyr Glu
<210> 3471
   <211> 15
   <212> PRT
   <213> Homo sapiens
<400> 3471
   Gly Pro Val Lys Val Trp Gly Ser Ile Lys Gly Leu Thr Glu Gly
   1 5 10 15
<210> 3472
   <211> 15
   <212> PRT
   <213> Homo sapiens
<400> 3472
   Gly Gln Tyr Cys Tyr Glu Leu Asp Glu Lys Ala Val Arg Pro Gly
   1               5                   10                  15
<210> 3473
   <211> 17
   <212> PRT
   <213> Homo sapiens
<400> 3473
   Gly Ser Ala Gly His Trp Thr Ser Glu Ser Ser Val Ser Gly Ser Thr
   1               5                   10                  15
   Gly
<210> 3474
   <211> 16
   <212> PRT
   <213> Homo sapiens
<400> 3474
   Gly Ser His Ser Leu Lys Tyr Phe His Thr Ser Val Ser Arg Pro Gly
   1               5                   10                  15
<210> 3475
   <211> 18
   <212> PRT
   <213> Homo sapiens
<400> 3475
   Gly Thr Asp Val Pro Lys Trp Ile Ser Ile Met Thr Glu Arg Ser Val
   1               5                   10                  15
   Pro His
<210> 3476
   <211> 18
   <212> PRT
   <213> Homo sapiens
<400> 3476
   Gly Thr Gly Glu Asn Tyr Arg Gly Asn Val Ala Val Thr Val Ser Gly
   1               5                   10                  15
   His Thr
<210> 3477
   <211> 19
   <212> PRT
   <213> Homo sapiens
<400> 3477
   Gly Val Asp Glu Val Thr Ile Val Asn Ile Leu Thr Asn Arg Ser Asn
   1 5 10 15
   Ala Gln Arg
<210> 3478
   <211> 12
   <212> PRT
   <213> Homo sapiens
<400> 3478
   Gly Val Ile Lys Val Phe Asn Asp Met Lys Val Arg
   1 5 10
<210> 3479
   <211> 13
   <212> PRT
   <213> Homo sapiens
<400> 3479
   Gly Tyr Val Arg Asp Glu Thr Val Arg Ala Ala Pro Tyr
   1 5 10
<210> 3480
   <211> 18
   <212> PRT
   <213> Homo sapiens
<400> 3480
   His Leu Pro Asp Asn Ile Asn Val Tyr Ala Thr Thr Ala Ala Asn Pro
   1 5 10 15
   Arg Glu
<210> 3481
   <211> 16
   <212> PRT
   <213> Homo sapiens
<400> 3481
   His Asn Gly Asn Leu Ile Pro Thr His Thr Gln Pro Ser Tyr Arg Phe
   1 5 10 15
<210> 3482
   <211> 17
   <212> PRT
   <213> Homo sapiens
<400> 3482
   Ile Gly Gly Val Ile Leu Phe His Glu Thr Leu Tyr Gln Lys Ala Asp
   1               5                   10                  15
   Asp
<210> 3483
   <211> 14
   <212> PRT
   <213> Homo sapiens
<400> 3483
   Ile Lys Ile Ile Ser Lys Ile Glu Asn His Glu Gly Val Arg
   1               5                   10
<210> 3484
   <211> 14
   <212> PRT
   <213> Homo sapiens
<400> 3484
   Ile Leu Glu Trp Ala Ala Glu Arg Gly Pro Ile Thr Ser Ala
   1               5                   10
<210> 3485
   <211> 17
   <212> PRT
   <213> Homo sapiens
<400> 3485
   Ile Gln Ser Thr Tyr Asp Gln Asp His Thr Val Pro Ala Tyr Val Pro
   1               5                   10                  15
   Arg
<210> 3486
   <211> 12
   <212> PRT
   <213> Homo sapiens
<400> 3486
   Ile Ser Thr Pro Glu Glu Leu Gly Leu Asp Lys Val
   1               5                   10
<210> 3487
   <211> 16
   <212> PRT
   <213> Homo sapiens
<400> 3487
   Ile Val His Arg Tyr Met Thr Ile Thr Ser Glu Arg Ser Val Pro Ala
   1               5                   10                  15
<210> 3488
   <211> 11
   <212> PRT
   <213> Homo sapiens
<400> 3488
   Ile Tyr Val Phe His Thr Leu Gly Gln Tyr Phe
   1               5                   10
<210> 3489
   <211> 15
   <212> PRT
   <213> Homo sapiens
<400> 3489
   Lys Ile Gly Arg Phe Val Ile Glu Glu Val Pro Gly Glu Leu Met
   1               5                   10                  15
<210> 3490
   <211> 24
   <212> PRT
   <213> Homo sapiens
<400> 3490
   Lys Lys Val Pro Gln Val Ser Thr Pro Thr Leu Val Glu Val Ser Arg
   1               5                   10                  15
   Asn Leu Gly Lys Val Gly Ser Lys
               20
<210> 3491
   <211> 14
   <212> PRT
   <213> Homo sapiens
<400> 3491
   Lys Pro Gln Tyr Met Val Leu Val Pro Ser Leu Leu His Thr
   1               5                   10
<210> 3492
   <211> 16
   <212> PRT
   <213> Homo sapiens
<400> 3492
   Lys Thr Gly Lys Leu Val Ser Leu Ser Ala Gln Asn Leu Val Asp Cys
   1               5                   10                  15
<210> 3493
   <211> 14
   <212> PRT
   <213> Homo sapiens
<400> 3493
   Leu Ala Glu Phe Ala Phe Ser Leu Tyr Arg Gln Leu Ala His
   1 5 10
<210> 3494
   <211> 14
   <212> PRT
   <213> Homo sapiens
<400> 3494
   Leu Ala Val Val Lys Ser Ile Arg Ser Ile Pro Ala Tyr Leu
   1 5 10
<210> 3495
   <211> 15
   <212> PRT
   <213> Homo sapiens
<400> 3495
   Leu Asp Asp Pro Lys Tyr Ser Ser Asp Glu Asp Leu Pro Ser Lys
   1 5 10 15
<210> 3496
   <211> 15
   <212> PRT
   <213> Homo sapiens
<400> 3496
   Leu Asp Phe Leu Lys Ala Val Asp Thr Asn Arg Ala Ser Val Gly
   1 5 10 15
<210> 3497
   <211> 12
   <212> PRT
   <213> Homo sapiens
<400> 3497
   Leu Gly Glu Phe Val Ser Glu Thr Glu Ser Arg Gly
   1 5 10
<210> 3498
   <211> 15
   <212> PRT
   <213> Homo sapiens
<400> 3498
   Leu Gly Gln His Leu Leu Pro Trp Met Asp Arg Leu Ser Leu Glu
   1 5 10 15
<210> 3499
   <211> 15
   <212> PRT
   <213> Homo sapiens
<400> 3499
   Leu Ile Asn Glu Tyr Trp Val Leu Thr Ala Ala His Val Val Glu
   1 5 10 15
<210> 3500
   <211> 17
   <212> PRT
   <213> Homo sapiens
<400> 3500
   Leu Lys Gln Glu Tyr Phe Val Val Ala Ala Thr Leu Gln Asp Ile Ile
   1 5 10 15
   Arg
<210> 3501
   <211> 17
   <212> PRT
   <213> Homo sapiens
<400> 3501
   Leu Lys Thr Gly Lys Leu Val Ser Leu Ser Ala Gln Asn Leu Val Asp
   1 5 10 15
   Cys
<210> 3502
   <211> 16
   <212> PRT
   <213> Homo sapiens
<400> 3502
   Leu Met His Pro Asp Ala Leu Glu Glu Pro Asp Asp Gln Asn Arg Ile
   1 5 10 15
<210> 3503
   <211> 16
   <212> PRT
   <213> Homo sapiens
<400> 3503
   Leu Asn Gln Glu Leu Arg Ala Asp Gly Thr Val Asn Gln Ile Glu Gly
   1 5 10 15
<210> 3504
   <211> 18
   <212> PRT
   <213> Homo sapiens
<400> 3504
   Leu Pro Gln Pro Asp Leu Arg Tyr Leu Phe Leu Asn Gly Asn Lys Leu
   1 5 10 15
   Ala Arg
<210> 3505
   <211> 17
   <212> PRT
   <213> Homo sapiens
<400> 3505
   Leu Arg Val Leu Arg Ala Gln Pro Val Gln Val Ala Glu Gly Ser Glu
   1 5 10 15
   Pro
<210> 3506
   <211> 16
   <212> PRT
   <213> Homo sapiens
<400> 3506
   Leu Ser Lys Glu Leu Gln Ala Ala Gln Ala Arg Leu Gly Ala Asp Met
   1 5 10 15
<210> 3507
   <211> 14
   <212> PRT
   <213> Homo sapiens
<400> 3507
   Leu Val Ala Tyr Tyr Thr Leu Ile Gly Ala Ser Gly Gln Arg
   1 5 10
<210> 3508
   <211> 13
   <212> PRT
   <213> Homo sapiens
<400> 3508
   Met Gln Gln Lys Leu Val Asp Gln Asn Ile Phe Ser Phe
   1 5 10
<210> 3509
   <211> 13
   <212> PRT
   <213> Homo sapiens
<400> 3509
   Met Val Lys Val Leu Asp Ala Val Arg Gly Ser Pro Ala
   1 5 10
<210> 3510
   <211> 16
   <212> PRT
   <213> Homo sapiens
<400> 3510
   Asn Asp Val Lys Asn Val Ile Ile Trp Gly Asn His Ser Ser Thr Gln
   1 5 10 15
<210> 3511
   <211> 16
   <212> PRT
   <213> Homo sapiens
<400> 3511
   Asn Glu Glu Glu Ile Arg Ala Asn Val Ala Val Val Ser Gly Ala Pro
   1 5 10 15
<210> 3512
   <211> 23
   <212> PRT
   <213> Homo sapiens
<400> 3512
   Asn Leu Asp Phe Leu Lys Ala Val Asp Thr Asn Arg Ala Ser Val Gly
   1 5 10 15
   Gln Asp Ser Pro Glu Pro Arg
   20
<210> 3513
   <211> 15
   <212> PRT
   <213> Homo sapiens
<400> 3513
   Asn Pro Ala Glu Phe Val Val Asn Thr Ser Asn Ala Gly Ala Gly
   1 5 10 15
<210> 3514
   <211> 15
   <212> PRT
   <213> Homo sapiens
<400> 3514
   Asn Pro Gly Thr Phe Arg Ile Leu Val Gly Asn Lys Gly Cys Ser
   1 5 10 15
<210> 3515
   <211> 18
   <212> PRT
   <213> Homo sapiens
<400> 3515
   Asn Pro Gly Thr Phe Arg Ile Leu Val Gly Asn Lys Gly Cys Ser His
   1 5 10 15
   Pro Ser
<210> 3516
   <211> 17
   <212> PRT
   <213> Homo sapiens
<400> 3516
   Asn Pro Leu Glu Ile Val Ser Ile Pro Asp Asn His Gly His Ile Asp
   1 5 10 15
   Pro
<210> 3517
   <211> 15
   <212> PRT
   <213> Homo sapiens
<400> 3517
   Asn Ser Asn Gln Ile Lys Ile Leu Gly Asn Gln Gly Ser Phe Leu
   1 5 10 15
<210> 3518
   <211> 16
   <212> PRT
   <213> Homo sapiens
<400> 3518
   Asn Val Pro Thr Ile Asp Ile His Met Asn Gln Ile Gly Phe Glu Arg
   1 5 10 15
<210> 3519
   <211> 16
   <212> PRT
   <213> Homo sapiens
<400> 3519
   Gln Gly Gly Thr Tyr Ser Lys Ala Ser Thr Pro Asn Gly Tyr Asp Asn
   1 5 10 15
<210> 3520
   <211> 16
   <212> PRT
   <213> Homo sapiens
<400> 3520
   Gln Pro Leu Gly Met Ile Ser Leu Met Lys Arg Pro Pro Gly Phe Ser
   1 5 10 15
<210> 3521
   <211> 15
   <212> PRT
   <213> Homo sapiens
<400> 3521
   Gln Ser Gly Glu Tyr Trp Ile Asp Pro Asn Gln Gly Ser Val Glu
   1 5 10 15
<210> 3522
   <211> 25
   <212> PRT
   <213> Homo sapiens
<400> 3522
   Gln Ser Leu Pro His Glu Asn Lys Pro Leu Thr Leu Ser Asn Tyr Gln
   1 5 10 15
   Thr Asn Lys Ala Lys His Asp Glu Leu
   20 25
<210> 3523
   <211> 19
   <212> PRT
   <213> Homo sapiens
<400> 3523
   Gln Thr Val Ala Val Gly Val Ile Lys Ala Val Asp Lys Lys Ala Ala
   1 5 10 15
   Gly Ala Gly
<210> 3524
   <211> 13
   <212> PRT
   <213> Homo sapiens
<400> 3524
   Arg Ile Glu Gly Ser Asn Lys Val Pro Val Asp Pro Ala
   1 5 10
<210> 3525
   <211> 16
   <212> PRT
   <213> Homo sapiens
<400> 3525
   Arg Leu Pro Gly Leu Ala Lys Gln Pro Ser Phe Arg Gln Tyr Ser Gly
   1 5 10 15
<210> 3526
   <211> 16
   <212> PRT
   <213> Homo sapiens
<400> 3526
   Arg Pro Leu Leu Asp Lys Trp Pro Thr Thr Leu Ser Arg His Leu Gly
   1 5 10 15
<210> 3527
   <211> 16
   <212> PRT
   <213> Homo sapiens
<400> 3527
   Arg Pro Arg Ala Pro Ile Ile Ala Val Thr Arg Asn Pro Gln Thr Ala
   1 5 10 15
<210> 3528
   <211> 15
   <212> PRT
   <213> Homo sapiens
<400> 3528
   Ser Ala Arg Leu Thr Trp Asp Pro Thr Ser Arg Gln Ile Asn Gly
   1 5 10 15
<210> 3529
   <211> 16
   <212> PRT
   <213> Homo sapiens
<400> 3529
   Ser Asp Thr Ser Tyr Val Ser Leu Lys Ala Pro Leu Thr Lys Pro Leu
   1 5 10 15
<210> 3530
   <211> 15
   <212> PRT
   <213> Homo sapiens
<400> 3530
   Ser Asp Val Asp Leu Ile Pro Met Asn Asp His Asn Ala Tyr Arg
   1 5 10 15
<210> 3531
   <211> 13
   <212> PRT
   <213> Homo sapiens
<400> 3531
   Ser Phe Tyr Leu Ser Arg Asp Pro Asp Ala Gln Pro Gly
   1 5 10
<210> 3532
   <211> 16
   <212> PRT
   <213> Homo sapiens
<400> 3532
   Ser Gly Asp Val Phe Thr Ala Leu Ile Gly Glu Ile Ala Ser Pro Asn
   1 5 10 15
<210> 3533
   <211> 18
   <212> PRT
   <213> Homo sapiens
<400> 3533
   Ser Gly Lys Pro Gln Tyr Met Val Leu Val Pro Ser Leu Leu His Thr
   1 5 10 15
   Glu Thr
<210> 3534
   <211> 18
   <212> PRT
   <213> Homo sapiens
<400> 3534
   Ser Gly Leu Glu Val Ile Asp Ala Met Arg Ser Gln Leu Gly Lys Ala
   1 5 10 15
   Thr Ala
<210> 3535
   <211> 17
   <212> PRT
   <213> Homo sapiens
<400> 3535
   Ser Lys Asn Thr Leu Tyr Leu Gln Met Asn Ser Leu Arg Ala Glu Asp
   1 5 10 15
   Thr
<210> 3536
   <211> 19
   <212> PRT
   <213> Homo sapiens
<400> 3536
   Ser Leu Arg Pro Lys Asp Tyr Glu Val Asp Ala Thr Leu Lys Ser Leu
   1 5 10 15
   Asn Asn Gln
<210> 3537
   <211> 17
   <212> PRT
   <213> Homo sapiens
<400> 3537
   Ser Pro Asp Arg Ile Phe Phe His Leu Asn Ala Val Ala Leu Gly Asp
   1 5 10 15
   Gly
<210> 3538
   <211> 18
   <212> PRT
   <213> Homo sapiens
<400> 3538
   Ser Pro Glu Arg Pro Phe Leu Ala Ile Leu Gly Gly Ala Lys Val Ala
   1 5 10 15
   Asp Lys
<210> 3539
   <211> 17
   <212> PRT
   <213> Homo sapiens
<400> 3539
   Ser Pro Leu Ser Val Ala Leu Ala Leu Ser His Leu Ala Leu Gly Ala
   1 5 10 15
   Gln
<210> 3540
   <211> 14
   <212> PRT
   <213> Homo sapiens
<400> 3540
   Ser Pro Met Tyr Ser Ile Ile Thr Pro Asn Ile Leu Arg Leu
   1               5                   10
<210> 3541
   <211> 19
   <212> PRT
   <213> Homo sapiens
<400> 3541
   Ser Pro Pro Ile Ser Tyr Ile Trp Tyr Lys Gln Gln Thr Asn Asn Gln
   1               5                   10                  15
   Glu Pro Ile
<210> 3542
   <211> 19
   <212> PRT
   <213> Homo sapiens
<400> 3542
   Ser Pro Val Thr Gly Tyr Arg Val Thr Thr Thr Pro Lys Asn Gly Pro
   1               5                   10                  15
   Gly Pro Thr
<210> 3543
   <211> 17
   <212> PRT
   <213> Homo sapiens
<400> 3543
   Ser Arg Leu Pro Ile Ile Asp Val Ala Pro Leu Asp Val Gly Ala Pro
   1               5                   10                  15
   Asp
<210> 3544
   <211> 16
   <212> PRT
   <213> Homo sapiens
<400> 3544
   Ser Tyr Ala Gly Lys Tyr Val Pro Ala Ile Ala His Leu Ile His Ser
   1               5                   10                  15
<210> 3545
   <211> 15
   <212> PRT
   <213> Homo sapiens
<400> 3545
   Thr Ala Ala Asp Tyr Lys Ile Leu Gly Gly Ser Val Leu His Leu
   1 5 10 15
<210> 3546
   <211> 21
   <212> PRT
   <213> Homo sapiens
<400> 3546
   Thr Glu Glu Glu Phe Val Glu Gly Ile Tyr Lys Val Glu Ile Asp Thr
   1 5 10 15
   Lys Ser Tyr Trp Lys
   20
<210> 3547
   <211> 18
   <212> PRT
   <213> Homo sapiens
<400> 3547
   Thr Gly Ala Gln Glu Leu Leu Arg Val Leu Arg Ala Gln Pro Val Gln
   1 5 10 15
   Val Ala
<210> 3548
   <211> 18
   <212> PRT
   <213> Homo sapiens
<400> 3548
   Thr Gly Asp Ala Tyr Val Ile Leu Lys Thr Val Gln Leu Arg Asn Gly
   1 5 10 15
   Asn Leu
<210> 3549
   <211> 14
   <212> PRT
   <213> Homo sapiens
<400> 3549
   Thr Gly Lys Leu Val Ser Leu Ser Ala Gln Asn Leu Val Asp
   1 5 10
<210> 3550
   <211> 15
   <212> PRT
   <213> Homo sapiens
<400> 3550
   Thr Gly Lys Leu Val Ser Leu Ser Ala Gln Asn Leu Val Asp Cys
   1 5 10 15
<210> 3551
   <211> 15
   <212> PRT
   <213> Homo sapiens
<400> 3551
   Thr Gly Asn Tyr Arg Ile Glu Ser Val Leu Ser Ser Ser Gly Lys
   1 5 10 15
<210> 3552
   <211> 15
   <212> PRT
   <213> Homo sapiens
<400> 3552
   Thr His Met Leu Asp Val Met Gln Asp His Phe Ser Arg Ala Ser
   1 5 10 15
<210> 3553
   <211> 16
   <212> PRT
   <213> Homo sapiens
<400> 3553
   Thr Ile Tyr Val Ile Ala Leu Lys Asn Asn Gln Lys Ser Glu Pro Leu
   1 5 10 15
<210> 3554
   <211> 16
   <212> PRT
   <213> Homo sapiens
<400> 3554
   Thr Leu Asn Gln Ile Asp Glu Val Lys Val Trp Pro Gln Gln Pro Ser
   1 5 10 15
<210> 3555
   <211> 16
   <212> PRT
   <213> Homo sapiens
<400> 3555
   Thr Leu Gln Ser Phe Arg Gln Asp Val Asp Asn Ala Ser Leu Ala Arg
   1               5                   10                  15
<210> 3556
   <211> 17
   <212> PRT
   <213> Homo sapiens
<400> 3556
   Thr Ser Ala Thr Asn Ile Val Lys Trp Ile Thr Lys Gln Gln Asn Ala
   1               5                   10                  15
   Gln
<210> 3557
   <211> 15
   <212> PRT
   <213> Homo sapiens
<400> 3557
   Thr Thr Ala Phe Gln Tyr Ile Ile Asp Asn Lys Gly Ile Asp Ser
   1               5                   10                  15
<210> 3558
   <211> 19
   <212> PRT
   <213> Homo sapiens
<400> 3558
   Val Ala Ile Gln Leu Asn Asp Thr His Pro Ser Leu Ala Ile Pro Glu
   1               5                   10                  15
   Leu Met Arg
<210> 3559
   <211> 21
   <212> PRT
   <213> Homo sapiens
<400> 3559
   Val Asp Asp Thr Gln Phe Val Arg Phe Asp Ser Asp Ala Ala Ser Pro
   1               5                   10                  15
   Arg Thr Glu Pro Arg
               20
<210> 3560
   <211> 18
   <212> PRT
   <213> Homo sapiens
<400> 3560
   Val Asp Asp Thr Ser Ile Val Val Arg Trp Ser Arg Pro Gln Ala Pro
   1 5 10 15
   Ile Thr
<210> 3561
   <211> 17
   <212> PRT
   <213> Homo sapiens
<400> 3561
   Val Asp Met Asp Phe Glu Val Glu Asn Ala Val Leu Gly Lys Asp Phe
   1 5 10 15
   Lys
<210> 3562
   <211> 15
   <212> PRT
   <213> Homo sapiens
<400> 3562
   Val Phe Asn Ile Leu Arg Met Met Pro Lys Gly Ala Ala Leu His
   1 5 10 15
<210> 3563
   <211> 19
   <212> PRT
   <213> Homo sapiens
<400> 3563
   Val Gly Pro Val Asp Glu Val Arg Glu Leu Gln Lys Ala Ile Gly Ala
   1 5 10 15
   Val Pro Leu
<210> 3564
   <211> 15
   <212> PRT
   <213> Homo sapiens
<400> 3564
   Val His Pro Lys Val Thr Val Tyr Pro Ser Lys Thr Gln Pro Leu
   1 5 10 15
<210> 3565
   <211> 15
   <212> PRT
   <213> Homo sapiens
<400> 3565
   Val Pro Pro Glu Trp Lys Ala Leu Thr Asp Met Pro Gln Met Arg
   1 5 10 15
<210> 3566
   <211> 16
   <212> PRT
   <213> Homo sapiens
<400> 3566
   Val Pro Val Pro Val Phe Cys Asp Met Thr Thr Glu Gly Gly Lys Trp
   1 5 10 15
<210> 3567
   <211> 17
   <212> PRT
   <213> Homo sapiens
<400> 3567
   Val Pro Val Pro Val Phe Cys Asp Met Thr Thr Glu Gly Gly Lys Trp
   1 5 10 15
   Thr
<210> 3568
   <211> 14
   <212> PRT
   <213> Homo sapiens
<400> 3568
   Val Pro Tyr Val Ile Val Pro Leu Lys Thr Gly Leu Gln Glu
   1 5 10
<210> 3569
   <211> 15
   <212> PRT
   <213> Homo sapiens
<400> 3569
   Val Ser Gly Thr Leu Val Leu Leu Gln Gly Ala Arg Gly Phe Ala
   1 5 10 15
<210> 3570
   <211> 15
   <212> PRT
   <213> Homo sapiens
<400> 3570
   Val Ser Gln Tyr Phe Gln Thr Val Thr Asp Tyr Gly Lys Asp Leu
   1 5 10 15
<210> 3571
   <211> 15
   <212> PRT
   <213> Homo sapiens
<400> 3571
   Val Ser Thr Ala Phe Val Tyr Thr Lys Asn Pro Asn Gly Tyr Ser
   1 5 10 15
<210> 3572
   <211> 19
   <212> PRT
   <213> Homo sapiens
<400> 3572
   Val Val Leu Pro Ser Tyr Glu Glu Ala Leu Ser Leu Pro Ser Lys Thr
   1 5 10 15
   Pro Glu Gly
<210> 3573
   <211> 15
   <212> PRT
   <213> Homo sapiens
<400> 3573
   Val Val Tyr Tyr Arg Val Gln Asn Ala Thr Leu Ala Val Ala Asn
   1 5 10 15
<210> 3574
   <211> 18
   <212> PRT
   <213> Homo sapiens
<400> 3574
   Tyr Asp Gly Lys Asp Tyr Ile Ala Leu Lys Glu Asp Leu Arg Ser Trp
   1 5 10 15
   Thr Ala
<210> 3575
   <211> 19
   <212> PRT
   <213> Homo sapiens
<400> 3575
   Tyr Asp Gly Ser His Arg Tyr Val Ile Leu Lys Ser Glu Pro Val His
   1 5 10 15
   Pro Phe Gly
<210> 3576
   <211> 26
   <212> PRT
   <213> Homo sapiens
<400> 3576
   Tyr Asp Leu Arg His Thr Phe Met Gly Val Val Ser Leu Gly Ser Pro
   1 5 10 15
   Ser Gly Glu Val Ser His Pro Arg Lys Thr
   20 25
<210> 3577
   <211> 12
   <212> PRT
   <213> Homo sapiens
<400> 3577
   Tyr Leu Gln Met Asn Ser Leu Arg Val Glu Asp Thr
   1 5 10
<210> 3578
   <211> 12
   <212> PRT
   <213> Homo sapiens
<400> 3578
   Tyr Leu Gln Met Asn Ser Val Arg Ala Glu Asp Thr
   1 5 10
<210> 3579
   <211> 11
   <212> PRT
   <213> Homo sapiens
<400> 3579
   Tyr Val Asn Gly Leu Thr Leu Gly Gly Gln Lys
   1 5 10
<210> 3580
   <211> 15
   <212> PRT
   <213> Homo sapiens
<400> 3580
   Cys Asp Met Asn Thr Glu Asn Gly Gly Trp Thr Val Ile Gln Asn
   1 5 10 15
<210> 3581
   <211> 15
   <212> PRT
   <213> Homo sapiens
<400> 3581
   Asp Val Ser Ala Gln Met Glu Tyr Cys Arg Thr Pro Cys Thr Val
   1 5 10 15
<210> 3582
   <211> 15
   <212> PRT
   <213> Homo sapiens
<400> 3582
   Asp Trp Pro Phe Cys Ser Asp Glu Asp Trp Asn Tyr Lys Cys Pro
   1 5 10 15
<210> 3583
   <211> 15
   <212> PRT
   <213> Homo sapiens
<400> 3583
   Glu Ala Val Gln Ser Thr Ser Ser Ser Ser Gln Phe Tyr Met Tyr
   1 5 10 15
<210> 3584
   <211> 15
   <212> PRT
   <213> Homo sapiens
<400> 3584
   Phe Thr Asn Arg Ile Asn Lys Leu Lys Asn Ser Leu Phe Glu Tyr
   1 5 10 15
<210> 3585
   <211> 15
   <212> PRT
   <213> Homo sapiens
<400> 3585
   Gly Asp Lys Glu Leu Arg Thr Gly Lys Glu Lys Val Thr Ser Gly
   1 5 10 15
<210> 3586
   <211> 12
   <212> PRT
   <213> Mycobacterium tuberculosis
<400> 3586
   Gly Leu Glu Pro Gly Val Val Ala Glu Lys Val Arg
   1 5 10
<210> 3587
   <211> 15
   <212> PRT
   <213> Homo sapiens
<400> 3587
   Gly Ser Ser Glu Arg Gly Ser Ala Gly His Trp Thr Ser Glu Ser
   1 5 10 15
<210> 3588
   <211> 15
   <212> PRT
   <213> Homo sapiens
<400> 3588
   Ile Arg Val Leu Arg Ser Ile Leu Glu Asn Leu Arg Ser Lys Ile
   1 5 10 15
<210> 3589
   <211> 15
   <212> PRT
   <213> Homo sapiens
<400> 3589
   Ile Thr Ser Asp Pro Arg Lys Gln Cys Ser Lys Glu Asp Gly Gly
   1 5 10 15
<210> 3590
   <211> 15
   <212> PRT
   <213> Homo sapiens
<400> 3590
   Leu Glu Asn Leu Arg Ser Lys Ile Gln Lys Leu Glu Ser Asp Val
   1 5 10 15
<210> 3591
   <211> 11
   <212> PRT
   <213> Mycobacterium tuberculosis
<400> 3591
   Leu Glu Pro Gly Val Val Ala Glu Lys Val Arg
   1 5 10
<210> 3592
   <211> 15
   <212> PRT
   <213> Homo sapiens
<400> 3592
   Leu Leu Gln Gln Glu Arg Pro Ile Arg Asn Ser Val Asp Glu Leu
   1 5 10 15
<210> 3593
   <211> 15
   <212> PRT
   <213> Homo sapiens
<400> 3593
   Leu Met Gly Glu Asn Arg Thr Met Thr Ile His Asn Gly Met Phe
   1 5 10 15
<210> 3594
   <211> 15
   <212> PRT
   <213> Homo sapiens
<400> 3594
   Asn Ser Asn Ile Pro Thr Asn Leu Arg Val Leu Arg Ser Ile Leu
   1 5 10 15
<210> 3595
   <211> 15
   <212> PRT
   <213> Homo sapiens
<400> 3595
   Pro Arg Lys Gln Cys Ser Lys Glu Asp Gly Gly Gly Trp Trp Tyr
   1 5 10 15
<210> 3596
   <211> 15
   <212> PRT
   <213> Homo sapiens
<400> 3596
   Gln Lys Leu Glu Ser Asp Val Ser Ala Gln Met Glu Tyr Cys Arg
   1 5 10 15
<210> 3597
   <211> 15
   <212> PRT
   <213> Homo sapiens
<400> 3597
   Gln Lys Gln Leu Glu Gln Val Ile Ala Lys Asp Leu Leu Pro Ser
   1 5 10 15
<210> 3598
   <211> 15
   <212> PRT
   <213> Homo sapiens
<400> 3598
   Gln Met Glu Tyr Cys Arg Thr Pro Cys Thr Val Ser Cys Asn Ile
   1 5 10 15
<210> 3599
   <211> 15
   <212> PRT
   <213> Homo sapiens
<400> 3599
   Gln Asn Pro Gly Ser Pro Arg Pro Gly Ser Thr Gly Thr Trp Asn
   1 5 10 15
<210> 3600
   <211> 15
   <212> PRT
   <213> Homo sapiens
<400> 3600
   Ser Glu Thr Glu Ser Arg Gly Ser Glu Ser Gly Ile Phe Thr Asn
   1 5 10 15
<210> 3601
   <211> 15
   <212> PRT
   <213> Homo sapiens
<400> 3601
   Ser Gly Cys Arg Met Lys Gly Leu Ile Asp Glu Val Asn Gln Asp
   1 5 10 15
<210> 3602
   <211> 15
   <212> PRT
   <213> Homo sapiens
<400> 3602
   Ser Gly Pro Gly Ser Thr Gly Asn Arg Asn Pro Gly Ser Ser Gly
   1 5 10 15
<210> 3603
   <211> 15
   <212> PRT
   <213> Homo sapiens
<400> 3603
   Thr Pro Cys Thr Val Ser Cys Asn Ile Pro Val Val Ser Gly Lys
   1 5 10 15
<210> 3604
   <211> 15
   <212> PRT
   <213> Homo sapiens
<400> 3604
   Thr Thr Arg Arg Ser Cys Ser Lys Thr Val Thr Lys Thr Val Ile
   1 5 10 15
<210> 3605
   <211> 15
   <212> PRT
   <213> Homo sapiens
<400> 3605
   Thr Val Gln Asn Glu Ala Asn Lys Tyr Gln Ile Ser Val Asn Lys
   1 5 10 15
<210> 3606
   <211> 15
   <212> PRT
   <213> Homo sapiens
<400> 3606
   Val Asp Ile Asp Ile Lys Ile Arg Ser Cys Arg Gly Ser Cys Ser
   1 5 10 15
<210> 3607
   <211> 15
   <212> PRT
   <213> Homo sapiens
<400> 3607
   Val Asp Ile Asp Ile Lys Ile Arg Ser Cys Arg Gly Ser Cys Ser
   1 5 10 15
<210> 3608
   <211> 15
   <212> PRT
   <213> Homo sapiens
<400> 3608
   Val Ser Pro Gly Thr Arg Arg Glu Tyr His Thr Glu Lys Leu Val
   1 5 10 15
<210> 3609
   <211> 15
   <212> PRT
   <213> Homo sapiens
<400> 3609
   Val Val Trp Met Asn Trp Lys Gly Ser Trp Tyr Ser Met Arg Lys
   1 5 10 15
<210> 3610
   <211> 15
   <212> PRT
   <213> Homo sapiens
<400> 3610
   Trp Tyr Asn Arg Cys His Ala Ala Asn Pro Asn Gly Arg Tyr Tyr
   1 5 10 15
<210> 3611
   <211> 15
   <212> PRT
   <213> Homo sapiens
<400> 3611
   Trp Tyr Ser Met Arg Lys Met Ser Met Lys Ile Arg Pro Phe Phe
   1 5 10 15
<210> 3612
   <211> 15
   <212> PRT
   <213> Homo sapiens
<400> 3612
   Tyr Cys Gly Leu Pro Gly Glu Tyr Trp Leu Gly Asn Asp Lys Ile
   1 5 10 15
<210> 3613
   <211> 17
   <212> PRT
   <213> Homo sapiens
<400> 3613
   Asn Glu Glu Gly Phe Phe Ser Ala Arg Gly His Arg Pro Leu Asp Lys
   1 5 10 15
   Lys
<210> 3614
   <211> 15
   <212> PRT
   <213> Homo sapiens
<400> 3614
   Gly Pro Arg Val Val Glu Arg His Gln Ser Ala Cys Lys Asp Ser
   1 5 10 15
<210> 3615
   <211> 17
   <212> PRT
   <213> Homo sapiens
<400> 3615
   Lys Ile His Ala Arg Glu Ile Phe Asp Ser Arg Gly Asn Pro Thr Val
   1 5 10 15
   Glu
<210> 3616
   <211> 15
   <212> PRT
   <213> Homo sapiens
<400> 3616
   Val Tyr Ala Thr Arg Ser Ser Ala Val Arg Leu Arg Ser Ser Val
   1 5 10 15
<210> 3617
   <211> 20
   <212> PRT
   <213> Homo sapiens
<400> 3617
   Gly Val Tyr Ala Thr Arg Ser Ser Ala Val Arg Leu Arg Ser Ser Val
   1 5 10 15
   Pro Gly Val Arg
   20
<210> 3618
   <211> 20
   <212> PRT
   <213> Homo sapiens
<400> 3618
   Gly Val Tyr Ala Thr Arg Ser Ser Ala Val Arg Leu Arg Ser Ser Val
   1 5 10 15
   Pro Gly Val Arg
   20
<210> 3619
   <211> 5
   <212> PRT
   <213> Homo sapiens
<400> 3619
   Gly Tyr Arg Ala Arg
   1 5
<210> 3620
   <211> 4
   <212> PRT
   <213> Homo sapiens
<400> 3620
   Arg Val Val Glu
   1
<210> 3621
   <211> 15
   <212> PRT
   <213> Homo sapiens
<400> 3621
   Ser Gly Ile Gly Thr Leu Asp Gly Phe Arg His Arg His Pro Asp
   1 5 10 15
<210> 3622
   <211> 5
   <212> PRT
   <213> Homo sapiens
<400> 3622
   Val Glu Arg His Gln
   1 5
<210> 3623
   <211> 20
   <212> PRT
   <213> Homo sapiens
<400> 3623
   Ala Ala Ala Ile Ala Tyr Gly Leu Asp Lys Arg Glu Gly Glu Lys Asn
   1               5                   10                  15
   Ile Leu Val Phe
               20
<210> 3624
   <211> 20
   <212> PRT
   <213> Homo sapiens
<400> 3624
   Ala Gly Thr Ile Ala Gly Leu Asn Val Met Arg Ile Ile Asn Glu Pro
   1               5                   10                  15
   Thr Ala Ala Ala
               20
<210> 3625
   <211> 20
   <212> PRT
   <213> Homo sapiens
<400> 3625
   Ala Lys Phe Glu Glu Leu Asn Met Asp Leu Phe Arg Ser Thr Met Lys
   1               5                   10                  15
   Pro Val Gln Lys
               20
<210> 3626
   <211> 20
   <212> PRT
   <213> Homo sapiens
<400> 3626
   Ala Lys Arg Ala Leu Ser Ser Gln His Gln Ala Arg Ile Glu Ile Glu
   1               5                   10                  15
   Ser Phe Tyr Glu
               20
<210> 3627
   <211> 20
   <212> PRT
   <213> Homo sapiens
<400> 3627
   Ala Lys Arg Ala Leu Ser Ser Gln His Gln Ala Arg Ile Glu Ile Glu
   1 5 10 15
   Ser Phe Tyr Glu
   20
<210> 3628
   <211> 20
   <212> PRT
   <213> Homo sapiens
<400> 3628
   Ala Thr Asn Gly Asp Thr His Leu Gly Gly Glu Asp Phe Asp Gln Arg
   1 5 10 15
   Val Met Glu His
   20
<210> 3629
   <211> 20
   <212> PRT
   <213> Homo sapiens
<400> 3629
   Glu Asp Lys Lys Glu Asp Val Gly Thr Val Val Gly Ile Asp Leu Gly
   1 5 10 15
   Thr Thr Tyr Ser
   20
<210> 3630
   <211> 21
   <212> PRT
   <213> Homo sapiens
<400> 3630
   Glu Asp Lys Lys Leu Lys Glu Arg Ile Asp Thr Arg Asn Glu Leu Glu
   1 5 10 15
   Ser Tyr Ala Tyr Ser
   20
<210> 3631
   <211> 20
   <212> PRT
   <213> Homo sapiens
<400> 3631
   Glu Ser His Gln Asp Ala Asp Ile Glu Asp Phe Lys Ala Lys Lys Lys
   1 5 10 15
   Glu Leu Glu Glu
   20
<210> 3632
   <211> 20
   <212> PRT
   <213> Homo sapiens
<400> 3632
   Phe Ala Glu Glu Asp Lys Lys Leu Lys Glu Arg Ile Asp Thr Arg Asn
   1 5 10 15
   Glu Leu Glu Ser
   20
<210> 3633
   <211> 20
   <212> PRT
   <213> Homo sapiens
<400> 3633
   Gly Ser Ala Gly Pro Pro Pro Thr Gly Glu Glu Asp Thr Ala Glu Leu
   1 5 10 15
   His His His His
   20
<210> 3634
   <211> 20
   <212> PRT
   <213> Homo sapiens
<400> 3634
   Ile Asp Glu Ile Val Leu Val Gly Gly Ser Thr Arg Ile Pro Lys Ile
   1 5 10 15
   Gln Gln Leu Val
   20
<210> 3635
   <211> 20
   <212> PRT
   <213> Homo sapiens
<400> 3635
   Lys Asp Asn Arg Ala Val Gln Lys Leu Arg Arg Glu Val Glu Lys Ala
   1               5                   10                  15
   Lys Arg Ala Leu
               20
<210> 3636
   <211> 20
   <212> PRT
   <213> Homo sapiens
<400> 3636
   Lys Glu Thr Met Glu Lys Ala Val Glu Glu Lys Ile Glu Trp Leu Glu
   1               5                   10                  15
   Ser His Gln Asp
               20
<210> 3637
   <211> 16
   <212> PRT
   <213> Homo sapiens
<400> 3637
   Lys Ile His Ala Arg Glu Ile Phe Asp Ser Arg Gly Asn Pro Thr Val
   1               5                   10                  15
<210> 3638
   <211> 20
   <212> PRT
   <213> Homo sapiens
<400> 3638
   Lys Leu Arg Arg Glu Val Glu Lys Ala Lys Arg Ala Leu Ser Ser Gln
   1               5                   10                  15
   His Gln Ala Arg
               20
<210> 3639
   <211> 20
   <212> PRT
   <213> Homo sapiens
<400> 3639
   Lys Thr Gly Lys Asp Val Arg Lys Asp Asn Arg Ala Val Gln Lys Leu
   1               5                   10                  15
   Arg Arg Glu Val
               20
<210> 3640
   <211> 20
   <212> PRT
   <213> Homo sapiens
<400> 3640
   Lys Thr Gly Lys Asp Val Arg Lys Asp Asn Arg Ala Val Gln Lys Leu
   1 5 10 15
   Arg Arg Glu Val
   20
<210> 3641
   <211> 20
   <212> PRT
   <213> Homo sapiens
<400> 3641
   Leu Tyr Lys Lys Lys Thr Gly Lys Asp Val Arg Lys Asp Asn Arg Ala
   1 5 10 15
   Val Gln Lys Leu
   20
<210> 3642
   <211> 20
   <212> PRT
   <213> Homo sapiens
<400> 3642
   Asn Lys Ile Thr Ile Thr Asn Asp Gln Asn Arg Leu Thr Pro Glu Glu
   1 5 10 15
   Ile Glu Arg Met
   20
<210> 3643
   <211> 20
   <212> PRT
   <213> Homo sapiens
<400> 3643
   Asn Pro Glu Asn Thr Val Phe Asp Ala Lys Arg Leu Ile Gly Arg Thr
   1 5 10 15
   Trp Asn Asp Pro
   20
<210> 3644
   <211> 20
   <212> PRT
   <213> Homo sapiens
<400> 3644
   Gln Asn Arg Leu Thr Pro Glu Glu Ile Glu Arg Met Val Asn Asp Ala
   1               5                   10                  15
   Glu Lys Phe Ala
               20
<210> 3645
   <211> 20
   <212> PRT
   <213> Homo sapiens
<400> 3645
   Arg Lys Asp Asn Arg Ala Val Gln Lys Leu Arg Arg Glu Val Glu Lys
   1               5                   10                  15
   Ala Lys Arg Ala
   20
<210> 3646
   <211> 20
   <212> PRT
   <213> Homo sapiens
<400> 3646
   Arg Lys Asp Asn Arg Ala Val Gln Lys Leu Arg Arg Glu Val Glu Lys
   1               5                   10                  15
   Ala Lys Arg Ala
               20
<210> 3647
   <211> 20
   <212> PRT
   <213> Homo sapiens
<400> 3647
   Ser Tyr Val Ala Phe Thr Pro Glu Gly Glu Arg Leu Ile Gly Asp Ala
   1               5                   10                  15
   Ala Lys Asn Gln
               20
<210> 3648
   <211> 20
   <212> PRT
   <213> Homo sapiens
<400> 3648
   Thr Phe Glu Ile Asp Val Asn Gly Ile Leu Arg Val Thr Ala Glu Asp
   1 5 10 15
   Lys Gly Thr Gly
   20
<210> 3649
   <211> 20
   <212> PRT
   <213> Homo sapiens
<400> 3649
   Thr Phe Glu Ile Asp Val Asn Gly Ile Leu Arg Val Thr Ala Glu Asp
   1 5 10 15
   Lys Gly Thr Gly
   20
<210> 3650
   <211> 20
   <212> PRT
   <213> Homo sapiens
<400> 3650
   Thr Val Gly Gly Val Met Thr Lys Leu Ile Pro Arg Asn Thr Val Val
   1 5 10 15
   Pro Thr Lys Lys
   20
<210> 3651
   <211> 20
   <212> PRT
   <213> Homo sapiens
<400> 3651
   Thr Val Pro Ala Tyr Phe Asn Asp Ala Gln Arg Gln Ala Thr Lys Asp
   1 5 10 15
   Ala Gly Thr Ile
   20
<210> 3652
   <211> 20
   <212> PRT
   <213> Homo sapiens
<400> 3652
   Thr Val Thr Ile Lys Val Tyr Glu Gly Glu Arg Pro Leu Thr Lys Asp
   1               5                   10                  15
   Asn His Leu Leu
               20
<210> 3653
   <211> 20
   <212> PRT
   <213> Homo sapiens
<400> 3653
   Val Glu Ile Ile Ala Asn Asp Gln Gly Asn Arg Ile Thr Pro Ser Tyr
   1               5                   10                  15
   Val Ala Phe Thr
               20
<210> 3654
   <211> 20
   <212> PRT
   <213> Homo sapiens
<400> 3654
   Val Lys Glu Phe Phe Asn Gly Lys Glu Pro Ser Arg Gly Ile Asn Pro
   1               5                   10                  15
   Asp Glu Ala Val
               20
<210> 3655
   <211> 20
   <212> PRT
   <213> Homo sapiens
<400> 3655
   Tyr Glu Gly Glu Asp Phe Ser Glu Thr Leu Thr Arg Ala Lys Phe Glu
   1               5                   10                  15
   Glu Leu Asn Met
               20
<210> 3656
   <211> 327
   <212> PRT
   <213> Homo sapiens
<400> 3656
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
   115 120 125
   Asp Thr Leu Met Ile Ser Arg Thr Pro Glu Val Thr Cys Val Val Val
   130 135 140
   Asp Val Ser Gln Glu Asp Pro Glu Val Gln Phe Asn Trp Tyr Val Asp
   145 150 155 160
   Gly Val Glu Val His Asn Ala Lys Thr Lys Pro Arg Glu Glu Gln Phe
   165 170 175
   Asn Ser Thr Tyr Arg Val Val Ser Val Leu Thr Val Leu His Gln Asp
   180 185 190
   Trp Leu Asn Gly Lys Glu Tyr Lys Cys Lys Val Ser Asn Lys Gly Leu
   195 200 205
   Pro Ser Ser Ile Glu Lys Thr Ile Ser Lys Ala Lys Gly Gln Pro Arg
   210 215 220
   Glu Pro Gln Val Tyr Thr Leu Pro Pro Ser Gln Glu Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Arg Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu Gly Lys
   325
<210> 3657
   <211> 15
   <212> PRT
   <213> Rattus norvegicus
<400> 3657
   Gly Ile Ala Gly Phe Lys Gly Glu Gln Gly Pro Lys Gly Glu Thr
   1 5 10 15
<210> 3658
   <211> 15
   <212> PRT
   <213> Rattus norvegicus
<400> 3658
   Gly Ile Ala Gly Phe Lys Gly Glu Gln Gly Pro Lys Gly Glu Thr
   1 5 10 15
<210> 3659
   <211> 15
   <212> PRT
   <213> Rattus norvegicus
<400> 3659
   Gly Ile Ala Gly Phe Lys Gly Glu Gln Gly Pro Lys Gly Glu Thr
   1 5 10 15
<210> 3660
   <211> 15
   <212> PRT
   <213> Unknown
<220>
   <223> epitope involved in rheumatoid arthritis
<400> 3660
   Gly Ile Ala Gly Asn Lys Gly Asp Gln Gly Pro Lys Gly Glu Asx
   1 5 10 15
<210> 3661
   <211> 20
   <212> PRT
   <213> Mycobacterium tuberculosis
<400> 3661
   Thr Leu Val Val Asn Lys Ile Arg Gly Thr Phe Lys Ser Val Ala Val
   1 5 10 15
   Lys Ala Pro Gly
   20
<210> 3662
   <211> 20
   <212> PRT
   <213> Chlamydia pneumoniae
<400> 3662
   Thr Leu Val Val Asn Lys Leu Gln Gly Leu Leu Gln Val Thr Val Val
   1 5 10 15
   Thr Ile Pro Gln
   20
<210> 3663
   <211> 14
   <212> PRT
   <213> Homo sapiens
<400> 3663
   Leu Thr Val Gly Leu Thr Arg Arg Gly Gln Pro Arg Gln Tyr
   1 5 10
<210> 3664
   <211> 14
   <212> PRT
   <213> Homo sapiens
<400> 3664
   Leu Thr Val Gly Leu Thr Arg Arg Gly Gln Pro Arg Gln Tyr
   1 5 10
<210> 3665
   <211> 13
   <212> PRT
   <213> Homo sapiens
<400> 3665
   Tyr Asn Gln Tyr Ser Gln Arg Tyr His Gln Arg Thr Asn
   1 5 10
<210> 3666
   <211> 13
   <212> PRT
   <213> Homo sapiens
<400> 3666
   Ala Pro Ala Pro Thr Ala Arg Asn Lys Leu Thr Ser Glu
   1 5 10
<210> 3667
   <211> 21
   <212> PRT
   <213> Homo sapiens
<400> 3667
   Asp Ala Pro Arg Ala Val Phe Pro Ser Ile Val Gly Arg Pro Arg His
   1 5 10 15
   Gln Gly Val Met Val
   20
<210> 3668
   <211> 12
   <212> PRT
   <213> Homo sapiens
<400> 3668
   Glu Ala Gln Ser Lys Arg Gly Ile Leu Thr Leu Lys
   1 5 10
<210> 3669
   <211> 11
   <212> PRT
   <213> Homo sapiens
<400> 3669
   Glu Arg Leu Gly Pro Leu Val Glu Gln Gly Arg
   1 5 10
<210> 3670
   <211> 10
   <212> PRT
   <213> Homo sapiens
<400> 3670
   Gly Pro Ser Ile Val His Arg Lys Cys Phe
   1 5 10
<210> 3671
   <211> 16
   <212> PRT
   <213> Homo sapiens
<400> 3671
   Ile Trp His His Thr Phe Tyr Asn Glu Leu Arg Val Ala Pro Glu Glu
   1 5 10 15
<210> 3672
   <211> 15
   <212> PRT
   <213> Homo sapiens
<400> 3672
   Lys Leu Thr Ser Glu Ala Arg Gly Arg Ile Pro Val Ala Gln Lys
   1 5 10 15
<210> 3673
   <211> 20
   <212> PRT
   <213> Homo sapiens
<400> 3673
   Leu Ala Val Tyr Gln Ala Gly Ala Arg Glu Gly Ala Glu Arg Gly Leu
   1 5 10 15
   Ser Ala Ile Arg
   20
<210> 3674
   <211> 14
   <212> PRT
   <213> Homo sapiens
<400> 3674
   Leu Asp Leu Ala Gly Arg Asp Leu Thr Asp Tyr Leu Met Lys
   1 5 10
<210> 3675
   <211> 27
   <212> PRT
   <213> Homo sapiens
<400> 3675
   Met Lys Ile Leu Thr Glu Arg Gly Tyr Ser Phe Thr Thr Thr Ala Glu
   1 5 10 15
   Arg Glu Ile Val Arg Asp Ile Lys Glu Lys Leu
   20 25
<210> 3676
   <211> 11
   <212> PRT
   <213> Homo sapiens
<400> 3676
   Pro Gly Ile Ala Asp Arg Met Gln Lys Glu Ile
   1 5 10
<210> 3677
   <211> 13
   <212> PRT
   <213> Homo sapiens
<400> 3677
   Arg Gln Val Asp Ala Arg Arg Asn Val Pro Gln Asn Asp
   1 5 10
<210> 3678
   <211> 13
   <212> PRT
   <213> Homo sapiens
<400> 3678
   Gln Tyr Met Arg Ala Asp Gln Ala Ala Gly Gly Leu Arg
   1 5 10
<210> 3679
   <211> 13
   <212> PRT
   <213> Homo sapiens
<400> 3679
   Ser Ala Val Arg Ala Arg Ser Ser Val Pro Gly Val Arg
   1 5 10
<210> 3680
   <211> 19
   <212> PRT
   <213> Homo sapiens
<400> 3680
   Ala Leu Met Arg Gly Lys Ser Thr Glu Glu Ala Arg Lys Glu Leu Gln
   1 5 10 15
   Ala Ala Gly
<210> 3681
   <211> 19
   <212> PRT
   <213> Homo sapiens
<400> 3681
   Ala Met Leu Pro Tyr Asp Gln Tyr Leu His Arg Phe Ala Ala Tyr Phe
   1 5 10 15
   Gln Gln Gly
<210> 3682
   <211> 19
   <212> PRT
   <213> Homo sapiens
<400> 3682
   Ala Ser Thr Asn Gly Leu Ile Asn Phe Ile Lys Gln Gln Arg Glu Ala
   1 5 10 15
   Arg Val Gln
<210> 3683
   <211> 19
   <212> PRT
   <213> Homo sapiens
<400> 3683
   Asp Leu Ala Lys Ser Arg Gly Val Glu Ala Ala Arg Glu Arg Met Phe
   1 5 10 15
   Asn Gly Glu
<210> 3684
   <211> 19
   <212> PRT
   <213> Homo sapiens
<400> 3684
   Glu Asp Leu Glu Arg Leu Leu Pro His Lys Val Phe Glu Gly Asn Arg
   1 5 10 15
   Pro Thr Asn
<210> 3685
   <211> 19
   <212> PRT
   <213> Homo sapiens
<400> 3685
   Phe Glu Phe Trp Asp Trp Val Gly Gly Arg Tyr Ser Leu Trp Ser Ala
   1 5 10 15
   Ile Gly Leu
<210> 3686
   <211> 19
   <212> PRT
   <213> Homo sapiens
<400> 3686
   Lys Leu Gln Gln Trp Tyr Arg Glu His Arg Ser Glu Leu Asn Leu Arg
   1 5 10 15
   Arg Leu Phe
<210> 3687
   <211> 19
   <212> PRT
   <213> Homo sapiens
<400> 3687
   Asn Tyr Thr Glu Gly Arg Ala Val Leu His Val Ala Leu Arg Asn Arg
   1               5                   10                  15
   Ser Asn Thr
<210> 3688
   <211> 19
   <212> PRT
   <213> Homo sapiens
<400> 3688
   Ser Phe Cys Gln Arg Val Arg Ser Gly Asp Trp Lys Gly Tyr Thr Gly
   1               5                   10                  15
   Lys Thr Ile
<210> 3689
   <211> 16
   <212> PRT
   <213> Homo sapiens
<400> 3689
   Val Tyr Ala Thr Arg Ser Ser Ala Val Arg Leu Arg Ser Ser Val Pro
   1               5                   10                  15
<210> 3690
   <211> 17
   <212> PRT
   <213> Homo sapiens
<400> 3690
   Asn Glu Glu Gly Phe Phe Ser Ala Arg Gly His Arg Pro Leu Asp Lys
   1               5                   10                  15
   Lys
<210> 3691
   <211> 19
   <212> PRT
   <213> Homo sapiens
<400> 3691
   Ser His Gln Glu Ser Thr Arg Gly Arg Ser Arg Gly Arg Ser Gly Arg
   1               5                   10                  15
   Ser Gly Ser
<210> 3692
   <211> 5
   <212> PRT
   <213> Homo sapiens
<400> 3692
   Thr Arg Gly Arg Ser
   1               5
<210> 3693
   <211> 36
   <212> PRT
   <213> Unknown
<220>
   <223> epitope involved in rheumatoid arthritis
<400> 3693
   His Ser Thr Lys Arg Gly His Ala Lys Ser Arg Pro Val Arg Gly His
   1               5                   10                  15
   Gln Ser His Gln Glu Ser Thr Arg Gly Arg Ser Arg Gly Arg Ser Gly
               20                  25                  30
   Arg Ser Gly Ser
           35
<210> 3694
   <211> 15
   <212> PRT
   <213> Trypanosoma cruzi
<400> 3694
   Ala Glu Glu Glu Glu Asp Asp Asp Met Gly Phe Gly Leu Phe Asp
   1               5                   10                  15
<210> 3695
   <211> 32
   <212> PRT
   <213> Trypanosoma cruzi
<400> 3695
   Ala Ser Ala Pro Thr Ala Ala Ala Ala Ala Ser Gly Gly Ala Ala Ala
   1               5                   10                  15
   Pro Ala Ala Ala Ala Glu Glu Glu Glu Asp Asp Asp Met Gly Phe Gly
               20                  25                  30
<210> 3696
   <211> 35
   <212> PRT
   <213> Trypanosoma cruzi
<400> 3696
   Ala Ser Ala Pro Thr Ala Ala Ala Ala Ala Ser Gly Gly Ala Ala Ala
   1 5 10 15
   Pro Ala Ala Ala Ala Glu Glu Glu Glu Asp Asp Asp Met Gly Phe Gly
   20 25 30
   Leu Phe Asp
   35
<210> 3697
   <211> 18
   <212> PRT
   <213> Homo sapiens
<400> 3697
   Cys Ala Pro Glu Ser Ile Glu Phe Pro Val Ser Glu Ala Arg Val Leu
   1 5 10 15
   Glu Asp
<210> 3698
   <211> 10
   <212> PRT
   <213> Homo sapiens
<400> 3698
   Asp Asp Asp Met Gly Phe Gly Leu Phe Asp
   1 5 10
<210> 3699
   <211> 16
   <212> PRT
   <213> Mycobacterium tuberculosis
<400> 3699
   Asp Leu Ser Leu Leu Gly Lys Ala Arg Lys Val Val Val Thr Lys Asp
   1 5 10 15
<210> 3700
   <211> 13
   <212> PRT
   <213> Trypanosoma cruzi
<400> 3700
   Glu Glu Glu Asp Asp Asp Met Gly Phe Gly Leu Phe Asp
   1 5 10
<210> 3701
   <211> 13
   <212> PRT
   <213> Homo sapiens
<400> 3701
   Glu Glu Ser Asp Asp Asp Met Gly Phe Gly Leu Phe Asp
   1 5 10
<210> 3702
   <211> 17
   <212> PRT
   <213> Homo sapiens
<400> 3702
   Glu Asn Pro Val Val His Phe Phe Lys Asn Ile Val Thr Pro Arg Thr
   1 5 10 15
   Pro
<210> 3703
   <211> 5
   <212> PRT
   <213> Homo sapiens
<400> 3703
   Glu Arg Lys Arg Arg
   1 5
<210> 3704
   <211> 12
   <212> PRT
   <213> Mus musculus
<400> 3704
   Phe Tyr Asn Gln Lys Phe Lys Gly Lys Ala Thr Leu
   1 5 10
<210> 3705
   <211> 15
   <212> PRT
   <213> Human herpesvirus
<400> 3705
   Gly Asp Asp Gly Asp Asp Gly Asp Glu Gly Gly Asp Gly Asp Glu
   1 5 10 15
<210> 3706
   <211> 24
   <212> PRT
   <213> Human herpesvirus
<400> 3706
   Gly Gly Asp Asn His Gly Arg Gly Arg Gly Arg Gly Arg Gly Arg Gly
   1 5 10 15
   Gly Gly Arg Pro Gly Ala Pro Gly
   20
<210> 3707
   <211> 9
   <212> PRT
   <213> Human herpesvirus
<400> 3707
   Gly Leu Cys Thr Leu Val Ala Met Leu
   1 5
<210> 3708
   <211> 18
   <212> PRT
   <213> Homo sapiens
<400> 3708
   Gly Pro Ser Gln Gln Val Met Thr Pro Gln Gly Arg Gly Thr Val Ala
   1 5 10 15
   Ala Ala
<210> 3709
   <211> 10
   <212> PRT
   <213> Homo sapiens
<400> 3709
   His Leu Lys Ala Val Lys Met Thr Leu Lys
   1 5 10
<210> 3710
   <211> 9
   <212> PRT
   <213> Homo sapiens
<400> 3710
   Ile Leu Gln Asp Gly Arg Ile Phe Ile
   1 5
<210> 3711
   <211> 22
   <212> PRT
   <213> Artemia franciscana
<400> 3711
   Lys Lys Glu Glu Lys Lys Glu Glu Ser Glu Glu Glu Asp Glu Asp Met
   1 5 10 15
   Gly Phe Gly Leu Phe Asp
   20
<210> 3712
   <211> 24
   <212> PRT
   <213> Mus musculus
<400> 3712
   Lys Arg His Arg Lys Val Leu Arg Asp Asn Ile Gln Gly Ile Thr Lys
   1 5 10 15
   Pro Ala Ile Arg Arg Leu Ala Arg
   20
<210> 3713
   <211> 17
   <212> PRT
   <213> Homo sapiens
<400> 3713
   Lys Arg Val Leu Gly Leu Val Leu Leu Arg Gly Glu Asn Leu Val Ser
   1 5 10 15
   Met
<210> 3714
   <211> 7
   <212> PRT
   <213> Trypanosoma cruzi
<400> 3714
   Met Gly Phe Gly Leu Phe Asp
   1 5
<210> 3715
   <211> 20
   <212> PRT
   <213> Homo sapiens
<400> 3715
   Pro Thr Arg Ala Glu Thr Arg Glu Glu Arg Met Glu Arg Lys Arg Arg
   1 5 10 15
   Glu Lys Ile Glu
   20
<210> 3716
   <211> 16
   <212> PRT
   <213> Homo sapiens
<400> 3716
   Gln Pro His Asp Leu Gly Lys Val Gly Glu Val Ile Val Thr Lys Asp
   1 5 10 15
<210> 3717
   <211> 9
   <212> PRT
   <213> Homo sapiens
<400> 3717
   Ser Ile Arg Gly Asn Asn Ile Arg Tyr
   1 5
<210> 3718
   <211> 24
   <212> PRT
   <213> Mus musculus
<400> 3718
   Thr Tyr Thr Glu His Ala Lys Arg Lys Thr Val Thr Ala Met Asp Val
   1 5 10 15
   Val Tyr Ala Leu Lys Arg Gln Gly
   20
<210> 3719
   <211> 13
   <212> PRT
   <213> Human herpesvirus
<400> 3719
   Arg Gly Gln Gly Asp Gly Gly Arg Arg Lys Lys Gly Gly
   1 5 10
<210> 3720
   <211> 21
   <212> PRT
   <213> Mus musculus
<400> 3720
   Ala Ala Met Ala Ala Arg Pro His Ser Ile Asp Gly Arg Val Val Glu
   1 5 10 15
   Pro Lys Arg Ala Val
   20
<210> 3721
   <211> 22
   <212> PRT
   <213> Mus musculus
<400> 3721
   Ala Glu Val Arg Lys Ala Leu Ser Arg Gln Glu Met Gln Glu Val Gln
   1 5 10 15
   Ser Ser Arg Ser Gly Arg
   20
<210> 3722
   <211> 16
   <212> PRT
   <213> Homo sapiens
<400> 3722
   Cys Lys Pro Ile Ser Gly His Asn Ser Leu Phe Trp Tyr Arg Gln Thr
   1 5 10 15
<210> 3723
   <211> 16
   <212> PRT
   <213> Homo sapiens
<400> 3723
   Asp Ala Gly Val Ile Gln Ser Pro Arg His Glu Val Thr Glu Met Gly
   1 5 10 15
<210> 3724
   <211> 21
   <212> PRT
   <213> Mus musculus
<400> 3724
   Asp Lys Ile Val Leu Gln Lys Tyr His Thr Ile Asn Gly His Asn Ala
   1 5 10 15
   Glu Val Arg Lys Ala
   20
<210> 3725
   <211> 10
   <212> PRT
   <213> Homo sapiens
<400> 3725
   Asp Pro Ile Ser Gly His Val Ser Leu Phe
   1               5                   10
<210> 3726
   <211> 16
   <212> PRT
   <213> Homo sapiens
<400> 3726
   Lys Ile Gln Pro Ser Glu Pro Arg Asp Ser Ala Val Tyr Phe Cys Ala
   1               5                   10                  15
<210> 3727
   <211> 10
   <212> PRT
   <213> Homo sapiens
<400> 3727
   Lys Pro Ile Ser Gly His Asn Ser Leu Phe
   1               5                   10
<210> 3728
   <211> 21
   <212> PRT
   <213> Mus musculus
<400> 3728
   Leu Thr Asp Cys Val Val Met Arg Asp Pro Ala Ser Lys Arg Ser Arg
   1               5                   10                  15
   Gly Phe Gly Phe Val
               20
<210> 3729
   <211> 21
   <212> PRT
   <213> Mus musculus
<400> 3729
   Met Glu Arg Glu Lys Glu Gln Phe Arg Lys Leu Phe Ile Gly Gly Leu
   1               5                   10                  15
   Ser Phe Glu Thr Thr
               20
<210> 3730
   <211> 16
   <212> PRT
   <213> Homo sapiens
<400> 3730
   Gln Pro Leu Lys Glu Gln Pro Ala Leu Asn Asp Ser Arg Tyr Cys Leu
   1 5 10 15
<210> 3731
   <211> 21
   <212> PRT
   <213> Mus musculus
<400> 3731
   Arg Gly Phe Gly Phe Val Thr Phe Ser Ser Met Ala Glu Val Asp Ala
   1 5 10 15
   Ala Met Ala Ala Arg
   20
<210> 3732
   <211> 11
   <212> PRT
   <213> Homo sapiens
<400> 3732
   Arg Ser Leu Asp Phe Gln Ala Thr Thr Met Phe
   1 5 10
<210> 3733
   <211> 27
   <212> PRT
   <213> Mus musculus
<400> 3733
   Ser Gly Lys Pro Gly Ala His Val Thr Val Lys Lys Leu Phe Val Gly
   1 5 10 15
   Gly Ile Lys Glu Asp Thr Glu Glu His His Leu
   20 25
<210> 3734
   <211> 10
   <212> PRT
   <213> Homo sapiens
<400> 3734
   Ser Pro Arg Ser Gly Asp Leu Ser Val Tyr
   1 5 10
<210> 3735
   <211> 25
   <212> PRT
   <213> Homo sapiens
<400> 3735
   Ala Gln Gly Thr Leu Ser Lys Ile Phe Lys Leu Gly Gly Arg Asp Ser
   1               5                   10                  15
   Arg Ser Gly Ser Pro Met Ala Arg Arg
               20                  25
<210> 3736
   <211> 19
   <212> PRT
   <213> Mus musculus
<400> 3736
   Leu Ser Arg Phe Ser Trp Gly Ala Glu Gly Gln Lys Pro Gly Phe Gly
   1               5                   10                  15
   Tyr Gly Gly
<210> 3737
   <211> 21
   <212> PRT
   <213> Mus musculus
<400> 3737
   Met Glu Val Gly Trp Tyr Arg Ser Pro Phe Ser Arg Val Val His Leu
   1               5                   10                  15
   Tyr Arg Asn Gly Lys
               20
<210> 3738
   <211> 18
   <212> PRT
   <213> Homo sapiens
<400> 3738
   Lys Glu Gln Phe Leu Asp Gly Asp Gly Trp Thr Ser Arg Trp Ile Glu
   1               5                   10                  15
   Ser Lys
<210> 3739
   <211> 21
   <212> PRT
   <213> Homo sapiens
<400> 3739
   Arg Ile His Met Val Tyr Ser Lys Arg Ser Gly Lys Pro Arg Gly Tyr
   1               5                   10                  15
   Ala Phe Ile Glu Tyr
               20
<210> 3740
   <211> 10
   <212> PRT
   <213> Homo sapiens
<400> 3740
   Tyr Thr Cys Pro Leu Cys Arg Ala Pro Val
   1               5                   10
<210> 3741
   <211> 10
   <212> PRT
   <213> Homo sapiens
<400> 3741
   Asp Trp Glu Tyr Ser Val Trp Leu Ser Asn
   1               5                   10
<210> 3742
   <211> 19
   <212> PRT
   <213> Homo sapiens
<400> 3742
   Gly Tyr Tyr Trp Ser Trp Ile Arg Gln Pro Pro Gly Lys Gly Glu Glu
   1               5                   10                  15
   Trp Ile Gly
<210> 3743
   <211> 9
   <212> PRT
   <213> Homo sapiens
<400> 3743
   Lys Gly Glu Gln Gly Glu Pro Gly Ala
   1               5
<210> 3744
   <211> 18
   <212> PRT
   <213> Homo sapiens
<400> 3744
   Val Thr Trp Glu Val Leu Glu Gly Glu Val Glu Lys Glu Ala Leu Lys
   1 5 10 15
   Lys Ile
<210> 3745
   <211> 26
   <212> PRT
   <213> Homo sapiens
<400> 3745
   Ala Gly Ala Ala Arg Asp Ala Leu Gln Gly Phe Lys Ile Thr Gln Asn
   1 5 10 15
   Asn Ala Met Lys Ile Ser Phe Ala Lys Lys
   20 25
<210> 3746
   <211> 20
   <212> PRT
   <213> Homo sapiens
<400> 3746
   Ala Asn Asn Gly Asn Leu Gln Leu Arg Asn Lys Glu Val Thr Trp Glu
   1 5 10 15
   Val Leu Glu Gly
   20
<210> 3747
   <211> 21
   <212> PRT
   <213> Homo sapiens
<400> 3747
   Asp Gly Tyr Val Trp Gln Val Thr Asp Met Asn Arg Leu His Arg Phe
   1 5 10 15
   Leu Cys Phe Gly Ser
   20
<210> 3748
   <211> 15
   <212> PRT
   <213> Bos taurus
<400> 3748
   Asp Val Val Tyr Ala Leu Lys Arg Gln Gly Arg Thr Leu Tyr Gly
   1 5 10 15
<210> 3749
   <211> 18
   <212> PRT
   <213> Bos taurus
<400> 3749
   Asp Val Val Tyr Ala Leu Lys Arg Gln Gly Arg Thr Leu Tyr Gly Phe
   1               5                   10                  15
   Gly Gly
<210> 3750
   <211> 13
   <212> PRT
   <213> Artemia franciscana
<400> 3750
   Glu Glu Glu Asp Glu Asp Met Gly Phe Gly Leu Phe Asp
   1               5                   10
<210> 3751
   <211> 22
   <212> PRT
   <213> Homo sapiens
<400> 3751
   Glu Leu Tyr Lys Glu Lys Ala Leu Ser Val Glu Thr Glu Lys Leu Leu
   1               5                   10                  15
   Lys Tyr Leu Glu Ala Val
               20
<210> 3752
   <211> 24
   <212> PRT
   <213> Homo sapiens
<400> 3752
   Glu Pro Ala Val Tyr Phe Lys Glu Gln Phe Leu Asp Gly Asp Gly Trp
   1               5                   10                  15
   Thr Ser Arg Trp Ile Glu Ser Lys
               20
<210> 3753
   <211> 15
   <212> PRT
   <213> Bos taurus
<400> 3753
   Gly Ile Thr Lys Pro Ala Ile Arg Arg Leu Ala Arg Arg Gly Gly
   1 5 10 15
<210> 3754
   <211> 23
   <212> PRT
   <213> Homo sapiens
<400> 3754
   Gly Arg Gly Arg Gly Arg Gly Arg Gly Arg Gly Arg Gly Arg Gly Arg
   1 5 10 15
   Gly Arg Gly Gly Pro Arg Arg
   20
<210> 3755
   <211> 16
   <212> PRT
   <213> Homo sapiens
<400> 3755
   Gly Ser Gly Lys Gly Lys Val Gln Phe Gln Gly Lys Lys Thr Lys Phe
   1 5 10 15
<210> 3756
   <211> 17
   <212> PRT
   <213> Homo sapiens
<400> 3756
   Lys Ala Leu Ser Val Glu Thr Glu Lys Leu Leu Lys Tyr Leu Glu Ala
   1 5 10 15
   Val
<210> 3757
   <211> 24
   <212> PRT
   <213> Homo sapiens
<400> 3757
   Lys Leu Ile Val Cys Gly Met Thr Ser Asn Gly Phe Thr Ile Ala Asp
   1 5 10 15
   Pro Asp Asp Arg Gly Met Leu Asp
   20
<210> 3758
   <211> 24
   <212> PRT
   <213> Homo sapiens
<400> 3758
   Lys Met Thr Leu Lys Asn Arg Glu Pro Val Gln Leu Glu Thr Leu Ser
   1 5 10 15
   Ile Arg Gly Asn Arg Ile Arg Tyr
   20
<210> 3759
   <211> 15
   <212> PRT
   <213> Homo sapiens
<400> 3759
   Lys Pro Lys Ala Ala Lys Pro Lys Lys Ala Ala Ala Lys Lys Lys
   1 5 10 15
<210> 3760
   <211> 11
   <212> PRT
   <213> Homo sapiens
<400> 3760
   Met Ala Val Pro Glu Thr Arg Pro Asn His Thr
   1 5 10
<210> 3761
   <211> 23
   <212> PRT
   <213> Homo sapiens
<400> 3761
   Met Glu Glu Ser Val Asn Gln Met Gln Pro Leu Asn Glu Lys Gln Ile
   1 5 10 15
   Ala Asn Ser Gln Asp Gly Tyr
   20
<210> 3762
   <211> 20
   <212> PRT
   <213> Homo sapiens
<400> 3762
   Met Lys Leu Val Arg Phe Leu Met Lys Leu Ser His Glu Thr Val Thr
   1 5 10 15
   Ile Glu Leu Lys
   20
<210> 3763
   <211> 17
   <212> PRT
   <213> Enterovirus A
<400> 3763
   Met Val Thr Ser Thr Ile Thr Glu Lys Leu Leu Lys Asn Leu Val Lys
   1 5 10 15
   Ile
<210> 3764
   <211> 10
   <212> PRT
   <213> Homo sapiens
<400> 3764
   Asn Gly Trp Ser His Lys Asp Leu Leu Arg
   1 5 10
<210> 3765
   <211> 7
   <212> PRT
   <213> Homo sapiens
<400> 3765
   Pro Pro Gly Met Arg Pro Pro
   1 5
<210> 3766
   <211> 21
   <212> PRT
   <213> Mus musculus
<400> 3766
   Pro Thr Arg Ala Glu Thr Arg Glu Glu Arg Met Glu Arg Lys Arg Arg
   1 5 10 15
   Glu Lys Ile Glu Arg
   20
<210> 3767
   <211> 11
   <212> PRT
   <213> Homo sapiens
<400> 3767
   Pro Thr Arg Ala Pro Ser Gly Pro Arg Pro Pro
   1 5 10
<210> 3768
   <211> 21
   <212> PRT
   <213> Mus musculus
<400> 3768
   Arg Ile His Met Val Tyr Ser Lys Arg Ser Gly Lys Pro Arg Gly Tyr
   1 5 10 15
   Ala Phe Ile Glu Tyr
   20
<210> 3769
   <211> 21
   <212> PRT
   <213> Mus musculus
<400> 3769
   Arg Ile His Met Val Tyr Ser Lys Arg Ser Gly Lys Pro Arg Gly Tyr
   1 5 10 15
   Ala Phe Ile Glu Tyr
   20
<210> 3770
   <211> 20
   <212> PRT
   <213> Homo sapiens
<400> 3770
   Arg Pro Asp Ala Glu Tyr Trp Asn Ser Gln Lys Asp Leu Leu Glu Gln
   1 5 10 15
   Lys Arg Gly Arg
   20
<210> 3771
   <211> 20
   <212> PRT
   <213> Homo sapiens
<400> 3771
   Arg Pro Asp Ala Glu Tyr Trp Asn Ser Gln Lys Asp Leu Leu Glu Gln
   1 5 10 15
   Arg Arg Ala Ala
   20
<210> 3772
   <211> 11
   <212> PRT
   <213> Homo sapiens
<400> 3772
   Arg Arg Arg Glu Gly Pro Asp Arg Ser Pro Arg
   1               5                   10
<210> 3773
   <211> 29
   <212> PRT
   <213> Bos taurus
<400> 3773
   Ser Gly Arg Gly Lys Gly Gly Lys Gly Leu Gly Lys Gly Gly Ala Lys
   1               5                   10                  15
   Arg His Arg Lys Val Leu Arg Asp Asn Ile Gln Gly Ile
               20                  25
<210> 3774
   <211> 24
   <212> PRT
   <213> Homo sapiens
<400> 3774
   Ser Gln Phe Gly Gln Ile Leu Asp Ile Leu Val Ser Arg Ser Leu Lys
   1               5                   10                  15
   Met Arg Gly Gln Ala Phe Val Ile
               20
<210> 3775
   <211> 15
   <212> PRT
   <213> Homo sapiens
<400> 3775
   Thr Gly Ala Leu Asp Val Ile Arg Asn Phe Thr Leu Asp Met Ile
   1               5                   10                  15
<210> 3776
   <211> 18
   <212> PRT
   <213> Bos taurus
<400> 3776
   Thr Lys Pro Ala Ile Arg Arg Leu Ala Arg Arg Gly Gly Val Lys Arg
   1               5                   10                  15
   Ile Ser
<210> 3777
   <211> 22
   <212> PRT
   <213> Homo sapiens
<400> 3777
   Thr Lys Tyr Lys Gln Arg Asn Gly Trp Ser His Lys Asp Leu Leu Arg
   1               5                   10                  15
   Leu Ser His Leu Lys Pro
               20
<210> 3778
   <211> 20
   <212> PRT
   <213> Homo sapiens
<400> 3778
   Thr Leu His Lys Ala Phe Lys Gly Ser Ile Phe Val Val Phe Asp Ser
   1               5                   10                  15
   Ile Glu Ser Ala
               20
<210> 3779
   <211> 21
   <212> PRT
   <213> Homo sapiens
<400> 3779
   Val Cys Glu Lys Leu Cys Asn Glu Lys Leu Leu Lys Lys Ala Arg Ile
   1               5                   10                  15
   His Pro Phe His Ile
               20
<210> 3780
   <211> 37
   <212> PRT
   <213> Homo sapiens
<400> 3780
   Val Glu Pro Lys Val Lys Ser Lys Lys Arg Glu Ala Val Ala Gly Arg
   1               5                   10                  15
   Gly Arg Gly Arg Gly Arg Gly Arg Gly Arg Gly Arg Gly Arg Gly Arg
               20                  25                  30
   Gly Gly Pro Arg Arg
           35
<210> 3781
   <211> 15
   <212> PRT
   <213> Homo sapiens
<400> 3781
   Val Ser Lys Asp Asp Ser Ile Ile Ser Ser Leu Asp Val Thr Asp
   1               5                   10                  15
<210> 3782
   <211> 20
   <212> PRT
   <213> Mus musculus
<400> 3782
   Tyr Asp Glu Arg Pro Gly Pro Ser Pro Leu Pro His Arg Asp Arg Asp
   1               5                   10                  15
   Arg Asp Arg Glu
               20
<210> 3783
   <211> 21
   <212> PRT
   <213> Mus musculus
<400> 3783
   Tyr Leu Pro Pro Leu Glu Lys Leu Pro His Glu Lys His His Asn Gln
   1               5                   10                  15
   Pro Tyr Cys Gly Ile
               20
<210> 3784
   <211> 15
   <212> PRT
   <213> Homo sapiens
<400> 3784
   Ala Ala Arg Gly Arg Gly Arg Gly Met Gly Arg Gly Asn Ile Phe
   1               5                   10                  15
<210> 3785
   <211> 15
   <212> PRT
   <213> Homo sapiens
<400> 3785
   Ala Ala Arg Gly Arg Gly Arg Gly Met Gly Arg Gly Asn Ile Phe
   1               5                   10                  15
<210> 3786
   <211> 15
   <212> PRT
   <213> Homo sapiens
<400> 3786
   Ala Ile Ala Leu Arg Glu Tyr Arg Lys Lys Met Asp Ile Pro Ala
   1               5                   10                  15
<210> 3787
   <211> 20
   <212> PRT
   <213> Homo sapiens
<400> 3787
   Ala Ile Val Thr Lys Tyr Ile Thr Lys Gly Trp Lys Glu Val His Glu
   1               5                   10                  15
   Leu Tyr Lys Glu
               20
<210> 3788
   <211> 20
   <212> PRT
   <213> Homo sapiens
<400> 3788
   Ala Asn Asn Gly Asn Leu Gln Leu Arg Asn Lys Glu Val Thr Trp Glu
   1               5                   10                  15
   Val Leu Glu Gly
               20
<210> 3789
   <211> 15
   <212> PRT
   <213> Homo sapiens
<400> 3789
   Ala Arg Gly Arg Gly Arg Gly Met Gly Arg Gly Asn Ile Phe Gln
   1               5                   10                  15
<210> 3790
   <211> 3
   <212> PRT
   <213> Homo sapiens
<400> 3790
   Cys Val Pro
   1
<210> 3791
   <211> 6
   <212> PRT
   <213> Homo sapiens
<400> 3791
   Asp Gly Gln Phe Cys His
   1               5
<210> 3792
   <211> 20
   <212> PRT
   <213> Homo sapiens
<400> 3792
   Asp Leu Leu Arg Leu Ser His Leu Lys Pro Ser Ser Glu Gly Leu Ala
   1               5                   10                  15
   Ile Val Thr Lys
               20
<210> 3793
   <211> 15
   <212> PRT
   <213> Homo sapiens
<400> 3793
   Asp Val Glu Pro Lys Val Lys Ser Lys Lys Arg Glu Ala Val Ala
   1               5                   10                  15
<210> 3794
   <211> 15
   <212> PRT
   <213> Homo sapiens
<400> 3794
   Asp Val Glu Pro Lys Val Lys Ser Lys Lys Arg Glu Ala Val Ala
   1               5                   10                  15
<210> 3795
   <211> 15
   <212> PRT
   <213> Homo sapiens
<400> 3795
   Glu Ala Val Ala Gly Arg Gly Arg Gly Arg Gly Arg Gly Arg Gly
   1               5                   10                  15
<210> 3796
   <211> 18
   <212> PRT
   <213> Homo sapiens
<400> 3796
   Glu Lys Ala Leu Ser Val Glu Thr Glu Lys Leu Leu Lys Tyr Leu Glu
   1 5 10 15
   Ala Val
<210> 3797
   <211> 13
   <212> PRT
   <213> Homo sapiens
<400> 3797
   Glu Arg Asp Arg Lys Arg Glu Lys Arg Lys Pro Lys Ser
   1 5 10
<210> 3798
   <211> 8
   <212> PRT
   <213> Homo sapiens
<400> 3798
   Gly Gln Ile Pro Pro Gly Ala Met
   1 5
<210> 3799
   <211> 15
   <212> PRT
   <213> Homo sapiens
<400> 3799
   Gly Arg Gly Arg Gly Met Gly Arg Gly Asn Ile Phe Gln Lys Arg
   1 5 10 15
<210> 3800
   <211> 13
   <212> PRT
   <213> Homo sapiens
<400> 3800
   Gly Arg Gly Arg Gly Arg Gly Arg Gly Gly Pro Arg Arg
   1 5 10
<210> 3801
   <211> 15
   <212> PRT
   <213> Homo sapiens
<400> 3801
   Gly Arg Gly Arg Gly Arg Gly Arg Gly Arg Gly Gly Pro Arg Arg
   1 5 10 15
<210> 3802
   <211> 15
   <212> PRT
   <213> Homo sapiens
<400> 3802
   Gly Arg Gly Arg Gly Arg Gly Arg Gly Arg Gly Gly Pro Arg Arg
   1 5 10 15
<210> 3803
   <211> 13
   <212> PRT
   <213> Homo sapiens
<400> 3803
   Gly Arg Gly Arg Gly Arg Gly Arg Gly Arg Gly Arg Gly
   1 5 10
<210> 3804
   <211> 15
   <212> PRT
   <213> Homo sapiens
<400> 3804
   Gly Arg Gly Arg Gly Arg Gly Arg Gly Arg Gly Arg Gly Gly Pro
   1 5 10 15
<210> 3805
   <211> 15
   <212> PRT
   <213> Homo sapiens
<400> 3805
   Gly Arg Gly Arg Gly Arg Gly Arg Gly Arg Gly Arg Gly Arg Gly
   1 5 10 15
<210> 3806
   <211> 13
   <212> PRT
   <213> Homo sapiens
<400> 3806
   Gly Arg Gly Thr Val Ala Ala Ala Ala Ala Ala Ala Thr
   1 5 10
<210> 3807
   <211> 16
   <212> PRT
   <213> Homo sapiens
<400> 3807
   Gly Ser Gly Lys Gly Lys Val Gln Phe Gln Gly Lys Lys Thr Lys Phe
   1 5 10 15
<210> 3808
   <211> 12
   <212> PRT
   <213> Homo sapiens
<400> 3808
   His Met Val Tyr Ser Lys Arg Ser Gly Lys Pro Arg
   1 5 10
<210> 3809
   <211> 27
   <212> PRT
   <213> Homo sapiens
<400> 3809
   Ile Pro Pro Gly Arg Gly Thr Pro Met Gly Met Pro Pro Pro Gly Met
   1 5 10 15
   Arg Pro Pro Pro Pro Gly Met Arg Gly Leu Leu
   20 25
<210> 3810
   <211> 20
   <212> PRT
   <213> Homo sapiens
<400> 3810
   Lys Ala Leu Ser Val Glu Thr Glu Lys Leu Leu Lys Tyr Leu Glu Ala
   1 5 10 15
   Val Glu Lys Val
   20
<210> 3811
   <211> 12
   <212> PRT
   <213> Homo sapiens
<400> 3811
   Lys Glu Glu Gly Ser Arg Ser Asn Leu Gly Trp Leu
   1 5 10
<210> 3812
   <211> 12
   <212> PRT
   <213> Mus musculus
<400> 3812
   Lys Phe Lys Gly Lys Ala Thr Leu Thr Ser Asp Lys
   1 5 10
<210> 3813
   <211> 15
   <212> PRT
   <213> Homo sapiens
<400> 3813
   Lys Arg Glu Ala Val Ala Gly Arg Gly Arg Gly Arg Gly Arg Gly
   1 5 10 15
<210> 3814
   <211> 20
   <212> PRT
   <213> Homo sapiens
<400> 3814
   Lys Arg Thr Lys Asp Glu Leu Glu Val Ile His Leu Ile Glu Glu His
   1 5 10 15
   Arg Leu Val Arg
   20
<210> 3815
   <211> 13
   <212> PRT
   <213> Homo sapiens
<400> 3815
   Lys Val Lys Ser Lys Lys Arg Glu Ala Val Ala Gly Arg
   1 5 10
<210> 3816
   <211> 15
   <212> PRT
   <213> Homo sapiens
<400> 3816
   Leu Asp Gly Pro Thr Tyr Lys Arg Arg Leu Asp Glu Cys Glu Glu
   1 5 10 15
<210> 3817
   <211> 13
   <212> PRT
   <213> Homo sapiens
<400> 3817
   Leu Lys Ala Val Lys Met Thr Leu Lys Asn Arg Glu Pro
   1 5 10
<210> 3818
   <211> 15
   <212> PRT
   <213> Homo sapiens
<400> 3818
   Leu Lys Tyr Tyr Leu Ala Pro Lys Ile Glu Asp Glu Glu Gly Ser
   1               5                   10                  15
<210> 3819
   <211> 39
   <212> PRT
   <213> Homo sapiens
<400> 3819
   Met Phe Glu Ala Arg Leu Val Gln Gly Ser Ile Leu Lys Lys Val Leu
   1               5                   10                  15
   Glu Ala Leu Lys Asp Leu Ile Asn Glu Ala Cys Trp Asp Ile Ser Ser
               20                  25                  30
   Ser Gly Val Asn Leu Gln Ser
           35
<210> 3820
   <211> 13
   <212> PRT
   <213> Homo sapiens
<400> 3820
   Asn Thr His Leu Lys Ala Val Lys Met Thr Leu Lys Asn
   1               5                   10
<210> 3821
   <211> 12
   <212> PRT
   <213> Homo sapiens
<400> 3821
   Asn Trp His Gln Leu His Gly Lys Lys Glu Ala Tyr
   1               5                   10
<210> 3822
   <211> 13
   <212> PRT
   <213> Bos taurus
<400> 3822
   Pro Glu Pro Ala Lys Ser Ala Pro Ala Pro Lys Lys Gly
   1               5                   10
<210> 3823
   <211> 25
   <212> PRT
   <213> Bos taurus
<400> 3823
   Pro Glu Pro Ala Lys Ser Ala Pro Ala Pro Lys Lys Gly Ser Lys Lys
   1               5                   10                  15
   Ala Val Thr Lys Ala Gln Lys Lys Asp
               20                  25
<210> 3824
   <211> 7
   <212> PRT
   <213> Simian virus 40
<400> 3824
   Pro Lys Lys Lys Arg Lys Val
   1               5
<210> 3825
   <211> 15
   <212> PRT
   <213> Homo sapiens
<400> 3825
   Pro Lys Val Lys Ser Lys Lys Arg Glu Ala Val Ala Gly Arg Gly
   1               5                   10                  15
<210> 3826
   <211> 15
   <212> PRT
   <213> Homo sapiens
<400> 3826
   Pro Lys Val Lys Ser Lys Lys Arg Glu Ala Val Ala Gly Arg Gly
   1               5                   10                  15
<210> 3827
   <211> 35
   <212> PRT
   <213> Homo sapiens
<400> 3827
   Pro Lys Val Lys Ser Lys Lys Arg Glu Ala Val Ala Gly Arg Gly Arg
   1               5                   10                  15
   Gly Arg Gly Arg Gly Arg Gly Arg Gly Arg Gly Arg Gly Arg Gly Gly
               20                  25                  30
   Pro Arg Arg
           35
<210> 3828
   <211> 12
   <212> PRT
   <213> Homo sapiens
<400> 3828
   Gln Gly Phe Pro Phe Tyr Asp Lys Pro Met Arg Ile
   1               5                   10
<210> 3829
   <211> 15
   <212> PRT
   <213> Homo sapiens
<400> 3829
   Gln Val Ala Ala Arg Gly Arg Gly Arg Gly Met Gly Arg Gly Asn
   1               5                   10                  15
<210> 3830
   <211> 6
   <212> PRT
   <213> Homo sapiens
<400> 3830
   Gln Val Lys Gly Phe Val
   1               5
<210> 3831
   <211> 40
   <212> PRT
   <213> Homo sapiens
<400> 3831
   Gln Tyr Pro Pro Gly Arg Gly Gly Pro Pro Pro Pro Met Gly Arg Gly
   1               5                   10                  15
   Ala Pro Pro Pro Gly Met Met Gly Pro Pro Pro Gly Met Arg Pro Pro
               20                  25                  30
   Met Gly Pro Pro Met Gly Ile Pro
           35                  40
<210> 3832
   <211> 12
   <212> PRT
   <213> Homo sapiens
<400> 3832
   Arg Cys Arg Leu Cys Asp Glu Gly His Gly Leu Glu
   1 5 10
<210> 3833
   <211> 13
   <212> PRT
   <213> Homo sapiens
<400> 3833
   Arg Glu Ala Val Ala Gly Arg Gly Arg Gly Arg Gly Arg
   1 5 10
<210> 3834
   <211> 15
   <212> PRT
   <213> Homo sapiens
<400> 3834
   Arg Gly Arg Gly Met Gly Arg Gly Asn Ile Phe Gln Lys Arg Arg
   1 5 10 15
<210> 3835
   <211> 15
   <212> PRT
   <213> Homo sapiens
<400> 3835
   Arg Gly Arg Gly Arg Gly Met Gly Arg Gly Asn Ile Phe Gln Lys
   1 5 10 15
<210> 3836
   <211> 13
   <212> PRT
   <213> Homo sapiens
<400> 3836
   Arg Gly Arg Gly Arg Gly Arg Gly Arg Gly Arg Gly Gly
   1 5 10
<210> 3837
   <211> 13
   <212> PRT
   <213> Homo sapiens
<400> 3837
   Arg Gly Arg Gly Arg Gly Arg Gly Arg Gly Arg Gly Arg
   1 5 10
<210> 3838
   <211> 13
   <212> PRT
   <213> Homo sapiens
<400> 3838
   Ser Lys Lys Arg Glu Ala Val Ala Gly Arg Gly Arg Gly
   1               5                   10
<210> 3839
   <211> 15
   <212> PRT
   <213> Homo sapiens
<400> 3839
   Ser Lys Lys Arg Glu Ala Val Ala Gly Arg Gly Arg Gly Arg Gly
   1               5                   10                  15
<210> 3840
   <211> 22
   <212> PRT
   <213> Homo sapiens
<400> 3840
   Thr Lys Tyr Lys Gln Arg Asn Gly Trp Ser His Lys Asp Leu Leu Arg
   1               5                   10                  15
   Leu Ser His Leu Lys Pro
               20
<210> 3841
   <211> 22
   <212> PRT
   <213> Homo sapiens
<400> 3841
   Thr Lys Tyr Lys Gln Arg Asn Gly Trp Ser His Lys Asp Leu Leu Arg
   1               5                   10                  15
   Leu Ser His Leu Lys Pro
               20
<210> 3842
   <211> 12
   <212> PRT
   <213> Homo sapiens
<400> 3842
   Thr Val Glu Thr Gln Asn Leu Glu Gly Leu His His
   1               5                   10
<210> 3843
   <211> 15
   <212> PRT
   <213> Homo sapiens
<400> 3843
   Val Ala Ala Arg Gly Arg Gly Arg Gly Met Gly Arg Gly Asn Ile
   1               5                   10                  15
<210> 3844
   <211> 13
   <212> PRT
   <213> Homo sapiens
<400> 3844
   Val Ala Gly Arg Gly Arg Gly Arg Gly Arg Gly Arg Gly
   1               5                   10
<210> 3845
   <211> 15
   <212> PRT
   <213> Homo sapiens
<400> 3845
   Val Ala Gly Arg Gly Arg Gly Arg Gly Arg Gly Arg Gly Arg Gly
   1               5                   10                  15
<210> 3846
   <211> 25
   <212> PRT
   <213> Homo sapiens
<400> 3846
   Val Ala Gly Arg Gly Arg Gly Arg Gly Arg Gly Arg Gly Arg Gly Arg
   1               5                   10                  15
   Gly Arg Gly Arg Gly Gly Pro Arg Arg
               20                  25
<210> 3847
   <211> 6
   <212> PRT
   <213> Homo sapiens
<400> 3847
   Val Cys Glu His Cys Asp
   1               5
<210> 3848
   <211> 35
   <212> PRT
   <213> Homo sapiens
<400> 3848
   Val Glu Pro Lys Val Lys Lys Arg Glu Ala Val Ala Gly Arg Gly Arg
   1               5                   10                  15
   Gly Arg Gly Arg Gly Arg Gly Arg Gly Arg Gly Arg Gly Arg Gly Gly
               20                  25                  30
   Pro Arg Arg
           35
<210> 3849
   <211> 13
   <212> PRT
   <213> Homo sapiens
<400> 3849
   Val Glu Pro Lys Val Lys Ser Lys Lys Arg Glu Ala Val
   1               5                   10
<210> 3850
   <211> 15
   <212> PRT
   <213> Homo sapiens
<400> 3850
   Val Glu Pro Lys Val Lys Ser Lys Lys Arg Glu Ala Val Ala Gly
   1               5                   10                  15
<210> 3851
   <211> 15
   <212> PRT
   <213> Homo sapiens
<400> 3851
   Val Glu Pro Lys Val Lys Ser Lys Lys Arg Glu Ala Val Ala Gly
   1               5                   10                  15
<210> 3852
   <211> 15
   <212> PRT
   <213> Homo sapiens
<400> 3852
   Val Lys Ser Lys Lys Arg Glu Ala Val Ala Gly Arg Gly Arg Gly
   1               5                   10                  15
<210> 3853
   <211> 15
   <212> PRT
   <213> Homo sapiens
<400> 3853
   Val Lys Ser Lys Lys Arg Glu Ala Val Ala Gly Arg Gly Arg Gly
   1 5 10 15
<210> 3854
   <211> 11
   <212> PRT
   <213> Homo sapiens
<400> 3854
   Val Leu Val Asp Val Glu Arg Gly Arg Thr Val
   1 5 10
<210> 3855
   <211> 13
   <212> PRT
   <213> Homo sapiens
<400> 3855
   Val Ser Met Asn Thr His Leu Lys Ala Val Lys Met Thr
   1 5 10
<210> 3856
   <211> 9
   <212> PRT
   <213> Homo sapiens
<400> 3856
   Tyr Ile Thr Thr Ile Ala Gly Val Met
   1 5
<210> 3857
   <211> 20
   <212> PRT
   <213> Homo sapiens
<400> 3857
   Tyr Lys Gln Arg Asn Gly Trp Ser His Lys Asp Leu Leu Arg Leu Ser
   1 5 10 15
   His Leu Lys Pro
   20
<210> 3858
   <211> 609
   <212> PRT
   <213> Homo sapiens
<400> 3858
   Met Ser Gly Trp Glu Ser Tyr Tyr Lys Thr Glu Gly Asp Glu Glu Ala
   1               5                   10                  15
   Glu Glu Glu Gln Glu Glu Asn Leu Glu Ala Ser Gly Asp Tyr Lys Tyr
               20                  25                  30
   Ser Gly Arg Asp Ser Leu Ile Phe Leu Val Asp Ala Ser Lys Ala Met
           35                  40                  45
   Phe Glu Ser Gln Ser Glu Asp Glu Leu Thr Pro Phe Asp Met Ser Ile
       50                  55                  60
   Gln Cys Ile Gln Ser Val Tyr Ile Ser Lys Ile Ile Ser Ser Asp Arg
   65                  70                  75                  80
   Asp Leu Leu Ala Val Val Phe Tyr Gly Thr Glu Lys Asp Lys Asn Ser
                   85                  90                  95
   Val Asn Phe Lys Asn Ile Tyr Val Leu Gln Glu Leu Asp Asn Pro Gly
               100                 105                 110
   Ala Lys Arg Ile Leu Glu Leu Asp Gln Phe Lys Gly Gln Gln Gly Gln
           115                 120                 125
   Lys Arg Phe Gln Asp Met Met Gly His Gly Ser Asp Tyr Ser Leu Ser
       130                 135                 140
   Glu Val Leu Trp Val Cys Ala Asn Leu Phe Ser Asp Val Gln Phe Lys
   145                 150                 155                 160
   Met Ser His Lys Arg Ile Met Leu Phe Thr Asn Glu Asp Asn Pro His
                   165                 170                 175
   Gly Asn Asp Ser Ala Lys Ala Ser Arg Ala Arg Thr Lys Ala Gly Asp
               180                 185                 190
   Leu Arg Asp Thr Gly Ile Phe Leu Asp Leu Met His Leu Lys Lys Pro
           195                 200                 205
   Gly Gly Phe Asp Ile Ser Leu Phe Tyr Arg Asp Ile Ile Ser Ile Ala
       210                 215                 220
   Glu Asp Glu Asp Leu Arg Val His Phe Glu Glu Ser Ser Lys Leu Glu
   225                 230                 235                 240
   Asp Leu Leu Arg Lys Val Arg Ala Lys Glu Thr Arg Lys Arg Ala Leu
                   245                 250                 255
   Ser Arg Leu Lys Leu Lys Leu Asn Lys Asp Ile Val Ile Ser Val Gly
   260 265 270
   Ile Tyr Asn Leu Val Gln Lys Ala Leu Lys Pro Pro Pro Ile Lys Leu
   275 280 285
   Tyr Arg Glu Thr Asn Glu Pro Val Lys Thr Lys Thr Arg Thr Phe Asn
   290 295 300
   Thr Ser Thr Gly Gly Leu Leu Leu Pro Ser Asp Thr Lys Arg Ser Gln
   305 310 315 320
   Ile Tyr Gly Ser Arg Gln Ile Ile Leu Glu Lys Glu Glu Thr Glu Glu
   325 330 335
   Leu Lys Arg Phe Asp Asp Pro Gly Leu Met Leu Met Gly Phe Lys Pro
   340 345 350
   Leu Val Leu Leu Lys Lys His His Tyr Leu Arg Pro Ser Leu Phe Val
   355 360 365
   Tyr Pro Glu Glu Ser Leu Val Ile Gly Ser Ser Thr Leu Phe Ser Ala
   370 375 380
   Leu Leu Ile Lys Cys Leu Glu Lys Glu Val Ala Ala Leu Cys Arg Tyr
   385 390 395 400
   Thr Pro Arg Arg Asn Ile Pro Pro Tyr Phe Val Ala Leu Val Pro Gln
   405 410 415
   Glu Glu Glu Leu Asp Asp Gln Lys Ile Gln Val Thr Pro Pro Gly Phe
   420 425 430
   Gln Leu Val Phe Leu Pro Phe Ala Asp Asp Lys Arg Lys Met Pro Phe
   435 440 445
   Thr Glu Lys Ile Met Ala Thr Pro Glu Gln Val Gly Lys Met Lys Ala
   450 455 460
   Ile Val Glu Lys Leu Arg Phe Thr Tyr Arg Ser Asp Ser Phe Glu Asn
   465 470 475 480
   Pro Val Leu Gln Gln His Phe Arg Asn Leu Glu Ala Leu Ala Leu Asp
   485 490 495
   Leu Met Glu Pro Glu Gln Ala Val Asp Leu Thr Leu Pro Lys Val Glu
   500 505 510
   Ala Met Asn Lys Arg Leu Gly Ser Leu Val Asp Glu Phe Lys Glu Leu
   515 520 525
   Val Tyr Pro Pro Asp Tyr Asn Pro Glu Gly Lys Val Thr Lys Arg Lys
   530 535 540
   His Asp Asn Glu Gly Ser Gly Ser Lys Arg Pro Lys Val Glu Tyr Ser
   545 550 555 560
   Glu Glu Glu Leu Lys Thr His Ile Ser Lys Gly Thr Leu Gly Lys Phe
   565 570 575
   Thr Val Pro Met Leu Lys Glu Ala Cys Arg Ala Tyr Gly Leu Lys Ser
   580 585 590
   Gly Leu Lys Lys Gln Glu Leu Leu Glu Ala Leu Thr Lys His Phe Gln
   595 600 605
   Asp
<210> 3859
   <211> 119
   <212> PRT
   <213> Homo sapiens
<400> 3859
   Met Lys Leu Val Arg Phe Leu Met Lys Leu Ser His Glu Thr Val Thr
   1               5                   10                  15
   Ile Glu Leu Lys Asn Gly Thr Gln Val His Gly Thr Ile Thr Gly Val
               20                  25                  30
   Asp Val Ser Met Asn Thr His Leu Lys Ala Val Lys Met Thr Leu Lys
           35                  40                  45
   Asn Arg Glu Pro Val Gln Leu Glu Thr Leu Ser Ile Arg Gly Asn Asn
       50                  55                  60
   Ile Arg Tyr Phe Ile Leu Pro Asp Ser Leu Pro Leu Asp Thr Leu Leu
   65                  70                  75                  80
   Val Asp Val Glu Pro Lys Val Lys Ser Lys Lys Arg Glu Ala Val Ala
                   85                  90                  95
   Gly Arg Gly Arg Gly Arg Gly Arg Gly Arg Gly Arg Gly Arg Gly Arg
                   100                 105                 110
   Gly Arg Gly Gly Pro Arg Arg
           115
<210> 3860
   <211> 10
   <212> PRT
   <213> Homo sapiens
<400> 3860
   Ala Ala Phe Tyr Lys Thr Phe Lys Thr Val
   1               5                   10
<210> 3861
   <211> 15
   <212> PRT
   <213> Homo sapiens
<400> 3861
   Ala Glu Met Lys Ser Leu Glu Glu Lys Ile Gly Cys Leu Leu Lys
   1               5                   10                  15
<210> 3862
   <211> 11
   <212> PRT
   <213> Homo sapiens
<400> 3862
   Ala Leu Leu Arg Asn Leu Gly Lys Met Thr Ala
   1 5 10
<210> 3863
   <211> 14
   <212> PRT
   <213> Homo sapiens
<400> 3863
   Ala Leu Arg Glu Tyr Arg Lys Lys Met Asp Ile Pro Ala Lys
   1 5 10
<210> 3864
   <211> 9
   <212> PRT
   <213> Homo sapiens
<400> 3864
   Ala Met Lys Ile Ser Phe Ala Lys Lys
   1 5
<210> 3865
   <211> 7
   <212> PRT
   <213> Homo sapiens
<400> 3865
   Ala Pro Gly Met Arg Pro Pro
   1 5
<210> 3866
   <211> 10
   <212> PRT
   <213> Homo sapiens
<400> 3866
   Ala Pro Pro Pro Gly Met Arg Pro Pro Met
   1 5 10
<210> 3867
   <211> 19
   <212> PRT
   <213> Mus musculus
<400> 3867
   Ala Pro Gln Gly Ser Leu Gly Glu Tyr Leu Phe Glu Arg Leu Thr Leu
   1 5 10 15
   Lys His Asp
<210> 3868
   <211> 15
   <212> PRT
   <213> Homo sapiens
<400> 3868
   Ala Thr Leu Asp Asp Ile Lys Glu Trp Leu Glu Asp Lys Gly Gln
   1               5                   10                  15
<210> 3869
   <211> 14
   <212> PRT
   <213> Homo sapiens
<400> 3869
   Cys Ser Leu Pro Met Ile Trp Ala Gln Lys Thr Asn Thr Pro
   1               5                   10
<210> 3870
   <211> 9
   <212> PRT
   <213> Homo sapiens
<400> 3870
   Asp Arg Asn Gly Thr His Leu Asp Ala
   1               5
<210> 3871
   <211> 20
   <212> PRT
   <213> Mus musculus
<400> 3871
   Asp Ser Cys Pro Leu Asp Cys Lys Val Tyr Val Gly Asn Leu Gly Asn
   1               5                   10                  15
   Asn Gly Asn Lys
               20
<210> 3872
   <211> 9
   <212> PRT
   <213> Homo sapiens
<400> 3872
   Glu Ala Leu Thr Lys His Phe Gln Asp
   1               5
<210> 3873
   <211> 14
   <212> PRT
   <213> Homo sapiens
<400> 3873
   Glu Ala Val Glu Lys Val Lys Arg Thr Lys Asp Glu Leu Glu
   1               5                   10
<210> 3874
   <211> 14
   <212> PRT
   <213> Homo sapiens
<400> 3874
   Glu Lys Val Lys Arg Thr Lys Asp Glu Leu Glu Val Ile His
   1               5                   10
<210> 3875
   <211> 15
   <212> PRT
   <213> Homo sapiens
<400> 3875
   Glu Gln Glu Ala Lys Gln Lys Leu Glu Glu Asp Ala Glu Met Lys
   1               5                   10                  15
<210> 3876
   <211> 50
   <212> PRT
   <213> Homo sapiens
<400> 3876
   Phe Asn Gly Thr Phe Val Lys Leu Asn Lys Ala Ser Ile Asn Met Leu
   1               5                   10                  15
   Arg Ile Val Glu Pro Tyr Ile Ala Trp Gly Tyr Pro Asn Leu Lys Ser
               20                  25                  30
   Val Asn Glu Leu Ile Tyr Lys Arg Gly Tyr Gly Lys Ile Asn Lys Lys
           35                  40                  45
   Arg Ile
       50
<210> 3877
   <211> 30
   <212> PRT
   <213> Homo sapiens
<400> 3877
   Gly Asp Ala Phe Lys Thr Leu Phe Val Ala Arg Val Asn Tyr Asp Thr
   1               5                   10                  15
   Thr Glu Ser Lys Leu Arg Arg Glu Phe Glu Val Tyr Gly Pro
               20                  25                  30
<210> 3878
   <211> 21
   <212> PRT
   <213> Homo sapiens
<400> 3878
   Gly Ile Ile Asp Leu Ile Glu Lys Arg Lys Phe Asn Gln Asn Ser Asn
   1               5                   10                  15
   Ser Thr Tyr Cys Val
               20
<210> 3879
   <211> 10
   <212> PRT
   <213> Homo sapiens
<400> 3879
   Gly Ile Met Ala Pro Pro Pro Gly Met Arg
    1               5                   10
<210> 3880
   <211> 10
   <212> PRT
   <213> Homo sapiens
<400> 3880
   Gly Met Arg Pro Pro Met Gly Pro Pro Ile
   1               5                   10
<210> 3881
   <211> 13
   <212> PRT
   <213> Homo sapiens
<400> 3881
   Gly Gln Ile Leu Asp Ile Leu Val Ser Arg Ser Leu Lys
   1               5                   10
<210> 3882
   <211> 20
   <212> PRT
   <213> Mus musculus
<400> 3882
   His His Asn Thr Leu Val Cys Ser Val Thr Asp Phe Tyr Pro Ala Lys
   1               5                   10                  15
   Ile Lys Val Arg
               20
<210> 3883
   <211> 8
   <212> PRT
   <213> Homo sapiens
<400> 3883
   His Lys Ala Phe Lys Gly Ser Ile
   1               5
<210> 3884
   <211> 8
   <212> PRT
   <213> Homo sapiens
<400> 3884
   His Lys Gly Phe Lys Gly Val Asp
   1               5
<210> 3885
   <211> 16
   <212> PRT
   <213> Homo sapiens
<400> 3885
   His Leu Leu Thr Asn His Leu Lys Ser Lys Glu Val Trp Lys Ala Leu
   1               5                   10                  15
<210> 3886
   <211> 20
   <212> PRT
   <213> Mus musculus
<400> 3886
   His Leu Gln Leu Ala Ile Arg Asn Asp Glu Glu Leu Asn Lys Leu Leu
   1               5                   10                  15
   Gly Lys Val Thr
               20
<210> 3887
   <211> 18
   <212> PRT
   <213> Mus musculus
<400> 3887
   His Pro Pro His Ile Glu Ile Gln Met Leu Lys Asn Gly Lys Lys Ile
   1 5 10 15
   Pro Lys
<210> 3888
   <211> 9
   <212> PRT
   <213> Homo sapiens
<400> 3888
   Ile Ile Ala Lys Met Lys Gly Thr Phe
   1 5
<210> 3889
   <211> 10
   <212> PRT
   <213> Homo sapiens
<400> 3889
   Ile Met Ala Pro Pro Pro Gly Met Arg Pro
   1 5 10
<210> 3890
   <211> 15
   <212> PRT
   <213> Homo sapiens
<400> 3890
   Lys Phe Val Glu Thr Pro Gly Gln Lys Tyr Lys Glu Thr Asp Leu
   1 5 10 15
<210> 3891
   <211> 11
   <212> PRT
   <213> Homo sapiens
<400> 3891
   Lys Ile Lys Lys Asp Glu Leu Lys Lys Ser Leu
   1 5 10
<210> 3892
   <211> 15
   <212> PRT
   <213> Homo sapiens
<400> 3892
   Leu Ala Val Thr Lys Tyr Lys Gln Arg Asn Gly Trp Ser His Lys
   1 5 10 15
<210> 3893
   <211> 40
   <212> PRT
   <213> Homo sapiens
<400> 3893
   Leu Glu Lys Asp Glu Arg Glu Gln Leu Arg Ile Leu Gly Glu Lys Glu
   1               5                   10                  15
   Ala Lys Leu Ala Gln Gln Ser Gln Ala Leu Gln Glu Leu Ile Ser Glu
               20                  25                  30
   Leu Asp Arg Arg Cys His Ser Ser
           35                  40
<210> 3894
   <211> 9
   <212> PRT
   <213> Homo sapiens
<400> 3894
   Leu Lys Arg Val Cys Ala Gln Ile Asn
   1               5
<210> 3895
   <211> 36
   <212> PRT
   <213> Homo sapiens
<400> 3895
   Leu Gln Glu Leu Glu Lys Asp Glu Arg Glu Gln Leu Arg Ile Leu Gly
   1               5                   10                  15
   Glu Lys Glu Ala Lys Leu Ala Gln Gln Ser Gln Ala Leu Gln Glu Leu
               20                  25                  30
   Ile Ser Glu Leu
           35
<210> 3896
   <211> 49
   <212> PRT
   <213> Homo sapiens
<400> 3896
   Leu Gln Glu Leu Glu Lys Asp Glu Arg Glu Gln Leu Arg Ile Leu Gly
   1               5                   10                  15
   Glu Lys Glu Ala Lys Leu Ala Gln Gln Ser Gln Ala Leu Gln Glu Leu
               20                  25                  30
   Ile Ser Glu Leu Asp Arg Arg Cys His Ser Ser Ala Leu Glu Leu Leu
           35                  40                  45
   Gln
<210> 3897
   <211> 9
   <212> PRT
   <213> Homo sapiens
<400> 3897
   Leu Arg Leu Ser His Leu Lys Pro Ser
   1               5
<210> 3898
   <211> 30
   <212> PRT
   <213> Homo sapiens
<400> 3898
   Leu Thr Leu Pro Lys Val Glu Ala Met Asn Lys Arg Leu Gly Ser Leu
   1               5                   10                  15
   Val Asp Glu Phe Lys Glu Leu Val Tyr Pro Pro Asp Tyr Asn
               20                  25                  30
<210> 3899
   <211> 8
   <212> PRT
   <213> Homo sapiens
<400> 3899
   Leu Tyr Lys Glu Lys Ala Leu Ser
   1               5
<210> 3900
   <211> 10
   <212> PRT
   <213> Homo sapiens
<400> 3900
   Met Ala Pro Pro Pro Gly Met Arg Pro Pro
   1               5                   10
<210> 3901
   <211> 26
   <212> PRT
   <213> Homo sapiens
<400> 3901
   Met Glu Gly Val Glu Glu Lys Lys Lys Glu Val Pro Ala Val Pro Glu
   1 5 10 15
   Thr Leu Lys Lys Lys Arg Arg Asn Phe Ala
   20 25
<210> 3902
   <211> 12
   <212> PRT
   <213> Homo sapiens
<400> 3902
   Met Gly Arg Gly Ala Pro Pro Pro Gly Met Met Gly
   1 5 10
<210> 3903
   <211> 9
   <212> PRT
   <213> Homo sapiens
<400> 3903
   Met Lys Cys Gly Met Trp Gly Arg Ala
   1 5
<210> 3904
   <211> 24
   <212> PRT
   <213> Homo sapiens
<400> 3904
   Met Leu Arg Lys Ala Arg Arg Lys Leu Ile Tyr Glu Lys Ala Lys His
   1 5 10 15
   Tyr His Lys Glu Tyr Arg Gln Met
   20
<210> 3905
   <211> 10
   <212> PRT
   <213> Homo sapiens
<400> 3905
   Met Arg Pro Pro Met Gly Pro Pro Ile Gly
   1 5 10
<210> 3906
   <211> 11
   <212> PRT
   <213> Homo sapiens
<400> 3906
   Met Trp Gly Arg Ala Leu Arg Lys Ala Ile Ala
   1 5 10
<210> 3907
   <211> 14
   <212> PRT
   <213> Homo sapiens
<400> 3907
   Asn Glu Lys Leu Leu Lys Lys Ala Arg Ile His Pro Phe His
   1 5 10
<210> 3908
   <211> 9
   <212> PRT
   <213> Homo sapiens
<400> 3908
   Asn Leu Asn Glu Lys Ile Lys Lys Asp
   1 5
<210> 3909
   <211> 10
   <212> PRT
   <213> Homo sapiens
<400> 3909
   Pro Gly Ile Met Ala Pro Pro Pro Gly Met
   1 5 10
<210> 3910
   <211> 10
   <212> PRT
   <213> Homo sapiens
<400> 3910
   Pro Gly Met Arg Pro Pro Met Gly Pro Pro
   1 5 10
<210> 3911
   <211> 20
   <212> PRT
   <213> Mus musculus
<400> 3911
   Pro Gly Val Thr Val Lys Asp Val Asn Gln Gln Glu Phe Val Arg Ala
   1 5 10 15
   Leu Ala Ala Phe
   20
<210> 3912
   <211> 10
   <212> PRT
   <213> Homo sapiens
<400> 3912
   Pro Pro Gly Met Arg Pro Pro Met Gly Pro
   1 5 10
<210> 3913
   <211> 8
   <212> PRT
   <213> Homo sapiens
<400> 3913
   Pro Pro Pro Gly Ile Arg Gly Pro
   1 5
<210> 3914
   <211> 7
   <212> PRT
   <213> Homo sapiens
<400> 3914
   Pro Pro Pro Gly Met Arg Pro
   1 5
<210> 3915
   <211> 8
   <212> PRT
   <213> Homo sapiens
<400> 3915
   Pro Pro Pro Gly Met Arg Pro Pro
   1 5
<210> 3916
   <211> 10
   <212> PRT
   <213> Homo sapiens
<400> 3916
   Pro Pro Pro Gly Met Arg Pro Pro Met Gly
   1 5 10
<210> 3917
   <211> 16
   <212> PRT
   <213> Homo sapiens
<400> 3917
   Gln Glu Met Pro Leu Thr Ala Leu Leu Arg Asn Leu Gly Lys Met Thr
   1 5 10 15
<210> 3918
   <211> 9
   <212> PRT
   <213> Homo sapiens
<400> 3918
   Gln Glu Thr Pro Ala Thr Lys Lys Ala
   1 5
<210> 3919
   <211> 24
   <212> PRT
   <213> Homo sapiens
<400> 3919
   Arg Ala Tyr Gly Leu Lys Ser Gly Leu Lys Lys Gln Glu Leu Leu Glu
   1 5 10 15
   Ala Leu Thr Lys His Phe Gln Asp
   20
<210> 3920
   <211> 34
   <212> PRT
   <213> Homo sapiens
<400> 3920
   Arg Ile Leu Glu Leu Asp Gln Phe Lys Gly Gln Gln Gly Gln Lys Arg
   1 5 10 15
   Phe Gln Asp Met Met Gly His Gly Ser Asp Tyr Ser Leu Ser Glu Val
   20 25 30
   Leu Trp
<210> 3921
   <211> 18
   <212> PRT
   <213> Homo sapiens
<400> 3921
   Arg Lys Gly His Phe Leu Leu Ser Ser Lys Ser Gly Phe Trp Thr Ile
   1 5 10 15
   Trp Leu
<210> 3922
   <211> 14
   <212> PRT
   <213> Mus musculus
<400> 3922
   Arg Met Val Asn His Phe Ile Ala Glu Phe Lys Arg Lys His
   1 5 10
<210> 3923
   <211> 15
   <212> PRT
   <213> Mus musculus
<400> 3923
   Arg Gln Val Arg Phe Tyr Asp Gly Val Ile Glu Leu Ser Leu Thr
   1 5 10 15
<210> 3924
   <211> 10
   <212> PRT
   <213> Homo sapiens
<400> 3924
   Arg Ser Gly Lys Pro Arg Gly Tyr Ala Phe
   1 5 10
<210> 3925
   <211> 10
   <212> PRT
   <213> Homo sapiens
<400> 3925
   Arg Ser Leu Lys Met Arg Gly Gln Ala Phe
   1 5 10
<210> 3926
   <211> 11
   <212> PRT
   <213> Homo sapiens
<400> 3926
   Ser Asp Asp Asp Met Gly Phe Gly Leu Phe Asp
   1 5 10
<210> 3927
   <211> 12
   <212> PRT
   <213> Homo sapiens
<400> 3927
   Ser Glu Glu Glu Leu Lys Thr His Ile Ser Lys Gly
   1 5 10
<210> 3928
   <211> 50
   <212> PRT
   <213> Homo sapiens
<400> 3928
   Ser Glu Glu Glu Leu Lys Thr His Ile Ser Lys Gly Thr Leu Gly Lys
   1 5 10 15
   Phe Thr Val Pro Met Leu Lys Glu Ala Cys Arg Ala Tyr Gly Leu Lys
   20 25 30
   Ser Gly Leu Lys Lys Gln Glu Leu Leu Glu Ala Leu Thr Lys His Phe
   35 40 45
   Gln Asp
   50
<210> 3929
   <211> 9
   <212> PRT
   <213> Homo sapiens
<400> 3929
   Ser Gln Glu Gly Arg Thr Thr Lys Gln
   1 5
<210> 3930
   <211> 21
   <212> PRT
   <213> Homo sapiens
<400> 3930
   Ser Arg Ile His Ala Glu Phe Val Gln Gln Lys Asn Phe Leu Val Glu
   1 5 10 15
   Glu Glu Gln Arg Gln
   20
<210> 3931
   <211> 10
   <212> PRT
   <213> Homo sapiens
<400> 3931
   Ser Thr Lys Gln Ala Ala Phe Lys Ala Val
   1               5                   10
<210> 3932
   <211> 8
   <212> PRT
   <213> Homo sapiens
<400> 3932
   Thr Glu Lys Leu Leu Lys Tyr Leu
   1               5
<210> 3933
   <211> 12
   <212> PRT
   <213> Homo sapiens
<400> 3933
   Thr Phe Ile Gln Phe Lys Lys Asp Leu Lys Glu Ser
   1               5                   10
<210> 3934
   <211> 11
   <212> PRT
   <213> Homo sapiens
<400> 3934
   Thr Tyr Tyr Ile Lys Glu Gln Lys Leu Gly Leu
   1               5                   10
<210> 3935
   <211> 9
   <212> PRT
   <213> Homo sapiens
<400> 3935
   Val Glu Pro Thr Gly Lys Arg Phe Leu
   1               5
<210> 3936
   <211> 19
   <212> PRT
   <213> Mus musculus
<400> 3936
   Val Phe Arg Arg Tyr Val Glu Val Gly Arg Val Ala Tyr Ile Ser Phe
   1 5 10 15
   Ser Pro His
<210> 3937
   <211> 12
   <212> PRT
   <213> Homo sapiens
<400> 3937
   Val Gly Arg Ala Thr Pro Pro Pro Gly Ile Met Ala
   1 5 10
<210> 3938
   <211> 21
   <212> PRT
   <213> Homo sapiens
<400> 3938
   Val Ser Leu Val Cys Glu Glu Lys Leu Cys Asn Glu Lys Leu Leu Lys
   1 5 10 15
   Lys Ala Arg Ile His
   20
<210> 3939
   <211> 13
   <212> PRT
   <213> Homo sapiens
<400> 3939
   Val Thr Lys Tyr Ile Thr Lys Gly Trp Lys Glu Val His
   1 5 10
<210> 3940
   <211> 17
   <212> PRT
   <213> Mus musculus
<400> 3940
   Val Val Lys Lys Gly Thr Asp Phe Gln Leu Asn Gln Leu Glu Gly Lys
   1 5 10 15
   Lys
<210> 3941
   <211> 15
   <212> PRT
   <213> Homo sapiens
<400> 3941
   Tyr Lys Thr Gly His Gly Leu Arg Gly Lys Leu Lys Trp Arg Pro
   1 5 10 15
<210> 3942
   <211> 8
   <212> PRT
   <213> Homo sapiens
<400> 3942
   Ala Ala Ala Ala Ala Pro Ala Lys
   1 5
<210> 3943
   <211> 22
   <212> PRT
   <213> Mus musculus
<400> 3943
   Ala Gly Arg Gly Arg Gly Arg Gly Arg Gly Arg Gly Arg Gly Arg Gly
   1 5 10 15
   Arg Gly Arg Gly Ser Leu
   20
<210> 3944
   <211> 22
   <212> PRT
   <213> Homo sapiens
<400> 3944
   Ala Lys Val Glu Ala Lys Glu Glu Ser Glu Glu Ser Asp Glu Asp Met
   1 5 10 15
   Gly Phe Gly Leu Phe Asp
   20
<210> 3945
   <211> 15
   <212> PRT
   <213> Homo sapiens
<400> 3945
   Ala Leu Gly Gly Asn Ser Ser Pro Ser Ala Lys Asp Ile Lys Lys
   1 5 10 15
<210> 3946
   <211> 15
   <212> PRT
   <213> Homo sapiens
<400> 3946
   Ala Pro Ala Ala Gly Ser Ala Pro Ala Ala Ala Glu Glu Lys Lys
   1 5 10 15
<210> 3947
   <211> 24
   <212> PRT
   <213> Homo sapiens
<400> 3947
   Ala Val His Gly Glu Arg Leu His Leu Phe Cys Glu Lys Asp Gly Lys
   1 5 10 15
   Ala Leu Cys Trp Val Cys Ala Gln
   20
<210> 3948
   <211> 16
   <212> PRT
   <213> Homo sapiens
<400> 3948
   Cys Ala Phe Thr Gly Pro Leu Arg Pro Phe Phe Ser Pro Gly Phe Asn
   1 5 10 15
<210> 3949
   <211> 15
   <212> PRT
   <213> Homo sapiens
<400> 3949
   Cys His Ser Ser Ala Leu Glu Leu Leu Gln Glu Val Ile Ile Val
   1 5 10 15
<210> 3950
   <211> 15
   <212> PRT
   <213> Homo sapiens
<400> 3950
   Asp Asp Arg Leu Asn Lys Val Ile Ser Glu Leu Asn Gly Lys Asn
   1 5 10 15
<210> 3951
   <211> 15
   <212> PRT
   <213> Homo sapiens
<400> 3951
   Asp Glu Lys Lys Glu Glu Ser Glu Glu Ser Asp Asp Asp Met Gly
   1 5 10 15
<210> 3952
   <211> 8
   <212> PRT
   <213> Homo sapiens
<400> 3952
   Asp Met Gly Phe Gly Leu Phe Asp
   1 5
<210> 3953
   <211> 16
   <212> PRT
   <213> Homo sapiens
<400> 3953
   Glu Cys Ile Ser Gln Val Gly Lys Gly Gly Gly Ser Val Cys Ala Val
   1 5 10 15
<210> 3954
   <211> 24
   <212> PRT
   <213> Homo sapiens
<400> 3954
   Glu Glu Val Thr Cys Pro Ile Cys Leu Asp Pro Phe Val Glu Pro Val
   1 5 10 15
   Ser Ile Glu Cys Gly His Ser Phe
   20
<210> 3955
   <211> 15
   <212> PRT
   <213> Homo sapiens
<400> 3955
   Glu Ser Glu Glu Ser Asp Asp Asp Met Gly Phe Gly Leu Phe Asp
   1 5 10 15
<210> 3956
   <211> 16
   <212> PRT
   <213> Homo sapiens
<400> 3956
   Phe His Ser Gly Lys His Tyr Trp Glu Val Asp Val Thr Gly Lys Glu
   1 5 10 15
<210> 3957
   <211> 15
   <212> PRT
   <213> Unknown
<220>
   <223> epitope involved in systemic lupus
<400> 3957
   Phe Ile Glu Trp Asn Lys Leu Arg Phe Arg Gln Gly Leu Glu Trp
   1 5 10 15
<210> 3958
   <211> 13
   <212> PRT
   <213> Unknown
<220>
   <223> epitope involved in systemic lupus
<400> 3958
   Gly Gly Gly Arg Asp Arg Asp Arg Asp Arg Gly Gly Gly
   1 5 10
<210> 3959
   <211> 8
   <212> PRT
   <213> Homo sapiens
<400> 3959
   Gly Ile Ala Arg Val Pro Leu Ala
   1 5
<210> 3960
   <211> 15
   <212> PRT
   <213> Homo sapiens
<400> 3960
   Gly Ile Glu Ala Asp Asp Asp Arg Leu Asn Lys Val Ile Ser Glu
   1 5 10 15
<210> 3961
   <211> 16
   <212> PRT
   <213> Homo sapiens
<400> 3961
   Gly Lys Ala Leu Cys Trp Val Cys Ala Gln Ser Arg Lys His Arg Asp
   1 5 10 15
<210> 3962
   <211> 20
   <212> PRT
   <213> Homo sapiens
<400> 3962
   Gly Lys Ala Leu Cys Trp Val Cys Ala Gln Ser Arg Lys His Arg Asp
   1 5 10 15
   His Ala Met Val
   20
<210> 3963
   <211> 23
   <212> PRT
   <213> Homo sapiens
<400> 3963
   Gly Lys Lys Lys Ser Lys Pro Val Asn Lys Asp Arg Tyr Ile Ser Lys
   1 5 10 15
   Met Phe Leu Arg Gly Asp Ser
   20
<210> 3964
   <211> 15
   <212> PRT
   <213> Human Endogenous Retrovirus
<400> 3964
   Gly Lys Thr Cys Pro Lys Glu Ile Pro Lys Gly Ser Lys Asn Thr
   1 5 10 15
<210> 3965
   <211> 16
   <212> PRT
   <213> Homo sapiens
<400> 3965
   Gly Leu Lys Lys Met Leu Arg Thr Cys Ala Val His Ile Thr Leu Asp
   1 5 10 15
<210> 3966
   <211> 11
   <212> PRT
   <213> Homo sapiens
<400> 3966
   Gly Ser Ile Tyr Asn Pro Glu Val Leu Asp Ile
   1 5 10
<210> 3967
   <211> 8
   <212> PRT
   <213> Homo sapiens
<400> 3967
   Gly Val Gly Gly Pro Ser Gln Gln
   1 5
<210> 3968
   <211> 8
   <212> PRT
   <213> Homo sapiens
<400> 3968
   Gly Val Gly Arg Ala Ala Gly Arg
   1 5
<210> 3969
   <211> 8
   <212> PRT
   <213> Homo sapiens
<400> 3969
   Gly Val Pro Ala Gly Val Pro Ile
   1 5
<210> 3970
   <211> 15
   <212> PRT
   <213> Homo sapiens
<400> 3970
   Ile Glu Asp Val Ile Ala Gln Gly Ile Gly Lys Leu Ala Ser Val
   1 5 10 15
<210> 3971
   <211> 17
   <212> PRT
   <213> Homo sapiens
<400> 3971
   Ile Trp Leu Trp Asn Lys Gln Lys Tyr Glu Ala Gly Thr Tyr Pro Gln
   1 5 10 15
   Thr
<210> 3972
   <211> 23
   <212> PRT
   <213> Homo sapiens
<400> 3972
   Lys Ala Gln Val Ala Ala Arg Gly Arg Gly Arg Gly Met Gly Arg Gly
   1 5 10 15
   Asn Ile Phe Gln Lys Arg Arg
   20
<210> 3973
   <211> 15
   <212> PRT
   <213> Homo sapiens
<400> 3973
   Lys Asp Ile Lys Lys Ile Leu Asp Ser Val Gly Ile Glu Ala Asp
   1 5 10 15
<210> 3974
   <211> 18
   <212> PRT
   <213> Homo sapiens
<400> 3974
   Lys Glu Ile Ser Gln Glu Ala Arg Glu Gly Thr Gln Gly Glu Arg Cys
   1 5 10 15
   Ala Val
<210> 3975
   <211> 15
   <212> PRT
   <213> Homo sapiens
<400> 3975
   Leu Asn Gly Lys Asn Ile Glu Asp Val Ile Ala Gln Gly Ile Gly
   1 5 10 15
<210> 3976
   <211> 16
   <212> PRT
   <213> Homo sapiens
<400> 3976
   Leu Ser Glu Asp Arg Arg Gln Val Arg Leu Gly Asp Thr Gln Gln Ser
   1               5                   10                  15
<210> 3977
   <211> 13
   <212> PRT
   <213> Homo sapiens
<400> 3977
   Met Ala Ser Ala Ala Arg Leu Thr Met Met Trp Glu Glu
   1               5                   10
<210> 3978
   <211> 31
   <212> PRT
   <213> Homo sapiens
<400> 3978
   Met Leu Lys Asn Ala Pro Met Leu Lys Ser Met Lys Asn Lys Asn Gln
   1               5                   10                  15
   Gly Ser Gly Ala Gly Arg Gly Lys Ala Ala Ile Leu Lys Ala Gln
               20                  25                  30
<210> 3979
   <211> 8
   <212> PRT
   <213> Homo sapiens
<400> 3979
   Met Met Arg Lys Ala Ile Arg Gly
   1               5
<210> 3980
   <211> 13
   <212> PRT
   <213> Homo sapiens
<400> 3980
   Met Ser Leu Leu Asn Lys Pro Lys Ser Glu Met Thr Pro
   1               5                   10
<210> 3981
   <211> 17
   <212> PRT
   <213> Homo sapiens
<400> 3981
   Asn Ile Thr Asp His Gly Ser Leu Ile Tyr Ser Phe Ser Glu Cys Ala
   1 5 10 15
   Phe
<210> 3982
   <211> 21
   <212> PRT
   <213> Homo sapiens
<400> 3982
   Asn Ser Gln Asp Gly Tyr Val Trp Gln Val Thr Asp Met Asn Arg Leu
   1 5 10 15
   His Arg Phe Leu Cys
   20
<210> 3983
   <211> 15
   <212> PRT
   <213> Homo sapiens
<400> 3983
   Pro Ala Gly Gly Ala Val Ala Val Ser Ala Ala Pro Gly Ser Ala
   1 5 10 15
<210> 3984
   <211> 8
   <212> PRT
   <213> Homo sapiens
<400> 3984
   Pro Ala Pro Gly Met Arg Pro Pro
   1 5
<210> 3985
   <211> 18
   <212> PRT
   <213> Mus musculus
<400> 3985
   Pro Glu Pro Ala Lys Ser Ala Pro Ala Pro Lys Lys Gly Ser Lys Lys
   1 5 10 15
   Ala Val
<210> 3986
   <211> 18
   <212> PRT
   <213> Mus musculus
<400> 3986
   Pro Glu Pro Ala Lys Ser Ala Pro Ala Pro Lys Lys Gly Ser Lys Lys
   1               5                   10                  15
   Ala Val
<210> 3987
   <211> 49
   <212> PRT
   <213> Mus musculus
<400> 3987
   Pro Leu Phe Pro Ala Gly Arg Gly Arg Gly Arg Gly Arg Gly Arg Gly
   1               5                   10                  15
   Arg Gly Arg Gly Arg Gly Arg Gly Arg Gly Arg Gly Arg Gly Arg Gly
               20                  25                  30
   Arg Gly Arg Gly Arg Gly Arg Gly Arg Gly Arg Gly Ser Leu Ile Tyr
           35                  40                  45
   Ile
<210> 3988
   <211> 7
   <212> PRT
   <213> Homo sapiens
<400> 3988
   Pro Pro Gly Ile Arg Gly Pro
   1               5
<210> 3989
   <211> 8
   <212> PRT
   <213> Homo sapiens
<400> 3989
   Pro Pro Pro Gly Met Ile Pro Pro
   1               5
<210> 3990
   <211> 8
   <212> PRT
   <213> Homo sapiens
<400> 3990
   Pro Pro Pro Gly Met Arg Gly Pro
   1               5
<210> 3991
   <211> 19
   <212> PRT
   <213> Homo sapiens
<400> 3991
   Pro Gln Thr Pro Leu His Leu Gln Val Pro Pro Cys Gln Val Gly Ile
   1 5 10 15
   Phe Leu Asp
<210> 3992
   <211> 13
   <212> PRT
   <213> Homo sapiens
<400> 3992
   Gln Ala Leu Gly Ile Thr Thr Lys Ile Ser Arg Gly Thr
   1 5 10
<210> 3993
   <211> 9
   <212> PRT
   <213> Homo sapiens
<400> 3993
   Gln Val Tyr Ile Arg Gly Ser Lys Ile
   1 5
<210> 3994
   <211> 8
   <212> PRT
   <213> Homo sapiens
<400> 3994
   Arg Ala Thr Trp Lys Ser Asn Tyr
   1 5
<210> 3995
   <211> 15
   <212> PRT
   <213> Homo sapiens
<400> 3995
   Arg Asp Met Leu Leu Ala Asn Lys Val Pro Ala Ala Ala Arg Ala
   1 5 10 15
<210> 3996
   <211> 11
   <212> PRT
   <213> Homo sapiens
<400> 3996
   Arg Asp Arg Asp Arg Asp Arg Asp Arg Asp Arg
   1 5 10
<210> 3997
   <211> 8
   <212> PRT
   <213> Homo sapiens
<400> 3997
   Arg Asp Arg Asp Arg Asp Arg Glu
   1 5
<210> 3998
   <211> 8
   <212> PRT
   <213> Homo sapiens
<400> 3998
   Arg Asn Pro Leu Ile Ala Gly Lys
   1 5
<210> 3999
   <211> 13
   <212> PRT
   <213> Bos taurus
<400> 3999
   Ser Ala Pro Ala Pro Lys Lys Gly Ser Lys Lys Ala Val
   1 5 10
<210> 4000
   <211> 17
   <212> PRT
   <213> Homo sapiens
<400> 4000
   Ser Pro Gly Phe Asn Asp Gly Gly Lys Asn Thr Ala Pro Leu Thr Leu
   1 5 10 15
   Cys
<210> 4001
   <211> 17
   <212> PRT
   <213> Homo sapiens
<400> 4001
   Ser Gln Ala Leu Gln Glu Leu Ile Ser Glu Leu Asp Arg Arg Cys His
   1 5 10 15
   Ser
<210> 4002
   <211> 15
   <212> PRT
   <213> Homo sapiens
<400> 4002
   Ser Ser Pro Ser Ala Lys Asp Ile Lys Lys Ile Leu Asp Ser Val
   1 5 10 15
<210> 4003
   <211> 12
   <212> PRT
   <213> Homo sapiens
<400> 4003
   Ser Val Leu Thr Gln Ser Val Lys Asn Asn Thr Gln
   1 5 10
<210> 4004
   <211> 15
   <212> PRT
   <213> Homo sapiens
<400> 4004
   Ser Val Ser Tyr Asp Asp Trp Asp Tyr Ser Leu Glu Ala Arg Val
   1 5 10 15
<210> 4005
   <211> 14
   <212> PRT
   <213> Homo sapiens
<400> 4005
   Thr Gly Glu Val Tyr Arg Gly Lys Leu Ile Glu Ala Glu Asp
   1 5 10
<210> 4006
   <211> 15
   <212> PRT
   <213> Homo sapiens
<400> 4006
   Val Ala Val Ser Ala Ala Pro Gly Ser Ala Ala Pro Ala Ala Gly
   1 5 10 15
<210> 4007
   <211> 15
   <212> PRT
   <213> Homo sapiens
<400> 4007
<210> 4008
   <211> 16
   <212> PRT
   <213> Homo sapiens
<400> 4008
   Val His Gly Glu Arg Leu His Leu Phe Cys Glu Lys Asp Gly Lys Ala
   1 5 10 15
<210> 4009
   <211> 11
   <212> PRT
   <213> Homo sapiens
<400> 4009
   Trp Thr Glu Val Pro Lys Ser Gly Lys Gly Lys
   1 5 10
<210> 4010
   <211> 17
   <212> PRT
   <213> Homo sapiens
<400> 4010
   Tyr Gln Glu Lys Leu Gln Val Ala Leu Gly Glu Leu Arg Arg Lys Gln
   1 5 10 15
   Glu
<210> 4011
   <211> 14
   <212> PRT
   <213> Homo sapiens
<400> 4011
   Ala Glu Thr Arg Glu Glu Arg Met Glu Arg Lys Arg Arg Glu
   1 5 10
<210> 4012
   <211> 9
   <212> PRT
   <213> Homo sapiens
<400> 4012
   Ala Phe Gln Gln Gly Lys Ile Pro Pro
   1 5
<210> 4013
   <211> 22
   <212> PRT
   <213> Homo sapiens
<400> 4013
   Ala Lys Gly Lys Pro Asp Ala Ala Lys Lys Gly Val Val Lys Ala Glu
   1 5 10 15
   Lys Ser Lys Lys Lys Lys
   20
<210> 4014
   <211> 20
   <212> PRT
   <213> Homo sapiens
<400> 4014
   Glu Glu Ala Ser Gly Ser Ser Val Thr Ala Glu Glu Ala Lys Lys Phe
   1 5 10 15
   Leu Ala Pro Lys
   20
<210> 4015
   <211> 8
   <212> PRT
   <213> Homo sapiens
<400> 4015
   Gly Met Met Pro Ala Pro His Met
   1 5
<210> 4016
   <211> 9
   <212> PRT
   <213> Homo sapiens
<400> 4016
   Gly Pro Ala Pro Gly Met Arg Pro Pro
   1 5
<210> 4017
   <211> 9
   <212> PRT
   <213> Homo sapiens
<400> 4017
   Gly Pro Pro Met Met Arg Pro Pro Ala
   1               5
<210> 4018
   <211> 10
   <212> PRT
   <213> Homo sapiens
<400> 4018
   Gly Pro Pro Arg Pro Gly Met Met Pro Ala
   1               5                   10
<210> 4019
   <211> 8
   <212> PRT
   <213> Homo sapiens
<400> 4019
   His Asp Ser Pro Ser Val Arg Lys
   1               5
<210> 4020
   <211> 8
   <212> PRT
   <213> Homo sapiens
<400> 4020
   Lys Asp Tyr Tyr Gln Lys Trp Met
   1               5
<210> 4021
   <211> 8
   <212> PRT
   <213> Homo sapiens
<400> 4021
   Lys Ile Pro Pro Thr Pro Phe Ser
   1               5
<210> 4022
   <211> 30
   <212> PRT
   <213> Homo sapiens
<400> 4022
   Leu Ala Leu Val Phe Glu Ala Pro Asn Gln Glu Lys Val Ser Asp Tyr
   1               5                   10                  15
   Glu Met Lys Leu Met Asp Leu Asp Val Glu Gln Leu Gly Ile
               20                  25                  30
<210> 4023
   <211> 7
   <212> PRT
   <213> Homo sapiens
<400> 4023
   Met Glu Arg Lys Arg Arg Glu
   1 5
<210> 4024
   <211> 8
   <212> PRT
   <213> Homo sapiens
<400> 4024
   Pro Phe Ser Ala Pro Pro Pro Ala
   1 5
<210> 4025
   <211> 7
   <212> PRT
   <213> Human herpesvirus
<400> 4025
   Pro Pro Pro Gly Arg Arg Pro
   1 5
<210> 4026
   <211> 9
   <212> PRT
   <213> Homo sapiens
<400> 4026
   Pro Pro Pro Pro Gly Met Met Pro Val
   1 5
<210> 4027
   <211> 8
   <212> PRT
   <213> Homo sapiens
<400> 4027
   Pro Pro Pro Pro Ser Leu Pro Gly
   1 5
<210> 4028
   <211> 32
   <212> PRT
   <213> Homo sapiens
<400> 4028
   Arg Glu Arg Arg Arg Ser His Arg Ser Glu Arg Glu Arg Arg Arg Asp
   1 5 10 15
   Arg Asp Arg Asp Arg Asp Arg Asp Arg Glu His Lys Arg Gly Glu Arg
   20 25 30
<210> 4029
   <211> 10
   <212> PRT
   <213> Homo sapiens
<400> 4029
   Arg Lys Thr His Cys Ser Gly Arg Lys His
   1 5 10
<210> 4030
   <211> 10
   <212> PRT
   <213> Homo sapiens
<400> 4030
   Thr Arg Pro Gly Met Thr Arg Pro Asp Arg
   1 5 10
<210> 4031
   <211> 20
   <212> PRT
   <213> Mus musculus
<400> 4031
   Tyr Tyr Cys Ala Arg Phe Leu Trp Glu Pro Tyr Ala Met Asp Tyr Trp
   1 5 10 15
   Gly Gln Gly Ser
   20
<210> 4032
   <211> 34
   <212> PRT
   <213> Homo sapiens
<400> 4032
   Ala Val Ala Ala Thr Ala Ser Ile Ala Gly Ala Pro Thr Gln Tyr Pro
   1 5 10 15
   Pro Gly Arg Gly Thr Pro Pro Pro Pro Val Gly Arg Ala Thr Pro Pro
   20 25 30
   Pro Gly
<210> 4033
   <211> 13
   <212> PRT
   <213> Homo sapiens
<400> 4033
   Gly Ile Met Ala Pro Pro Pro Gly Met Arg Pro Pro Met
   1 5 10
<210> 4034
   <211> 22
   <212> PRT
   <213> Homo sapiens
<400> 4034
   Gly Val Gly Gly Pro Ser Gln Gln Val Met Thr Pro Gln Gly Arg Gly
   1 5 10 15
   Thr Val Ala Ala Ala Ala
   20
<210> 4035
   <211> 11
   <212> PRT
   <213> Homo sapiens
<400> 4035
   Met Gly Pro Pro Ile Gly Leu Pro Pro Ala Arg
   1 5 10
<210> 4036
   <211> 32
   <212> PRT
   <213> Homo sapiens
<400> 4036
   Arg Gly Thr Pro Ile Gly Met Pro Pro Pro Gly Met Arg Pro Pro Pro
   1 5 10 15
   Pro Gly Ile Arg Gly Pro Pro Pro Pro Gly Met Arg Pro Pro Arg Pro
   20 25 30
<210> 4037
   <211> 20
   <212> PRT
   <213> Homo sapiens
<400> 4037
   Glu Ala Leu Lys Lys Ile Ile Glu Asp Gln Gln Glu Ser Leu Asn Lys
   1 5 10 15
   Trp Lys Ser Lys
   20
<210> 4038
   <211> 20
   <212> PRT
   <213> Homo sapiens
<400> 4038
   Glu Thr Pro Gly Gln Lys Tyr Lys Glu Thr Asp Leu Leu Ile Leu Phe
   1 5 10 15
   Lys Asp Asp Tyr
   20
<210> 4039
   <211> 20
   <212> PRT
   <213> Homo sapiens
<400> 4039
   Phe Ser Asn His Gly Glu Ile Lys Trp Ile Asp Phe Val Arg Gly Ala
   1 5 10 15
   Lys Glu Gly Ile
   20
<210> 4040
   <211> 6
   <212> PRT
   <213> Homo sapiens
<400> 4040
   Gly Leu Glu Leu Pro Glu
   1 5
<210> 4041
   <211> 20
   <212> PRT
   <213> Homo sapiens
<400> 4041
   His Pro Met Leu Pro Asn Tyr Lys Asn Phe Lys Gly Thr Ile Gln Glu
   1 5 10 15
   Leu Gly Gln Asn
   20
<210> 4042
   <211> 6
   <212> PRT
   <213> Bos taurus
<400> 4042
   Ile Arg Gly Glu Arg Ala
   1               5
<210> 4043
   <211> 4
   <212> PRT
   <213> Homo sapiens
<400> 4043
   Lys Ile Arg Tyr
   1
<210> 4044
   <211> 4
   <212> PRT
   <213> Homo sapiens
<400> 4044
   Lys Lys Ile Lys
   1
<210> 4045
   <211> 4
   <212> PRT
   <213> Homo sapiens
<400> 4045
   Lys Asn Asp Lys
   1
<210> 4046
   <211> 4
   <212> PRT
   <213> Homo sapiens
<400> 4046
   Leu Asp Lys Lys
   1
<210> 4047
   <211> 20
   <212> PRT
   <213> Homo sapiens
<400> 4047
   Leu Ile Leu Phe Lys Asp Asp Tyr Phe Ala Lys Lys Asn Glu Glu Arg
   1 5 10 15
   Lys Gln Asn Lys
   20
<210> 4048
   <211> 12
   <212> PRT
   <213> Homo sapiens
<400> 4048
   Asn Gly Asn Leu Gln Leu Arg Asn Lys Glu Val Thr
   1 5 10
<210> 4049
   <211> 5
   <212> PRT
   <213> Homo sapiens
<400> 4049
   Pro Ile Val Glu Thr
   1 5
<210> 4050
   <211> 7
   <212> PRT
   <213> Homo sapiens
<400> 4050
   Gln Phe Ile Gly Tyr Pro Ile
   1 5
<210> 4051
   <211> 20
   <212> PRT
   <213> Homo sapiens
<400> 4051
   Ser Asp Tyr Lys Ser Ala His Lys Gly Phe Lys Gly Val Asp Ala Gln
   1 5 10 15
   Gly Thr Leu Ser
   20
<210> 4052
   <211> 5
   <212> PRT
   <213> Homo sapiens
<400> 4052
   Ser Lys Glu Gln Val
   1 5
<210> 4053
   <211> 20
   <212> PRT
   <213> Homo sapiens
<400> 4053
   Val Arg Gly Ala Lys Glu Gly Ile Ile Leu Phe Lys Glu Lys Ala Lys
   1 5 10 15
   Glu Ala Leu Gly
   20
<210> 4054
   <211> 10
   <212> PRT
   <213> Homo sapiens
<400> 4054
   Glu Trp Glu Asp Thr Tyr Gly Ile Val Leu
   1 5 10
<210> 4055
   <211> 10
   <212> PRT
   <213> Staphylococcus aureus
<400> 4055
   Glu Trp Glu Ser Thr Tyr Ser Ile Leu Gly
   1 5 10
<210> 4056
   <211> 50
   <212> PRT
   <213> Homo sapiens
<400> 4056
   Gly Tyr Gly Lys Ile Asn Lys Lys Arg Ile Ala Leu Thr Asp Asn Ala
   1 5 10 15
   Leu Ile Ala Arg Ser Leu Gly Lys Tyr Gly Ile Ile Cys Met Glu Asp
   20 25 30
   Leu Ile His Glu Ile Tyr Thr Val Gly Lys Arg Phe Lys Glu Ala Asn
   35 40 45
   Asn Phe
   50
<210> 4057
   <211> 23
   <212> PRT
   <213> Homo sapiens
<400> 4057
   Lys Met Leu Lys Asn Arg Glu Pro Val Gln Leu Glu Thr Leu Ser Ile
   1 5 10 15
   Arg Gly Asn Arg Ile Arg Tyr
   20
<210> 4058
   <211> 10
   <212> PRT
   <213> Human herpesvirus
<400> 4058
   Leu Trp Asp Gln Thr Tyr Gly Val Pro Asp
   1 5 10
<210> 4059
   <211> 25
   <212> PRT
   <213> Homo sapiens
<400> 4059
   Pro Ala Val Pro Glu Thr Leu Lys Lys Lys Arg Arg Asn Phe Ala Glu
   1 5 10 15
   Leu Lys Ile Lys Arg Leu Arg Lys Lys
   20 25
<210> 4060
   <211> 19
   <212> PRT
   <213> Homo sapiens
<400> 4060
   Arg Met Ala Arg Met Ala Arg Lys Ala Gly Asn Phe Tyr Val Pro Ala
   1 5 10 15
   Glu Pro Lys
<210> 4061
   <211> 10
   <212> PRT
   <213> Human immunodeficiency virus
<400> 4061
   Thr Trp Asn Asp Thr Tyr Gly Ser Asn Asn
   1 5 10
<210> 4062
   <211> 10
   <212> PRT
   <213> Homo sapiens
<400> 4062
   Thr Tyr Gly Ile Val Leu Leu Ser Gly Val
   1 5 10
<210> 4063
   <211> 14
   <212> PRT
   <213> Bos taurus
<400> 4063
   Ala Leu Gln Glu Ala Cys Glu Ala Tyr Leu Val Gly Leu Phe
   1 5 10
<210> 4064
   <211> 11
   <212> PRT
   <213> Trypanosoma cruzi
<400> 4064
   Glu Asp Asp Asp Met Gly Phe Gly Leu Phe Asp
   1 5 10
<210> 4065
   <211> 21
   <212> PRT
   <213> Homo sapiens
<400> 4065
   Gly Gly Gly Gly Gly Gly Ser Gly Gly Gly Gly Ser Gly Gly Gly Gly
   1 5 10 15
   Gly Gly Gly Ser Ser
   20
<210> 4066
   <211> 18
   <212> PRT
   <213> Bos taurus
<400> 4066
   Arg Phe Gln Ser Ser Ala Val Met Ala Leu Gln Glu Ala Cys Glu Ala
   1 5 10 15
   Tyr Leu
<210> 4067
   <211> 11
   <212> PRT
   <213> Homo sapiens
<400> 4067
   Thr Phe Ile Gln Phe Lys Lys Asp Leu Lys Glu
   1 5 10
<210> 4068
   <211> 21
   <212> PRT
   <213> Bos taurus
<400> 4068
   Ala Arg Thr Lys Gln Thr Ala Arg Lys Ser Thr Gly Gly Lys Ala Pro
   1 5 10 15
   Arg Lys Gln Leu Ala
   20
<210> 4069
   <211> 16
   <212> PRT
   <213> Bos taurus
<400> 4069
   Ala Val Thr Tyr Thr Glu His Ala Lys Arg Lys Thr Val Thr Ala Met
   1 5 10 15
<210> 4070
   <211> 18
   <212> PRT
   <213> Bos taurus
<400> 4070
   Gly Ala Gly Ala Pro Val Tyr Leu Ala Ala Val Leu Glu Tyr Leu Thr
   1 5 10 15
   Ala Glu
<210> 4071
   <211> 7
   <212> PRT
   <213> Escherichia coli
<400> 4071
   Ile Leu Pro Pro Pro Gly Tyr
   1 5
<210> 4072
   <211> 20
   <212> PRT
   <213> Bos taurus
<400> 4072
   Ile Gln Thr Ala Val Arg Leu Leu Leu Pro Gly Glu Leu Ala Lys His
   1 5 10 15
   Ala Val Ser Glu
   20
<210> 4073
   <211> 15
   <212> PRT
   <213> Bos taurus
<400> 4073
   Lys Gln Leu Ala Thr Lys Ala Ala Arg Lys Ser Ala Pro Ala Thr
   1 5 10 15
<210> 4074
   <211> 21
   <212> PRT
   <213> Bos taurus
<400> 4074
   Leu Ala Gly Asn Ala Ala Arg Asp Asn Lys Lys Thr Arg Ile Ile Pro
   1 5 10 15
   Arg His Leu Gln Leu
   20
<210> 4075
   <211> 16
   <212> PRT
   <213> Bos taurus
<400> 4075
   Leu Ala Ile Arg Asn Asp Glu Glu Leu Asn Lys Leu Leu Gly Lys Val
   1 5 10 15
<210> 4076
   <211> 14
   <212> PRT
   <213> Bos taurus
<400> 4076
   Leu Pro Lys Lys Thr Glu Ser His His Lys Ala Lys Gly Lys
   1 5 10
<210> 4077
   <211> 15
   <212> PRT
   <213> Bos taurus
<400> 4077
   Met Ser Gly Arg Gly Lys Gln Gly Gly Lys Ala Arg Ala Lys Ala
   1 5 10 15
<210> 4078
   <211> 16
   <212> PRT
   <213> Bos taurus
<400> 4078
   Pro Ala Thr Gly Gly Val Lys Lys Pro His Arg Tyr Arg Pro Gly Thr
   1 5 10 15
<210> 4079
   <211> 16
   <212> PRT
   <213> Bos taurus
<400> 4079
   Arg Tyr Arg Pro Gly Thr Val Ala Leu Arg Glu Ile Arg Arg Tyr Gln
   1 5 10 15
<210> 4080
   <211> 7
   <212> PRT
   <213> Mus musculus
<400> 4080
   Ser Pro Leu Asn Val Leu Met
   1 5
<210> 4081
   <211> 7
   <212> PRT
   <213> Human herpesvirus
<400> 4081
   Ser Pro Pro Glu Trp Leu Lys
   1 5
<210> 4082
   <211> 16
   <212> PRT
   <213> Bos taurus
<400> 4082
   Val His Pro Asp Thr Gly Ile Ser Ser Lys Ala Met Gly Ile Met Asn
   1 5 10 15
<210> 4083
   <211> 15
   <212> PRT
   <213> Bos taurus
<400> 4083
   Val Ser Glu Gly Thr Lys Ala Val Thr Lys Tyr Thr Ser Ser Lys
   1 5 10 15
<210> 4084
   <211> 8
   <212> PRT
   <213> Homo sapiens
<400> 4084
   Ala Asp Gly Lys Lys Ile Asp Gly
   1 5
<210> 4085
   <211> 8
   <212> PRT
   <213> Homo sapiens
<400> 4085
   Ala Ile Ala Leu Arg Glu Tyr Arg
   1 5
<210> 4086
   <211> 8
   <212> PRT
   <213> Homo sapiens
<400> 4086
   Ala Ile Cys Ser Gln Cys Ser Asp
   1 5
<210> 4087
   <211> 8
   <212> PRT
   <213> Unknown
<220>
   <223> epitope involved in systemic lupus
<400> 4087
   Ala Lys Ala Lys Ala Lys Ala Lys
   1               5
<210> 4088
   <211> 16
   <212> PRT
   <213> Homo sapiens
<400> 4088
   Ala Leu Ala Val Thr Lys Tyr Lys Gln Arg Asn Gly Trp Ser His Lys
   1               5                   10                  15
<210> 4089
   <211> 8
   <212> PRT
   <213> Homo sapiens
<400> 4089
   Ala Leu Phe Ala Pro Arg Asp Pro
   1               5
<210> 4090
   <211> 10
   <212> PRT
   <213> Homo sapiens
<400> 4090
   Ala Ser Ala Ala Arg Leu Thr Met Met Trp
   1               5                   10
<210> 4091
   <211> 11
   <212> PRT
   <213> Homo sapiens
<400> 4091
   Ala Val Glu Lys Val Lys Arg Thr Lys Asp Glu
   1               5                   10
<210> 4092
   <211> 16
   <212> PRT
   <213> Homo sapiens
<400> 4092
   Asp Val Glu Pro Lys Val Lys Ser Lys Lys Arg Glu Ala Val Ala Gly
   1               5                   10                  15
<210> 4093
   <211> 22
   <212> PRT
   <213> Homo sapiens
<400> 4093
   Glu Lys Asp Ser Tyr Val Val Ala Phe Ser Asp Glu Met Val Pro Cys
   1               5                   10                  15
   Pro Val Thr Thr Asp Met
               20
<210> 4094
   <211> 10
   <212> PRT
   <213> Homo sapiens
<400> 4094
   Glu Arg Met Glu Arg Lys Arg Arg Glu Lys
   1               5                   10
<210> 4095
   <211> 15
   <212> PRT
   <213> Homo sapiens
<400> 4095
   Phe Thr Phe Ile Gln Phe Lys Lys Asp Leu Lys Glu Ser Met Lys
   1               5                   10                  15
<210> 4096
   <211> 8
   <212> PRT
   <213> Homo sapiens
<400> 4096
   Gly Thr Gln Val His Gly Thr Ile
   1               5
<210> 4097
   <211> 8
   <212> PRT
   <213> Homo sapiens
<400> 4097
   His Pro Ala Ile Ala Leu Arg Glu
   1               5
<210> 4098
   <211> 8
   <212> PRT
   <213> Homo sapiens
<400> 4098
   Ile Ala Leu Arg Glu Tyr Arg Lys
   1 5
<210> 4099
   <211> 8
   <212> PRT
   <213> Homo sapiens
<400> 4099
   Ile Leu Pro Asp Ser Leu Pro Leu
   1 5
<210> 4100
   <211> 15
   <212> PRT
   <213> Homo sapiens
<400> 4100
   Lys Asp Lys Asp Arg Asp Arg Lys Arg Arg Ser Ser Arg Ser Arg
   1 5 10 15
<210> 4101
   <211> 8
   <212> PRT
   <213> Unknown
<220>
   <223> epitope involved in systemic lupus
<400> 4101
   Lys Lys Ala Lys Ala Lys Ala Lys
   1 5
<210> 4102
   <211> 15
   <212> PRT
   <213> Homo sapiens
<400> 4102
   Lys Lys Ala Pro Ala Lys Lys Gly Glu Lys Val Pro Lys Gly Lys
   1 5 10 15
<210> 4103
   <211> 4
   <212> PRT
   <213> Unknown
<220>
   <223> epitope involved in systemic lupus
<400> 4103
   Lys Lys Lys Lys
   1
<210> 4104
   <211> 16
   <212> PRT
   <213> Homo sapiens
<400> 4104
   Leu Cys Asn Glu Lys Leu Leu Lys Lys Ala Arg Ile His Pro Phe His
   1 5 10 15
<210> 4105
   <211> 16
   <212> PRT
   <213> Homo sapiens
<400> 4105
   Leu Phe Lys Pro Pro Lys Tyr His Pro Asp Val Pro Tyr Val Lys Arg
   1 5 10 15
<210> 4106
   <211> 8
   <212> PRT
   <213> Homo sapiens
<400> 4106
   Leu Leu Val Asp Val Glu Pro Lys
   1 5
<210> 4107
   <211> 12
   <212> PRT
   <213> Homo sapiens
<400> 4107
   Leu Pro Met Ile Trp Ala Gln Lys Thr Asn Thr Pro
   1 5 10
<210> 4108
   <211> 15
   <212> PRT
   <213> Homo sapiens
<400> 4108
   Leu Arg Lys Phe Lys Asn Ile Ser Tyr Phe Pro Gly Lys Leu Arg
   1 5 10 15
<210> 4109
   <211> 8
   <212> PRT
   <213> Homo sapiens
<400> 4109
   Leu Thr Ala Leu Leu Arg Asn Leu
   1 5
<210> 4110
   <211> 22
   <212> PRT
   <213> Homo sapiens
<400> 4110
   Met Glu Glu Ser Val Asn Gln Met Gln Pro Leu Asn Glu Lys Gln Ile
   1 5 10 15
   Ala Asn Ser Gln Asp Gly
   20
<210> 4111
   <211> 8
   <212> PRT
   <213> Homo sapiens
<400> 4111
   Met Pro Leu Thr Ala Leu Leu Arg
   1 5
<210> 4112
   <211> 14
   <212> PRT
   <213> Homo sapiens
<400> 4112
   Asn Thr His Leu Lys Ala Val Lys Met Thr Leu Lys Asn Arg
   1 5 10
<210> 4113
   <211> 8
   <212> PRT
   <213> Homo sapiens
<400> 4113
   Pro Ala Ile Ala Leu Arg Glu Tyr
   1 5
<210> 4114
   <211> 8
   <212> PRT
   <213> Homo sapiens
<400> 4114
   Pro Leu Thr Ala Leu Leu Arg Asn
   1 5
<210> 4115
   <211> 15
   <212> PRT
   <213> Homo sapiens
<400> 4115
   Gln Arg Arg Ser Ala Arg Leu Ser Ala Lys Pro Ala Pro Pro Lys
   1 5 10 15
<210> 4116
   <211> 8
   <212> PRT
   <213> Unknown
<220>
   <223> epitope involved in systemic lupus
<400> 4116
   Arg Ala Arg Ala Arg Ala Arg Ala
   1 5
<210> 4117
   <211> 28
   <212> PRT
   <213> Homo sapiens
<400> 4117
   Arg Glu Ala Val Ala Gly Arg Gly Arg Gly Arg Gly Arg Gly Arg Gly
   1 5 10 15
   Arg Gly Arg Gly Arg Gly Arg Gly Gly Pro Arg Arg
   20 25
<210> 4118
   <211> 8
   <212> PRT
   <213> Homo sapiens
<400> 4118
   Arg Phe Leu Met Lys Leu Ser His
   1 5
<210> 4119
   <211> 8
   <212> PRT
   <213> Unknown
<220>
   <223> epitope involved in systemic lupus
<400> 4119
   Arg Arg Ala Arg Ala Arg Ala Arg
   1 5
<210> 4120
   <211> 4
   <212> PRT
   <213> Unknown
<220>
   <223> epitope involved in systemic lupus
<400> 4120
   Arg Arg Arg Arg
   1
<210> 4121
   <211> 8
   <212> PRT
   <213> Homo sapiens
<400> 4121
   Arg Arg Arg Ser Arg Glu Arg Ser
   1 5
<210> 4122
   <211> 8
   <212> PRT
   <213> Homo sapiens
<400> 4122
   Arg Arg Ser His Arg Ser Glu Arg
   1 5
<210> 4123
   <211> 8
   <212> PRT
   <213> Homo sapiens
<400> 4123
   Arg Arg Ser Arg Ser Arg Asp Lys
   1 5
<210> 4124
   <211> 8
   <212> PRT
   <213> Homo sapiens
<400> 4124
   Ser Glu Gly Leu Ala Ile Val Thr
   1 5
<210> 4125
   <211> 17
   <212> PRT
   <213> Homo sapiens
<400> 4125
   Ser Lys Leu Ile Lys Ile Phe Gln Asp His Pro Leu Gln Lys Thr Tyr
   1 5 10 15
   Asn
<210> 4126
   <211> 8
   <212> PRT
   <213> Homo sapiens
<400> 4126
   Ser Arg Glu Arg Ser Lys Asp Lys
   1 5
<210> 4127
   <211> 8
   <212> PRT
   <213> Homo sapiens
<400> 4127
   Thr Ala Leu Leu Arg Asn Leu Gly
   1 5
<210> 4128
   <211> 8
   <212> PRT
   <213> Homo sapiens
<400> 4128
   Thr Leu Lys Asn Arg Glu Pro Val
   1 5
<210> 4129
   <211> 16
   <212> PRT
   <213> Homo sapiens
<400> 4129
   Thr Tyr Lys Thr Gly His Gly Leu Arg Gly Lys Leu Lys Trp Arg Pro
   1 5 10 15
<210> 4130
   <211> 8
   <212> PRT
   <213> Homo sapiens
<400> 4130
   Val Glu Arg Gly Arg Thr Val Lys
   1 5
<210> 4131
   <211> 8
   <212> PRT
   <213> Homo sapiens
<400> 4131
   Val His Pro Ala Ile Ala Leu Arg
   1 5
<210> 4132
   <211> 8
   <212> PRT
   <213> Homo sapiens
<400> 4132
   Val Lys Gly Trp Arg Pro Arg Arg
   1 5
<210> 4133
   <211> 12
   <212> PRT
   <213> Homo sapiens
<400> 4133
   Val Tyr Ser Lys Arg Ser Gly Lys Pro Arg Gly Tyr
   1 5 10
<210> 4134
   <211> 15
   <212> PRT
   <213> Homo sapiens
<400> 4134
   Tyr Lys His Ala Asp Gly Lys Lys Ile Asp Gly Arg Arg Val Leu
   1 5 10 15
<210> 4135
   <211> 15
   <212> PRT
   <213> Mus musculus
<400> 4135
   Ala Lys Ser Ala Pro Ala Pro Lys Lys Gly Ser Lys Lys Ala Val
   1 5 10 15
<210> 4136
   <211> 15
   <212> PRT
   <213> Mus musculus
<400> 4136
   Ala Pro Ala Pro Lys Lys Gly Ser Lys Lys Ala Val Thr Lys Ala
   1 5 10 15
<210> 4137
   <211> 15
   <212> PRT
   <213> Bos taurus
<400> 4137
   Ala Tyr Leu Val Gly Leu Phe Glu Asp Thr Asn Leu Cys Ala Ile
   1 5 10 15
<210> 4138
   <211> 15
   <212> PRT
   <213> Mus musculus
<400> 4138
   Asp Gly Lys Lys Arg Lys Arg Ser Arg Lys Glu Ser Tyr Ser Val
   1 5 10 15
<210> 4139
   <211> 15
   <212> PRT
   <213> Homo sapiens
<400> 4139
   Phe Leu Glu Asn Val Ile Arg Asp Ala Val Thr Tyr Thr Glu His
   1 5 10 15
<210> 4140
   <211> 15
   <212> PRT
   <213> Mus musculus
<400> 4140
   Gly Gly Ala Lys Arg His Arg Lys Val Leu Arg Asp Asn Ile Gln
   1 5 10 15
<210> 4141
   <211> 15
   <212> PRT
   <213> Mus musculus
<400> 4141
   Gly Ile Thr Lys Pro Ala Ile Arg Arg Ile Ala Arg Arg Gly Gly
   1 5 10 15
<210> 4142
   <211> 15
   <212> PRT
   <213> Homo sapiens
<400> 4142
   Gly Lys Gly Gly Lys Gly Leu Gly Lys Gly Gly Ala Lys Arg His
   1               5                   10                  15
<210> 4143
   <211> 15
   <212> PRT
   <213> Mus musculus
<400> 4143
   Gly Lys Gly Leu Gly Lys Gly Gly Ala Lys Arg His Arg Lys Val
   1               5                   10                  15
<210> 4144
   <211> 15
   <212> PRT
   <213> Mus musculus
<400> 4144
   Ile Met Met Ser Phe Val Asn Asp Ile Phe Glu Arg Ile Ala Gly
   1               5                   10                  15
<210> 4145
   <211> 15
   <212> PRT
   <213> Mus musculus
<400> 4145
   Ile Arg Arg Ile Ala Arg Arg Gly Gly Val Lys Arg Ile Ser Gly
   1               5                   10                  15
<210> 4146
   <211> 15
   <212> PRT
   <213> Mus musculus
<400> 4146
   Ile Ser Gly Leu Ile Tyr Glu Glu Thr Arg Gly Val Leu Lys Val
   1               5                   10                  15
<210> 4147
   <211> 15
   <212> PRT
   <213> Mus musculus
<400> 4147
   Lys Pro Ala Ile Arg Arg Ile Ala Arg Arg Gly Gly Val Lys Arg
   1               5                   10                  15
<210> 4148
   <211> 24
   <212> PRT
   <213> Mus musculus
<400> 4148
   Lys Arg His Arg Lys Val Leu Arg Asp Asn Ile Gln Gly Ile Thr Lys
   1               5                   10                  15
   Pro Ala Ile Arg Arg Ile Ala Arg
               20
<210> 4149
   <211> 15
   <212> PRT
   <213> Mus musculus
<400> 4149
   Leu Gly Lys Gly Gly Ala Lys Arg His Arg Lys Val Leu Arg Asp
   1               5                   10                  15
<210> 4150
   <211> 15
   <212> PRT
   <213> Mus musculus
<400> 4150
   Leu Lys Val Phe Leu Glu Asn Val Ile Arg Asp Ala Val Thr Tyr
   1               5                   10                  15
<210> 4151
   <211> 15
   <212> PRT
   <213> Mus musculus
<400> 4151
   Asn Asp Ile Phe Glu Arg Ile Ala Gly Glu Ala Ser Arg Leu Ala
   1               5                   10                  15
<210> 4152
   <211> 15
   <212> PRT
   <213> Homo sapiens
<400> 4152
   Asn Val Ile Arg Asp Ala Val Thr Tyr Thr Glu His Ala Lys Arg
   1               5                   10                  15
<210> 4153
   <211> 15
   <212> PRT
   <213> Mus musculus
<400> 4153
   Pro Glu Pro Ala Lys Ser Ala Pro Ala Pro Lys Lys Gly Ser Lys
   1               5                   10                  15
<210> 4154
   <211> 24
   <212> PRT
   <213> Mus musculus
<400> 4154
   Pro Lys Lys Gly Ser Lys Lys Ala Val Thr Lys Ala Gln Lys Lys Asp
   1               5                   10                  15
   Gly Lys Lys Arg Lys Arg Ser Arg
               20
<210> 4155
   <211> 15
   <212> PRT
   <213> Mus musculus
<400> 4155
   Gln Lys Lys Asp Gly Lys Lys Arg Lys Arg Ser Arg Lys Glu Ser
   1               5                   10                  15
<210> 4156
   <211> 15
   <212> PRT
   <213> Mus musculus
<400> 4156
   Arg Asp Ala Val Thr Tyr Thr Glu His Ala Lys Arg Lys Thr Val
   1               5                   10                  15
<210> 4157
   <211> 15
   <212> PRT
   <213> Mus musculus
<400> 4157
   Arg Gly Val Leu Lys Val Phe Leu Glu Asn Val Ile Arg Asp Ala
   1               5                   10                  15
<210> 4158
   <211> 15
   <212> PRT
   <213> Homo sapiens
<400> 4158
   Ser Gly Arg Gly Lys Gly Gly Lys Gly Leu Gly Lys Gly Gly Ala
   1               5                   10                  15
<210> 4159
   <211> 15
   <212> PRT
   <213> Mus musculus
<400> 4159
   Ser Arg Glu Ile Gln Thr Ala Val Arg Leu Leu Leu Pro Gly Glu
   1               5                   10                  15
<210> 4160
   <211> 15
   <212> PRT
   <213> Homo sapiens
<400> 4160
   Tyr Ala Leu Lys Arg Gln Gly Arg Thr Leu Tyr Gly Phe Gly Gly
   1               5                   10                  15
<210> 4161
   <211> 10
   <212> PRT
   <213> Homo sapiens
<400> 4161
   Ala Ala Phe Gln Gln Gly Lys Ile Pro Pro
   1               5                   10
<210> 4162
   <211> 8
   <212> PRT
   <213> Homo sapiens
<400> 4162
   Ala Met Pro Pro Gln Gln Leu Met
   1               5
<210> 4163
   <211> 10
   <212> PRT
   <213> Homo sapiens
<400> 4163
   Ala Pro Gly Met Arg Pro Pro Met Gly Gly
   1               5                   10
<210> 4164
   <211> 8
   <212> PRT
   <213> Homo sapiens
<400> 4164
   Ala Thr Lys Lys Ala Val Gln Gly
   1 5
<210> 4165
   <211> 8
   <212> PRT
   <213> Homo sapiens
<400> 4165
   Glu Val Arg Leu Val Pro Gly Arg
   1 5
<210> 4166
   <211> 10
   <212> PRT
   <213> Homo sapiens
<400> 4166
   Phe Gln Gln Gly Lys Ile Pro Pro Thr Pro
   1 5 10
<210> 4167
   <211> 8
   <212> PRT
   <213> Homo sapiens
<400> 4167
   Gly Ala Met Pro Pro Gln Gln Leu
   1 5
<210> 4168
   <211> 8
   <212> PRT
   <213> Homo sapiens
<400> 4168
   Gly Met Ile Pro Pro Pro Gly Leu
   1 5
<210> 4169
   <211> 8
   <212> PRT
   <213> Homo sapiens
<400> 4169
   Gly Met Pro Pro Met Thr Gln Ala
   1 5
<210> 4170
   <211> 10
   <212> PRT
   <213> Homo sapiens
<400> 4170
   Gly Pro Ala Pro Gly Met Arg Pro Pro Met
   1               5                   10
<210> 4171
   <211> 8
   <212> PRT
   <213> Homo sapiens
<400> 4171
   Gly Gln Pro Pro Tyr Met Pro Pro
   1               5
<210> 4172
   <211> 8
   <212> PRT
   <213> Homo sapiens
<400> 4172
   Ile Gly Thr Phe Lys Ala Phe Asp
   1               5
<210> 4173
   <211> 8
   <212> PRT
   <213> Homo sapiens
<400> 4173
   Ile Pro Pro Gly Ala Met Pro Pro
   1               5
<210> 4174
   <211> 8
   <212> PRT
   <213> Homo sapiens
<400> 4174
   Ile Pro Pro Pro Gly Leu Ala Pro
   1               5
<210> 4175
   <211> 8
   <212> PRT
   <213> Homo sapiens
<400> 4175
   Lys Pro Lys Ser Gln Glu Thr Pro
   1               5
<210> 4176
   <211> 8
   <212> PRT
   <213> Homo sapiens
<400> 4176
   Met Ile Pro Pro Pro Gly Leu Ala
   1 5
<210> 4177
   <211> 8
   <212> PRT
   <213> Homo sapiens
<400> 4177
   Met Pro Pro Met Thr Gln Ala Pro
   1 5
<210> 4178
   <211> 8
   <212> PRT
   <213> Homo sapiens
<400> 4178
   Met Pro Pro Pro Gly Met Ile Pro
   1 5
<210> 4179
   <211> 8
   <212> PRT
   <213> Homo sapiens
<400> 4179
   Met Pro Pro Gln Gln Leu Met Pro
   1 5
<210> 4180
   <211> 8
   <212> PRT
   <213> Homo sapiens
<400> 4180
   Met Thr Gln Ala Pro Arg Ile Met
   1 5
<210> 4181
   <211> 8
   <212> PRT
   <213> Homo sapiens
<400> 4181
   Pro Ala Thr Lys Lys Ala Val Gln
   1 5
<210> 4182
   <211> 8
   <212> PRT
   <213> Homo sapiens
<400> 4182
   Pro Gly Ala Met Pro Pro Gln Gln
   1 5
<210> 4183
   <211> 8
   <212> PRT
   <213> Homo sapiens
<400> 4183
   Pro Gly Met Ile Pro Pro Pro Gly
   1 5
<210> 4184
   <211> 8
   <212> PRT
   <213> Homo sapiens
<400> 4184
   Pro Met Thr Gln Ala Pro Arg Ile
   1 5
<210> 4185
   <211> 8
   <212> PRT
   <213> Homo sapiens
<400> 4185
   Pro Pro Ala Gln Pro Leu Ser Glu
   1 5
<210> 4186
   <211> 8
   <212> PRT
   <213> Homo sapiens
<400> 4186
   Pro Pro Gly Ala Met Pro Pro Gln
   1 5
<210> 4187
   <211> 8
   <212> PRT
   <213> Homo sapiens
<400> 4187
   Pro Pro Gly Met Ile Pro Pro Pro
   1 5
<210> 4188
   <211> 8
   <212> PRT
   <213> Homo sapiens
<400> 4188
   Pro Pro Met Thr Gln Ala Pro Arg
   1 5
<210> 4189
   <211> 8
   <212> PRT
   <213> Homo sapiens
<400> 4189
   Pro Pro Pro Gly Leu Ala Pro Gly
   1 5
<210> 4190
   <211> 8
   <212> PRT
   <213> Homo sapiens
<400> 4190
   Pro Pro Gln Gln Leu Met Pro Gly
   1 5
<210> 4191
   <211> 8
   <212> PRT
   <213> Homo sapiens
<400> 4191
   Pro Pro Tyr Met Pro Pro Pro Gly
   1 5
<210> 4192
   <211> 10
   <212> PRT
   <213> Homo sapiens
<400> 4192
   Pro Val Gly Pro Ala Pro Gly Met Arg Pro
   1 5 10
<210> 4193
   <211> 8
   <212> PRT
   <213> Homo sapiens
<400> 4193
   Pro Tyr Met Pro Pro Pro Gly Met
   1 5
<210> 4194
   <211> 8
   <212> PRT
   <213> Homo sapiens
<400> 4194
   Gln Pro Pro Tyr Met Pro Pro Pro
   1 5
<210> 4195
   <211> 8
   <212> PRT
   <213> Homo sapiens
<400> 4195
   Arg Gly Val Gly Gly Pro Ser Gln
   1 5
<210> 4196
   <211> 8
   <212> PRT
   <213> Homo sapiens
<400> 4196
   Thr Pro Ala Thr Lys Lys Ala Val
   1 5
<210> 4197
   <211> 8
   <212> PRT
   <213> Homo sapiens
<400> 4197
   Tyr Met Pro Pro Pro Gly Met Ile
   1 5
<210> 4198
   <211> 40
   <212> PRT
   <213> Homo sapiens
<400> 4198
   Arg Arg Glu Phe Glu Val Tyr Gly Pro Ile Lys Arg Ile His Met Val
   1 5 10 15
   Tyr Ser Lys Arg Ser Gly Lys Pro Arg Gly Tyr Ala Phe Ile Glu Tyr
   20 25 30
   Glu His Glu Arg Asp Met His Ser
   35 40
<210> 4199
   <211> 17
   <212> PRT
   <213> Homo sapiens
<400> 4199
   Lys Ile Leu Gly Asn Gln Gly Ser Phe Leu Thr Lys Gly Pro Ser Lys
   1 5 10 15
   Leu
<210> 4200
   <211> 12
   <212> PRT
   <213> Homo sapiens
<400> 4200
   Thr Tyr Ile Cys Glu Val Glu Asp Gln Lys Glu Glu
   1 5 10
<210> 4201
   <211> 18
   <212> PRT
   <213> Homo sapiens
<400> 4201
   Ala Glu Ile Glu Thr Asp Lys Ala Thr Ile Gly Phe Glu Val Gln Glu
   1 5 10 15
   Glu Gly
<210> 4202
   <211> 15
   <212> PRT
   <213> Homo sapiens
<400> 4202
   Gly Leu Lys Lys Met Leu Arg Thr Cys Ala Val His Ile Thr Leu
   1 5 10 15
<210> 4203
   <211> 18
   <212> PRT
   <213> Homo sapiens
<400> 4203
   Ser Ala Pro Ala Thr Gly Gly Val Lys Lys Pro His Arg Tyr Arg Pro
   1               5                   10                  15
   Gly Thr
<210> 4204
   <211> 15
   <212> PRT
   <213> Homo sapiens
<400> 4204
   Ala Gly Ala Gly Arg Val Arg Thr Ser Ala Arg Ala Gly Ser Thr
   1               5                   10                  15
<210> 4205
   <211> 15
   <212> PRT
   <213> Homo sapiens
<400> 4205
   Ala Pro Ala Pro Arg Thr Arg Tyr Pro Thr Arg Ala Pro Ser Gly
   1               5                   10                  15
<210> 4206
   <211> 15
   <212> PRT
   <213> Homo sapiens
<400> 4206
   Ala Thr Ala Arg Arg Lys Arg Arg Trp Ala Thr Arg Gly Pro Ala
   1               5                   10                  15
<210> 4207
   <211> 15
   <212> PRT
   <213> Torque teno virus
<400> 4207
   Ala Val Leu Arg Ala Pro Gln Asn Pro Pro Pro Pro Gly Pro Gln
   1               5                   10                  15
<210> 4208
   <211> 15
   <212> PRT
   <213> Homo sapiens
<400> 4208
   Cys Ala Gly Trp Ala Leu Trp Arg Cys Ala Leu Arg Trp Ala Glu
   1 5 10 15
<210> 4209
   <211> 15
   <212> PRT
   <213> Torque teno virus
<400> 4209
   Asp Glu Tyr Lys Arg Phe Met Asn Tyr Trp Thr Ala Ser Asn Glu
   1 5 10 15
<210> 4210
   <211> 15
   <212> PRT
   <213> Human herpesvirus
<400> 4210
   Asp Pro Val Gly His Pro Ala Ala Pro Arg Ala Pro Gly Pro Glu
   1 5 10 15
<210> 4211
   <211> 15
   <212> PRT
   <213> Torque teno virus
<400> 4211
   Asp Arg Gly Glu Gly Asn Met Leu Trp Ile Asp Trp Leu Ser Lys
   1 5 10 15
<210> 4212
   <211> 15
   <212> PRT
   <213> Homo sapiens
<400> 4212
   Asp Arg Arg Arg Glu Gly Pro Asp Arg Ser Pro Arg Gln Pro Pro
   1 5 10 15
<210> 4213
   <211> 15
   <212> PRT
   <213> Homo sapiens
<400> 4213
   Gly Pro Asp Arg Ser Pro Arg Gln Pro Pro Val Leu Pro Ala Ala
   1 5 10 15
<210> 4214
   <211> 15
   <212> PRT
   <213> Homo sapiens
<400> 4214
   Gly Pro Gly Ala Gly Asp Arg Arg Arg Glu Gly Pro Asp Arg Ser
   1               5                   10                  15
<210> 4215
   <211> 15
   <212> PRT
   <213> Homo sapiens
<400> 4215
   His Pro Ala Phe Ala Arg Ala His Gly Glu Ala Gly Ala Gly Arg
   1               5                   10                  15
<210> 4216
   <211> 15
   <212> PRT
   <213> Homo sapiens
<400> 4216
   His Arg Ala Leu Ala Arg Gly Pro Pro Ala Arg Leu Gly Gly Glu
   1               5                   10                  15
<210> 4217
   <211> 15
   <212> PRT
   <213> Homo sapiens
<400> 4217
   Lys Arg Arg Trp Ala Thr Arg Gly Pro Ala His Pro Ala Phe Ala
   1               5                   10                  15
<210> 4218
   <211> 15
   <212> PRT
   <213> Homo sapiens
<400> 4218
   Leu Arg Trp Ala Glu Arg Gln Val Gly Ala Leu Gly Ala Glu Ser
   1               5                   10                  15
<210> 4219
   <211> 15
   <212> PRT
   <213> Homo sapiens
<400> 4219
   Leu Trp Arg Cys Ala Leu Arg Trp Ala Glu Arg Gln Val Gly Ala
   1               5                   10                  15
<210> 4220
   <211> 15
   <212> PRT
   <213> Homo sapiens
<400> 4220
   Pro Gln Asp Pro Arg Ser Pro Gly Pro Ala Pro Arg His Arg Arg
   1               5                   10                  15
<210> 4221
   <211> 15
   <212> PRT
   <213> Homo sapiens
<400> 4221
   Pro Arg Ala Pro Pro Ala Arg Ala Ser Tyr Arg Arg Phe Arg Thr
   1               5                   10                  15
<210> 4222
   <211> 15
   <212> PRT
   <213> Human herpesvirus
<400> 4222
   Pro Arg His Gly His Ser Gln Gly Pro Trp Lys Pro Trp Ser Ala
   1               5                   10                  15
<210> 4223
   <211> 15
   <212> PRT
   <213> Homo sapiens
<400> 4223
   Pro Arg His Arg His Pro Gln Asp Pro Arg Ser Pro Gly Pro Ala
   1               5                   10                  15
<210> 4224
   <211> 15
   <212> PRT
   <213> Homo sapiens
<400> 4224
   Pro Arg His Arg Arg Pro Pro Arg Pro Asp Pro Arg Ala Pro Pro
   1               5                   10                  15
<210> 4225
   <211> 15
   <212> PRT
   <213> Torque teno virus
<400> 4225
   Pro Arg Ser Arg His Pro Gly Gly Pro Gly Thr Pro Gln Ile Arg
   1               5                   10                  15
<210> 4226
   <211> 15
   <212> PRT
   <213> Human adenovirus
<400> 4226
   Pro Ser Arg Arg Arg Arg Arg Arg Lys Ala Thr Leu Ala Pro Ala
   1 5 10 15
<210> 4227
   <211> 15
   <212> PRT
   <213> Homo sapiens
<400> 4227
   Arg Ala His Gly Glu Ala Gly Ala Gly Arg Val Arg Thr Ser Ala
   1 5 10 15
<210> 4228
   <211> 15
   <212> PRT
   <213> Homo sapiens
<400> 4228
   Arg Ala Pro Ser Gly Pro Arg Pro Pro Ser Arg Ser Gln Ala Gln
   1 5 10 15
<210> 4229
   <211> 15
   <212> PRT
   <213> Homo sapiens
<400> 4229
   Arg Leu Gly Gly Glu Gly Pro Gly Ala Gly Asp Arg Arg Arg Glu
   1 5 10 15
<210> 4230
   <211> 15
   <212> PRT
   <213> Homo sapiens
<400> 4230
   Arg Leu Ser Pro Gly His Arg Ala Leu Ala Arg Gly Pro Pro Ala
   1 5 10 15
<210> 4231
   <211> 15
   <212> PRT
   <213> Human herpesvirus
<400> 4231
   Arg Val Pro Arg Ile Pro Arg Asp Pro Arg Pro Pro Arg Pro Pro
   1 5 10 15
<210> 4232
   <211> 15
   <212> PRT
   <213> Homo sapiens
<400> 4232
   Ser Pro Gly Pro Ala Pro Arg His Arg Arg Pro Pro Arg Pro Asp
   1 5 10 15
<210> 4233
   <211> 15
   <212> PRT
   <213> Homo sapiens
<400> 4233
   Ser Trp Glu Arg Arg Arg Leu Ser Pro Gly His Arg Ala Leu Ala
   1 5 10 15
<210> 4234
   <211> 15
   <212> PRT
   <213> Homo sapiens
<400> 4234
   Thr Leu Arg Pro Ala Ala Thr Ala Arg Arg Lys Arg Arg Trp Ala
   1 5 10 15
<210> 4235
   <211> 15
   <212> PRT
   <213> Homo sapiens
<400> 4235
   Thr Pro Pro Arg Ser Val Pro Arg Leu Arg Pro Arg His Arg His
   1 5 10 15
<210> 4236
   <211> 15
   <212> PRT
   <213> Homo sapiens
<400> 4236
   Thr Arg Gly Pro Ala His Pro Ala Phe Ala Arg Ala His Gly Glu
   1 5 10 15
<210> 4237
   <211> 15
   <212> PRT
   <213> Human herpesvirus
<400> 4237
   Val Ala Ser Arg Pro Leu Phe Pro Pro Arg Ser Pro Gly Pro Ser
   1 5 10 15
<210> 4238
   <211> 15
   <212> PRT
   <213> Homo sapiens
<400> 4238
   Val Pro Arg Leu Arg Pro Arg His Arg His Pro Gln Asp Pro Arg
   1 5 10 15
<210> 4239
   <211> 15
   <212> PRT
   <213> Torque teno virus
<400> 4239
   Trp Trp Ala Arg Arg Arg Arg Arg Trp Arg Arg Trp Lys Arg Arg
   1 5 10 15
<210> 4240
   <211> 15
   <212> PRT
   <213> Sus scrofa
<400> 4240
   Ala Lys Lys Gly Glu Lys Val Pro Lys Gly Lys Lys Gly Lys Ala
   1 5 10 15
<210> 4241
   <211> 15
   <212> PRT
   <213> Sus scrofa
<400> 4241
   Ala Lys Pro Ala Pro Pro Lys Pro Glu Pro Lys Pro Lys Lys Ala
   1 5 10 15
<210> 4242
   <211> 15
   <212> PRT
   <213> Sus scrofa
<400> 4242
   Ala Arg Leu Ser Ala Lys Pro Ala Pro Pro Lys Pro Glu Pro Lys
   1 5 10 15
<210> 4243
   <211> 15
   <212> PRT
   <213> Sus scrofa
<400> 4243
   Glu Lys Val Pro Lys Gly Lys Lys Gly Lys Ala Asp Ala Gly Lys
   1               5                   10                  15
<210> 4244
   <211> 15
   <212> PRT
   <213> Sus scrofa
<400> 4244
   Glu Pro Lys Pro Lys Lys Ala Pro Ala Lys Lys Gly Glu Lys Val
   1               5                   10                  15
<210> 4245
   <211> 14
   <212> PRT
   <213> Human herpesvirus
<400> 4245
   Gly Arg Gly Arg Gly Arg Gly Arg Gly Arg Gly Gly Arg Gly
   1               5                   10
<210> 4246
   <211> 14
   <212> PRT
   <213> Homo sapiens
<400> 4246
   Ile Lys Lys Val Ala Thr Pro Lys Lys Ala Ser Pro Lys Lys
   1               5                   10
<210> 4247
   <211> 15
   <212> PRT
   <213> Sus scrofa
<400> 4247
   Lys Asp Glu Pro Gln Arg Arg Ser Ala Arg Leu Ser Ala Lys Pro
   1               5                   10                  15
<210> 4248
   <211> 15
   <212> PRT
   <213> Homo sapiens
<400> 4248
   Lys Ser Pro Lys Lys Ala Lys Ala Ala Lys Pro Lys Lys Ala Pro
   1               5                   10                  15
<210> 4249
   <211> 15
   <212> PRT
   <213> Unknown
<220>
   <223> epitope involved in systemic lupus
<400> 4249
   Lys Thr Pro Lys Lys Ala Lys Lys Pro Lys Thr Pro Lys Lys Ala
   1               5                   10                  15
<210> 4250
   <211> 15
   <212> PRT
   <213> Homo sapiens
<400> 4250
   Pro Lys Lys Ser Ala Lys Lys Thr Pro Lys Lys Ala Lys Lys Pro
   1               5                   10                  15
<210> 4251
   <211> 15
   <212> PRT
   <213> Sus scrofa
<400> 4251
   Pro Lys Arg Lys Ala Glu Gly Asp Ala Lys Gly Asp Lys Ala Lys
   1               5                   10                  15
<210> 4252
   <211> 15
   <212> PRT
   <213> Sus scrofa
<400> 4252
   Pro Pro Lys Pro Glu Pro Lys Pro Lys Lys Ala Pro Ala Lys Lys
   1               5                   10                  15
<210> 4253
   <211> 16
   <212> PRT
   <213> Homo sapiens
<400> 4253
   Ala Ala Ala Gly Tyr Asp Val Glu Lys Asn Asn Ser Arg Ile Lys Leu
   1               5                   10                  15
<210> 4254
   <211> 15
   <212> PRT
   <213> Homo sapiens
<400> 4254
   Ala Ala Ala Pro Ala Glu Glu Lys Lys Val Glu Ala Lys Lys Glu
   1 5 10 15
<210> 4255
   <211> 15
   <212> PRT
   <213> Homo sapiens
<400> 4255
   Ala Ala Ala Pro Val Ala Ala Ala Thr Thr Ala Ala Pro Ala Ala
   1 5 10 15
<210> 4256
   <211> 16
   <212> PRT
   <213> Homo sapiens
<400> 4256
   Ala Ala Lys Pro Lys Thr Ala Lys Pro Lys Ala Ala Lys Pro Lys Lys
   1 5 10 15
<210> 4257
   <211> 15
   <212> PRT
   <213> Homo sapiens
<400> 4257
   Ala Ala Pro Ala Lys Val Glu Ala Lys Glu Glu Ser Glu Glu Ser
   1 5 10 15
<210> 4258
   <211> 15
   <212> PRT
   <213> Homo sapiens
<400> 4258
   Ala Glu Glu Lys Lys Val Glu Ala Lys Lys Glu Glu Ser Glu Glu
   1 5 10 15
<210> 4259
   <211> 8
   <212> PRT
   <213> Homo sapiens
<400> 4259
   Ala Phe Asp Lys His Met Asn Leu
   1 5
<210> 4260
   <211> 16
   <212> PRT
   <213> Homo sapiens
<400> 4260
   Ala Gly Ala Ala Lys Arg Lys Ala Ser Gly Pro Pro Val Ser Glu Leu
   1 5 10 15
<210> 4261
   <211> 15
   <212> PRT
   <213> Homo sapiens
<400> 4261
   Ala Gly Gly Pro Ala Pro Ala Ala Gly Ala Ala Pro Ala Gly Gly
   1 5 10 15
<210> 4262
   <211> 16
   <212> PRT
   <213> Homo sapiens
<400> 4262
   Ala Lys Lys Ala Gly Ala Ala Lys Ala Lys Lys Pro Ala Gly Ala Ala
   1 5 10 15
<210> 4263
   <211> 8
   <212> PRT
   <213> Homo sapiens
<400> 4263
   Ala Leu Leu Arg Asn Leu Gly Lys
   1 5
<210> 4264
   <211> 8
   <212> PRT
   <213> Homo sapiens
<400> 4264
   Ala Leu Arg Glu Tyr Arg Lys Lys
   1 5
<210> 4265
   <211> 15
   <212> PRT
   <213> Homo sapiens
<400> 4265
   Ala Pro Ala Ala Gly Ala Ala Pro Ala Gly Gly Pro Ala Pro Ser
   1 5 10 15
<210> 4266
   <211> 16
   <212> PRT
   <213> Homo sapiens
<400> 4266
   Ala Pro Lys Ser Pro Ala Lys Ala Lys Ala Val Lys Pro Lys Ala Ala
   1 5 10 15
<210> 4267
   <211> 15
   <212> PRT
   <213> Homo sapiens
<400> 4267
   Ala Pro Ser Thr Ala Ala Ala Pro Ala Glu Glu Lys Lys Val Glu
   1 5 10 15
<210> 4268
   <211> 16
   <212> PRT
   <213> Homo sapiens
<400> 4268
   Ala Thr Pro Lys Lys Ser Ala Lys Lys Thr Pro Lys Lys Ala Lys Lys
   1 5 10 15
<210> 4269
   <211> 8
   <212> PRT
   <213> Homo sapiens
<400> 4269
   Ala Thr Pro Pro Pro Gly Ile Met
   1 5
<210> 4270
   <211> 8
   <212> PRT
   <213> Homo sapiens
<400> 4270
   Cys Asp Glu Phe Arg Lys Ile Lys
   1 5
<210> 4271
   <211> 15
   <212> PRT
   <213> Homo sapiens
<400> 4271
   Cys Asn Val Gly Ala Gly Gly Pro Ala Pro Ala Ala Gly Ala Ala
   1 5 10 15
<210> 4272
   <211> 15
   <212> PRT
   <213> Homo sapiens
<400> 4272
   Asp Asn Val Gly Ser Lys Gln Met Gln Gln Ile Arg Met Ser Leu
   1 5 10 15
<210> 4273
   <211> 15
   <212> PRT
   <213> Homo sapiens
<400> 4273
   Asp Arg Ala Thr Trp Lys Ser Asn Tyr Phe Leu Lys Ile Ile Gln
   1 5 10 15
<210> 4274
   <211> 15
   <212> PRT
   <213> Homo sapiens
<400> 4274
   Asp Arg Leu Asn Lys Val Ile Ser Glu Leu Asn Gly Lys Asn Ile
   1 5 10 15
<210> 4275
   <211> 15
   <212> PRT
   <213> Homo sapiens
<400> 4275
   Asp Val Gln Leu Ile Lys Thr Gly Asp Lys Val Gly Ala Ser Glu
   1 5 10 15
<210> 4276
   <211> 15
   <212> PRT
   <213> Homo sapiens
<400> 4276
   Glu Ala Asp Asp Asp Arg Leu Asn Lys Val Ile Ser Glu Leu Asn
   1 5 10 15
<210> 4277
   <211> 15
   <212> PRT
   <213> Homo sapiens
<400> 4277
   Glu Asp Lys Ile Asn Ala Leu Ile Lys Ala Ala Gly Val Asn Val
   1 5 10 15
<210> 4278
   <211> 8
   <212> PRT
   <213> Homo sapiens
<400> 4278
   Glu Gly Pro Pro Pro Lys Asp Thr
   1 5
<210> 4279
   <211> 15
   <212> PRT
   <213> Homo sapiens
<400> 4279
   Glu Ile Leu Ser Asp Val Gln Leu Ile Lys Thr Gly Asp Lys Val
   1 5 10 15
<210> 4280
   <211> 15
   <212> PRT
   <213> Homo sapiens
<400> 4280
   Glu Lys Lys Glu Glu Ser Glu Glu Ser Asp Asp Asp Met Gly Phe
   1 5 10 15
<210> 4281
   <211> 16
   <212> PRT
   <213> Homo sapiens
<400> 4281
   Glu Lys Thr Pro Val Lys Lys Lys Ala Arg Lys Ser Ala Gly Ala Ala
   1 5 10 15
<210> 4282
   <211> 15
   <212> PRT
   <213> Homo sapiens
<400> 4282
   Glu Lys Thr Ser Phe Phe Gln Ala Leu Gly Ile Thr Thr Lys Ile
   1 5 10 15
<210> 4283
   <211> 15
   <212> PRT
   <213> Homo sapiens
<400> 4283
   Glu Leu Asn Gly Lys Asn Ile Glu Asp Val Ile Ala Gln Gly Ile
   1 5 10 15
<210> 4284
   <211> 15
   <212> PRT
   <213> Homo sapiens
<400> 4284
   Glu Ser Glu Glu Ser Asp Glu Asp Met Gly Phe Gly Leu Phe Asp
   1 5 10 15
<210> 4285
   <211> 8
   <212> PRT
   <213> Homo sapiens
<400> 4285
   Glu Tyr Arg Lys Lys Met Asp Ile
   1 5
<210> 4286
   <211> 15
   <212> PRT
   <213> Homo sapiens
<400> 4286
   Phe Phe Gln Ala Leu Gly Ile Thr Thr Lys Ile Ser Arg Gly Thr
   1 5 10 15
<210> 4287
   <211> 8
   <212> PRT
   <213> Homo sapiens
<400> 4287
   Phe Lys Ala Phe Asp Lys His Met
   1 5
<210> 4288
   <211> 15
   <212> PRT
   <213> Homo sapiens
<400> 4288
   Gly Ala Val Ala Val Ser Ala Ala Pro Gly Ser Ala Ala Pro Ala
   1 5 10 15
<210> 4289
   <211> 15
   <212> PRT
   <213> Homo sapiens
<400> 4289
   Gly Gly Asn Ser Ser Pro Ser Ala Lys Asp Ile Lys Lys Ile Leu
   1 5 10 15
<210> 4290
   <211> 15
   <212> PRT
   <213> Homo sapiens
<400> 4290
   Gly Lys Ala Val Val Leu Met Gly Lys Asn Thr Met Met Arg Lys
   1 5 10 15
<210> 4291
   <211> 15
   <212> PRT
   <213> Homo sapiens
<400> 4291
   Gly Leu Gly Pro Glu Lys Thr Ser Phe Phe Gln Ala Leu Gly Ile
   1 5 10 15
<210> 4292
   <211> 8
   <212> PRT
   <213> Homo sapiens
<400> 4292
   Gly Leu Pro Pro Ala Arg Gly Thr
   1 5
<210> 4293
   <211> 8
   <212> PRT
   <213> Homo sapiens
<400> 4293
   Gly Met Pro Pro Pro Gly Met Arg
   1 5
<210> 4294
   <211> 8
   <212> PRT
   <213> Homo sapiens
<400> 4294
   Gly Pro Pro Pro Pro Gly Met Arg
   1 5
<210> 4295
   <211> 15
   <212> PRT
   <213> Homo sapiens
<400> 4295
   Gly Ser Leu Ile Cys Asn Val Gly Ala Gly Gly Pro Ala Pro Ala
   1 5 10 15
<210> 4296
   <211> 8
   <212> PRT
   <213> Homo sapiens
<400> 4296
   Gly Thr Phe Lys Ala Phe Asp Lys
   1 5
<210> 4297
   <211> 8
   <212> PRT
   <213> Homo sapiens
<400> 4297
   Ile Gly Leu Pro Pro Ala Arg Gly
   1 5
<210> 4298
   <211> 16
   <212> PRT
   <213> Homo sapiens
<400> 4298
   Ile Lys Leu Gly Leu Lys Ser Leu Val Ser Lys Gly Thr Leu Val Gln
   1 5 10 15
<210> 4299
   <211> 15
   <212> PRT
   <213> Homo sapiens
<400> 4299
   Ile Lys Thr Gly Asp Lys Val Gly Ala Ser Glu Ala Thr Leu Leu
   1 5 10 15
<210> 4300
   <211> 15
   <212> PRT
   <213> Homo sapiens
<400> 4300
   Ile Gln Gln Val Phe Asp Asn Gly Ser Ile Tyr Asn Pro Glu Val
   1 5 10 15
<210> 4301
   <211> 8
   <212> PRT
   <213> Homo sapiens
<400> 4301
   Ile Trp Ala Gln Lys Thr Asn Thr
   1 5
<210> 4302
   <211> 15
   <212> PRT
   <213> Homo sapiens
<400> 4302
   Lys Ala Ala Gly Val Asn Val Glu Pro Phe Trp Pro Gly Leu Phe
   1 5 10 15
<210> 4303
   <211> 8
   <212> PRT
   <213> Homo sapiens
<400> 4303
   Lys Ala Phe Asp Lys His Met Asn
   1 5
<210> 4304
   <211> 16
   <212> PRT
   <213> Homo sapiens
<400> 4304
   Lys Ala Lys Lys Pro Ala Ala Ala Ala Gly Ala Lys Lys Ala Lys Ser
   1 5 10 15
<210> 4305
   <211> 15
   <212> PRT
   <213> Homo sapiens
<400> 4305
   Lys Glu Glu Ser Glu Glu Ser Asp Glu Asp Met Gly Phe Gly Leu
   1 5 10 15
<210> 4306
   <211> 17
   <212> PRT
   <213> Homo sapiens
<400> 4306
   Lys Glu Ile Lys Lys Val Ala Thr Pro Lys Lys Ala Ser Lys Pro Lys
   1               5                   10                  15
   Lys
<210> 4307
   <211> 15
   <212> PRT
   <213> Homo sapiens
<400> 4307
   Lys Lys Glu Glu Ser Glu Glu Ser Asp Asp Asp Met Gly Phe Gly
   1               5                   10                  15
<210> 4308
   <211> 16
   <212> PRT
   <213> Homo sapiens
<400> 4308
   Lys Leu Asn Lys Lys Ala Ala Ser Gly Glu Ala Lys Pro Lys Ala Lys
   1               5                   10                  15
<210> 4309
   <211> 15
   <212> PRT
   <213> Homo sapiens
<400> 4309
   Lys Asn Ile Glu Asp Val Ile Ala Gln Gly Ile Gly Lys Leu Ala
   1               5                   10                  15
<210> 4310
   <211> 16
   <212> PRT
   <213> Homo sapiens
<400> 4310
   Lys Ser Pro Lys Lys Ala Lys Ala Ala Lys Pro Lys Lys Ala Pro Lys
   1               5                   10                  15
<210> 4311
   <211> 15
   <212> PRT
   <213> Gallus gallus
<400> 4311
   Lys Ser Arg Lys Ala Ser Lys Ala Lys Lys Val Lys Arg Ser Lys
   1               5                   10                  15
<210> 4312
   <211> 15
   <212> PRT
   <213> Homo sapiens
<400> 4312
   Lys Val Glu Ala Lys Glu Glu Ser Glu Glu Ser Asp Glu Asp Met
   1 5 10 15
<210> 4313
   <211> 15
   <212> PRT
   <213> Homo sapiens
<400> 4313
   Lys Val Glu Ala Lys Lys Glu Glu Ser Glu Glu Ser Asp Asp Asp
   1 5 10 15
<210> 4314
   <211> 15
   <212> PRT
   <213> Homo sapiens
<400> 4314
   Leu Ala Ala Leu Gly Gly Asn Ser Ser Pro Ser Ala Lys Asp Ile
   1 5 10 15
<210> 4315
   <211> 15
   <212> PRT
   <213> Homo sapiens
<400> 4315
   Leu Ala Asp Pro Ser Ala Phe Val Ala Ala Ala Pro Val Ala Ala
   1 5 10 15
<210> 4316
   <211> 8
   <212> PRT
   <213> Homo sapiens
<400> 4316
   Leu Pro Pro Ala Arg Gly Thr Pro
   1 5
<210> 4317
   <211> 11
   <212> PRT
   <213> Homo sapiens
<400> 4317
   Leu Gln Glu Leu Glu Lys Asp Glu Arg Glu Gln
   1 5 10
<210> 4318
   <211> 8
   <212> PRT
   <213> Homo sapiens
<400> 4318
   Leu Arg Glu Tyr Arg Lys Lys Met
   1 5
<210> 4319
   <211> 8
   <212> PRT
   <213> Homo sapiens
<400> 4319
   Met Ala Pro Pro Pro Gly Met Arg
   1 5
<210> 4320
   <211> 15
   <212> PRT
   <213> Homo sapiens
<400> 4320
   Met Ser Leu Arg Gly Lys Ala Val Val Leu Met Gly Lys Asn Thr
   1 5 10 15
<210> 4321
   <211> 15
   <212> PRT
   <213> Homo sapiens
<400> 4321
   Pro Ala Ala Ala Ala Ala Pro Ala Lys Val Glu Ala Lys Glu Glu
   1 5 10 15
<210> 4322
   <211> 16
   <212> PRT
   <213> Homo sapiens
<400> 4322
   Pro Ala Gly Ala Ala Lys Lys Pro Lys Lys Ala Thr Gly Ala Ala Thr
   1 5 10 15
<210> 4323
   <211> 15
   <212> PRT
   <213> Homo sapiens
<400> 4323
   Pro Ala Leu Glu Lys Leu Leu Pro His Ile Arg Gly Asn Val Gly
   1               5                   10                  15
<210> 4324
   <211> 8
   <212> PRT
   <213> Homo sapiens
<400> 4324
   Pro Gly Arg Gly Thr Pro Pro Pro
   1               5
<210> 4325
   <211> 8
   <212> PRT
   <213> Homo sapiens
<400> 4325
   Pro Pro Ala Arg Gly Thr Pro Ile
   1               5
<210> 4326
   <211> 8
   <212> PRT
   <213> Homo sapiens
<400> 4326
   Pro Pro Gly Arg Gly Thr Pro Pro
   1               5
<210> 4327
   <211> 8
   <212> PRT
   <213> Homo sapiens
<400> 4327
   Pro Pro Pro Gly Ile Met Ala Pro
   1               5
<210> 4328
   <211> 8
   <212> PRT
   <213> Homo sapiens
<400> 4328
   Pro Thr Gln Tyr Pro Pro Gly Arg
   1               5
<210> 4329
   <211> 15
   <212> PRT
   <213> Homo sapiens
<400> 4329
   Gln Gln Ile Arg Met Ser Leu Arg Gly Lys Ala Val Val Leu Met
   1 5 10 15
<210> 4330
   <211> 8
   <212> PRT
   <213> Homo sapiens
<400> 4330
   Gln Val Met Thr Pro Gln Gly Arg
   1 5
<210> 4331
   <211> 8
   <212> PRT
   <213> Homo sapiens
<400> 4331
   Arg Ala Thr Pro Pro Pro Gly Ile
   1 5
<210> 4332
   <211> 8
   <212> PRT
   <213> Homo sapiens
<400> 4332
   Arg Glu Tyr Arg Lys Lys Met Asp
   1 5
<210> 4333
   <211> 8
   <212> PRT
   <213> Homo sapiens
<400> 4333
   Arg Gly Thr Pro Pro Pro Pro Val
   1 5
<210> 4334
   <211> 8
   <212> PRT
   <213> Homo sapiens
<400> 4334
   Arg Lys Lys Met Asp Ile Pro Ala
   1 5
<210> 4335
   <211> 8
   <212> PRT
   <213> Homo sapiens
<400> 4335
   Arg Pro Pro Pro Pro Gly Ile Arg
   1               5
<210> 4336
   <211> 16
   <212> PRT
   <213> Homo sapiens
<400> 4336
   Arg Ser Gly Val Ser Leu Ala Ala Leu Lys Lys Ala Leu Ala Ala Ala
   1               5                   10                  15
<210> 4337
   <211> 6
   <212> PRT
   <213> Gallus gallus
<400> 4337
   Ser His Pro Thr Tyr Ser
   1               5
<210> 4338
   <211> 6
   <212> PRT
   <213> Gallus gallus
<400> 4338
   Ser His Tyr Lys Val Gly
   1               5
<210> 4339
   <211> 16
   <212> PRT
   <213> Gallus gallus
<400> 4339
   Ser Lys Pro Arg Ala Lys Ser Gly Ala Arg Lys Ser Pro Lys Lys Lys
   1               5                   10                  15
<210> 4340
   <211> 15
   <212> PRT
   <213> Homo sapiens
<400> 4340
   Ser Lys Gln Met Gln Gln Ile Arg Met Ser Leu Arg Gly Lys Ala
   1 5 10 15
<210> 4341
   <211> 15
   <212> PRT
   <213> Homo sapiens
<400> 4341
   Ser Pro Ser Ala Lys Asp Ile Lys Lys Ile Leu Asp Ser Val Gly
   1 5 10 15
<210> 4342
   <211> 6
   <212> PRT
   <213> Gallus gallus
<400> 4342
   Ser Ser Arg Gln Ser Ile
   1 5
<210> 4343
   <211> 31
   <212> PRT
   <213> Gallus gallus
<400> 4343
   Thr Glu Ser Leu Val Leu Ser Pro Ala Pro Ala Lys Pro Lys Arg Val
   1 5 10 15
   Lys Ala Ser Arg Arg Ser Ala Ser His Pro Thr Tyr Ser Glu Met
   20 25 30
<210> 4344
   <211> 8
   <212> PRT
   <213> Homo sapiens
<400> 4344
   Thr Phe Lys Ala Phe Asp Lys His
   1 5
<210> 4345
   <211> 8
   <212> PRT
   <213> Homo sapiens
<400> 4345
   Thr Pro Pro Pro Gly Ile Met Ala
   1 5
<210> 4346
   <211> 15
   <212> PRT
   <213> Homo sapiens
<400> 4346
   Thr Thr Ala Ala Pro Ala Ala Ala Ala Ala Pro Ala Lys Val Glu
   1 5 10 15
<210> 4347
   <211> 15
   <212> PRT
   <213> Homo sapiens
<400> 4347
   Thr Val Ala Ser Val Pro His Ser Ile Ile Asn Gly Tyr Lys Arg
   1 5 10 15
<210> 4348
   <211> 15
   <212> PRT
   <213> Homo sapiens
<400> 4348
   Val Lys Ala Phe Leu Ala Asp Pro Ser Ala Phe Val Ala Ala Ala
   1 5 10 15
<210> 4349
   <211> 15
   <212> PRT
   <213> Homo sapiens
<400> 4349
   Val Leu Met Gly Lys Asn Thr Met Met Arg Lys Ala Ile Arg Gly
   1 5 10 15
<210> 4350
   <211> 15
   <212> PRT
   <213> Homo sapiens
<400> 4350
   Val Asn Val Glu Pro Phe Trp Pro Gly Leu Phe Ala Lys Ala Leu
   1 5 10 15
<210> 4351
   <211> 8
   <212> PRT
   <213> Homo sapiens
<400> 4351
   Val Arg Gly Val Gly Gly Pro Ser
   1 5
<210> 4352
   <211> 15
   <212> PRT
   <213> Homo sapiens
<400> 4352
   Val Ser Ala Ala Pro Gly Ser Ala Ala Pro Ala Ala Gly Ser Ala
   1               5                   10                  15
<210> 4353
   <211> 16
   <212> PRT
   <213> Homo sapiens
<400> 4353
   Val Ser Glu Leu Ile Thr Lys Ala Val Ala Ala Ser Lys Glu Arg Ser
   1               5                   10                  15
<210> 4354
   <211> 15
   <212> PRT
   <213> Homo sapiens
<400> 4354
   Val Thr Val Thr Glu Asp Lys Ile Asn Ala Leu Ile Lys Ala Ala
   1               5                   10                  15
<210> 4355
   <211> 8
   <212> PRT
   <213> Homo sapiens
<400> 4355
   Tyr Arg Lys Lys Met Asp Ile Pro
   1               5
<210> 4356
   <211> 14
   <212> PRT
   <213> Homo sapiens
<400> 4356
   Phe Lys Glu Gln Phe Leu Asp Gly Asp Gly Trp Thr Ser Arg
   1               5                   10
<210> 4357
   <211> 345
   <212> PRT
   <213> Homo sapiens
<400> 4357
   Met Ile Ser Pro Val Leu Ile Leu Phe Ser Ser Phe Leu Cys His Val
   1               5                   10                  15
   Ala Ile Ala Gly Arg Thr Cys Pro Lys Pro Asp Asp Leu Pro Phe Ser
               20                  25                  30
   Thr Val Val Pro Leu Lys Thr Phe Tyr Glu Pro Gly Glu Glu Ile Thr
           35                  40                  45
   Tyr Ser Cys Lys Pro Gly Tyr Val Ser Arg Gly Gly Met Arg Lys Phe
       50                  55                  60
   Ile Cys Pro Leu Thr Gly Leu Trp Pro Ile Asn Thr Leu Lys Cys Thr
   65                  70                  75                  80
   Pro Arg Val Cys Pro Phe Ala Gly Ile Leu Glu Asn Gly Ala Val Arg
                   85                  90                  95
   Tyr Thr Thr Phe Glu Tyr Pro Asn Thr Ile Ser Phe Ser Cys Asn Thr
               100                 105                 110
   Gly Phe Tyr Leu Asn Gly Ala Asp Ser Ala Lys Cys Thr Glu Glu Gly
           115                 120                 125
   Lys Trp Ser Pro Glu Leu Pro Val Cys Ala Pro Ile Ile Cys Pro Pro
       130                 135                 140
   Pro Ser Ile Pro Thr Phe Ala Thr Leu Arg Val Tyr Lys Pro Ser Ala
   145                 150                 155                 160
   Gly Asn Asn Ser Leu Tyr Arg Asp Thr Ala Val Phe Glu Cys Leu Pro
                   165                 170                 175
   Gln His Ala Met Phe Gly Asn Asp Thr Ile Thr Cys Thr Thr His Gly
               180                 185                 190
   Asn Trp Thr Lys Leu Pro Glu Cys Arg Glu Val Lys Cys Pro Phe Pro
           195                 200                 205
   Ser Arg Pro Asp Asn Gly Phe Val Asn Tyr Pro Ala Lys Pro Thr Leu
       210                 215                 220
   Tyr Tyr Lys Asp Lys Ala Thr Phe Gly Cys His Asp Gly Tyr Ser Leu
   225                 230                 235                 240
   Asp Gly Pro Glu Glu Ile Glu Cys Thr Lys Leu Gly Asn Trp Ser Ala
                   245                 250                 255
   Met Pro Ser Cys Lys Ala Ser Cys Lys Leu Pro Val Lys Lys Ala Thr
               260                 265                 270
   Val Val Tyr Gln Gly Glu Arg Val Lys Ile Gln Glu Lys Phe Lys Asn
           275                 280                 285
   Gly Met Leu His Gly Asp Lys Val Ser Phe Phe Cys Lys Asn Lys Glu
       290                 295                 300
   Lys Lys Cys Ser Tyr Thr Glu Asp Ala Gln Cys Ile Asp Gly Thr Ile
   305                 310                 315                 320
   Glu Val Pro Lys Cys Phe Lys Glu His Ser Ser Leu Ala Phe Trp Lys
                   325                 330                 335
   Thr Asp Ala Ser Asp Val Lys Pro Cys
               340                 345
<210> 4358
   <211> 7
   <212> PRT
   <213> Homo sapiens
<400> 4358
   Ala Pro Ser Val Thr Leu Phe
   1               5
<210> 4359
   <211> 7
   <212> PRT
   <213> Homo sapiens
<400> 4359
   Ala Thr Leu Val Cys Leu Ile
   1 5
<210> 4360
   <211> 7
   <212> PRT
   <213> Homo sapiens
<400> 4360
   Ala Trp His Gln Gln Gln Pro
   1 5
<210> 4361
   <211> 7
   <212> PRT
   <213> Homo sapiens
<400> 4361
   Glu Arg Gly Leu Arg Tyr Leu
   1 5
<210> 4362
   <211> 7
   <212> PRT
   <213> Homo sapiens
<400> 4362
   Glu Arg Ile Leu Thr Ile Ser
   1 5
<210> 4363
   <211> 7
   <212> PRT
   <213> Homo sapiens
<400> 4363
   Gly Ala Ser Val Lys Val Thr
   1 5
<210> 4364
   <211> 7
   <212> PRT
   <213> Homo sapiens
<400> 4364
   Gly Ala Val Thr Val Ala Trp
   1 5
<210> 4365
   <211> 7
   <212> PRT
   <213> Homo sapiens
<400> 4365
   Gly Ser His Thr Arg Gly Asp
   1 5
<210> 4366
   <211> 20
   <212> PRT
   <213> Homo sapiens
<400> 4366
   Ile Arg Gly Asn Arg Ile Arg Tyr Phe Ile Leu Pro Asp Ser Leu Pro
   1 5 10 15
   Leu Asp Thr Ile
   20
<210> 4367
   <211> 7
   <212> PRT
   <213> Homo sapiens
<400> 4367
   Leu Met Lys Ile Asn Ser Asp
   1 5
<210> 4368
   <211> 19
   <212> PRT
   <213> Homo sapiens
<400> 4368
   Met Lys Leu Val Arg Phe Leu Met Lys Leu Ser His Glu Thr Val Thr
   1 5 10 15
   Ile Glu Leu
<210> 4369
   <211> 7
   <212> PRT
   <213> Homo sapiens
<400> 4369
   Pro Asp Arg Phe Ser Gly Ser
   1 5
<210> 4370
   <211> 20
   <212> PRT
   <213> Homo sapiens
<400> 4370
   Pro Leu Asp Thr Ile Arg Val Asp Val Glu Pro Lys Val Lys Ser Lys
   1               5                   10                  15
   Lys Arg Glu Ala
               20
<210> 4371
   <211> 7
   <212> PRT
   <213> Homo sapiens
<400> 4371
   Arg Gly Tyr Asp Ser Tyr Ala
   1               5
<210> 4372
   <211> 7
   <212> PRT
   <213> Homo sapiens
<400> 4372
   Ser Tyr Leu Ser Leu Thr Pro
   1               5
<210> 4373
   <211> 7
   <212> PRT
   <213> Homo sapiens
<400> 4373
   Ser Tyr Ser Cys Gln Val Thr
   1               5
<210> 4374
   <211> 20
   <212> PRT
   <213> Homo sapiens
<400> 4374
   Thr Gly Val Asp Val Ser Met Asn Thr His Leu Lys Ala Val Lys Met
   1               5                   10                  15
   Thr Leu Lys Asn
               20
<210> 4375
   <211> 20
   <212> PRT
   <213> Homo sapiens
<400> 4375
   Val Thr Ile Glu Leu Lys Asn Gly Thr Gln Val His Gly Thr Ile Thr
   1               5                   10                  15
   Gly Val Asp Val
               20
<210> 4376
   <211> 7
   <212> PRT
   <213> Homo sapiens
<400> 4376
   Trp Lys Ala Pro Ser Ser Pro
   1               5
<210> 4377
   <211> 7
   <212> PRT
   <213> Homo sapiens
<400> 4377
   Tyr Ala Ala Ser Ser Tyr Leu
   1               5
<210> 4378
   <211> 7
   <212> PRT
   <213> Homo sapiens
<400> 4378
   Tyr Cys Gln Thr Trp Gly Ser
   1               5
<210> 4379
   <211> 7
   <212> PRT
   <213> Homo sapiens
<400> 4379
   Tyr Asp Ser Tyr Ala Ile Ala
   1               5
<210> 4380
   <211> 8
   <212> PRT
   <213> Homo sapiens
<400> 4380
   Glu Val Phe Ile Ser Ala Pro Arg
   1               5
<210> 4381
   <211> 10
   <212> PRT
   <213> Mus musculus
<400> 4381
   Pro Ala Lys Ser Ala Pro Ala Pro Lys Lys
   1               5                   10
<210> 4382
   <211> 30
   <212> PRT
   <213> Mus musculus
<400> 4382
   Pro Glu Pro Ala Lys Ser Ala Pro Ala Pro Lys Lys Gly Ser Lys Lys
   1               5                   10                  15
   Ala Val Thr Lys Ala Gln Lys Lys Asp Gly Lys Lys Arg Lys
               20                  25                  30
<210> 4383
   <211> 21
   <212> PRT
   <213> Human T-lymphotropic virus
<400> 4383
   Pro Pro Pro Pro Ser Ser Pro Thr His Asp Pro Pro Asp Ser Asp Pro
   1               5                   10                  15
   Gln Ile Pro Pro Pro
               20
<210> 4384
   <211> 8
   <212> PRT
   <213> Homo sapiens
<400> 4384
   Pro Gln Ile Val Pro Glu Pro Met
   1               5
<210> 4385
   <211> 21
   <212> PRT
   <213> Homo sapiens
<400> 4385
   Pro Thr Arg Ala Pro Ser Gly Pro Arg Pro Pro Ser Arg Ser Gln Ala
   1 5 10 15
   Gln Thr Pro Pro Arg
   20
<210> 4386
   <211> 10
   <212> PRT
   <213> Homo sapiens
<400> 4386
   Asp Gly Pro Pro Gly Glu Leu Gly Pro Asp
   1 5 10
<210> 4387
   <211> 10
   <212> PRT
   <213> Homo sapiens
<400> 4387
   Asp Lys Glu Arg Glu Arg Arg Arg Ser Arg
   1 5 10
<210> 4388
   <211> 10
   <212> PRT
   <213> Homo sapiens
<400> 4388
   Glu Lys Ile Glu Arg Arg Gln Gln Glu Val
   1 5 10
<210> 4389
   <211> 10
   <212> PRT
   <213> Homo sapiens
<400> 4389
   Glu Asn Gly Tyr Leu Met Glu Ala Ala Pro
   1 5 10
<210> 4390
   <211> 10
   <212> PRT
   <213> Homo sapiens
<400> 4390
   Glu Arg Asp Lys Glu Arg Glu Arg Arg Arg
   1 5 10
<210> 4391
   <211> 10
   <212> PRT
   <213> Homo sapiens
<400> 4391
   Glu Arg Pro Gly Pro Ser Pro Leu Pro His
   1 5 10
<210> 4392
   <211> 10
   <212> PRT
   <213> Homo sapiens
<400> 4392
   Glu Arg Ser Arg Glu Arg Asp Lys Glu Arg
   1 5 10
<210> 4393
   <211> 10
   <212> PRT
   <213> Homo sapiens
<400> 4393
   Gly Glu Arg Gly Ser Glu Arg Gly Arg Asp
   1 5 10
<210> 4394
   <211> 10
   <212> PRT
   <213> Homo sapiens
<400> 4394
   Gly Gly Gly Leu Gly Gly Thr Arg Arg Gly
   1 5 10
<210> 4395
   <211> 10
   <212> PRT
   <213> Homo sapiens
<400> 4395
   Ile Glu Arg Arg Gln Gln Glu Val Glu Thr
   1 5 10
<210> 4396
   <211> 10
   <212> PRT
   <213> Homo sapiens
<400> 4396
   Lys Glu Glu Arg Arg Arg Ser Arg Glu Arg
   1 5 10
<210> 4397
   <211> 8
   <212> PRT
   <213> Homo sapiens
<400> 4397
   Lys Met Lys Gly Thr Phe Val Glu
   1 5
<210> 4398
   <211> 10
   <212> PRT
   <213> Homo sapiens
<400> 4398
   Lys Arg Gly Glu Arg Gly Ser Glu Arg Gly
   1 5 10
<210> 4399
   <211> 10
   <212> PRT
   <213> Homo sapiens
<400> 4399
   Met Glu Arg Lys Arg Arg Glu Lys Ile Glu
   1 5 10
<210> 4400
   <211> 10
   <212> PRT
   <213> Homo sapiens
<400> 4400
   Met Met Pro Val Gly Pro Ala Pro Gly Met
   1 5 10
<210> 4401
   <211> 10
   <212> PRT
   <213> Homo sapiens
<400> 4401
   Met Thr Gln Phe Leu Pro Pro Asn Leu Leu
   1 5 10
<210> 4402
   <211> 10
   <212> PRT
   <213> Homo sapiens
<400> 4402
   Pro Gly Met Met Pro Val Gly Pro Ala Pro
   1 5 10
<210> 4403
   <211> 10
   <212> PRT
   <213> Homo sapiens
<400> 4403
   Pro Gly Pro Ser Pro Leu Pro His Arg Asp
   1 5 10
<210> 4404
   <211> 10
   <212> PRT
   <213> Homo sapiens
<400> 4404
   Pro Pro Gly Glu Leu Gly Pro Asp Gly Pro
   1 5 10
<210> 4405
   <211> 10
   <212> PRT
   <213> Homo sapiens
<400> 4405
   Pro Pro Pro Gly Met Met Pro Val Gly Pro
   1 5 10
<210> 4406
   <211> 10
   <212> PRT
   <213> Homo sapiens
<400> 4406
   Pro Arg Arg Leu Gly Gly Gly Leu Gly Gly
   1 5 10
<210> 4407
   <211> 10
   <212> PRT
   <213> Homo sapiens
<400> 4407
   Pro Ser Pro Leu Pro His Arg Asp Arg Asp
   1 5 10
<210> 4408
   <211> 10
   <212> PRT
   <213> Homo sapiens
<400> 4408
   Arg Glu Glu Arg Met Glu Arg Lys Arg Arg
   1 5 10
<210> 4409
   <211> 10
   <212> PRT
   <213> Homo sapiens
<400> 4409
   Arg Glu Arg Glu Arg Arg Glu Arg Ser Arg
   1 5 10
<210> 4410
   <211> 10
   <212> PRT
   <213> Homo sapiens
<400> 4410
   Arg Glu Arg Arg Glu Arg Ser Arg Glu Arg
   1 5 10
<210> 4411
   <211> 10
   <212> PRT
   <213> Homo sapiens
<400> 4411
   Arg Lys Arg Arg Glu Lys Ile Glu Arg Arg
   1 5 10
<210> 4412
   <211> 10
   <212> PRT
   <213> Homo sapiens
<400> 4412
   Arg Leu Gly Gly Gly Leu Gly Gly Thr Arg
   1 5 10
<210> 4413
   <211> 10
   <212> PRT
   <213> Homo sapiens
<400> 4413
   Arg Arg Glu Lys Ile Glu Arg Arg Gln Gln
   1 5 10
<210> 4414
   <211> 10
   <212> PRT
   <213> Homo sapiens
<400> 4414
   Arg Arg Glu Arg Ser Arg Glu Arg Asp Lys
   1 5 10
<210> 4415
   <211> 10
   <212> PRT
   <213> Homo sapiens
<400> 4415
   Ser Arg Glu Arg Asp Lys Glu Arg Glu Arg
   1 5 10
<210> 4416
   <211> 10
   <212> PRT
   <213> Homo sapiens
<400> 4416
   Ser Arg Tyr Asp Glu Arg Pro Gly Pro Ser
   1 5 10
<210> 4417
   <211> 10
   <212> PRT
   <213> Homo sapiens
<400> 4417
   Tyr Asp Glu Arg Pro Gly Pro Ser Pro Leu
   1 5 10
<210> 4418
   <211> 21
   <212> PRT
   <213> Mus musculus
<400> 4418
   Ser Thr Asp His Pro Lys Tyr Ser Asp Met Ile Val Ala Ala Ile Gln
   1 5 10 15
   Ala Glu Lys Asn Arg
   20
<210> 4419
   <211> 13
   <212> PRT
   <213> Homo sapiens
<400> 4419
   Ala Glu Val Glu Thr Asp Lys Ala Thr Val Gly Phe Glu
   1 5 10
<210> 4420
   <211> 21
   <212> PRT
   <213> Homo sapiens
<400> 4420
   Gly Pro Pro Pro Pro Gly Met Met Pro Val Gly Pro Ala Pro Gly Met
   1 5 10 15
   Arg Pro Pro Met Gly
   20
<210> 4421
   <211> 18
   <212> PRT
   <213> Homo sapiens
<400> 4421
   Val Gly Pro Ala Pro Gly Met Arg Pro Pro Met Gly Gly His Met Pro
   1 5 10 15
   Met Met
<210> 4422
   <211> 14
   <212> PRT
   <213> Gallus gallus
<400> 4422
   Asp Trp Met Glu Glu Glu Glu Gly Ala Gln Arg Glu Lys Glu
   1 5 10
<210> 4423
   <211> 8
   <212> PRT
   <213> Homo sapiens
<400> 4423
   Gly Thr Leu His Val Val Gln Arg
   1 5
<210> 4424
   <211> 8
   <212> PRT
   <213> Homo sapiens
<400> 4424
   Ile Ile Trp Arg Ser Thr Gln Gly
   1 5
<210> 4425
   <211> 8
   <212> PRT
   <213> Homo sapiens
<400> 4425
   Ile Trp Arg Ser Thr Gln Gly Val
   1 5
<210> 4426
   <211> 8
   <212> PRT
   <213> Homo sapiens
<400> 4426
   Leu Gly Thr Leu His Val Val Gln
   1 5
<210> 4427
   <211> 8
   <212> PRT
   <213> Homo sapiens
<400> 4427
   Arg Ile Ile Trp Arg Ser Thr Gln
   1 5
<210> 4428
   <211> 8
   <212> PRT
   <213> Homo sapiens
<400> 4428
   Arg Val Arg Val Ser Trp Ser Pro
   1 5
<210> 4429
   <211> 8
   <212> PRT
   <213> Homo sapiens
<400> 4429
   Arg Val Ser Trp Ser Pro Val Pro
   1 5
<210> 4430
   <211> 21
   <212> PRT
   <213> Gallus gallus
<400> 4430
   Ser Ala Ser His Pro Thr Tyr Ser Glu Met Ile Ala Ala Ala Ile Arg
   1               5                   10                  15
   Ala Glu Lys Ser Arg
               20
<210> 4431
   <211> 14
   <212> PRT
   <213> Gallus gallus
<400> 4431
   Ser Gln Lys Glu Glu Glu Glu Gly Ala Gln Arg Glu Lys Glu
   1               5                   10
<210> 4432
   <211> 8
   <212> PRT
   <213> Homo sapiens
<400> 4432
   Thr Leu His Val Val Gln Arg Gly
   1               5
<210> 4433
   <211> 8
   <212> PRT
   <213> Homo sapiens
<400> 4433
   Val Pro Arg Ala Gln Gly Phe Leu
   1               5
<210> 4434
   <211> 8
   <212> PRT
   <213> Homo sapiens
<400> 4434
   Val Arg Val Ser Trp Ser Pro Val
   1               5
<210> 4435
   <211> 21
   <212> PRT
   <213> Homo sapiens
<400> 4435
   Glu Cys Leu Pro Gln His Ala Met Phe Gly Asn Asp Thr Ile Thr Cys
   1 5 10 15
   Thr Thr His Gly Asn
   20
<210> 4436
   <211> 15
   <212> PRT
   <213> Homo sapiens
<400> 4436
   Lys Leu Pro Val Lys Lys Ala Thr Val Val Tyr Gln Gly Glu Arg
   1 5 10 15
<210> 4437
   <211> 16
   <212> PRT
   <213> Homo sapiens
<400> 4437
   Leu Pro Val Lys Lys Ala Thr Val Val Tyr Gln Gly Glu Arg Val Lys
   1 5 10 15
<210> 4438
   <211> 14
   <212> PRT
   <213> Homo sapiens
<400> 4438
   Pro Val Lys Lys Ala Thr Val Val Tyr Gln Gly Glu Arg Val
   1 5 10
<210> 4439
   <211> 17
   <212> PRT
   <213> Unknown
<220>
   <223> epitope involved in systemic lupus
<400> 4439
   Ala Pro Val Cys Val Trp Glu Arg Leu Ser Pro Tyr Arg Glu Arg Cys
   1 5 10 15
   Val
<210> 4440
   <211> 17
   <212> PRT
   <213> Unknown
<220>
   <223> epitope involved in systemic lupus
<400> 4440
   Ala Pro Val Cys Val Trp Glu Arg Leu Ser Pro Tyr Arg Glu Arg Cys
   1 5 10 15
   Val
<210> 4441
   <211> 21
   <212> PRT
   <213> Unknown
<220>
   <223> epitope involved in systemic lupus
<400> 4441
   Ala Pro Val Cys Val Trp Glu Arg Leu Ser Pro Tyr Arg Glu Arg Cys
   1 5 10 15
   Val Pro Ala Glu Gly
   20
<210> 4442
   <211> 18
   <212> PRT
   <213> Unknown
<220>
   <223> epitope involved in systemic lupus
<400> 4442
   Cys His Trp Asp Pro Phe Ser Leu Ser Ala Tyr Phe Pro Gly Gly Gly
   1 5 10 15
   Ser Cys
<210> 4443
   <211> 12
   <212> PRT
   <213> Unknown
<220>
   <223> epitope involved in systemic lupus
<400> 4443
   His Trp Asp Pro Phe Ser Leu Ser Ala Tyr Phe Pro
   1 5 10
<210> 4444
   <211> 12
   <212> PRT
   <213> Unknown
<220>
   <223> epitope involved in systemic lupus
<400> 4444
   His Trp Asp Pro Phe Ser Leu Ser Ala Tyr Phe Pro
   1               5                   10
<210> 4445
   <211> 16
   <212> PRT
   <213> Unknown
<220>
   <223> epitope involved in systemic lupus
<400> 4445
   His Trp Asp Pro Phe Ser Leu Ser Ala Tyr Phe Pro Gly Gly Gly Ser
   1               5                   10                  15
<210> 4446
   <211> 22
   <212> PRT
   <213> Mus musculus
<400> 4446
   Ser Gly Arg Gly Lys Gly Gly Lys Gly Leu Gly Lys Gly Gly Ala Lys
   1               5                   10                  15
   Arg His Arg Lys Val Leu
               20
<210> 4447
   <211> 22
   <212> PRT
   <213> Mus musculus
<400> 4447
   Ser Gly Arg Gly Lys Gly Gly Lys Gly Leu Gly Lys Gly Gly Ala Lys
   1               5                   10                  15
   Arg His Arg Lys Val Leu
                                       20
<210> 4448
   <211> 5
   <212> PRT
   <213> Unknown
<220>
   <223> epitope involved in systemic lupus
<400> 4448
   Asp Trp Glu Tyr Ser
   1               5
<210> 4449
   <211> 10
   <212> PRT
   <213> Unknown
<220>
   <223> epitope involved in systemic lupus
<400> 4449
   Asp Trp Glu Tyr Ser Val Trp Ser Leu Asn
   1               5                   10
<210> 4450
   <211> 18
   <212> PRT
   <213> Mus musculus
<400> 4450
   Leu Pro Asp Pro Tyr Gln Lys Gln Cys Asp Asp Phe Val Ala Glu Tyr
   1               5                   10                  15
   Glu Pro
<210> 4451
   <211> 21
   <212> PRT
   <213> Mus musculus
<400> 4451
   Lys Glu Gly Tyr Lys Val Arg Leu Asp Leu Asn Ile Thr Leu Glu Phe
   1               5                   10                  15
   Arg Thr Thr Ser Lys
               20
<210> 4452
   <211> 15
   <212> PRT
   <213> Homo sapiens
<400> 4452
   Pro Pro Gly Met Arg Pro Pro Met Gly Pro Met Gly Ile Pro Pro
   1 5 10 15
<210> 4453
   <211> 9
   <212> PRT
   <213> Burkholderia fungorum
<400> 4453
   Arg Ala Gly Thr Asp Glu Gly Phe Gly
   1 5
<210> 4454
   <211> 17
   <212> PRT
   <213> Mus musculus
<400> 4454
   Asp Gly Lys Trp His Thr Val Lys Thr Glu Tyr Ile Lys Arg Lys Ala
   1 5 10 15
   Phe
<210> 4455
   <211> 12
   <212> PRT
   <213> Homo sapiens
<400> 4455
   Gly Ala Arg Gly Leu Thr Gly Arg Pro Gly Asp Ala
   1 5 10
<210> 4456
   <211> 12
   <212> PRT
   <213> Homo sapiens
<400> 4456
   Gly Ala Arg Gly Leu Thr Gly Arg Pro Gly Asp Ala
   1 5 10
<210> 4457
   <211> 19
   <212> PRT
   <213> Mus musculus
<400> 4457
   Gly Arg Leu His Phe Met Phe Asp Leu Gly Lys Gly Arg Thr Lys Val
   1 5 10 15
   Ser His Pro
<210> 4458
   <211> 17
   <212> PRT
   <213> Mus musculus
<400> 4458
   Lys Ala Thr Pro Met Leu Lys Met Arg Thr Ser Phe His Gly Cys Ile
   1               5                   10                  15
   Lys
<210> 4459
   <211> 21
   <212> PRT
   <213> Mus musculus
<400> 4459
   Lys Asn Leu Glu Ile Ser Arg Ser Thr Phe Asp Leu Leu Arg Asn Ser
   1               5                   10                  15
   Tyr Gly Val Arg Lys
               20
<210> 4460
   <211> 22
   <212> PRT
   <213> Mus musculus
<400> 4460
   Lys Gln Asn Cys Leu Ser Ser Arg Ala Ser Phe Arg Gly Cys Val Arg
   1               5                   10                  15
   Asn Leu Arg Leu Ser Arg
               20
<210> 4461
   <211> 22
   <212> PRT
   <213> Mus musculus
<400> 4461
   Arg Pro Val Arg His Ala Gln Cys Arg Val Cys Asp Gly Asn Ser Thr
   1               5                   10                  15
   Asn Pro Arg Glu Arg His
               20
<210> 4462
   <211> 19
   <212> PRT
   <213> Mus musculus
<400> 4462
   Thr Ser Leu Arg Lys Ala Leu Leu His Ala Pro Thr Gly Ser Tyr Ser
   1 5 10 15
   Asp Gly Gln
<210> 4463
   <211> 15
   <212> PRT
   <213> Unknown
<220>
   <223> epitope involved in systemic lupus
<400> 4463
   Asp His Arg Ser Pro Pro Trp Leu Thr Ser Leu Leu Thr Ile Ser
   1 5 10 15
<210> 4464
   <211> 15
   <212> PRT
   <213> Unknown
<220>
   <223> epitope involved in systemic lupus
<400> 4464
   Asp Thr Trp Pro Thr Ala Arg Leu Thr Ser Ser Met Gln Tyr Ile
   1 5 10 15
<210> 4465
   <211> 15
   <212> PRT
   <213> Unknown
<220>
   <223> epitope involved in systemic lupus
<400> 4465
   Gly His Arg Tyr Thr Ser Ser Val Ser Leu Thr Glu Ala Cys Pro
   1 5 10 15
<210> 4466
   <211> 15
   <212> PRT
   <213> Unknown
<220>
   <223> epitope involved in systemic lupus
<400> 4466
   His Thr Tyr Thr Ser His Leu Arg Tyr Val Pro Pro Ile Ser Leu
   1 5 10 15
<210> 4467
   <211> 15
   <212> PRT
   <213> Unknown
<220>
   <223> epitope involved in systemic lupus
<400> 4467
   Lys Leu Thr Ser Ser Leu Arg Cys Asn Cys Pro Pro Leu Arg Phe
   1 5 10 15
<210> 4468
   <211> 15
   <212> PRT
   <213> Unknown
<220>
   <223> epitope involved in systemic lupus
<400> 4468
   Lys Leu Thr Ser Ser Leu Arg Tyr Asn Ser Pro Pro Leu Cys Phe
   1 5 10 15
<210> 4469
   <211> 15
   <212> PRT
   <213> Unknown
<220>
   <223> epitope involved in systemic lupus
<400> 4469
   Lys Ser Thr Ser Ser Leu Arg Asp Asn Ser Pro Pro Val Cys Phe
   1 5 10 15
<210> 4470
   <211> 15
   <212> PRT
   <213> Unknown
<220>
   <223> epitope involved in systemic lupus
<400> 4470
   Gln Ala Pro Arg Leu Met Ser Ser Leu Ser Tyr Phe Pro Gln Ser
   1 5 10 15
<210> 4471
   <211> 15
   <212> PRT
   <213> Unknown
<220>
   <223> epitope involved in systemic lupus
<400> 4471
   Arg Asp Gly Gln Arg Leu Thr Ser Ser Lys Thr Met Leu Pro Tyr
   1 5 10 15
<210> 4472
   <211> 10
   <212> PRT
   <213> Unknown
<220>
   <223> epitope involved in systemic lupus
<400> 4472
   Arg Leu Thr Ser Ser Leu Arg Tyr Asn Pro
   1 5 10
<210> 4473
   <211> 15
   <212> PRT
   <213> Unknown
<220>
   <223> epitope involved in systemic lupus
<400> 4473
   Arg Arg Thr Pro Asp Pro Ala Val Ser Pro Trp Gln Leu Thr Tyr
   1 5 10 15
<210> 4474
   <211> 15
   <212> PRT
   <213> Unknown
<220>
   <223> epitope involved in systemic lupus
<400> 4474
   Thr His Asp Leu Ser Ser Arg Ala Ser Ser Ser Leu Ser Tyr Asn
   1 5 10 15
<210> 4475
   <211> 7
   <212> PRT
   <213> Homo sapiens
<400> 4475
   Ala Thr Val Gln Leu Pro Gln
   1 5
<210> 4476
   <211> 7
   <212> PRT
   <213> Homo sapiens
<400> 4476
   Gln Pro His Ser Arg Pro Tyr
   1 5
<210> 4477
   <211> 15
   <212> PRT
   <213> Homo sapiens
<400> 4477
   Ala Ser Gly Phe Thr Phe Ser Asn Tyr Ala Met His Trp Val Arg
   1 5 10 15
<210> 4478
   <211> 15
   <212> PRT
   <213> Homo sapiens
<400> 4478
   Gly Leu Glu Trp Val Ala Val Ile Ser Tyr Asp Gly Gly Asn Lys
   1 5 10 15
<210> 4479
   <211> 15
   <212> PRT
   <213> Homo sapiens
<400> 4479
   Lys Tyr Tyr Ala Asp Ser Val Lys Gly Arg Phe Thr Ile Ser Arg
   1 5 10 15
<210> 4480
   <211> 14
   <212> PRT
   <213> Homo sapiens
<400> 4480
   Met His Trp Val Arg Gln Ala Pro Gly Lys Gly Leu Glu Trp
   1 5 10
<210> 4481
   <211> 14
   <212> PRT
   <213> Homo sapiens
<400> 4481
   Met Tyr Trp Val Arg Gln Ala Pro Gly Lys Gly Leu Glu Trp
   1 5 10
<210> 4482
   <211> 15
   <212> PRT
   <213> Homo sapiens
<400> 4482
   Gln Ala Pro Gly Lys Gly Leu Glu Trp Val Ala Val Ile Ser Tyr
   1 5 10 15
<210> 4483
   <211> 15
   <212> PRT
   <213> Homo sapiens
<400> 4483
   Val Ala Val Ile Ser Tyr Asp Gly Gly Asn Lys Tyr Tyr Ala Asp
   1 5 10 15
<210> 4484
   <211> 13
   <212> PRT
   <213> Homo sapiens
<400> 4484
   Ala Leu Arg Glu Tyr Arg Lys Lys Met Asp Ile Pro Ala
   1 5 10
<210> 4485
   <211> 29
   <212> PRT
   <213> Homo sapiens
<400> 4485
   Gly Gly Pro Pro Met Met Pro Met Met Gly Pro Pro Pro Pro Gly Met
   1 5 10 15
   Met Pro Val Gly Pro Ala Pro Gly Met Arg Pro Pro Met
   20 25
<210> 4486
   <211> 31
   <212> PRT
   <213> Homo sapiens
<400> 4486
   His Lys Glu Asn Val Lys Asp Tyr Tyr Gln Lys Trp Met Glu Glu Gln
   1               5                   10                  15
   Ala Gln Ser Leu Ile Asp Lys Thr Thr Ala Ala Phe Gln Gln Gly
               20                  25                  30
<210> 4487
   <211> 8
   <212> PRT
   <213> Homo sapiens
<400> 4487
   Ala Pro Pro Asp Asp Gly Pro Pro
   1               5
<210> 4488
   <211> 8
   <212> PRT
   <213> Homo sapiens
<400> 4488
   Cys Glu Thr Asn Thr Gly Glu Val
   1               5
<210> 4489
   <211> 8
   <212> PRT
   <213> Homo sapiens
<400> 4489
   Glu Glu Glu Glu Asp Gly Glu Met
   1               5
<210> 4490
   <211> 8
   <212> PRT
   <213> Homo sapiens
<400> 4490
   Glu Glu Leu Gln Lys Arg Glu Glu
   1               5
<210> 4491
   <211> 8
   <212> PRT
   <213> Homo sapiens
<400> 4491
   Glu Ile His Ser Lys Thr Lys Ser
   1               5
<210> 4492
   <211> 8
   <212> PRT
   <213> Homo sapiens
<400> 4492
   Asn Thr Glu Glu Tyr Ile Asp Gly
   1               5
<210> 4493
   <211> 8
   <212> PRT
   <213> Homo sapiens
<400> 4493
   Pro Asp Gly Pro Asp Gly Pro Glu
   1               5
<210> 4494
   <211> 8
   <212> PRT
   <213> Homo sapiens
<400> 4494
   Trp Ser Lys Ala His Pro Pro Glu
   1               5
<210> 4495
   <211> 24
   <212> PRT
   <213> Homo sapiens
<400> 4495
   Phe Cys Pro Asp Val Glu Phe Lys Arg Arg Phe Lys Glu Ala Phe Ser
   1               5                   10                  15
   Lys Ala Ala Gln Gln Thr Lys Gly
               20
<210> 4496
   <211> 26
   <212> PRT
   <213> Homo sapiens
<400> 4496
   Phe Gly His Ile Ser Gly Gly His Ile Asn Pro Ala Val Thr Val Ala
   1               5                   10                  15
   Met Val Cys Thr Arg Lys Ile Ser Ile Ala
               20                  25
<210> 4497
   <211> 22
   <212> PRT
   <213> Homo sapiens
<400> 4497
   Met Ser Asp Arg Pro Thr Ala Arg Arg Trp Gly Lys Cys Gly Pro Leu
   1               5                   10                  15
   Cys Thr Arg Glu Asn Ile
               20
<210> 4498
   <211> 6
   <212> PRT
   <213> Homo sapiens
<400> 4498
   Pro Pro Pro Gly Ile Arg
   1               5
<210> 4499
   <211> 6
   <212> PRT
   <213> Homo sapiens
<400> 4499
   Pro Pro Pro Gly Met Arg
   1               5
<210> 4500
   <211> 4
   <212> PRT
   <213> Homo sapiens
<400> 4500
   Pro Gln Gly Arg
   1
<210> 4501
   <211> 18
   <212> PRT
   <213> Homo sapiens
<400> 4501
   Pro Glu Ser Phe Asp Gly Asp Pro Ala Ser Asn Thr Ala Pro Leu Gln
   1               5                   10                  15
   Pro Glu
<210> 4502
   <211> 17
   <212> PRT
   <213> Homo sapiens
<400> 4502
   Lys Ile His Ala Arg Glu Ile Phe Asp Ser Arg Gly Asn Pro Thr Val
   1               5                   10                  15
   Glu
<210> 4503
   <211> 8
   <212> PRT
   <213> Homo sapiens
<400> 4503
   Pro Asp Asp Leu Pro Phe Ser Thr
   1               5
<210> 4504
   <211> 13
   <212> PRT
   <213> Homo sapiens
<400> 4504
   Gly Arg Pro Gly Arg Arg Gly Arg Pro Gly Leu Lys Gly
   1               5                   10
<210> 4505
   <211> 13
   <212> PRT
   <213> Homo sapiens
<400> 4505
   Pro Gly Lys Val Gly Pro Lys Gly Pro Met Gly Pro Lys
   1               5                   10
<210> 4506
   <211> 13
   <212> PRT
   <213> Homo sapiens
<400> 4506
   Tyr Asn Gln Tyr Ser Gln Arg Tyr His Gln Arg Thr Asn
   1               5                   10
<210> 4507
   <211> 15
   <212> PRT
   <213> Homo sapiens
<400> 4507
   Ala Asp Ser Ala Ser Ser Ser Glu Ser Glu Arg Asn Ile Thr Ile
   1 5 10 15
<210> 4508
   <211> 15
   <212> PRT
   <213> Homo sapiens
<400> 4508
   Ala Lys Ala Thr Lys Asp Val Glu Arg Asn Leu Ser Arg Asp Leu
   1 5 10 15
<210> 4509
   <211> 15
   <212> PRT
   <213> Homo sapiens
<400> 4509
   Asp Asp Lys Lys Lys Lys Glu Ala Ala Gln Lys Lys Ala Thr Glu
   1 5 10 15
<210> 4510
   <211> 15
   <212> PRT
   <213> Homo sapiens
<400> 4510
   Asp Gly Leu Arg Asn Ser Thr Gly Leu Gly Ser Gln Asn Lys Phe
   1 5 10 15
<210> 4511
   <211> 15
   <212> PRT
   <213> Homo sapiens
<400> 4511
   Asp Val Glu Arg Asn Leu Ser Arg Asp Leu Val Gln Glu Glu Glu
   1 5 10 15
<210> 4512
   <211> 15
   <212> PRT
   <213> Homo sapiens
<400> 4512
   His Tyr Glu Asn Ser Gln Arg Gly Pro Val Ser Ser Thr Ser Asp
   1 5 10 15
<210> 4513
   <211> 15
   <212> PRT
   <213> Homo sapiens
<400> 4513
   Lys Glu Ala Ala Gln Lys Lys Ala Thr Glu Gln Lys Ile Lys Val
   1               5                   10                  15
<210> 4514
   <211> 15
   <212> PRT
   <213> Homo sapiens
<400> 4514
   Lys Lys Ala Thr Glu Gln Lys Ile Lys Val Pro Glu Gln Ile Lys
   1               5                   10                  15
<210> 4515
   <211> 15
   <212> PRT
   <213> Homo sapiens
<400> 4515
   Lys Lys Lys Lys Lys Asp Asp Lys Lys Lys Lys Glu Ala Ala Gln
   1               5                   10                  15
<210> 4516
   <211> 15
   <212> PRT
   <213> Homo sapiens
<400> 4516
   Lys Asn Ala Val Val Ser Asp Leu Ser Glu Lys Glu Ala Trp Pro
   1               5                   10                  15
<210> 4517
   <211> 15
   <212> PRT
   <213> Homo sapiens
<400> 4517
   Lys Gln Leu Leu Lys Arg Gly Gln His Phe Pro Val Ile Ala Ala
   1               5                   10                  15
<210> 4518
   <211> 15
   <212> PRT
   <213> Homo sapiens
<400> 4518
   Lys Arg Ala Thr Ala Asn Asn Gln Gln Pro Gln Gln Gln Gln Gln
   1               5                   10                  15
<210> 4519
   <211> 15
   <212> PRT
   <213> Homo sapiens
<400> 4519
   Leu Ser Arg Asp Leu Val Gln Glu Glu Glu Gln Leu Met Glu Glu
   1 5 10 15
<210> 4520
   <211> 15
   <212> PRT
   <213> Homo sapiens
<400> 4520
   Met Arg Glu Leu Glu Ala Lys Ala Thr Lys Asp Val Glu Arg Asn
   1 5 10 15
<210> 4521
   <211> 15
   <212> PRT
   <213> Homo sapiens
<400> 4521
   Asn Leu Gly Ser Ala Val Lys Val Leu Asn Ser Gln Ser Glu Ser
   1 5 10 15
<210> 4522
   <211> 15
   <212> PRT
   <213> Homo sapiens
<400> 4522
   Asn Asn Gln Gln Pro Gln Gln Gln Gln Gln Gln Gln Gln Pro Gln
   1 5 10 15
<210> 4523
   <211> 15
   <212> PRT
   <213> Homo sapiens
<400> 4523
   Asn Ser Lys Asn Gln Ser Asp Ile Asn His Ser Thr Ser Gly Ser
   1 5 10 15
<210> 4524
   <211> 15
   <212> PRT
   <213> Homo sapiens
<400> 4524
   Pro Gln Ala Leu Pro Arg Tyr Pro Arg Glu Val Pro Pro Arg Phe
   1 5 10 15
<210> 4525
   <211> 15
   <212> PRT
   <213> Homo sapiens
<400> 4525
   Pro Val Ile Ala Ala Asn Leu Gly Ser Ala Val Lys Val Leu Asn
   1 5 10 15
<210> 4526
   <211> 15
   <212> PRT
   <213> Homo sapiens
<400> 4526
   Gln Lys Ile Lys Val Pro Glu Gln Ile Lys Pro Ser Val Ser Gln
   1 5 10 15
<210> 4527
   <211> 15
   <212> PRT
   <213> Homo sapiens
<400> 4527
   Gln Leu Met Glu Glu Lys Lys Lys Lys Lys Asp Asp Lys Lys Lys
   1 5 10 15
<210> 4528
   <211> 15
   <212> PRT
   <213> Homo sapiens
<400> 4528
   Gln Pro Gln Gln Gln Pro Gln Ala Leu Pro Arg Tyr Pro Arg Glu
   1 5 10 15
<210> 4529
   <211> 15
   <212> PRT
   <213> Homo sapiens
<400> 4529
   Gln Pro Gln Gln Gln Gln Pro Gln Gln Gln Pro Gln Ala Leu Pro
   1 5 10 15
<210> 4530
   <211> 15
   <212> PRT
   <213> Homo sapiens
<400> 4530
   Gln Gln Pro Gln Pro Gln Pro Gln Gln Gln Gln Pro Gln Gln Gln
   1 5 10 15
<210> 4531
   <211> 15
   <212> PRT
   <213> Homo sapiens
<400> 4531
   Gln Gln Gln Pro Gln Gln Gln Pro Gln Pro Gln Pro Gln Gln Gln
   1 5 10 15
<210> 4532
   <211> 15
   <212> PRT
   <213> Homo sapiens
<400> 4532
   Gln Gln Gln Pro Gln Gln Gln Gln Pro Gln Gln Gln Pro Gln Pro
   1 5 10 15
<210> 4533
   <211> 15
   <212> PRT
   <213> Homo sapiens
<400> 4533
   Gln Gln Gln Gln Gln Gln Gln Gln Pro Gln Gln Gln Gln Pro Gln
   1 5 10 15
<210> 4534
   <211> 15
   <212> PRT
   <213> Homo sapiens
<400> 4534
   Gln Arg Gly Pro Val Ser Ser Thr Ser Asp Ser Ser Thr Asn Cys
   1 5 10 15
<210> 4535
   <211> 15
   <212> PRT
   <213> Homo sapiens
<400> 4535
   Arg His Gln Glu His Lys Gln Leu Leu Lys Arg Gly Gln His Phe
   1 5 10 15
<210> 4536
   <211> 15
   <212> PRT
   <213> Homo sapiens
<400> 4536
   Arg Asn Ile Thr Ile Met Ala Ser Gly Asn Thr Gly Gly Glu Lys
   1 5 10 15
<210> 4537
   <211> 15
   <212> PRT
   <213> Homo sapiens
<400> 4537
   Arg Tyr Pro Arg Glu Val Pro Pro Arg Phe Arg His Gln Glu His
   1 5 10 15
<210> 4538
   <211> 15
   <212> PRT
   <213> Homo sapiens
<400> 4538
   Ser Ala Leu Thr Asn Gln Gln Pro Gln Asn Asn Gly Glu Val Gln
   1 5 10 15
<210> 4539
   <211> 15
   <212> PRT
   <213> Homo sapiens
<400> 4539
   Ser Asp Ile Asn His Ser Thr Ser Gly Ser His Tyr Glu Asn Ser
   1 5 10 15
<210> 4540
   <211> 15
   <212> PRT
   <213> Homo sapiens
<400> 4540
   Ser Asn Asn Gly Thr Ser Thr Ala Thr Ser Thr Asn Asn Asn Ala
   1 5 10 15
<210> 4541
   <211> 15
   <212> PRT
   <213> Homo sapiens
<400> 4541
   Ser Ser Thr Ser Asp Ser Ser Thr Asn Cys Lys Asn Ala Val Val
   1 5 10 15
<210> 4542
   <211> 15
   <212> PRT
   <213> Homo sapiens
<400> 4542
   Ser Thr Ala Thr Ser Thr Asn Asn Asn Ala Lys Arg Ala Thr Ala
   1 5 10 15
<210> 4543
   <211> 15
   <212> PRT
   <213> Homo sapiens
<400> 4543
   Ser Thr Gly Leu Gly Ser Gln Asn Lys Phe Val Val Gly Ser Ser
   1 5 10 15
<210> 4544
   <211> 15
   <212> PRT
   <213> Homo sapiens
<400> 4544
   Thr Asn Asn Asn Ala Lys Arg Ala Thr Ala Asn Asn Gln Gln Pro
   1 5 10 15
<210> 4545
   <211> 15
   <212> PRT
   <213> Homo sapiens
<400> 4545
   Val Lys Val Leu Asn Ser Gln Ser Glu Ser Ser Ala Leu Thr Asn
   1 5 10 15
<210> 4546
   <211> 15
   <212> PRT
   <213> Homo sapiens
<400> 4546
   Val Pro Pro Arg Phe Arg His Gln Glu His Lys Gln Leu Leu Lys
   1 5 10 15
<210> 4547
   <211> 15
   <212> PRT
   <213> Homo sapiens
<400> 4547
   Val Gln Glu Glu Glu Gln Leu Met Glu Glu Lys Lys Lys Lys Lys
   1 5 10 15
<210> 4548
   <211> 15
   <212> PRT
   <213> Homo sapiens
<400> 4548
   Lys Leu Thr Ser Glu Ala Arg Gly Arg Ile Pro Val Ala Gln Lys
   1               5                   10                  15
<210> 4549
   <211> 19
   <212> PRT
   <213> Homo sapiens
<400> 4549
   Ala Leu Met Arg Gly Lys Ser Thr Glu Glu Ala Arg Lys Glu Leu Gln
   1               5                   10                  15
   Ala Ala Gly
<210> 4550
   <211> 19
   <212> PRT
   <213> Homo sapiens
<400> 4550
   Ala Ser Thr Asn Gly Leu Ile Asn Phe Ile Lys Gln Gln Arg Glu Ala
   1               5                   10                  15
   Arg Val Gln
<210> 4551
   <211> 19
   <212> PRT
   <213> Homo sapiens
<400> 4551
   Glu Asp Leu Glu Arg Leu Leu Pro His Lys Val Phe Glu Gly Asn Arg
   1               5                   10                  15
   Pro Thr Asn
<210> 4552
   <211> 19
   <212> PRT
   <213> Homo sapiens
<400> 4552
   Phe Glu Phe Trp Asp Trp Val Gly Gly Arg Tyr Ser Leu Trp Ser Ala
   1 5 10 15
   Ile Gly Leu
<210> 4553
   <211> 19
   <212> PRT
   <213> Homo sapiens
<400> 4553
   Asn Tyr Thr Glu Gly Arg Ala Val Leu His Val Ala Leu Arg Asn Arg
   1 5 10 15
   Ser Asn Thr
<210> 4554
   <211> 19
   <212> PRT
   <213> Homo sapiens
<400> 4554
   Ser Phe Cys Gln Arg Val Arg Ser Gly Asp Trp Lys Gly Tyr Thr Gly
   1 5 10 15
   Lys Thr Ile
<210> 4555
   <211> 21
   <212> PRT
   <213> Homo sapiens
<400> 4555
   Tyr Leu Ala Ser Ala Ser Thr Met Asp His Ala Arg His Gly Phe Leu
   1 5 10 15
   Pro Arg Arg His Arg
   20
<210> 4556
   <211> 5
   <212> PRT
   <213> Homo sapiens
<400> 4556
   Pro Ala Pro Lys Lys
   1 5
<210> 4557
   <211> 5
   <212> PRT
   <213> Homo sapiens
<400> 4557
   Pro Ala Pro Lys Lys
   1 5
<210> 4558
   <211> 7
   <212> PRT
   <213> Homo sapiens
<400> 4558
   Pro Ala Pro Lys Lys Gly Ser
   1 5
<210> 4559
   <211> 8
   <212> PRT
   <213> Homo sapiens
<400> 4559
   Pro Ala Pro Lys Lys Gly Ser Lys
   1 5
<210> 4560
   <211> 9
   <212> PRT
   <213> Homo sapiens
<400> 4560
   Pro Ala Pro Lys Lys Gly Ser Lys Lys
   1 5
<210> 4561
   <211> 5
   <212> PRT
   <213> Homo sapiens
<400> 4561
   Pro Glu Pro Ala Lys
   1 5
<210> 4562
   <211> 21
   <212> PRT
   <213> Homo sapiens
<400> 4562
   Pro Glu Pro Ala Lys Ser Ala Pro Ala Pro Lys Lys Gly Ser Lys Lys
   1               5                   10                  15
   Ala Val Thr Lys Ala
               20
<210> 4563
   <211> 21
   <212> PRT
   <213> Homo sapiens
<400> 4563
   Pro Glu Pro Ala Lys Ser Ala Pro Ala Pro Lys Lys Gly Ser Lys Lys
   1               5                   10                  15
   Ala Val Thr Lys Ala
               20
<210> 4564
   <211> 21
   <212> PRT
   <213> Homo sapiens
<400> 4564
   Pro Glu Pro Ala Lys Ser Ala Pro Ala Pro Lys Lys Gly Ser Lys Lys
   1               5                   10                  15
   Ala Val Thr Lys Ala
               20
<210> 4565
   <211> 9
   <212> PRT
   <213> Mus musculus
<400> 4565
   Lys Thr Glu Lys Asp Pro Glu Ser Glu
   1               5
<210> 4566
   <211> 12
   <212> PRT
   <213> Bos taurus
<400> 4566
   Ala Ala Gly Ala Val Met Arg Gly Phe Val Phe Thr
   1               5                   10
<210> 4567
   <211> 13
   <212> PRT
   <213> Homo sapiens
<400> 4567
   Ile Pro Ser Thr Val Lys Ala Gly Glu Leu Glu Lys Ile
   1 5 10
<210> 4568
   <211> 12
   <212> PRT
   <213> Homo sapiens
<400> 4568
   Ile Ser Arg Cys Gln Val Cys Met Lys Lys Arg His
   1 5 10
<210> 4569
   <211> 5
   <212> PRT
   <213> Homo sapiens
<400> 4569
   Met Lys Lys Arg His
   1 5
<210> 4570
   <211> 11
   <212> PRT
   <213> Homo sapiens
<400> 4570
   Ser Leu Asn Pro Glu Arg Met Phe Arg Lys Pro
   1 5 10
<210> 4571
   <211> 36
   <212> PRT
   <213> Homo sapiens
<400> 4571
   Ser Leu Asn Pro Glu Arg Met Phe Arg Lys Pro Ile Pro Ser Thr Val
   1 5 10 15
   Lys Ala Gly Glu Leu Glu Lys Ile Ile Ser Arg Cys Gln Val Cys Met
   20 25 30
   Lys Lys Arg His
   35
<210> 4572
   <211> 15
   <212> PRT
   <213> Homo sapiens
<400> 4572
   Thr Ala Ile Pro Ser Cys Pro Glu Gly Thr Val Pro Leu Tyr Ser
   1               5                   10                  15
<210> 4573
   <211> 9
   <212> PRT
   <213> Homo sapiens
<400> 4573
   Ile Ser Leu Trp Lys Gly Phe Ser Phe
   1               5
<210> 4574
   <211> 15
   <212> PRT
   <213> Homo sapiens
<400> 4574
   Thr Asp Ile Pro Pro Cys Pro His Gly Trp Ile Ser Leu Trp Lys
   1               5                   10                  15
<210> 4575
   <211> 20
   <212> PRT
   <213> Bos taurus
<400> 4575
   Cys Asn Phe Ala Ser Arg Asn Asp Tyr Ser Tyr Trp Leu Ser Thr Pro
   1               5                   10                  15
   Glu Pro Met Pro
               20
<210> 4576
   <211> 9
   <212> PRT
   <213> Homo sapiens
<400> 4576
   Gly Ser Pro Ala Thr Trp Thr Thr Arg
   1               5
<210> 4577
   <211> 239
   <212> PRT
   <213> Homo sapiens
<400> 4577
   Arg Gly Asp Ser Gly Ser Pro Ala Thr Trp Thr Thr Arg Gly Phe Val
   1               5                   10                  15
   Phe Thr Arg His Ser Gln Thr Thr Ala Ile Pro Ser Cys Pro Glu Gly
               20                  25                  30
   Thr Val Pro Leu Tyr Ser Gly Phe Ser Phe Leu Phe Val Gln Gly Asn
           35                  40                  45
   Gln Arg Ala His Gly Gln Asp Leu Gly Thr Leu Gly Ser Cys Leu Gln
       50                  55                  60
   Arg Phe Thr Thr Met Pro Phe Leu Phe Cys Asn Val Asn Asp Val Cys
   65                  70                  75                  80
   Asn Phe Ala Ser Arg Asn Asp Tyr Ser Tyr Trp Leu Ser Thr Pro Ala
                   85                  90                  95
   Leu Met Pro Met Asn Met Ala Pro Ile Thr Gly Arg Ala Leu Glu Pro
               100                 105                 110
   Tyr Ile Ser Arg Cys Thr Val Cys Glu Gly Pro Ala Ile Ala Ile Ala
           115                 120                 125
   Val His Ser Gln Thr Thr Asp Ile Pro Pro Cys Pro His Gly Trp Ile
       130                 135                 140
   Ser Leu Trp Lys Gly Phe Ser Phe Ile Met Phe Thr Ser Ala Gly Ser
   145                 150                 155                 160
   Glu Gly Thr Gly Gln Ala Leu Ala Ser Pro Gly Ser Cys Leu Glu Glu
                   165                 170                 175
   Phe Arg Ala Ser Pro Phe Leu Glu Cys His Gly Arg Gly Thr Cys Asn
               180                 185                 190
   Tyr Tyr Ser Asn Ser Tyr Ser Phe Trp Leu Ala Ser Leu Asn Pro Glu
           195                 200                 205
   Arg Met Phe Arg Lys Pro Ile Pro Ser Thr Val Lys Ala Gly Glu Leu
       210                 215                 220
   Glu Lys Ile Ile Ser Arg Cys Gln Val Cys Met Lys Lys Arg His
   225                 230                 235
<210> 4578
   <211> 15
   <212> PRT
   <213> Bos taurus
<400> 4578
   Cys Phe Glu Gly Gln Glu Lys Ala His Asn Gln Asp Leu Gly Leu
   1               5                   10                  15
<210> 4579
   <211> 15
   <212> PRT
   <213> Bos taurus
<400> 4579
   Cys His Gly Arg Gly Thr Cys Asn Tyr Tyr Ala Asn Ala Tyr Ser
   1               5                   10                  15
<210> 4580
   <211> 19
   <212> PRT
   <213> Bos taurus
<400> 4580
   Cys Asn Tyr Tyr Ser Asn Ser Tyr Ser Phe Trp Leu Ala Ser Leu Asp
   1               5                   10                  15
   Pro Lys Arg
<210> 4581
   <211> 13
   <212> PRT
   <213> Bos taurus
<400> 4581
   Cys Pro Ala Gly Trp Ile Ser Leu Trp Lys Gly Phe Ser
   1               5                   10
<210> 4582
   <211> 23
   <212> PRT
   <213> Bos taurus
<400> 4582
   Cys Thr Ile Glu Arg Ser Glu Met Phe Lys Pro Thr Pro Ser Thr Leu
   1               5                   10                  15
   Lys Ala Gly Glu Leu Arg Thr
               20
<210> 4583
   <211> 14
   <212> PRT
   <213> Bos taurus
<400> 4583
   Cys Tyr Tyr Ala Ser Arg Asn Asp Lys Ser Tyr Trp Leu Ser
   1               5                   10
<210> 4584
   <211> 18
   <212> PRT
   <213> Bos taurus
<400> 4584
   Asp Val Cys Asn Phe Ala Ser Arg Asn Asp Tyr Ser Tyr Trp Leu Ser
   1               5                   10                  15
   Thr Pro
<210> 4585
   <211> 6
   <212> PRT
   <213> Bos taurus
<400> 4585
   Gly Leu Gly Phe Tyr Gly
   1               5
<210> 4586
   <211> 18
   <212> PRT
   <213> Homo sapiens
<400> 4586
   Lys Phe Asp Val Pro Cys Gly Gly Arg Asp Cys Ser Gly Gly Cys Gln
   1               5                   10                  15
   Cys Tyr
<210> 4587
   <211> 14
   <212> PRT
   <213> Bos taurus
<400> 4587
   Leu Glu Pro Tyr Ile Ser Arg Cys Thr Val Cys Glu Gly Pro
   1               5                   10
<210> 4588
   <211> 13
   <212> PRT
   <213> Bos taurus
<400> 4588
   Pro Tyr Ala Leu Gly Lys Glu Glu Arg Asp Arg Tyr Arg
   1 5 10
<210> 4589
   <211> 20
   <212> PRT
   <213> Homo sapiens
<400> 4589
   Ala Ile Ala Val His Ser Gln Thr Thr Asp Ile Pro Pro Cys Pro His
   1 5 10 15
   Gly Trp Ile Ser
   20
<210> 4590
   <211> 20
   <212> PRT
   <213> Homo sapiens
<400> 4590
   Ala Leu Ala Ser Pro Gly Ser Cys Leu Glu Glu Phe Arg Ala Ser Pro
   1 5 10 15
   Phe Leu Glu Cys
   20
<210> 4591
   <211> 20
   <212> PRT
   <213> Homo sapiens
<400> 4591
   Ala Leu Met Pro Met Asn Met Ala Pro Ile Thr Gly Arg Ala Leu Glu
   1 5 10 15
   Pro Tyr Ile Ser
   20
<210> 4592
   <211> 20
   <212> PRT
   <213> Homo sapiens
<400> 4592
   Glu Phe Arg Ala Ser Pro Phe Leu Glu Cys His Gly Arg Gly Thr Cys
   1 5 10 15
   Asn Tyr Tyr Ser
   20
<210> 4593
   <211> 20
   <212> PRT
   <213> Homo sapiens
<400> 4593
   Phe Thr Thr Met Pro Phe Leu Phe Cys Asn Val Asn Asp Val Cys Asn
   1               5                   10                  15
   Phe Ala Ser Arg
               20
<210> 4594
   <211> 20
   <212> PRT
   <213> Homo sapiens
<400> 4594
   His Gly Arg Gly Thr Cys Asn Tyr Tyr Ser Asn Ser Tyr Ser Phe Trp
   1               5                   10                  15
   Leu Ala Ser Leu
               20
<210> 4595
   <211> 20
   <212> PRT
   <213> Homo sapiens
<400> 4595
   Ile Pro Pro Cys Pro His Gly Trp Ile Ser Leu Trp Lys Gly Phe Ser
   1               5                   10                  15
   Phe Ile Met Phe
               20
<210> 4596
   <211> 20
   <212> PRT
   <213> Homo sapiens
<400> 4596
   Ile Pro Ser Cys Pro Glu Gly Thr Val Pro Leu Tyr Ser Gly Phe Ser
   1               5                   10                  15
   Phe Leu Phe Val
               20
<210> 4597
   <211> 14
   <212> PRT
   <213> Homo sapiens
<400> 4597
   Lys Ile Ile Ser Arg Cys Gln Val Cys Met Lys Lys Arg His
   1 5 10
<210> 4598
   <211> 20
   <212> PRT
   <213> Homo sapiens
<400> 4598
   Leu Gly Thr Leu Gly Ser Cys Leu Gln Arg Phe Thr Thr Met Pro Phe
   1 5 10 15
   Leu Phe Cys Asn
   20
<210> 4599
   <211> 20
   <212> PRT
   <213> Homo sapiens
<400> 4599
   Leu Trp Lys Gly Phe Ser Phe Ile Met Phe Thr Ser Ala Gly Ser Glu
   1 5 10 15
   Gly Thr Gly Gln
   20
<210> 4600
   <211> 20
   <212> PRT
   <213> Homo sapiens
<400> 4600
   Leu Tyr Ser Gly Phe Ser Phe Leu Phe Val Gln Gly Asn Gln Arg Ala
   1 5 10 15
   His Gly Gln Asp
   20
<210> 4601
   <211> 20
   <212> PRT
   <213> Homo sapiens
<400> 4601
   Asn Asp Tyr Ser Tyr Trp Leu Ser Ile Pro Ala Leu Met Pro Met Asn
   1               5                   10                  15
   Met Ala Pro Ile
               20
<210> 4602
   <211> 20
   <212> PRT
   <213> Homo sapiens
<400> 4602
   Asn Ser Tyr Ser Phe Trp Leu Ala Ser Leu Asn Pro Glu Arg Met Phe
   1               5                   10                  15
   Arg Lys Pro Ile
               20
<210> 4603
   <211> 20
   <212> PRT
   <213> Homo sapiens
<400> 4603
   Pro Ser Thr Val Lys Ala Gly Glu Leu Glu Lys Ile Ile Ser Arg Cys
   1               5                   10                  15
   Gln Val Cys Met
               20
<210> 4604
   <211> 20
   <212> PRT
   <213> Homo sapiens
<400> 4604
   Arg Cys Thr Val Cys Glu Gly Pro Ala Ile Ala Ile Ala Val His Ser
   1               5                   10                  15
   Gln Thr Thr Asp
               20
<210> 4605
   <211> 20
   <212> PRT
   <213> Homo sapiens
<400> 4605
   Ser Pro Ala Thr Trp Thr Thr Arg Gly Phe Val Phe Thr Arg His Ser
   1               5                   10                  15
   Gln Thr Thr Ala
               20
<210> 4606
   <211> 20
   <212> PRT
   <213> Homo sapiens
<400> 4606
   Thr Gly Arg Ala Leu Glu Pro Tyr Ile Ser Arg Cys Thr Val Cys Glu
   1               5                   10                  15
   Gly Pro Ala Ile
               20
<210> 4607
   <211> 20
   <212> PRT
   <213> Homo sapiens
<400> 4607
   Thr Ser Ala Gly Ser Glu Gly Thr Gly Gln Ala Leu Ala Ser Pro Gly
   1               5                   10                  15
   Ser Cys Leu Glu
               20
<210> 4608
   <211> 21
   <212> PRT
   <213> Homo sapiens
<400> 4608
   Val Phe Thr Arg His Ser Gln Thr Thr Ala Ile Pro Ser Cys Pro Glu
   1               5                   10                  15
   Gly Thr Val Pro Leu
               20
<210> 4609
   <211> 21
   <212> PRT
   <213> Homo sapiens
<400> 4609
   Val Asn Asp Val Cys Asn Phe Ala Ser Arg Asn Asp Tyr Ser Tyr Trp
   1               5                   10                  15
   Leu Ser Thr Pro Ala
               20
<210> 4610
   <211> 44
   <212> PRT
   <213> Homo sapiens
<400> 4610
   Pro Gly Leu Lys Gly Lys Arg Gly Asp Ser Gly Ser Pro Ala Thr Trp
   1               5                   10                  15
   Thr Thr Arg Gly Phe Val Phe Thr Arg His Ser Gln Thr Thr Ala Ile
               20                  25                  30
   Pro Ser Cys Pro Glu Gly Thr Val Pro Leu Tyr Ser
           35                  40
<210> 4611
   <211> 1670
   <212> PRT
   <213> Homo sapiens
<400> 4611
   Met Ser Ala Arg Thr Ala Pro Arg Pro Gln Val Leu Leu Leu Pro Leu
   1               5                   10                  15
   Leu Leu Val Leu Leu Ala Ala Ala Pro Ala Ala Ser Lys Gly Cys Val
               20                  25                  30
   Cys Lys Asp Lys Gly Gln Cys Phe Cys Asp Gly Ala Lys Gly Glu Lys
           35                  40                  45
   Gly Glu Lys Gly Phe Pro Gly Pro Pro Gly Ser Pro Gly Gln Lys Gly
       50                  55                  60
   Phe Thr Gly Pro Glu Gly Leu Pro Gly Pro Gln Gly Pro Lys Gly Phe
   65                  70                  75                  80
   Pro Gly Leu Pro Gly Leu Thr Gly Ser Lys Gly Val Arg Gly Ile Ser
   85 90 95
   Gly Leu Pro Gly Phe Ser Gly Ser Pro Gly Leu Pro Gly Thr Pro Gly
   100 105 110
   Asn Thr Gly Pro Tyr Gly Leu Val Gly Val Pro Gly Cys Ser Gly Ser
   115 120 125
   Lys Gly Glu Gln Gly Phe Pro Gly Leu Pro Gly Thr Pro Gly Tyr Pro
   130 135 140
   Gly Ile Pro Gly Ala Ala Gly Leu Lys Gly Gln Lys Gly Ala Pro Ala
   145 150 155 160
   Lys Glu Glu Asp Ile Glu Leu Asp Ala Lys Gly Asp Pro Gly Leu Pro
   165 170 175
   Gly Ala Pro Gly Pro Gln Gly Leu Pro Gly Pro Pro Gly Phe Pro Gly
   180 185 190
   Pro Val Gly Pro Pro Gly Pro Pro Gly Phe Phe Gly Phe Pro Gly Ala
   195 200 205
   Met Gly Pro Arg Gly Pro Lys Gly His Met Gly Glu Arg Val Ile Gly
   210 215 220
   His Lys Gly Glu Arg Gly Val Lys Gly Leu Thr Gly Pro Pro Gly Pro
   225 230 235 240
   Pro Gly Thr Val Ile Val Thr Leu Thr Gly Pro Asp Asn Arg Thr Asp
   245 250 255
   Leu Lys Gly Glu Lys Gly Asp Lys Gly Ala Met Gly Glu Pro Gly Pro
   260 265 270
   Pro Gly Pro Ser Gly Leu Pro Gly Glu Ser Tyr Gly Ser Glu Lys Gly
   275 280 285
   Ala Pro Gly Asp Pro Gly Leu Gln Gly Lys Pro Gly Lys Asp Gly Val
   290 295 300
   Pro Gly Phe Pro Gly Ser Glu Gly Val Lys Gly Asn Arg Gly Phe Pro
   305 310 315 320
   Gly Leu Met Gly Glu Tyr Gly Ile Lys Gly Gln Lys Gly Asp Ile Gly
   325 330 335
   Pro Pro Gly Phe Arg Gly Pro Thr Glu Tyr Tyr Asp Thr Tyr Gln Glu
               340                 345                 350
   Lys Gly Asp Glu Gly Thr Pro Gly Pro Pro Gly Pro Arg Gly Ala Arg
           355                 360                 365
   Gly Pro Gln Gly Pro Ser Gly Pro Pro Gly Val Pro Gly Ser Pro Gly
       370                 375                 380
   Ser Ser Arg Pro Gly Leu Arg Gly Ala Pro Gly Trp Pro Gly Leu Lys
   385                 390                 395                 400
   Gly Ser Lys Gly Glu Arg Gly Arg Pro Gly Lys Asp Ala Met Gly Thr
                   405                 410                 415
   Pro Gly Ser Pro Gly Cys Ala Gly Ser Pro Gly Leu Pro Gly Ser Pro
               420                 425                 430
   Gly Pro Pro Gly Pro Pro Gly Asp Ile Val Phe Arg Lys Gly Pro Pro
           435                 440                 445
   Gly Asp His Gly Leu Pro Gly Tyr Leu Gly Ser Pro Gly Ile Pro Gly
       450                 455                 460
   Val Asp Gly Pro Lys Gly Glu Pro Gly Leu Leu Cys Thr Gln Cys Pro
   465                 470                 475                 480
   Tyr Ile Pro Gly Pro Pro Gly Leu Pro Gly Leu Pro Gly Leu His Gly
                   485                 490                 495
   Val Lys Gly Ile Pro Gly Arg Gln Gly Ala Ala Gly Leu Lys Gly Ser
               500                 505                 510
   Pro Gly Ser Pro Gly Asn Thr Gly Leu Pro Gly Phe Pro Gly Phe Pro
           515                 520                 525
   Gly Ala Gln Gly Asp Pro Gly Leu Lys Gly Glu Lys Gly Glu Thr Leu
       530                 535                 540
   Gln Pro Glu Gly Gln Val Gly Val Pro Gly Asp Pro Gly Leu Arg Gly
   545                 550                 555                 560
   Gln Pro Gly Arg Lys Gly Leu Asp Gly Ile Pro Gly Thr Pro Gly Val
                   565                 570                 575
   Lys Gly Leu Pro Gly Pro Lys Gly Glu Leu Ala Leu Ser Gly Glu Lys
               580                 585                 590
   Gly Asp Gln Gly Pro Pro Gly Asp Pro Gly Ser Pro Gly Ser Pro Gly
   595 600 605
   Pro Ala Gly Pro Ala Gly Pro Pro Gly Tyr Gly Pro Gln Gly Glu Pro
   610 615 620
   Gly Leu Gln Gly Thr Gln Gly Val Pro Gly Ala Pro Gly Pro Pro Gly
   625 630 635 640
   Glu Ala Gly Pro Arg Gly Glu Leu Ser Val Ser Thr Pro Val Pro Gly
   645 650 655
   Pro Pro Gly Pro Pro Gly Pro Pro Gly His Pro Gly Pro Gln Gly Pro
   660 665 670
   Pro Gly Ile Pro Gly Ser Leu Gly Lys Cys Gly Asp Pro Gly Leu Pro
   675 680 685
   Gly Pro Asp Gly Glu Pro Gly Ile Pro Gly Ile Gly Phe Pro Gly Pro
   690 695 700
   Pro Gly Pro Lys Gly Asp Gln Gly Phe Pro Gly Thr Lys Gly Ser Leu
   705 710 715 720
   Gly Cys Pro Gly Lys Met Gly Glu Pro Gly Leu Pro Gly Lys Pro Gly
   725 730 735
   Leu Pro Gly Ala Lys Gly Glu Pro Ala Val Ala Met Pro Gly Gly Pro
   740 745 750
   Gly Thr Pro Gly Phe Pro Gly Glu Arg Gly Asn Ser Gly Glu His Gly
   755 760 765
   Glu Ile Gly Leu Pro Gly Leu Pro Gly Leu Pro Gly Thr Pro Gly Asn
   770 775 780
   Glu Gly Leu Asp Gly Pro Arg Gly Asp Pro Gly Gln Pro Gly Pro Pro
   785 790 795 800
   Gly Glu Gln Gly Pro Pro Gly Arg Cys Ile Glu Gly Pro Arg Gly Ala
   805 810 815
   Gln Gly Leu Pro Gly Leu Asn Gly Leu Lys Gly Gln Gln Gly Arg Arg
   820 825 830
   Gly Lys Thr Gly Pro Lys Gly Asp Pro Gly Ile Pro Gly Leu Asp Arg
   835 840 845
   Ser Gly Phe Pro Gly Glu Thr Gly Ser Pro Gly Ile Pro Gly His Gln
       850                 855                 860
   Gly Glu Met Gly Pro Leu Gly Gln Arg Gly Tyr Pro Gly Asn Pro Gly
   865                 870                 875                 880
   Ile Leu Gly Pro Pro Gly Glu Asp Gly Val Ile Gly Met Met Gly Phe
                   885                 890                 895
   Pro Gly Ala Ile Gly Pro Pro Gly Pro Pro Gly Asn Pro Gly Arg Pro
               900                 905                 910
   Gly Gln Arg Gly Ser Pro Gly Ile Pro Gly Val Lys Gly Gln Arg Gly
           915                 920                 925
   Thr Pro Gly Ala Lys Gly Glu Gln Gly Asp Lys Gly Asn Pro Gly Pro
       930                 935                 940
   Ser Glu Ile Ser His Val Ile Gly Asp Lys Gly Glu Pro Gly Leu Lys
   945                 950                 955                 960
   Gly Phe Ala Gly Asn Pro Gly Glu Lys Gly Asn Arg Gly Val Pro Gly
                   965                 970                 975
   Met Pro Gly Leu Lys Gly Leu Lys Gly Leu Pro Gly Pro Ala Gly Pro
               980                 985                 990
   Pro Gly Pro Arg Gly Asp Leu Gly  Ser Thr Gly Asn Pro  Gly Glu Pro
           995                 1000                1005
   Gly Leu  Arg Gly Ile Pro Gly  Ser Met Gly Asn Met  Gly Met Pro
       1010                1015                1020
   Gly Ser  Lys Gly Lys Arg Gly  Thr Leu Gly Phe Pro  Gly Arg Ala
       1025                1030                1035
   Gly Arg  Pro Gly Leu Pro Gly  Ile His Gly Leu Gln  Gly Asp Lys
       1040                1045                1050
   Gly Glu  Pro Gly Tyr Ser Glu  Gly Thr Arg Pro Gly  Pro Pro Gly
       1055                1060                1065
   Pro Thr  Gly Asp Pro Gly Leu  Pro Gly Asp Met Gly  Lys Lys Gly
       1070                1075                1080
   Glu Met  Gly Gln Pro Gly Pro  Pro Gly His Leu Gly  Pro Ala Gly
   1085 1090 1095
   Pro Glu Gly Ala Pro Gly Ser Pro Gly Ser Pro Gly Leu Pro Gly
   1100 1105 1110
   Lys Pro Gly Pro His Gly Asp Leu Gly Phe Lys Gly Ile Lys Gly
   1115 1120 1125
   Leu Leu Gly Pro Pro Gly Ile Arg Gly Pro Pro Gly Leu Pro Gly
   1130 1135 1140
   Phe Pro Gly Ser Pro Gly Pro Met Gly Ile Arg Gly Asp Gln Gly
   1145 1150 1155
   Arg Asp Gly Ile Pro Gly Pro Ala Gly Glu Lys Gly Glu Thr Gly
   1160 1165 1170
   Leu Leu Arg Ala Pro Pro Gly Pro Arg Gly Asn Pro Gly Ala Gln
   1175 1180 1185
   Gly Ala Lys Gly Asp Arg Gly Ala Pro Gly Phe Pro Gly Leu Pro
   1190 1195 1200
   Gly Arg Lys Gly Ala Met Gly Asp Ala Gly Pro Arg Gly Pro Thr
   1205 1210 1215
   Gly Ile Glu Gly Phe Pro Gly Pro Pro Gly Leu Pro Gly Ala Ile
   1220 1225 1230
   Ile Pro Gly Gln Thr Gly Asn Arg Gly Pro Pro Gly Ser Arg Gly
   1235 1240 1245
   Ser Pro Gly Ala Pro Gly Pro Pro Gly Pro Pro Gly Ser His Val
   1250 1255 1260
   Ile Gly Ile Lys Gly Asp Lys Gly Ser Met Gly His Pro Gly Pro
   1265 1270 1275
   Lys Gly Pro Pro Gly Thr Ala Gly Asp Met Gly Pro Pro Gly Arg
   1280 1285 1290
   Leu Gly Ala Pro Gly Thr Pro Gly Leu Pro Gly Pro Arg Gly Asp
   1295 1300 1305
   Pro Gly Phe Gln Gly Phe Pro Gly Val Lys Gly Glu Lys Gly Asn
   1310 1315 1320
   Pro Gly  Phe Leu Gly Ser Ile  Gly Pro Pro Gly Pro  Ile Gly Pro
       1325                 1330                 1335
   Lys Gly  Pro Pro Gly Val Arg  Gly Asp Pro Gly Thr  Leu Lys Ile
       1340                 1345                 1350
   Ile Ser  Leu Pro Gly Ser Pro  Gly Pro Pro Gly Thr  Pro Gly Glu
       1355                 1360                 1365
   Pro Gly  Met Gln Gly Glu Pro  Gly Pro Pro Gly Pro  Pro Gly Asn
       1370                 1375                 1380
   Leu Gly  Pro Cys Gly Pro Arg  Gly Lys Pro Gly Lys  Asp Gly Lys
       1385                 1390                 1395
   Pro Gly  Thr Pro Gly Pro Ala  Gly Glu Lys Gly Asn  Lys Gly Ser
       1400                 1405                 1410
   Lys Gly  Glu Pro Gly Pro Ala  Gly Ser Asp Gly Leu  Pro Gly Leu
       1415                 1420                 1425
   Lys Gly  Lys Arg Gly Asp Ser  Gly Ser Pro Ala Thr  Trp Thr Thr
       1430                 1435                 1440
   Arg Gly  Phe Val Phe Thr Arg  His Ser Gln Thr Thr  Ala Ile Pro
       1445                 1450                 1455
   Ser Cys  Pro Glu Gly Thr Val  Pro Leu Tyr Ser Gly  Phe Ser Phe
       1460                 1465                 1470
   Leu Phe  Val Gln Gly Asn Gln  Arg Ala His Gly Gln  Asp Leu Gly
       1475                 1480                 1485
   Thr Leu  Gly Ser Cys Leu Gln  Arg Phe Thr Thr Met  Pro Phe Leu
       1490                 1495                 1500
   Phe Cys  Asn Val Asn Asp Val  Cys Asn Phe Ala Ser  Arg Asn Asp
       1505                 1510                 1515
   Tyr Ser  Tyr Trp Leu Ser Thr  Pro Ala Leu Met Pro  Met Asn Met
       1520                 1525                 1530
   Ala Pro  Ile Thr Gly Arg Ala  Leu Glu Pro Tyr Ile  Ser Arg Cys
       1535                 1540                 1545
   Thr Val  Cys Glu Gly Pro Ala  Ile Ala Ile Ala Val  His Ser Gln
       1550                 1555                 1560
   Thr Thr Asp Ile Pro Pro Cys Pro His Gly Trp Ile Ser Leu Trp
   1565 1570 1575
   Lys Gly Phe Ser Phe Ile Met Phe Thr Ser Ala Gly Ser Glu Gly
   1580 1585 1590
   Thr Gly Gln Ala Leu Ala Ser Pro Gly Ser Cys Leu Glu Glu Phe
   1595 1600 1605
   Arg Ala Ser Pro Phe Leu Glu Cys His Gly Arg Gly Thr Cys Asn
   1610 1615 1620
   Tyr Tyr Ser Asn Ser Tyr Ser Phe Trp Leu Ala Ser Leu Asn Pro
   1625 1630 1635
   Glu Arg Met Phe Arg Lys Pro Ile Pro Ser Thr Val Lys Ala Gly
   1640 1645 1650
   Glu Leu Glu Lys Ile Ile Ser Arg Cys Gln Val Cys Met Lys Lys
   1655 1660 1665
   Arg His
   1670
<210> 4612
   <211> 20
   <212> PRT
   <213> Mus musculus
<400> 4612
   Cys Pro Gln Asp Trp Val Ser Leu Trp Lys Gly Phe Ser Phe Ile Met
   1 5 10 15
   Phe Thr Ser Ala
   20
<210> 4613
   <211> 11
   <212> PRT
   <213> Mus musculus
<400> 4613
   Asp Trp Val Ser Leu Trp Lys Gly Phe Ser Phe
   1 5 10
<210> 4614
   <211> 16
   <212> PRT
   <213> Mycobacterium tuberculosis
<400> 4614
   Asp Leu Ser Leu Leu Gly Lys Ala Arg Lys Val Val Val Thr Lys Asp
   1               5                   10                  15
<210> 4615
   <211> 15
   <212> PRT
   <213> Mycobacterium bovis
<400> 4615
   Asn Pro Leu Gly Leu Lys Arg Gly Ile Glu Lys Ala Val Glu Lys
   1               5                   10                  15
<210> 4616
   <211> 16
   <212> PRT
   <213> Homo sapiens
<400> 4616
   Gln Pro His Asp Leu Gly Lys Val Gly Glu Val Ile Val Thr Lys Asp
   1               5                   10                  15
<210> 4617
   <211> 13
   <212> PRT
   <213> Human herpesvirus
<400> 4617
   Ser Tyr Leu Lys Asp Ser Asp Phe Leu Asp Ala Ala Leu
   1               5                   10
<210> 4618
   <211> 13
   <212> PRT
   <213> Homo sapiens
<400> 4618
   Ser Tyr Leu Gln Asp Ser Asp Pro Asp Ser Phe Gln Asp
   1               5                   10
<210> 4619
   <211> 14
   <212> PRT
   <213> Homo sapiens
<400> 4619
   Ala Leu Asn Glu Asp Leu Arg Ser Trp Thr Ala Ala Asp Thr
   1               5                   10
<210> 4620
   <211> 14
   <212> PRT
   <213> Homo sapiens
<400> 4620
   Ala Leu Asn Glu Asp Leu Ser Ser Trp Thr Ala Ala Asp Thr
   1 5 10
<210> 4621
   <211> 15
   <212> PRT
   <213> Homo sapiens
<400> 4621
   Asn Pro Val Glu Ile Arg Arg Gly Val Met Leu Ala Val Asp Ala
   1 5 10 15
<210> 4622
   <211> 25
   <212> PRT
   <213> Homo sapiens
<400> 4622
   Gln Val Ser Gly Pro Glu Arg Thr Ile Pro Ile Thr Arg Glu Glu Lys
   1 5 10 15
   Pro Ala Val Thr Ala Ala Pro Lys Lys
   20 25
<210> 4623
   <211> 14
   <212> PRT
   <213> Homo sapiens
<400> 4623
   Asp Leu Tyr Ile Leu Met Ser His Thr Ser Gly Ser Ala Ala
   1 5 10
<210> 4624
   <211> 14
   <212> PRT
   <213> Homo sapiens
<400> 4624
   Phe Leu Gly Glu Leu Thr Ser Ser Glu Val Ala Thr Glu Val
   1 5 10
<210> 4625
   <211> 14
   <212> PRT
   <213> Homo sapiens
<400> 4625
   Gly Val Tyr Leu Leu Thr Ser His Arg Thr Ala Thr Ala Ala
   1 5 10
<210> 4626
   <211> 16
   <212> PRT
   <213> Homo sapiens
<400> 4626
   Lys Ser Met Val Ile Leu Thr Ser Thr Val Thr Ala Gly Thr Ala Glu
   1 5 10 15
<210> 4627
   <211> 12
   <212> PRT
   <213> Bos taurus
<400> 4627
   Asn Asn Arg Glu Arg Arg Gly Ile Ala Leu Asp Gly
   1 5 10
<210> 4628
   <211> 15
   <212> PRT
   <213> Bos taurus
<400> 4628
   Ala Asp Gly Ser Ser Trp Glu Gly Val Gly Val Val Pro Asp Val
   1 5 10 15
<210> 4629
   <211> 20
   <212> PRT
   <213> Homo sapiens
<400> 4629
   Gly Pro Thr His Leu Phe Gln Pro Ser Leu Val Leu Asp Met Ala Lys
   1 5 10 15
   Val Leu Leu Asp
   20
<210> 4630
   <211> 20
   <212> PRT
   <213> Homo sapiens
<400> 4630
   Ser Gly Ile Pro Tyr Ile Ile Ser Tyr Leu His Pro Gly Asn Thr Ile
   1 5 10 15
   Leu His Val Asp
   20
<210> 4631
   <211> 20
   <212> PRT
   <213> Homo sapiens
<400> 4631
   Ala Ser Ser Thr Ile Ile Lys Glu Gly Ile Asp Arg Thr Val Leu Gly
   1 5 10 15
   Ile Leu Val Ser
   20
<210> 4632
   <211> 13
   <212> PRT
   <213> Homo sapiens
<400> 4632
   Asp Thr Asn Leu Ala Ser Ser Thr Ile Ile Lys Glu Gly
   1 5 10
<210> 4633
   <211> 20
   <212> PRT
   <213> Homo sapiens
<400> 4633
   Gly Phe Leu Gly Glu Leu Thr Ser Ser Glu Val Ala Thr Glu Val Pro
   1 5 10 15
   Phe Arg Leu Met
   20
<210> 4634
   <211> 16
   <212> PRT
   <213> Bos taurus
<400> 4634
   Leu Pro Leu Leu Ala Asn Asn Arg Glu Arg Arg Gly Ile Ala Leu Asp
   1 5 10 15
<210> 4635
   <211> 14
   <212> PRT
   <213> Bos taurus
<400> 4635
   Asn Leu Tyr Leu Thr Ile Pro Thr Ala Arg Ser Val Gly Ala
   1 5 10
<210> 4636
   <211> 20
   <212> PRT
   <213> Homo sapiens
<400> 4636
   Asn Arg Glu Arg Arg Gly Ile Ala Leu Asp Gly Lys Ile Lys His Glu
   1 5 10 15
   Asp Thr Asn Leu
   20
<210> 4637
   <211> 12
   <212> PRT
   <213> Homo sapiens
<400> 4637
   Pro Thr Ala Arg Ser Val Gly Ala Ala Asp Gly Ser
   1 5 10
<210> 4638
   <211> 23
   <212> PRT
   <213> Bos taurus
<400> 4638
   Pro Thr Ala Arg Ser Val Gly Ala Ala Asp Gly Ser Ser Trp Glu Gly
   1 5 10 15
   Val Gly Val Val Pro Asp Val
   20
<210> 4639
   <211> 14
   <212> PRT
   <213> Bos taurus
<400> 4639
   Val Gly Ala Ala Asp Gly Ser Ser Trp Glu Gly Val Gly Val
   1 5 10
<210> 4640
   <211> 20
   <212> PRT
   <213> Homo sapiens
<400> 4640
   Tyr Gln Ile Lys Val Lys Leu Thr Val Ser Gly Phe Leu Gly Glu Leu
   1               5                   10                  15
   Thr Ser Ser Glu
               20
<210> 4641
   <211> 20
   <212> PRT
   <213> Homo sapiens
<400> 4641
   Leu Leu Ala Asn Asn Arg Glu Arg Arg Gly Ile Ala Leu Asp Gly Lys
   1               5                   10                  15
   Ile Lys His Glu
               20
<210> 4642
   <211> 16
   <212> PRT
   <213> Homo sapiens
<400> 4642
   Asn Arg Glu Arg Arg Gly Ile Ala Leu Asp Gly Lys Ile Lys His Glu
   1               5                   10                  15
<210> 4643
   <211> 25
   <212> PRT
   <213> Homo sapiens
<400> 4643
   Asp Lys Phe Val Ile Phe Leu Asp Val Lys His Phe Ser Pro Glu Asp
   1               5                   10                  15
   Leu Thr Val Lys Val Gln Asp Asp Phe
               20                  25
<210> 4644
   <211> 25
   <212> PRT
   <213> Homo sapiens
<400> 4644
   Gly Leu Asp Ala Thr His Ala Glu Arg Ala Ile Pro Val Ser Arg Glu
   1               5                   10                  15
   Glu Lys Pro Thr Ser Ala Pro Ser Ser
   20 25
<210> 4645
   <211> 25
   <212> PRT
   <213> Homo sapiens
<400> 4645
   Gly Met Leu Thr Phe Cys Gly Pro Lys Ile Gln Thr Gly Leu Asp Ala
   1 5 10 15
   Thr His Ala Glu Arg Ala Ile Pro Val
   20 25
<210> 4646
   <211> 25
   <212> PRT
   <213> Homo sapiens
<400> 4646
   Leu Ser Ser Asp Gly Val Leu Thr Val Asn Gly Pro Arg Lys Gln Val
   1 5 10 15
   Ser Gly Pro Glu Arg Thr Ile Pro Ile
   20 25
<210> 4647
   <211> 25
   <212> PRT
   <213> Homo sapiens
<400> 4647
   Pro Glu Glu Leu Lys Val Lys Val Leu Gly Asp Val Ile Glu Val His
   1 5 10 15
   Gly Lys His Glu Glu Arg Gln Asp Glu
   20 25
<210> 4648
   <211> 25
   <212> PRT
   <213> Homo sapiens
<400> 4648
   Arg Leu Glu Lys Asp Arg Phe Ser Val Asn Leu Asp Val Lys His Phe
   1 5 10 15
   Ser Pro Glu Glu Leu Lys Val Lys Val
   20 25
<210> 4649
   <211> 25
   <212> PRT
   <213> Homo sapiens
<400> 4649
   Thr Val Lys Val Gln Asp Asp Phe Val Glu Ile His Gly Lys His Asn
   1 5 10 15
   Glu Arg Gln Asp Asp His Gly Tyr Ile
   20 25
<210> 4650
   <211> 14
   <212> PRT
   <213> Mus musculus
<400> 4650
   Gln Thr Trp Glu Gly Ser Gly Val Leu Pro Cys Val Gly Thr
   1 5 10
<210> 4651
   <211> 20
   <212> PRT
   <213> Mus musculus
<400> 4651
   Ser Tyr Ser Ser Ala Val Pro Leu Leu Cys Ser Tyr Phe Phe Glu Ala
   1 5 10 15
   Glu Pro Arg Gln
   20
<210> 4652
   <211> 20
   <212> PRT
   <213> Unknown
<220>
   <223> epitope involved in autoimmune uvetisis
<400> 4652
   Glu Asn Ser Asp Ser Asp Tyr Asp Arg Glu Ala Leu Gln Glu Arg Leu
   1 5 10 15
   Ala Lys Leu Ala
   20
<210> 4653
   <211> 20
   <212> PRT
   <213> Mycobacterium leprae
<400> 4653
   Glu Asn Ser Asp Ser Asp Tyr Asp Arg Glu Lys Leu Gln Glu Arg Leu
   1               5                   10                  15
   Ala Lys Leu Ala
               20
<210> 4654
   <211> 15
   <212> PRT
   <213> Homo sapiens
<400> 4654
   Leu Ser Ser Val Val Val Asp Pro Ser Ile Arg His Phe Asp Val
   1               5                   10                  15
<210> 4655
   <211> 15
   <212> PRT
   <213> Homo sapiens
<400> 4655
   Asp Val Gly Glu Tyr Arg Ala Val Thr Glu Leu Gly Arg Pro Asp
   1               5                   10                  15
<210> 4656
   <211> 15
   <212> PRT
   <213> Homo sapiens
<400> 4656
   Asp Val Gly Val Tyr Arg Ala Val Thr Pro Gln Gly Arg Pro Asp
   1               5                   10                  15
<210> 4657
   <211> 14
   <212> PRT
   <213> Homo sapiens
<400> 4657
   Gly Asn Arg Ile Ala Gln Trp Gln Ser Phe Gln Leu Glu Gly
   1               5                   10
<210> 4658
   <211> 15
   <212> PRT
   <213> Homo sapiens
<400> 4658
   Lys Val Lys Lys Ile Tyr Leu Asp Glu Lys Arg Leu Leu Ala Gly
   1 5 10 15
<210> 4659
   <211> 31
   <212> PRT
   <213> Homo sapiens
<400> 4659
   Val His Leu Thr Pro Glu Glu Lys Ser Ala Val Thr Ala Leu Trp Gly
   1 5 10 15
   Lys Val Asn Val Asp Glu Val Gly Gly Glu Ala Leu Gly Arg Leu
   20 25 30
<210> 4660
   <211> 11
   <212> PRT
   <213> Homo sapiens
<400> 4660
   Val Leu Ser Pro Ala Asp Lys Thr Asn Val Lys
   1 5 10
<210> 4661
   <211> 31
   <212> PRT
   <213> Homo sapiens
<400> 4661
   Val Leu Ser Pro Ala Asp Lys Thr Asn Val Lys Ala Ala Trp Gly Lys
   1 5 10 15
   Val Gly Ala His Ala Gly Glu Tyr Gly Ala Glu Ala Leu Glu Arg
   20 25 30
<210> 4662
   <211> 14
   <212> PRT
   <213> Homo sapiens
<400> 4662
   Val Val Ala Gly Val Ala Asn Ala Leu Ala His Lys Tyr His
   1 5 10
<210> 4663
   <211> 19
   <212> PRT
   <213> Homo sapiens
<400> 4663
   Gly Leu Pro Arg Leu Glu Thr Pro Ala Asp Leu Ser Thr Ala Ile Ala
   1 5 10 15
   Ser Arg Ser
<210> 4664
   <211> 11
   <212> PRT
   <213> Homo sapiens
<400> 4664
   His Ala Arg Leu Arg Asp Ser Gly Arg Ala Tyr
   1 5 10
<210> 4665
   <211> 20
   <212> PRT
   <213> Homo sapiens
<400> 4665
   Cys Asn Glu Ser Ser Met Gln Ser Leu Arg Gln Arg Lys Ser Val Asn
   1 5 10 15
   Ala Leu Asn Ser
   20
<210> 4666
   <211> 20
   <212> PRT
   <213> Homo sapiens
<400> 4666
   Phe Gln Asp Thr His Asn Asn Ala His Tyr Tyr Val Phe Phe Glu Glu
   1 5 10 15
   Gln Glu Asp Glu
   20
<210> 4667
   <211> 19
   <212> PRT
   <213> Homo sapiens
<400> 4667
   Gly Ile Phe Asn Thr Gly Leu Lys Met Phe Pro Asp Leu Thr Lys Val
   1               5                   10                  15
   Tyr Ser Thr
<210> 4668
   <211> 16
   <212> PRT
   <213> Homo sapiens
<400> 4668
   Gly Pro Ser Leu Leu Asp Val Ser Gln Thr Ser Val Thr Ala Leu Pro
   1               5                   10                  15
<210> 4669
   <211> 19
   <212> PRT
   <213> Homo sapiens
<400> 4669
   Ile Gln Arg Ile Pro Ser Leu Pro Pro Ser Thr Gln Thr Leu Lys Leu
   1               5                   10                  15
   Ile Glu Thr
<210> 4670
   <211> 19
   <212> PRT
   <213> Homo sapiens
<400> 4670
   Ile Thr Asp Asn Pro Tyr Met Thr Ser Ile Pro Val Asn Ala Phe Gln
   1               5                   10                  15
   Gly Leu Cys
<210> 4671
   <211> 20
   <212> PRT
   <213> Homo sapiens
<400> 4671
   Lys Asn Pro Gln Glu Glu Thr Leu Gln Ala Phe Asp Ser His Tyr Asp
   1               5                   10                  15
   Tyr Thr Ile Cys
               20
<210> 4672
   <211> 20
   <212> PRT
   <213> Homo sapiens
<400> 4672
   Leu Thr Arg Ala Asp Leu Ser Tyr Pro Ser His Cys Cys Ala Phe Lys
   1 5 10 15
   Asn Gln Lys Lys
   20
<210> 4673
   <211> 19
   <212> PRT
   <213> Homo sapiens
<400> 4673
   Pro Asp Ala Leu Lys Glu Leu Pro Leu Leu Lys Phe Leu Gly Ile Phe
   1 5 10 15
   Asn Thr Gly
<210> 4674
   <211> 20
   <212> PRT
   <213> Homo sapiens
<400> 4674
   Pro Pro Ala Arg Ala Leu Lys Arg Ser Leu Trp Val Glu Val Asp Leu
   1 5 10 15
   Leu Ile Gly Ser
   20
<210> 4675
   <211> 20
   <212> PRT
   <213> Homo sapiens
<400> 4675
   Pro Tyr Glu Phe Ser Arg Lys Val Pro Thr Phe Ala Thr Pro Trp Pro
   1 5 10 15
   Asp Phe Val Pro
   20
<210> 4676
   <211> 19
   <212> PRT
   <213> Homo sapiens
<400> 4676
   Thr Lys Val Tyr Ser Thr Asp Ile Phe Phe Ile Leu Glu Ile Thr Asp
   1               5                   10                  15
   Asn Pro Tyr
<210> 4677
   <211> 20
   <212> PRT
   <213> Homo sapiens
<400> 4677
   Val Asn Ala Leu Asn Ser Pro Leu His Gln Glu Tyr Glu Glu Asn Leu
   1               5                   10                  15
   Gly Asp Ser Ile
               20
<210> 4678
   <211> 20
   <212> PRT
   <213> Homo sapiens
<400> 4678
   Ala Ser Arg Ser Val Ala Asp Lys Ile Leu Asp Leu Tyr Lys His Pro
   1               5                   10                  15
   Asp Asn Ile Asp
               20
<210> 4679
   <211> 12
   <212> PRT
   <213> Homo sapiens
<400> 4679
   Asp Pro Leu Ile Arg Gly Leu Leu Ala Arg Pro Ala
   1               5                   10
<210> 4680
   <211> 18
   <212> PRT
   <213> Homo sapiens
<400> 4680
   Glu Pro Thr Ser Gly Val Ile Ala Arg Ala Ala Glu Ile Met Glu Thr
   1               5                   10                  15
   Ser Ile
<210> 4681
   <211> 19
   <212> PRT
   <213> Homo sapiens
<400> 4681
   Phe Gln Gln Tyr Val Gly Pro Tyr Glu Gly Tyr Asp Ser Thr Ala Asn
   1 5 10 15
   Pro Thr Val
<210> 4682
   <211> 20
   <212> PRT
   <213> Homo sapiens
<400> 4682
   His Gly Leu Pro Gly Tyr Asn Glu Trp Arg Glu Phe Cys Gly Leu Pro
   1 5 10 15
   Arg Leu Glu Thr
   20
<210> 4683
   <211> 20
   <212> PRT
   <213> Homo sapiens
<400> 4683
   His Pro Asp Asn Ile Asp Val Trp Leu Gly Gly Leu Ala Glu Asn Phe
   1 5 10 15
   Leu Pro Arg Ala
   20
<210> 4684
   <211> 18
   <212> PRT
   <213> Homo sapiens
<400> 4684
   His Trp Ser Ala Asp Ala Val Tyr Gln Glu Ala Arg Lys Val Val Gly
   1 5 10 15
   Ala Leu
<210> 4685
   <211> 20
   <212> PRT
   <213> Homo sapiens
<400> 4685
   Ile Pro Gly Met Met Leu Glu Ala Trp Arg Glu Thr Phe Pro Gln Asp
   1               5                   10                  15
   Asp Lys Cys Gly
               20
<210> 4686
   <211> 20
   <212> PRT
   <213> Homo sapiens
<400> 4686
   Lys Phe Pro Glu Asp Phe Glu Ser Cys Asp Ser Ile Pro Gly Met Met
   1               5                   10                  15
   Leu Glu Ala Trp
               20
<210> 4687
   <211> 19
   <212> PRT
   <213> Homo sapiens
<400> 4687
   Lys Val Val Gly Ala Leu His Gln Ile Ile Thr Leu Arg Asp Tyr Ile
   1               5                   10                  15
   Pro Arg Ile
<210> 4688
   <211> 20
   <212> PRT
   <213> Homo sapiens
<400> 4688
   Leu Ile Gly Lys Gln Met Lys Ala Leu Arg Asp Gly Asp Trp Phe Trp
   1               5                   10                  15
   Trp Glu Asn Ser
               20
<210> 4689
   <211> 20
   <212> PRT
   <213> Homo sapiens
<400> 4689
   Leu Pro Arg Leu Glu Thr Pro Ala Asp Leu Ser Thr Ala Ile Ala Ser
   1 5 10 15
   Arg Ser Val Ala
   20
<210> 4690
   <211> 20
   <212> PRT
   <213> Homo sapiens
<400> 4690
   Asn Phe Leu Pro Arg Ala Arg Thr Gly Pro Leu Phe Ala Cys Leu Ile
   1 5 10 15
   Gly Lys Gln Met
   20
<210> 4691
   <211> 18
   <212> PRT
   <213> Homo sapiens
<400> 4691
   Asn Arg Leu Ala Ala Ala Leu Lys Ala Leu Asn Ala His Trp Ser Ala
   1 5 10 15
   Asp Ala
<210> 4692
   <211> 18
   <212> PRT
   <213> Homo sapiens
<400> 4692
   Arg Asp Tyr Ile Pro Arg Ile Leu Gly Pro Glu Ala Phe Gln Gln Tyr
   1 5 10 15
   Val Gly
<210> 4693
   <211> 13
   <212> PRT
   <213> Homo sapiens
<400> 4693
   Arg Gly Leu Leu Ala Arg Pro Ala Lys Leu Gln Val Gln
   1 5 10
<210> 4694
   <211> 18
   <212> PRT
   <213> Homo sapiens
<400> 4694
   Thr Ala Ala Phe Arg Phe Gly His Ala Thr Ile His Pro Leu Val Arg
   1               5                   10                  15
   Arg Leu
<210> 4695
   <211> 933
   <212> PRT
   <213> Homo sapiens
<400> 4695
<210> 4696
   <211> 26
   <212> PRT
   <213> Homo sapiens
<400> 4696
   Asp Asn Thr Gly Leu Thr Arg Val Pro Met Asp Ala Phe Gln Val Gly
   1 5 10 15
   Lys Phe Pro Glu Asp Phe Glu Ser Cys Asp
   20 25
<210> 4697
   <211> 20
   <212> PRT
   <213> Homo sapiens
<400> 4697
   Asp Ser His Tyr Asp Tyr Thr Ile Cys Gly Asp Ser Glu Asp Met Val
   1 5 10 15
   Cys Thr Pro Lys
   20
<210> 4698
   <211> 20
   <212> PRT
   <213> Homo sapiens
<400> 4698
   Phe Gln Gly Leu Cys Asn Glu Thr Leu Thr Leu Lys Leu Tyr Asn Asn
   1 5 10 15
   Gly Phe Thr Ser
   20
<210> 4699
   <211> 33
   <212> PRT
   <213> Homo sapiens
<400> 4699
   Gly Tyr Asn Glu Trp Arg Glu Phe Cys Gly Leu Pro Arg Leu Glu Thr
   1 5 10 15
   Pro Ala Asp Leu Ser Thr Ala Ile Ala Ser Arg Ser Val Ala Asp Lys
   20 25 30
   Ile
<210> 4700
   <211> 45
   <212> PRT
   <213> Homo sapiens
<400> 4700
   Ile Asn Leu Gln Arg Gly Arg Asp His Gly Leu Pro Gly Tyr Asn Glu
   1 5 10 15
   Trp Arg Glu Phe Cys Gly Leu Pro Arg Leu Glu Thr Pro Ala Asp Leu
   20 25 30
   Ser Thr Ala Ile Ala Ser Arg Ser Val Ala Asp Lys Ile
   35 40 45
<210> 4701
   <211> 20
   <212> PRT
   <213> Homo sapiens
<400> 4701
   Ile Arg Gly Ile Leu Glu Ser Leu Met Cys Asn Glu Ser Ser Met Gln
   1 5 10 15
   Ser Leu Arg Gln
   20
<210> 4702
   <211> 20
   <212> PRT
   <213> Homo sapiens
<400> 4702
   Lys Leu Pro Leu Ser Leu Ser Phe Leu His Leu Thr Arg Ala Asp Leu
   1 5 10 15
   Ser Tyr Pro Ser
   20
<210> 4703
   <211> 20
   <212> PRT
   <213> Homo sapiens
<400> 4703
   Leu Asp Ala Val Tyr Leu Asn Lys Asn Lys Tyr Leu Thr Val Ile Asp
   1 5 10 15
   Lys Asp Ala Phe
   20
<210> 4704
   <211> 20
   <212> PRT
   <213> Homo sapiens
<400> 4704
   Leu His Gln Glu Tyr Glu Glu Asn Leu Gly Asp Ser Ile Val Gly Tyr
   1 5 10 15
   Lys Glu Lys Ser
   20
<210> 4705
   <211> 20
   <212> PRT
   <213> Homo sapiens
<400> 4705
   Asn Gly Phe Thr Ser Val Gln Gly Tyr Ala Phe Asn Gly Thr Lys Leu
   1 5 10 15
   Asp Ala Val Tyr
   20
<210> 4706
   <211> 13
   <212> PRT
   <213> Homo sapiens
<400> 4706
   Gln Val Gly Lys Phe Pro Glu Asp Phe Glu Ser Cys Asp
   1 5 10
<210> 4707
   <211> 12
   <212> PRT
   <213> Homo sapiens
<400> 4707
   Ser Thr Asp Asp Tyr Ala Ser Phe Ser Arg Ala Leu
   1 5 10
<210> 4708
   <211> 20
   <212> PRT
   <213> Homo sapiens
<400> 4708
   Tyr Val Ser Ile Asp Val Thr Leu Gln Gln Leu Glu Ser His Ser Phe
   1               5                   10                  15
   Tyr Asn Leu Ser
               20
<210> 4709
   <211> 20
   <212> PRT
   <213> Homo sapiens
<400> 4709
   Tyr Tyr Val Phe Phe Glu Glu Gln Glu Asp Glu Ile Ile Gly Phe Gly
   1               5                   10                  15
   Gln Glu Leu Lys
               20
<210> 4710
   <211> 21
   <212> PRT
   <213> Homo sapiens
<400> 4710
   Asp Leu Tyr Thr His Ser Glu Tyr Tyr Asn His Ala Ile Asp Trp Gln
   1               5                   10                  15
   Thr Gly Pro Gly Cys
               20
<210> 4711
   <211> 19
   <212> PRT
   <213> Mus musculus
<400> 4711
   Asn Thr Thr Asp Met Met Ile Phe Asp Leu Ile His Asn Tyr Asn Arg
   1               5                   10                  15
   Phe Pro Asp
<210> 4712
   <211> 16
   <212> PRT
   <213> Homo sapiens
<400> 4712
   Tyr Ala Phe Asn Gly Thr Lys Leu Asp Ala Val Tyr Leu Asn Lys Asn
   1 5 10 15
<210> 4713
   <211> 11
   <212> PRT
   <213> Homo sapiens
<400> 4713
   Asn Lys Pro Leu Ile Thr Val Thr Asn Ser Lys
   1 5 10
<210> 4714
   <211> 9
   <212> PRT
   <213> Rattus norvegicus
<400> 4714
   Pro Pro Gly Glu Gln Thr Met Gly Val
   1 5
<210> 4715
   <211> 20
   <212> PRT
   <213> Homo sapiens
<400> 4715
   Val Cys Thr Pro Lys Ser Asp Glu Phe Asn Pro Cys Glu Asp Ile Met
   1 5 10 15
   Gly Tyr Lys Phe
   20
<210> 4716
   <211> 50
   <212> PRT
   <213> Homo sapiens
<400> 4716
   Ala Thr Lys Lys Glu Glu Glu Lys Lys Gly Gly Asp Arg Asn Thr Gly
   1 5 10 15
   Leu Ser Arg Asp Lys Asp Lys Lys Arg Glu Glu Met Lys Glu Val Ala
   20 25 30
   Lys Lys Glu Asp Asp Glu Lys Val Lys Gly Glu Arg Arg Asn Thr Asp
   35 40 45
   Thr Arg
   50
<210> 4717
   <211> 39
   <212> PRT
   <213> Homo sapiens
<400> 4717
   Leu Leu Lys Glu Asn Thr Ser Leu Leu Lys Leu Gly Tyr His Phe Glu
   1 5 10 15
   Leu Ala Gly Pro Arg Met Thr Val Thr Asn Leu Leu Ser Arg Asn Met
   20 25 30
   Asp Lys Gln Arg Gln Lys Arg
   35
<210> 4718
   <211> 37
   <212> PRT
   <213> Homo sapiens
<400> 4718
   Arg Gly Arg Asp Ala Ser Lys Lys Ala Leu Gly Pro Arg Arg Asn Ser
   1 5 10 15
   Asp Leu Gly Lys Glu Pro Lys Arg Gly Gly Leu Lys Lys Ser Phe Ser
   20 25 30
   Arg Asp Arg Asp Glu
   35
<210> 4719
   <211> 27
   <212> PRT
   <213> Homo sapiens
<400> 4719
   Ala Asp Leu Ser Thr Ala Ile Ala Ser Arg Ser Val Ala Asp Lys Ile
   1 5 10 15
   Leu Asp Leu Tyr Lys His Pro Asp Asn Ile Asp
   20 25
<210> 4720
   <211> 20
   <212> PRT
   <213> Homo sapiens
<400> 4720
   Ala Phe Gln Val Gly Lys Phe Pro Glu Asp Phe Glu Ser Cys Asp Ser
   1 5 10 15
   Ile Pro Gly Met
   20
<210> 4721
   <211> 20
   <212> PRT
   <213> Homo sapiens
<400> 4721
   Ala His Pro Pro Cys His Ala Ser Ala Arg Cys Arg Asn Thr Lys Gly
   1 5 10 15
   Gly Phe Gln Cys
   20
<210> 4722
   <211> 20
   <212> PRT
   <213> Homo sapiens
<400> 4722
   Ala Leu Lys Ala Leu Asn Ala His Trp Ser Ala Asp Ala Val Tyr Gln
   1 5 10 15
   Glu Ala Arg Lys
   20
<210> 4723
   <211> 20
   <212> PRT
   <213> Homo sapiens
<400> 4723
   Ala Pro Glu Pro Gly Ile Pro Gly Glu Thr Arg Gly Pro Cys Phe Leu
   1 5 10 15
   Ala Gly Asp Gly
   20
<210> 4724
   <211> 20
   <212> PRT
   <213> Homo sapiens
<400> 4724
   Asp Pro Leu Ile Arg Gly Leu Leu Ala Arg Pro Ala Lys Leu Gln Val
   1 5 10 15
   Gln Asp Gln Leu
   20
<210> 4725
   <211> 20
   <212> PRT
   <213> Homo sapiens
<400> 4725
   Glu Glu Ser Gly Arg Arg Val Leu Val Tyr Ser Cys Arg His Gly Tyr
   1 5 10 15
   Glu Leu Gln Gly
   20
<210> 4726
   <211> 20
   <212> PRT
   <213> Homo sapiens
<400> 4726
   Glu Gly Trp Asp Phe Gln Pro Pro Leu Cys Lys Asp Val Asn Glu Cys
   1 5 10 15
   Ala Asp Gly Ala
   20
<210> 4727
   <211> 20
   <212> PRT
   <213> Homo sapiens
<400> 4727
   Glu Ser Arg Val Ser Ser Val Leu Glu Glu Ser Lys Arg Leu Val Asp
   1 5 10 15
   Thr Ala Met Tyr
   20
<210> 4728
   <211> 20
   <212> PRT
   <213> Homo sapiens
<400> 4728
   Phe Ser Lys Leu Pro Glu Pro Thr Ser Gly Val Ile Ala Arg Ala Ala
   1               5                   10                  15
   Glu Ile Met Glu
               20
<210> 4729
   <211> 21
   <212> PRT
   <213> Homo sapiens
<400> 4729
   Gly Gly Ala Asp Cys Gln Met Thr Cys Glu Asn Gln Asn Pro Cys Phe
   1               5                   10                  15
   Pro Ile Gln Leu Pro
               20
<210> 4730
   <211> 20
   <212> PRT
   <213> Homo sapiens
<400> 4730
   Gly Leu Trp Leu His Gln Ala Phe Phe Ser Pro Trp Thr Leu Leu Arg
   1               5                   10                  15
   Gly Gly Gly Leu
               20
<210> 4731
   <211> 21
   <212> PRT
   <213> Homo sapiens
<400> 4731
   Gly Met Asn Leu Glu Ala Trp Arg Glu Thr Phe Pro Asp Gln Asp Asp
   1               5                   10                  15
   Lys Cys Gly Phe Pro
               20
<210> 4732
   <211> 20
   <212> PRT
   <213> Homo sapiens
<400> 4732
   Gly Arg Leu Pro Arg Ala Thr Trp Ile Ser Met Ser Leu Ala Ala Leu
   1 5 10 15
   Leu Ile Gly Gly
   20
<210> 4733
   <211> 20
   <212> PRT
   <213> Homo sapiens
<400> 4733
   His Ser Leu Ser Arg Val Ile Cys Asp Asn Thr Gly Leu Thr Arg Val
   1 5 10 15
   Pro Met Asp Ala
   20
<210> 4734
   <211> 20
   <212> PRT
   <213> Homo sapiens
<400> 4734
   His Val Ile Gln Val Ser Asn Glu Val Val Thr Asp Asp Asp Arg Tyr
   1 5 10 15
   Ser Asp Leu Leu
   20
<210> 4735
   <211> 20
   <212> PRT
   <213> Homo sapiens
<400> 4735
   Lys Cys Pro Asn Thr Cys Leu Ala Asn Lys Tyr Arg Pro Ile Thr Gly
   1 5 10 15
   Ala Cys Asn Asn
   20
<210> 4736
   <211> 20
   <212> PRT
   <213> Homo sapiens
<400> 4736
   Leu Pro Ile Ser Glu Thr Gly Gly Gly Thr Pro Glu Leu Arg Cys Gly
   1               5                   10                  15
   Lys His Gln Ala
               20
<210> 4737
   <211> 20
   <212> PRT
   <213> Homo sapiens
<400> 4737
   Met Glu Thr Ser Ile Gln Ala Met Lys Arg Lys Val Asn Leu Lys Thr
   1               5                   10                  15
   Gln Gln Ser Gln
               20
<210> 4738
   <211> 20
   <212> PRT
   <213> Homo sapiens
<400> 4738
   Asn Glu Trp Arg Glu Phe Cys Gly Leu Pro Arg Leu Glu Thr Pro Ala
   1               5                   10                  15
   Asp Leu Ser Thr
               20
<210> 4739
   <211> 22
   <212> PRT
   <213> Homo sapiens
<400> 4739
   Asn Glu Trp Arg Glu Phe Cys Gly Leu Pro Arg Leu Glu Thr Pro Ala
   1               5                   10                  15
   Asp Leu Ser Thr Ala Ile
               20
<210> 4740
   <211> 12
   <212> PRT
   <213> Homo sapiens
<400> 4740
   Asn Ile Phe Glu Tyr Gln Val Asp Ala Gln Pro Leu
   1               5                   10
<210> 4741
   <211> 20
   <212> PRT
   <213> Homo sapiens
<400> 4741
   Pro Cys Phe Leu Ala Gly Asp Gly Arg Ala Ser Glu Val Pro Ser Leu
   1               5                   10                  15
   Thr Ala Leu His
                                       20
<210> 4742
   <211> 25
   <212> PRT
   <213> Homo sapiens
<400> 4742
   Pro Gly Tyr Asn Glu Trp Arg Glu Phe Cys Gly Leu Pro Arg Leu Glu
   1               5                   10                  15
   Thr Pro Ala Asp Leu Ser Thr Ala Ile
               20                  25
<210> 4743
   <211> 45
   <212> PRT
   <213> Homo sapiens
<400> 4743
   Pro Gly Tyr Asn Glu Trp Arg Glu Phe Cys Gly Leu Pro Arg Leu Glu
   1               5                   10                  15
   Thr Pro Ala Asp Leu Ser Thr Ala Ile Ala Ser Arg Ser Val Ala Asp
               20                  25                  30
   Lys Ile Leu Asp Leu Tyr Lys His Pro Asp Asn Ile Asp
           35                  40                  45
<210> 4744
   <211> 18
   <212> PRT
   <213> Homo sapiens
<400> 4744
   Gln Glu Ser Ala Gly Met Glu Gly Arg Asp Thr His Arg Leu Pro Arg
   1               5                   10                  15
   Ala Leu
<210> 4745
   <211> 20
   <212> PRT
   <213> Homo sapiens
<400> 4745
   Gln Gln Ser Gln His Pro Thr Asp Ala Leu Ser Glu Asp Leu Leu Ser
   1               5                   10                  15
   Ile Ile Ala Asn
               20
<210> 4746
   <211> 20
   <212> PRT
   <213> Homo sapiens
<400> 4746
   Gln Arg Asn Leu Lys Lys Arg Gly Ile Leu Ser Pro Ala Gln Leu Leu
   1               5                   10                  15
   Ser Phe Ser Lys
               20
<210> 4747
   <211> 20
   <212> PRT
   <213> Homo sapiens
<400> 4747
   Arg Pro Ile Thr Gly Ala Cys Asn Asn Arg Asp His Pro Arg Trp Gly
   1               5                   10                  15
   Ala Ser Asn Thr
               20
<210> 4748
   <211> 20
   <212> PRT
   <213> Homo sapiens
<400> 4748
   Thr Glu Ala Phe Phe Pro Phe Ile Ser Arg Gly Lys Glu Leu Leu Trp
   1               5                   10                  15
   Gly Lys Pro Glu
               20
<210> 4749
   <211> 20
   <212> PRT
   <213> Homo sapiens
<400> 4749
   Val Gly Pro Tyr Glu Gly Tyr Asp Ser Thr Ala Asn Pro Thr Val Ser
   1               5                   10                  15
   Asn Val Phe Ser
               20
<210> 4750
   <211> 20
   <212> PRT
   <213> Homo sapiens
<400> 4750
   Tyr Glu Asp Gly Phe Ser Gln Pro Arg Gly Trp Asn Pro Gly Phe Leu
   1               5                   10                  15
   Tyr Asn Gly Phe
               20
<210> 4751
   <211> 20
   <212> PRT
   <213> Homo sapiens
<400> 4751
   Tyr Glu Leu Gln Gly Arg Glu Gln Leu Thr Cys Thr Gln Glu Gly Trp
   1               5                   10                  15
   Asp Phe Gln Pro
               20
<210> 4752
   <211> 15
   <212> PRT
   <213> Homo sapiens
<400> 4752
   Ala Leu Lys Ala Leu Asn Ala His Trp Ser Ala Asp Ala Val Tyr
   1               5                   10                  15
<210> 4753
   <211> 20
   <212> PRT
   <213> Homo sapiens
<400> 4753
   Ala Met Lys Arg Lys Val Asn Leu Lys Thr Gln Gln Ser Gln His Pro
   1               5                   10                  15
   Thr Asp Ala Leu
               20
<210> 4754
   <211> 18
   <212> PRT
   <213> Homo sapiens
<400> 4754
   Gly Leu Pro Arg Leu Glu Thr Pro Ala Asp Leu Ser Thr Ala Ile Ala
   1               5                   10                  15
   Ser Arg
<210> 4755
   <211> 13
   <212> PRT
   <213> Homo sapiens
<400> 4755
   His Pro Thr Asp Ala Leu Ser Glu Asp Leu Leu Ser Ile
   1               5                   10
<210> 4756
   <211> 13
   <212> PRT
   <213> Homo sapiens
<400> 4756
   His Val Ile Gln Val Ser Asn Glu Val Val Thr Asp Asp
   1               5                   10
<210> 4757
   <211> 15
   <212> PRT
   <213> Homo sapiens
<400> 4757
   Ile Ala Ser Arg Ser Val Ala Asp Lys Ile Leu Asp Leu Tyr Lys
   1               5                   10                  15
<210> 4758
   <211> 20
   <212> PRT
   <213> Homo sapiens
<400> 4758
   Leu Tyr Lys His Pro Asp Asn Ile Asp Val Trp Leu Gly Gly Leu Ala
   1 5 10 15
   Glu Asn Phe Leu
   20
<210> 4759
   <211> 15
   <212> PRT
   <213> Homo sapiens
<400> 4759
   Met Thr Cys Glu Asn Gln Asn Pro Cys Phe Pro Ile Gln Leu Pro
   1 5 10 15
<210> 4760
   <211> 18
   <212> PRT
   <213> Homo sapiens
<400> 4760
   Val Gly Ala Leu His Gln Ile Ile Thr Leu Arg Asp Tyr Ile Pro Arg
   1 5 10 15
   Ile Leu
<210> 4761
   <211> 10
   <212> PRT
   <213> Homo sapiens
<400> 4761
   Leu Val Val Tyr Pro Trp Thr Gln Arg Phe
   1 5 10
<210> 4762
   <211> 15
   <212> PRT
   <213> Homo sapiens
<400> 4762
   Ala Ala Gly Leu Leu Ser Thr Tyr Arg Ala Phe Leu Ser Ser His
   1 5 10 15
<210> 4763
   <211> 27
   <212> PRT
   <213> Homo sapiens
<400> 4763
   Ala Ala His Leu Pro Ala Glu Phe Thr Pro Ala Val His Ala Ser Leu
   1               5                   10                  15
   Asp Lys Phe Leu Ala Ser Val Ser Thr Val Leu
               20                  25
<210> 4764
   <211> 32
   <212> PRT
   <213> Homo sapiens
<400> 4764
   Ala Ala His Leu Pro Ala Glu Phe Thr Pro Ala Val His Ala Ser Leu
   1               5                   10                  15
   Asp Lys Phe Leu Ala Ser Val Ser Thr Val Leu Thr Ser Lys Tyr Arg
               20                  25                  30
<210> 4765
   <211> 21
   <212> PRT
   <213> Homo sapiens
<400> 4765
   Ala Ala Trp Gly Lys Val Gly Ala His Ala Gly Glu Tyr Gly Ala Glu
   1               5                   10                  15
   Ala Leu Glu Arg Met
               20
<210> 4766
   <211> 29
   <212> PRT
   <213> Homo sapiens
<400> 4766
   Ala Ala Trp Gly Lys Val Gly Ala His Ala Gly Glu Tyr Gly Ala Glu
   1               5                   10                  15
   Ala Leu Glu Arg Met Phe Leu Ser Phe Pro Thr Thr Lys
               20                  25
<210> 4767
   <211> 19
   <212> PRT
   <213> Homo sapiens
<400> 4767
   Ala Phe Thr Pro Glu Gly Glu Arg Leu Ile Gly Asp Ala Ala Lys Asn
   1 5 10 15
   Gln Leu Thr
<210> 4768
   <211> 14
   <212> PRT
   <213> Homo sapiens
<400> 4768
   Ala Gly Leu Leu Ser Thr Tyr Arg Ala Phe Leu Ser Ser His
   1 5 10
<210> 4769
   <211> 15
   <212> PRT
   <213> Homo sapiens
<400> 4769
   Ala Gly Asn Leu Gly Gly Gly Val Val Thr Ile Glu Arg Ser Lys
   1 5 10 15
<210> 4770
   <211> 15
   <212> PRT
   <213> Homo sapiens
<400> 4770
   Ala His Leu Leu Ile Leu Arg Asp Thr Lys Thr Tyr Met Leu Ala
   1 5 10 15
<210> 4771
   <211> 16
   <212> PRT
   <213> Homo sapiens
<400> 4771
   Ala His Leu Leu Ile Leu Arg Asp Thr Lys Thr Tyr Met Leu Ala Phe
   1 5 10 15
<210> 4772
   <211> 13
   <212> PRT
   <213> Homo sapiens
<400> 4772
   Ala Lys Ala Val Lys Gln Phe Glu Glu Ser Gln Gly Arg
   1 5 10
<210> 4773
   <211> 17
   <212> PRT
   <213> Homo sapiens
<400> 4773
   Ala Lys Ala Val Lys Gln Phe Glu Glu Ser Gln Gly Arg Thr Ser Ser
   1 5 10 15
   Lys
<210> 4774
   <211> 12
   <212> PRT
   <213> Homo sapiens
<400> 4774
   Ala Lys Phe Glu Glu Leu Asn Met Asp Leu Phe Arg
   1 5 10
<210> 4775
   <211> 18
   <212> PRT
   <213> Homo sapiens
<400> 4775
   Ala Lys Val Ala Val Leu Gly Ala Ser Gly Gly Ile Gly Gln Pro Leu
   1 5 10 15
   Ser Leu
<210> 4776
   <211> 16
   <212> PRT
   <213> Homo sapiens
<400> 4776
   Ala Ser Gly Asn Tyr Ala Thr Val Ile Ser His Asn Pro Glu Thr Lys
   1 5 10 15
<210> 4777
   <211> 12
   <212> PRT
   <213> Homo sapiens
<400> 4777
   Ala Ser Val Ser Thr Val Leu Thr Ser Lys Tyr Arg
   1 5 10
<210> 4778
   <211> 16
   <212> PRT
   <213> Homo sapiens
<400> 4778
   Ala Val Pro Ser Pro Pro Pro Ala Ser Pro Arg Ser Gln Tyr Asn Phe
   1 5 10 15
<210> 4779
   <211> 19
   <212> PRT
   <213> Homo sapiens
<400> 4779
   Ala Val Pro Ser Pro Pro Pro Ala Ser Pro Arg Ser Gln Tyr Asn Phe
   1 5 10 15
   Ile Ala Asp
<210> 4780
   <211> 24
   <212> PRT
   <213> Homo sapiens
<400> 4780
   Ala Val Thr Glu Leu Gly Arg Pro Asp Ala Glu Tyr Trp Asn Ser Gln
   1 5 10 15
   Lys Asp Leu Leu Glu Gln Lys Arg
   20
<210> 4781
   <211> 17
   <212> PRT
   <213> Homo sapiens
<400> 4781
   Ala Tyr Val Arg Leu Ala Pro Asp Tyr Asp Ala Leu Asp Val Ala Asn
   1 5 10 15
   Lys
<210> 4782
   <211> 18
   <212> PRT
   <213> Homo sapiens
<400> 4782
   Asp Gly Arg Gly Ala Leu Gln Asn Ile Ile Pro Ala Ser Thr Gly Ala
   1 5 10 15
   Ala Lys
<210> 4783
   <211> 13
   <212> PRT
   <213> Homo sapiens
<400> 4783
   Asp Gly Val Ile Lys Val Phe Asn Asp Met Lys Val Arg
   1 5 10
<210> 4784
   <211> 15
   <212> PRT
   <213> Homo sapiens
<400> 4784
   Asp Ile Pro Glu Leu Val Asn Met Gly Gln Trp Lys Ile Arg Ala
   1 5 10 15
<210> 4785
   <211> 16
   <212> PRT
   <213> Homo sapiens
<400> 4785
   Asp Lys Phe Leu Ala Ser Val Ser Thr Val Leu Thr Ser Lys Tyr Arg
   1 5 10 15
<210> 4786
   <211> 18
   <212> PRT
   <213> Homo sapiens
<400> 4786
   Asp Pro Ser Pro Ser Pro Val Leu Gly Tyr Lys Ile Val Tyr Lys Pro
   1 5 10 15
   Val Gly
<210> 4787
   <211> 10
   <212> PRT
   <213> Homo sapiens
<400> 4787
   Asp Arg Val Tyr Ile His Pro Phe His Leu
   1 5 10
<210> 4788
   <211> 13
   <212> PRT
   <213> Homo sapiens
<400> 4788
   Asp Thr Asn Ala Asp Lys Gln Leu Ser Phe Glu Glu Phe
   1 5 10
<210> 4789
   <211> 16
   <212> PRT
   <213> Homo sapiens
<400> 4789
   Asp Val Pro Lys Trp Ile Ser Ile Met Thr Glu Arg Ser Val Pro His
   1 5 10 15
<210> 4790
   <211> 12
   <212> PRT
   <213> Homo sapiens
<400> 4790
   Glu Asp Phe Arg Asp Gly Leu Lys Leu Met Leu Leu
   1 5 10
<210> 4791
   <211> 14
   <212> PRT
   <213> Homo sapiens
<400> 4791
   Glu Asp Ile Val Ala Asp His Val Ala Ser Tyr Gly Val Asn
   1 5 10
<210> 4792
   <211> 15
   <212> PRT
   <213> Homo sapiens
<400> 4792
   Glu Gly Leu Thr Phe Gln Met Lys Lys Asn Ala Glu Glu Leu Lys
   1 5 10 15
<210> 4793
   <211> 11
   <212> PRT
   <213> Homo sapiens
<400> 4793
   Glu Ile Thr Ala Leu Ala Pro Ser Thr Met Lys
   1 5 10
<210> 4794
   <211> 15
   <212> PRT
   <213> Homo sapiens
<400> 4794
   Glu Gln Phe Tyr Val Asp Leu Asp Lys Lys Glu Thr Val Trp His
   1 5 10 15
<210> 4795
   <211> 14
   <212> PRT
   <213> Homo sapiens
<400> 4795
   Phe Asp Pro Ile Ile Glu Asp Arg His Gly Gly Tyr Lys Pro
   1 5 10
<210> 4796
   <211> 15
   <212> PRT
   <213> Homo sapiens
<400> 4796
   Phe Asp Pro Ile Ile Glu Asp Arg His Gly Gly Tyr Lys Pro Ser
   1 5 10 15
<210> 4797
   <211> 11
   <212> PRT
   <213> Homo sapiens
<400> 4797
   Phe Asp Ser Asp Val Gly Glu Tyr Arg Ala Val
   1 5 10
<210> 4798
   <211> 18
   <212> PRT
   <213> Homo sapiens
<400> 4798
   Phe Glu Ser Phe Gly Asp Leu Ser Thr Pro Asp Ala Val Met Gly Asn
   1 5 10 15
   Pro Lys
<210> 4799
   <211> 20
   <212> PRT
   <213> Homo sapiens
<400> 4799
   Phe Glu Ser Phe Gly Asp Leu Ser Thr Pro Asp Ala Val Met Gly Asn
   1 5 10 15
   Pro Lys Val Lys
   20
<210> 4800
   <211> 14
   <212> PRT
   <213> Homo sapiens
<400> 4800
   Phe Leu Ala Ser Val Ser Thr Val Leu Thr Ser Lys Tyr Arg
   1 5 10
<210> 4801
   <211> 10
   <212> PRT
   <213> Homo sapiens
<400> 4801
   Phe Leu Glu Asn Val Ile Arg Asp Ala Val
   1 5 10
<210> 4802
   <211> 24
   <212> PRT
   <213> Homo sapiens
<400> 4802
   Phe Leu Ser Phe Pro Thr Thr Lys Thr Tyr Phe Pro His Phe Asp Leu
   1 5 10 15
   Ser His Gly Ser Ala Gln Val Lys
   20
<210> 4803
   <211> 28
   <212> PRT
   <213> Homo sapiens
<400> 4803
   Phe Leu Ser Phe Pro Thr Thr Lys Thr Tyr Phe Pro His Phe Asp Leu
   1 5 10 15
   Ser His Gly Ser Ala Gln Val Lys Gly His Gly Lys
   20 25
<210> 4804
   <211> 29
   <212> PRT
   <213> Homo sapiens
<400> 4804
   Phe Leu Ser Phe Pro Thr Thr Lys Thr Tyr Phe Pro His Phe Asp Leu
   1 5 10 15
   Ser His Gly Ser Ala Gln Val Lys Gly His Gly Lys Lys
   20 25
<210> 4805
   <211> 15
   <212> PRT
   <213> Homo sapiens
<400> 4805
   Gly Ala Pro Ala Ala Ala Thr Ala Pro Ala Pro Thr Ala His Lys
   1 5 10 15
<210> 4806
   <211> 15
   <212> PRT
   <213> Homo sapiens
<400> 4806
   Gly Glu Leu Glu Lys Glu Glu Ala Gln Pro Ile Val Thr Lys Tyr
   1 5 10 15
<210> 4807
   <211> 15
   <212> PRT
   <213> Homo sapiens
<400> 4807
   Gly Gly Glu Leu Arg Phe Thr Val Thr Gln Arg Ser Gln Pro Gly
   1 5 10 15
<210> 4808
   <211> 15
   <212> PRT
   <213> Homo sapiens
<400> 4808
   Gly Lys Glu Tyr Phe Ala Ile Asp Asn Ser Gly Arg Ile Ile Thr
   1 5 10 15
<210> 4809
   <211> 12
   <212> PRT
   <213> Homo sapiens
<400> 4809
   Gly Leu Thr Phe Gln Met Lys Lys Asn Ala Glu Glu
   1 5 10
<210> 4810
   <211> 14
   <212> PRT
   <213> Homo sapiens
<400> 4810
   Gly Leu Thr Phe Gln Met Lys Lys Asn Ala Glu Glu Leu Lys
   1 5 10
<210> 4811
   <211> 15
   <212> PRT
   <213> Homo sapiens
<400> 4811
   Gly Asn Arg Ile Ala Gln Trp Gln Ser Phe Gln Leu Glu Gly Gly
   1 5 10 15
<210> 4812
   <211> 18
   <212> PRT
   <213> Homo sapiens
<400> 4812
   Gly Arg Met His Ala Pro Gly Lys Gly Leu Ser Gln Ser Ala Leu Pro
   1 5 10 15
   Tyr Arg
<210> 4813
   <211> 14
   <212> PRT
   <213> Homo sapiens
<400> 4813
   Gly Thr Leu Ile Ile Arg Asp Val Lys Glu Ser Asp Gln Gly
   1 5 10
<210> 4814
   <211> 17
   <212> PRT
   <213> Homo sapiens
<400> 4814
   Gly Thr Gln Gly Lys Ile Val Asp Leu Val Lys Glu Leu Asp Arg Asp
   1 5 10 15
   Thr
<210> 4815
   <211> 12
   <212> PRT
   <213> Homo sapiens
<400> 4815
   Ile Gly Ser Ser Gln Asp Asp Gly Leu Ile Asn Arg
   1 5 10
<210> 4816
   <211> 16
   <212> PRT
   <213> Homo sapiens
<400> 4816
   Ile Gly Ser Ser Tyr Phe Pro Glu His Gly Tyr Phe Arg Ala Pro Glu
   1 5 10 15
<210> 4817
   <211> 9
   <212> PRT
   <213> Homo sapiens
<400> 4817
   Ile Lys Glu Glu Ser Lys Thr Ala Val
   1 5
<210> 4818
   <211> 8
   <212> PRT
   <213> Homo sapiens
<400> 4818
   Ile Pro Asp Trp Phe Leu Asn Arg
   1 5
<210> 4819
   <211> 16
   <212> PRT
   <213> Homo sapiens
<400> 4819
   Ile Arg Ala Ser Tyr Ala Gln Gln Pro Ala Glu Ser Arg Val Ser Gly
   1 5 10 15
<210> 4820
   <211> 13
   <212> PRT
   <213> Homo sapiens
<400> 4820
   Ile Ser Trp Tyr Asp Asn Glu Phe Gly Tyr Ser Asn Arg
   1 5 10
<210> 4821
   <211> 18
   <212> PRT
   <213> Homo sapiens
<400> 4821
   Ile Val Asn Thr Asn Val Pro Arg Ala Ser Val Pro Asp Gly Phe Leu
   1 5 10 15
   Ser Glu
<210> 4822
   <211> 22
   <212> PRT
   <213> Homo sapiens
<400> 4822
   Ile Val Asn Thr Asn Val Pro Arg Ala Ser Val Pro Asp Gly Phe Leu
   1 5 10 15
   Ser Glu Leu Thr Gln Gln
   20
<210> 4823
   <211> 18
   <212> PRT
   <213> Homo sapiens
<400> 4823
   Ile Tyr Thr Arg Asn Thr Lys Gly Gly Asp Ala Pro Ala Ala Gly Glu
   1 5 10 15
   Asp Ala
<210> 4824
   <211> 13
   <212> PRT
   <213> Homo sapiens
<400> 4824
   Lys Ala Val Leu Thr Ile Asp Glu Lys Gly Thr Glu Ala
   1 5 10
<210> 4825
   <211> 15
   <212> PRT
   <213> Homo sapiens
<400> 4825
   Lys Ala Val Leu Thr Ile Asp Glu Lys Gly Thr Glu Ala Ala Gly
   1 5 10 15
<210> 4826
   <211> 14
   <212> PRT
   <213> Homo sapiens
<400> 4826
   Lys Glu Tyr Phe Ala Ile Asp Asn Ser Gly Arg Ile Ile Thr
   1 5 10
<210> 4827
   <211> 15
   <212> PRT
   <213> Homo sapiens
<400> 4827
   Lys Phe Leu Ala Ser Val Ser Thr Val Leu Thr Ser Lys Tyr Arg
   1 5 10 15
<210> 4828
   <211> 15
   <212> PRT
   <213> Homo sapiens
<400> 4828
   Lys Pro Gly Gln Phe Ile Arg Ser Val Asp Pro Asp Ser Pro Ala
   1 5 10 15
<210> 4829
   <211> 26
   <212> PRT
   <213> Homo sapiens
<400> 4829
   Lys Thr Asn Val Lys Ala Ala Trp Gly Lys Val Gly Ala His Ala Gly
   1 5 10 15
   Glu Tyr Gly Ala Glu Ala Leu Glu Arg Met
   20 25
<210> 4830
   <211> 21
   <212> PRT
   <213> Homo sapiens
<400> 4830
   Leu Ala Gln Ala Thr Gly Lys Pro Pro Gln Tyr Ile Ala Val His Val
   1 5 10 15
   Val Pro Asp Gln Leu
   20
<210> 4831
   <211> 14
   <212> PRT
   <213> Homo sapiens
<400> 4831
   Leu Ile Ser Trp Tyr Asp Asn Glu Phe Gly Tyr Ser Asn Arg
   1 5 10
<210> 4832
   <211> 12
   <212> PRT
   <213> Homo sapiens
<400> 4832
   Leu Ile Thr Pro Ala Val Val Ser Glu Arg Leu Lys
   1 5 10
<210> 4833
   <211> 13
   <212> PRT
   <213> Homo sapiens
<400> 4833
   Leu Lys Lys Tyr Leu Tyr Glu Ile Ala Arg Arg His Pro
   1 5 10
<210> 4834
   <211> 19
   <212> PRT
   <213> Homo sapiens
<400> 4834
   Leu Leu Thr Glu Ala Pro Leu Asn Pro Lys Ala Asn Arg Glu Lys Met
   1 5 10 15
   Thr Gln Ile
<210> 4835
   <211> 10
   <212> PRT
   <213> Homo sapiens
<400> 4835
   Leu Leu Val Val Tyr Pro Trp Thr Gln Arg
   1 5 10
<210> 4836
   <211> 11
   <212> PRT
   <213> Homo sapiens
<400> 4836
   Leu Leu Val Val Tyr Pro Trp Thr Gln Arg Phe
   1 5 10
<210> 4837
   <211> 9
   <212> PRT
   <213> Homo sapiens
<400> 4837
   Leu Arg Val Asp Pro Val Asn Phe Lys
   1 5
<210> 4838
   <211> 10
   <212> PRT
   <213> Homo sapiens
<400> 4838
   Leu Ser Leu Gln Glu Pro Gly Ser Lys Thr
   1 5 10
<210> 4839
   <211> 22
   <212> PRT
   <213> Homo sapiens
<400> 4839
   Leu Val Thr Leu Ala Ala His Leu Pro Ala Glu Phe Thr Pro Ala Val
   1 5 10 15
   His Ala Ser Leu Asp Lys
   20
<210> 4840
   <211> 16
   <212> PRT
   <213> Homo sapiens
<400> 4840
   Asn Ile Phe Ser Phe Tyr Leu Ser Arg Asp Pro Asp Ala Gln Pro Gly
   1 5 10 15
<210> 4841
   <211> 21
   <212> PRT
   <213> Homo sapiens
<400> 4841
   Pro Met Phe Ile Val Asn Thr Asn Val Pro Arg Ala Ser Val Pro Asp
   1 5 10 15
   Gly Phe Leu Ser Glu
   20
<210> 4842
   <211> 15
   <212> PRT
   <213> Homo sapiens
<400> 4842
   Gln Leu Gln Pro Asp Asn Ser Thr Leu Thr Trp Val Lys Pro Thr
   1 5 10 15
<210> 4843
   <211> 18
   <212> PRT
   <213> Homo sapiens
<400> 4843
   Gln Val Asp Gln Phe Leu Gly Val Pro Tyr Ala Ala Pro Pro Leu Ala
   1 5 10 15
   Glu Arg
<210> 4844
   <211> 18
   <212> PRT
   <213> Homo sapiens
<400> 4844
   Gln Val Thr Gln Pro Thr Val Gly Met Asn Phe Lys Thr Pro Arg Gly
   1 5 10 15
   Pro Val
<210> 4845
   <211> 13
   <212> PRT
   <213> Homo sapiens
<400> 4845
   Arg Glu Ile Phe Asn Met Ala Arg Asp Gln Arg Ser Arg
   1               5                   10
<210> 4846
   <211> 12
   <212> PRT
   <213> Homo sapiens
<400> 4846
   Arg Phe Asp Ser Asp Val Gly Glu Phe Arg Ala Val
   1               5                   10
<210> 4847
   <211> 14
   <212> PRT
   <213> Homo sapiens
<400> 4847
   Arg Gly Asn Ser Ile Ile Met Leu Glu Ala Leu Glu Arg Val
   1               5                   10
<210> 4848
   <211> 13
   <212> PRT
   <213> Homo sapiens
<400> 4848
   Arg Leu Leu Leu Pro Gly Glu Leu Ala Lys His Ala Val
   1               5                   10
<210> 4849
   <211> 18
   <212> PRT
   <213> Homo sapiens
<400> 4849
   Arg Leu Leu Leu Pro Gly Glu Leu Ala Lys His Ala Val Ser Glu Gly
   1               5                   10                  15
   Thr Lys
<210> 4850
   <211> 16
   <212> PRT
   <213> Homo sapiens
<400> 4850
   Arg Leu Pro Ile Ile Asp Val Ala Pro Leu Asp Val Gly Ala Pro Asp
   1               5                   10                  15
<210> 4851
   <211> 16
   <212> PRT
   <213> Homo sapiens
<400> 4851
   Arg Pro Lys Asp Tyr Glu Val Asp Ala Thr Leu Lys Ser Leu Asn Asn
   1 5 10 15
<210> 4852
   <211> 16
   <212> PRT
   <213> Homo sapiens
<400> 4852
   Arg Val Met Thr Ile Ala Pro Gly Leu Phe Gly Thr Pro Leu Leu Thr
   1 5 10 15
<210> 4853
   <211> 13
   <212> PRT
   <213> Homo sapiens
<400> 4853
   Arg Tyr Ser Val Asp Ile Pro Leu Asp Lys Thr Val Val
   1 5 10
<210> 4854
   <211> 21
   <212> PRT
   <213> Homo sapiens
<400> 4854
   Ser Glu Thr Ala Pro Ala Ala Pro Ala Ala Ala Pro Pro Ala Glu Lys
   1 5 10 15
   Ala Pro Val Lys Lys
   20
<210> 4855
   <211> 22
   <212> PRT
   <213> Homo sapiens
<400> 4855
   Ser Glu Thr Ala Pro Ala Ala Pro Ala Ala Ala Pro Pro Ala Glu Lys
   1 5 10 15
   Ala Pro Val Lys Lys Lys
   20
<210> 4856
   <211> 33
   <212> PRT
   <213> Homo sapiens
<400> 4856
   Ser Gly Asn Phe Gly Gly Ser Arg Asn Met Gly Gly Pro Tyr Gly Gly
   1 5 10 15
   Gly Asn Tyr Gly Pro Gly Gly Ser Gly Gly Ser Gly Gly Tyr Gly Gly
   20 25 30
   Arg
<210> 4857
   <211> 14
   <212> PRT
   <213> Homo sapiens
<400> 4857
   Ser Arg Ala Gly Leu Glu Leu Gly Ala Glu Pro Glu Thr Ile
   1 5 10
<210> 4858
   <211> 11
   <212> PRT
   <213> Homo sapiens
<400> 4858
   Ser Arg Thr Ser Gly Leu Leu Ser Ser Trp Lys
   1 5 10
<210> 4859
   <211> 14
   <212> PRT
   <213> Homo sapiens
<400> 4859
   Ser Val Pro Arg Tyr Leu Pro Arg Pro Ala Asn Pro Asp Glu
   1 5 10
<210> 4860
   <211> 16
   <212> PRT
   <213> Homo sapiens
<400> 4860
   Ser Trp Asn Ser Gly Ala Leu Thr Ser Gly Val His Thr Phe Pro Ala
   1 5 10 15
<210> 4861
   <211> 14
   <212> PRT
   <213> Homo sapiens
<400> 4861
   Ser Trp Tyr Asp Asn Glu Phe Gly Tyr Ser Asn Arg Val Val
   1               5                   10
<210> 4862
   <211> 18
   <212> PRT
   <213> Homo sapiens
<400> 4862
   Ser Tyr Glu Leu Pro Asp Gly Gln Val Ile Thr Ile Gly Asn Glu Arg
   1               5                   10                  15
   Phe Arg
<210> 4863
   <211> 16
   <212> PRT
   <213> Homo sapiens
<400> 4863
   Thr Gly Ala Ala Pro Ile Ile Asp Val Val Arg Ser Gly Tyr Tyr Lys
   1               5                   10                  15
<210> 4864
   <211> 23
   <212> PRT
   <213> Homo sapiens
<400> 4864
   Thr Gly Gly Ala Ser Gly Leu Gly Leu Ala Thr Ala Glu Arg Leu Val
   1               5                   10                  15
   Gly Gln Gly Ala Ser Ala Val
               20
<210> 4865
   <211> 13
   <212> PRT
   <213> Homo sapiens
<400> 4865
   Thr Gly Lys Asn Lys Trp Phe Phe Gln Lys Leu Arg Phe
   1               5                   10
<210> 4866
   <211> 15
   <212> PRT
   <213> Homo sapiens
<400> 4866
   Thr Asn Val Pro Pro Glu Val Thr Val Leu Thr Asn Ser Pro Val
   1 5 10 15
<210> 4867
   <211> 14
   <212> PRT
   <213> Homo sapiens
<400> 4867
   Thr Gln Gly Lys Ile Val Asp Leu Val Lys Glu Leu Asp Arg
   1 5 10
<210> 4868
   <211> 16
   <212> PRT
   <213> Homo sapiens
<400> 4868
   Thr Gln Gly Lys Ile Val Asp Leu Val Lys Glu Leu Asp Arg Asp Thr
   1 5 10 15
<210> 4869
   <211> 11
   <212> PRT
   <213> Homo sapiens
<400> 4869
   Thr Val Asp Gly Pro Ser Gly Lys Leu Trp Arg
   1 5 10
<210> 4870
   <211> 17
   <212> PRT
   <213> Homo sapiens
<400> 4870
   Thr Trp Leu Arg Asn Gly Lys Pro Val Thr Thr Gly Val Ser Glu Thr
   1 5 10 15
   Val
<210> 4871
   <211> 16
   <212> PRT
   <213> Homo sapiens
<400> 4871
   Thr Tyr Phe Pro His Phe Asp Leu Ser His Gly Ser Ala Gln Val Lys
   1 5 10 15
<210> 4872
   <211> 21
   <212> PRT
   <213> Homo sapiens
<400> 4872
   Thr Tyr Phe Pro His Phe Asp Leu Ser His Gly Ser Ala Gln Val Lys
   1 5 10 15
   Gly His Gly Lys Lys
   20
<210> 4873
   <211> 29
   <212> PRT
   <213> Homo sapiens
<400> 4873
   Val Ala Asp Ala Leu Thr Asn Ala Val Ala His Val Asp Asp Met Pro
   1 5 10 15
   Asn Ala Leu Ser Ala Leu Ser Asp Leu His Ala His Lys
   20 25
<210> 4874
   <211> 15
   <212> PRT
   <213> Homo sapiens
<400> 4874
   Val Gly Ala His Ala Gly Glu Tyr Gly Ala Glu Ala Leu Glu Arg
   1 5 10 15
<210> 4875
   <211> 24
   <212> PRT
   <213> Homo sapiens
<400> 4875
   Val Gly Ala His Ala Gly Glu Tyr Gly Ala Glu Ala Leu Glu Arg Met
   1 5 10 15
   Phe Leu Ser Phe Pro Thr Thr Lys
   20
<210> 4876
   <211> 17
   <212> PRT
   <213> Homo sapiens
<400> 4876
   Val His Lys Ala Val Leu Thr Ile Asp Glu Lys Gly Thr Glu Ala Ala
   1               5                   10                  15
   Gly
<210> 4877
   <211> 8
   <212> PRT
   <213> Homo sapiens
<400> 4877
   Val His Leu Thr Pro Glu Glu Lys
   1               5
<210> 4878
   <211> 32
   <212> PRT
   <213> Homo sapiens
<400> 4878
   Val His Leu Thr Pro Glu Glu Lys Ser Ala Val Thr Ala Leu Trp Gly
   1               5                   10                  15
   Lys Val Asn Val Asp Glu Val Gly Gly Glu Ala Leu Gly Arg Leu Leu
               20                  25                  30
<210> 4879
   <211> 16
   <212> PRT
   <213> Homo sapiens
<400> 4879
   Val Leu Ser Pro Ala Asp Lys Thr Asn Val Lys Ala Ala Trp Gly Lys
   1               5                   10                  15
<210> 4880
   <211> 32
   <212> PRT
   <213> Homo sapiens
<400> 4880
   Val Leu Ser Pro Ala Asp Lys Thr Asn Val Lys Ala Ala Trp Gly Lys
   1               5                   10                  15
   Val Gly Ala His Ala Gly Glu Tyr Gly Ala Glu Ala Leu Glu Arg Met
               20                  25                  30
<210> 4881
   <211> 33
   <212> PRT
   <213> Homo sapiens
<400> 4881
   Val Leu Ser Pro Ala Asp Lys Thr Asn Val Lys Ala Ala Trp Gly Lys
   1               5                   10                  15
   Val Gly Ala His Ala Gly Glu Tyr Gly Ala Glu Ala Leu Glu Arg Met
               20                  25                  30
   Phe
<210> 4882
   <211> 12
   <212> PRT
   <213> Homo sapiens
<400> 4882
   Val Val Asp Leu Met Ala His Met Ala Ser Lys Glu
   1               5                   10
<210> 4883
   <211> 11
   <212> PRT
   <213> Homo sapiens
<400> 4883
   Val Trp Arg Leu Glu Glu Phe Gly Arg Phe Ala
   1               5                   10
<210> 4884
   <211> 16
   <212> PRT
   <213> Homo sapiens
<400> 4884
   Trp Leu Arg Asn Gly Lys Pro Val Thr Thr Gly Val Ser Glu Thr Val
   1               5                   10                  15
<210> 4885
   <211> 10
   <212> PRT
   <213> Homo sapiens
<400> 4885
   Trp Arg Leu Glu Glu Phe Gly Arg Phe Ala
   1 5 10
<210> 4886
   <211> 14
   <212> PRT
   <213> Homo sapiens
<400> 4886
   Tyr Asp His Asn Phe Val Lys Ala Ile Asn Ala Ile Gln Lys
   1 5 10
<210> 4887
   <211> 16
   <212> PRT
   <213> Homo sapiens
<400> 4887
   Tyr Gly Gly Glu Leu Arg Phe Thr Val Thr Gln Arg Ser Gln Pro Gly
   1 5 10 15
<210> 4888
   <211> 16
   <212> PRT
   <213> Homo sapiens
<400> 4888
   Tyr Pro Lys Ser Leu His Met Tyr Ala Asn Arg Leu Leu Asp His Arg
   1 5 10 15
<210> 4889
   <211> 12
   <212> PRT
   <213> Homo sapiens
<400> 4889
   Tyr Tyr Lys Val Asp Glu Asn Gly Lys Ile Ser Arg
   1 5 10
<210> 4890
   <211> 23
   <212> PRT
   <213> Homo sapiens
<400> 4890
   Ala Val Thr Glu Leu Gly Arg Pro Asp Ala Glu Tyr Trp Asn Ser Gln
   1 5 10 15
   Lys Asp Ile Leu Glu Gln Lys
   20
<210> 4891
   <211> 18
   <212> PRT
   <213> Homo sapiens
<400> 4891
   Cys Pro Thr Pro Cys Gln Leu Gln Ala Glu Gln Ala Phe Leu Arg Thr
   1 5 10 15
   Val Gln
<210> 4892
   <211> 17
   <212> PRT
   <213> Homo sapiens
<400> 4892
   Phe Ile Val Asn Thr Asn Val Pro Arg Ala Ser Val Pro Asp Gly Phe
   1 5 10 15
   Leu
<210> 4893
   <211> 11
   <212> PRT
   <213> Homo sapiens
<400> 4893
   Leu Leu Ile Pro Thr Ser Phe Ser Pro Leu Lys
   1 5 10
<210> 4894
   <211> 20
   <212> PRT
   <213> Homo sapiens
<400> 4894
   Pro Ala Ala Pro Ala Ala Pro Ala Pro Ala Glu Lys Thr Pro Val Lys
   1 5 10 15
   Lys Lys Ala Arg
   20
<210> 4895
   <211> 764
   <212> PRT
   <213> Homo sapiens
<400> 4895
   Met Arg Pro Ala Asp Leu Leu Gln Leu Val Leu Leu Leu Asp Leu Pro
   1               5                   10                  15
   Arg Asp Leu Gly Gly Met Gly Cys Ser Ser Pro Pro Cys Glu Cys His
   20 25 30
   Gln Glu Glu Asp Phe Arg Val Thr Cys Lys Asp Ile Gln Arg Ile Pro
   35 40 45
   Ser Leu Pro Pro Ser Thr Gln Thr Leu Lys Leu Ile Glu Thr His Leu
   50 55 60
   Arg Thr Ile Pro Ser His Ala Phe Ser Asn Leu Pro Asn Ile Ser Arg
   65 70 75 80
   Ile Tyr Val Ser Ile Asp Val Thr Leu Gln Gln Leu Glu Ser His Ser
   85 90 95
   Phe Tyr Asn Leu Ser Lys Val Thr His Ile Glu Ile Arg Asn Thr Arg
   100 105 110
   Asn Leu Thr Tyr Ile Asp Pro Asp Ala Leu Lys Glu Leu Pro Leu Leu
   115 120 125
   Lys Phe Leu Gly Ile Phe Asn Thr Gly Leu Lys Met Phe Pro Asp Leu
   130 135 140
   Thr Lys Val Tyr Ser Thr Asp Ile Phe Phe Ile Leu Glu Ile Thr Asp
   145 150 155 160
   Asn Pro Tyr Met Thr Ser Ile Pro Val Asn Ala Phe Gln Gly Leu Cys
   165 170 175
   Asn Glu Thr Leu Thr Leu Lys Leu Tyr Asn Asn Gly Phe Thr Ser Val
   180 185 190
   Gln Gly Tyr Ala Phe Asn Gly Thr Lys Leu Asp Ala Val Tyr Leu Asn
   195 200 205
   Lys Asn Lys Tyr Leu Thr Val Ile Asp Lys Asp Ala Phe Gly Gly Val
   210 215 220
   Tyr Ser Gly Pro Ser Leu Leu Asp Val Ser Gln Thr Ser Val Thr Ala
   225 230 235 240
   Leu Pro Ser Lys Gly Leu Glu His Leu Lys Glu Leu Ile Ala Arg Asn
   245 250 255
   Thr Trp Thr Leu Lys Lys Leu Pro Leu Ser Leu Ser Phe Leu His Leu
   260 265 270
   Thr Arg Ala Asp Leu Ser Tyr Pro Ser His Cys Cys Ala Phe Lys Asn
   275 280 285
   Gln Lys Lys Ile Arg Gly Ile Leu Glu Ser Leu Met Cys Asn Glu Ser
   290 295 300
   Ser Met Gln Ser Leu Arg Gln Arg Lys Ser Val Asn Ala Leu Asn Ser
   305 310 315 320
   Pro Leu His Gln Glu Tyr Glu Glu Asn Leu Gly Asp Ser Ile Val Gly
   325 330 335
   Tyr Lys Glu Lys Ser Lys Phe Gln Asp Thr His Asn Asn Ala His Tyr
   340 345 350
   Tyr Val Phe Phe Glu Glu Gln Glu Asp Glu Ile Ile Gly Phe Gly Gln
   355 360 365
   Glu Leu Lys Asn Pro Gln Glu Glu Thr Leu Gln Ala Phe Asp Ser His
   370 375 380
   Tyr Asp Tyr Thr Ile Cys Gly Asp Ser Glu Asp Met Val Cys Thr Pro
   385 390 395 400
   Lys Ser Asp Glu Phe Asn Pro Cys Glu Asp Ile Met Gly Tyr Lys Phe
   405 410 415
   Leu Arg Ile Val Val Trp Phe Val Ser Leu Leu Ala Leu Leu Gly Asn
   420 425 430
   Val Phe Val Leu Leu Ile Leu Leu Thr Ser His Tyr Lys Leu Asn Val
   435 440 445
   Pro Arg Phe Leu Met Cys Asn Leu Ala Phe Ala Asp Phe Cys Met Gly
   450 455 460
   Met Tyr Leu Leu Leu Ile Ala Ser Val Asp Leu Tyr Thr His Ser Glu
   465 470 475 480
   Tyr Tyr Asn His Ala Ile Asp Trp Gln Thr Gly Pro Gly Cys Asn Thr
   485 490 495
   Ala Gly Phe Phe Thr Val Phe Ala Ser Glu Leu Ser Val Tyr Thr Leu
   500 505 510
   Thr Val Ile Thr Leu Glu Arg Trp Tyr Ala Ile Thr Phe Ala Met Arg
   515 520 525
   Leu Asp Arg Lys Ile Arg Leu Arg His Ala Cys Ala Ile Met Val Gly
       530                 535                 540
   Gly Trp Val Cys Cys Phe Leu Leu Ala Leu Leu Pro Leu Val Gly Ile
   545                 550                 555                 560
   Ser Ser Tyr Ala Lys Val Ser Ile Cys Leu Pro Met Asp Thr Glu Thr
                   565                 570                 575
   Pro Leu Ala Leu Ala Tyr Ile Val Phe Val Leu Thr Leu Asn Ile Val
               580                 585                 590
   Ala Phe Val Ile Val Cys Cys Cys Tyr Val Lys Ile Tyr Ile Thr Val
           595                 600                 605
   Arg Asn Pro Gln Tyr Asn Pro Gly Asp Lys Asp Thr Lys Ile Ala Lys
       610                 615                 620
   Arg Met Ala Val Leu Ile Phe Thr Asp Phe Ile Cys Met Ala Pro Ile
   625                 630                 635                 640
   Ser Phe Tyr Ala Leu Ser Ala Ile Leu Asn Lys Pro Leu Ile Thr Val
                   645                 650                 655
   Ser Asn Ser Lys Ile Leu Leu Val Leu Phe Tyr Pro Leu Asn Ser Cys
               660                 665                 670
   Ala Asn Pro Phe Leu Tyr Ala Ile Phe Thr Lys Ala Phe Gln Arg Asp
           675                 680                 685
   Val Phe Ile Leu Leu Ser Lys Phe Gly Ile Cys Lys Arg Gln Ala Gln
       690                 695                 700
   Ala Tyr Arg Gly Gln Arg Val Pro Pro Lys Asn Ser Thr Asp Ile Gln
   705                 710                 715                 720
   Val Gln Lys Val Thr His Asp Met Arg Gln Gly Leu His Asn Met Glu
                   725                 730                 735
   Asp Val Tyr Glu Leu Ile Glu Asn Ser His Leu Thr Pro Lys Lys Gln
               740                 745                 750
   Gly Gln Ile Ser Glu Glu Tyr Met Gln Thr Val Leu
           755                 760
<210> 4896
   <211> 9
   <212> PRT
   <213> Homo sapiens
<400> 4896
   Ala Leu Ala Val Leu Ser Val Thr Leu
   1 5
<210> 4897
   <211> 9
   <212> PRT
   <213> Homo sapiens
<400> 4897
   Ala Leu Ser Glu Asp Leu Leu Ser Ile
   1 5
<210> 4898
   <211> 9
   <212> PRT
   <213> Homo sapiens
<400> 4898
   Gly Leu Leu Asp Gln Val Ala Ala Leu
   1 5
<210> 4899
   <211> 9
   <212> PRT
   <213> Homo sapiens
<400> 4899
   Gly Leu Arg Glu Asp Leu Leu Ser Leu
   1 5
<210> 4900
   <211> 9
   <212> PRT
   <213> Homo sapiens
<400> 4900
   Leu Leu Ile Gly Gly Phe Ala Gly Leu
   1 5
<210> 4901
   <211> 10
   <212> PRT
   <213> Homo sapiens
<400> 4901
   Ser Leu Gln Asp Val Pro Leu Ala Ala Leu
   1 5 10
<210> 4902
   <211> 9
   <212> PRT
   <213> Streptococcus pyogenes
<400> 4902
   Ala Leu Glu Lys Leu Asn Lys Glu Leu
   1               5
<210> 4903
   <211> 9
   <212> PRT
   <213> Homo sapiens
<400> 4903
   Glu Phe Gln Lys Met Arg Arg Asp Leu
   1               5
<210> 4904
   <211> 9
   <212> PRT
   <213> Streptococcus pyogenes
<400> 4904
   Lys Leu Asn Lys Glu Leu Glu Glu Ser
   1               5
<210> 4905
   <211> 9
   <212> PRT
   <213> Homo sapiens
<400> 4905
   Lys Met Arg Arg Asp Leu Glu Glu Ala
   1               5
<210> 4906
   <211> 43
   <212> PRT
   <213> Homo sapiens
<400> 4906
   Ala Glu Arg Ala Ala Leu Glu Glu Leu Val Lys Leu Gln Gly Glu Arg
   1               5                   10                  15
   Val Arg Gly Leu Lys Gln Gln Lys Ala Ser Ala Glu Leu Ile Glu Glu
               20                  25                  30
   Glu Val Ala Lys Leu Leu Lys Leu Lys Ala Gln
           35                  40
<210> 4907
   <211> 25
   <212> PRT
   <213> Homo sapiens
<400> 4907
   Val Ala Gly Arg Gly Arg Gly Arg Gly Arg Gly Arg Gly Arg Gly Arg
   1 5 10 15
   Gly Arg Gly Arg Gly Gly Pro Arg Arg
   20 25
<210> 4908
   <211> 10
   <212> PRT
   <213> Homo sapiens
<400> 4908
   Gly Ser Ala Ser Pro Met Glu Leu Leu Ser
   1 5 10
<210> 4909
   <211> 10
   <212> PRT
   <213> Homo sapiens
<400> 4909
   Ile Phe Met Ser Asn Ser Tyr Pro Arg Asp
   1 5 10
<210> 4910
   <211> 12
   <212> PRT
   <213> Homo sapiens
<400> 4910
   Arg Leu Asp Asn Arg Tyr Gln Pro Met Glu Pro Asn
   1 5 10
<210> 4911
   <211> 10
   <212> PRT
   <213> Homo sapiens
<400> 4911
   Ser Ala Arg Ile Pro Cys Phe Leu Ala Gly
   1 5 10
<210> 4912
   <211> 10
   <212> PRT
   <213> Homo sapiens
<400> 4912
   Trp Asp Gly Glu Arg Leu Tyr Gln Glu Ala
   1 5 10
<210> 4913
   <211> 10
   <212> PRT
   <213> Homo sapiens
<400> 4913
   Trp Thr Pro Gly Val Lys Arg Asn Gly Phe
   1 5 10
<210> 4914
   <211> 10
   <212> PRT
   <213> Homo sapiens
<400> 4914
   Tyr Arg Ser Tyr Asn Asp Ser Val Asp Pro
   1 5 10
<210> 4915
   <211> 7
   <212> PRT
   <213> Helicobacter pylori
<400> 4915
   Ala Lys Glu Glu Arg Arg Tyr
   1 5
<210> 4916
   <211> 7
   <212> PRT
   <213> Unknown
<220>
   <223> epitope involved in autoimmune pancreatitis
<400> 4916
   Arg Phe Glu Gln Pro Arg Val
   1 5
<210> 4917
   <211> 7
   <212> PRT
   <213> Unknown
<220>
   <223> epitope involved in autoimmune pancreatitis
<400> 4917
   Ser Lys Asp Glu Arg Arg Phe
   1 5
<210> 4918
   <211> 15
   <212> PRT
   <213> Mycobacterium avium
<400> 4918
   Thr Cys Arg Arg Met Leu Ala Phe Leu Lys Asp Lys Glu Asn Arg
   1 5 10 15
<210> 4919
   <211> 15
   <212> PRT
   <213> Homo sapiens
<400> 4919
   Gly Glu Ala Leu Ser Thr Leu Val Leu Asn Arg Leu Lys Val Gly
   1 5 10 15
<210> 4920
   <211> 15
   <212> PRT
   <213> Mycobacterium sp.
<400> 4920
   Gly Glu Ala Leu Ser Thr Leu Val Val Asn Lys Ile Arg Gly Thr
   1 5 10 15
<210> 4921
   <211> 15
   <212> PRT
   <213> Mycobacterium sp.
<400> 4921
   Ile Ala Gly Leu Phe Leu Thr Thr Glu Ala Val Val Ala Asp Lys
   1 5 10 15
<210> 4922
   <211> 15
   <212> PRT
   <213> Homo sapiens
<400> 4922
   Val Ala Ser Leu Leu Thr Thr Ala Glu Val Val Val Thr Glu Ile
   1 5 10 15
<210> 4923
   <211> 9
   <212> PRT
   <213> Unknown
<220>
   <223> epitope involved in Crohn's disease
<400> 4923
   Gly Gly Ile Tyr Gln Asp Leu Val Ser
   1 5
<210> 4924
   <211> 9
   <212> PRT
   <213> Unknown
<220>
   <223> epitope involved in Crohn's disease
<400> 4924
   Gly Leu Leu Ala Gln Gln Met Asp Tyr
   1 5
<210> 4925
   <211> 9
   <212> PRT
   <213> Unknown
<220>
   <223> epitope involved in Crohn's disease
<400> 4925
   Arg Leu Val Gly Gln Gln Val Met Gln
   1 5
<210> 4926
   <211> 9
   <212> PRT
   <213> Unknown
<220>
   <223> epitope involved in Crohn's disease
<400> 4926
   Arg Gln Ser Asp Gly Gln Tyr Gln Met
   1 5
<210> 4927
   <211> 9
   <212> PRT
   <213> Unknown
<220>
   <223> epitope involved in Crohn's disease
<400> 4927
   Tyr Arg Trp Leu Pro Pro Ser Ser Ala
   1               5
<210> 4928
   <211> 15
   <212> PRT
   <213> Mycobacterium avium
<400> 4928
   Ala Gly Asp Leu Ser Lys Val Asp Ala Lys Gln Pro Gly Asp Tyr
   1               5                   10                  15
<210> 4929
   <211> 15
   <212> PRT
   <213> Homo sapiens
<400> 4929
   Cys Gln Ala Leu Val Arg Met Leu Ala Lys Lys Pro Gly Trp Lys
   1               5                   10                  15
<210> 4930
   <211> 15
   <212> PRT
   <213> Homo sapiens
<400> 4930
   Asp Phe Thr Tyr Asp Gln Val Ala Phe Asn Gly Leu Pro Gln Phe
   1               5                   10                  15
<210> 4931
   <211> 15
   <212> PRT
   <213> Homo sapiens
<400> 4931
   Asp Lys Ile Arg Gly Glu Gly Met Arg Tyr Ile Ile Ile Leu Asp
   1               5                   10                  15
<210> 4932
   <211> 15
   <212> PRT
   <213> Mycobacterium avium
<400> 4932
   Glu Arg Pro Leu Lys Gln Ile Ala Phe Asn Gly Gly Leu Glu Pro
   1               5                   10                  15
<210> 4933
   <211> 15
   <212> PRT
   <213> Homo sapiens
<400> 4933
   Gly Arg Gly Asn Gly Asp Pro Gly Gly Gly Met Glu Lys Asp Gly
   1               5                   10                  15
<210> 4934
   <211> 15
   <212> PRT
   <213> Mycobacterium avium
<400> 4934
   Leu Ala Lys Leu Ala Gly Gly Val Ala Val Ile Cys Ala Gly Ala
   1               5                   10                  15
<210> 4935
   <211> 15
   <212> PRT
   <213> Homo sapiens
<400> 4935
   Leu Ala Lys Leu Gly Gly Ala Gly Gln Ala Ala Arg Ala Gly Thr
   1               5                   10                  15
<210> 4936
   <211> 14
   <212> PRT
   <213> Homo sapiens
<400> 4936
   Asn Glu His Pro Val Phe Ala Tyr Leu Lys Asp Lys Leu Pro
   1               5                   10
<210> 4937
   <211> 15
   <212> PRT
   <213> Mycobacterium avium
<400> 4937
   Gln Leu Glu Leu Thr Glu Gly Met Arg Phe Asp Lys Gly Tyr Ile
   1               5                   10                  15
<210> 4938
   <211> 11
   <212> PRT
   <213> Unknown
<220>
   <223> epitope involved in Crohn's disease
<400> 4938
   Cys Asn Leu Trp Glu Val Met His Gly Thr Cys
   1               5                   10
<210> 4939
   <211> 11
   <212> PRT
   <213> Unknown
<220>
   <223> epitope involved in Crohn's disease
<400> 4939
   Cys Ser Ile Gly Glu Ala Leu Ser His Gly Cys
   1 5 10
<210> 4940
   <211> 9
   <212> PRT
   <213> Unknown
<220>
   <223> epitope involved in Crohn's disease
<400> 4940
   Pro Trp Ala Thr Leu Val Ala Glu Ser
   1 5
<210> 4941
   <211> 9
   <212> PRT
   <213> Unknown
<220>
   <223> epitope involved in Crohn's disease
<400> 4941
   Thr Met Trp Asp Ala Leu Glu Ala Pro
   1 5
<210> 4942
   <211> 15
   <212> PRT
   <213> Mycobacterium avium
<400> 4942
   Thr Cys Arg Arg Met Leu Ala Phe Leu Lys Asp Lys Glu Asn Arg
   1 5 10 15
<210> 4943
   <211> 5
   <212> PRT
   <213> Homo sapiens
<400> 4943
   Pro Lys Lys Ala Lys
   1 5
<210> 4944
   <211> 15
   <212> PRT
   <213> Mycobacterium avium
<400> 4944
   Ala Gly Asp Leu Ser Lys Val Asp Ala Lys Gln Pro Gly Asp Tyr
   1 5 10 15
<210> 4945
   <211> 15
   <212> PRT
   <213> Homo sapiens
<400> 4945
   Cys Gln Ala Leu Val Arg Met Leu Ala Lys Lys Pro Gly Trp Lys
   1 5 10 15
<210> 4946
   <211> 15
   <212> PRT
   <213> Homo sapiens
<400> 4946
   Asp Phe Thr Tyr Asp Gln Val Ala Phe Asn Gly Leu Pro Gln Phe
   1 5 10 15
<210> 4947
   <211> 15
   <212> PRT
   <213> Homo sapiens
<400> 4947
   Asp Lys Ile Arg Gly Glu Gly Met Arg Tyr Ile Ile Ile Leu Asp
   1 5 10 15
<210> 4948
   <211> 15
   <212> PRT
   <213> Mycobacterium avium
<400> 4948
   Glu Arg Pro Leu Lys Gln Ile Ala Phe Asn Gly Gly Leu Glu Pro
   1 5 10 15
<210> 4949
   <211> 15
   <212> PRT
   <213> Homo sapiens
<400> 4949
   Gly Arg Gly Asn Gly Asp Pro Gly Gly Gly Met Glu Lys Asp Gly
   1 5 10 15
<210> 4950
   <211> 14
   <212> PRT
   <213> Homo sapiens
<400> 4950
   Asn Glu His Pro Val Phe Ala Tyr Leu Lys Asp Lys Leu Pro
   1 5 10
<210> 4951
   <211> 15
   <212> PRT
   <213> Mycobacterium avium
<400> 4951
   Gln Leu Glu Leu Thr Glu Gly Met Arg Phe Asp Lys Gly Tyr Ile
   1 5 10 15
<210> 4952
   <211> 9
   <212> PRT
   <213> Homo sapiens
<400> 4952
   Ala His Trp Ser Gly His Cys Cys Leu
   1 5
<210> 4953
   <211> 15
   <212> PRT
   <213> Mycobacterium avium
<400> 4953
   Thr Cys Arg Arg Met Leu Ala Phe Leu Lys Asp Lys Glu Asn Arg
   1 5 10 15
<210> 4954
   <211> 5
   <212> PRT
   <213> Homo sapiens
<400> 4954
   Pro Lys Lys Ala Lys
   1 5
<210> 4955
   <211> 15
   <212> PRT
   <213> Mycobacterium avium
<400> 4955
   Ala Gly Asp Leu Ser Lys Val Asp Ala Lys Gln Pro Gly Asp Tyr
   1 5 10 15
<210> 4956
   <211> 15
   <212> PRT
   <213> Homo sapiens
<400> 4956
   Cys Gln Ala Leu Val Arg Met Leu Ala Lys Lys Pro Gly Trp Lys
   1 5 10 15
<210> 4957
   <211> 15
   <212> PRT
   <213> Homo sapiens
<400> 4957
   Asp Phe Thr Tyr Asp Gln Val Ala Phe Asn Gly Leu Pro Gln Phe
   1 5 10 15
<210> 4958
   <211> 15
   <212> PRT
   <213> Homo sapiens
<400> 4958
   Asp Lys Ile Arg Gly Glu Gly Met Arg Tyr Ile Ile Ile Leu Asp
   1 5 10 15
<210> 4959
   <211> 15
   <212> PRT
   <213> Mycobacterium avium
<400> 4959
   Glu Arg Pro Leu Lys Gln Ile Ala Phe Asn Gly Gly Leu Glu Pro
   1 5 10 15
<210> 4960
   <211> 15
   <212> PRT
   <213> Homo sapiens
<400> 4960
   Gly Arg Gly Asn Gly Asp Pro Gly Gly Gly Met Glu Lys Asp Gly
   1 5 10 15
<210> 4961
   <211> 14
   <212> PRT
   <213> Homo sapiens
<400> 4961
   Asn Glu His Pro Val Phe Ala Tyr Leu Lys Asp Lys Leu Pro
   1 5 10
<210> 4962
   <211> 15
   <212> PRT
   <213> Mycobacterium avium
<400> 4962
   Gln Leu Glu Leu Thr Glu Gly Met Arg Phe Asp Lys Gly Tyr Ile
   1 5 10 15
<210> 4963
   <211> 9
   <212> PRT
   <213> Homo sapiens
<400> 4963
   Ala His Trp Ser Gly His Cys Cys Leu
   1 5
<210> 4964
   <211> 15
   <212> PRT
   <213> Mycobacterium avium
<400> 4964
   Thr Cys Arg Arg Met Leu Ala Phe Leu Lys Asp Lys Glu Asn Arg
   1 5 10 15
<210> 4965
   <211> 5
   <212> PRT
   <213> Homo sapiens
<400> 4965
   Pro Lys Lys Ala Lys
   1 5
<210> 4966
   <211> 15
   <212> PRT
   <213> Mycobacterium avium
<400> 4966
   Ala Gly Asp Leu Ser Lys Val Asp Ala Lys Gln Pro Gly Asp Tyr
   1 5 10 15
<210> 4967
   <211> 15
   <212> PRT
   <213> Homo sapiens
<400> 4967
   Cys Gln Ala Leu Val Arg Met Leu Ala Lys Lys Pro Gly Trp Lys
   1 5 10 15
<210> 4968
   <211> 15
   <212> PRT
   <213> Homo sapiens
<400> 4968
   Asp Phe Thr Tyr Asp Gln Val Ala Phe Asn Gly Leu Pro Gln Phe
   1 5 10 15
<210> 4969
   <211> 15
   <212> PRT
   <213> Homo sapiens
<400> 4969
   Asp Lys Ile Arg Gly Glu Gly Met Arg Tyr Ile Ile Ile Leu Asp
   1 5 10 15
<210> 4970
   <211> 15
   <212> PRT
   <213> Mycobacterium avium
<400> 4970
   Glu Arg Pro Leu Lys Gln Ile Ala Phe Asn Gly Gly Leu Glu Pro
   1 5 10 15
<210> 4971
   <211> 15
   <212> PRT
   <213> Homo sapiens
<400> 4971
   Gly Arg Gly Asn Gly Asp Pro Gly Gly Gly Met Glu Lys Asp Gly
   1 5 10 15
<210> 4972
   <211> 14
   <212> PRT
   <213> Homo sapiens
<400> 4972
   Asn Glu His Pro Val Phe Ala Tyr Leu Lys Asp Lys Leu Pro
   1 5 10
<210> 4973
   <211> 15
   <212> PRT
   <213> Mycobacterium avium
<400> 4973
   Gln Leu Glu Leu Thr Glu Gly Met Arg Phe Asp Lys Gly Tyr Ile
   1 5 10 15
<210> 4974
   <211> 9
   <212> PRT
   <213> Homo sapiens
<400> 4974
   Ala His Trp Ser Gly His Cys Cys Leu
   1 5
<210> 4975
   <211> 304
   <212> PRT
   <213> Homo sapiens
<400> 4975
   Met Gly Asn His Ala Gly Lys Arg Glu Leu Asn Ala Glu Lys Ala Ser
   1 5 10 15
   Thr Asn Ser Glu Thr Asn Arg Gly Glu Ser Glu Lys Lys Arg Asn Leu
   20 25 30
   Gly Glu Leu Ser Arg Thr Thr Ser Glu Asp Asn Glu Val Phe Gly Glu
   35 40 45
   Ala Asp Ala Asn Gln Asn Asn Gly Thr Ser Ser Gln Asp Thr Ala Val
   50 55 60
   Thr Asp Ser Lys Arg Thr Ala Asp Pro Lys Asn Ala Trp Gln Asp Ala
   65 70 75 80
   His Pro Ala Asp Pro Gly Ser Arg Pro His Leu Ile Arg Leu Phe Ser
   85 90 95
   Arg Asp Ala Pro Gly Arg Glu Asp Asn Thr Phe Lys Asp Arg Pro Ser
                   100                 105                 110
   Glu Ser Asp Glu Leu Gln Thr Ile Gln Glu Asp Ser Ala Ala Thr Ser
           115                 120                 125
   Glu Ser Leu Asp Val Met Ala Ser Gln Lys Arg Pro Ser Gln Arg His
       130                 135                 140
   Gly Ser Lys Tyr Leu Ala Thr Ala Ser Thr Met Asp His Ala Arg His
   145                 150                 155                 160
   Gly Phe Leu Pro Arg His Arg Asp Thr Gly Ile Leu Asp Ser Ile Gly
                   165                 170                 175
   Arg Phe Phe Gly Gly Asp Arg Gly Ala Pro Lys Arg Gly Ser Gly Lys
               180                 185                 190
   Asp Ser His His Pro Ala Arg Thr Ala His Tyr Gly Ser Leu Pro Gln
           195                 200                 205
   Lys Ser His Gly Arg Thr Gln Asp Glu Asn Pro Val Val His Phe Phe
       210                 215                 220
   Lys Asn Ile Val Thr Pro Arg Thr Pro Pro Pro Ser Gln Gly Lys Gly
   225                 230                 235                 240
   Arg Gly Leu Ser Leu Ser Arg Phe Ser Trp Gly Ala Glu Gly Gln Arg
                   245                 250                 255
   Pro Gly Phe Gly Tyr Gly Gly Arg Ala Ser Asp Tyr Lys Ser Ala His
               260                 265                 270
   Lys Gly Phe Lys Gly Val Asp Ala Gln Gly Thr Leu Ser Lys Ile Phe
           275                 280                 285
   Lys Leu Gly Gly Arg Asp Ser Arg Ser Gly Ser Pro Met Ala Arg Arg
       290                 295                 300
<210> 4976
   <211> 203
   <212> PRT
   <213> Homo sapiens
<400> 4976
<210> 4977
   <211> 277
   <212> PRT
   <213> Homo sapiens
<400> 4977
<210> 4978
   <211> 295
   <212> PRT
   <213> Homo sapiens
<400> 4978
<210> 4979
   <211> 323
   <212> PRT
   <213> Homo sapiens
<400> 4979
   Met Ser Asp Arg Pro Thr Ala Arg Arg Trp Gly Lys Cys Gly Pro Leu
   1 5 10 15
   Cys Thr Arg Glu Asn Ile Met Val Ala Phe Lys Gly Val Trp Thr Gln
   20 25 30
   Ala Phe Trp Lys Ala Val Thr Ala Glu Phe Leu Ala Met Leu Ile Phe
   35 40 45
   Val Leu Leu Ser Leu Gly Ser Thr Ile Asn Trp Gly Gly Thr Glu Lys
   50 55 60
   Pro Leu Pro Val Asp Met Val Leu Ile Ser Leu Cys Phe Gly Leu Ser
   65 70 75 80
   Ile Ala Thr Met Val Gln Cys Phe Gly His Ile Ser Gly Gly His Ile
   85 90 95
   Asn Pro Ala Val Thr Val Ala Met Val Cys Thr Arg Lys Ile Ser Ile
   100 105 110
   Ala Lys Ser Val Phe Tyr Ile Ala Ala Gln Cys Leu Gly Ala Ile Ile
   115 120 125
   Gly Ala Gly Ile Leu Tyr Leu Val Thr Pro Pro Ser Val Val Gly Gly
   130 135 140
   Leu Gly Val Thr Met Val His Gly Asn Leu Thr Ala Gly His Gly Leu
   145 150 155 160
   Leu Val Glu Leu Ile Ile Thr Phe Gln Leu Val Phe Thr Ile Phe Ala
   165 170 175
   Ser Cys Asp Ser Lys Arg Thr Asp Val Thr Gly Ser Ile Ala Leu Ala
   180 185 190
   Ile Gly Phe Ser Val Ala Ile Gly His Leu Phe Ala Ile Asn Tyr Thr
   195 200 205
   Gly Ala Ser Met Asn Pro Ala Arg Ser Phe Gly Pro Ala Val Ile Met
   210 215 220
   Gly Asn Trp Glu Asn His Trp Ile Tyr Trp Val Gly Pro Ile Ile Gly
   225 230 235 240
   Ala Val Leu Ala Gly Gly Leu Tyr Glu Tyr Val Phe Cys Pro Asp Val
   245 250 255
   Glu Phe Lys Arg Arg Phe Lys Glu Ala Phe Ser Lys Ala Ala Gln Gln
   260 265 270
   Thr Lys Gly Ser Tyr Met Glu Val Glu Asp Asn Arg Ser Gln Val Glu
   275 280 285
   Thr Asp Asp Leu Ile Leu Lys Pro Gly Val Val His Val Ile Asp Val
   290 295 300
   Asp Arg Gly Glu Glu Lys Lys Gly Lys Asp Gln Ser Gly Glu Val Leu
   305 310 315 320
   Ser Ser Val
<210> 4980
   <211> 582
   <212> PRT
   <213> Homo sapiens
<400> 4980
   Met Ile Phe Leu Thr Ala Leu Pro Leu Phe Trp Ile Met Ile Ser Ala
   1 5 10 15
   Ser Arg Gly Gly His Trp Gly Ala Trp Met Pro Ser Ser Ile Ser Ala
   20 25 30
   Phe Glu Gly Thr Cys Val Ser Ile Pro Cys Arg Phe Asp Phe Pro Asp
           35                  40                  45
   Glu Leu Arg Pro Ala Val Val His Gly Val Trp Tyr Phe Asn Ser Pro
       50                  55                  60
   Tyr Pro Lys Asn Tyr Pro Pro Val Val Phe Lys Ser Arg Thr Gln Val
   65                  70                  75                  80
   Val His Glu Ser Phe Gln Gly Arg Ser Arg Leu Leu Gly Asp Leu Gly
                   85                  90                  95
   Leu Arg Asn Cys Thr Leu Leu Leu Ser Asn Val Ser Pro Glu Leu Gly
               100                 105                 110
   Gly Lys Tyr Tyr Phe Arg Gly Asp Leu Gly Gly Tyr Asn Gln Tyr Thr
           115                 120                 125
   Phe Ser Glu His Ser Val Leu Asp Ile Val Asn Thr Pro Asn Ile Val
       130                 135                 140
   Val Pro Pro Glu Val Val Ala Gly Thr Glu Val Glu Val Ser Cys Met
   145                 150                 155                 160
   Val Pro Asp Asn Cys Pro Glu Leu Arg Pro Glu Leu Ser Trp Leu Gly
                   165                 170                 175
   His Glu Gly Leu Gly Glu Pro Ala Val Leu Gly Arg Leu Arg Glu Asp
               180                 185                 190
   Glu Gly Thr Trp Val Gln Val Ser Leu Leu His Phe Val Pro Thr Arg
           195                 200                 205
   Glu Ala Asn Gly His Arg Leu Gly Cys Gln Ala Ser Phe Pro Asn Thr
       210                 215                 220
   Thr Leu Gln Phe Glu Gly Tyr Ala Ser Met Asp Val Lys Tyr Pro Pro
   225                 230                 235                 240
   Val Ile Val Glu Met Asn Ser Ser Val Glu Ala Ile Glu Gly Ser His
                   245                 250                 255
   Val Ser Leu Leu Cys Gly Ala Asp Ser Asn Pro Pro Pro Leu Leu Thr
               260                 265                 270
   Trp Met Arg Asp Gly Thr Val Leu Arg Glu Ala Val Ala Glu Ser Leu
           275                 280                 285
   Leu Leu Glu Leu Glu Glu Val Thr Pro Ala Glu Asp Gly Val Tyr Ala
       290                 295                 300
   Cys Leu Ala Glu Asn Ala Tyr Gly Gln Asp Asn Arg Thr Val Gly Leu
   305                 310                 315                 320
   Ser Val Met Tyr Ala Pro Trp Lys Pro Thr Val Asn Gly Thr Met Val
                   325                 330                 335
   Ala Val Glu Gly Glu Thr Val Ser Ile Leu Cys Ser Thr Gln Ser Asn
               340                 345                 350
   Pro Asp Pro Ile Leu Thr Ile Phe Lys Glu Lys Gln Ile Leu Ser Thr
           355                 360                 365
   Val Ile Tyr Glu Ser Glu Leu Gln Leu Glu Leu Pro Ala Val Ser Pro
       370                 375                 380
   Glu Asp Asp Gly Glu Tyr Trp Cys Val Ala Glu Asn Gln Tyr Gly Gln
   385                 390                 395                 400
   Arg Ala Thr Ala Phe Asn Leu Ser Val Glu Phe Ala Pro Val Leu Leu
                   405                 410                 415
   Leu Glu Ser His Cys Ala Ala Ala Arg Asp Thr Val Gln Cys Leu Cys
               420                 425                 430
   Val Val Lys Ser Asn Pro Glu Pro Ser Val Ala Phe Glu Leu Pro Ser
           435                 440                 445
   Arg Asn Val Thr Val Asn Glu Ser Glu Arg Glu Phe Val Tyr Ser Glu
       450                 455                 460
   Arg Ser Gly Leu Val Leu Thr Ser Ile Leu Thr Leu Arg Gly Gln Ala
   465                 470                 475                 480
   Gln Ala Pro Pro Arg Val Ile Cys Thr Ala Arg Asn Leu Tyr Gly Ala
                   485                 490                 495
   Lys Ser Leu Glu Leu Pro Phe Gln Gly Ala His Arg Leu Met Trp Ala
               500                 505                 510
   Lys Ile Gly Pro Val Gly Ala Val Val Ala Phe Ala Ile Leu Ile Ala
           515                 520                 525
   Ile Val Cys Tyr Ile Thr Gln Thr Arg Arg Lys Lys Asn Val Thr Glu
       530                 535                 540
   Ser Pro Ser Phe Ser Ala Gly Asp Asn Pro Pro Val Leu Phe Ser Ser
   545 550 555 560
   Asp Phe Arg Ile Ser Gly Ala Pro Glu Lys Tyr Glu Ser Lys Glu Val
   565 570 575
   Ser Thr Leu Glu Ser His
   580
<210> 4981
   <211> 110
   <212> PRT
   <213> Homo sapiens
<400> 4981
<210> 4982
   <211> 180
   <212> PRT
   <213> Homo sapiens
<400> 4982
   Ile Ala Pro Val Phe Val Leu Met Glu Gln Ile Thr Leu Lys Lys Met
   1 5 10 15
   Arg Lys Ile Val Gly Trp Ser Asn Lys Asp Gly Asp Gly Ile Phe Ser
   20 25 30
   Pro Gly Gly Ala Ile Ser Asn Met Tyr Ser Ile Met Ala Ala Arg Tyr
   35 40 45
   Lys Tyr Phe Pro Glu Val Lys Thr Lys Gly Met Ala Ala Val Pro Lys
   50 55 60
   Leu Val Leu Phe Thr Ser Glu His Ser His Tyr Ser Ile Lys Lys Ala
   65 70 75 80
   Gly Ala Ala Leu Gly Phe Gly Thr Asp Asn Val Ile Leu Ile Lys Cys
   85 90 95
   Asn Glu Arg Gly Lys Ile Ile Pro Ala Asp Leu Glu Ala Lys Ile Leu
   100 105 110
   Asp Ala Lys Gln Lys Gly Tyr Val Pro Leu Tyr Val Asn Ala Thr Ala
   115 120 125
   Gly Thr Thr Val Tyr Gly Ala Phe Asp Pro Ile Gln Glu Ile Ala Asp
   130 135 140
   Ile Cys Glu Lys Tyr Asn Leu Trp Leu His Val Asp Ala Ala Trp Gly
   145 150 155 160
   Gly Gly Leu Leu Met Ser Arg Lys His Arg His Lys Leu Ser Gly Ile
   165 170 175
   Glu Arg Ala Asn
   180
<210> 4983
   <211> 296
   <212> PRT
   <213> Triticum urartu
<400> 4983
   Met Lys Thr Phe Leu Ile Leu Ala Leu Leu Ala Ile Val Ala Thr Thr
   1 5 10 15
   Ala Thr Thr Ala Val Arg Val Pro Val Pro Gln Pro Gln Pro Gln Asn
   20 25 30
   Pro Ser Gln Pro Gln Pro Gln Arg Gln Val Pro Leu Val Gln Gln Gln
   35 40 45
   Gln Phe Pro Gly Gln Gln Gln Gln Phe Pro Pro Gln Gln Pro Tyr Pro
   50 55 60
   Gln Pro Gln Pro Phe Pro Ser Gln Gln Pro Tyr Leu Gln Leu Gln Pro
   65 70 75 80
   Phe Pro Gln Pro Gln Pro Phe Pro Pro Gln Leu Pro Tyr Pro Gln Pro
   85 90 95
   Pro Pro Phe Ser Pro Gln Gln Pro Tyr Pro Gln Pro Gln Pro Gln Tyr
   100 105 110
   Pro Gln Pro Gln Gln Pro Ile Ser Gln Gln Gln Ala Gln Gln Gln Gln
   115 120 125
   Gln Gln Gln Gln Gln Gln Gln Gln Gln Gln Gln Gln Gln Gln Ile Leu
   130 135 140
   Pro Gln Ile Leu Gln Gln Gln Leu Ile Pro Cys Arg Asp Val Val Leu
   145 150 155 160
   Gln Gln His Asn Ile Ala His Ala Arg Ser Gln Val Leu Gln Gln Ser
   165 170 175
   Thr Tyr Gln Pro Leu Gln Gln Leu Cys Cys Gln Gln Leu Trp Gln Ile
   180 185 190
   Pro Glu Gln Ser Arg Cys Gln Ala Ile His Asn Val Val His Ala Ile
   195 200 205
   Ile Leu His Gln Gln Gln Gln Gln Gln Gln Pro Ser Ser Gln Val Ser
   210 215 220
   Leu Gln Gln Pro Gln Gln Gln Tyr Pro Ser Gly Gln Gly Phe Phe Gln
   225 230 235 240
   Pro Ser Gln Gln Asn Pro Gln Ala Gln Gly Ser Val Gln Pro Gln Gln
   245 250 255
   Leu Pro Gln Phe Glu Glu Ile Arg Asn Leu Ala Leu Gln Thr Leu Pro
   260 265 270
   Arg Met Cys Asn Val Tyr Ile Pro Pro Tyr Cys Ser Thr Thr Thr Ala
   275 280 285
   Pro Phe Gly Ile Phe Gly Thr Asn
   290 295
<210> 4984
   <211> 275
   <212> PRT
   <213> Triticum durum
<400> 4984
   Met Asn Ile Gln Val Asp Pro Ser Ser Gln Val Gln Trp Pro Gln Gln
   1               5                   10                  15
   Gln Pro Phe Leu Gln Pro His Gln Pro Phe Ser Gln Gln Pro Gln Gln
               20                  25                  30
   Ile Phe Pro Gln Pro Gln Gln Thr Phe Pro His Gln Pro Gln Gln Gln
           35                  40                  45
   Phe Pro Gln Pro Gln Gln Pro Gln Arg Gln Phe Leu Gln Pro Arg Gln
       50                  55                  60
   Pro Phe Pro Gln Gln Pro Gln Gln Pro Tyr Pro Gln Gln Pro Gln Gln
   65                  70                  75                  80
   Pro Phe Pro Gln Thr Gln Gln Pro Gln Gln Pro Phe Pro Gln Ser Lys
                   85                  90                  95
   Gln Pro Gln Gln Pro Phe Pro Gln Pro Gln Gln Pro Gln Gln Ser Phe
               100                 105                 110
   Pro Gln Gln Gln Pro Ser Leu Ile Gln Gln Ser Leu Gln Gln Gln Leu
           115                 120                 125
   Asn Pro Cys Lys Asn Phe Leu Leu Gln Gln Cys Lys Pro Val Ser Leu
       130                 135                 140
   Val Ser Ser Leu Trp Ser Ile Ile Leu Pro Pro Ser Asp Cys Gln Val
   145                 150                 155                 160
   Met Arg Gln Gln Cys Cys Gln Gln Leu Ala Gln Ile Pro Gln Gln Leu
                   165                 170                 175
   Gln Cys Ala Ala Ile His Ser Val Val His Ser Ile Ile Met Gln Gln
               180                 185                 190
   Glu Gln Gln Glu Gln Leu Gln Gly Val Gln Ile Leu Val Pro Leu Ser
           195                 200                 205
   Gln Gln Gln Gln Val Gly Gln Gly Ile Leu Val Gln Gly Gln Gly Ile
       210                 215                 220
   Ile Gln Pro Gln Gln Pro Ala Gln Leu Glu Val Ile Arg Ser Leu Val
   225                 230                 235                 240
   Leu Gln Thr Leu Pro Thr Met Cys Asn Val Tyr Val Pro Pro Tyr Cys
   245 250 255
   Ser Thr Ile Arg Ala Pro Phe Ala Ser Ile Val Ala Gly Ile Gly Gly
   260 265 270
   Gln Tyr Arg
   275
<210> 4985
   <211> 282
   <212> PRT
   <213> Triticum aestivum
<400> 4985
   Met Asn Ile Gln Val Asp Pro Ser Ser Gln Val Pro Trp Pro Gln Gln
   1 5 10 15
   Gln Pro Phe Pro Gln Pro His Gln Pro Phe Ser Gln Gln Pro Gln Gln
   20 25 30
   Thr Phe Pro Gln Pro Gln Gln Thr Phe Pro His Gln Pro Gln Gln Gln
   35 40 45
   Phe Ser Gln Pro Gln Gln Pro Gln Gln Gln Phe Ile Gln Pro Gln Gln
   50 55 60
   Pro Phe Pro Gln Gln Pro Gln Gln Thr Tyr Pro Gln Arg Pro Gln Gln
   65 70 75 80
   Pro Phe Pro Gln Thr Gln Gln Pro Gln Gln Pro Phe Pro Gln Ser Gln
   85 90 95
   Gln Pro Gln Gln Pro Phe Pro Gln Pro Gln Gln Gln Phe Pro Gln Pro
   100 105 110
   Gln Gln Pro Gln Gln Ser Phe Pro Gln Gln Gln Pro Ser Leu Ile Gln
   115 120 125
   Gln Ser Leu Gln Gln Gln Leu Asn Pro Cys Lys Asn Phe Leu Leu Gln
   130 135 140
   Gln Cys Lys Pro Val Ser Leu Val Ser Ser Leu Trp Ser Met Ile Leu
   145 150 155 160
   Pro Arg Ser Asp Cys Gln Val Met Arg Gln Gln Cys Cys Gln Gln Leu
   165 170 175
   Ala Gln Ile Pro Gln Gln Leu Gln Cys Ala Ala Ile His Ser Ile Val
   180 185 190
   His Ser Ile Ile Met Gln Gln Glu Gln Gln Glu Gln Arg Gln Gly Val
   195 200 205
   Gln Ile Leu Val Pro Leu Ser Gln Gln Gln Gln Val Gly Gln Gly Thr
   210 215 220
   Leu Val Gln Gly Gln Gly Ile Ile Gln Pro Gln Gln Pro Ala Gln Leu
   225 230 235 240
   Glu Val Ile Arg Ser Leu Val Leu Gln Thr Leu Ala Thr Met Cys Asn
   245 250 255
   Val Tyr Val Pro Pro Tyr Cys Ser Thr Ile Arg Ala Pro Phe Ala Ser
   260 265 270
   Ile Val Ala Gly Ile Gly Gly Gln Tyr Arg
   275 280
<210> 4986
   <211> 9
   <212> PRT
   <213> Triticum sp.
<400> 4986
   Pro Phe Pro Gln Pro Glu Leu Pro Tyr
   1 5
<210> 4987
   <211> 9
   <212> PRT
   <213> Triticum sp.
<400> 4987
   Pro Tyr Pro Gln Pro Glu Leu Pro Tyr
   1 5
<210> 4988
   <211> 9
   <212> PRT
   <213> Triticum sp.
<400> 4988
   Pro Gln Pro Glu Leu Pro Tyr Pro Trp
   1 5
<210> 4989
   <211> 10
   <212> PRT
   <213> Triticum sp.
<400> 4989
   Phe Arg Pro Pro Glu Gln Pro Tyr Pro Gln
   1 5 10
<210> 4990
   <211> 9
   <212> PRT
   <213> Triticum sp.
<400> 4990
   Pro Gln Gln Ser Phe Pro Glu Gln Gln
   1 5
<210> 4991
   <211> 9
   <212> PRT
   <213> Triticum sp.
<400> 4991
   Ile Gln Pro Glu Gln Pro Ala Gln Leu
   1 5
<210> 4992
   <211> 9
   <212> PRT
   <213> Triticum sp.
<400> 4992
   Gln Gln Pro Glu Gln Pro Tyr Pro Gln
   1 5
<210> 4993
   <211> 9
   <212> PRT
   <213> Triticum sp.
<400> 4993
   Ser Gln Pro Glu Gln Glu Phe Pro Gln
   1 5
<210> 4994
   <211> 9
   <212> PRT
   <213> Triticum sp.
<400> 4994
   Pro Gln Pro Glu Gln Glu Phe Pro Gln
   1 5
<210> 4995
   <211> 9
   <212> PRT
   <213> Triticum sp.
<400> 4995
   Gln Gln Pro Glu Gln Pro Phe Pro Gln
   1 5
<210> 4996
   <211> 9
   <212> PRT
   <213> Triticum sp.
<400> 4996
   Pro Gln Pro Glu Gln Pro Phe Cys Gln
   1 5
<210> 4997
   <211> 9
   <212> PRT
   <213> Triticum sp.
<400> 4997
   Gln Gln Pro Phe Pro Glu Gln Pro Gln
   1 5
<210> 4998
   <211> 9
   <212> PRT
   <213> Triticum sp.
<400> 4998
   Pro Phe Pro Gln Pro Glu Gln Pro Phe
   1 5
<210> 4999
   <211> 9
   <212> PRT
   <213> Triticum sp.
<400> 4999
   Pro Gln Pro Glu Gln Pro Phe Pro Trp
   1 5
<210> 5000
   <211> 9
   <212> PRT
   <213> Triticum sp.
<400> 5000
   Pro Phe Ser Glu Gln Glu Gln Pro Val
   1               5
<210> 5001
   <211> 9
   <212> PRT
   <213> Triticum sp.
<400> 5001
   Phe Ser Gln Gln Gln Glu Ser Pro Phe
   1               5
<210> 5002
   <211> 9
   <212> PRT
   <213> Triticum sp.
<400> 5002
   Pro Phe Pro Gln Pro Glu Gln Pro Phe
   1               5
<210> 5003
   <211> 9
   <212> PRT
   <213> Triticum sp.
<400> 5003
   Pro Gln Pro Glu Gln Pro Phe Pro Gln
   1               5
<210> 5004
   <211> 9
   <212> PRT
   <213> Triticum sp.
<400> 5004
   Pro Ile Pro Glu Gln Pro Gln Pro Tyr
   1               5
<210> 5005
   <211> 9
   <212> PRT
   <213> Triticum sp.
<400> 5005
   Pro Phe Pro Gln Pro Glu Gln Pro Phe
   1               5
<210> 5006
   <211> 9
   <212> PRT
   <213> Triticum sp.
<400> 5006
   Pro Gln Pro Glu Gln Pro Phe Pro Gln
   1 5
<210> 5007
   <211> 9
   <212> PRT
   <213> Triticum sp.
<400> 5007
   Pro Tyr Pro Glu Gln Glu Glu Pro Phe
   1 5
<210> 5008
   <211> 9
   <212> PRT
   <213> Triticum sp.
<400> 5008
   Pro Tyr Pro Glu Gln Glu Gln Pro Phe
   1 5
<210> 5009
   <211> 9
   <212> PRT
   <213> Triticum sp.
<400> 5009
   Pro Phe Ser Glu Gln Glu Gln Pro Val
   1 5
<210> 5010
   <211> 9
   <212> PRT
   <213> Triticum sp.
<400> 5010
   Glu Gly Ser Phe Gln Pro Ser Gln Glu
   1 5
<210> 5011
   <211> 9
   <212> PRT
   <213> Triticum sp.
<400> 5011
   Glu Gln Pro Gln Gln Pro Phe Pro Gln
   1 5
<210> 5012
   <211> 9
   <212> PRT
   <213> Triticum sp.
<400> 5012
   Glu Gln Pro Gln Gln Pro Tyr Pro Glu
   1 5
<210> 5013
   <211> 9
   <212> PRT
   <213> Triticum sp.
<400> 5013
   Gln Phe Tyr Tyr Pro Thr Ser Pro Gln
   1 5
<210> 5014
   <211> 9
   <212> PRT
   <213> Triticum sp.
<400> 5014
   Glu Gly Ser Phe Gln Pro Ser Gln Glu
   1 5
<210> 5015
   <211> 9
   <212> PRT
   <213> Triticum sp.
<400> 5015
   Pro Gln Gln Ser Phe Pro Glu Gln Glu
   1 5
<210> 5016
   <211> 9
   <212> PRT
   <213> Triticum sp.
<400> 5016
   Gln Phe Tyr Tyr Pro Thr Ser Pro Gln
   1 5
<210> 5017
   <211> 239
   <212> PRT
   <213> Homo sapiens
<400> 5017
   Arg Gly Asp Ser Gly Ser Pro Ala Thr Trp Thr Thr Arg Gly Phe Val
   1               5                   10                  15
   Phe Thr Arg His Ser Gln Thr Thr Ala Ile Pro Ser Cys Pro Glu Gly
               20                  25                  30
   Thr Val Pro Leu Tyr Ser Gly Phe Ser Phe Leu Phe Val Gln Gly Asn
           35                  40                  45
   Gln Arg Ala His Gly Gln Asp Leu Gly Thr Leu Gly Ser Cys Leu Gln
       50                  55                  60
   Arg Phe Thr Thr Met Pro Phe Leu Phe Cys Asn Val Asn Asp Val Cys
   65                  70                  75                  80
   Asn Phe Ala Ser Arg Asn Asp Tyr Ser Tyr Trp Leu Ser Thr Pro Ala
                   85                  90                  95
   Leu Met Pro Met Asn Met Ala Pro Ile Thr Gly Arg Ala Leu Glu Pro
               100                 105                 110
   Tyr Ile Ser Arg Cys Thr Val Cys Glu Gly Pro Ala Ile Ala Ile Ala
           115                 120                 125
   Val His Ser Gln Thr Thr Asp Ile Pro Pro Cys Pro His Gly Trp Ile
       130                 135                 140
   Ser Leu Trp Lys Gly Phe Ser Phe Ile Met Phe Thr Ser Ala Gly Ser
   145                 150                 155                 160
   Glu Gly Thr Gly Gln Ala Leu Ala Ser Pro Gly Ser Cys Leu Glu Glu
                   165                 170                 175
   Phe Arg Ala Ser Pro Phe Leu Glu Cys His Gly Arg Gly Thr Cys Asn
               180                 185                 190
   Tyr Tyr Ser Asn Ser Tyr Ser Phe Trp Leu Ala Ser Leu Asn Pro Glu
           195                 200                 205
   Arg Met Phe Arg Lys Pro Ile Pro Ser Thr Val Lys Ala Gly Glu Leu
       210                 215                 220
   Glu Lys Ile Ile Ser Arg Cys Gln Val Cys Met Lys Lys Arg His
   225                 230                 235
<210> 5018
   <211> 20
   <212> PRT
   <213> Homo sapiens
<400> 5018
   Ser Pro Ala Thr Trp Thr Thr Arg Gly Phe Val Phe Thr Arg His Ser
   1 5 10 15
   Gln Thr Thr Ala
   20
<210> 5019
   <211> 21
   <212> PRT
   <213> Homo sapiens
<400> 5019
   Val Phe Thr Arg His Ser Gln Thr Thr Ala Ile Pro Ser Cys Pro Glu
   1 5 10 15
   Gly Thr Val Pro Leu
   20
<210> 5020
   <211> 20
   <212> PRT
   <213> Homo sapiens
<400> 5020
   Ile Pro Ser Cys Pro Glu Gly Thr Val Pro Leu Tyr Ser Gly Phe Ser
   1 5 10 15
   Phe Leu Phe Val
   20
<210> 5021
   <211> 20
   <212> PRT
   <213> Homo sapiens
<400> 5021
   Leu Tyr Ser Gly Phe Ser Phe Leu Phe Val Gln Gly Asn Gln Arg Ala
   1 5 10 15
   His Gly Gln Asp
   20
<210> 5022
   <211> 20
   <212> PRT
   <213> Homo sapiens
<400> 5022
   Phe Thr Thr Met Pro Phe Leu Phe Cys Asn Val Asn Asp Val Cys Asn
   1               5                   10                  15
   Phe Ala Ser Arg
               20
<210> 5023
   <211> 21
   <212> PRT
   <213> Homo sapiens
<400> 5023
   Val Asn Asp Val Cys Asn Phe Ala Ser Arg Asn Asp Tyr Ser Tyr Trp
   1               5                   10                  15
   Leu Ser Thr Pro Ala
               20
<210> 5024
   <211> 20
   <212> PRT
   <213> Homo sapiens
<400> 5024
   Thr Gly Arg Ala Leu Glu Pro Tyr Ile Ser Arg Cys Thr Val Cys Glu
   1               5                   10                  15
   Gly Pro Ala Ile
               20
<210> 5025
   <211> 19
   <212> PRT
   <213> Homo sapiens
<400> 5025
   Arg Cys Thr Val Cys Glu Pro Ala Ile Ala Ile Ala Val His Ser Gln
   1               5                   10                  15
   Thr Thr Asp
<210> 5026
   <211> 20
   <212> PRT
   <213> Homo sapiens
<400> 5026
   Ile Pro Pro Cys Pro His Gly Trp Ile Ser Leu Trp Lys Gly Phe Ser
   1 5 10 15
   Phe Ile Met Phe
   20
<210> 5027
   <211> 20
   <212> PRT
   <213> Homo sapiens
<400> 5027
   Leu Trp Lys Gly Phe Ser Phe Ile Met Phe Thr Ser Ala Gly Ser Glu
   1 5 10 15
   Gly Thr Gln Gly
   20
<210> 5028
   <211> 20
   <212> PRT
   <213> Homo sapiens
<400> 5028
   Asn Ser Tyr Ser Phe Trp Leu Ala Ser Leu Asn Pro Glu Arg Met Phe
   1 5 10 15
   Arg Lys Pro Ile
   20
<210> 5029
   <211> 19
   <212> PRT
   <213> Mus musculus
<400> 5029
   Ala Thr Gly Thr Arg Met Arg Gly Phe Ile Phe Thr Arg His Ser Thr
   1 5 10 15
   Thr Ala Ile
<210> 5030
   <211> 20
   <212> PRT
   <213> Mus musculus
<400> 5030
   Phe Ile Phe Thr Arg His Ser Gln Thr Thr Ala Ile Pro Ser Cys Pro
   1               5                   10                  15
   Glu Gly Thr Gln
                                       20
<210> 5031
   <211> 20
   <212> PRT
   <213> Mus musculus
<400> 5031
   Thr Thr Ala Ile Pro Ser Cys Pro Glu Gly Thr Gln Pro Leu Tyr Ser
   1               5                   10                  15
   Gly Phe Ser Leu
               20
<210> 5032
   <211> 20
   <212> PRT
   <213> Mus musculus
<400> 5032
   Glu Gly Thr Gln Pro Leu Tyr Ser Gly Phe Ser Leu Leu Phe Val Gln
   1               5                   10                  15
   Gly Asn Lys Arg
               20
<210> 5033
   <211> 20
   <212> PRT
   <213> Mus musculus
<400> 5033
   Phe Leu Phe Cys Asn Ile Asn Asn Val Cys Asn Phe Ala Ser Arg Asn
   1               5                   10                  15
   Asp Tyr Ser Tyr
               20
<210> 5034
   <211> 20
   <212> PRT
   <213> Mus musculus
<400> 5034
   Val Cys Asn Phe Ala Ser Arg Asn Asp Tyr Ser Tyr Trp Leu Ser Thr
   1               5                   10                  15
   Pro Ala Leu Met
               20
<210> 5035
   <211> 20
   <212> PRT
   <213> Mus musculus
<400> 5035
   Ala Pro Ile Ser Gly Arg Ala Leu Glu Pro Tyr Ile Ser Arg Cys Thr
   1               5                   10                  15
   Val Cys Glu Gly
               20
<210> 5036
   <211> 20
   <212> PRT
   <213> Mus musculus
<400> 5036
   Glu Pro Tyr Ile Ser Arg Cys Thr Val Cys Glu Gly Pro Ala Met Ala
   1               5                   10                  15
   Ile Ala Val His
               20
<210> 5037
   <211> 20
   <212> PRT
   <213> Mus musculus
<400> 5037
   Ala Ile Pro Pro Cys Pro Gln Asp Trp Val Ser Leu Trp Lys Gly Phe
   1               5                   10                  15
   Ser Phe Ile Met
               20
<210> 5038
   <211> 21
   <212> PRT
   <213> Mus musculus
<400> 5038
   Trp Val Ser Leu Trp Lys Gly Glu Phe Ser Phe Ile Met Phe Thr Ser
   1               5                   10                  15
   Ala Gly Ser Glu Gly
               20
<210> 5039
   <211> 20
   <212> PRT
   <213> Mus musculus
<400> 5039
   Ser Phe Trp Leu Ala Ser Leu Asn Pro Glu Arg Met Phe Arg Lys Pro
   1               5                   10                  15
   Ile Pro Ser Thr
               20
<210> 5040
   <211> 20
   <212> PRT
   <213> Triticum sp.
<400> 5040
   Pro Gln Pro Phe Pro Pro Gln Leu Pro Tyr Pro Gln Pro Gln Leu Pro
   1               5                   10                  15
   Tyr Pro Gln Pro
               20
<210> 5041
   <211> 20
   <212> PRT
   <213> Triticum sp.
<400> 5041
   Met Gln Leu Gln Pro Phe Pro Gln Pro Gln Leu Pro Tyr Pro Gln Pro
   1               5                   10                  15
   Gln Leu Pro Tyr
               20
<210> 5042
   <211> 20
   <212> PRT
   <213> Triticum sp.
<400> 5042
   Gln Pro Phe Pro Gln Pro Gln Gln Pro Phe Pro Trp Gln Pro Gln Gln
   1 5 10 15
   Pro Phe Pro Gln
   20
<210> 5043
   <211> 20
   <212> PRT
   <213> Triticum sp.
<400> 5043
   Pro Gln Gln Pro Gln Gln Pro Phe Pro Gln Pro Gln Gln Pro Ile Pro
   1 5 10 15
   Val Gln Pro Gln
   20
<210> 5044
   <211> 20
   <212> PRT
   <213> Triticum sp.
<400> 5044
   Pro Gln Gln Gln Gln Pro Phe Pro Gln Pro Gln Gln Pro Phe Ser Gln
   1 5 10 15
   Gln Pro Gln Gln
   20
<210> 5045
   <211> 20
   <212> PRT
   <213> Triticum sp.
<400> 5045
   Gln Gln Pro Gln Gln Pro Phe Pro Gln Pro Gln Leu Pro Phe Pro Gln
   1 5 10 15
   Gln Ser Glu Gln
   20
<210> 5046
   <211> 20
   <212> PRT
   <213> Triticum sp.
<400> 5046
   Trp Pro Gln Gln Gln Pro Phe Pro Gln Pro Gln Gln Pro Phe Cys Gln
   1 5 10 15
   Gln Pro Gln Gln
   20
<210> 5047
   <211> 20
   <212> PRT
   <213> Triticum sp.
<400> 5047
   Leu Gln Leu Gln Pro Phe Pro Gln Pro Gln Leu Pro Tyr Ser Gln Pro
   1 5 10 15
   Gln Pro Phe Arg
   20
<210> 5048
   <211> 20
   <212> PRT
   <213> Triticum sp.
<400> 5048
   Leu Gln Pro Phe Pro Gln Pro Gln Pro Phe Leu Pro Gln Leu Pro Tyr
   1 5 10 15
   Pro Gln Pro Gln
   20
<210> 5049
   <211> 20
   <212> PRT
   <213> Triticum sp.
<400> 5049
   Ser Gln Gln Pro Gln Gln Gln Phe Ser Gln Pro Gln Gln Gln Phe Pro
   1 5 10 15
   Gln Pro Gln Gln
   20
<210> 5050
   <211> 20
   <212> PRT
   <213> Triticum sp.
<400> 5050
   Gln Ala Phe Pro Gln Pro Gln Gln Thr Phe Pro His Gln Pro Gln Gln
   1 5 10 15
   Gln Phe Pro Gln
   20
<210> 5051
   <211> 20
   <212> PRT
   <213> Triticum sp.
<400> 5051
   Cys Lys Val Phe Leu Gln Gln Gln Cys Ser Pro Val Ala Met Pro Gln
   1 5 10 15
   Arg Leu Ala Arg
   20
<210> 5052
   <211> 20
   <212> PRT
   <213> Triticum sp.
<400> 5052
   Leu Gln Leu Gln Pro Phe Pro Gln Pro Gln Leu Pro Tyr Leu Gln Pro
   1 5 10 15
   Gln Pro Phe Arg
   20
<210> 5053
   <211> 20
   <212> PRT
   <213> Triticum sp.
<400> 5053
   Gln Gln Gln Phe Ile Gln Pro Gln Gln Pro Phe Pro Gln Gln Pro Gln
   1 5 10 15
   Gln Thr Tyr Pro
   20
<210> 5054
   <211> 20
   <212> PRT
   <213> Triticum sp.
<400> 5054
   Ser His Ile Pro Gly Leu Glu Arg Pro Trp Gln Gln Gln Pro Leu Pro
   1               5                   10                  15
   Pro Gln Gln Thr
               20
<210> 5055
   <211> 20
   <212> PRT
   <213> Triticum sp.
<400> 5055
   Ser Gln Gln Pro Gln Gln Pro Phe Pro Gln Pro Gln Gln Gln Phe Pro
   1               5                   10                  15
   Gln Pro Gln Gln
               20
<210> 5056
   <211> 20
   <212> PRT
   <213> Triticum sp.
<400> 5056
   Gln Gln Pro Phe Pro Gln Pro Gln Gln Pro Gln Leu Pro Phe Pro Gln
   1               5                   10                  15
   Gln Pro Gln Gln
               20
<210> 5057
   <211> 20
   <212> PRT
   <213> Triticum sp.
<400> 5057
   Pro Gln Leu Pro Tyr Pro Gln Pro Gln Leu Pro Tyr Pro Gln Pro Gln
   1               5                   10                  15
   Pro Phe Arg Pro
               20
<210> 5058
   <211> 20
   <212> PRT
   <213> Triticum sp.
<400> 5058
   Gln Pro Phe Pro Gln Pro Gln Gln Pro Phe Pro Trp Gln Pro Gln Gln
   1 5 10 15
   Pro Phe Pro Gln
   20
<210> 5059
   <211> 20
   <212> PRT
   <213> Triticum sp.
<400> 5059
   Phe Pro Glu Leu Gln Gln Pro Ile Pro Gln Gln Pro Gln Gln Pro Phe
   1 5 10 15
   Pro Leu Gln Pro
   20
<210> 5060
   <211> 20
   <212> PRT
   <213> Triticum sp.
<400> 5060
   Gln Gly Gln Gln Gly Tyr Tyr Pro Ile Ser Pro Gln Gln Ser Gly Gln
   1 5 10 15
   Gly Gln Gln Pro
   20
<210> 5061
   <211> 20
   <212> PRT
   <213> Triticum sp.
<400> 5061
   Leu Gln Pro Gly Gln Gly Gln Pro Gly Tyr Tyr Pro Thr Ser Pro Gln
   1 5 10 15
   Gln Ile Gly Gln
   20
<210> 5062
   <211> 20
   <212> PRT
   <213> Triticum sp.
<400> 5062
   Gln Gln Phe Leu Gln Pro Gln Gln Pro Phe Pro Gln Gln Pro Gln Gln
   1 5 10 15
   Pro Tyr Pro Gln
   20
<210> 5063
   <211> 20
   <212> PRT
   <213> Triticum sp.
<400> 5063
   Pro Gly Gln Gly Gln Ser Gly Tyr Tyr Pro Thr Ser Pro Gln Gln Ser
   1 5 10 15
   Gly Gln Lys Gln
   20
<210> 5064
   <211> 20
   <212> PRT
   <213> Triticum sp.
<400> 5064
   Thr Pro Ile Gln Pro Gln Gln Pro Phe Pro Gln Gln Pro Gln Gln Pro
   1 5 10 15
   Gln Gln Pro Phe
   20
<210> 5065
   <211> 20
   <212> PRT
   <213> Triticum sp.
<400> 5065
   Pro Gln Gln Pro Gln Gln Pro Phe Pro Leu Gln Pro Gln Gln Pro Phe
   1 5 10 15
   Pro Gln Gln Pro
   20
<210> 5066
   <211> 20
   <212> PRT
   <213> Triticum sp.
<400> 5066
   Pro Phe Thr Gln Pro Gln Gln Pro Thr Pro Ile Gln Pro Gln Gln Pro
   1 5 10 15
   Phe Pro Gln Gln
   20
<210> 5067
   <211> 20
   <212> PRT
   <213> Triticum sp.
<400> 5067
   Pro Gln Gln Pro Phe Pro Gln Pro Gln Gln Thr Phe Pro Gln Gln Pro
   1 5 10 15
   Gln Leu Pro Phe
   20
<210> 5068
   <211> 20
   <212> PRT
   <213> Triticum sp.
<400> 5068
   Gly Gln Gly Gln Ser Gly Tyr Tyr Pro Thr Ser Pro Gln Gln Ser Gly
   1 5 10 15
   Gln Glu Ala Thr
   20
<210> 5069
   <211> 20
   <212> PRT
   <213> Triticum sp.
<400> 5069
   Pro Leu Gln Pro Gln Gln Pro Phe Pro Gln Gln Pro Gln Gln Pro Phe
   1 5 10 15
   Pro Gln Pro Gln
   20
<210> 5070
   <211> 20
   <212> PRT
   <213> Triticum sp.
<400> 5070
   Gln Pro Phe Pro Gln Leu Gln Gln Pro Gln Gln Pro Leu Pro Gln Pro
   1 5 10 15
   Gln Gln Pro Gln
   20
<210> 5071
   <211> 20
   <212> PRT
   <213> Triticum sp.
<400> 5071
   Gln Gln Pro Phe Pro Gln Gln Pro Gln Gln Pro Phe Pro Gln Pro Gln
   1 5 10 15
   Gln Pro Ile Pro
   20
<210> 5072
   <211> 20
   <212> PRT
   <213> Triticum sp.
<400> 5072
   Pro Gln Gln Pro Phe Pro Gln Pro Gln Gln Thr Phe Pro Gln Gln Pro
   1 5 10 15
   Gln Leu Pro Phe
   20
<210> 5073
   <211> 20
   <212> PRT
   <213> Triticum sp.
<400> 5073
   Val Ala His Ala Ile Ile Met His Gln Gln Gln Gln Gln Gln Gln Glu
   1 5 10 15
   Gln Lys Gln Gln
   20
<210> 5074
   <211> 20
   <212> PRT
   <213> Triticum sp.
<400> 5074
   Pro Gln Gln Pro Phe Pro Gln Gln Pro Gln Gln Gln Phe Pro Gln Pro
   1 5 10 15
   Gln Gln Pro Gln
   20
<210> 5075
   <211> 20
   <212> PRT
   <213> Triticum sp.
<400> 5075
   Gln Gln Pro Ala Gln Tyr Glu Val Ile Arg Ser Leu Val Leu Arg Thr
   1 5 10 15
   Leu Pro Asn Met
   20
<210> 5076
   <211> 20
   <212> PRT
   <213> Triticum sp.
<400> 5076
   Ala Thr Ala Asn Met Gln Val Asp Pro Ser Gly Gln Val Gln Trp Pro
   1 5 10 15
   Gln Gln Gln Pro
   20
<210> 5077
   <211> 20
   <212> PRT
   <213> Hordeum vulgare
<400> 5077
   Gln Pro Phe Pro Gln Pro Gln Gln Pro Phe Pro Trp Gln Pro Gln Gln
   1 5 10 15
   Pro Phe Pro Gln
   20
<210> 5078
   <211> 20
   <212> PRT
   <213> Hordeum vulgare
<400> 5078
   Trp Gln Pro Gln Gln Pro Phe Pro Gln Pro Gln Gln Pro Phe Pro Leu
   1 5 10 15
   Gln Pro Gln Gln
   20
<210> 5079
   <211> 20
   <212> PRT
   <213> Hordeum vulgare
<400> 5079
   Gln Pro Gln Gln Pro Phe Pro Gln Pro Gln Gln Pro Ile Pro Tyr Gln
   1 5 10 15
   Pro Gln Gln Pro
   20
<210> 5080
   <211> 20
   <212> PRT
   <213> Hordeum vulgare
<400> 5080
   Gln Pro Gln Gln Pro Gln Pro Phe Pro Gln Gln Pro Val Pro Gln Gln
   1 5 10 15
   Pro Gln Pro Tyr
   20
<210> 5081
   <211> 20
   <212> PRT
   <213> Hordeum vulgare
<400> 5081
   Pro Gln Pro Phe Pro Gln Gln Pro Ile Pro Gln Gln Pro Gln Pro Tyr
   1 5 10 15
   Pro Gln Gln Pro
   20
<210> 5082
   <211> 20
   <212> PRT
   <213> Hordeum vulgare
<400> 5082
   Gln Gln Pro Gln Pro Phe Ser Gln Gln Pro Ile Pro Gln Gln Pro Gln
   1               5                   10                  15
   Pro Tyr Pro Gln
               20
<210> 5083
   <211> 20
   <212> PRT
   <213> Hordeum vulgare
<400> 5083
   Gln Ser Gln Gln Gln Phe Pro Gln Pro Gln Gln Pro Phe Pro Gln Gln
   1               5                   10                  15
   Pro Gln Gln Pro
               20
<210> 5084
   <211> 20
   <212> PRT
   <213> Hordeum vulgare
<400> 5084
   Pro Gln Pro Phe Pro Gln Gln Pro Ile Pro Gln Gln Pro Gln Pro Tyr
   1               5                   10                  15
   Pro Gln Gln Pro
               20
<210> 5085
   <211> 20
   <212> PRT
   <213> Hordeum vulgare
<400> 5085
   Gln Gln Pro Phe Pro Gln Gln Pro Phe Pro Gln Gln Pro Gln Pro Tyr
   1               5                   10                  15
   Pro Gln Gln Pro
               20
<210> 5086
   <211> 20
   <212> PRT
   <213> Hordeum vulgare
<400> 5086
   Pro Gln Gln Pro Gln Gln Pro Phe Pro Gln Pro Gln Gln Pro Phe Ser
   1 5 10 15
   Trp Gln Pro Gln
   20
<210> 5087
   <211> 20
   <212> PRT
   <213> Hordeum vulgare
<400> 5087
   Gln Pro Gln Pro Tyr Pro Gln Gln Pro Gln Pro Tyr Pro Gln Gln Pro
   1 5 10 15
   Phe Gln Pro Gln
   20
<210> 5088
   <211> 20
   <212> PRT
   <213> Hordeum vulgare
<400> 5088
   Gln Gln Pro Phe Pro Gln Gln Pro Phe Pro Gln Gln Pro Gln Pro Tyr
   1 5 10 15
   Pro Gln Gln Pro
   20
<210> 5089
   <211> 20
   <212> PRT
   <213> Hordeum vulgare
<400> 5089
   Pro Gln Pro Tyr Pro Gln Gln Pro Gln Pro Phe Pro Gln Gln Pro Pro
   1 5 10 15
   Phe Cys Gln Gln
   20
<210> 5090
   <211> 20
   <212> PRT
   <213> Hordeum vulgare
<400> 5090
   Phe Gln Gln Pro Gln Gln Ser Tyr Pro Val Gln Pro Gln Gln Pro Phe
   1 5 10 15
   Pro Gln Pro Gln
   20
<210> 5091
   <211> 20
   <212> PRT
   <213> Hordeum vulgare
<400> 5091
   Tyr Pro Gln Gln Pro Gln Pro Phe Pro Gln Gln Pro Ile Pro Gln Gln
   1 5 10 15
   Pro Gln Pro Tyr
   20
<210> 5092
   <211> 20
   <212> PRT
   <213> Hordeum vulgare
<400> 5092
   Gln Gln Gln Pro Phe Pro Gln Gln Pro Ile Pro Gln Gln Pro Gln Pro
   1 5 10 15
   Tyr Pro Gln Gln
   20
<210> 5093
   <211> 20
   <212> PRT
   <213> Hordeum vulgare
<400> 5093
   Gln Pro Gln Gln Pro Gln Pro Phe Pro Gln Gln Pro Val Pro Gln Gln
   1 5 10 15
   Pro Gln Pro Tyr
   20
<210> 5094
   <211> 20
   <212> PRT
   <213> Hordeum vulgare
<400> 5094
   Gln Pro Gln Pro Phe Pro Gln Gln Pro Ile Pro Leu Gln Pro His Gln
   1 5 10 15
   Pro Tyr Thr Gln
   20
<210> 5095
   <211> 20
   <212> PRT
   <213> Hordeum vulgare
<400> 5095
   Leu Pro Arg Pro Gln Gln Pro Phe Pro Trp Gln Pro Gln Gln Pro Phe
   1 5 10 15
   Pro Gln Pro Gln
   20
<210> 5096
   <211> 20
   <212> PRT
   <213> Hordeum vulgare
<400> 5096
   Gln Gln Pro Phe Pro Leu Gln Pro Gln Gln Pro Phe Pro Gln Pro Gln
   1 5 10 15
   Pro Phe Pro Gln
   20
<210> 5097
   <211> 20
   <212> PRT
   <213> Hordeum vulgare
<400> 5097
   Pro Phe Pro Gln Gln Pro Gln Gln Pro Phe Pro Gln Pro Gln Gln Pro
   1 5 10 15
   Phe Arg Gln Gln
   20
<210> 5098
   <211> 20
   <212> PRT
   <213> Hordeum vulgare
<400> 5098
   Pro Gln Gln Pro Phe Gln Pro Gln Gln Pro Phe Pro Gln Gln Thr Ile
   1               5                   10                  15
   Pro Gln Gln Pro
               20
<210> 5099
   <211> 20
   <212> PRT
   <213> Hordeum vulgare
<400> 5099
   Asn Pro Leu Gln Pro Gln Gln Pro Phe Pro Leu Gln Pro Gln Pro Pro
   1               5                   10                  15
   Gln Gln Pro Phe
               20
<210> 5100
   <211> 20
   <212> PRT
   <213> Hordeum vulgare
<400> 5100
   Asn Pro Leu Gln Pro Gln Gln Pro Phe Pro Leu Gln Pro Gln Pro Pro
   1               5                   10                  15
   Gln Gln Pro Phe
               20
<210> 5101
   <211> 20
   <212> PRT
   <213> Hordeum vulgare
<400> 5101
   Pro Phe Pro Gln Gln Pro Gln Gln Pro Phe Pro Gln Pro Gln Gln Pro
   1               5                   10                  15
   Phe Arg Gln Gln
               20
<210> 5102
   <211> 20
   <212> PRT
   <213> Hordeum vulgare
<400> 5102
   Gln Pro Gln Gln Pro Phe Pro Leu Gln Pro Gln Gln Pro Phe Pro Trp
   1 5 10 15
   Gln Pro Gln Gln
   20
<210> 5103
   <211> 20
   <212> PRT
   <213> Hordeum vulgare
<400> 5103
   Thr Phe Pro Pro Ser Gln Gln Pro Asn Pro Leu Gln Pro Gln Gln Pro
   1 5 10 15
   Phe Pro Leu Gln
   20
<210> 5104
   <211> 20
   <212> PRT
   <213> Hordeum vulgare
<400> 5104
   Pro Gln Gln Thr Ile Pro Gln Gln Pro Gln Gln Pro Phe Pro Leu Gln
   1 5 10 15
   Pro Gln Gln Pro
   20
<210> 5105
   <211> 20
   <212> PRT
   <213> Hordeum vulgare
<400> 5105
   Gln Pro Gln Gln Pro Phe Ser Phe Ser Gln Gln Pro Gln Gln Pro Phe
   1 5 10 15
   Pro Leu Gln Pro
   20
<210> 5106
   <211> 20
   <212> PRT
   <213> Hordeum vulgare
<400> 5106
   Gln Gln Pro Phe Pro Gln Gln Pro Phe Pro Gln Gln Pro Gln Pro Tyr
   1 5 10 15
   Pro Gln Gln Pro
   20
<210> 5107
   <211> 20
   <212> PRT
   <213> Secale cereale
<400> 5107
   Gln Gln Leu Pro Leu Gln Pro Gln Gln Pro Phe Pro Gln Pro Gln Gln
   1 5 10 15
   Pro Ile Pro Gln
   20
<210> 5108
   <211> 20
   <212> PRT
   <213> Secale cereale
<400> 5108
   Ser Ile Pro Gln Pro Gln Gln Pro Phe Pro Gln Pro Gln Gln Pro Phe
   1 5 10 15
   Pro Gln Ser Gln
   20
<210> 5109
   <211> 20
   <212> PRT
   <213> Secale cereale
<400> 5109
   Gln Pro Phe Pro Gln Pro Gln Gln Pro Thr Pro Ile Gln Pro Gln Gln
   1 5 10 15
   Pro Phe Pro Gln
   20
<210> 5110
   <211> 20
   <212> PRT
   <213> Secale cereale
<400> 5110
   Gln Pro Phe Pro Gln Pro Gln Gln Pro Thr Pro Ile Gln Pro Gln Gln
   1 5 10 15
   Pro Phe Pro Gln
   20
<210> 5111
   <211> 20
   <212> PRT
   <213> Secale cereale
<400> 5111
   Pro Ala Pro Ile Gln Pro Gln Gln Pro Phe Pro Gln Gln Pro Gln Gln
   1 5 10 15
   Pro Phe Pro Gln
   20
<210> 5112
   <211> 20
   <212> PRT
   <213> Secale cereale
<400> 5112
   Pro Gln Gln Pro Phe Pro Gln Gln Pro Glu Gln Ile Ile Pro Gln Gln
   1 5 10 15
   Pro Gln Gln Pro
   20
<210> 5113
   <211> 20
   <212> PRT
   <213> Secale cereale
<400> 5113
   Gln Tyr Ser Pro Tyr Gln Pro Gln Gln Pro Phe Pro Gln Pro Gln Gln
   1 5 10 15
   Pro Thr Pro Ile
   20
<210> 5114
   <211> 20
   <212> PRT
   <213> Secale cereale
<400> 5114
   Ser Gln Gln Pro Gln Arg Pro Gln Gln Pro Phe Pro Gln Gln Pro Gln
   1 5 10 15
   Gln Ile Ile Pro
   20
<210> 5115
   <211> 20
   <212> PRT
   <213> Secale cereale
<400> 5115
   Gln Gln Leu Pro Leu Gln Pro Gln Gln Pro Phe Pro Gln Pro Gln Gln
   1               5                   10                  15
   Pro Ile Pro Gln
               20
<210> 5116
   <211> 20
   <212> PRT
   <213> Secale cereale
<400> 5116
   Phe Pro Leu Gln Pro Gln Gln Pro Phe Pro Gln Gln Pro Glu Gln Ile
   1               5                   10                  15
   Ile Ser Gln Gln
               20
<210> 5117
   <211> 20
   <212> PRT
   <213> Secale cereale
<400> 5117
   Pro Gln Gln Pro Phe Pro Gln Gln Pro Glu Gln Ile Ile Pro Gln Gln
   1               5                   10                  15
   Pro Gln Gln Pro
               20
<210> 5118
   <211> 20
   <212> PRT
   <213> Secale cereale
<400> 5118
   Phe Pro Gln Gln Pro Gln Gln Pro Phe Pro Gln Pro Gln Gln Gln Leu
   1               5                   10                  15
   Pro Leu Gln Pro
               20
<210> 5119
   <211> 20
   <212> PRT
   <213> Secale cereale
<400> 5119
   Ser Pro Gln Pro Gln Gln Pro Tyr Pro Gln Gln Pro Phe Pro Gln Gln
   1               5                   10                  15
   Pro Gln Gln Pro
               20
<210> 5120
   <211> 20
   <212> PRT
   <213> Secale cereale
<400> 5120
   Gln Gln Pro Gln Gln Pro Phe Pro Leu Gln Pro Gln Gln Pro Val Pro
   1               5                   10                  15
   Gln Gln Pro Gln
               20
<210> 5121
   <211> 20
   <212> PRT
   <213> Secale cereale
<400> 5121
   Gln Pro Gln Gln Ile Ile Pro Gln Gln Pro Gln Gln Pro Phe Pro Leu
   1               5                   10                  15
   Gln Pro Gln Gln
               20
<210> 5122
   <211> 20
   <212> PRT
   <213> Secale cereale
<400> 5122
   Pro Gln Gln Pro Phe Pro Gln Gln Pro Glu Gln Ile Ile Pro Gln Gln
   1               5                   10                  15
   Pro Gln Gln Pro
               20
<210> 5123
   <211> 20
   <212> PRT
   <213> Secale cereale
<400> 5123
   Gln Thr Gln Gln Ser Ile Pro Gln Pro Gln Gln Pro Phe Pro Gln Pro
   1               5                   10                  15
   Gln Gln Pro Phe
               20
<210> 5124
   <211> 20
   <212> PRT
   <213> Secale cereale
<400> 5124
   Gln Gln Pro Phe Leu Leu Gln Pro Gln Gln Pro Phe Ser Gln Pro Gln
   1               5                   10                  15
   Gln Pro Phe Leu
               20
<210> 5125
   <211> 20
   <212> PRT
   <213> Secale cereale
<400> 5125
   Gln Gln Pro Gln Gln Pro Phe Pro Leu Gln Pro Gln Gln Pro Val Pro
   1               5                   10                  15
   Gln Gln Pro Gln
               20
<210> 5126
   <211> 20
   <212> PRT
   <213> Secale cereale
<400> 5126
   Glu Gln Ile Ile Ser Gln Gln Pro Phe Pro Leu Gln Pro Gln Gln Pro
   1               5                   10                  15
   Phe Ser Gln Pro
               20
<210> 5127
   <211> 20
   <212> PRT
   <213> Secale cereale
<400> 5127
   Asn Met Gln Val Gly Pro Ser Gly Gln Val Glu Trp Pro Gln Gln Gln
   1               5                   10                  15
   Pro Leu Pro Gln
               20
<210> 5128
   <211> 20
   <212> PRT
   <213> Secale cereale
<400> 5128
   Pro Gln Gln Leu Phe Pro Leu Pro Gln Gln Pro Phe Pro Gln Pro Gln
   1               5                   10                  15
   Gln Pro Phe Pro
               20
<210> 5129
   <211> 20
   <212> PRT
   <213> Secale cereale
<400> 5129
   Pro Gln Thr Gln Gln Pro Gln Gln Pro Phe Pro Gln Pro Gln Gln Pro
   1               5                   10                  15
   Gln Gln Leu Phe
               20
<210> 5130
   <211> 20
   <212> PRT
   <213> Secale cereale
<400> 5130
   Ser Pro Gln Gln Pro Gln Leu Pro Phe Pro Gln Pro Gln Gln Pro Phe
   1               5                   10                  15
   Val Val Val Val
               20
<210> 5131
   <211> 20
   <212> PRT
   <213> Secale cereale
<400> 5131
   Phe Pro Gln Gln Pro Glu Gln Ile Ile Ser Gln Gln Pro Gln Gln Pro
   1 5 10 15
   Phe Pro Leu Gln
   20
<210> 5132
   <211> 20
   <212> PRT
   <213> Secale cereale
<400> 5132
   Pro Ala Pro Ile Gln Pro Gln Gln Pro Phe Pro Gln Gln Pro Gln Gln
   1 5 10 15
   Pro Phe Pro Gln
   20
<210> 5133
   <211> 20
   <212> PRT
   <213> Secale cereale
<400> 5133
   Pro Gln Glu Pro Gln Gln Leu Phe Pro Gln Ser Gln Gln Pro Gln Gln
   1 5 10 15
   Pro Phe Pro Gln
   20
<210> 5134
   <211> 20
   <212> PRT
   <213> Secale cereale
<400> 5134
   Ser Pro Gln Pro Gln Gln Pro Tyr Pro Gln Gln Pro Phe Pro Gln Gln
   1 5 10 15
   Pro Gln Gln Pro
   20
<210> 5135
   <211> 20
   <212> PRT
   <213> Secale cereale
<400> 5135
   Pro Thr Pro Ile Gln Pro Gln Gln Pro Phe Pro Gln Arg Pro Gln Gln
   1               5                   10                  15
   Pro Phe Pro Gln
   20
<210> 5136
   <211> 15
   <212> PRT
   <213> Artificial Sequence
<220>
   <223> NPL033
<400> 5136
   Leu Gln Pro Phe Pro Gln Pro Glu Leu Pro Tyr Pro Gln Pro Gln
   1               5                   10                  15
<210> 5137
   <211> 14
   <212> PRT
   <213> Artificial Sequence
<220>
   <223> NPL038
<400> 5137
   Gln Pro Phe Pro Gln Pro Glu Gln Pro Phe Pro Trp Gln Pro
   1               5                   10
<210> 5138
   <211> 15
   <212> PRT
   <213> Artificial Sequence
<220>
   <223> NPL034
<400> 5138
   Pro Glu Gln Pro Ile Pro Glu Gln Pro Gln Pro Tyr Pro Gln Gln
   1               5                   10                  15
<210> 5139
   <211> 16
   <212> PRT
   <213> Artificial Sequence
<220>
   <223> gluten derived peptide
<400> 5139
   Pro Gln Gln Pro Phe Pro Gln Pro Glu Gln Pro Phe Pro Trp Gln Pro
   1               5                   10                  15
<210> 5140
   <211> 16
   <212> PRT
   <213> Artificial Sequence
<220>
   <223> gluten derived peptide
<400> 5140
   Phe Pro Glu Gln Pro Ile Pro Glu Gln Pro Gln Pro Tyr Pro Gln Gln
   1               5                   10                  15

## Description

### RELATED APPLICATIONS

The application claims priority to U.S. Provisional Patent Application No. 61/865,389, filed August 13, 2013, U.S. Provisional Patent Application No. 61/869,279, filed August 23, 2013 and U.S Provisional Patent Application No. 61/887,112, filed October 4, 2013. This application is also related to PCT Application No. PCT/US2013/047079 filed June 21, 2013 which claims priority to U.S. Provisional Application no. 61/662,687, filed June 21, 2012.

### BACKGROUND OF INVENTION

Inflammatory diseases and disorders are conditions in which an abnormal or otherwise deregulated inflammatory response contributes to the etiology or severity of disease. Examples include autoimmune diseases such as type 1 diabetes and Celiac disease.

Many of these diseases are characterized by a mononuclear cell infiltration at a site of tissue injury or other insult. Examples of mononuclear cells that have been observed in these infiltrations include lymphocytes, especially T lymphocytes, and cells of the mononuclear phagocyte system (MPS cells) such as monocytes, macrophages, dendritic cells, microglial cells and others.

Many of the cells observed in the mononuclear cell infiltrates are suspected of having a role in these abnormal inflammatory responses. For example, in diseases such as multiple sclerosis, CD4⁺ T cells are known to play a central role in the pathologic autoimmune response. At an earlier time point in T cell activation, dendritic cells and other MPS cells may be responsible for activation of CD4⁺ T cells. MPS cells could also contribute to inflammation through phagocytosis although in at least some inflammatory diseases it is not clear whether such cells would be capable of this in the absence of CD4⁺ T cells.

Peripheral blood monocytes may be classified into one of two groups according to the expression or not of certain cell surface molecules. In particular, human "resident monocytes" or "mature monocytes" are understood to have a CD14^{lo}CD16⁺ phenotype (the mouse counterpart is CX₃CR1^{hi}CCR2⁻Gr1⁻). Another group of cells, the "inflammatory monocytes" or "immature monocytes" are understood to have a CD14⁺CD16⁻ phenotype (the mouse counterpart is CX₃CR1^{lo}CCR2⁺Gr1⁺). (Geissmann F. et al. 2003 Immunity 19: 71-82)

Importantly, while the latter are understood to be "inflammatory" in the sense that they are observed to migrate into inflamed tissue from bone marrow derived peripheral blood cells, these cells have not been shown to cause inflammation either directly or through the action of other cells. Further, the various MPS cells that may be formed when these cells differentiate have also not been shown to cause inflammation.

Conventional clinical strategies for general long-term immunosuppression in disorders associated with an undesired immune response are based on the long-term administration of broad acting immunosuppressive drugs, for example, signal 1 blockers such as cyclosporin A (CsA), FK506 (tacrolimus) and corticosteroids. Long-term use of high doses of these drugs can have toxic side-effects. Moreover, even in those patients that are able to tolerate these drugs, the requirement for life-long immunosuppressive drug therapy carries a significant risk of severe side effects, including tumors, serious infections, nephrotoxicity and metabolic disorders.

Methods of inducing antigen-specific tolerance have been developed, including cell coupling of an antigen or peptide. For example, in one method, peptide induced cell coupled tolerance involved collection, separation and treatment of peripheral blood cells with disease specific autoantigens and the ethylene carbodimide (ECDI) coupling reagent under sterile conditions, and subsequent re-infusion into the donor/patient. This process is costly and must be conducted under closely monitored conditions by skilled practitioners and is limited in the number of centers that can conduct the procedure. The use of red blood cells as the donor cell type expands the potential source to include allogeneic donors thus increasing the supply of source cells dramatically and potentially expanding the delivery of this therapy to any setting certified for blood transfusion. These approaches have significant limitations in terms of supply of source cells and necessity for tissue type matching to minimize immune response to the donor cells. In addition the local treatment of the cells to couple autoantigens via EDCI presents a significant quality control issue. Furthermore, these approaches also require at least some knowledge of the pathological antigen for which immune tolerance is sought.

Recently, peptide-coupled particles have been described which eliminates the requirement for a supply of source cells and circumvents the tissue-typing requirement of the prior approaches, *See* WO 2010/085509. Not withstanding, the use of antigens coupled to the outside of particles is associated with increased anaphylaxis and has significant chemistry, manufacturing and control issues. Surprisingly, when the antigen is encapsulated within the particle, these adverse events are avoided. Even more surprisingly, the size and the charge can be altered to enhance tolerance to specific antigens.

Antigen-specific tolerance is generally not ideal because specific antigens/epitopes are generally not known in human diseases. Furthermore, antigens can vary from subject to subject in order for an antigen specific approach to be effective, therefore it would be necessary to determine which antigens each individual patient would recognize, or it would require coupling a library of possible peptides to the particles prior to administration. The synthesis and individual coupling of these peptides is both time consuming and expensive. Therefore, a need exists for a therapy which solves both of these problems thereby eliminating the need to for a source of tissue matched cells.

### SUMMARY OF THE INVENTION

The invention is as defined in the claims.

In some instances, the present disclosure provides compositions (e.g., for induction of antigen-specific tolerance) comprising a carrier particle (e.g., PLG particle) attached to an antigenic peptide. In certain embodiments, the carrier particle is a poly(lactide-co-glycolide) (PLG) particle. In other embodiments, the carrier particle is a PLURIONICS^{®} stabilized polypropylene sulfide particle.

In some instances, the present disclosure provides compositions comprising: an antigen coupled to a carrier particle with a negative zeta potential. In some instances of the disclosure, the zeta potential of the particle is from about -100 mV to about 0 mV. In some embodiments, the zeta potential of the particle is from about -50 mV to about - 40 mV. In some embodiments, the particle is a co-polymer having a molar ratio from about 50:50, 80:20 to about 100:0. In some embodiments, the co-polymers ratio may be, but not limited to, polystyrene:poly(vinyl carboxylate)/80:20, polystyrene: poly(vinyl carboxylate)/90:10, poly(vinyl carboxylate):polystyrene/80:20, poly(vinyl carboxylate):polystyrene/90:10, polylactic acid: polyglycolic acid/80:20, or polylactic acid: polyglycolic acid/90:10, or polylactic acid: polyglycolic acid/50:50. Yet in other embodiments, the particle is a polystyrene particle, a carboxylated polysterene particle, PLURIONICS^{®} stabilized polypropylene sulfide particle, or a poly(lactic-co-glycolic acid) particle. In some embodiments, the particle is a poly(lactic-co-glycolic acid) particle.

The particle has an average diameter of between 0.1 µm to 10 µm. In the invention, the particle has an average diameter of between 0.2µm and about 2µm. In some instances of the disclosure, the particle has a diameter of between about 0.3 µm to about 5 µm. In some instances of the disclosure, the particle has a diameter of between about 0.5 µm to about 3 µm. In some embodiments, the particle has a diameter of between about 0.5 µm to about 1 µm. In some embodiments, the particle has a diameter of about 0.5 µm.

In further instances of the disclosure, the antigen comprises at least a portion of an autoimmune antigen, an antigen expressed on a tissue to be transplanted into a subject, an enzyme, or an allergen. In some instances of the disclosure, the antigen comprises at least a portion of myelin basic protein, acetylcholine receptor, endogenous antigen, myelin oligodendrocyte glycoprotein, pancreatic beta-cell antigen, insulin, proinsulin, islet-specific glucose-6-phophatase catalytic subunit-related protein (IGRP), glutamic acid decarboxylase (GAD), collagen type 11, human cartilage gp39, fp130-RAPS, proteolipid protein, fibrillarin, small nucleolar protein, thyroid stimulating factor receptor, histones, glycoprotein gp70, pyruvate dehydrogenase dehydrolipoamide acetyltransferase (PCD-E2), hair follicle antigen, aqua porin 4, Desmoglein 1, Desmoglein 3, nicotinic acetylcholine receptor, A-gliaden, and human tropomyosin isoform 5, Bahia grass pollen (BaGP), peach allergen Pru p 3, alpha s 1-Caein Milk allergen, Apig1 celery allergen, Bere1 Brazil nut allergen, B-Lactoglobulin Milk allergen, Bovine serum albumin, Cor a 1.04 Hazelnut allergen, Ovalbumin Egg allergen, Advate, antihemophilic factor, Kogenate, Eloctate, recombinant factor VIII Fc fusion protein, Refacto, Novo VIIa, recombinant factor VII, eptacog alfa, Helixate, Monanine, Coagulation Factor IX, Wilate, Ceredase, Alglucerase, Cerezyme, Imiglucerase, Elelso, taliglucerase alfa, Fabrazyme, Agalsidase beta, Aldurazyme, -I-iduronidase, Myozyme, Acid-glucosidase, Elaprase, iduronate-2-sulfatase, Naglazyme arylsufatase B, or N-acetylgalactosamin e-4-sulfatase, proteinaceous therapies used in enzyme or coagluation factor replacement such as myozyme, alglucerase, imiglucerase, taliglucerase, agalsidase beta, 1-iduronidase, acid glucosidase, Iduronate-2-sulfatase, N-acetylgalactosamnie-4-sulfatase, antihemophilic factor, factor VII, eptacogalfa, factor IX, miglustat, romiplastim, epotetin alpha, protein C, laronidase, lumizyme or Factor VIII.

In further instances of the disclosure, the antigen comprises an autoimmune antigen, an antigen expressed on a tissue to be transplanted into a subject, an enzyme, or an allergen. In instances of the disclosure, the antigen comprises, for example, myelin basic protein, acetylcholine receptor, endogenous antigen, myelin oligodendrocyte glycoprotein, pancreatic beta-cell antigen, insulin, glutamic acid decarboxylase (GAD), collagen type 11, human cartilage gp39, fp130-RAPS, proteolipid protein, fibrillarin, small nucleolar protein, thyroid stimulating factor receptor, histones, glycoprotein gp70, pyruvate dehydrogenase dehydrolipoamide acetyltransferase (PCD-E2), hair follicle antigen, aqua porin 4, Desmoglein 1, Desmoglein 3, nicotinic acetylcholine receptor, A-gliaden, and human tropomyosin isoform 5, Bahia grass pollen (BaGP), peach allergen Pru p 3, alpha s 1-Caein Milk allergen, Apig1 celery allergen, Bere1 Brazil nut allergen, B-Lactoglobulin Milk allergen, Bovine serum albumin, Cor a 1.04 Hazelnut allergen, insulin, proinsulin, islet-specific glucose-6-phophatase catalytic subunit-related protein (IGRP), Ovalbumin Egg allergen, proteinaceous therapies used in enzyme or coagluation factor replacement such as myozyme, alglucerase, imiglucerase, taliglucerase, agalsidase beta, 1-iduronidase, acid glucosidase, Iduronate-2-sulfatase, N-acetylgalactosamnie-4-sulfatase, antihemophilic factor, factor VII, eptacogalfa, factor IX, miglustat, romiplastim, epotetin alpha, protein C, laronidase, lumizyme Factor VIII.

In further instances of the disclosure, the particles are coupled to an antigen comprising one or more epitopes. In a further instance of the disclosure the epitope is associated with an allergy, an autoimmune disease, an enzyme used in enzyme replacement therapy, lysosomal storage disase, or an inflammatory disease or disorder. In one instance of the disclosure, the epitope is associated with type 1 diabetes, multiple sclerosis, Systemic Lupus, Neuromyelitis Optica, Idiopathic Thrombocytopenic Purpura, Thrombotic Thrombocytopenic Purpura, Membranous Nephropathy, Bullous Phemphigoid, Phemphigus Vulgaris, Myasthenia Gravis, a mucopolysaccharide storage disorder, gangliosidosis, alkaline hypophosphatasia, cholesterol ester storage disease, hyperuricemia, growth hormone deficiency, renal anemia, Gaucher's disease, Fabry's disease, Hurler's disease, Hunter's disease, Maroteaux-Lamy disease, hemophilia A, hemophilia B, von Wilebrand disease, venous thrombosis, purpura fulminans, mucopolysaccaridosis VI, pompe disease, Celiac's disease, or inflammatory bowel disease, including Crohn's disease or colitis, e.g. ulcerative colitis. In a further instance of the disclosure the epitopes are found within proteinaceous therapies used in enzyme or coagluation factor replacement such as myozyme, alglucerase, imiglucerase, taliglucerase, agalsidase beta, 1-iduronidase, acid glucosidase, Iduronate-2-sulfatase, N-acetylgalactosamnie-4-sulfatase, antihemophilic factor, factor VII, eptacogalfa, factor IX, miglustat, romiplastim, epotetin alpha, protein C, laronidase, lumizyme Factor VIII. In a further instance of the disclosure, the epitope is an epitope described in Tables 2 or 3. In one embodiment of the invention, the particles are coupled to antigens comprising only one epitope associated with one disease and/or disorder. In a further embodiment of the invention, antigens comprise more than one epitope associated with the same disease and/or disorder. In a further embodiment of the invention, the antigens comprise more than one epitope associated with different diseases and/or disorders.

In some instances of the disclosure, the antigen is coupled to said particle by a conjugate molecule. In some instances of the disclosure, the antigen is coupled to said particle by a linker. In some instances of the disclosure, the conjugate molecule is ethylene carbodiimide (ECDI). In certain instances of the disclosure, the antigen is linked by a streptavidin-biotin complex. In some instances of the disclosure, the linkers can include, but are not limited to, a variety of bifunctional protein coupling agents such as N-succinimidyl-3-(2-pyridyldithio)propionate (SPDP), succinimidyl-4-(N-maleimidomethyl)cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl)hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particular coupling agents include N-succinimidyl-3-(2-pyridyldithio)propionate (SPDP) and N-succinimidyl-4-(2-pyridylthio)pentanoate (SPP) to provide for a disulfide linkage.

In some instances of the disclosure, the antigen is coupled to the outside of the particle with a negative zeta potential. In the invention, the antigen is encapsulated in the particle which has a negative surface zeta potential. In the invention, the particle is biodegradable. In the invention, the particle is surface functionalized. In some embodiments, the particle is carboxylate surface functionalized.

In some instances, the present discloure provides methods of inducing antigen-specific tolerance in a subject comprising: administering to said subject an effective amount of a composition comprising an antigen-coupled particle to said subject, wherein said particle has a negative zeta potential, and wherein said particle and antigen induce tolerance of said antigen in said subject. In some instances of the disclosure, administering is performed to treat or prevent a disease or condition. In some instances of the disclosure, administering is performed prior or subsequent to onset of a disease or condition that is caused by said antigen. In some instances of the disclosure, the disease or condition is selected from the group consisting of: an autoimmune disease, inflammatory disease, an allergy, transplantation rejection, a lysosomal storage disease, an enzyme deficiency, inflammatory response and a hyperimmune response. In some embodiments, the disease or condition is selected from the group consisting of: multiple sclerosis, type 1 diabetes, asthma, a food allergy, an environmental allergy, Celiac disease, inflammatory bowel disease, including Crohn's disease or ulcerative colitis, and a condition caused by said antigen in said subject to reduce overreaction to said antigen. In some instances of the disclosure, methods further comprise repeating said administration of said composition into said subject.

In a further instance of the disclosure, the administration of the particles results in activation induced death of effector T cells.

In a further instance of the disclosure, the administration of the particles results in anergy of effector T cells.

In a further instance of the disclosure, the administration of particles results in apoptosis of effector T cells.

In a further instances of the disclosure, the administration of particles results in the conversion of effector T cells to regulatory T cells.

In a further instances of the disclosure, the administration of particles results in the induction and expansion of both antigen specific and non-specific regulatory T cells. In a further instance of the disclosure, the administration of particles results in the isolation of effector T cells in the lymph nodes and spleen inhibiting their ability to traffic to peripheral sites and cause inflammation.

In a further instance of the disclosure, the administration of particles results in the down regulation of T cell dependent antibody production.

The present invention provides composition for use in treating celiac disease in a subject comprising administering to said subject an effective amount of a composition comprising an antigen-coupled particle to the subject, wherein the particle has a negative zeta potential. The antigen is gliaden or a gliaden epitope. In some embodiments, the antigen is one or more antigens selected from the group consisting of SEQ ID NOs: 1295-1724, SEQ ID NOs: 1726-1766 and SEQ ID NOs: 4986-5140. In some instances of the disclosure, the antigen is gliaden and the antigen-coupled particle has a post-synthesis average size of about 600-1500 nanometers and a post-synthesis average charge of about -30 to about -80 mV. In some embodiments, the particle has a post-synthesis average size of about 600-1200 nanometers and a post-synthesis average charge of about -40 to about -70 mV. In certain embodiments, the particle has a post-synthesis average size of about 600 microns and a post-synthesis average charge of about -50 mV. In further embodiments, the particle is a polystyrene particle, a carboxylated polysterene particle, a PLURIONICS stabilized polypropylene sulfide particle, or a poly(lactic-co-glycolic acid) particle.
In some instances, the present disclosure provides methods of treating diabetes in a subject comprising administering to said subject an effective amount of a composition comprising an antigen-coupled particle to the subject, wherein the particle has a negative zeta potential. In some instances of the disclosure, the diabetes is type I diabetes. In some instances of the disclosure, the diabetes is type II diabetes.

In some instances of the disclosure, the antigen is insulin, proinsulin, islet-specific glucose-6-phophatase catalytic subunit-related protein (IGRP) or epitopes derived from insulin proinsulin, or IGRP. In some instances of the disclosure, the antigen is one or more antigen selected from the group consisting of ID NOs: 1767-1840, SEQ ID NOs: 1842-1962, SEQ ID NOs: 1964-2027, SEQ ID NOs: 2029-2073, SEQ ID NOs: 2075-2113, SEQ ID NOs: 2115-2197, SEQ ID NOs: 2199-2248, SEQ ID NOs: 2250-2259, SEQ ID NOs: 2261-2420, SEQ ID NOs: 2422-2486, and SEQ ID NOs: 2489-2505. In some instances of the disclosure, antigen is insulin and the antigen-coupled particle has a post-synthesis average size of about 300-800 nanometers and a post-synthesis average charge of about -30-to about -70 mV. In some embodiments, the particle has a post-synthesis average size of about 350-600 nanometers and a post-synthesis average charge of about -40 to about -60 mV. In some embodiments, the particle has a post-synthesis average size of about 500 nanometers and a post-synthesis average charge of about -50 mV. In some embodiments, the antigen is proinsulin and the antigen-coupled particle has a post-synthesis average size of about 300-800 nanometers and a post-synthesis average charge of about -30 to about -70 mV. In certain embodiments,the particle has a has a post-synthesis average size of about 400-600 nanometers and a post-synthesis average charge of about -40 to about -60 mV. In some embodiments, the particle has a post-synthesis average size of about 570 nanometers and a post-synthesis average charge of about -45. In some instances of the disclosure, the antigen is IGRP and the antigen-coupled particle has a post-synthesis average size of about 300-800 nanometers and a post-synthesis average charge of about -30 to about -70 mV. In some embodiments, the particle has a has a post-synthesis average size of about 400-700 nanometers and a post-synthesis average charge of about -40 to about -60 mV.In some embodiments, the particle has a a post-synthesis average size of about 600 nanometers and a post-synthesis average charge of about -40. In certain embodiments, the particle is a polystyrene particle, a carboxylated polysterene particle, a PLURIONICS stabilized polypropylene sulfide particle, or a poly(lactic-co-glycolic acid) particle.

In some instances, the present disclosure provides methods of treating a subject undergoing enzyme replacement therapy, comprising administering to said subject an effective amount of a composition comprising an antigen-coupled particle to the subject, wherein the particle has a negative zeta potential. In some instances of the disclosure, the subject is undergoing enzyme replacement therapy for treatment of a disease selected from the group consisting of Hemophilia, Hemophilia A, Hemophilia B, von Willebrand disease, a mucopolysaccharide storage disorder, gangliosidosis, alkaline hypophosphatasia, cholesterol ester storage disease, hyperuricemia, growth hormone deficiency, renal anemia Gaucher's Disease, Fabry's Disease, Hurler's Disease, Pompe's Disease, Hunter's Disease, and Maroteaux-Lary Disease. In some instances of the disclosure the antigen coupled particle comprises one or more enzyme selected from the group consisting of Advate, antihemophilic factor, Kogenate, Eloctate, recombinant factor VIII Fc fusion protein, Refacto, Novo VIIa, recombinant factor VII, eptacog alfa, Helixate, Monanine, Coagulation Factor IX, Wilate, Ceredase, Alglucerase, Cerezyme, Imiglucerase, Elelso, taliglucerase alfa, Fabrazyme, Agalsidase beta, Aldurazyme, -I-iduronidase, Myozyme, Acid-glucosidase, Elaprase, iduronate-2-sulfatase, Naglazyme arylsufatase B, and N-acetylgalactosamin e-4-sulfatase. In some embodiments, the particle is a polystyrene particle, a carboxylated polysterene particle, a PLURIONICS stabilized polypropylene sulfide particle, or a poly(lactic-co-glycolic acid) particle. In certain embodiments, the particle is a co-polymer having a molar ratio from about 80:20 to about 100:0. In certain embodiments, the particle is a polystyrene particle, a carboxylated polysterene particle, a PLURIONICS stabilized polypropylene sulfide particle, or a poly(lactic-co-glycolic acid) particle. In other embodiments, the particle is a poly(lactic-co-glycolic acid) particle and has a copolymer ratio of about 50:50 polylactic acid:polyglycolic acid. In some embodiments, the particle is a poly(lactic-co-glycolic acid) particle and has a copolymer ratio of about 50:50 polylactic acid:polyglycolic acid.

In a further instance of the disclosure, the administration of the particles of the invention prevents the accumulation of neutrophils and other granulocytes in a subject. In a further instance of the disclosure the particles of the invention are administered to a subject who has cancer.

In one instance of the disclosure, administration of the particles of the invention increases regeneration of damaged tissue. In a further instance of the disclosure, the particles increase regeneration of epithelial cells. In yet a further instance of the disclosure, the particles increase remyelination of neurons. In another instance of the disclosure, the subject has an autoimmune disease. In yet another instance of the disclosure, the subject has inflammatory bowel disease, including ulcerative colitis, and/or Crohn's disease. In yet another instance of the disclosure, the subject has multiple sclerosis.

In some instances of the disclosure the composition is administered intravenously. In some instances of the disclosure, the composition is administered subcutaneously, orally, intramuscularly, intra-lymphatically, portally or via aerosol. In one instances of the disclosure, administration of the negatively charged particles induces antigen-specific tolerance in a subject. In one instance of the disclosure, the particles that induce antigen-specific tolerance comprise one or more epitopes associated with an allergy, autoimmune disease, and/or inflammatory disease. In one instance of the disclosure, the epitopes are selected from those described in Tables 2 or 3. In one embodiment of the invention, the negatively charged particles are polystyrene, diamond, PLURONICS^{®} stabilized polypropylene sulfide, or poly(lacti-co-glycolic acid) particles. In one embodiment of the invention the particles are carboxylated. In one instance of the disclosure, the particles have a zeta potential of less than about -100 mV. In one instance of the disclosure, the particles have a zeta potential between about -75 mV and 0mV, for example, between -50mV and 0mV, or between -100mV and -50mV or between -75 mV and -50mV or between -50 mV and -40 mV. In one instance of the disclosure, the particle has an average diameter of about 0.1 µm to about 10 µm, for example from about 0.2µm to about 2µm or about 0.3 µm to about 5 µm, or 0.5 µm to about 3 µm or about 0.5 µm to about 1 µm.

In one instance of the disclosure, the subject has an autoimmune disease. In one instance of the disclosure, the autoimmune disease is multiple sclerosis, scleroderma, type-I diabetes, rheumatoid arthritis, thyroiditis, systemic lupus erythmatosis, Reynaud's syndrome, Sjorgen's syndrome, autoimmune uveitis, autoimmune myocarditis, inflammatory bowel disease, Amyotrophic Lateral Sclerosis (ALS), Systemic Lupus, Neuromyelitis Optica, Idiopathic Thrombocytopenic Purpura, Thrombotic Thrombocytopenic Purpura, Membranous Nephropathy, Bullous Phemphigoid, Phemphigus Vulgaris, Myasthenia Gravis, Celiac disease, ulcerative colitis, or Crohn's disease. In one embodiment of the invention, the particle comprises a full-length polypeptide or fragment thereof. In one instance of the disclosure, the particle comprises one or more myelin basic protein epitopes. In one instance of the disclosure, the myelin basic protein epitope is from SEQ ID NO: 4975 or SEQ ID NO: 4976. In one instance of the disclosure, the particles comprise one or more myelin oligodendrocyte glycoprotein epitopes. In one instance of the disclosure, the myelin oligodendrocyte glycoprotein epitope is from SEQ ID NO: 1 or SEQ ID NO: 4978. In one instance of the disclosure, the particle contains one or more insulin epitopes. In one instance of the disclosure, the one or more insulin epitopes is from SEQ ID NO: 4981. In one instance of the disclosure, the particle comprises one or more glutamic acid decarboxylase epitopes. In one instance of the disclosure, the glutamic acid decarboxylase epitopes is from SEQ ID NO: 4982. In one instance of the disclosure, the particle contains one or more proteolipid protein epitopes. In one instance of the disclosure, the proteolipid protein epitope is from SEQ ID NO: 4977. In the invention, the particle comprises one or more gliaden epitopes. In one embodiment of the invention, the gliaden epitopes comprise SEQ ID NOs: 4983-4985.

In some instances, the present disclosure further provides a process for the preparation an immune modified particle with a negative zeta potential said process comprising: contacting an immune modified particle precursor with a buffer solution under conditions effective to form the immune modified particle with a negative zeta potential. In some instance of the disclosure, the immune modified particle precursor is formed by co-polymerization. In some embodiments, the buffer solution has a basic pH. In some instance of the disclosure, buffer solution is sodium bicarbonate, potassium bicarbonate, lithium bicarbonate, potassium dihydrogen phosphate, sodium dihydrogen phosphate, or lithium dihydrogen phosphate.

In some embodiments, the present invention provides a composition comprising an antigen encapsulated within the core of a surface-functionalized liposome. In a further embodiment, the liposome is composed at a 30:30:40 ratio of phosphatidylcholine :phosphatidylglycerol:cholesterol. In yet a further instance of the disclosure, said antigen comprises an autoimmune antigen, an antigen expressed on a tissue to be transplanted into a subject, or an allergen. In some embodiments, the particle is a poly(lactic-co-glycolic acid) particle and has a copolymer ratio of about 50:50 polylactic acid:polyglycolic acid. In some embodiments, the particles comprise PEMA. In some embodiments, the PEMA is present at about 0.1% to about 2.0%.

### BRIEF DESCRIPTION OF THE DRAWINGS

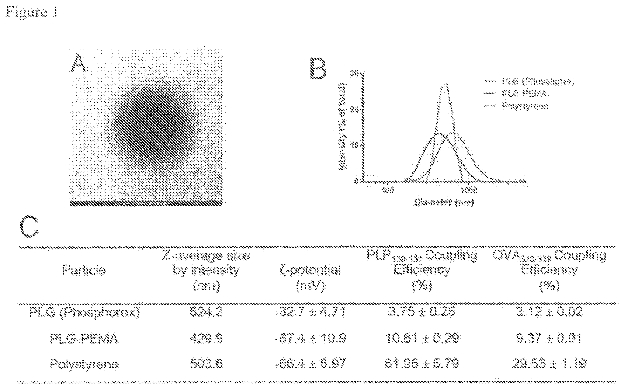

Figure 1 shows (A) a micrograph of a poly(lactide-co-glycolide) (PLG) particle. B and C show the characterization of surface-functionalized poly(lactide-co-glycolide) particles by dynamic light scattering analysis, including the size distribution, average size (nm), ζ potential (mV), and peptide coupling efficiency (%) of OVA₃₂₃₋₃₃₉ and PLP₁₃₉₋₁₅₁ peptides with PLG-PEMA particles. Surface-functionalized poly(lactide-co-glycolide) particles were analyzed on a Malvern Zetasizer Nano ZS (Malvern Instruments, Westborough, MA) at a count rate of 2.5 x 10⁵ counts per second in 18.2 MΩ water. The population of surface-functionalized poly(lactide-co-glycolide) particles varied by 5-15% per batch but generally had a Z-average diameter of 567 nm, a peak diameter of 670 nm and a polydispersity index of 0.209.

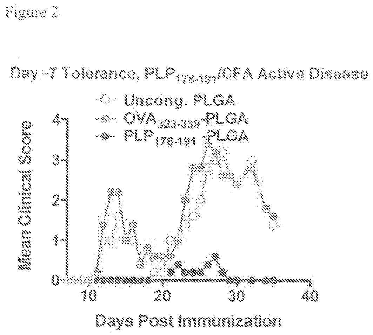

Figure 2 shows that PLG nanoparticles induce antigen-specific tolerance. The immunodominant proteolipid protein PLP₁₃₉₋₁₅₁ epitope (PLG-PLP₁₃₉₋₁₅₁) was used to induce tolerance for prevention of Relapsing Experimental Autoimmune Encephalitis (R-EAE). Mice were treated with either PLP₁₃₉₋₁₅₁-PLGA (N=5), OVA₃₂₃₋₃₃₉-PLGA (N=5), or uncongugated PLGA (N=5) on day -7 relative to the time of immunization (day 0). Peak disease was typically observed around day 12 to 14, and mice are scored for clinical disease. Particles without peptide, or modified with the control peptide OVA₃₂₃₋₃₃₉ did not prevent disease induction. However, PLGA particles modified with PLP₁₃₉₋₁₅₁ produced a clinical score of 0 (no disease) at all except low clinical scores of under 1 exhibited between days 20 and 30.

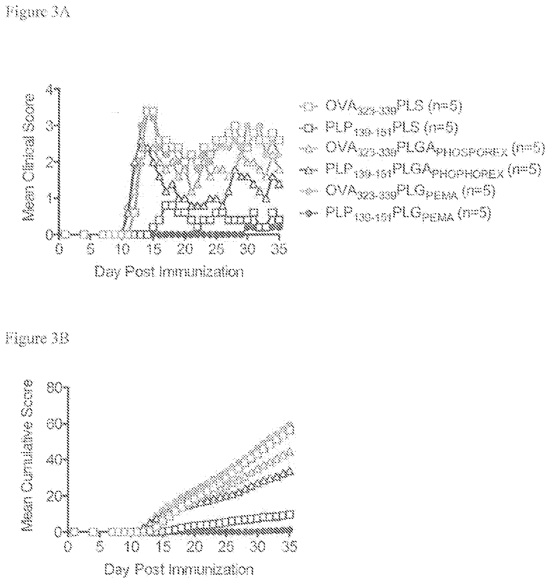

Figure 3 shows that the type of particle administered has an effect on the development of EAE in the mouse model. A) shows the mean clinical score and B) shows the mean cumulative score of the EAE animals. Mice were treated with either OVA₃₂₃₋₃₃₉-PLS (N=5), OVA₃₂₃₋₃₃₉-PLGA_{PHOSPOREX} (N=5), OVA₃₂₃₋₃₃₉-PLGA_{PEMA} (N=5), PLP₁₃₉₋₁₅₁-PLA (N=5), PLP₁₃₉₋₁₅₁-PLGA_{PHOSPHOREX} (N=5), or PLP₁₃₉₋₁₅₁-PLG_{PEMA} (N=5) on day -7 relative to the time of immunization (day 0). Peak disease was typically observed around day 12 to 14, and mice are scored for clinical disease. Particles, of any composition that were modified with the control peptide OVA₃₂₃₋₃₃₉ did not prevent disease induction. However, the PLP₁₃₉₋₁₅₁ coupled PLG beads were more effective in down-regulating induction of R-EAE than PLP₁₃₉₋₁₅₁ coupled commercial (phosphorex) PLG or polystyrene.

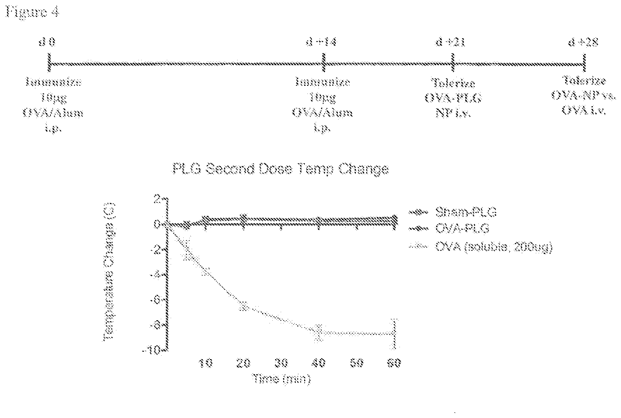

Figure 4 shows that those mice treated with soluble OVA on day 28 exhibited a decrease in temperature compared with those animals treated with the OVA-PLG particle. No decrease in body temperature was observed within 1 hour of delivering the particles.

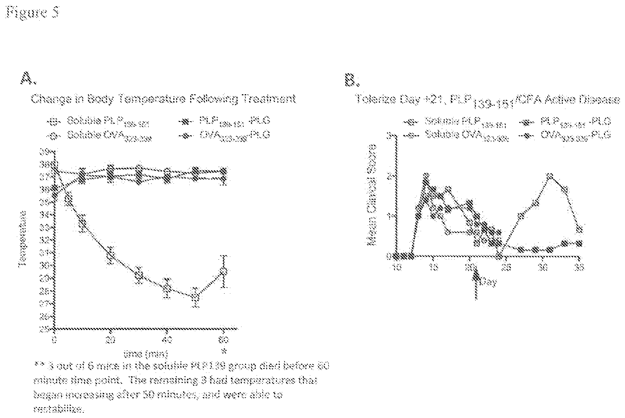

Figure 5 shows that administration of PLP-PLG during remission does not result in any anaphylaxis-associated mortality. EAE was induced in six to eight week old female SJL/J mice by subcutaneous injection of PLP₁₃₉₋₁₅₁ in CFA, and development of clinical disease was monitored and recorded (B). On day 21 relative to disease induction, mice were given iv injections of soluble PLP₁₃₉₋₁₅₁ (clear squares), soluble OVA₃₂₃₋₃₃₉ (clear circles), or the same peptides coupled to PLG nanoparticles (solids). Temperature of animals was monitored and recorded every 10 minutes for 1 hour following injection (A)

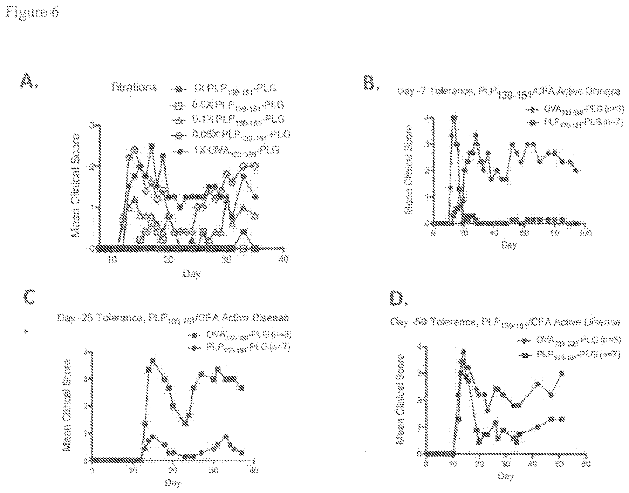

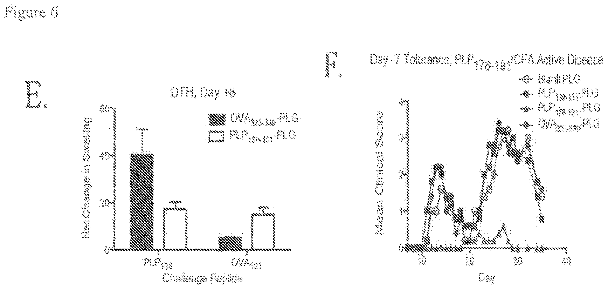

Figure 6 shows the optimal dosing of PLP₁₃₉₋₁₅₁-PLG administered intravenously seven days prior to disease induction. Development of clinical disease was measured in comparison to SJL/J mice treated with OVA₃₂₃₋₃₃₉ -PLG (A). Six to eight week old female SJL/J mice were injected iv with either PLP₁₃₉₋₁₅₁ (square)- or OVA₃₂₃₋₃₃₉ (circle)- coupled PLG nanoparticles. EAE was induced by subcutaneous injection of PLP₁₃₉₋₁₅₁ in CFA 7 days (B), 25 days (C), or 50 days (D) later. Animals from panel B were followed for clinical disease for 100 days. On day 8 relative to disease induction, a delayed-type hypersensitivity (DTH) reaction was carried out in a subset of the mice shown in panel B (E). Selected representative animals from the PLP₁₃₉₋₁₅₁/CFA primed groups in panel B (OVA₃₂₃₋₃₃₉-PLG and PLP₁₃₉₋₁₅₁-PLG) were ear-challenged with the priming PLP₁₃₉₋₁₅₁ epitope and the OVA₃₂₃₋₃₃₉ control peptide. Ear swelling as a measure of DTH was determined 24h later and responses prior to challenge were subtracted. Six to eight-week old female SJL/J mice were injected intravenously with PLP₁₇₈₋₁₉₁ (triangle)-, OVA₃₂₃₋₃₃₉ (circle), or PLP₁₃₉₋₁₅₁ (square)-coupled PLG nanoparticles, or with uncoupled particles alone (outlined circle) (F). EAE was induced 7 days afterward by subcutaneous injection of PLP₁₇₈₋₁₉₁ in CFA, and disease was monitored at the time points shown.

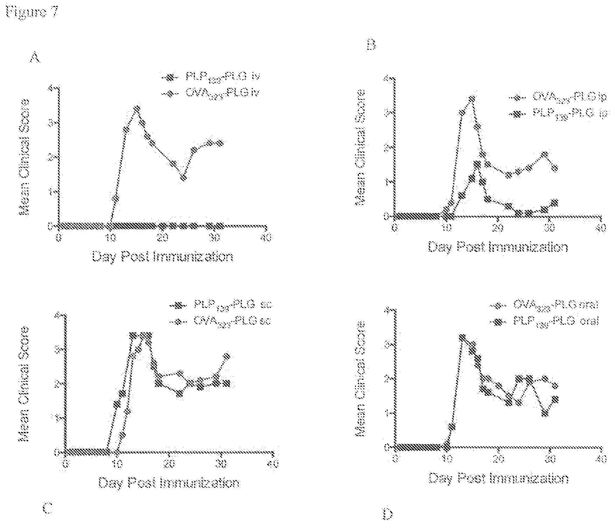

Figure 7A-D shows prophylactic tolerance is most efficient when the PLG-PLP₁₃₉₋₁₅₁ particles are administered either intravenously or intraperitoneally. Animals treated with PLP₁₃₉₋₁₅₁-PLG administered intravenously did not develop disease and had mean clinical scores of 0 at most time points.

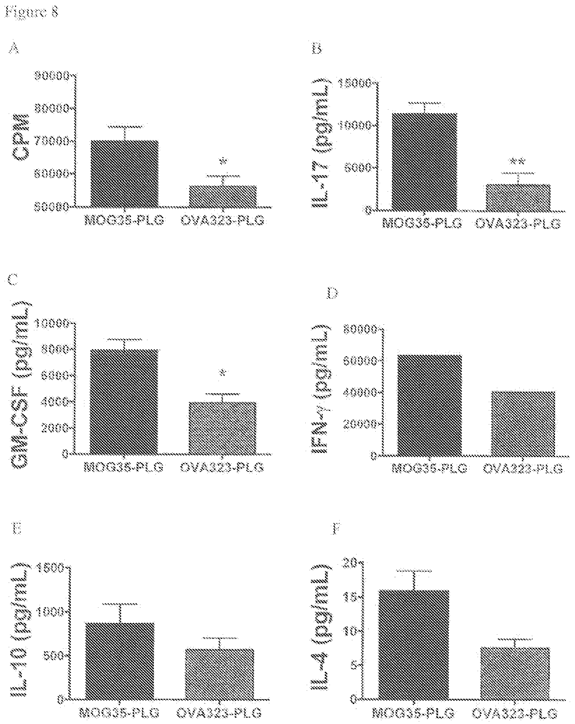

Figure 8A-F shows that the administration of OVA₃₂₃₋₃₃₉-PLG particles inhibited the Th1 and Th17 responses in the treated animals.

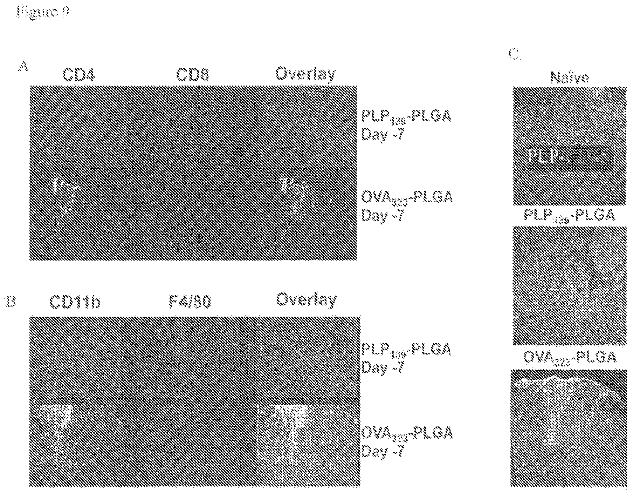

Figure 9A-C shows a reduction in immune cell infiltration within the spinal cord of animals treated with PLP₁₃₉₋₁₅₁-PLG that and was more similar to native tissue than to tissue from OVA₃₂₃₋₃₃₉-PLG treated animals. OVA₃₂₃₋₃₃₉-PLG treated animals had positive staining for CD45, CD4, and CD11b; whereas, PLP₁₃₉₋₁₅₁-PLG treated animals had minimal staining for these factors.

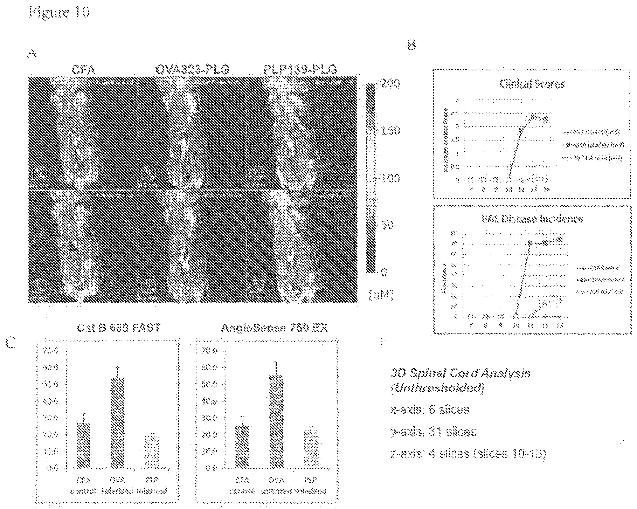

Figure 10A-C shows that administration of PLP₁₃₉₋₁₅₁-PLG particles inhibits blood brain barrier (BBB) disruption and macrophage activation in the spinal cord of treated mice. Animals were treated with either Complete Freund's Adjuvant (CFA), OVA₃₂₃₋₃₃₉ PLG particles, or PLP₁₃₉₋₁₅₁-PLG particles. The clinical scores and percent incidence of EAE were determined (B) and the spinal cords observed via *in vivo* imaging (A and C).

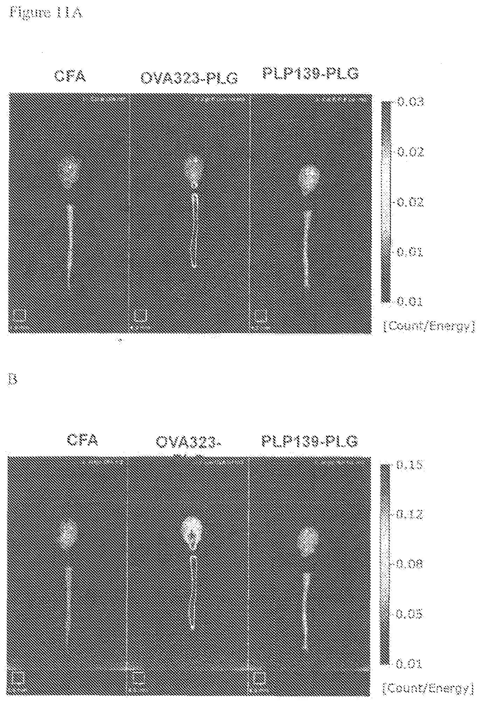

Figure 11A and B shows the spinal cords of treated mice via *in vivo* imaging. C-F are graphs showing the quantification of the image data.

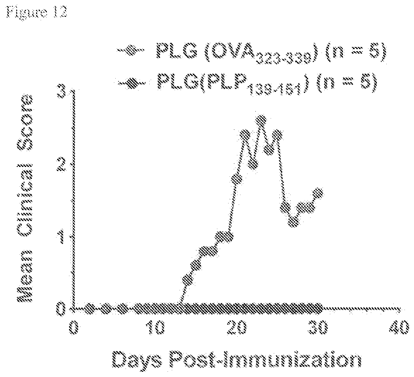

Figure 12 shows that the administration of PLG particles in which PLP₁₃₉₋₁₅₁ has been encapsulated inhibits the induction of R-EAE in mice. The ability to encapsulate autoantigens allows for the used of complex mixtures of proteins or even organ homogenates not possible with surface binding, allowing for more antigen coverage and thus more effectively deal with epitope spreading.

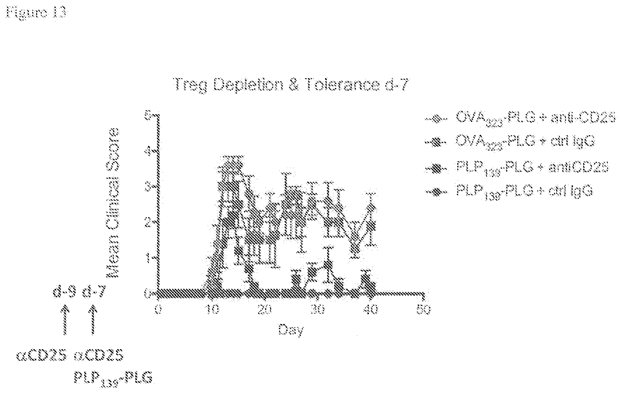

Figure 13 shows that animals treated with the PLP₁₃₉₋₁₅₁-PLG particles and an anti-CD25 antibody demonstrated, at times, a greater mean clinical score than those animals treated with PLP₁₃₉₋₁₅₁-PLG particles and a control IgG antibody.

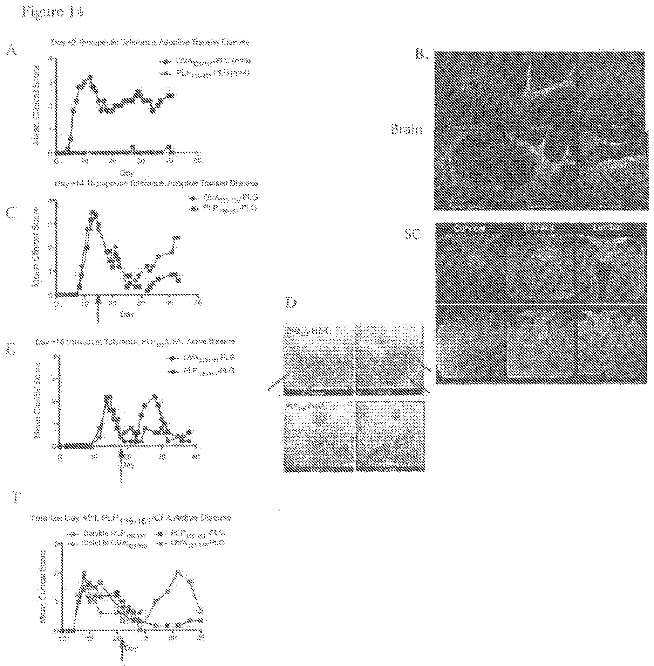

Figure 14 shows that therapeutic tolerance induced by PLP₁₃₉₋₁₅₁-PLG particles in active and adoptive EAE. Adoptive EAE was induced in six to eight-week old female SJL/J mice by adoptive transfer of 2.5x10⁶ PLP₁₃₉₋₁₅₁ -activated blasts. Mice were injected iv with PLP₁₃₉₋₁₅₁ (squares) or OVA₃₂₃₋₃₃₉ (circles) peptide coupled to 500nm PLG nanoparticles 2 days (A), 14 days (C), 18 days (E), or 21 days (F) following disease induction. Clinical disease scores were compared to those following treatment with antigen-coupled splenocytes (A). Brain and spinal cord were collected from PLP₁₃₉₋₁₅₁ - or OVA₃₂₃₋₃₃₉ -tolerized mice for histological analysis on day 42. Sections from mice from panel A were stained for PLP protein and CD45 (B). Spinal cord sections from mice from panel (C) were stained with Luxol Fast Blue (D). Areas of demyelination and cellular infiltration are indicated by arrows.

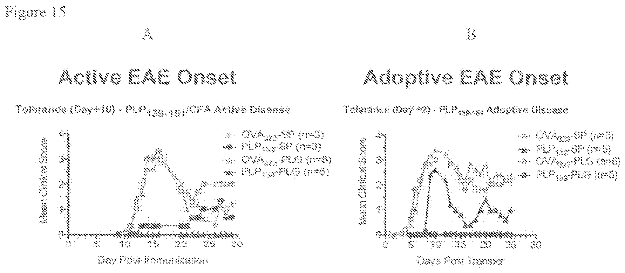

Figure 15 shows graphs depicting the mean clinical scores of mice with active EAE and adoptive EAE after treatment with either SP or PLG particles conjugated to OVA₃₂₃₋₃₃₉ or PLP₁₃₉₋₁₅₁. Mice were injected iv with PLP₁₃₉₋₁₅₁-SP, PLP₁₃₉₋₁₅₁-PLG, or OVA₃₂₃₋₃₃₉-SP, or OVA₃₂₃₋₃₃₉ - PLG peptide coupled to 500nm nanoparticles 10 days (A) or 2 days (B) following disease induction and the mean clinical score was determined. In both cases, administration of PLP₁₃₉₋₁₅₁-PLG particles induces tolerance in the mice.

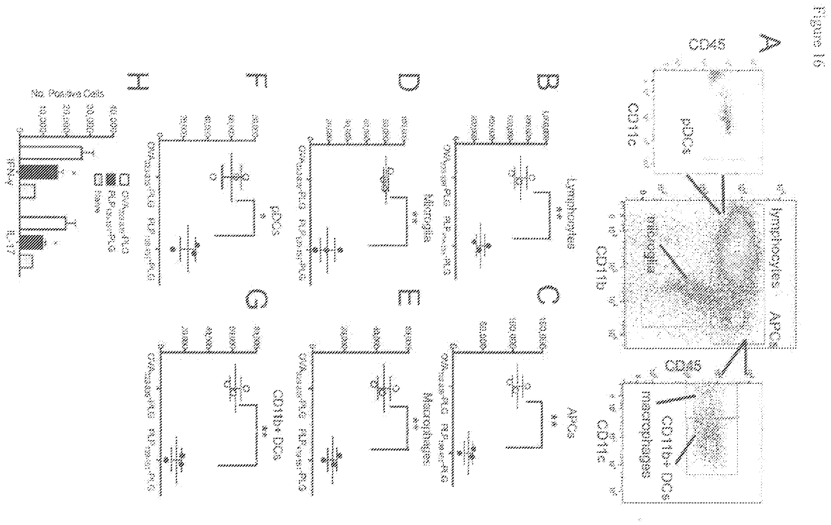

Figure 16 shows the infiltration of central nervous system immune cells is also drastically reduced in PLP-PLG tolerized mice. SJL/J mice were injected i.v. with 500nm PLG nanoparticles coupled with PLP₁₃₉₋₁₅₁ (squares) or OVA₃₂₃₋₃₃₉ (circles) 2 days following EAE induction by adoptive transfer. At the peak of disease (day 14) brains and spinal cords were removed and the number of lymphocytes (B), APCs (C), microglia (D), peripheral dendritic cells (E), myeloid dendritic cells (F) and macrophages (G) were enumerated by flow cytometry. The gating strategy for these populations is depicted in (A). CNS cell preparations were stimulated with PMA and ionomycin for 5 h prior to intracellular staining for IL-17A and IFN-γ (H).

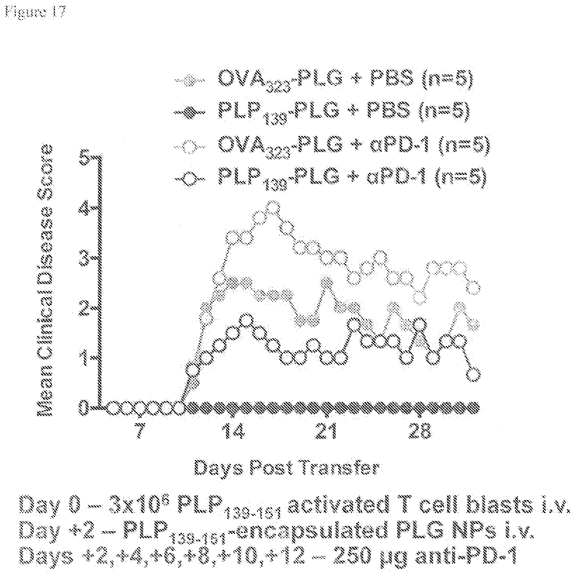

Figure 17 shows that administration of the PLP₁₃₉₋₁₅₁ peptide encapsulated in a PLG particle induces tolerance when the particle is administered with PBS. However, administration of the anti-PD-1 antibody decreases this tolerance.

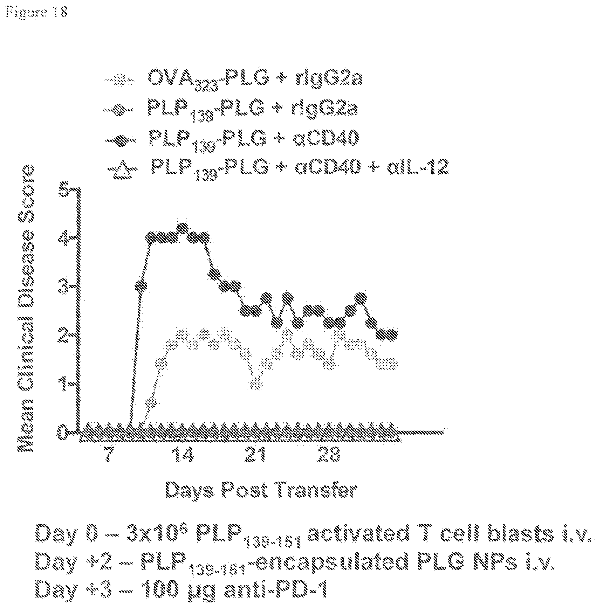

Figure 18 shows that administration of the PLP₁₃₉₋₁₅₁ peptide encapsulated in a PLG particle induces tolerance when the particle is administered with PBS. Administration of an anti-CD40 antibody decreases this tolerance, but this decrease in tolerance is reversed by the addition of an anti-IL-12 antibody.

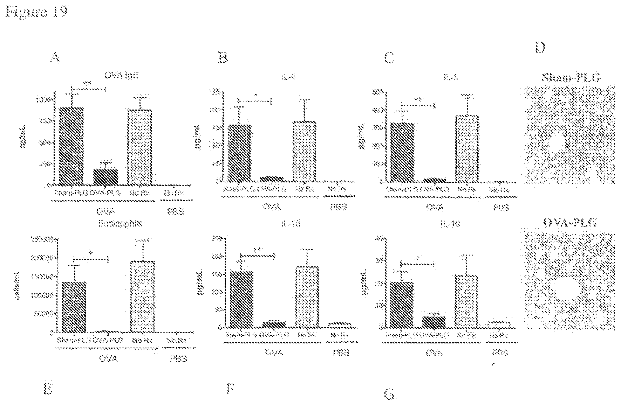

Figure 19A-G shows that the prophylactic administration of OVA-PLG decreased the secretion of IL-4, IL-5, IL-13 and IL-10, and reduced the levels of serum OVA IgE and eosinophils in the lung.

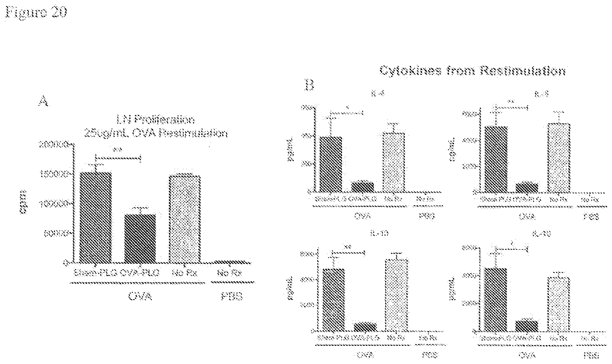

Figure 20 shows that OVA encapsulated in PLG particles prophylactically inhibits OVA-specific *in vitro* recall responses from mediastinal lymph nodes. The lymph node proliferation observed after restimulation with 25 µg OVA is decreased in those animals treated with OVA-PLG (A). Moreover treatment with OVA-PLG decreases the release of cytokines after restimulation with OVA. Levels of IL-4, IL-5, IL-13, and IL-10 are decreased in mice treated with OVA-PLG (B).

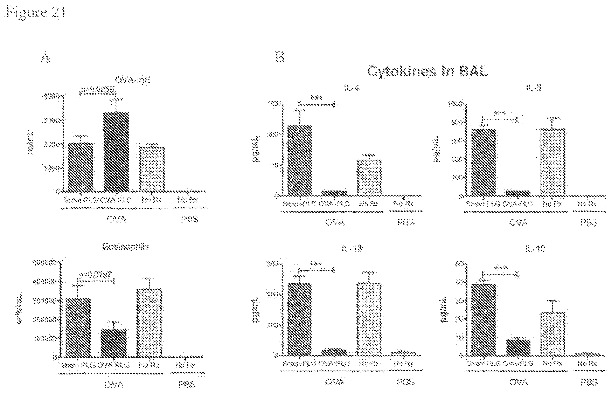

Figure 21A and B shows that the therapeutic administration of OVA-PLG decreased the secretion of IL-4, IL-5, IL-13 and IL-10, and reduced the levels of serum OVA IgE and eosinophils in the lung.

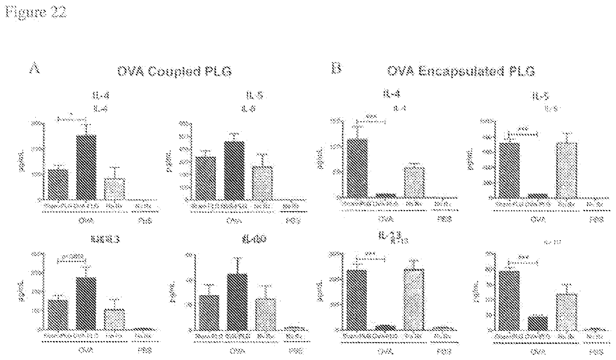

Figure 22 shows that OVA encapsulated in PLG particles therapeutically downregulates OVA-Specific Th2 Cytokines in the BAL fluid better than OVA-coupled PLG particles. Mice were treated intraperitoneally with OVA/Alum at a dose of 10µg/mouse on day 0 and day 14. The mice were intravenously administered with either OVA-coupled to PLG particles or OVA encapsulated in PLG particles on days 28, and 42. Between days 56-58, the mice were treated three times with aerosolized OVA. The graphs depict cytokine secretion when the animals were treated with either OVA coupled to PLG particles (A) or OVA encapsulated within PLG particles (B).

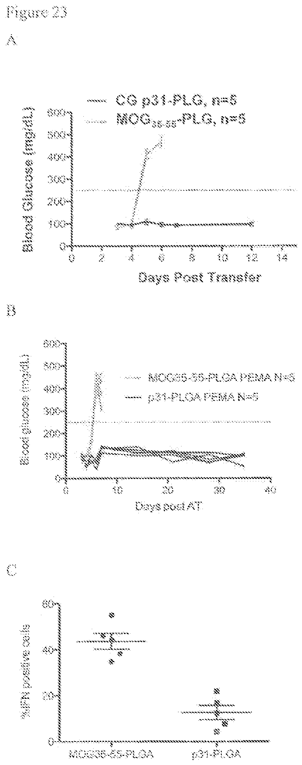

Figure 23 shows the blood glucose levels of type 1 diabetic animals after treatment with p31-PLG particles. Administration of the p31 peptide coupled PLG particles resulted in lower blood glucose levels compared to those seen after administration with the MOG₃₅₋₅₅ peptide coupled particles (A and B). The percent of IFNγ secreting cells observed in the animals was also reduced in the p31-PLG treated mice compared with the MOG₃₅₋₅₅ peptide-PLG treated mice (C).

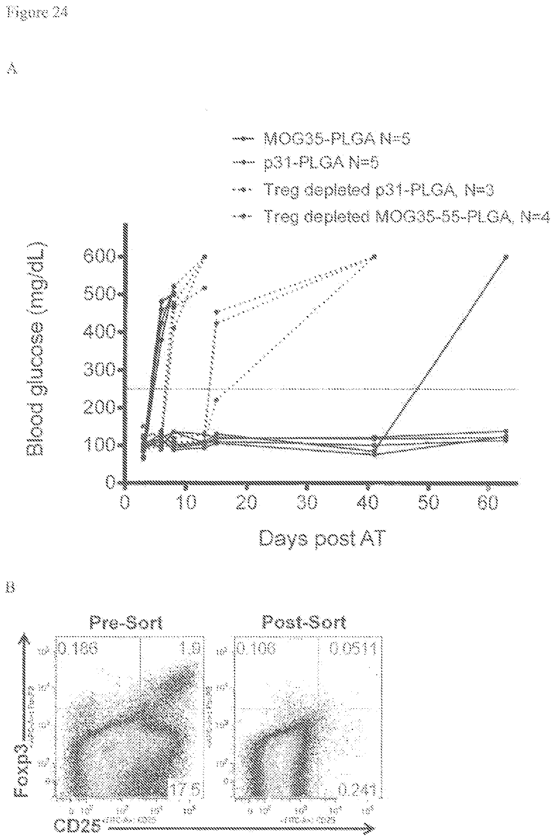

Figure 24A-B shows p31-PLG induced tolerance requires Tregs. Type 1 diabetes was induced in mice by adoptive transfer. Two hours after the activated cells were transferred to the NOD.SCID mice, the mice were tolerized with either p31-PLG or MOG₃₅₋₅₅ PLG particles. Depletion of Tregs abrogates the tolerance induced by administration of p31-PLG particles.

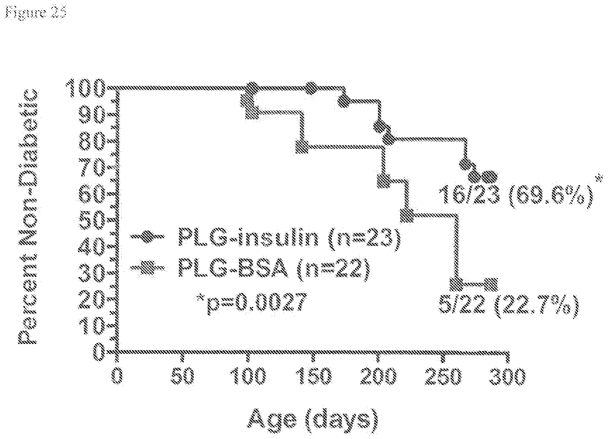

Figure 25 shows administration of insulin coupled PLG particles significantly increased the percentage of mice that did not develop diabetes over 300 days (69.6% compared to 22.7%; p=0.0027). NOD mice were treated with either BSA (N=22) or insulin (N=23) coupled PLG particles via intravenous administration at 6, 8, and 10 weeks of age. The mice were then assayed for the development of diabetes.

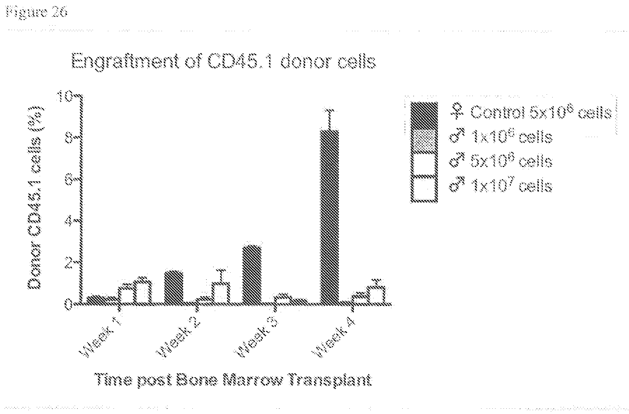

Figure 26 shows the percent of CD45.1 donor cells observed in the recipient mice. Female CD45.2 mice were tolerized with either OVA-PLG or Dby-PLG on day -7. On day - 1, the mice were irradiated with 200 rads and were then transplanted with 1x10⁶, 5x10⁶, or 1x10⁷ bone marrow cells from male CD45.1 mice on day 0. The recipient mice were then tolerized with either OVA-PLG, Dby-SP, or Dby-PLG on day 1 and the blood harvested for FACS analysis of chimerism.

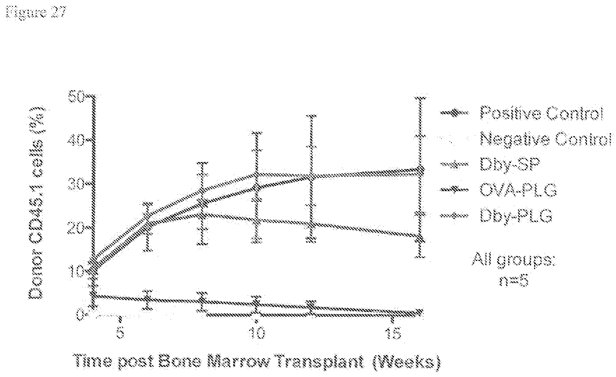

Figure 27 shows the percent of donor CD45.1 cells in the recipient mice after tolerization with either OVA-PLG, Dby-SP, or Dby-PLG on day 1. One positive control mouse did not demonstrate significant engraftment (-10%). All negative control mice did not engraft donor cells. One Dby-SP mouse did not demonstrate significant engraftment (-10%). Two OVA-PLG mice engrafted donor cells (-10%): one completely rejected by Week 16. One Dby-PLG mouse started to reject at Week 12 and was at 10% by Week 16. The Dby-PLG group ranged from 10%-56% engraftment by Week 16. The OVA-PLG mice demonstrated: 1) Spontaneous engraftment, 2) Sequence homology between OVA323 and Dby, or 3) tolerogenic properties of particles. Dby-PLG allows for more engraftment than Dby-SP and OVA-PLG.

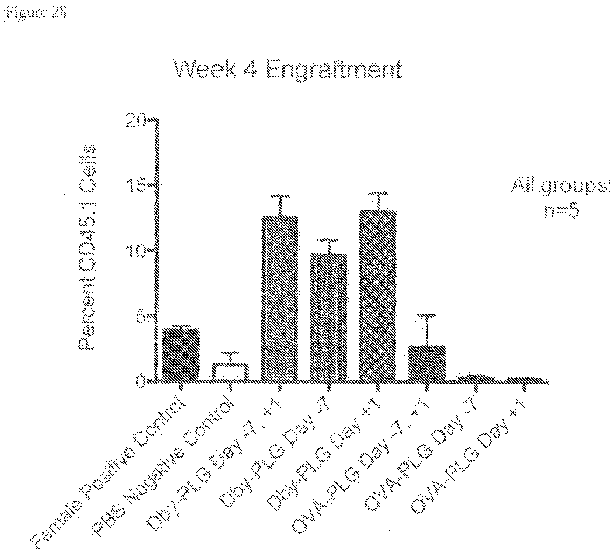

Figure 28 shows that the timing tolerance has an effect on the percent of CD45.1 cells in the recipient mouse. Positive Controls show less engraftment (-4%) than expected (-10%). One Negative control mouse had 5% engraftment Out of all 3 OVA-PLG groups, one mouse in the Day -7, Day +1 group showed engraftment (12%). Tolerance on day 1 is more clinically relevant than tolerance on day -7.

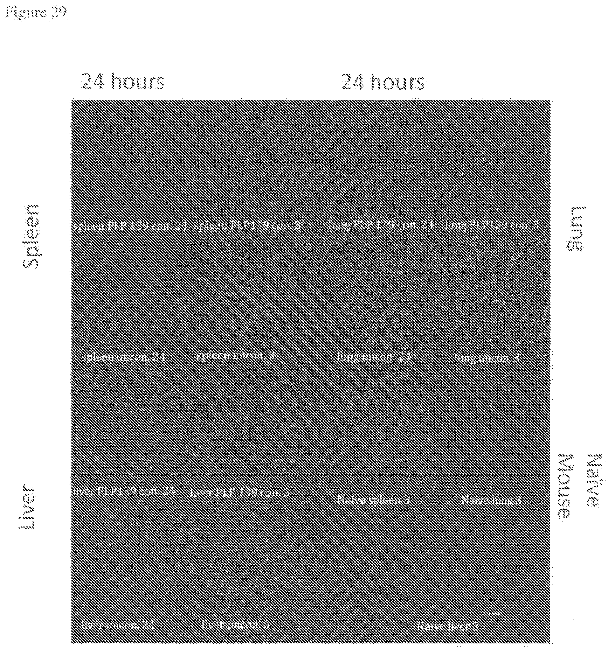

Figure 29 shows that coumarin-6 PLGA particles, that were either coupled to an antigen or were antigen-free, were detectable at 3 hours post-administration, but not at 24 hours post-administration. The particles were detectable at 3 hours post-administration, but not at 24 hours post-administration. Naive uninjected mouse (top row) as compared to i.v. fluorescent PLGA/PEMA microparticle injected mouse spleen (left column), liver (middle column) and lung (left column) sections at 3-hours post injection (middle row) and 24-hours (bottom row) post-injection, counterstained with DAPI.

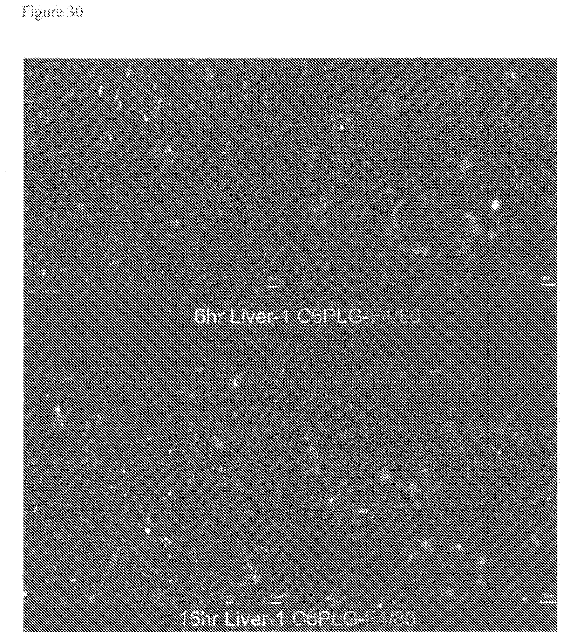

Figure 30 shows that PLGA particles co-localized after 6 and 15 hours with F4/80⁺ cells in the liver.

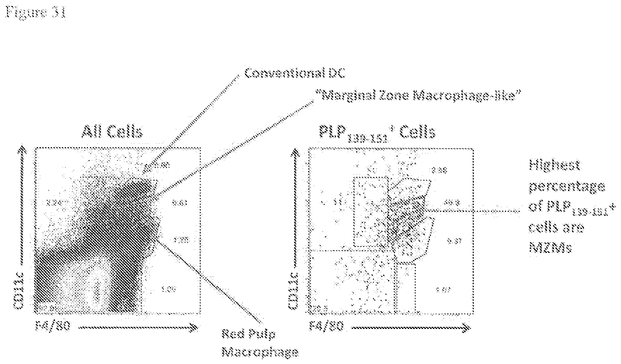

Figure 31 shows that marginal zone macrophages predominantly uptake TAMRA-labeled PLP₁₃₉₋₁₅₁-coupled particles 24 hours after intravenous infusion. The highest percentage of PLP₁₃₉₋₁₅₁+ cells are marginal zone macrophages.

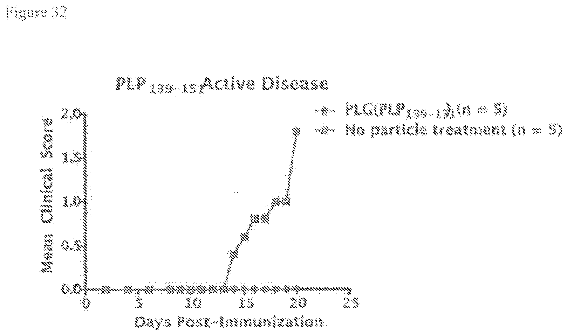

Figure 32 depicts the daily mean clinical score against the number of days PLP139-151/CFA priming. PLP139-151/CFA-induced R-EAE is inhibited in SJL/J mice by the induction of immunological tolerance using surface-functionalized poly(lactide-co-glycolide) particles containing soluble PLP139-151 within their cores.

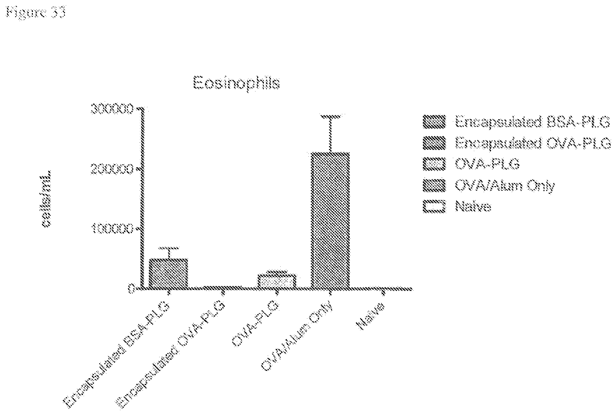

Figure 33 shows that mice treated with encapsulated OVA-PLG showed the greatest reduction in eosinophil accumulation.

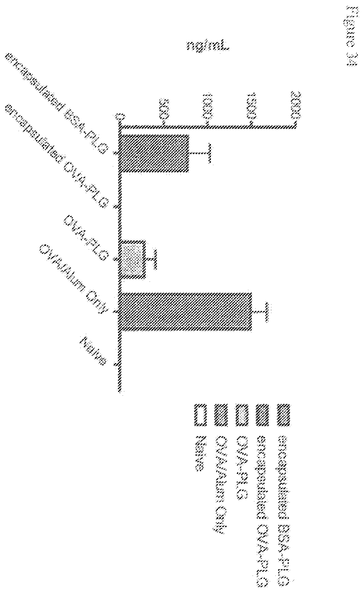

Figure 34 shows that mice treated with encapsulated OVA-PLG showed the greatest reduction in serum IgE levels compared to untreated or control treated animals.

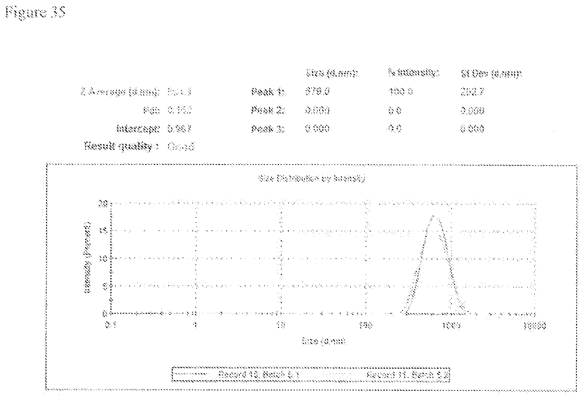

Figure 35 shows the characterization of surface-functionalized poly(lactide-*co*-glycolide) particles containing soluble PLP139-151 within their cores by dynamic light scattering analysis. Surface-functionalized poly(lactide-co-glycolide) particles were analyzed on a Malvern Zetasizer Nano ZS (Malvern Instruments, Westborough, MA) at a count rate of 1.792 x 105 counts per second in 18.2 MΩ water. The population of surface-functionalized poly(lactide-co-glycolide) particles had a Z-average diameter of 584 nm, a peak diameter of 679 nm and a polydispersity index of 0.162. These results are representative of 6 batches of syntheses, following the protocol written above.

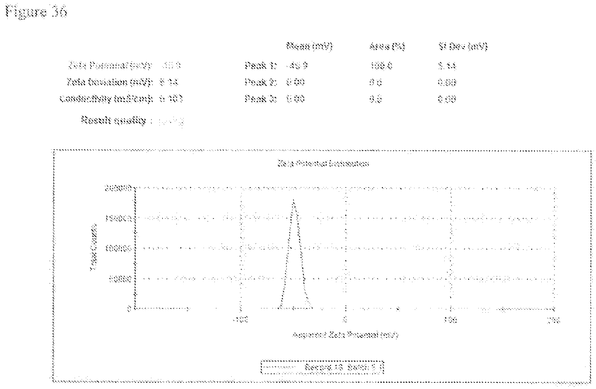

Figure 36 shows the characterization of surface-functionalized poly(lactide-*co*-glycolide) particles containing soluble PLP139-151 within their cores by ζ-potential measurement. Surface functionalized poly(lactide-co-glycolide) particles were analyzed on a Malvern Zetasizer Nano ZS (Malvern Instruments, Westborough, MA) at a count rate of 6.67 x 104 counts per second in 18.2 MΩ water. The population of surface-functionalized poly(lactide-co-glycolide) particles had a peak ζ-potential of -48.9 mV and a ζ deviation of 5.14 mV. These results are representative of 6 batches of syntheses, following the protocol written above.

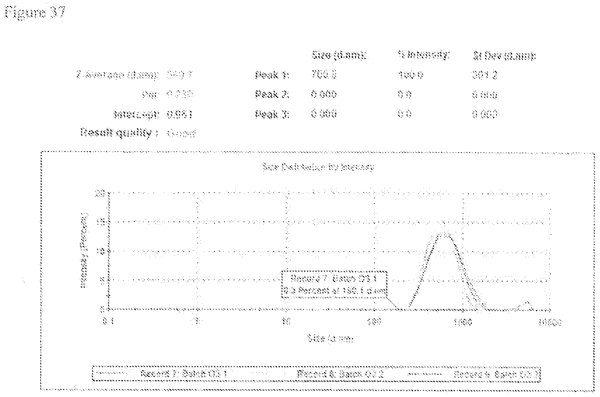

Figure 37 shows the characterization of surface-functionalized poly(lactide-*co*-glycolide) particles containing soluble ovalbumin within their cores by dynamic light scattering analysis. Surface-functionalized poly(lactide-co-glycolide) particles were analyzed on a Malvern Zetasizer Nano ZS (Malvern Instruments, Westborough, MA) at a count rate of 1.822 x 105 counts per second in 18.2 MΩ water. The population of surface-functionalized poly(lactide-*co*-glycolide) particles had a Z-average diameter of 569.7 nm, a peak diameter of 700.3 nm and a polydispersity index of 0.230. These results are representative of 3 batches of syntheses, following the protocol written above.

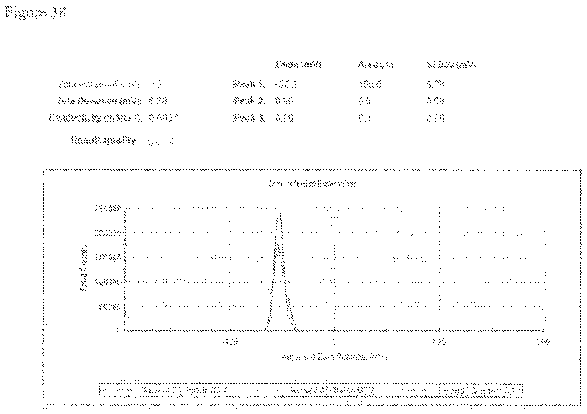

Figure 38 shows characterization of surface-functionalized poly(lactide-*co*-glycolide) particles containing soluble ovalbumin within their cores by ζ-potential measurement. Surface functionalized poly(lactide-co-glycolide) particles were analyzed on a Malvern Zetasizer Nano ZS (Malvern Instruments, Westborough, MA) at a count rate of 2.67 x 104 counts per second in 18.2 MΩ water. The population of surface-functionalized poly(lactide-*co*-glycolide) particles had a peak ζ-potential of -52.2 mV and a ζ deviation of 5.38 mV. These results are representative of 3 batches of syntheses, following the protocol written above.

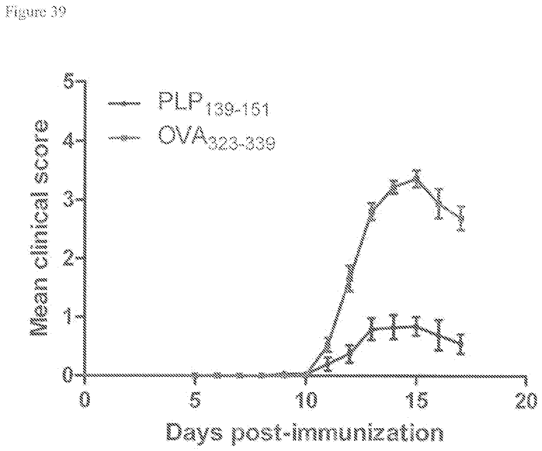

Figure 39 shows a graph demonstrating that surface-functionalized liposomes containing soluble PLP₁₃₉₋₁₅₁ peptide within their cores induce immunological tolerance in the murine model of multiple sclerosis. Animals were treated with either surface-functionalized liposomes containing soluble PLP₁₃₉₋₁₅₁ peptide within their cores (circles) or surface-functionalized liposomes containing soluble OVA₃₂₃₋₃₃₉ peptide (squares). The mean clinical scores of those animals receiving the PLP₁₃₉₋₁₅₁ peptide liposomes was lower than that of animals receiving the OVA₃₂₃₋₃₃₉ peptide liposomes.

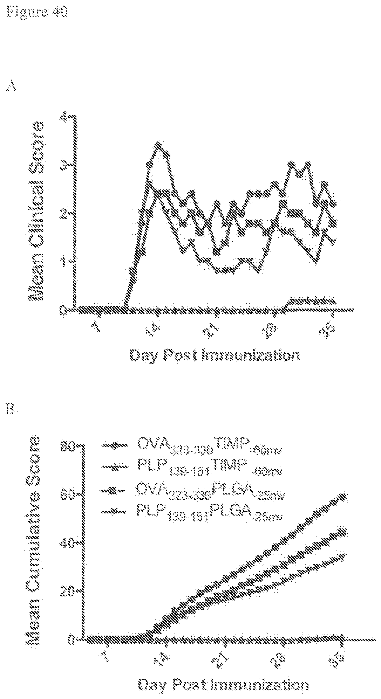

Figure 40 shows that the charge of the particle administered has an effect on the development of EAE in the mouse model. Panel (A) shows the mean clinical score, and Panel (B) shows the mean cumulative score of the EAE animals. Mice received TIMP (tolerogenic immune modifying particles) having a charge of either -60mv or -25mv conjugated to an antigen. Mice were treated with either OVA₃₂₃₋₃₃₉-TIMP₋₆₀ₘᵥ, OVA₃₂₃₋₃₃₉-PLGA₋₂₅ₘᵥ, PLP₁₃₉₋₁₅₁-TIMP₋₆₀ₘᵥ, or PLP₁₃₉₋₁₅₁-PLGA₋₂₅ₘᵥ and scored for clinical disease. The more negatively charged particles, TIMP₋₆₀ₘᵥ, induce tolerance more effectively than the PLGA-25mv particles.

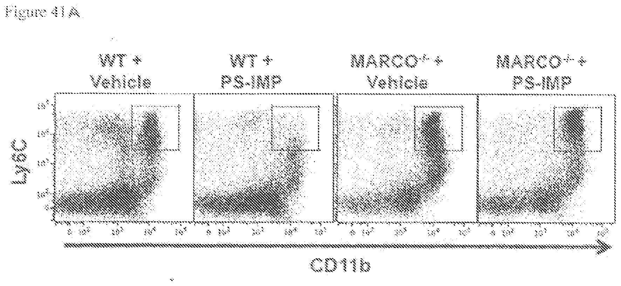

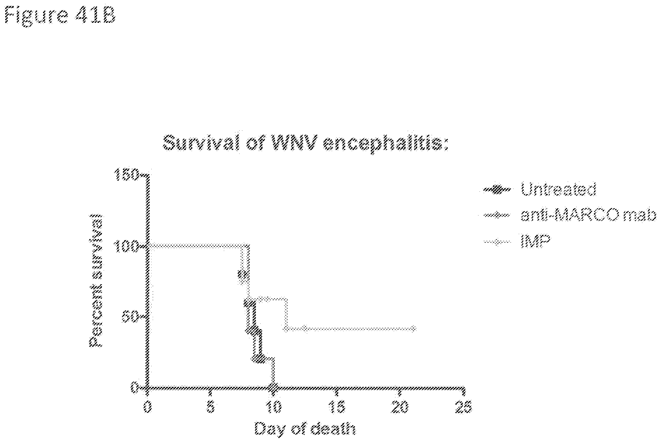

Figure 41 shows that the charge of the immune-modifying particle is important for targeting the immune modifying particle to the antigen presenting cell. Wild type or MARCO -/+ animals were treated with either PS-IMP or vehicle. The results indicate that particles with a reduced negative charge have a lower efficacy because there is less interaction with the scavenger receptor MARCO (A). AntiMARCO antibody alone is not capable of providing similar efficacy at PLGA IMP (B).

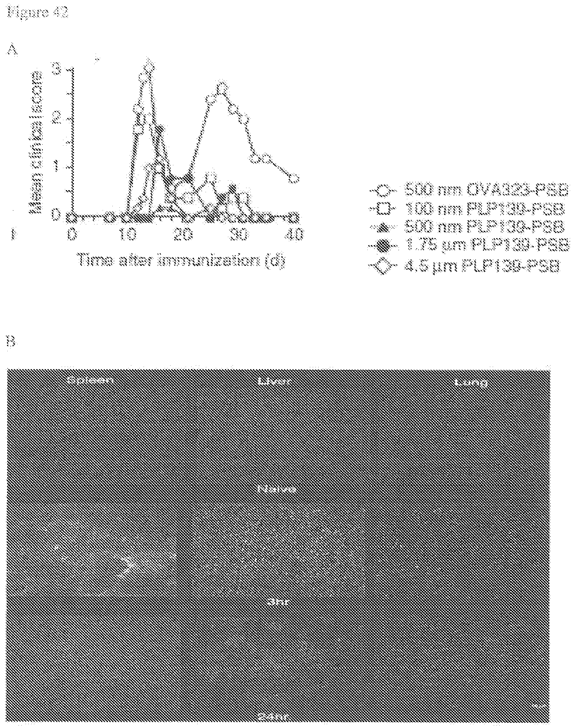

Figure 42 demonstrates the key particle parameters required for tolerance in the EAE murine model. Panel (A) shows that the most effective average particle size is 500 nm. Mice were treated with either 500nm OVA₃₂₃₋₃₃₉-PSB, 100 nm PLP₁₃₉₋₁₅₁-PSB, 500 nm PLP₁₃₉₋₁₅₁-PSB, 1.75 µm PLP₁₃₉₋₁₅₁-PSB, or 4.5 µm PLP₁₃₉₋₁₅₁-PSB and scored for clinical disease. Panel (B) shows that 24 hours after i.v. infusion fluorescently labelled particles with a 50:50 lactide:glycolide ratio have significantly cleared from the spleen, liver and lung.

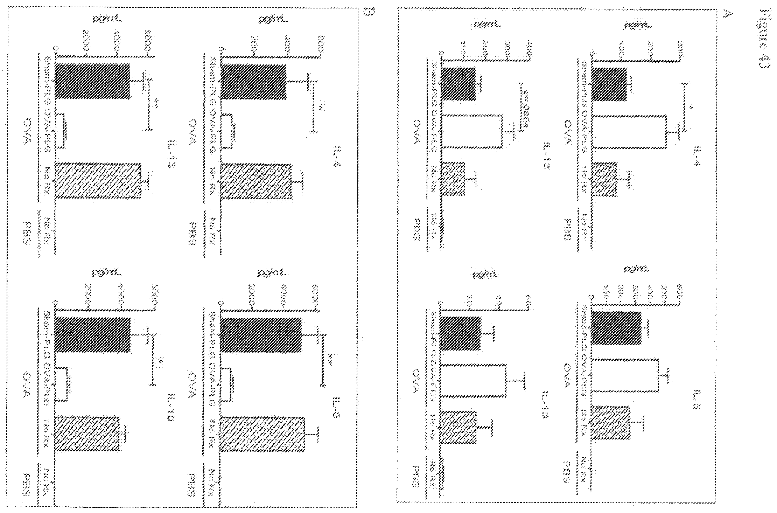

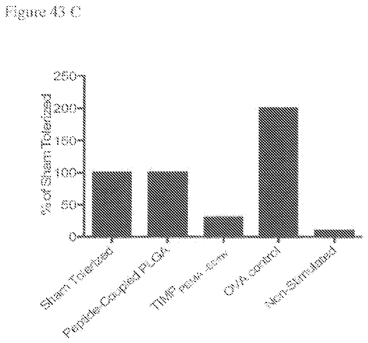

Figure 43 demonstrates that TIMPs with encapsulated antigens are superior to peptide-coupled particles. In the murine allergy model, animals were exposed to OVA as an allergen, and were then treated with either a sham-PLG, no treatment, a PLGA particle with OVA coupled to the outside of the particle (A) or a PLGA particle with OVA encapsulated within the particle (TIMP) (B) Panel (A) shows that OVA-PLG surface coupled particles fail to reduce the TH2 response. Panel (B) shows that TIMP_{PEMA-60mv} (OVA encapsulated within the particle) inhibit the TH2 response. Panel (C) shows that TIMP_{PEMA-60mv} (OVA encapsulated within the particle) inhibit recall responses.

### DETAILED DESCRIPTION OF THE INVENTION

The invention is as defined in the claims.

The present inventors have found that nanoparticles coupled to an antigen can induce tolerance to autoimmune disease and decrease the immune response. These particles can induce tolerance regardless of whether they are bound to the surface of the particle or encapsulated within. These, particles, therefore, may be useful in the treatment of any disease or condition characterized by an excessive inflammatory immune response, such as autoimmune diseases or allergies.

"Particle" as used herein refers to any non-tissue derived composition of matter, it may be a sphere or sphere-like entity, bead, or liposome. The term "particle", the term "immune modifying particle", the term "carrier particle", and the term "bead" may be used interchangeably depending on the context. Additionally, the term "particle" may be used to encompass beads and spheres.

"Negatively charged particle" as used herein refers to particles which have been modified to possess a net surface charge that is less than zero.

"Carboxylated particles" or "carboxylated beads" or "carboxylated spheres" includes any particle that has been modified to contain a carboxyl group on its surface. In some embodiments the addition of the carboxyl group enhances phagocyte/monocyte uptake of the particles from circulation, for instance through the interaction with scavenger receptors such as MARCO. Carboxylation of the particles can be achieved using any compound which adds carboxyl groups, including, but not limited to, Poly(ethylene-maleic anhydride) (PEMA).

"Antigenic moiety" as used herein refers to any moiety, for example a peptide, that is recognized by the host's immune system. Examples of antigenic moieties include, but are not limited to, autoantigens, enzymes, and/or bacterial or viral proteins, peptides, drugs or components. Without being bound by theory, while the carboxylated beads themselves may be recognized by the immune system, the carboxylated beads with nothing more attached thereto are not considered an "antigenic moiety" for the purposes of the invention.

"Naked beads" or "naked particles" or "naked spheres" as used herein refers to beads, particles or spheres that have not been carboxylated.

"Pro-inflammatory mediators" or "pro-inflammatory polypeptides" as used herein refers to polypeptides or fragments thereof which induce, maintain, or prolong inflammation in a subject. Examples of pro-inflammatory mediators include, but are not limited to, cytokines and chemokines.

As used herein, the term "Inflammatory monocyte" refers to any myeloid cell expressing any combination of CD14/CD26 and CCR2.

As used herein, the term "inhibitory neutrophil" refers to neutrophils, and/or monocyte derived suppressor cells.

As used herein, the term "Th cell" or "helper T cell" refers to CD4⁺ cells. CD4⁺ T cells assist other white blood cells with immunologic processes, including maturation of B cells into plasma cells and memory B cells, and activation of cytotoxic T cells and macrophages. T cells become activated when they are presented with peptide antigens by MHC class II molecules, which are expressed on the surface of antigen-presenting cells (APCs).

As used herein, the term "Thl cell" refers to a subset of Th cells which produce proinflammatory mediators. Th1 cells secrete cytokines to facilitate immune response and play a role in host defense against pathogens in part by mediating the recruitment of neutrophils and macrophages to infected tissues. Th1 cells secrete cytokines including IFN-gamma, IL2, IL-10, and TNF alpha/beta to coordinate defense against intracellular pathogens such as viruses and some bacteria.

As used herein, the term "Th2 cell" refers to a subset of Th cells that mediate the activation and maintenance of the antibody-mediated immune response against extracellular parasites, bacteria, allergens, and toxins. Th2 cells mediate these functions by producing various cytokines such as IL-4, IL-5, IL-6, IL-9, IL-13, and IL-17E (IL-25) that are responsible for antibody production, eosinophil activation, and inhibition of several macrophage functions, thus providing phagocyte-independent protective responses.

As used herein, the term "Thl7 cell" refers to a subset of Th cells. Thl7 cells secrete cytokines to facilitate immune response and play a role in host defense against pathogens by mediating the recruitment of neutrophils and macrophages to infected tissues. TH17 cells secrete cytokines such as IL17, IL21, IL22, IL24, IL26 and TNF alpha to coordinate defense against extracellular pathogens including fungi and bacteria.

"Coupled" as used herein refers to an antigen fixed to the outside of a particle or encapsulated within a particle. Thus, an antigen coupled to a particle includes both surface coupling as well as encapsulation within the particle.

The term "IMP" as used herein refers to immune-modifying particles which are not coupled to an antigen. The term "TIMP" as used herein refers to tolerizing immune modifying particles which are coupled to an antigen. In some instances of the disclosure, the antigen is attached to the surface of the TIMP. In other embodiments, the antigen is encapsulated within the TIMP.

The particle may have any particle shape or conformation. However, in some embodiments it is preferred to use particles that are less likely to clump in vivo. Examples of particles within these embodiments are those that have a spherical shape.

Another instance of the disclosurerelates to a composition which comprises an immune modified particle having a negative zeta potential and free from antigenic moieties. In a further embodiment, the invention provides compositions comprising an immune modified particle with a negative zeta potential coupled to an antigen. In a further instance of the disclosure, the antigen is coupled to the outside of the particle. In the invention, the antigen is encapsulated within the particle.

Yet another instance of the disclosurerelates to a process for the preparation an immune modified particle with a negative zeta potential and free from antigenic moieties. The process involves contacting an immune modified particle precursor with a buffer solution under conditions effective to form the immune modified particle with a negative zeta potential. In some instances of the disclosure, the immune modified particle precursor is formed via co-polymerization. The particle microstructure may depend on the method of co-polymerization.

In some instances of the disclosure, an antigenic peptide molecule is coupled to the carrier particle (e.g. immune modified particle) by a conjugate molecule and/or linker group. In some instances of the disclosure, coupling of the antigenic peptide and/or apoptotic signalling molecule to the carrier (e.g., PLG particle) comprises one or more covalent and/or non-covalent interactions. In some instances of the disclosure, the antigenic peptide is attached to the surface of the carrier particle with a negative zeta potential. In the invention, the antigenic peptide is encapsulated within the carrier particle with a negative zeta potential.

In one instance of the disclosure, the buffer solution contacting the immune modified particle may have a basic pH. Suitable basic pH for the basic solution include 7.1, 7.5, 8.0, 8.5, 9.5, 10.0 10.5, 11.0, 11.5, 12.0, 12.5, 13.0, and 13.5. The buffer solution may also be made of any suitable base and its conjugate. In some instances of the disclosure, the buffer solution may include, without limitation, sodium bicarbonate, potassium bicarbonate, lithium bicarbonate, potassium dihydrogen phosphate, sodium dihydrogen phosphate, or lithium dihydrogen phosphate and conjugates thereof.

In one embodiment of the invention, the immune modified particles contain co-polymers. These co-polymers may have varying molar ratio. Suitable co-polymer ratio of present immune modified particles may be 25:75, 30:70, 35:65, 40:60, 45:55, 50:50, 55:45, 60:40, 65:35, 70:30, 75:25, 80:20, 81:19, 82:18, 83:17, 84:16, 85:15, 86:14, 87:13, 88:12, 89:11, 90:10, 91;:9, 92:8, 93:7, 94:6, 95:5, 96:4, 97:3, 98:2, 99:1, or 100:0. In another embodiment, the co-polymer may be periodical, statistical, linear, branched (including star, brush, or comb co-polymers) co-polymers. In some embodiments, the co-polymers ratio may be, but not limited to, polystyrene:poly(vinyl carboxylate)/80:20, polystyrene: poly(vinyl carboxylate)/90:10, poly(vinyl carboxylate):polystyrene/80:20, poly(vinyl carboxylate):polystyrene/90:10, polylactic acid: polyglycolic acid/50:50, polylactic acid: polyglycolic acid/80:20, or polylactic acid: polyglycolic acid/90:10.

In one instance of the disclosure, the particles of the invention are made by adding a composition comprising the polymer (e.g. PLGA) to a solution of Poly(ethylene-maleic anhydride) (PEMA). The concentration of PEMA in the solution can be between about 0.1% and about 10%. In one instance of the disclosure, the concentration of PEMA in the solution is between about 0.2% and about 5%. In another instance of the disclosure, the concentration of PEMA in the solution is between about 0.1% and 4%. In another instance of the disclosure, the concentration of PEMA in the solution is between about 0.1% and 2%. In another instance of the disclosure, the concentration of PEMA in the solution is between about 0.5% and 1%. In one instance of the disclosure, the percentage of PEMA in solution is 0.1%, 0.2%, 0.3%, 0.4%, 0.5%, 0.6% 0.7%, 0.8%, 0.9%, 1%, 1.5%, 2%, 2.5%, 3%, 3.5%, 4%, 4.5%, 5%, 6%, 6.5%, 7%, 7.5%, 8%, 8.5%, 9%, 9.5% or 10%. In one instance of the disclosure, the percentage of PEMA in the solution is about 0.5%. In another instance of the disclosure, the percentage of PEMA in the solution is about 1.0%. Other compounds that may be used include, but are not limited to, Poly(ethylene-*alt*-maleic anhydride), Poly(isobutylene-*co*-maleic acid), Poly(methyl vinyl ether-*alt*-maleic acid), Poly(methyl vinyl ether-*alt*-maleic acid monoethyl ester), Poly(methyl vinyl ether-*alt*-maleic anhydride), Poly(methyl vinyl ether-*alt*-maleic anhydride) cross-linked with 1,9-decadiene powder, and/or Poly(styrene-*alt*-maleic acid) sodium salt.

In one embodiment, the particle is a liposome. In a further embodiment, the particle is a liposome composed of the following lipids at the following molar ratios - 30:30:40 phosphatidylcholine:phosphatidylglycerol:cholesterol. In yet a further embodiment, the particle is encapsulated within a liposome.

It is not necessary that each particle be uniform in size, although the particles must generally be of a size sufficient to be sequestered in the spleen or liver and trigger phagocytosis or uptake through receptor or non-receptor mediated mechanism by an antigen presenting cell, including endothelial cell or other MPS cell. Preferably, the particles are microscopic or nanoscopic in size, in order to enhance solubility, avoid possible complications caused by aggregation *in vivo* and to facilitate pinocytosis. Particle size can be a factor for uptake from the interstitial space into areas of lymphocyte maturation. A particle having a diameter of from about 0.1µm to about 10 µm is capable of triggering phagocytosis. Thus in one embodiment, the particle has a diameter within these limits. In another instance of the disclosure, the particle has an average diameter of about 0.3 µm to about 5 µm. In still another instances of the disclosure, the particle has an average diameter of about 0.5 µm to about 3 µm. In the invention, the particle has an average diameter of about 0.2µm to about 2µm. In a further instance of the disclosure the particle has an average size of about 0.1 µm, or about 0.2 µm or about 0.3 µm or about 0.4 µm or about 0.5 µm or about 1.0 µm or about 1.5 µm or about 2.0 µm or about 2.5 µm or about 3.0 µm or about 3.5 µm or about 4.0 µm or about 4.5 µm or about 5.0 µm. In a particular embodiment the particle has an average size of about 0.5 µm. In some embodiments, the overall weights of the particles are less than about 10,000 kDa, less than about 5,000 kDa, or less than about 1,000 kDa, 500 kDa, 400 kDa, 300 kDa, 200 kDa, 100 kDa, 50 kDa, 20 kDa, 10 kDa. The particles in a composition need not be of uniform diameter. By way of example, a pharmaceutical formulation may contain a plurality of particles, some of which are about 0.5 µm, while others are about 1.0 µm. Any mixture of particle sizes within these given ranges will be useful.

The particles of the current disclosure can possess a particular zeta potential. In certain instances of the disclosure, the zeta potential is negative. In one instance of the disclosure, the zeta potential is less than about -100 mV. In one instance of the disclosure, the zeta potential is less than about -50 mV. In certain instances of the disclosure, the particles possess a zeta potential between -100 mV and 0 mV. In a further instance of the disclosure, the particles possess a zeta potential between -75 mV and 0 mV. In a further instance of the disclosure, the particles possess a zeta potential between -60 mV and 0 mV. In a further instance of the disclosure, the particles possess a zeta potential between -50 mV and 0 mV. In still a further instance of the disclosure, the particles possess a zeta potential between -40 mV and 0 mV. In a further instance of the disclosure, the particles possess a zeta potential between -30 mV and 0 mV. In a further instance of the disclosure, the particles possess a zeta potential between -20 mV and +0 mV. In a further instance of the disclosure, the particles possess a zeta potential between -10 mV and -0 mV. In an embodiment of the invention, the particles possess a zeta potential between -100mV and -50mV. In another particular embodiment, the particles possess a zeta potential between -75 mV and -50mV. In a particular embodiment, the particles possess a zeta potential between -50 mV and -40mV.

In some instances of the disclosure, the charge of a carrier (e.g., positive, negative, neutral) is selected to impart application-specific benefits (e.g., physiological compatibility, beneficial surface-peptide interactions, etc.). In some instances of the disclosure, a carrier has a net neutral or negative charge (e.g., to reduce non-specific binding to cell surfaces which, in general, bear a net negative charge). In certain instances of the disclosure carriers are capable of being conjugated, either directly or indirectly, to an antigen to which tolerance is desired (also referred to herein as an antigen-specific peptide, antigenic peptide, autoantigen, inducing antigen or tolerizing antigen). In some instances, a carrier has multiple binding sites (e.g., 2, 3, 4, 5, 6, 7, 8, 9, 10... 20... 50... 100, or more) in order to have multiple copies of an antigen-specific peptide, or multiple different peptides, exposed on the surface (e.g., to increase the likelihood of a tolerance response). In some embodiments, a carrier displays a single type of antigenic peptide. In some embodiments, a carrier displays multiple different antigenic peptides on the surface. In some instances of the disclosure, a carrier surface displays functional groups for the covalent attachment of selected moieties (e.g., antigenic peptides). In some instances of the disclosure, carrier surface functional groups provide sites for non-covalent interaction with selected moieties (e.g., antigenic peptides). In some instances of the disclosure, a carrier has a surface to which conjugating moieties may be adsorbed without chemical bond formation.

The size and charge of the particles are critical for tolerance induction. While the particles will differ in size and charge based on the antigen encapsulated within them (See Table 1 for examples of specific particles), in general, particles of the current disclosure are effective at inducing tolerance when they are between about 100 nanometers and about 1500 nanometers and have a charge of 0 to about -70 mV. Particles of the disclosure are most effective at inducing tolerance when they are 400-800 nanometers and have a charge of between about -25mV and - 70mV. Furthermore, as shown in Table 1, due in part to the concentration of the particles and presence of sucrose and D-mannitol in the lyophilisation process, the average particle size and charge of the particles can be slightly altered in the lyophilisation process, therefore, both post-synthesis averages and post-lyophilization averages are given below. As used herein, the term "post-synthesis size" and "post synthesis charge" refer to the size and charge of the particle prior to lyophilization. The term "post lyophilization size" and "post lyophilization charge" refer to the size and charge of the particle after lyophilization.

**TABLE 1 REPRESENTATIVE PARTICLE ANALYSIS**

| Antigen | Particle Material | Copolymer ratio | Surfactant used | Average Size post synthesis (post lyophilisation) nm | Average Charge post synthesis (post lyophilisation) mV |
|---|---|---|---|---|---|
| OVA | PLGA (carboxylated) | 50:50 | PEMA | 566.5 | 51.2 |
| | | | | (538.5) | (66.0) |
| Insulin | PLGA (carboxylated) | 50:50 | PEMA | 500.9 | 48.4 |
| | | | | (385.2) | (53.7) |
| PLP₁₃₉₋₁₅₁ | PLGA (carboxylated) | 50:50 | PEMA | 429.9 | 53.7 |
| | | | | (359.6) | (69.4) |
| Gliaden | PLGA (carboxylated) | 50:50 | PEMA | 606.1 | 48.8 |
| | | | | (1104.0) | (68.1) |
| Proinsulin | PLGA (carboxylated) | 50:50 | PEMA | 566.6 (407.2) | 44.1 (49.7) |
| | | | | | |
| Lysozyme | PLGA (carboxylated) | 50:50 | PEMA | 435.3 | 48.1 |
| | | | | (393.3) | (65.1) |
| IGRP | PLGA (carboxylated) | 50:50 | PEMA | 612.5 (399.3) | 41.0 (57.6) |
| | | | | | |
| Empty particle (No As) | PLGA (carboxylated) | 50:50 | PEMA | 383.9 | 52.6 |
| | | | | (343.3) | (61.3) |

In some instances of the disclosure, the particle is non-metallic. In these instances of the disclosure the particle may be formed from a polymer. In the invention, the particle is biodegradable in an individual. In this embodiment, the particles can be provided in an individual across multiple doses without there being an accumulation of particles in the individual. Examples of suitable particles include polystyrene particles, PLGA particles, PLURIONICS stabilized polypropylene sulfide particles, and diamond particles.

Preferably the particle surface is composed of a material that minimizes non-specific or unwanted biological interactions. Interactions between the particle surface and the interstitium may be a factor that plays a role in lymphatic uptake. The particle surface may be coated with a material to prevent or decrease non-specific interactions. Steric stabilization by coating particles with hydrophilic layers such as poly(ethylene glycol) (PEG) and its copolymers such as PLURONICS^{®} (including copolymers of poly(ethylene glycol)-bl-poly(propylene glycol)-bl-poly(ethylene glycol)) may reduce the non-specific interactions with proteins of the interstitium as demonstrated by improved lymphatic uptake following subcutaneous injections. All of these facts point to the significance of the physical properties of the particles in terms of lymphatic uptake. Biodegradable polymers may be used to make all or some of the polymers and/or particles and/or layers. Biodegradable polymers may undergo degradation, for example, by a result of functional groups reacting with the water in the solution. The term "degradation" as used herein refers to becoming soluble, either by reduction of molecular weight or by conversion of hydrophobic groups to hydrophilic groups. Polymers with ester groups are generally subject to spontaneous hydrolysis, e.g., polylactides and polyglycolides.

Particles of the present invention may also contain additional components. For example, carriers may have imaging agents incorporated or conjugated to the carrier. An example of a carrier nanosphere having an imaging agent that is currently commercially available is the Kodak X-sight nanospheres. Inorganic quantum-confined luminescent nanocrystals, known as quantum dots (QDs), have emerged as ideal donors in FRET applications: their high quantum yield and tunable size-dependent Stokes Shifts permit different sizes to emit from blue to infrared when excited at a single ultraviolet wavelength. (Bruchez, et al., Science, 1998, 281, 2013; Niemeyer, C. M Angew. Chem. Int. Ed. 2003, 42, 5796; Waggoner, A. Methods Enzymol. 1995, 246, 362; Brus, L. E. J. Chem. Phys. 1993, 79, 5566). Quantum dots, such as hybrid organic/inorganic quantum dots based on a class of polymers known as dendrimers, may used in biological labeling, imaging, and optical biosensing systems. (Lemon, et al., J. Am. Chem. Soc. 2000, 122, 12886). Unlike the traditional synthesis of inorganic quantum dots, the synthesis of these hybrid quantum dot nanoparticles does not require high temperatures or highly toxic, unstable reagents. (Etienne, et al., Appl. Phys. Lett. 87, 181913, 2005).

Particles can be formed from a wide range of materials. The particle is preferably composed of a material suitable for biological use. For example, particles may be composed of glass, silica, polyesters of hydroxy carboxylic acids, polyanhydrides of dicarboxylic acids, or copolymers of hydroxy carboxylic acids and dicarboxylic acids. More generally, the carrier particles may be composed of polyesters of straight chain or branched, substituted or unsubstituted, saturated or unsaturated, linear or cross-linked, alkanyl, haloalkyl, thioalkyl, aminoalkyl, aryl, aralkyl, alkenyl, aralkenyl, heteroaryl, or alkoxy hydroxy acids, or polyanhydrides of straight chain or branched, substituted or unsubstituted, saturated or unsaturated, linear or cross-linked, alkanyl, haloalkyl, thioalkyl, aminoalkyl, aryl, aralkyl, alkenyl, aralkenyl, heteroaryl, or alkoxy dicarboxylic acids. Additionally, carrier particles can be quantum dots, or composed of quantum dots, such as quantum dot polystyrene particles (Joumaa et al. (2006) Langmuir 22: 1810-6). Carrier particles including mixtures of ester and anhydride bonds (e.g., copolymers of glycolic and sebacic acid) may also be employed. For example, carrier particles may comprise materials including polyglycolic acid polymers (PGA), polylactic acid polymers (PLA), polysebacic acid polymers (PSA), poly(lactic-co-glycolic) acid copolymers (PLGA or PLG; the terms are interchangeable), [rho]oly(lactic-co-sebacic) acid copolymers (PLSA), poly(glycolic-co-sebacic) acid copolymers (PGSA), polypropylene sulfide polymers, poly(caprolactone), chitosan, etc. Other biocompatible, biodegradable polymers useful in the present invention include polymers or copolymers of caprolactones, carbonates, amides, amino acids, orthoesters, acetals, cyanoacrylates and degradable urethanes, as well as copolymers of these with straight chain or branched, substituted or unsubstituted, alkanyl, haloalkyl, thioalkyl, aminoalkyl, alkenyl, or aromatic hydroxy- or di-carboxylic acids. In addition, the biologically important amino acids with reactive side chain groups, such as lysine, arginine, aspartic acid, glutamic acid, serine, threonine, tyrosine and cysteine, or their enantiomers, may be included in copolymers with any of the aforementioned materials to provide reactive groups for conjugating to antigen peptides and proteins or conjugating moieties. Biodegradable materials suitable for the present invention include diamond, PLA, PGA, polypropylene sulfide, and PLGA polymers. Biocompatible but non-biodegradable materials may also be used in the carrier particles of the disclosure. For example, non-biodegradable polymers of acrylates, ethylene-vinyl acetates, acyl substituted cellulose acetates, non-degradable urethanes, styrenes, vinyl chlorides, vinyl fluorides, vinyl imidazoles, chlorosulphonated olefins, ethylene oxide, vinyl alcohols, TEFLON^{®} (DuPont, Wilmington, Del.), and nylons may be employed.

The particles of the instant invention can be manufactured by any means commonly known in the art. Exemplary methods of manufacturing particles include, but are not limited to, microemulsion polymerization, interfacial polymerization, precipitation polymerization, emulsion evaporation, emulsion diffusion, solvent displacement, and salting out (Astete and Sabliov, J. Biomater. Sci. Polymer Edn., 17:247-289(2006)). Manipulation of the manufacturing process for PLGA particles can control particle properties (e.g. size, size distribution, zeta potential, morphology, hydrophobicity/hydrophilicity, polypeptide entrapment, etc). The size of the particle is influenced by a number of factors including, but not limited to, the concentration of PLGA, the solvent used in the manufacture of the particle, the nature of the organic phase, the surfactants used in manufacturing, the viscosity of the continuous and discontinuous phase, the nature of the solvent used, the temperature of the water used, sonication, evaporation rate, additives, shear stress, sterilization, and the nature of any encapsulated antigen or polypeptide.

Particle size is affected by the polymer concentration; higher particles are formed from higher polymer concentrations. For example, an increase in PLGA concentration from 1% to 4% (w/v) can increase mean particle size from about 205 nm to about 290 nm when the solvent propylene carbonate is used. Alternatively, in ethyl acetate and 5% Pluronic F-127, an increase in PLGA concentration from 1% to 5% (w/v) increases the mean particle size from 120 nm to 230 nm.

The viscosity of the continuous and discontinuous phase is also an important parameter that affects the diffusion process, a key step in forming smaller particles. The size of the particles increases with an increase in viscosity of the dispersed phase, whereas the size of the particles decreases with a more viscous continuous phase. In general, the lower the phase ratio of organic to aqueous solvent, the smaller the particle size.

Homogenizer speed and agitation also affect particle size; in general, higher speeds and agitation cause a decrease in particle size, although there is a point where further increases in speed and agitation no longer decrease particle size. There is a favorable impact in the size reduction when the emulsion is homogenized with a high pressure homogenizer compared with just high stirring. For example, at a phase ration of 20% in 5% PVA, the mean particle size with stirring is 288 nm and the mean particle size with homogenization (high pressure of 300 bars) is 231 nm.

An important size reduction of the particles can be achieved by varying the temperature of the water added to improve the diffusion of the solvent. The mean particle size decreases with an increase in water temperature.

The nature of the polypeptide encapsulated in the particle also affects particle size. In general, encapsulation of hydrophobic polypeptides leads to the formation of smaller particles compared with the encapsulation of more hydrophilic polypeptides. In the double emulsion process, the entrapment of more hydrophilic polypeptides is improved by using high molecular mass PLGA and a high molecular mass of the first surfactant which causes a higher inner phase viscosity. The interaction between the solvent, polymer, and polypeptide affects the efficiency of incorporating the polypeptide into the particle.

The PLGA molecular mass impacts the final mean particle size. In general, the higher the molecular mass, the higher the mean particle size. For example, as the composition and molecular mass of PLGA varies (e.g. 12 to 48 kDa for 50 : 50 PLGA; 12 to 98 kDa for 75 : 25 PLGA) the mean particle size varies (about 102 nm -154 nm; about 132 nm to 152 nm respectively). Even when particles are the same molecular mass, their composition can affect average particle size; for example, particles with a 50 : 50 ratio generally form particles smaller than those with a 75 : 25 ratio. The end groups on the polymer also affects particle size. For example, particles prepared with ester end-groups form particles with an average size of 740nm (PI=0.394) compared with the mean size for the acid PLGA end-group is 240 nm (PI=0.225).

The solvent used can also affect particle size; solvents that reduce the surface tension of the solution also reduce particle size.

The organic solvent is removed by evaporation in a vacuum to avoid polymer and polypeptide damage and to promote final particle size reduction. Evaporation of the organic solvent under vacuum is more efficient in forming smaller particles. For example, evaporation in vacuum produces a mean particle size around 30% smaller than the mean particle size produced under a normal rate of evaporation.

The amplitude of the sonication wavelength also affects the particle characteristics. The amplitude of the wavelength should be over 20% with 600 to 800 s of sonication to form sable miniemulsions with no more droplet size changes. However, the main draw-back of sonication is the lack of monodispersity of the emulsion formed.

Organic phases that may be used in the production of the particles of the invention include, but are not limited to, ethyl acetate, methyl ethyl ketone, propylene carbonate, and benzyl alcohol. The continuous phases that may be used, include but are not limited to the surfactant poloxamer 188.

A variety of surfactants can be used in the manufacturing of the particles of the invention. The surfactant can be anionic, cationic, or nonionic. Surfactants in the poloxamer and poloaxamines family are commonly used in particle synthesis. Surfactants that may be used, include, but are not limited to PEG, Tween-80, gelatin, dextran, pluronic L-63, PVA, methylcellulose, lecithin and DMAB. Additionally, biodegradable and biocompatible surfactants including, but not limited to, vitamin E TPGS (D-α-tocopheryl polyethylene glycol 1000 succinate). In certain instances of the disclosure, two surfactants are needed (e.g. in the double emulsion evaporation method). These two surfactants can include a hydrophobic surfactant for the first emulsion, and a hydrophobic surfactant for the second emulsion.

Solvents that may be used in the production of the particles of the invention include, but are not limited to, acetone, Tetrahydrofuran (THF), chloroform, and members of the chlorinate family, methyl chloride. The choice of organic solvents require two selection criteria: the polymer must be soluble in this solvent, and the solvent must be completely immiscible with the aqueous phase.

Salts that may be used in the production of the particles of the invention include, but are not limited to magnesium chloride hexahydrate, magnesium acetate tetrahydrate.

Common salting-out agents include, but are not limited to, electrolytes (e.g. sodium chloride, magnesium acetate, magnesium chloride), or non-electrolytes (e.g. sucrose).

The stability and size of the particles of the invention may be improved by the addition of compounds including, but not limited to, fatty acids or short chains of carbons. The addition of the longer carbon chain of lauric acid is associated with the improvement of particle characteristics. Furthermore, the addition of hydrophobic additives can improve the particle size, incorporation of the polypeptide into the particle, and release profile. Preparations of particles can be stabilized by lyophilization. The addition of a cryoprotectant such as trehalose can decrease aggregation of the particles upon lyophilization.

Suitable beads which are currently available commercially include polystyrene beads such as FluoSpheres (Molecular Probes, Eugene, Oreg.).

In some instances, the present disclosure provides systems comprising: (a) a delivery scaffold configured for the delivery of chemical and/or biological agents to a subject; and (b) antigen-coupled poly(lactide-co-glycolide) particles for induction of antigen-specific tolerance. In some instances of the disclosure, at least a portion of said delivery scaffold is microporous. In some instances of the disclosure, the antigen-coupled poly(lactide-co-glycolide) particles are encapsulated within said scaffold. In some instances of the disclosure, the chemical and/or biological agents are selected from the group consisting of: protein, peptide, small molecules, nucleic acids, cells, and particles. In some instances of the disclosure, chemical and/or biological agents comprise cell, and said cells comprise pancreatic islet cells.

Physical properties are also related to a nanoparticle's usefulness after uptake and retention in areas having immature lymphocytes. These include mechanical properties such as rigidity or rubberiness. Some embodiments are based on a rubbery core, e.g., a poly(propylene sulfide) (PPS) core with an overlayer, e.g., a hydrophilic overlayer, as in PEG, as in the PPS-PEG system recently developed and characterized for systemic (but not targeted or immune) delivery. The rubbery core is in contrast to a substantially rigid core as in a polystyrene or metal nanoparticle system. The term rubbery refers to certain resilient materials besides natural or synthetic rubbers, with rubbery being a term familiar to those in the polymer arts. For example, cross-linked PPS can be used to form a hydrophobic rubbery core. PPS is a polymer that degrades under oxidative conditions to polysulphoxide and finally polysulphone, transitioning from a hydrophobic rubber to a hydrophilic, water-soluble polymer. Other sulphide polymers may be adapted for use, with the term sulphide polymer referring to a polymer with a sulphur in the backbone of the mer. Other rubbery polymers that may be used are polyesters with glass transition temperature under hydrated conditions that is less than about 37° C. A hydrophobic core can be advantageously used with a hydrophilic overlayer since the core and overlayer will tend not to mingle, so that the overlayer tends to sterically expand away from the core. A core refers to a particle that has a layer on it. A layer refers to a material covering at least a portion of the core. A layer may be adsorbed or covalently bound. A particle or core may be solid or hollow. Rubbery hydrophobic cores are advantageous over rigid hydrophobic cores, such as crystalline or glassy (as in the case of polystyrene) cores, in that higher loadings of hydrophobic drugs can be carried by the particles with the rubbery hydrophobic cores.

Another physical property is the surface's hydrophilicity. A hydrophilic material may have a solubility in water of at least 1 gram per liter when it is uncrosslinked. Steric stabilization of particles with hydrophilic polymers can improve uptake from the interstitium by reducing non-specific interactions; however, the particles' increased stealth nature can also reduce internalization by phagocytic cells in areas having immature lymphocytes. The challenge of balancing these competing features has been met, however, and this application documents the creation of nanoparticles for effective lymphatic delivery to DCs and other APCs in lymph nodes. Some embodiments include a hydrophilic component, e.g., a layer of hydrophilic material. Examples of suitable hydrophilic materials are one or more of polyalkylene oxides, polyethylene oxides, polysaccharides, polyacrylic acids, and polyethers. The molecular weight of polymers in a layer can be adjusted to provide a useful degree of steric hindrance in vivo, e.g., from about 1,000 to about 100,000 or even more; artisans will immediately appreciate that all the ranges and values within the explicitly stated ranges are contemplated, e.g., between 10,000 and 50,000.

The nanoparticles may incorporate functional groups for further reaction. Functional groups for further reaction include electrophiles or nucleophiles; these are convenient for reacting with other molecules. Examples of nucleophiles are primary amines, thiols, and hydroxyls. Examples of electrophiles are succinimidyl esters, aldehydes, isocyanates, and maleimides.

A great variety of means, well known in the art, may be used to conjugate antigenic peptides and proteins to carriers. These methods include any standard chemistries which do not destroy or severely limit the biological activity of the antigen peptides and proteins, and which allow for a sufficient number of antigen peptides and proteins to be conjugated to the carrier in an orientation which allows for interaction of the antigen peptide or protein with a cognate T cell receptor. Generally, methods are preferred which conjugate the C-terminal regions of an antigen peptide or protein, or the C-terminal regions of an antigen peptide or protein fusion protein, to the earner. The exact chemistries will, of course, depend upon the nature of the earner material, the presence or absence of C-terminal fusions to the antigen peptide or protein, and/or the presence or absence of conjugating moieties.

Functional groups can be located on the particle as needed for availability. One location can be as side groups or termini on the core polymer or polymers that are layers on a core or polymers otherwise tethered to the particle. For instance, examples are included herein that describe PEG stabilizing the nanoparticles that can be readily functionalized for specific cell targeting or protein and peptide drug delivery.

Conjugates such as ethylene carbodiimide (ECDI), hexamethylene diisocyanate, propyleneglycol di-glycidylether which contain 2 epoxy residues, and epichlorohydrin may be used for fixation of peptides or proteins to the carrier surface. Without being bound by theory, ECDI is suspected of carrying out two major functions for induction of tolerance: (a) it chemically couples the protein/peptides to the cell surface via catalysis of peptide bond formation between free amino and free carboxyl groups; and (b) it induces the carrier to mimic apoptotic cell death such that they are picked up by host antigen presenting cells (which may include endothelial cells) in the spleen and induce tolerance. It is this presentation to host T-cells in a non-immunogenic fashion that leads to direct induction of anergy in autoreactive cells. In addition, ECDI serves as a potent stimulus for the induction of specific regulatory T cells.

In one series of instances of the disclosure, the antigen peptides and proteins are bound to the carrier via a covalent chemical bond. For example, a reactive group or moiety near the C-terminus of the antigen (e.g., the C-terminal carboxyl group, or a hydroxyl, thiol, or amine group from an amino acid side chain) may be conjugated directly to a reactive group or moiety on the surface of the carrier (e.g., a hydroxyl or carboxyl group of a PLA or PGA polymer, a terminal amine or carboxyl group of a dendrimer, or a hydroxyl, carboxyl or phosphate group of a phospholipid) by direct chemical reaction. Alternatively, there may be a conjugating moiety which covalently conjugates to both the antigen peptides and proteins and the carrier, thereby linking them together.

Reactive carboxyl groups on the surface of a carrier may be joined to free amines (e.g., from Lys residues) on the antigen peptide or protein, by reacting them with, for example, 1 -ethyl-3-[3,9-dimethyl aminopropyl] carbodiimide hydrochloride (EDC) or N-hydroxysuccinimide ester (NHS). Similarly, the same chemistry may be used to conjugate free amines on the surface of a carrier with free carboxyls (e.g., from the C-terminus, or from Asp or Glu residues) on the antigen peptide or protein. Alternatively, free amine groups on the surface of a carrier may be covalently bound to antigen peptides and proteins, or antigen peptide or protein fusion proteins, using sulfo-SIAB chemistry, essentially as described by Arano et al. (1991) Chem. 2:71-6.

In another instance of the disclosure, a non-covalent bond between a ligand bound to the antigen peptide or protein and an anti-ligand attached to the carrier may conjugate the antigen to the carrier. For example, a biotin ligase recognition sequence tag may be joined to the C-terminus of an antigen peptide or protein, and this tag may be biotinylated by biotin ligase. The biotin may then serve as a ligand to non-covalently conjugate the antigen peptide or protein to avidin or streptavidin which is adsorbed or otherwise bound to the surface of the carrier as an anti-ligand. Alternatively, if the antigen peptides and proteins are fused to an immunoglobulin domain bearing an Fc region, as described above, the Fc domain may act as a ligand, and protein A, either covalently or non-covalently bound to the surface of the carrier, may serve as the anti-ligand to non-covalently conjugate the antigen peptide or protein to the carrier. Other means are well known in the art which may be employed to non-covalently conjugate antigen peptides and proteins to carriers, including metal ion chelation techniques (e.g., using a poly-His tag at the C-terminus of the antigen peptide or protein or antigen peptide or protein fusion proteins, and a Ni⁺-coated carrier), and these methods may be substituted for those described here.

Conjugation of a nucleic acid moiety to a platform molecule can be effected in any number of ways, typically involving one or more crosslinking agents and functional groups on the nucleic acid moiety and platform molecule. Linking groups are added to platforms using standard synthetic chemistry techniques. Linking groups can be added to nucleic acid moieties using standard synthetic techniques. The practitioner has a number of choices for antigens used in the combinations of this invention. The inducing antigen present in the combination contributes to the specificity of the tolerogenic response that is induced. It may or may not be the same as the target antigen, which is the antigen present or to be placed in the subject being treated which is a target for the unwanted immunological response, and for which tolerance is desired.

Within the scope of the claims, an inducing antigen of this invention may be a polypeptide, polynucleotide, carbohydrate, glycolipid, or other molecule isolated from a biological source, or it may be a chemically synthesized small molecule, polymer, or derivative of a biological material, providing it has the ability to induce tolerance according to this description when combined with the mucosal binding component.

In some instances, the present disclosure provides a carrier (e.g., immune modifying particle) coupled to one or more peptides, polypeptides, and/or proteins. In some instances of the disclosure, a carrier (e.g., PLG carrier), such as those described herein, are effective to induce antigen-specific tolerance and/or prevent the onset of an immune related disease (such as EAE in a mouse model) and/or diminish the severity of a pre-existing immune related disease. In some instances of the disclosure, the compositions and methods of the present invention can cause T cells to undertake early events associated with T-cell activation, but do not allow T-cells to acquire effector function. For example, administration of compositions of the present disclosure can result in T-cells having a quasi-activated phenotype, such as CD69 and/or CD44 upregulation, but do not display effector function, such as indicated by a lack of IFN-γ or IL-17 synthesis. In some instances of the disclosure, administration of compositions of the present disclosure results in T-cells having a quasi-activated phenotype without having conversion of naive antigen-specific T-cells to a regulatory phenotype, such as those having CD25^{+/}Foxp3⁺ phenotypes.

In some instances of the disclosure, the surface of a carrier (e.g., particle) comprises chemical moieties and/or functional groups that allow attachment (e.g., covalently, non-covalently) of antigenic peptides and/or other functional elements to the carrier. In some instances of the disclosure, the number, orientation, spacing, etc. of chemical moieties and/or functional groups on the carrier (e.g., particle) vary according to carrier chemistry, desired application, etc.

In some embodiments, a carrier comprises one or more biological or chemical agents adhered to, adsorbed on, encapsulated within, and/or contained throughout the carrier. In some embodiments, a chemical or biological agent is encapsulated in and/or contained throughout the particles. The present invention is not limited by the nature of the chemical or biological agents. Such agents include, but are not limited to, proteins, nucleic acid molecules, small molecule drugs, lipids, carbohydrates, cells, cell components, and the like. In some embodiments, two or more (e.g., 3, 4, 5, etc.) different chemical or biological agents are included on or within the carrier. In some embodiments, agents are configured for specific release rates. In some embodiments, multiple different agents are configured for different release rates. For example, a first agent may release over a period of hours while a second agent releases over a longer period of time (e.g., days, weeks, months, etc.). In some embodiments, the carrier or a portion thereof is configured for slow-release of biological or chemical agents. In some embodiments, the slow release provides release of biologically active amounts of the agent over a period of at least 30 days (e.g., 40 days, 50 days, 60 days, 70 days, 80 days, 90 days, 100 days, 180 days, etc.). In some embodiments, the carrier or a portion thereof is configured to be sufficiently porous to permit ingrowth of cells into the pores. The size of the pores may be selected for particular cell types of interest and/or for the amount of ingrowth desired. In some embodiments, the particles comprise the antigen of interest without other non-peptide active agents, such as drugs or immunomodulators. Furthermore, in some embodiments the particles of the invention do not contain immunostimulatory or immunosuppressive peptides in addition to the antigen of interest. Furthermore, in some embodiments, the particles do not contain other proteins or peptides (e.g. costimulatory molecules, MHC molecules, immunostimulatory peptides or immunosuppressive peptides) either on the surface or encapsulated within the particle.

Encapsulation of the antigen, biological, and/or chemical agents in the particle of the invention has been surprisingly found to induce immunological tolerance and has several advantages. First, the encapsulated particles have a slower cytokine response. Second, when using multiple antigens, biological, and/or chemical agents, encapsulation removes the competition between these various molecules that might occur if the agents were attached to the surface of the particle. Third, encapsulation allows more antigens, biological, and/or chemical agents to be incorporated with the particle. Fourth, encapsulation allows for easier use of complex protein antigens or organ homogenates (e.g. pancreas homogenate for type 1 diabetes or peanut extract in peanut allergy). Finally, encapsulation of antigens, biological, and/or chemical agents within the particle instead of conjugation to the surface of the particle maintains the net negative charge on the surface of the particle. The encapsulation of the antigen, biological, and/or chemical agents in the particles of the invention may be performed by any method known in the art. In one instance of the disclosurepolypeptide antigens are encapsulated in the particles by a double-emulsion process. In a further embodiment, the polypeptide antigens are water soluble.

In another instance of the disclosure, the polypeptide antigens are encapsulated in the particles by a single-emulsion process. In a further embodiment, the polypeptide antigens are more hydrophobic. Sometimes, the double emulsion process leads to the formation of large particles which may result in the leakage of the hydrophilic active component and low entrapment efficiencies. The coalescence and Ostwald ripening are two mechanisms that may destabilize the double-emulsion droplet, and the diffusion through the organic phase of the hydrophilic active component is the main mechanism responsible of low levels of entrapped active component. In some instances of the disclosure, it may be beneficial to reduce the nanoparticle size. One strategy to accomplish this is to apply a second strong shear rate. The leakage effect can be reduced by using a high polymer concentration and a high polymer molecular mass, accompanied by an increase in the viscosity of the inner water phase and in increase in the surfactant molecular mass.

In certain embodiments, the present invention provides carriers having thereon (or therein) cells or other biological or chemical agents. Where cells are employed, the carriers are not limited to a particular type of cells. In some embodiments, the carriers have thereon pancreatic islet cells. In some embodiments, the microporous carriers additionally have thereon ECM proteins and/or exendin-4. The carriers are not limited to a particular type. In some embodiments, a carrier has regions of varying porosity (e.g., varying pore size, pore depth, and/or pore density). In some embodiments, carriers have thereon (or therein) pharmaceutical agents, DNA, RNA, extracellular matrix proteins, exendin-4, etc. In certain instances, the present disclosure provides methods for transplanting pancreatic islet cells with such carriers. In certain embodiments of this invention, the inducing antigen is a single isolated or recombinantly produced molecule. For treating conditions where the target antigen is disseminated to various locations in the host, it is generally necessary that the inducing antigen be identical to or immunologically related to the target antigen. Examples of such antigens are most polynucleotide antigens, and some carbohydrate antigens (such as blood group antigens).

Any suitable antigens may find use within the scope of the present disclosure. In some embodiments, the inducing antigen contributes to the specificity of the tolerogenic response that is induced. The inducing antigen may or may not be the same as the target antigen, which is the antigen present or to be placed in the subject being treated which is a target for the unwanted immunological response, and for which tolerance is desired.

Where the target antigen is preferentially expressed on a particular organ, cell, or tissue type, the practitioner again has the option of using an inducing antigen which is identical with or immunologically related to the target antigen. However, there is also the additional option of using an antigen which is a bystander for the target. This is an antigen which may not be immunologically related to the target antigen, but is preferentially expressed in a tissue where the target antigen is expressed. A working theory as to the effectiveness of bystander suppression is that suppression is an active cell-mediated process that down-regulates the effector arm of the immune response at the target cells. The suppressor cells are specifically stimulated by the inducer antigen at the mucosal surface, and home to a tissue site where the bystander antigen is preferentially expressed. Through an interactive or cytokine-mediated mechanism, the localized suppressor cells then down-regulate effector cells (or inducers of effector cells) in the neighborhood, regardless of what they are reactive against. If the effector cells are specific for a target different from the inducing antigen, then the result is a bystander effect. For further elaboration of the bystander reaction and a list of tolerogenic peptides having this effect, the reader is referred to International Patent Publication WO 93/16724. An implication of bystander theory is that one of ordinary skill need not identify or isolate a particular target antigen against which tolerance is desired in order to practice the present invention. The practitioner need only be able to obtain at least one molecule preferentially expressed at the target site for use as an inducing antigen.

In certain embodiments of this invention, the inducing antigen is not in the same form as expressed in the individual being treated, but is a fragment or derivative thereof. Inducing antigens of this invention include peptides based on a molecule of the appropriate specificity but adapted by fragmentation, residue substitution, labeling, conjugation, and/or fusion with peptides having other functional properties. The adaptation may be performed for any desirable purposes, including but not limited to the elimination of any undesirable property, such as toxicity or immunogenicity; or to enhance any desirable property, such as mucosal binding, mucosal penetration, or stimulation of the tolerogenic arm of the immune response. Terms such as insulin peptide, collagen peptide, and myelin basic protein peptide, as used herein, refer not only to the intact subunit, but also to allotypic and synthetic variants, fragments, fusion peptides, conjugates, and other derivatives that contain a region that is homologous (preferably 70% identical, more preferably 80% identical and even more preferably 90% identical at the amino acid level) to at least 10 and preferably 20 consecutive amino acids of the respective molecule for which it is an analog, wherein the homologous region of the derivative shares with the respective parent molecule an ability to induce tolerance to the target antigen.

It is recognized that tolerogenic regions of an inducing antigen are often different from immunodominant epitopes for the stimulation of an antibody response. Tolerogenic regions are generally regions that can be presented in particular cellular interactions involving T cells. Tolerogenic regions may be present and capable of inducing tolerance upon presentation of the intact antigen. Some antigens contain cryptic tolerogenic regions, in that the processing and presentation of the native antigen does not normally trigger tolerance. An elaboration of cryptic antigens and their identification is found in International Patent Publication WO 94/27634.

In certain embodiments of this invention, two, three, or a higher plurality of inducing antigens is used. It may be desirable to implement these embodiments of the invention when there are a plurality of target antigens, or to provide a plurality of bystanders for the target. For example, both insulin and glucagon can be mixed with a mucosal binding component in the treatment of diabetes. It may also be desirable to provide a cocktail of antigens to cover several possible alternative targets. For example, a cocktail of histocompatibility antigen fragments could be used to tolerize a subject in anticipation of future transplantation with an allograft of unknown phenotype. Allovariant regions of human leukocyte antigens are known in the art: e.g., Immunogenetics 29:231, 1989. In another example, a mixture of allergens may serve as inducing antigen for the treatment of atopy.

Inducing antigens can be prepared by a number of techniques known in the art, depending on the nature of the molecule. Polynucleotide, polypeptide, and carbohydrate antigens can be isolated from cells of the species to be treated in which they are enriched. Short peptides are conveniently prepared by amino acid synthesis. Longer proteins of known sequence can be prepared by synthesizing an encoding sequence or PCR-amplifying an encoding sequence from a natural source or vector, and then expressing the encoding sequence in a suitable bacterial or eukaryotic host cell.

In certain embodiments of this invention, the combination comprises a complex mixture of antigens obtained from a cell or tissue, one or more of which plays the role of inducing antigen. The antigens may be in the form of whole cells, either intact or treated with a fixative such as formaldehyde, glutaraldehyde, or alcohol. The antigens may be in the form of a cell lysate, created by detergent solubilization or mechanical rupture of cells or tissue, followed by clarification. The antigens may also be obtained by subcellular fractionation, particularly an enrichment of plasma membrane by techniques such as differential centrifugation, optionally followed by detergent solubilization and dialysis. Other separation techniques are also suitable, such as affinity or ion exchange chromatography of solubilized membrane proteins.

In one instance of the disclosure, the antigenic peptide or protein is an autoantigen, an alloantigen or a transplantation antigen. In yet another particular instance of the disclosure, the autoantigen is selected from the group consisting of myelin basic protein, collagen or fragments thereof, DNA, nuclear and nucleolar proteins, mitochondrial proteins and pancreatic β-cell proteins.

The disclosure provides for the induction of tolerance to an autoantigen for the treatment of autoimmune diseases by administering the antigen for which tolerance is desired. For example, autoantibodies directed against the myelin basic protein (MBP) are observed in patients with multiple sclerosis, and, accordingly, MBP antigenic peptides or proteins may be used in the disclosure to be delivered using the compositions of the present disclosure to treat and prevent multiple sclerosis.

By way of another non-limiting example, an individual who is a candidate for a transplant from a non-identical twin may suffer from rejection of the engrafted cells, tissues or organs, as the engrafted antigens are foreign to the recipient. Prior tolerance of the recipient individual to the intended graft abrogates or reduces later rejection. Reduction or elimination of chronic anti-rejection therapies may be achieved by the practice of the present disclosure. In another example, many autoimmune diseases are characterized by a cellular immune response to an endogenous or self antigen. Tolerance of the immune system to the endogenous antigen is desirable to control the disease.

In a further example, sensitization of an individual to an industrial pollutant or chemical, such as may be encountered on-the-job, presents a hazard of an immune response. Prior tolerance of the individual's immune system to the chemical, in particular in the form of the chemical reacted with the individual's endogenous proteins, may be desirable to prevent the later occupational development of an immune response.

Allergens are other antigens for which tolerance of the immune response thereto is also desirable. In the invention, the antigen is a gliaden. In a further embodiment, the antigen is A-gliaden.

Notably, even in diseases where the pathogenic autoantigen is unknown, bystander suppression may be induced using antigens present in the anatomical vicinity. For example, autoantibodies to collagen are observed in rheumatoid arthritis and, accordingly, a collagen-encoding gene may be utilized as the antigen-expressing gene module in order to treat rheumatoid arthritis (see e.g. Choy (2000) Curr Opin Investig Drugs 1 : 58-62). Furthermore, tolerance to beta cell autoantigens may be utilized to prevent development of type 1 diabetes (see e.g. Bach and Chatenoud (2001) Ann Rev Immunol 19: 131-161).

As another example, auto-antibodies directed against myelin oligodendrocyte glycoprotein (MOG) are observed in autoimmune encephalomyelitis and in many other CNS diseases as well as multiple sclerosis (see e.g. Iglesias et al. (2001) Glia 36: 22-34). Accordingly, use of MOG antigen expressing constructs in the disclosure allows for treatment of multiple sclerosis as well as related autoimmune disorders of the central nervous system.

Still other examples of candidate autoantigens for use in treating autoimmune disease include: pancreatic beta-cell antigens, insulin and GAD to treat insulin-dependent diabetes mellitus; collagen type 11, human cartilage gp 39 (HCgp39) and gpl30-RAPS for use in treating rheumatoid arthritis; myelin basic protein (MBP), proteo lipid protein (PLP) and myelin oligodendrocyte glycoprotein (MOG, see above) to treat multiple sclerosis; fibrillarin, and small nucleolar protein (snoRNP) to treat scleroderma; thyroid stimulating factor receptor (TSH-R) for use in treating Graves' disease; nuclear antigens, histones, glycoprotein gp70 and ribosomal proteins for use in treating systemic lupus erythematosus; pyruvate dehydrogenase dehydrolipoamide acetyltransferase (PCD-E2) for use in treating primary billiary cirrhosis; hair follicle antigens for use in treating alopecia areata; and human tropomyosin isoform 5 (hTM5) for use in treating ulcerative colitis.

In one instance of the disclosure, the particles of the invention are coupled to antigens comprising one or more epitopes associated with allergies, autoimmune diseases and/or inflammatory diseases or disorders. The antigens may comprise one or more copies of an epitope. In one embodiment, the antigens comprise a single epitope associated with one disease or disorder. In a further embodiment, the antigens comprise more than one epitope associated with the same disease or disorder. In yet a further embodiment, the antigens comprise more than one epitope associated with different diseases or disorders. In a further embodiment, the antigens comprise one or more epitopes associated with one or more allergies. In a further instance of the disclosure, the antigens comprise one or more epitopes associated with multiple sclerosis, type 1 diabetes. Celiac's disease, and/or inflammatory bowel disease, including Crohn's disease or ulcerative colitis. In one instance of the disclosure, the epitopes are from myelin basic protein (e.g. SEQ ID NOs:4975 & 4976), proteolipid protein (e.g. SEQ ID NO: 4977), myelin oligodendrocyte glycoprotein (e.g. SEQ ID NOs: 1 & 4978), aquaporin, (e.g. SEQ ID NO: 4979), myelin associated glycoprotein (e.g. SEQ ID NO: 4980), insulin (e.g. SEQ ID NO: 4981), glutamic acid decarboxylase (e.g. SEQ ID NO: 4982), gliadin (e.g. SEQ ID NOs:4983-4985 or 5136-5140), the α3 chain of type IV collagen (e.g. SEQ ID NO: 5017), or fragments, homologs, or isoforms thereof. In a further instance of the disclosure, the epitopes are from gluten, including from gliadin and/or glutenin. In one instance of the disclosure, the epitopes are from insulin homologs, such as those described in U.S. Patent No. 8,476,228. In one embodiment, the gliaden epitopes are SEQ ID NOs: 13, 14, 16, 320, or 321 in U.S. Application No. 20110293644.

Further non-limiting examples of epitopes associated with various autoimmune diseases and/or inflammatory diseases or disorders that are contemplated by the instant disclosure are described in Tables 2 and 3.

**Table 2 - Representative Linear Epitopes**

| Disease | Representative Epitopes |
|---|---|
| Multiple Sclerosis | SEQ ID NOs: 2-1294 |
| Celiac Disease | SEQ ID NOs: 1295-1724; |
| | SEQ ID NOs: 1726-1766; |
| | SEQ ID NOs: 4986-5140 |
| Diabetes | SEQ ID NOs: 1767-1840; |
| | SEQ ID NOs: 1842-1962; |
| | SEQ ID NOs: 1964-2027; |
| | SEQ ID NOs: 2029-2073; |
| | SEQ ID NOs: 2075-2113; |
| | SEQ ID NOs: 2115-2197; |
| | SEQ ID NOs: 2199-2248; |
| | SEQ ID NOs: 2250-2259; |
| | SEQ ID NOs: 2261-2420; |
| | SEQ ID NOs: 2422-2486; |
| | SEQ ID NOs: 2489-2505 |
| Rheumatoid Arthritis | SEQ ID NOs: 2506-3260; |
| | SEQ ID NOs:3262-3693 |
| Systemic Lupus Erythematosus | SEQ ID NOs: 3694-3857; |
| | SEQ ID NOs: 3860-4565 |
| Good Pasture's Syndrome | SEQ ID NOs: 4566-4576; |
| | SEQ ID NOs: 4578-4610; |
| | SEQ ID NOs: 4612-4613; |
| | SEQ ID NOs: 5018-5039 |
| Autoimmune Uveitis | SEQ ID NOs: 4614-4653 |
| Autoimmune Thyroiditis | SEQ ID NOs: 4654-4694; |
| | SEQ ID NOs: 4696-4894; |
| | SEQ ID NOs: 4896-4901 |
| Autoimmune Myositis | SEQ ID NOs: 4902-4906 |
| Autoimmune Vasculitis | SEQ ID NOs: 4907-4914 |
| Autoimmune Pancreatitis | SEQ ID NOs: 4915-4917 |
| Crohns Disease | SEQ ID NOs: 4918-4941 |
| Ulcerative Colitis | SEQ ID NOs: 4942-4952 |
| Psoriasis | SEQ ID NOs: 4953-4963 |
| Reactive Arthritis | SEQ ID NOs: 4964-4974 |

Not all epitopes are linear epitopes; epitopes can also be discontinuous, conformational epitopes. A number of discontinuous epitopes associated with autoimmune diseases or inflammatory diseases and/or disorders are known. Non-limiting examples of discontinuous epitopes are described in Table 3.

**Table 3- Representative Discontinuous Epitopes**

| **Disease** | **Epitope** | **Full Length Polypeptide** |
|---|---|---|
| Celiac Disease | D151, E153, E154, E155, E158; | Protein-glutamine gamma-glutamyltransferase 2 |
| | D306, N308, N310; | SEQ ID NO: 1725 |
| | D434, E435, E437, D438; | |
| | E329; | |
| | E153; | |
| | R19, E153, M659;or | |
| | C277, H335, D358 | |
| Diabetes | E517; | Glutamate decarboxylase 2 |
| | R255, F256, K257, K263, E264, K265, L270, P271, R272, L273, L285, K286, K287, 1294, G295, T296, D297, S298, R317, R318; | SEQ ID NOs: 1841, 1963, 2114, & 2249 |
| | N483, 1484, 1485, K486, N487, R488, E489, G490, Y491, E492, M493, V494, F495, D496, G497, K498, P499, F556, F557, R558, M559, V560, 1561, S562, N563, P564, A565, A566, T567, H568, Q569, D570, 1571, D572, F573, L574, 1575, E576, E577, 1578, E579, R580, L581, G582, Q583, D584, L585; | |
| | E264; | |
| | E517, E520, E521, S524, S527, V532; | |
| | E517, E521; | |
| | K358; | |
| | R536, Y540 | |
| Diabetes | P876, A877, E878, T880; | protein tyrosine phosphatase, receptor type, N precursor |
| | T804; | |
| | T804, V813, D821, R822, Q862, P886; | SEQ ID NOs: 2028 & 2074 |
| | T804, V813, D821, R822, Q862, P886; | |
| | W799, E836, N858; | |
| | D911; | |
| | Q862; | |
| | L831, H833, V834, E836, Q860; | |
| | W799, E836, N858; | |
| | W799, L831, H833, V834, Y835, E836, Q860; | |
| Diabetes | R325, R332, E333, K336, K340; R325; W325 | zinc transporter 8 isoform a SEQ ID NO: 2421 |
| Diabetes | E872, C945 | Receptor-type tyrosine-protein phosphatase N2 |
| | | SEQ ID NOs: 2198, 2260, & 2487 |
| Diabetes | W799, C909 | tyrosine phosphatase |
| | | SEQ ID NO: 2488 |
| Rheumatoid Arthritis | L14, M15, 116, S17, R18, N147, G148, S187, M191, H196, N197, H198, Y199, Q201, S203 | Chain A, Crystal Structure Of A Human Igm Rheumatoid Factor Fab In Complex With Its Autoantigen Igg Fc |
| | | SEQ ID NO: 3261 |
| Systemic Lupus Erythematosus | K591, S592, G593 | ATP-dependent DNA helicase 2 subunit 1 |
| | | SEQ ID NO: 3858 |
| Systemic Lupus Erythematosus | M1, K2, L3, V4, R5, F6, L7, M8, K9, L10, S11, H12, E13, T14, V15, T16, 117, E18, L19, K20, N21, G22, T23, Q24, V25, H26, P85, K86, V87, K88, S89, K90, K91, R92, E93, A94, V95, A96, G97, R98, G99, R100, G101, R102, G103, R104, G105, R106, G107, R108, G109, R110, G111, R112, G113, R114, G115, G116, P117, R118, R119 | Small nuclear ribonucleoprotein Sm D1 |
| | | SEQ ID NO: 3859 |
| Systemic Lupus Erythematosus | G59, R62 | beta-2-glycoprotein I |
| | | SEQ ID NO: 4357 |
| Good Pasture's Syndrome | T24, A25, I26, S28, E31, V34, P35, S38, Q64 | type IV collagen alpha3 chain |
| | | SEQ ID NO: 4577 |
| Good Pasture's Syndrome | T1455, A1456, I1457, S1459, E1462, T1464, V1465, P1466, Y1468, S1469, Q1495, T1537, T1565, P1569, H1572, K1579, A1634 | alpha3 type IV collagen |
| | | SEQ ID NO: 4611 |
| Autoimmune Thyroiditis | E604, D620, K627, D630; | Thyroid peroxidase |
| | R225, R646, D707; | SEQ ID NO: 4695 |
| | K627; | |
| | R225; | |
| | Y772; | |
| | K713, F714, P715, E716; P715, D717 | |
| Autoimmune Thyroiditis | D36, R38, K42, Q55, K58, 160, E61, R80, Y82, S84, T104, H105, E107, R109, N110, K129, F130, D151, F153, 1155, E157, T181, K183, D203 | Thyrotropin receptor |
| | | SEQ ID NO: 4895 |

Combinations of antigens and/or epitopes can be tested for their ability to promote tolerance by conducting experiments with isolated cells or in animal models.

In some embodiments, the tolerance inducing compositions of the present invention contain an apoptosis signaling molecule (e.g., in addition to an antigenic peptide or other antigenic molecule). In some embodiments, the apoptosis signaling molecule is coupled and/or associated with the surface of the carrier. In some embodiments an apoptotic signaling molecules allows a carrier to be perceived as an apoptotic body by antigen presenting cells of the host, such as cells of the host reticuloendothelial system; this allows presentation of the associated peptide epitopes in a tolerance-inducing manner. Without being bound by theory, this is presumed to prevent the upregulation of molecules involved in immune cell stimulation, such as MHC class I/II, and costimulatory molecules. These apoptosis signaling molecules may also serve as phagocytic markers. For example, apoptosis signaling molecules suitable for the present invention have been described in US Pat App No. 20050113297. Molecules suitable for the present invention include molecules that target phagocytes, which include macrophages, dendritic cells, monocytes, granulocytes and neutrophils.

In some embodiments, molecules suitable as apoptotic signalling molecules act to enhance tolerance of the associated peptides. Additionally, a carrier bound to an apoptotic signaling molecule can be bound by Clq in apoptotic cell recognition (Paidassi et al., (2008) J. Immunol. 180:2329-2338. For example, molecules that may be useful as apoptotic signalling molecules include phosphatidyl serine, annexin-1, annexin-5, milk fat globule-EGF-factor 8 (MFG-E8), or the family of thrombospondins (e.g., thrombospondin-1 (TSP-1)). Various molecules suitable for use as apoptotic signalling molecules with the present invention are discussed, for example, in U.S. Pat. App. 2012/0076831.

In some embodiments, the apoptotic signalling molecule may be conjugated to the antigen-specific peptide. In some instances, the apoptotic signalling molecule and antigen-specific peptide are conjugated by the creation of a fusion protein. For example a fusion protein may comprise at least one antigen-specific peptide (or a fragment or a variant thereof) coupled to at least one molecule of an apoptotic signalling molecule (or a fragment or a variant thereof). For the creation of fusion proteins, the terms "fusion protein," "fusion peptide," "fusion polypeptide," and "chimeric peptide" are used interchangably. Suitable fragments of the antigen-specific peptide include any fragment of the full-length peptide that retains the function of generating the desired antigen-specific tolerance function of the present invention. The fusion protein may be created by various means understood in the art (e.g., genetic fusion, chemical conjugation, etc.). The two proteins may be fused either directly or via an amino acid linker. The polypeptides forming the fusion protein are typically linked C-terminus to N-terminus, although they can also be linked C-terminus to C-terminus, N-terminus to N-terminus, or N-terminus to C-terminus. The polypeptides of the fusion protein can be in any order. A peptide linker sequence may be employed to separate the first and second polypeptide components by a distance sufficient to ensure that each polypeptide folds into its secondary and tertiary structures. Amino acid sequences which may be usefully employed as linkers include those disclosed in Maratea et. al., Gene 40:39-46 (1985); Murphy et al., Proc. Natl. Acad. Sci. USA 83:8258-8262 (1986); U.S. Pat. No. 4,935,233 and U.S. Pat. No. 4,751,180. The linker sequence may generally be from 1 to about 50 amino acids in length. In some embodiments, linker sequences are not required and/or utilized, for example, when the first and second polypeptides have non-essential N-terminal amino acid regions that can be used to separate the functional domains and prevent steric interference.

A proxy for tolerogenic activity is the ability of an intact antigen or fragment to stimulate the production of an appropriate cytokine at the target site. The immunoregulatory cytokine released by T suppressor cells at the target site is thought to be TGF-β (Miller et al., Proc. Natl. Acad. Sci. USA 89:421, 1992). Other factors that may be produced during tolerance are the cytokines IL4 and IL-10, and the mediator PGE. In contrast, lymphocytes in tissues undergoing active immune destruction secrete cytokines such as IL-I, IL-2, IL-6, and γ-IFN. Hence, the efficacy of a candidate inducing antigen can be evaluated by measuring its ability to stimulate the appropriate type of cytokines.

With this in mind, a rapid screening test for tolerogenic epitopes of the inducing antigen, effective mucosal binding components, effective combinations, or effective modes and schedules of mucosal administration can be conducted using syngeneic animals as donors for in vitro cell assays. Animals are treated at a mucosal surface with the test composition, and at some time are challenged with parenteral administration of the target antigen in complete Freund's adjuvant. Spleen cells are isolated, and cultured in vitro in the presence of the target antigen at a concentration of about 50 µg/mL. Target antigen can be substituted with candidate proteins or sub-fragments to map the location of tolerogenic epitopes. Cytokine secretion into the medium can be quantitated by standard immunoassay.

The ability of the cells to suppress the activity of other cells can be determined using cells isolated from an animal immunized with the target antigen, or by creating a cell line responsive to the target antigen (Ben-Nun et al., Eur. J. Immunol. 11 :195, 1981. In one variation of this experiment, the suppressor cell population is mildly irradiated (about 1000 to 1250 rads) to prevent proliferation, the suppressors are co-cultured with the responder cells, and then tritiated thymidine incorporation (or MTT) is used to quantitate the proliferative activity of the responders. In another variation, the suppressor cell population and the responder cell population are cultured in the upper and lower levels of a dual chamber transwell culture system (Costar, Cambridge Mass.), which permits the populations to coincubate within 1 mm of each other, separated by a polycarbonate membrane (WO 93/16724). In this approach, irradiation of the suppressor cell population is unnecessary, since the proliferative activity of the responders can be measured separately.

In embodiments of the invention where the target antigen is already present in the individual, there is no need to isolate the antigen or precombine it with the mucosal binding component. For example, the antigen may be expressed in the individual in a certain fashion as a result of a pathological condition (such as inflammatory bowel disease or Celiac disease) or through digestion of a food allergen. Testing is performed by giving the mucosal binding component in one or more doses or formulations, and determining its ability to promote tolerization against the antigen in situ.

The effectiveness of compositions and modes of administration for treatment of specific disease can also be elaborated in a corresponding animal disease model. The ability of the treatment to diminish or delay the symptomatology of the disease is monitored at the level of circulating biochemical and immunological hallmarks of the disease, immunohistology of the affected tissue, and gross clinical features as appropriate for the model being employed. Non-limiting examples of animal models that can be used for testing are included in the following section.

The invention contemplates modulation of tolerance by modulating TH1 response, TH2 response, TH17 response, or a combination of these responses. Modulating TH1 response encompasses changing expression of, e.g., interferon-gamma. Modulating TH2 response encompasses changing expression of, e.g., any combination of IL-4, IL-5, IL-10, and IL-13. Typically an increase (decrease) in TH2 response will comprise an increase (decrease) in expression of at least one of IL-4, IL-5, IL-10, or IL-13; more typically an increase (decrease) in TH2 response will comprise an increase in expression of at least two of IL-4, IL-5, IL-10, or EL-13, most typically an increase (decrease) in TH2 response will comprise an increase in at least three of DL-4, IL-5, IL-10, or IL-13, while ideally an increase (decrease) in TH2 response will comprise an increase (decrease) in expression of all of IL-4, IL-5, IL-10, and IL-13. Modulating TH 17 encompasses changing expression of, e.g., TGF-beta, IL-6, IL-21 and IL23, and effects levels of IL-17, IL-21 and IL-22.

Other suitable methods for assessing the effectiveness of compositions and methods of the present invention are understood in the art, as are discussed, for example, in U.S. Pat. App. 2012/0076831.

Certain embodiments of this invention relate to priming of immune tolerance in an individual not previously tolerized by therapeutic intervention. These embodiments generally involve a plurality of administrations of a combination of antigen and mucosal binding component. Typically, at least three administrations, frequently at least four administrations, and sometimes at least six administrations are performed during priming in order to achieve a long-lasting result, although the subject may show manifestations of tolerance early in the course of treatment. Most often, each dose is given as a bolus administration, but sustained formulations capable of mucosal release are also suitable. Where multiple administrations are performed, the time between administrations is generally between 1 day and 3 weeks, and typically between about 3 days and 2 weeks. Generally, the same antigen and mucosal binding component are present at the same concentration, and the administration is given to the same mucosal surface, but variations of any of these variables during a course of treatment may be accommodated.

Other embodiments of this invention relate to boosting or extending the persistence of a previously established immune tolerance. These embodiments generally involve one administration or a short course of treatment at a time when the established tolerance is declining or at risk of declining. Boosting is generally performed 1 month to 1 year, and typically 2 to 6 months after priming or a previous boost. This invention also includes embodiments that involve regular maintenance of tolerance on a schedule of administrations that occur semiweekly, weekly, biweekly, or on any other regular schedule.

The particles of the current invention can be given in any dose effective to dampen the inflammatory immune response in a subject in need thereof or to treat a bacterial or viral infection in a subject in need thereof. In certain embodiments, about 10² to about 10²⁰ particles are provided to the individual. In a further embodiment between about 10³ to about 10¹⁵ particles are provided. In yet a further embodiment between about 10⁶ to about 10¹² particles are provided. In still a further embodiment between about 10⁸ to about 10¹⁰ particles are provided. In a preferred embodiment the preferred dose is 0.1% solids/ml. Therefore, for 0.5 µm beads, a preferred dose is approximately 4 x 10⁹ beads, for 0.05µm beads, a preferred dose is approximately 4 x 10¹² beads, for 3µm beads, a preferred dose is 2 x 10⁷ beads. However, any dose that is effective in treating the particular condition to be treated is encompassed by the current invention.

The disclosureis useful for treatment of immune related disorders such as autoimmune disease, transplant rejection, enzyme deficiencies and allergic reactions. Substitution of a synthetic, biocompatible particle system to induce immune tolerance could lead to ease of manufacturing, broad availability of therapeutic agents, increase uniformity between samples, increase the number of potential treatment sites and dramatically reduce the potential for allergic responses to a carrier cell.

As used herein, the term "immune response" includes T cell mediated and/or B cell mediated immune responses. Exemplary immune responses include T cell responses, e.g., cytokine production and cellular cytotoxicity. In addition, the term immune response includes immune responses that are indirectly effected by T cell activation, e.g., antibody production (humoral responses) and activation of cytokine responsive cells, e.g., macrophages. Immune cells involved in the immune response include lymphocytes, such as B cells and T cells (CD4⁺, CD8⁺, Th1 and Th2 cells); antigen presenting cells (e.g., professional antigen presenting cells such as dendritic cells, macrophages, B lymphocytes, Langerhans cells, and nonprofessional antigen presenting cells such as keratinocytes, endothelial cells, astrocytes, fibroblasts, oligodendrocytes); natural killer cells; myeloid cells, such as macrophages, eosinophils, mast cells, basophils, and granulocytes. In some embodiments, the modified particles of the present invention are effective to reduce inflammatory cell trafficking to the site of inflammation.

As used herein, the term "anergy," "tolerance," or "antigen-specific tolerance" refers to insensitivity of T cells to T cell receptor-mediated stimulation. Such insensitivity is generally antigen- specific and persists after exposure to the antigenic peptide has ceased. For example, anergy in T cells is characterized by lack of cytokine production, e.g., IL-2. T-cell anergy occurs when T cells are exposed to antigen and receive a first signal (a T cell receptor or CD-3 mediated signal) in the absence of a second signal (a costimulatory signal). Under these conditions, re-exposure of the cells to the same antigen (even if re-exposure occurs in the presence of a costimulatory molecule) results in failure to produce cytokines and subsequently failure to proliferate. Thus, a failure to produce cytokines prevents proliferation. Anergic T cells can, however, proliferate if cultured with cytokines (e.g., IL-2). For example, T cell anergy can also be observed by the lack of IL-2 production by T lymphocytes as measured by ELISA or by a proliferation assay using an indicator cell line. Alternatively, a reporter gene construct can be used. For example, anergic T cells fail to initiate DL-2 gene transcription induced by a heterologous promoter under the control of the 5' IL-2 gene enhancer or by a multimer of the API sequence that can be found within the enhancer (Kang et al. 1992 Science. 257:1134).

As used herein, the term "immunological tolerance" refers to methods performed on a proportion of treated subjects in comparison with untreated subjects where: a) a decreased level of a specific immunological response (thought to be mediated at least in part by antigen-specific effector T lymphocytes, B lymphocytes, antibody, or their equivalents); b) a delay in the onset or progression of a specific immunological response; or c) a reduced risk of the onset or progression of a specific immunological response. "Specific" immunological tolerance occurs when immunological tolerance is preferentially invoked against certain antigens in comparison with others. "Non-Specific" immunological tolerance occurs when immunological tolerance is invoked indiscriminately against antigens which lead to an inflammatory immune response. "Quasi-Specific" immunological tolerance occurs when immunological tolerance is invoked semi-discriminately against antigens which lead to a pathogenic immune response but not to others which lead to a protective immune response.

Tolerance to autoantigens and autoimmune disease is achieved by a variety of mechanisms including negative selection of self-reactive T cells in the thymus and mechanisms of peripheral tolerance for those autoreactive T cells that escape thymic deletion and are found in the periphery. Examples of mechanisms that provide peripheral T cell tolerance include "ignorance" of self antigens, anergy or unresponsiveness to autoantigen, cytokine immune deviation, and activation-induced cell death of self- reactive T cells. In addition, regulatory T cells have been shown to be involved in mediating peripheral tolerance. See, for example, Walker et al. (2002) Nat. Rev. Immunol. 2: 11-19; Shevach et al. (2001) Immunol. Rev. 182:58-67. In some situations, peripheral tolerance to an autoantigen is lost (or broken) and an autoimmune response ensues. For example, in an animal model for EAE, activation of antigen presenting cells (APCs) through TLR innate immune receptors was shown to break self-tolerance and result in the induction of EAE (Waldner et al. (2004) J. Clin. Invest. 113:990-997).

Accordingly, in some embodiments, the invention provides methods for increasing antigen presentation while suppressing or reducing TLR7/8, TLR9, and/or TLR 7/8/9 dependent cell stimulation. As described herein, administration of particular modified particles results in antigen presentation by DCs or APCs while suppressing the TLR 7/8, TLR9, and/or TLR7/8/9 dependent cell responses associated with immunostimulatory polynucleotides. Such suppression may include decreased levels of one or more TLR-associated cytokines.

As discussed above this invention provides novel compounds that have biological properties useful for the treatment of Mac-1 and LFA-1 mediated disorders.

Accordingly, in another aspect of the present invention, pharmaceutical compositions are provided, which comprise the immune modifying particles and optionally comprise a pharmaceutically acceptable carrier. In certain embodiments, these compositions optionally further comprise one or more additional therapeutic agents. Alternatively, the modified particles of the current invention may be administered to a patient in need thereof in combination with the administration of one or more other therapeutic agents. For example, additional therapeutic agents for conjoint administration or inclusion in a pharmaceutical composition with a compound of this invention may be an approved anti-inflammatory agent, or it may be any one of a number of agents undergoing approval in the Food and Drug Administration that ultimately obtain approval for the treatment of any disorder characterized by an uncontrolled inflammatory immune response or a bacterial or viral infection. It will also be appreciated that certain of the modified particles of present invention can exist in free form for treatment, or where appropriate, as a pharmaceutically acceptable derivative thereof.

The pharmaceutical compositions of the present invention additionally comprise a pharmaceutically acceptable carrier, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutical compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutical composition, its use is contemplated to be within the scope of this invention. Some examples of materials which can serve as pharmaceutically acceptable carriers include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil, sesame oil; olive oil; corn oil and soybean oil; glycols; such as propylene glycol; esters such as ethyl oleate and ethyl laurate; agar; buffering agents such as magnesium hydroxide and aluminum hydroxide; alginic acid; pyrogenfree water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

The particles of the invention may be administered orally, nasally, intravenously, intramuscularly, ocularly, transdermally, intraperitoneally, or subcutaneously. In one embodiment, the particles of the invention are administered intravenously.

The effective amounts and method of administration of the present disclosure for modulation of an immune response can vary based on the individual, what condition is to be treated and other factors evident to one skilled in the art. Factors to be considered include route of administration and the number of doses to be administered. Such factors are known in the art and it is well within the skill of those in the art to make such determinations without undue experimentation. A suitable dosage range is one that provides the desired regulation of immune. Useful dosage ranges of the carrier, given in amounts of carrier delivered, may be, for example, from about any of the following: 0.5 to 10 mg/kg, 1 to 9 mg/kg, 2 to 8 mg/kg, 3 to 7 mg/kg, 4 to 6 mg/kg, 5 mg/kg, 1 to 10 mg/kg, 5 to 10 mg/kg. Alternatively, the dosage can be administered based on the number of particles. For example, useful dosages of the carrier, given in amounts of carrier delivered, may be, for example, about 10⁶, 10⁷, 10⁸, 10⁹, 10¹⁰, or greater number of particles per dose. The absolute amount given to each patient depends on pharmacological properties such as bioavailability, clearance rate and route of administration. Details of pharmaceutically acceptable carriers, diluents and excipients and methods of preparing pharmaceutical compositions and formulations are provided in Remmingtons Pharmaceutical Sciences 18th Edition, 1990, Mack Publishing Co., Easton, Pa., USA..

The effective amount and method of administration of the particular carrier formulation can vary based on the individual patient, desired result and/or type of disorder, the stage of the disease and other factors evident to one skilled in the art. The route(s) of administration useful in a particular application are apparent to one of skill in the art. Routes of administration include but are not limited to topical, dermal, transdermal, transmucosal, epidermal, parenteral, gastrointestinal, and naso-pharyngeal and pulmonary, including transbronchial and transalveolar. A suitable dosage range is one that provides sufficient IRP-containing composition to attain a tissue concentration of about 1-50 µM as measured by blood levels. The absolute amount given to each patient depends on pharmacological properties such as bioavailability, clearance rate and route of administration.

The present invention provides carrier formulations suitable for topical application including, but not limited to, physiologically acceptable implants, ointments, creams, rinses and gels. Exemplary routes of dermal administration are those which are least invasive such as transdermal transmission, epidermal administration and subcutaneous injection.

Transdermal administration is accomplished by application of a cream, rinse, gel, etc. capable of allowing the carrier to penetrate the skin and enter the blood stream. Compositions suitable for transdermal administration include, but are not limited to, pharmaceutically acceptable suspensions, oils, creams and ointments applied directly to the skin or incorporated into a protective carrier such as a transdermal device (so-called "patch"). Examples of suitable creams, ointments etc. can be found, for instance, in the Physician's Desk Reference. Transdermal transmission may also be accomplished by iontophoresis, for example using commercially available patches which deliver their product continuously through unbroken skin for periods of several days or more. Use of this method allows for controlled transmission of pharmaceutical compositions in relatively great concentrations, permits infusion of combination drugs and allows for contemporaneous use of an absorption promoter.

Parenteral routes of administration include but are not limited to electrical (iontophoresis) or direct injection such as direct injection into a central venous line, intravenous, intramuscular, intraperitoneal, intradermal, or subcutaneous injection. Formulations of carrier suitable for parenteral administration are generally formulated in USP water or water for injection and may further comprise pH buffers, salts bulking agents, preservatives, and other pharmaceutically acceptable excipients. Immunoregulatory polynucleotide for parenteral injection may be formulated in pharmaceutically acceptable sterile isotonic solutions such as saline and phosphate buffered saline for injection.

Gastrointestinal routes of administration include, but are not limited to, ingestion and rectal routes and can include the use of, for example, pharmaceutically acceptable powders, pills or liquids for ingestion and suppositories for rectal administration.

Naso-pharyngeal and pulmonary administration include are accomplished by inhalation, and include delivery routes such as intranasal, transbronchial and transalveolar routes. The invention includes formulations of carrier suitable for administration by inhalation including, but not limited to, liquid suspensions for forming aerosols as well as powder forms for dry powder inhalation delivery systems. Devices suitable for administration by inhalation of carrier formulations include, but are not limited to, atomizers, vaporizers, nebulizers, and dry powder inhalation delivery devices.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono-or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension or crystalline or amorphous material with poor water solubility. The rate of absorption of the drug then depends upon its rate of dissolution that, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include (poly(orthoesters) and poly(anhydrides). Depot injectable formulations are also prepared by entrapping the drug in liposomes or microemulsions which are compatible with body tissues.

In some embodiments, the synthetic, biodegradable particles of the present invention provide ease of manufacturing, broad availability of therapeutic agents, and increased treatment sites. In particular embodiments, surface-functionalized biodegradable poly(lactide-*co*-glycolide) particles with a high density of surface carboxylate groups, synthesized using the surfactant poly(ethylene-*alt*-maleic anhydride) provide a carrier that offers numerous advantages over other carrier particles and/or surfaces. Experiments conducted during development of embodiments of the present invention demonstrated the conjugation of peptides (e.g., PLP₁₃₉₋₁₅₁ peptide) to these particles. Such peptide-coupled particles have shown that they are effective for the prevention of disease development and the induction of immunological tolerance (e.g., in the SJL/J PLP₁₃₉₋₁₅₁ /CFA-induced R-EAE murine model of multiple sclerosis). Peptide coupled carriers of the present invention provide numerous advantages over other tolerance induction structures. In some embodiments, the particles are biodegradable, and therefore will not persist for long times in the body. The time for complete degradation can be controlled. In some embodiments, particles are functionalized to facilitate internalization without cell activation (e.g., phosphatidylserine loaded into PLG microspheres). In some embodiments, particles incorporate targeting ligands for a specific cell population. In some embodiments, anti-inflammatory cytokines such as IL-10 and TGF-β, are included on or within particles to limit activation of the cell type that is internalizing the particles and to facilitate the induction of tolerance via energy and/or deletion and the activation of regulatory T cells. The composition of the particles has been found to affect the length of time the particles persist in the body and tolerance requires rapid particle uptake and clearance/degradation. Since ratios of over 50:50 lactide:glycolide slow the degradation rate, the particles of the invention have a lactide:glycolide ratio of about 50:50 or below. In one embodiment the particles of the invention have about a 50:50 D,L-lactide:glycolide ratio.

Solid dosage forms for oral administration include capsules, tablets, pills, powders, and granules. In such solid dosage forms, the modified particles are mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polyethylene glycols and the like.

The modified particles can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose and starch. Such dosage forms may also comprise, as in normal practice, additional substances other than inert diluents, e.g., tableting lubricants and other tableting aids such as magnesium stearate and microcrystalline cellulose. In the case of capsules, tablets and pills, the dosage forms may also comprise buffering agents. They may optionally contain opacifying agents and can also be of a composition that they release the modified particles only, or preferentially, in a certain part of the intestinal tract, optionally, in a delayed manner. Examples of embedding compositions which can be used include polymeric substances and waxes.

The present invention encompasses pharmaceutically acceptable topical formulations of the inventive modified particles. The term "pharmaceutically acceptable topical formulation", as used herein, means any formulation which is pharmaceutically acceptable for intradermal administration of modified microparticles of the invention by application of the formulation to the epidermis. In certain embodiments of the invention, the topical formulation comprises a carrier system. Pharmaceutically effective carriers include, but are not limited to, solvents (e.g., alcohols, poly alcohols, water), creams, lotions, ointments, oils, plasters, liposomes, powders, emulsions, microemulsions, and buffered solutions (e.g., hypotonic or buffered saline) or any other carrier known in the art for topically administering pharmaceuticals. A more complete listing of art-known carriers is provided by reference texts that are standard in the art, for example, Remington's Pharmaceutical Sciences, 16th Edition, 1980 and 17th Edition, 1985, both published by Mack Publishing Company, Easton, Pa. In certain other embodiments, the topical formulations of the invention may comprise excipients. Any pharmaceutically acceptable excipient known in the art may be used to prepare the inventive pharmaceutically acceptable topical formulations. Examples of excipients that can be included in the topical formulations of the invention include, but are not limited to, preservatives, antioxidants, moisturizers, emollients, buffering agents, solubilizing agents, other penetration agents, skin protectants, surfactants, and propellants, and/or additional therapeutic agents used in combination to the modified particles. Suitable preservatives include, but are not limited to, alcohols, quaternary amines, organic acids, parabens, and phenols. Suitable antioxidants include, but are not limited to, ascorbic acid and its esters, sodium bisulfite, butylated hydroxytoluene, butylated hydroxyanisole, tocopherols, and chelating agents like EDTA and citric acid. Suitable moisturizers include, but are not limited to, glycerine, sorbitol, polyethylene glycols, urea, and propylene glycol. Suitable buffering agents for use with the invention include, but are not limited to, citric, hydrochloric, and lactic acid buffers. Suitable solubilizing agents include, but are not limited to, quaternary ammonium chlorides, cyclodextrins, benzyl benzoate, lecithin, and polysorbates. Suitable skin protectants that can be used in the topical formulations of the invention include, but are not limited to, vitamin E oil, allatoin, dimethicone, glycerin, petrolatum, and zinc oxide.

In certain embodiments, the pharmaceutically acceptable topical formulations of the invention comprise at least the modified particles of the invention and a penetration enhancing agent. The choice of topical formulation will depend or several factors, including the condition to be treated, the physicochemical characteristics of the inventive compound and other excipients present, their stability in the formulation, available manufacturing equipment, and costs constraints. As used herein the term "penetration enhancing agent" means an agent capable of transporting a pharmacologically active compound through the stratum corneum and into the epidermis or dermis, preferably, with little or no systemic absorption. A wide variety of compounds have been evaluated as to their effectiveness in enhancing the rate of penetration of drugs through the skin. See, for example, Percutaneous Penetration Enhancers, Maibach H. I. and Smith H. E. (eds.), CRC Press, Inc., Boca Raton, Fla. (1995), which surveys the use and testing of various skin penetration enhancers, and Buyuktimkin et al., Chemical Means of Transdermal Drug Permeation Enhancement in Transdermal and Topical Drug Delivery Systems, Gosh T. K., Pfister W. R., Yum S. I. (Eds.), Interpharm Press Inc., Buffalo Grove, III. (1997). In certain exemplary embodiments, penetration agents for use with the invention include, but are not limited to, triglycerides (e.g., soybean oil), aloe compositions (e.g., aloe-vera gel), ethyl alcohol, isopropyl alcohol, octolyphenylpolyethylene glycol, oleic acid, polyethylene glycol 400, propylene glycol, N-decylmethylsulfoxide, fatty acid esters (e.g., isopropyl myristate, methyl laurate, glycerol monooleate, and propylene glycol monooleate) and N-methylpyrrolidone.

In certain embodiments, the compositions may be in the form of ointments, pastes, creams, lotions, gels, powders, solutions, sprays, inhalants or patches. In certain exemplary embodiments, formulations of the compositions according to the invention are creams, which may further contain saturated or unsaturated fatty acids such as stearic acid, palmitic acid, oleic acid, palmito-oleic acid, cetyl or oleyl alcohols, stearic acid being particularly preferred. Creams of the invention may also contain a non-ionic surfactant, for example, polyoxy-40-stearate. In certain embodiments, the active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a compound to the body. Such dosage forms are made by dissolving or dispensing the compound in the proper medium. As discussed above, penetration enhancing agents can also be used to increase the flux of the compound across the skin. The rate can be controlled by either providing a rate controlling membrane or by dispersing the compound in a polymer matrix or gel.

The modified particles can be administered by aerosol. This is accomplished by preparing an aqueous aerosol, liposomal preparation or solid particles containing the modified particles. A nonaqueous (e.g., fluorocarbon propellant) suspension could be used.

Ordinarily, an aqueous aerosol is made by formulating an aqueous solution or suspension of the agent together with conventional pharmaceutically acceptable carriers and stabilizers. The carriers and stabilizers vary with the requirements of the particular compound, but typically include nonionic surfactants (Tweens, Pluronics^{®}, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

It will also be appreciated that the modified particles and pharmaceutical compositions of the present invention can be formulated and employed in combination therapies, that is, the compounds and pharmaceutical compositions can be formulated with or administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another anti-inflammatory agent), or they may achieve different effects (e.g., control of any adverse effects).

In certain embodiments, the pharmaceutical compositions containing the modified particles of the present invention further comprise one or more additional therapeutically active ingredients (e.g., anti-inflammatory and/or palliative). For purposes of the invention, the term "Palliative" refers to treatment that is focused on the relief of symptoms of a disease and/or side effects of a therapeutic regimen, but is not curative. For example, palliative treatment encompasses painkillers, antinausea medications and anti-sickness drugs.

The disclosure provides methods of regulating an immune response in an individual, preferably a mammal, more preferably a human, comprising administering to the individual the modified particles described herein. Methods of immunoregulation provided by the disclosure include those that suppress and/or inhibit an innate immune response or an adaptive immune response, including, but not limited to, an immune response stimulated by immunostimulatory polypeptides or viral or bacterial components.

The modified particles are administered in an amount sufficient to regulate an immune response. As described herein, regulation of an immune response may be humoral and/or cellular, and is measured using standard techniques in the art and as described herein.

In some embodiments, compositions described herein are administered along with (e.g., concurrent with, prior to, or following) an implant (e.g., device) and/or transplant (e.g., tissue, cells, organ) to mediate, negate, regulate and/or reduce the immune response associated therewith.

In certain instances of the disclosure, the individual suffers from a disorder associated with unwanted immune activation, such as allergic disease or condition, allergy and asthma. An individual having an allergic disease or asthma is an individual with a recognizable symptom of an existing allergic disease or asthma. Tolerance can be induced in such an individual, for example, by particles complexed with the specific foods (e.g. peanut proteins, etc.), injected substances (e.g. bee venom proteins, etc.), or inhaled substances (e.g. ragweed pollen proteins, pet dander proteins, etc.) which elicit the allergic reaction.

In certain embodiments, the individual suffers from a disorder associated with unwanted immune activation, such as autoimmune disease and inflammatory disease. An individual having an autoimmune disease or inflammatory disease is an individual with a recognizable symptom of an existing autoimmune disease or inflammatory disease. Tolerance can be induced in such an individual, for example, by particles complexed with the relevant autoantigens driving the particular autoimmune disease.

In certain instances of the disclosure, the individual suffers from a disorder associated with enzyme replacement therapy. Tolerance can be induced in such an individual, for example, by particles complexed with the enzymes which patients with genetic deficiencies fail to produce, to prevent them from making neutralizing antibody responses to recombinantly-produced enzymes administered to treat their particular deficiency, e.g. tolerance to human Factor VIII in patients with hemophilia due to a genetic deficiency in the ability to make Factor VIII. Another example may include enzyme replacement in for conditions such as mucopolysaccharide storage disorder, gangliosidosis, alkaline hypophosphatasia, cholesterol ester storage disease, hyperuricemia, growth hormone deficiency, renal anemia or with lysomal storage disorders including Fabry's disease, Gaucher's disease, Hurler's disease, Hunter's syndrome, Maroteaux-Lamy disease, Niemann-Pick disease, Tay-Sachs disease, and Pompe disease.

In certain instances of the disclosure, the individual suffers from a robust, or otherwise adverse, immune response towards an agent administered for the treatment of a disease that actually or potentially compromises patient health or treatment. Additionally, novel compounds provided by this invention may be provided to individuals who do not show an immune response to an agent but may potentially do so in the future. In certain instances of the disclosure, the individual is receiving enzyme replacement therapy. In certain instances of the disclosure , therapeutic agents include, but are not limited to, peptides or protein-based agents, DNA vaccines, siRNA, splice-site switching oligomers, and RNA-based nanoparticles. In some instances of the disclosure, the therapeutic agents include, but are not limited to, Advate, antihemophilic factor, Kogenate, Eloctate, recombinant factor VIII Fc fusion protein, Refacto, Novo VIIa, recombinant factor VII, eptacog alfa, Helixate, Monanine, Coagulation Factor IX, Wilate, Ceredase, Alglucerase, Cerezyme, Imiglucerase, Elelso, taliglucerase alfa, Fabrazyme, Agalsidase beta, Aldurazyme, -I-iduronidase, Myozyme, Acid-glucosidase, Elaprase, iduronate-2-sulfatase, Naglazyme arylsufatase B, and N-acetylgalactosamin e-4-sulfatase. In some instances of the disclosure, the individual is administered therapeutic agents administered to treat diseases including, but not limited to, Hemophilia, Hemophilia A, Hemophilia B, von Willebrand disease, Gaucher's Disease, Fabry's Disease, Hurler's Disease, Pompe's Disease, Hunter's Disease, mucopolysaccharide storage disorder, gangliosidosis, alkaline hypophosphatasia, cholesterol ester storage disease, hyperuricemia, growth hormone deficiency, renal anemia and Maroteaux-Lary Disease.

In certain instances of the disclosure, the individual suffers from an orphan autoimmune condition. Such conditions include, but are not limited to, idiopathic thrombocytopenic purpura, membranous nephropathy, bullous pemphigoid, pemphigus vulgaris, and Myasthenia Gravis.

In certain instances of the disclosure, the individual suffers from a disorder associated with disease therapy. In the case of recombinant antibodies, tolerance is induced for example, to a humanized antibody being employed in a therapeutic context to prevent a patient from making neutralizing antibodies against the antibody therapeutic, e.g. tolerance to a humanized immune subset depleting antibody or anti-cytokine antibody being used as a treatment for autoimmune disease.

Autoimmune diseases can be divided in two broad categories: organ-specific and systemic. Autoimmune diseases include, without limitation, rheumatoid arthritis (RA), systemic lupus erythematosus (SLE), type I diabetes mellitus, type II diabetes mellitus, multiple sclerosis (MS), immune- mediated infertility such as premature ovarian failure, scleroderma, Sjogren's disease, vitiligo, alopecia (baldness), polyglandular failure, Grave's disease, hypothyroidism, polymyositis, pemphigus vulgaris, pemphigus foliaceus, inflammatory bowel disease including Crohn's disease and ulcerative colitis, autoimmune hepatitis including that associated with hepatitis B virus (HBV) and hepatitis C virus (HCV), hypopituitarism, graft-versus-host disease (GvHD), myocarditis, Addison's disease, autoimmune skin diseases, uveitis, pernicious anemia, Celiac disease, hypoparathyroidism neuomyelitis optica, membraneous nephropathy, bullous pemphigoid, pemphigus vulgaris, myasthenia gravis .

Autoimmune diseases may also include, without limitation, Hashimoto's thyroiditis, Type I and Type II autoimmune polyglandular syndromes, paraneoplastic pemphigus, bullus pemphigoid, dermatitis herpetiformis, linear IgA disease, epidermolysis bullosa acquisita, erythema nodosa, pemphigoid gestationis, cicatricial pemphigoid, mixed essential cryoglobulinemia, chronic bullous disease of childhood, hemolytic anemia, thrombocytopenic purpura, Goodpasture's syndrome, autoimmune neutropenia, myasthenia gravis, Eaton-Lambert myasthenic syndrome, stiff-man syndrome, acute disseminated encephalomyelitis, Guillain-Barre syndrome, chronic inflammatory demyelinating polyradiculoneuropathy, multifocal motor neuropathy with conduction block, chronic neuropathy with monoclonal gammopathy, opsonoclonus-myoclonus syndrome, cerebellar degeneration, encephalomyelitis, retinopathy, primary biliary sclerosis, sclerosing cholangitis, gluten-sensitive enteropathy, ankylosing spondylitis, reactive arthritides, polymyositis/dermatomyositis, mixed connective tissue disease, Bechet's syndrome, psoriasis, polyarteritis nodosa, allergic anguitis and granulomatosis (Churg-Strauss disease), polyangiitis overlap syndrome, hypersensitivity vasculitis, Wegener's granulomatosis, temporal arteritis, Takayasu's arteritis, Kawasaki's disease, isolated vasculitis of the central nervous system, thromboangiutis obliterans, sarcoidosis, glomerulonephritis, and cryopathies. These conditions are well known in the medical arts and are described, for example, in Harrison's Principles of Internal Medicine, 14th ed., Fauci A S et al., eds., New York: McGraw-Hill, 1998.

Animal models for the study of autoimmune disease are known in the art. For example, animal models which appear most similar to human autoimmune disease include animal strains which spontaneously develop a high incidence of the particular disease. Examples of such models include, but are not limited to, the nonobese diabetic (NOD) mouse, which develops a disease similar to type 1 diabetes, and lupus-like disease prone animals, such as New Zealand hybrid, MRL-Fas^{lpr} and BXSB mice. Animal models in which an autoimmune disease has been induced include, but are not limited to, experimental autoimmune encephalomyelitis (EAE), which is a model for multiple sclerosis, collagen-induced arthritis (CIA), which is a model for rheumatoid arthritis, Desmoglein 3 transgenic T cell mouse, which can be used as an experimental model of Pemphigus Vulgaris and experimental autoimmune uveitis (EAU), which is a model for uveitis. Animal models for autoimmune disease have also been created by genetic manipulation and include, for example, IL-2/IL-10 knockout mice for inflammatory bowel disease, Fas or Fas ligand knockout for SLE, and IL-I receptor antagonist knockout for rheumatoid arthritis.

In certain instances of the disclosure, the individual suffers from a bacterial or viral infection. An individual having a bacterial or viral infection is an individual with a recognizable symptom of an existing bacterial or viral infection.

A non-limiting list of viral infections treatable with the modified particles of the current disclosure includes herpes virus infections, hepatitis virus infections, west nile virus infections, flavivrus infections, influenza virus infections, rhinovirus infections, papillomavirus infections, paromyxovirus infections, parainfluenza virus infections, and retrovirus infections. Preferred viruses are those viruses that infect the central nervous system of the subject. Most preferred viruses are those that cause hemorrgic fever, encephalitis or meningitis.

A non-limiting list of bacterial infections treatable with the modified particles of the current disclosure include staphlococcus infections, streptococcus infections, mycobacterial infections, bacillus infections, Salmonella infections, Vibrio infections, spirochete infections, and Neisseria infections. Preferred are bacteria that infect the central nervous system of the subject. Most preferred are bacteria that cause encephalitis or meningitis.

In some embodiments, the invention relates to uses of compositions of this invention prior to the onset of disease. In other embodiments, the invention relates to uses of the compositions of this invention to inhibit ongoing disease. In some embodiments, the invention relates to ameliorating disease in a subject. By ameliorating disease in a subject is meant to include treating, preventing or suppressing the disease in the subject.

In some embodiments, the invention relates to preventing the relapse of disease. For example, an unwanted immune response can occur at one region of a peptide (such as an antigenic determinant). Relapse of a disease associated with an unwanted immune response can occur by having an immune response attack at a different region of the peptide. Since the immune modifying particles of the current invention are free from attached peptides or antigenic moieties, the particles will be effective against multiple epitopes. T-cell responses in some immune response disorders, including MS and other ThI /17-mediated autoimmune diseases, can be dynamic and evolve during the course of relapsing-remitting and/or chronic-progressive disease. The dynamic nature of the T- cell repertoire has implications for treatment of certain diseases, since the target may change as the disease progresses. Previously, pre-existing knowledge of the pattern of responses was necessary to predict the progression of disease. The present invention provides compositions that can prevent the effect of dynamic changing disease, a function of "epitope spreading." A known model for relapse is an immune reaction to proteolipid protein (PLP) as a model for multiple sclerosis (MS). Initial immune response can occur by a response to PLP139-15,. Subsequent disease onset can occur by a relapse immune response to PLP[pi]s-iβi.

Other instances of the disclosure relate to transplantation. This refers to the transfer of a tissue sample or graft from a donor individual to a recipient individual, and is frequently performed on human recipients who need the tissue in order to restore a physiological function provided by the tissue. Tissues that are transplanted include (but are not limited to) whole organs such as kidney, liver, heart, lung; organ components such as skin grafts and the cornea of the eye; and cell suspensions such as bone marrow cells and cultures of cells selected and expanded from bone marrow or circulating blood, and whole blood transfusions.

A serious potential complication of any transplantation ensues from antigenic differences between the host recipient and the engrafted tissue. Depending on the nature and degree of the difference, there may be a risk of an immunological assault of the graft by the host, or of the host by the graft, or both, may occur. The extent of the risk is determined by following the response pattern in a population of similarly treated subjects with a similar phenotype, and correlating the various possible contributing factors according to well accepted clinical procedures. The immunological assault may be the result of a preexisting immunological response (such as preformed antibody), or one that is initiated about the time of transplantation (such as the generation of Th cells). Antibody, Th cells, or Tc cells may be involved in any combination with each other and with various effector molecules and cells. However, the antigens which are involved in the immune response are generally not known, therefore posing difficulties in designing antigen-specific therapies or inducing antigen-specific tolerance.

Certain instances of the disclosure relate to decreasing the risk of host versus graft disease, leading to rejection of the tissue graft by the recipient. The treatment may be performed to prevent or reduce the effect of a hyperacute, acute, or chronic rejection response. Treatment is preferentially initiated sufficiently far in advance of the transplant so that tolerance will be in place when the graft is installed; but where this is not possible, treatment can be initiated simultaneously with or following the transplant. Regardless of the time of initiation, treatment will generally continue at regular intervals for at least the first month following transplant. Follow-up doses may not be required if a sufficient accommodation of the graft occurs, but can be resumed if there is any evidence of rejection or inflammation of the graft. Of course, the tolerization procedures of this disclosure may be combined with other forms of immunosuppression to achieve an even lower level of risk.

Certain instances of the disclosure relate to decreasing or otherwise ameliorating the inflammatory response induced as a response to surgery. In one instance of the disclosure, the immune-modifying particles are administered before surgery. In a further instance of the disclosure, the immune-modifying particles are administered concurrently with or during surgery. In yet a further instance of the disclosure, the immune-modifying particles are administered after surgery.

The particles of the disclosure may also be used to treat abscesses or empyemas to decrease the inflammatory response produced in the subject after exposure to infectious agents such as bacteria or parasites. In one instance of the disclosure, the immune-modifying particles are administered in conjunction with anti-bacterial and/or anti-parasitic treatments known in the art.

The particles of the disclosure may also be used to decrease or otherwise ameliorate the inflammatory response induced as a response to physical trauma or injury including, but not limited to, a sports injury, a wound, a spinal cord injury, a brain injury, and/or a soft tissue injury. In one instance of the disclosure, the immune-modifying particles are administered after the subject experiences trauma or injury.

The particles of the disclosure may also be used to decrease the inflammatory response associated with the development and/or growth of cancer cells. Cancers that can be treated include, but are not limited to, central nervous system cancer, basal cell carcinoma, cancerous brain tumors, Burkitt's lymphoma, lymphoma, cervical cancer, ovarian cancer, testicular cancer, liver cancer, non-small cell and small cell lung cancers, melanoma, bladder cancer, breast cancer, colon and rectal cancers, endometrial cancer, kidney (renal cell) cancer, leukemia, Non-Hodgkin lymphoma, pancreatic cancer, prostate cancer, melanoma, and thyroid cancer. In one instances of the disclosure, the subcutaneous injection of the particles of the disclosure prevents the accumulation of inhibitory neutrophils, thereby decreasing inflammation in the cancer patient.

The particles of the disclosure are also useful for the regeneration of damaged tissue. In one instance of the disclosure, administration of the particles to a patient increases the regeneration of damaged epithelial cells in the digestive tract. In a further instance of the disclosure, the patient suffers from colitis, Crohn's disease, or inflammatory bowel disease. In another instances of the disclosure, administration of the particles of the disclosure to a patient increases remyelination of neurons. In a further instances of the disclosure, the patient suffers from multiple sclerosis.

In some embodiments, compositions of the present invention (e.g., PLG carrier coupled to antigenic molecule) find use with one or more scaffolds, matrices, and/or delivery systems (See, e.g., U.S. Pat. App. 2009/0238879; U.S. Pat. No. 7,846,466; U.S. Pat. No. 7,427,602; U.S. Pat. No. 7,029,697; U.S. Pat. No. 6,890,556; U.S. Pat. No.6,797,738; U.S. Pat. No. 6,281,256. In some embodiments, particles (e.g., antigen-coupled PLG particles) are associated with, adsorbed on, embedded within, conjugated to, etc. a scaffold, matrix, and/or delivery system (e.g., for delivery of chemical/biological material, cells, tissue, and/or an organ to a subject). In some embodiments, a scaffold, matrix, and/or delivery system (e.g., for delivery of chemical/biological material, cells, tissue, and/or an organ to a subject) comprises and/or is made from materials described herein (e.g., PLG conjugated to one or more antigenic peptides).

In some embodiments, microporous scaffolds (e.g., for transplanting biological material (e.g., cells, tissue, etc.) into a subject) are provided. In some embodiments, microporous scaffolds are provided having thereon agents (e.g., extracellular matrix proteins, exendin-4) and biological material (e.g., pancreatic islet cells). In some embodiments, the scaffolds are used in the treatment of diseases (e.g., type 1 diabetes), and related methods (e.g., diagnostic methods, research methods, drug screening). In some embodiments, scaffolds are provided with the antigen-conjugated carriers described herein on and/or within the scaffold. In some embodiments, scaffolds are produced from antigen conjugated materials (e.g., antigen conjugated PLG).

In some embodiments, a scaffold and/or delivery system comprises one or more layers and/or has one or more chemical and/or biological entities/agents (e.g., proteins, peptide-conjugated particles, small molecules, cells, tissue, etc.), see, e.g., U.S. Pat. App. 2009/0238879. In some embodiments, antigen-coupled particles are co-administered with a scaffold delivery system to elicit induction of immunological tolerance to the scaffold and the associated materials. In some embodiments, microporous scaffold is administered to a subject with particles described herein on or within the scaffold. In some embodiments, antigen-coupled particles coupled to a scaffold delivery system. In some embodiments, a scaffold delivery system comprises antigen-coupled particles.

Although specific embodiments have been described, it should be understood that the invention as claimed should not be unduly limited to such specific embodiments. For example, U.S. Pat. Applications 2012/0076831, 2002/0045672, 2005/0090008, 2006/0002978, and 2009/0238879 and U.S. Pat. Nos. 7,846,466; 7,427,602; 7,029,697; 6,890,556;.6,797,738; and 6,281,256 provide details, modifications, and variations that find use in various embodiments described herein. The invention is defined by the scope of the claims.

### EXAMPLES

The following examples are provided to further illustrate the advantages and features of the disclosure and invention, but are not intended to limit the scope of this disclosure. Examples not relating to gliadin particles are provided for reference.

### Materials and Methods

### Generation of chimeric mice

Six- to eight-week old B6.SJL-Ptprc^{a}Pep3^{b}/BoyJ (CD45.1) mice were irradiated with one dose of 950 rads. Twelve hours later, mice were reconstituted with 10⁷ bone marrow cells from C57BL/6-7.2fms-EGFP donors. Mice were given sulfamethoxazole (Sigma Aldrich) and trimethoprim (Sigma Aldrich) in the drinking water for 10 days following irradiation. Mice were infected with WNV six weeks after irradiation, as described above. Chimerism was checked using flow cytometry and was invariably found to be 96-99% of donor origin as previously demonstrated (Getts et al., J Neurochem. 103: 1019, 2007).

### Immunohistology

Mice were anesthetized and perfused with 50mL sterile PBS. With the exception of the heart, which were processed into paraffin blocks (Getts et al., J. Neurochem 103:10919-1030, 2007), all organs were isolated and snap frozen in Optimum Cutting Temperature Compound (OCT; Tissue-Tek, Tokyo, Japan). Eight-micron tissue sections were cut on a cryostat microtome, air-dried overnight and then stored at -80°C until required. Frozen sections were thawed and histology (standard haematoxylin and eosin staining) or immunohistochemistry was performed (Getts et al., J. Exp Med 205:2319-2337, 2008). Antibodies against MARCO, SIGN-R1 and SIGLEC-1 (R&D Systems, MN, USA), CD68 (Abcam, MA, USA) and Ki67 (Abeam), were used as indicated. Images were acquired on an Olympus BX-51 microscope using a DP-70 camera and DP manager 2.2.1 software (Olympus, Tokyo, Japan).

### Microscope and image acquisition

Images were acquired on an Olympus BX-51 microscope (Olympus, Japan), using a DP-70 camera and DP manager 2.2.1 software (Olympus).

### Isolation of leukocytes from the brain and liver

As previously described (Getts et al, J Exp Med. 29: 2319, 2007) leukocytes were obtained from the brains of PBS-perfused mice by digesting brains for 60 minutes at 37°C in PBS with deoxy-ribonuclease (0.005 g/ml; Sigma Aldrich) and collagenase IV (0.05 g/ml; Sigma Aldrich). Digestion was stopped with 10% FCS, and the homogenate was passed through a 70µm nylon cell strainer (Becton Dickinson, NJ, USA). The pellet, obtained after 10 minutes centrifugation at 340xg, was resuspended in 30% Percoll (Amersham, Norway) and layered over 80% Percoll. Leukocytes were collected from the 30%/80% interface after centrifugation at 1140xg for 25 minutes at room temperature. The same protocol is also used to derive leukocytes from the liver, with the tissue weighed before processing.

### Isolation of leukocytes from the spleen, blood and bone marrow

For flow cytometric analysis, the right femur was dissected out and bone marrow cells flushed out using PBS loaded syringes. For bone marrow precursor isolation, femurs and tibias from at least 4 mice were utilized. The cellular suspension achieved after flushing was filtered through a 70µm cell strainer and centrifuged for 5 mins at 340g. Red blood cells in the resulting pellet were lysed in NH₄Cl-based red cell lysis buffer (BD Pharm Lyse^{™}; BD Pharmingen), before centrifugation for 5 mins at 340xg. In the case of peripheral blood, blood was collected via cardiac puncture and immediately transferred into citrate buffer (mMol, Sigma Alrich). The resulting suspension was layered over 70% Percoll and centrifuged at 1140xg for 20 minutes at room temperature with the brake off. The interface was collected and the cells washed once in PBS, centrifuged at 340xg. For the isolation of splenic leukocytes, spleens were passed through a 7070µm cell strainer and centrifuged for 5 mins at 340g. Red blood cells in the resulting pellet were lysed in NH₄Cl-based red cell lysis buffer (BD Pharm Lyse^{™}; BD Pharmingen), before centrifugation for 5 mins at 340xg.

### Flow cytometry

Cells collected (as described above) from the brain, liver, blood, and bone marrow were washed in PBS, and blocked with anti-CD16/CD32 antibody (Biolegend). Viable cells were counted using trypan blue exclusion, which routinely showed >95% cell viability.

Cell surface molecule expression was measured and cell sorts carried out on a FACS ARIA (Becton Dickinson), equipped with an Argon ion and HeNe laser. Viable populations were gated by forward and side scatter and identified fluorescent populations determined by forward-gating thereafter. Sorting was carried out using specific fluorescent and scatter parameters identifying the population of interest. Sorting stringencies was set to purity to achieve >98% purity for bone marrow populations.

Acquired FACS data files were analysed using the flow cytometry program, Flow Jo (FlowJo, Ashland, OR, USA). Quantification of cell populations of interest were calculated based on flow cytometry percentages at analysis and absolute cell counts from each organ.

### Adoptive transfer

Experiments were conducted during development of embodiments of the present invention to investigate a second model of active disease termed adoptive transfer. Rather than immunizing the animals with the peptide, the lymphocytes from the spleen of mice with active disease were transferred to a recipient, who would subsequently develop disease. Experiments were conducted during development of embodiments of the present invention to characterize the ability of the PLG nanoparticles to deactivate the adoptively transferred activated effector cells. Mice treated with particles or splenocytes coupled with a control peptide had an increase in clinical score beginning at day 4. Mice treated with PLG-PLP₁₃₉₋₁₅₁ particles at day 2 had a mean clinical score of 0 for all but two time points through day 40, and the mean clinical score for those other time points was 0.25.

### Multiplex ELISA

Multiplexed plate ELISAs were performed according to the manufacturers instructions (Quansys Biosciences, Logan, Utah, USA). Briefly, brain, spleen, and liver tissue were homogenized in PBS, clarified by a 1000xg spin, and stored at -20°C until the assay was performed. Serum samples were also used. Thawed samples and standards were diluted in the provided buffer, and 30µl of each were plated in each well that contains 16 spots each containing a capture antibody for a particular soluble protein. Plates were then incubated for 1 hour on an orbital shaker at 120 r.p.m. Plates were washed 3 times, and 30µl of detection antibody was added to each well and incubated for another hour. After washing 3 times, strepavidin-HRP was added and incubated for a further 15 minutes. Plates were then washed 6 times, and substrate mix was added. Plates were immediately read on a CCD imager (Kodak, Rochester NY, USA). Plate images were analysed using Quansys Q-view software (Quansys Biosciences).

### Induction and evaluation of Experimental Autoimmune Encephalitis (EAE)

Mice were injected sub-cutaneously with emulsion containing 0.1mg MOG Peptide (MEVGWYRSPFSRVVHLYRNGK (SEQ ID NO:1); Auspep, Parkville, Victoria, Australia; >95% HPLC purified) and Complete Freund's adjuvant containing 2mg/mL Mycobacterium tuberculosis (Sigma Aldrich). Two days later, mice were administered 500µl Pertussis toxin (Sigma Aldrich) i.p. Mice were monitored for disease progression, and graded on the following scale: 1, limp tail and/or weakness of 1 hind limb; 2, weakness in more than one limb, gait disturbance; 3, paralysis in 1 limb; 4, paralysis in more than one limb, incontinence; 5, moribund.

### Statistics

Graphs were made and computerized statistical analysis was performed in GraphPad Prism, and InStat, respectively (both programs from GraphPad software, San Diego, CA, USA). Depending on the data, an unpaired, two-tailed Student t-test, or one way ANOVA with a Tukey-Kramer post test was performed, with P < 0.05 considered to be significant.

For correlation analysis between parameters such as weight loss, infiltration, and virus titre, a non-linear regression (curve fit) was used, with a second order polynomial (Y=A + B^{∗}X + C^{∗}X^2).

### Example 1

### Preparation of Negatively Charged Immune Modifying Particles (IMPs)

To a solution of Poly(ethylene-maleic anhydride) (PEMA) in D₂O (4 mL, 1% w/v) was added dropwise a solution of poly(lactide-co-glycolic acid) (PLG) in dichloromethane (DCM) (2 mL, 20% w/v). The mixture was allowed to sonicate on ice at 16 watts for 30 sec using the VC 30 Ultrasonic Processor. The resulting homogenized crude was then poured into a solution of D₂O (200 mL containing 0.5% w/v of PEMA). The homogenized slurry was allowed to stir overnight at speed setting of 3.5 using Bellco Glass, Inc., Bellstir Multi-stir 9 magnetic stirrer (10 W for 10 s, 16 W for 10 s, 16 W for 30 s).

### Results

After three hours of stirring, particle size analyses were performed using dynamic light scattering in disposable polystyrene cuvettes
a. 10 W, 10 s - Z-average = 499.9 nm - PdI = 0.23, Peak = 634.5 nm
b. 16 W, 10 s - Z-average = 528.9 nm - PdI = 0.227, Peak = 657.5 nm
c. 16 W, 30 s - Z-average = 471.6 nm - PdI = 0.228, Peak = 580.5 nm
d. 16 W, 60 s - Z-average = 491.1 nm - PdI = 0.275, Peak = 600.8 nm

After the reaction was complete, the resulting crude suspension was then purified.

### Purification

Fresh D₂O and 10x sodium bicarbonate buffer were chilled overnight to 4°C. Using a 40 µm cell strainer, 36 mL of particle suspension were filtered from each batch into an appropriately-labelled 50 mL centrifuge tube containing 4 mL chilled 10x sodium bicarbonate buffer. Each beaker produced approximately 6 such tubes. All tubes were centrifuged for about 15 minutes at 7000g at 4°C and the supernatant was aspirated. Preparation of the suspension was repeated using the above-mentioned procedure and much of the particle pellets were suspended as possible in 1 mL chilled D₂O.

The resuspended particles were transferred into a fresh tube with 4 mL of chilled 10x sodium bicarbonate buffer. (Step 1)

Resuspension of the particle was repeated until the entire particle pellets haves been successfully resuspended. (Step 2)

The 6 centrifugal tubes were then combined into one centrifuge tube (50 mL tube) and the tube was filled with the remaining volume to 40 mL of chilled D₂O (Wash 1).

The tube was centrifuged for 20 minutes at 7000g at 4°C and the supernatant was aspirated.

Step 1 and 2 and Wash 1 of the resulting particle were repeated each time at least two more times. Finally, the resulting particle pellets were then subjected to a flash-freeze in liquid nitrogen and lyophilized to dryness in the manifold to obtain negatively IMPs.

Figure 1 shows characterization of surface-functionalized poly(lactide-co-glycolide) particles by dynamic light scattering analysis. Surface-functionalized poly(lactide-co-glycolide) particles were analysed on a Malvern Zetasizer Nano ZS (Malvern Instruments, Westborough, MA) at a count rate of 2.5 x 10⁵ counts per second in 18.2 MΩ water. The population of surface-functionalized poly(lactide-co-glycolide) particles had a Z-average diameter of 567 nm, a peak diameter of 670 nm and a polydispersity index of 0.209.

Table 4 shows the measurements for surface functionalized PLG-PEMA particles. The data in the table is representative, as each batch is slightly different. The numbers in the table were based on combining several batches of particles though. The measurements for the double emulsion particles are similar to those in Table 2.

**Table 4 -Measurements for the surface functionalized PLG-PEMA particles**

| Particle | Z-average size by intensity (nm) | ζ-potential (mV) |
|---|---|---|
| PLG | (Phosphorex) 624.3 | -32.7 ± 4.71 |
| PLG-PEMA | 429.9 | -67.4 ± 10.9 |

### Example 2

### Administration of antigen-coupled PLGA beads prevents Relapsing Experimental Autoimmune Encephalitis

PLG nanoparticles were investigated with the immunodominant proteolipid protein PLP₁₃₉₋₁₅₁ epitope (PLG-PLP₁₃₉₋₁₅₁) to induce tolerance for prevention of Relapsing Experimental Autoimmune Encephalitis (R-EAE). The R-EAE mice were generated as described above.

The peptides administered to the animals were coupled to particles with the mean diameter of 500 nm. Mice were treated with either PLP₁₃₉₋₁₅₁-PLGA (N=5), OVA₃₂₃₋₃₃₉-PLGA (N=5), or uncongugated PLGA (N=5) on day -7 relative to the time of immunization (day 0). Peak disease was typically observed around day 12 to 14, and mice are scored for clinical disease. Particles without peptide, or modified with the control peptide OVA₃₂₃₋₃₃₉ did not prevent disease induction. However, PLGA particles modified with PLP₁₃₉₋₁₅₁ produced a clinical score of 0 (no disease) at all except low clinical scores of under 1 exhibited between days 20 and 30 (Figure 2). Previous studies with unmodified PLG or using polystyrene particles did not produce this effective disease reduction, with polystyrene bound particles commonly triggering anaphylaxis.

Furthermore, specific inactivation of myelin-specific CD4⁺ T cells was demonstrated by lack of delayed-type hypersensitivity (DTH) responses to both immunizing PLP₁₃₉₋₁₅₁ epitope. Taken together, prophylactic treatment with PLG-PLP₁₃₉₋₁₅₁ on day-7 specifically prevented EAE development, and represents an improvement in the ability of particles to prevent disease. The scores produced with the particles are as good as, and perhaps better, than the scores produced with antigen-coupled splenocytes.

The type of particle administered also has an effect on the development of EAE in the mouse model. Mice were treated with either OVA₃₂₃₋₃₃₉-PLS (N=5), OVA₃₂₃₋₃₃₉-PLGA_{PHOSPOREX} (N=5), OVA₃₂₃₋₃₃₉-PLGA_{PEMA} (N=5), PLP₁₃₉₋₁₅₁-PLA (N=5), PLP₁₃₉₋₁₅₁-PLGA_{PHOSPOREX} (N=5), or PLP₁₃₉₋₁₅₁-PLG_{PEMA} (N=5) on day -7 relative to the time of immunization (day 0). Peak disease was typically observed around day 12 to 14, and mice are scored for clinical disease. Particles, of any composition that were modified with the control peptide OVA₃₂₃₋₃₃₉ did not prevent disease induction. However, the PLP₁₃₉₋₁₅₁ coupled PLG beads were more effective in down-regulating induction of R-EAE than PLP₁₃₉₋₁₅₁ coupled commercial (Phosphorex) pLG or polystyrene (Figures 3A and 3B).

### Example 3

### Intravenous infusion of antigen coupled PLG particles does not induce anaphylaxis-induced temperature drop in OVA/Alum pre-sensitized animals

Due to the presence of active disease, anaphylaxis to the antigens is a concern, which could result in immediate mortality, and has been described with polystyrene bound particles. Anaphylaxis is associated with a significant drop in body temperature. To test whether intravenous administration of OVA-PLG induces an anaphylaxis-induced temperature drop in pre-sensitized animals, mice were immunized at day 0 with 10µg OVA/Alum via intraperitoneal injection. On day 14, the mice were again immunized with 10µg OVA/Alum via intraperitoneal injection, and then tolerized with OVA-PLG administered intravenously on day 21. On day 28, the mice were then tolerized with either OVA-PLG particles or OVA via intravenous administration.

As shown in Figure 4 those mice treated with soluble OVA on day 28 exhibited decrease in temperature compared with those animals treated with the OVA-PLG particle. No decrease in body temperature was observed within 1 hour of delivering the particles.

Figure 5 shows that administration of PLP-PLG during remission does not result in any anaphylaxis-associated mortality. EAE was induced in six to eight week old female SJL/J mice by subcutaneous injection of PLP₁₃₉₋₁₅₁ in CFA, and development of clinical disease was monitored and recorded (Figure 5B). On day 21 relative to disease induction, mice were given iv injections of soluble PLP₁₃₉₋₁₅₁ (clear squares), soluble OVA₃₂₃₋₃₃₉ (clear circles), or the same peptides coupled to PLG nanoparticles (solids). Temperature of animals was monitored and recorded every 10 minutes for 1 hour following injection (Figure 5A).

### Example 4

### Prophylactic treatment with PLP-PLG particles induces long-term, antigen-specific tolerance

Optimal dosing was determined by intravenous administration of increasing concentrations of PLP₁₃₉₋₁₅₁-PLG seven days prior to disease induction, and monitored for development of clinical disease in comparison to SJL/J mice treated with OVA₃₂₃₋₃₃₉ -PLG (Figure 6A). Six to eight week old female SJL/J mice were injected iv with either PLP₁₃₉₋₁₅₁ (square)- or OVA₃₂₃₋₃₃₉ (circle)- coupled PLG nanoparticles. EAE was induced by subcutaneous injection of PLP₁₃₉₋₁₅₁ in CFA 7 days (Figure 6B), 25 days (Figure 6C), or 50 days (Figure 6D) later. Animals from panel B were followed for clinical disease for 100 days. Figure 6E shows that on day 8 relative to disease induction, a delayed-type hypersensitivity (DTH) reaction was carried out in a subset of the mice shown in panel B. Selected representative animals from the PLP₁₃₉₋₁₅₁/CFA primed groups in panel B (OVA₃₂₃₋₃₃₉-PLG and PLP₁₃₉₋₁₅₁-PLG) were ear-challenged with the priming PLP₁₃₉₋₁₅₁ epitope and the OVA₃₂₃₋₃₃₉ control peptide. Ear swelling as a measure of DTH was determined 24h later and responses prior to challenge were subtracted. Figure 6F shows that six to eight-week old female SJL/J mice were injected intravenously with PLP₁₇₈₋₁₉₁ (triangle)-, OVA₃₂₃₋₃₃₉ (circle), or PLP₁₃₉₋₁₅₁ (square)-coupled PLG nanoparticles, or with uncoupled particles alone (outlined circle). EAE was induced 7 days afterward by subcutaneous injection of PLP₁₇₈₋₁₉₁ in CFA, and disease was monitored at the time points shown.

### Example 5

### Treatment of Relapsing Experimental Autoimmune Encephalitis with antigen-coupled particles

Experiments were conducted during development of embodiments of the present invention to investigate the ability of the PLG-PLP₁₃₉₋₁₅₁ particles to *treat* disease rather than prevent disease, and to determine whether the route of administration affected the development of disease. Mice were immunized at day 0 with PLP₁₃₉₋₁₅₁ and an adjuvant. Mice normally have maximal clinical scores at day 12-14. In this model, the mice were treated at day 10 with the PLG-PLP₁₃₉₋₁₅₁ particles or with control PLG-OVA₃₂₃₋₃₃₉ particles either via intravenous (iv) administration, intraperitoneal (ip) administration, subcutaneous (sc) administration, or orally. As shown in Figure 7, prophylactic tolerance is most efficient when the PLG-PLP₁₃₉₋₁₅₁ particles are administered either intravenously or intraperitoneally. Animals treated with PLP₁₃₉₋₁₅₁-PLG administered intravenously did not develop disease and had mean clinical scores of 0 at most time points. This is in contrast to animals treated with PLP₁₃₉₋₁₅₁ polystrene particles, whereby >70% of animals where observed to die from anaphylaxis.

### Example 6

### Antigen-coupled particle tolerance inhibits induction of antigen-specific Th1 and Th17 responses in active Relapsing Experimental Autoimmune Encephalitis

To determine whether administration of antigen-coupled particles inhibit induction of T-helper cells, either MOG₃₅₋₅₅₋PLG or OVA₃₂₃₋₃₃₉-PLG particles were administered intravenously to BALB/c mice at Day -7. On Day 0, OVA₃₂₃₋₃₃₉-PLG particles and Complete Freund's Adjuvant (CFA) were administered subcutaneously to the mice. The animals were re-stimulated with either MOG₃₅₋₅₅₋PLG or OVA₃₂₃₋₃₃₉-PLG particles on Day 10 and the draining lymph node cells were isolated. The CPM and levels of IL-17, GM-CSF, IFN-γ, IL-10, and IL-4 were measured at Day 10. As shown in Figure 8, the administration of OVA₃₂₃₋₃₃₉-PLG particles inhibited the Th1 and Th17 responses in the treated animals.

### Example 7

### Tolerance is induced by PLP-₁₃₉₋₁₅₁ coupled PLGA particles

An additional therapeutic tolerance strategy was performed by delivering PLP₁₃₉₋₁₅₁-PLG or OVA₃₂₃₋₃₃₉ PLG to mice. Histological analysis showed that the administration of the PLP₁₃₉₋₁₅₁-PLG particles inhibits cervical spinal cord inflammation and demyelination. Mice were treated with PLP-PLG or OVA₃₂₃₋₃₃₉-PLG and the tissue was retrieved at day 40. The cervical spinal cord was isolated and sectioned to investigate the immune response within the CNS, which underlies the pathology of R-EAE and multiple sclerosis. Figure 9 shows a reduction in immune cell infiltration within the spinal cord of animals treated with PLP₁₃₉₋₁₅₁-PLG that and was more similar to native tissue than to tissue from OVA₃₂₃₋₃₃₉-PLG treated animals. OVA₃₂₃₋₃₃₉-PLG treated animals had positive staining for CD45, CD4, and CD11b; whereas, PLP₁₃₉₋₁₅₁-PLG treated animals had minimal staining for these factors.

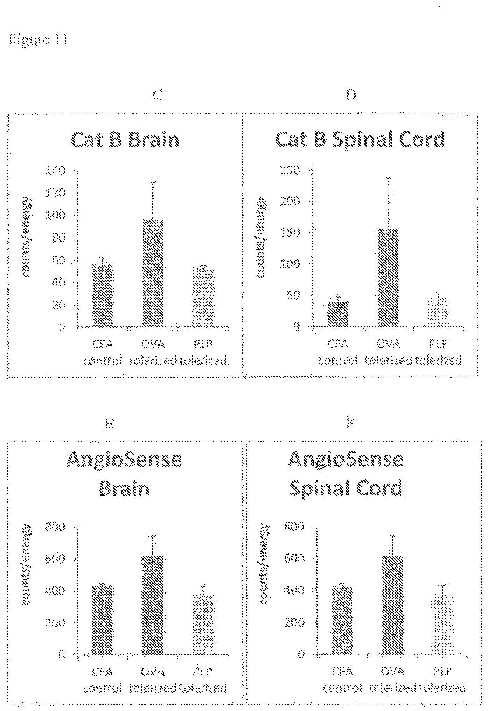

Administration of PLP₁₃₉₋₁₅₁-PLG particles also inhibits blood brain barrier (BBB) disruption and macrophage activation in the spinal cord of treated mice. Animals were treated with Complete Freund's Adjuvant (CFA), OVA₃₂₃₋₃₃₉ PLG particles, or PLP₁₃₉₋₁₅₁-PLG particles. The clinical scores and percent incidence of EAE were determined (Figure 10B) and the spinal cords observed via *in vivo* imaging (Figures 10A and 11). Angiosense measures vascular leak in the CNS and prosense reports activated macrophages (cathepsin activation cleaves the reporter revealing the fluorescent signal). The bar graphs give the numerical numbers to the signal strength shown in the brain and SC scans.

Tolerance can also be induced by particles in which the antigen has been encapsulated. Figure 12 shows that the administration of PLG particles in which PLP₁₃₉₋₁₅₁ has been encapsulated inhibits the induction of R-EAE in mice. The ability to encapuslate autoantigens allows for the used of complex mixtures of proteins or even organ homogenates to achieve more antigen coverage and thus more effectively deal with epitope spreading.

### Example 8

### Tolerance induced by PLP-₁₃₉₋₁₅₁ coupled PLGA particles is partially dependent on the expansion/activation of regulatory T-cells

SJL/J mice were treated with an anti-CD25 antibody, a common marker for regulatory T cells (Tregs) on Day -9, and then on Day -7 were treated with either OVA₃₂₃₋₃₃₉ PLG particles and anti-CD25 antibody, OVA₃₂₃₋₃₃₉ PLG particles and a control IgG antibody, PLP₁₃₉₋₁₅₁-PLG particles and an anti-CD25 antibody, or PLP₁₃₉₋₁₅₁-PLG particles and a control IgG antibody. As shown in Figure 13, animals treated with the PLP₁₃₉₋₁₅₁-PLG particles and the anti-CD25 antibody demonstrated, at times, a greater mean clinical score than those animals treated with PLP₁₃₉₋₁₅₁-PLG particles and a control IgG antibody. This confirms that Tregs, or at least T cells expressing CD25, play a role in the initiation of tolerance.

### Example 9

### Therapeutic tolerance is induced by PLP₁₃₉₋₁₅₁-PLG particles in active and adoptive EAE

Therapeutic tolerance induced by PLP₁₃₉₋₁₅₁-PLG particles was compared in active and adoptive EAE. Adoptive EAE was induced in six to eight-week old female SJL/J mice by adoptive transfer of 2.5x10⁶ PLP₁₃₉₋₁₅₁ -activated blasts. Mice were injected iv with PLP₁₃₉₋₁₅₁ (squares) or OVA₃₂₃₋₃₃₉ (circles) peptide coupled to 500nm PLG nanoparticles 2 days (Figure 14A), 14 days (Figure 14C), 18 days (Figure 14E), or 21 days (Figure 14F) following disease induction. Clinical disease scores were compared to those following treatment with antigen-coupled splenocytes (Figure 14A). Brain and spinal cord were collected from PLP₁₃₉₋₁₅₁ - or OVA₃₂₃₋₃₃₉ -tolerized mice for histological analysis on day 42. Sections from mice from panel A were stained for PLP protein and CD45 (Figure 14B). Spinal cord sections from mice from panel C were stained with Luxol Fast Blue (Figure 14D). Areas of demyelination and cellular infiltration are indicated by arrows. The results show that tolerance is induced by PLP₁₃₉₋₁₅₁-PLG particles in mice with adoptive EAE.

Figure 15 shows graphs depicting the mean clinical scores of mice with active EAE and adoptive EAE after treatment with either SP or PLG particles conjugated to OVA₃₂₃₋₃₃₉ or PLP₁₃₉₋₁₅₁. Mice were injected iv with PLP₁₃₉₋₁₅₁-SP, PLP₁₃₉₋₁₅₁-PLG, or OVA₃₂₃₋₃₃₉-SP, or OVA₃₂₃₋₃₃₉ - PLG peptide coupled to 500nm nanoparticles 10 days (Figure 15A) or 2 days (Figure 15B) following disease induction and the mean clinical score was determined. In both cases, administration of PLP₁₃₉₋₁₅₁-PLG particles reduced disease, indicative of tolerance induction.

The infiltration of central nervous system immune cells is also drastically reduced in PLP-PLG tolerized mice. SJL/J mice were injected i.v. with 500nm PLG nanoparticles coupled with PLP₁₃₉₋₁₅₁ (squares) or OVA₃₂₃₋₃₃₉ (circles) 2 days following EAE induction by adoptive transfer. At the peak of disease (day 14) brains and spinal cords were removed and the number of lymphocytes (Figure 16B), APCs (Figure 16C), microglia (Figure 16D), peripheral dendritic cells (Figure 16E), myeloid dendritic cells (Figure 16F) and macrophages (Figure 16G) were enumerated by flow cytometry. The gating strategy for these populations is depicted in (Figure 16A). CNS cell preparations were stimulated with PMA and ionomycin for 5 h prior to intracellular staining for IL-17A and IFN-γ (Figure 16H).

### Example 10

### Treatment with an anti-PD-1 monoclonal antibody abrogates tolerance induction with PLG nanoparticles encapsulating PLP₁₃₉₋₁₅₁ in adoptive transfer EAE

To test the effect of treatment with an anti-PD-1 antibody on PLP₁₃₉₋₁₅₁ induced tolerance in mice with adoptive EAE, on Day 0, mice received 3x10⁶ PLP₁₃₉₋₁₅₁ activated T-cell blasts via intravenous administration. On Day 2, they received PLP₁₃₉₋₁₅₁ or OVA₃₂₃₋₃₃₉ encapsulated in PLG particles via intravenous administration with either PBS or an anti-PD-1 antibody. On days 4, 6, 8, 10, and 12 all animals received either 250µg anti-PD-1 antibody or PBS.

As shown in Figure 17, administration of the PLP₁₃₉₋₁₅₁ peptide encapsulated in a PLG particle induces tolerance when the particle is administered with PBS. However, administration of the anti-PD-1 antibody decreases this tolerance.

### Example 11

### Treatment with an agonistic anti-CD40 monoclonal antibody abrogates tolerance induction with PLG nanoparticles encapsulating PLP₁₃₉₋₁₅₁ in adoptive transfer EAE in an IL-12 dependent manner

To test the effect of treatment with an agonistic anti-CD40 antibody on PLP₁₃₉₋₁₅₁ induced tolerance in mice with adoptive EAE, on Day 0, mice received 3x10⁶ PLP₁₃₉₋₁₅₁ activated T-cell blasts via intravenous administration. On Day 2, the mice received PLP₁₃₉₋₁₅₁ or OVA₃₂₃₋₃₃₉ encapsulated in PLG particles via intravenous administration. On day 3, the animals received a control IgG2a antibody, an anti-CD40 antibody, or an anti-CD40 antibody and an anti-Il-12 antibody.

As shown in Figure 18, administration of the PLP₁₃₉₋₁₅₁ peptide encapsulated in a PLG particle induces tolerance when the particle is administered with PBS. Administration of the agonistic anti-CD40 antibody decreases this tolerance, but this decrease in tolerance is reversed by the addition of an anti-IL-12 antibody.

### Example 12

### OVA encapsulated in PLG particles prophylactically inhibits allergic airway inflammation and in vivo OVA-specific Th2 responses

To test the prophylactic effect of OVA encapsulated in PLG particles on airway inflammation, mice were treated intravenously with OVA-PLG at day -7. On day 0, the mice received intraperitoneal injections of OVA/Alum at a dose of 10µg/mouse. On day 7, the mice were again treated intravenously with OVA-PLG and received another 10µg/mouse ip injection of OVA/Alum on day 14. Between days 28 and 30, the mice were treated three times with aerosolized OVA.

As shown in Figure 19, the prophylactic administration of OVA-PLG decreased the secretion of IL-4, IL-5, IL-13 and IL-10, and reduced the levels of serum OVA IgE and eosinophils in the lung.

OVA Encapsulated in PLG particles prophylactically inhibits OVA-specific in vitro recall responses from mediastinal lymph nodes. As shown in Figure 20A, the lymph node proliferation observed after restimulation with 25 µg OVA is decreased in those animals treated with OVA-PLG. Moreover treatment with OVA-PLG decreases the release of cytokines after restimulation with OVA. Figure 20B shows that levels of IL-4, IL-5, IL-13, and IL-10 are decreased in mice treated with OVA-PLG.

### Example 13

### OVA encapsulated in PLG particles therapeutically inhibits allergic airway inflammation and in vivo OVA-specific Th2 responses

To test the therapeutic effect of OVA encapsulated in PLG particles on airway inflammation, mice were treated intraperitoneally with OVA/Alum at a dose of 10µg/mouse on day 0 and day 14. The mice were intravenously administered with OVA-PLG on days 28, and 42. Between days 56-58, the mice were treated three times with aerosolized OVA.

As shown in Figure 21, the therapeutic administration of OVA-PLG decreased the secretion of IL-4, IL-5, IL-13 and IL-10, and reduced the levels of serum OVA IgE and eosinophils in the lung.

Figure 22 shows OVA Encapsulated in PLG particles therapeutically downregulates OVA-Specific Th2 Cytokines in the BAL Fluid Better than OVA-coupled PLG particles. The animals were treated as described above except that on days 28 and 42, the mice were treated with either OVA encapsulated in PLG particles, or OVA coupled to PLG particles. Surprisingly, the encapsulated OVA inhibited the secretion of Th2 cytokines more than the OVA peptide coupled to the surface of the PLG particle.

### Example 14

### Tolerance induced by chromogranis A p31 peptide-PLG particles inhibits Type 1 diabetes

Type 1diabetes was induced in BDC2.5 mice by isolating spleen, axillary, brachial, inguinal, and pancreatic lymph node cells from mice at 3 weeks. The isolated cells were cultured and activated in vitro by incubating 2x10⁶ cells/mL with 0.5µM p31 peptide for 96 hours. 5x10⁶ cells were transferred via intravenous administration to NOD.SCID mice (6-8 weeks) at Time 0. The mice were tolerized via intravenous administration with p31 or MOG₃₅₋₅₅ peptide coupled to SP or PLG 2 hours to 3 days later.

Figures 23A and 23B show the blood glucose levels in the animals after treatment. Administration of the p31 peptide coupled PLG resulted in lower blood glucose levels compared to those seen after administration with the MOG₃₅₋₅₅ peptide coupled particles. Figure 23C shows that the percent of IFNγ secreting cells observed in the animals was also reduced in the p31-PLG treated mice compared with the MOG₃₅₋₅₅ peptide-PLG treated mice.

p31-PLG induced tolerance requires Tregs. Type 1 diabetes was induced in mice as described above, and 2 hours after the activated cells were transferred to the NOD.SCID mice, the mice were tolerized with either p31-PLG or MOG₃₅₋₅₅ PLG particles. As shown in Figure 24, depletion of Tregs abrogates the tolerance induced by administration of p31-PLG particles.

### Example 15

### Tolerance induced by insulin-coupled PLG particles inhibits the development of spontaneous type 1 diabetes in NOD mice

NOD mice were treated with either BSA (N=22) or insulin (N=23) coupled PLG particles via intravenous administration at 6, 8, and 10 weeks of age. The mice were then assayed for the development of diabetes which was defined as blood glucose >250 mg/dL. As shown in Figure 25, administration of the insulin coupled PLG particles significantly increased the percentage of mice that did not develop diabetes over 300 days (69.6% compared to 22.7%; p=0.0027).

### Example 16

### Engraftment kinetics

Female CD45.2 mice were tolerized with either OVA-PLG or the control peptide Dby-PLG (the major H-Y antigen expressed by Male C57BL/6 mice) on day -7. On day -1, the mice were irradiated with 200 rads and were then transplanted with 1x10⁶, 5x10⁶, or 1x10⁷ bone marrow cells from male CD45.1 mice on day 0. The recipient mice were then tolerized with either OVA-PLG, Dby-SP, or Dby-PLG on day 1 and the blood harvested for FACS analysis of chimerism. Figure 26 shows the percent of CD45.1 donor cells observed in the recipient mice.

Figure 27 shows the percent of donor CD45.1 cells in the recipient mice after tolerization with either OVA-PLG, Dby-SP, or Dby-PLG on day 1. One positive control mouse did not demonstrate significant engraftment (∼10%). All negative control mice did not engraft donor cells. One Dby-SP mouse did not demonstrate significant engraftment (∼10%). Two OVA-PLG mice engrafted donor cells (∼10%): one completely rejected by Week 16. One Dby-PLG mouse started to reject at Week 12 and was at 10% by Week 16. The Dby-PLG group ranged from 10%-56% engraftment by Week 16. The OVA-PLG mice demonstrated: 1) Spontaneous engraftment, 2) Sequence homology between OVA323 and Dby, or 3) tolerogenic properties of particles. Dby-PLG allows for more engraftment than Dby-SP and OVA-PLG.

Figure 28 shows that the timing tolerance has an effect on the percent of CD45.1 cells in the recipient mouse. Positive Controls show less engraftment (∼4%) than expected (∼10%). One Negative control mouse had 5% engraftment Out of all 3 OVA-PLG groups, one mouse in the Day -7, Day +1 group showed engraftment (12%). Tolerance on day 1 is more clinically relevant than tolerance on day -7.

### Example 17

### Coumarin-6 PLGA particles are not detectable 24 hours after administration

Mice were treated with coumarin-6 PLGA particles that were either coupled to an antigen or antigen-free. As shown in Figure 29, the particles were detectable at 3 hours post-administration, but not at 24 hours post-administration. Naive uninjected mouse (top row) as compared to i.v. fluorescent PLGA/PEMA microparticle injected mouse spleen (left column), liver (middle column) and lung (left column) sections at 3-hours post injection (middle row) and 24-hours (bottom row) post-injection, counterstained with DAPI.

### Example 18

### Nanoparticles are associated with macrophages in vivo

Analysis of the liver 6 hours and 15 hours post-administration shows that PLGA particles co-localized with F4/80⁺ cells in the liver (Figure 30).

The marginal zone macrophages predominantly uptake TAMRA-labeled PLP₁₃₉₋₁₅₁-coupled particles 24 hours after intravenous infusion. As shown in Figure 31, the highest percentage of PLP₁₃₉₋₁₅₁+ cells are marginal zone macrophages.

### Example 19

### Inhibition of R-EAE in SJL/J mice using surface-functionalized poly(lactide-co-glycolide) particles containing soluble PLP139-151 within their cores.

Groups of SJL/J mice were injected IV with 2.5 mg 500 nm - 700 nm surface-functionalized poly(lactide-co-glycolide) particles with soluble PLP139-151 peptide within their cores on Day -7 and Day -1 before relative to priming with PLP139- 151/CFA on Day 0. Control mice were primed on Day 0 but did not receive particle treatment on Day -7 or Day - 1. Mice were observed for clinical signs of R-EAE for an additional 20 days.

The results depicted in Figure 32 depict the daily mean clinical score against the number of days PLP139-151/CFA priming. PLP139-151/CFA-induced R-EAE is inhibited in SJL/J mice by the induction of immunological tolerance using surface-functionalized poly(lactide-co-glycolide) particles containing soluble PLP139-151 within their cores.

### Example 20

### Inhibition of allergic airway inflammation by surface-functionalized poly(lactide-co-glycolide) particles containing soluble ovalbumin

Allergic airway inflammation (AIA) was induced in mice. Groups of Balb/c mice were injected intravenously with 2.5 mg 500 nm - 700 nm surface-functionalized poly(lactide-co-glycolide) particles with soluble ovalbumin or soluble bovine serum albumin (control) within their cores on Day -7 and Day +7 before priming with ovalbumin/alum on Days 0 and +14. Mice were challenged on Days +28-30 with aerosolized ovalbumin. Mice were then sacrificed and bronchoalveolar lavage fluid obtained. The serum levels of ovalbumin specific IgE were measured also.

Eosinophil counts within the bronchoalveolar lavage fluid indicate the severity of AAI - higher counts indicated worse disease. Serum levels of IgE indicate the severity of AAI - higher levels indicated worse disease.

Figure 33 shows that mice treated with encapsulated OVA-PLG showed the greatest reduction in eosinophil accumulation. Figure 34 shows that mice treated with encapsulated OVA-PLG showed the greatest reduction in serum IgE levels compared to untreated or control treated animals.

Ovalbumin/alum-induced allergic airway inflammation in Balb/c mice was inhibited by the induction of immunological tolerance using surface-functionalized poly(lactide-*co*-glycolide) particles containing soluble ovalbumin within their cores.

### Example 21

### _Synthesis of surface-functionalized poly(lactide-co-glycolide) particles encapsulating antigen

The present Example details the formulation and partial characterization of biodegradable poly(lactide-*co*-glycolide) particles that have been surface-functionalized with a high density of carboxylate groups and contain soluble antigen within their cores that are surrounded by a shell of poly(lactide-co-glycolide) for tolerance induction in autoimmune disease and for the treatment of allergies.

The high density of carboxylate groups was achieved by the use of poly(ethylene-*alt*-maleic anhydride (PEMA)), a polymer with carboxylate groups incorporated into its backbone, as the surfactant for the emulsification process.

As described above, biodegradable poly(lactide-*co*-glycolide) particles containing soluble PLP139-151 within their cores and surface-functionalized with a high density of carboxylate groups are effective for the induction of immunological tolerance in the SJL/J PLP139-151/CFA-induced R-EAE murine model of multiple sclerosis. Furthermore, biodegradable poly(lactide-co-glycolide) particles containing soluble ovalbumin within their cores and surface-functionalized with a high density of carboxylate groups are effective for the induction of immunological tolerance in the Balb/c ovalbumin/alum-induced AAI murine model of allergic asthma.

Poly(lactide-co-glycolide) particles containing soluble ovalbumin or bovine serum albumin within their cores and surface-functionalized with a high density of carboxylate groups were synthesized using a double emulsion-solvent evaporation method as follows:
1. 150 µL of 200 mg/mL ovalbumin or bovine serum albumin in endotoxin-free water was added dropwise to 2 mL of 20% w/v poly(lactide-co-glycolide) in dichloromethane in a 20 mL scintillation vial.
2. The resultant mixture was placed on ice and sonicated for 30 seconds at 10 watts using a probe sonicator.
3. 10 mL of 1% w/v poly(ethylene-*alt*-maleic anhydride) in water was added.
4. The resultant mixture was placed on ice and sonicated for 30 seconds at 16 watts using a probe sonicator.
5. The resultant emulsion was poured into 200 mL 0.5% w/v poly(ethylene-*alt*-maleic anhydride) in a 600 mL beaker and stirred overnight to allow for particle hardening.
6. The hardened particles were then purified by centrifugation and washed 3 times with bicarbonate buffer pH 9.6.
7. The purified particles were resuspended in 4% w/v sucrose and 3% w/v D-mannitol in water, flash-frozen in liquid nitrogen and lyophilized to dryness.

Figure 35 shows the characterization of surface-functionalized poly(lactide-*co*-glycolide) particles containing soluble PLP139-151 within their cores by dynamic light scattering analysis. Surface-functionalized poly(lactide-co-glycolide) particles were analyzed on a Malvern Zetasizer Nano ZS (Malvern Instruments, Westborough, MA) at a count rate of 1.792 x 105 counts per second in 18.2 MΩ water. The population of surface-functionalized poly(lactide-*co*-glycolide) particles had a Z-average diameter of 584 nm, a peak diameter of 679 nm and a polydispersity index of 0.162. These results are representative of 6 batches of syntheses, following the protocol written above.

Figure 36 shows the characterization of surface-functionalized poly(lactide-*co*-glycolide) particles containing soluble PLP139-151 within their cores by ζ-potential measurement. Surfacefunctionalized poly(lactide-co-glycolide) particles were analyzed on a Malvern Zetasizer Nano ZS (Malvern Instruments, Westborough, MA) at a count rate of 6.67 x 104 counts per second in 18.2 MΩ water. The population of surface-functionalized poly(lactide-*co*-glycolide) particles had a peak ζ-potential of -48.9 mV and a ζ deviation of 5.14 mV. These results are representative of 6 batches of syntheses, following the protocol written above.

Figure 37 shows the characterization of surface-functionalized poly(lactide-*co*-glycolide) particles containing soluble ovalbumin within their cores by dynamic light scattering analysis. Surface-functionalized poly(lactide-*co*-glycolide) particles were analyzed on a Malvern Zetasizer Nano ZS (Malvern Instruments, Westborough, MA) at a count rate of 1.822 x 105 counts per second in 18.2 MΩ water. The population of surface-functionalized poly(lactide-co-glycolide) particles had a Z-average diameter of 569.7 nm, a peak diameter of 700.3 nm and a polydispersity index of 0.230. These results are representative of 3 batches of syntheses, following the protocol written above.

Figure 38 shows Characterization of surface-functionalized poly(lactide-co-glycolide) particles containing soluble ovalbumin within their cores by ζ-potential measurement. Surfacefunctionalized poly(lactide-co-glycolide) particles were analyzed on a Malvern Zetasizer Nano ZS (Malvern Instruments, Westborough, MA) at a count rate of 2.67 x 104 counts per second in 18.2 MΩ water. The population of surface-functionalized poly(lactide-co-glycolide) particles had a peak ζ-potential of -52.2 mV and a ζ deviation of 5.38 mV. These results are representative of 3 batches of syntheses, following the protocol written above.

### Example 22

### Surface-functionalized liposomes containing soluble PLP₁₃₉₋₁₅₁ within their cores induce immunological tolerance in the murine R-EAE model of multiple sclerosis

The present inventors have also discovered that biodegradable liposomal delivery vehicles that have been surface-functionalized with a high density of negatively-charged groups and contain soluble antigen within their cores induce immunological tolerance in the R-EAE murine model of multiple sclerosis.

The liposomes used in this study were composed of the following lipids at the following molar ratios - 30:30:40 phosphatidylcholine:phosphatidylglycerol:cholesterol. Groups of SJL/J mice were injected IV with 200 nm nm surface-functionalized liposomes (10 µmol total lipid per mouse) with soluble PLP₁₃₉₋₁₅₁ peptide within their cores on Day -7 relative to priming with PLP₁₃₉₋₁₅₁/CFA on Day 0. Control mice were primed on Day 0 and received 500 nm - 700 nm surface-functionalized liposomes (10 µmol total lipid per mouse) with soluble OVA₃₂₃₋₃₃₉ peptide within their cores on Day -7. Mice were observed for clinical signs of R-EAE for an additional 17 days.

The results depict the daily mean clinical score against the number of days PLP₁₃₉₋₁₅₁/CFA priming. As shown in Figure 39, the animals treated with the surface-functionalized liposomes with soluble PLP₁₃₉₋₁₅₁ peptide within their cores had a lower clinical score than those animals treated with the surface-functionalized liposomes containing soluble OVA₃₂₃₋₃₃₉ peptide.

The results of this study demonstrate that biodegradable liposomes containing soluble PLP₁₃₉₋₁₅₁ within their cores and surface-functionalized with high density of negatively-charged groups are effective for the induction of immunological tolerance in the SJL/J PLP₁₃₉₋₁₅₁/CFA-induced R-EAE murine model of multiple sclerosis.

The tolerance induced by antigen-coupled or antigen-encapsulated particles is antigen-specific, dose dependent and long-lasting (>150 days). Tolerance is best induced by intravenous administration of a coupled particle that is between 500nm and 1µm in diameter with a zeta potential ≤ -5- mV. The induction of tolerance is dependent on the uptake of the particles by the MARCO scavenger receptor with sees polyanionic surfaces (e.g. carboxylated PS/PLG particles). The tolerance is induced and maintained by a combination of anergy (partially reversed by anti-PD-1 and agonistic anti-CD40 antibodies) and iTregs (partially reversed by anti-CD25 antibodies). The particles accumulate predominantly in the liver and splenic marginal zone macrophages (CD11b^{hi} CD11c^{lo} MARCO⁺ Sign-R1⁺ Siglec-1⁻).

There are numerous advantages of using antigen-coupled particles for the treatment of autoimmune diseases compared with using antigen-pulsed or antigen-directed immature tolerogenic dendritic cells or engineering antigen-specific Tregs. The rapidity and simplicity of tolerogen preparation and induction using a GMP manufacturable, off-the-shelf universal tolerogenic carrier; there is no need to isolate and expand immature dendritic cells or Tregs ex vivo; there is no need to be concerned with immature dendritic cells being activated upon ex vivo manipulation and becoming stimulatory rather than tolerogenic or of Tregs converting to Th1/17 after transfer; since the hose immature marginal zone APCs process and represent the antigen in a tolerogenic manner, host APCs can select the relevant immunodominant self epitopes from PLG particles encapsulating intact auto-antigens or tissue extracts (e.g. OVA encapsulated PLG particles prevent OVA/Alum-induced AAD); and the protocol is antigen-specific with no bystander suppression, is safe, highly efficient, and can induce unresponsiveness in both effector T cells (Th1, Th2, Th17, and CD8) and naive T cells involved with epitope spreading.

Synthetic, biodegradable particles and liposomes could lead to ease of manufacturing, broad availability of therapeutic agents, and increase the number of potential treatment sites. To this end, we have specifically engineered surface-functionalized biodegradable poly(lactide*co*- glycolide) particles with a high density of surface carboxylate groups, using the surfactant poly(ethylene-*alt*-maleic anhydride).

We have also developed surface-functionalized liposomes using a 30:30:40 ratio of phosphatidylcholine :pho sphatidylglycerol:cholesterol.

We have further engineered these particles to contain soluble ovalbumin within their cores so as to circumvent chemical contamination and purity issues surrounding surface-conjugation of peptide or protein. These surface-functionalized poly(lactide-co-glycolide) particles containing soluble ovalbumin within their cores are effective for the prevention of disease development and hence the induction of immunological tolerance in the Balb/c ovalbumin /alum-induced AAI murine model of allergic asthma. Peptide or protein conjugated to carboxylate-functionalized poly(lactide-*co*-glycolide) particles using EDC are attached in an indiscriminate fashion, resulting in antigen aggregates and particle-antigen-particle aggregates that are difficult to characterize and purify into homogeneous populations.

We have produced a homogeneous population of surface functionalized poly(lactide co-glycolide) particles containing soluble ovalbumin within their cores that do not require surface conjugation of antigen.

We have further demonstrated that biodegradable liposomes containing soluble PLP₁₃₉₋₁₅₁ within their cores and surface-functionalized with high density of negatively-charged groups are effective for the induction of immunological tolerance in the SJL/J PLP₁₃₉₋₁₅₁/CFA-induced R-EAE murine model of multiple sclerosis.

The liposomes and poly(lactide-*co*-glycolide) particles of the present invention offer numerous advantages. The advantages include:
1) Biodegradable particles will not persist for long times in the body, and the time for complete degradation can be controlled.
2) Particles and liposomes can be functionalized to facilitate internalization without cell activation. Toward this goal, we have loaded phosphatidylserine into PLG microspheres.
3) Particles and liposomes can also be designed to incorporate targeting ligands for a specific cell population.
4) Anti-inflammatory cytokines such as IL-10 and TGF-β, can also be included to limit activation of the cell type that is internalizing the particles and to facilitate the induction of tolerance *via* anergy and/or deletion and the activation of regulatory T cells.

This combinatorial function of the particle or liposome can target tolerance induction from multiple perspectives, thus designer particles are a significant advance relative to the polystyrene particles. Potential clinical applications of this tolerance inducing technology include:
(1) T cell- and antibody-mediated autoimmune diseases (such as multiple sclerosis, type 1 diabetes, rheumatoid arthritis, systemic lupus, *etc.)* - tolerance would be induced with particles complexed with the relevant autoantigens driving the particular autoimmune disease
(2) food and lung allergies, skin allergies, and asthma - tolerance would be induced with particles complexed with the specific foods (*e.g.* peanut proteins, *etc*.), injected (bee venom proteins, *etc*.), or inhaled substances (*e.g*., ragweed pollen proteins, pet dander proteins, *etc*.) which elicit the allergic reaction
(3) transplant rejection - tolerance would be induced to the transplant antigens on donor organs or cells prior to organ transplant to prevent rejection by the recipient
(4) enzyme replacement therapy - tolerance would be induced to enzymes which patients with genetic deficiencies fail to produce, to prevent them from making neutralizing antibody responses to recombinantly-produced enzymes administered to treat their particular deficiency.

### Example 23

### The particles most effective at inducing tolerance are negatively charged and an average diameter of 500nm

The key particle parameters for inducing tolerance are the size and charge of the composition. As shown in Figure 40A and B, the charge of the particle affects the efficacy of tolerance induction. A comparison of EAE mice treated with OVA conjugated particles with a -25mv or -60mv charge found that compositions comprising particles with a charge of - 60mv induce tolerance more effectively than those with a -25mV charge. Mice were treated with TIMP (tolerogenic immune modifying particles) having a charge of either -60mv or-25mv. Mice were treated with either OVA₃₂₃₋₃₃₉-TIMP-₆₀ₘᵥ, OVA₃₂₃₋₃₃₉-PLGA-₂₅ₘᵥ, PLP₁₃₉₋₁₅₁-TIMP-₆₀ₘᵥ, or PLP₁₃₉₋₁₅₁-PLGA₋₂₅ₘᵥ (all antigens are encapsulated) and scored for clinical disease. Panel (A) shows the mean clinical score and Panel (B) shows the mean cumulative score of the EAE animals.

The negative charge on the particle affects the ability of the particle to interaction with the MARCO scavenger receptor. Figure 41 shows that the charge of the immune-modifying particle is important for targeting the immune modifying particle to the antigen presenting cell. Wild type or MARCO -/+ animals were treated with either PS-IMP or vehicle. The results indicate that particles with a reduced negative charge have a lower efficacy because there is less interaction with the scavenger receptors such as MARCO that have positively charged collagen-like domains.

In addition to charge, the size and composition of the biodegradable TIMPs affects the induction of tolerance. As shown in Figure 42A, the most effective particles for inducing tolerance in the EAE model are those with a mean diameter of about 500 nm. Mice were treated with either 500nm OVA₃₂₃₋₃₃₉-PSB, 100 nm PLP₁₃₉₋₁₅₁-PSB, 500 nm PLP₁₃₉₋₁₅₁-PSB, 1.75 µm PLP₁₃₉₋₁₅₁-PSB, or 4.5 µm PLP₁₃₉₋₁₅₁-PSB and scored for clinical disease. PLGA carriers have slow release kinetics for over 1 month and changing the polymer ratio can impact release of the particles. Tolerance requires rapid particle uptake and clearance/degradation. Since ratios of over 50:50 lactide:glycolide slow the degradation rate, the particles of the invention in one embodiment are 50:50 lactide:glycolide. Figure 42B shows that the particles are rapidly destroyed.

In addition to the charge and average mean diameter of the TIMPs, antigens which are encapsulated within the particle are superior to particles coupled to antigen in the allergy model. In the allergy model, coupled nanoparticles have a propensity to cause anaphylaxis and are not an effective therapy. Conversely, as shown in Figure 43, TIMPs with a charge of -60mv are therapeutically effective in the murine allergy model. Animals were exposed to OVA as an allergen, and were then treated with either a sham-PLG or TIMP particle, a PLG or TIMP particle with OVA, or no treatment. Panel (A) shows that OVA-PLG particles fail to reduce the TH2 response in allergy. Panel (B) shows that TIMP_{PEMA-60mv} inhibit this TH2 response. Panel (C) shows that TIMP_{PEMA-60mv} inhibit recall responses.

### Example 24

### Single-emulsion synthesis of surface-functionalized poly(lactide-co-glycolide) particles encapsulating antigen

Polypeptide antigens can be incorporated into poly(lactide-*co*-glycolide) particles using a double-emulsion process (See, Example 21), however, the present inventors have found that when incorporating more hydrophobic polypeptides, such as gliaden, it is better to incorporate the antigen into the particle with a single-emulsion process using solvents.

Poly(lactide-co-glycolide) with carboxylate end groups, a 50:50 D,L-lactide:glycolide ratio, and inherent viscosity of 0.18 dl/g in hexafluoro-2-propanol was used to create particles containing gliaden. Poly(lactide-co-glycolide) particles containing gliaden within their cores and surface-functionalized with a high density of carboxylate groups were synthesized using a single emulsion-solvent evaporation method as follows:
1. Five milligrams of gliaden and 200 mg PLG was dissolved in 50 µL of trifluoroacetic acid (TFA) and 700 µL dimethylsulphoxide and 1250 µL dichloromethane (DCM).
2. The resultant mixture was added drop-wise to 4 mL 1% w/v aqueous PEMA and sonicated for 30 seconds at 100% amplitude.
3. The resultant emulsion was poured into 200 mL of 0.5% w/v aqueous PEMA under stirring for 12 hours to allow the DCM to completely evaporate.
4. The particles were then washed three times in 0.1M sodium carbonate-sodium bicarbonate buffer pH 9.6. Alternatively, ddH₂O can be used to wash the particles.
5. The purified particles were resuspended in 4% w/v sucrose and 3% w/v D-mannitol in water, gradually frozen to -80°C and lyophilized to dryness.

## Claims

1. A composition comprising surface functionalized biodegradable particles comprising encapsulated gliadin or one or more antigenic gliadin epitopes, wherein the particle has a negative zeta potential and wherein said particle is between 0.1 µm and 10 µm in diameter, wherein the particles have a zeta potential of -100 mV to -40 mV, and wherein the gliadin or one or more antigenic gliadin epitopes comprises one or more of the sequences set forth in SEQ ID NOS: 1295-1724, 1726-1766, 4983-4985 and 4986-5140.

2. The composition of claim 1, wherein the particles comprise poly(lactide-co-glycolide)(PLG).

3. The composition of claim 1, wherein the surface functionalization is carboxylation, optionally wherein the carboxylation is achieved by using poly(ethylene-maleic anhydride) (PEMA).

4. The composition of claim 1, further comprising pharmaceutically acceptable excipients.

## Patentansprüche

1. Zusammensetzung, umfassend oberflächenfunktionalisierte biologisch abbaubare Teilchen, umfassend eingekapseltes Gliadin oder ein oder mehrere antigenische Gliadin-Epitope, wobei das Teilchen ein negatives Zeta-Potential aufweist und wobei das Teilchen im Durchmesser zwischen 0,1 µm und 10 µm groß ist, wobei die Teilchen ein Zeta-Potential von -100 mV bis -40 mV aufweisen und wobei das Gliadin oder das eine oder die mehreren antigenische Gliadin-Epitope eine oder mehrere der Sequenzen umfassen, die in SEQ-ID-Nr: 1295-1724, 1726-1766, 4983-4985 und 4986-5140 dargelegt sind.

2. Zusammensetzung nach Anspruch 1, wobei die Teilchen Poly(laktid-glykolid) (PLG) umfassen.

3. Zusammensetzung nach Anspruch 1, wobei die Oberflächenfunktionalisierung Carboxylierung ist, wobei optional die Carboxylierung durch Verwenden von Poly(ethylen-maleinsäureanhydrid) (PEMA) erreicht wird.

4. Zusammensetzung nach Anspruch 1, weiter umfassend pharmazeutisch akzeptable Trägerstoffe.

## Revendications

1. Composition comprenant des particules biodégradables fonctionnalisées en surface comprenant de la gliadine encapsulée ou un ou plusieurs épitopes de gliadine antigéniques, dans laquelle la particule a un potentiel zêta négatif et dans laquelle ladite particule a un diamètre compris entre 0,1 µm et 10 µm, dans laquelle les particules ont un potentiel zêta de -100 mV à -40 mV et dans laquelle la gliadine ou un ou plusieurs épitopes de gliadine antigéniques comprend/comprennent une ou plusieurs des séquences établies dans les SEQ ID N°: 1295-1724, 1726-1766, 4983-4985 et 4986-5140.

2. Composition selon la revendication 1, dans laquelle les particules comprennent du poly(lactide-co-glycolide) (PLG) .

3. Composition selon la revendication 1, dans laquelle la fonctionnalisation de surface est une carboxylation, facultativement dans laquelle la carboxylation est réalisée en utilisant du poly(éthylène-anhydride maléique) (PEMA) .

4. Composition selon la revendication 1, comprenant en outre des excipients pharmaceutiquement acceptables.
